# EUROPEAN PATENT APPLICATION

(11) **EP 4 711 356 A1**
(43) Date of publication of application: **18.03.2026**
(21) Application number: 24803009.0
(22) Date of filing: 08.05.2024
(51) Int. Cl.: C07D 209/88, C07D 471/04, C07D 487/04, A61K 31/403, A61K 31/437, A61P 35/00

(54) **TETRAHYDROCARBAZOLE DERIVATIVE, PHARMACEUTICAL COMPOSITION, AND USE THEREOF**

(30) Priority: 10.05.2023 CN 202310523215; 28.09.2023 CN 202311274642
(71) Applicant: Nain Biotech (Hangzhou) Co., Ltd., Hangzhou, Zhejiang 311112 (CN)
(72) Inventor: WANG, Yingjie, Hangzhou, Zhejiang 311112 (CN); JIA, Wei, Hangzhou, Zhejiang 311112 (CN)
(74) Representative: dompatent
(86) International application number: PCT/CN2024/091697
(87) International publication number: WO 2024/230729

(57) **Abstract**

The present invention relates to a tetrahydrocarbazole compound, a pharmaceutical composition thereof, and uses thereof. The tetrahydrocarbazole compound is a compound represented by formula (I) or formula (II), an optical isomer, geometric isomer, hydrate, solvate, prodrug, or pharmaceutically acceptable salt thereof. The compound provided by the present invention can be used in the preparation of a medicament for treating and/or preventing cancer.

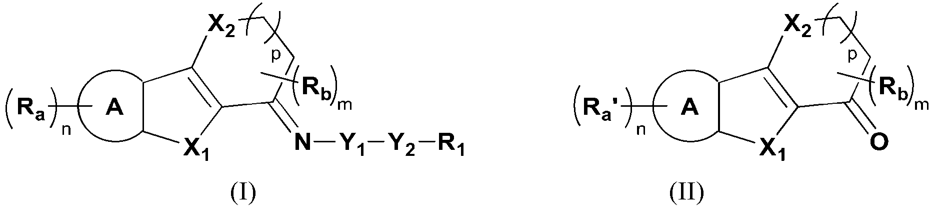

## Description

### Technical field

The present invention relates to the field of pharmaceutical technology, specifically to a tetrahydrocarbazole compound, its pharmaceutical composition, and uses thereof.

### Background technology

Tetrahydrocarbazolones are a class of synthetically produced carbazolone alkaloids that can serve as advanced intermediates for the synthesis of various naturally occurring carbazole alkaloids and other bioactive small molecule lead compounds. Tetrahydrocarbazole derivatives represent a highly significant subgroup within this category. In recent years, tetrahydrocarbazole compounds have become a hotspot in drug discovery due to their broad biological activities. Their main physiological and pharmacological effects include antitumor activity, antiviral activity, antifungal activity, and potential for treating neurodegenerative diseases (Chaudhari et al., 2021, *Org Biomol Chem 19:1926).*

Derivatizing the tetrahydrocarbazole scaffold to obtain novel-structure, unique-mechanism antitumor lead compounds has been a key focus in this research area. In recent years, tetrahydrocarbazole compounds targeting different sites have been continuously synthesized. In 2013, Tunbridge et al. found that the carbazolone derivative indanocine exhibited superior inhibitory activity against the HeLa cell line compared to the control drug ellipticine (Tunbridge et al., 2013, *MedChemComm 4:* 1452). In 2018, Murali et al. synthesized a series of carbazole derivatives with IC₅₀ values against A549 and MCF7 cells ranging from 13-20 µM (Murali et al., 2018, *Eur J Med Chem* 143: 292). In 2019, Saravanabhavan et al., starting from tetrahydrocarbazolone, synthesized a series of pyridocarbazole-based pentacyclic compounds with antitumor and antioxidant activities. These compounds showed in vitro cytotoxic IC₅₀ values against HeLa, MCF7, and HEp-2 cell lines ranging from 4 to 100 µM, and also demonstrated higher antioxidant activity than ascorbic acid (Saravanabhavan et al., 2019, *New J Chem* 43: 17231).

In summary, tetrahydrocarbazole derivatives exhibit certain antiproliferative and cytotoxic effects against various human tumor cell lines, making them valuable precursor compound or lead compounds for the development of novel antitumor drugs. However, the IC₅₀ values of currently reported tetrahydrocarbazole derivatives against human tumor cell lines are generally above 1 µM, indicating that their antitumor activity still requires further enhancement.

### Content of invention

In view of the aforementioned deficiencies in the prior art, either in whole or in part, the present invention provides a class of tetrahydrocarbazole compounds, pharmaceutical compositions, and uses thereof. The provided tetrahydrocarbazole compounds exhibit significantly enhanced pharmacological effects, with an IC₅₀ below 0.2 µM against human tumor cell lines. They can be used as ideal lead compounds in the development of drugs for treating and/or preventing cancer.

In the first aspect of the present invention, a compound represented by formula (I) or formula (II), a stereoisomer, geometric isomer, hydrate, solvate, prodrug, or pharmaceutically acceptable salt thereof is provided, 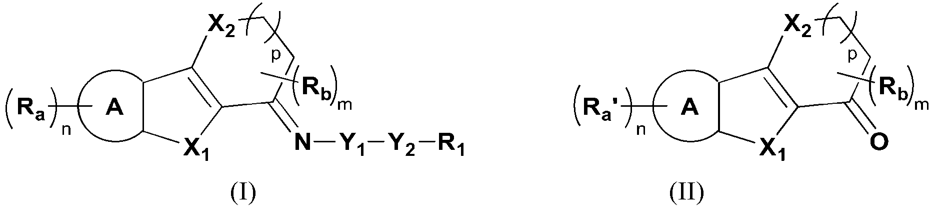
wherein ring A is selected from the group consisting of C₃-C₇ saturated or partially unsaturated carbocycle, 3-8 membered saturated or partially unsaturated heterocycle, C₆-C₁₀ aromatic ring, and 5-12 membered heteroaromatic ring; ring A may be a monocyclic, spirocyclic, fused, or bridged ring structure;
Y₁ and Y₂ are each independently selected from the group consisting of chemical bond, C₁-C₆ alkylene, C₃-C₁₀ saturated or partially unsaturated carbocycle, 3-10 membered saturated or partially unsaturated heterocycle, C₆-C₁₀ aromatic ring, 5-12 membered heteroaromatic ring, -NH-, 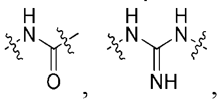 O or S; and Y₁ and Y₂ are each independently optionally substituted with 0 to 4 substituents selected from the group consisting of deuterium, hydroxyl, halogen, oxo (=O), 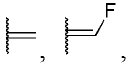 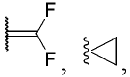 carboxyl, NH₂, mercapto, C₁-C₄ alkyl, C₂-C₄ alkenyl, C₂-C₄ alkynyl, C₂-C₄ acyl, C₂-C₆ ester group, C₁-C₄ alkoxy, C₁-C₄ alkylthio, C₁-C₄ haloalkyl, C₂-C₄ haloacyl, C₁-C₄ haloalkoxy, C₁-C₄ haloalkylthio, C₃-C₇ saturated or partially unsaturated carbocyclyl, 3-8 membered saturated or partially unsaturated heterocyclyl, C₆-C₁₀ aryl, and C₅-C₁₂ heteroaryl.

R₁ is selected from the group consisting of: hydrogen, deuterium, C₁-C₆ alkyl, C₂-C₆ alkenyl, C₂-C₆ alkynyl, C₁-C₆ alkylamino (C₁-C₆ alkyl-NH-), C₁-C₆ alkoxy, C₁-C₆ haloalkyl, C₂-C₆ haloalkenyl, C₂-C₆ haloalkynyl, C₁-C₆ haloalkylamino, C₁-C₆ haloalkoxy, C₃-C₇ saturated or partially unsaturated carbocyclyl, 3-8 membered saturated or partially unsaturated heterocyclyl, C₆-C₁₀ aryl, 5-12 membered heteroaryl, C₁-C₄ alkyl-C₃-C₇ saturated or partially unsaturated carbocyclyl, C₁-C₄ alkyl-3-8 membered saturated or partially unsaturated heterocyclyl, C₁-C₄ alkyl-C₆-C₁₀ aryl, C₁-C₄ alkyl-5-12 membered heteroaryl, and S(O)₂(C₁-C₆ alkyl); wherein said alkyl, alkenyl, alkynyl, alkylamino, alkoxy, haloalkyl, haloalkenyl, haloalkynyl, haloalkylamino, haloalkoxy, carbocyclyl, heterocyclyl, aryl, and heteroaryl are each independently optionally substituted with 0, 1, 2, 3, or 4 substituents selected from the group consisting of: deuterium, hydroxyl, halogen, oxo group (=O), carboxyl, NH₂, mercapto, C₁-C₄ alkyl, C₂-C₄ acyl, C₂-C₆ ester group, C₁-C₄ alkoxy, C₁-C₄ alkylthio, C₁-C₄ haloalkyl, C₂-C₄ haloacyl, C₁-C₄ haloalkoxy, C₁-C₄ haloalkylthio, C₃-C₇ saturated or partially unsaturated carbocyclyl, 3-8 membered saturated or partially unsaturated heterocyclyl, C₆-C₁₀ aryl, and C₅-C₁₂ heteroaryl; said carbocyclyl, heterocyclyl, aryl, and heteroaryl may be monocyclic, spirocyclic, fused, or bridged ring structures;
X₁ is selected from the group consisting of O, NR₂, and S; wherein R₂ is selected from the group consisting of H, C₁-C₄ alkyl, S(=O)₂R₁₁, S(=O)₂(C₁-C₆ alkylene)R₁₁, C(O)R₁₁, C(O)(C₁-C₆ alkylene)R₁₁, 3-12 membered saturated or partially unsaturated heterocyclyl, and 5-12 membered heteroaryl;
and one or more hydrogen atoms on R₂ may optionally be substituted by substituents selected from the group consisting of C₁-C₄ alkyl, C₂-C₄ alkenyl, C₂-C₄ alkynyl, C₁-C₄ alkoxy, and -C₀-C₄ alkylene-C₆-C₁₀ aryl; or two substituents on R₂ together form a -(CH₂)ₛ- structure; s is 2 or 3.

X₂ is selected from the group consisting of CH₂, CD₂, CHR_{b}, NH, NR_{b}, S, S(=O), S(=O)₂, and S(=O)(=NH);
each Rₐ is independently selected from the group consisting of deuterium, hydroxyl, halogen, amino, nitro, C₁-C₄ alkyl, C₂-C₄ alkenyl, C₂-C₄ alkynyl, C₂-C₆ ester group, C₁-C₄ alkoxy, C₁-C₄ haloalkyl, C₁-C₄ haloalkoxy, C₃-C₇ saturated or partially unsaturated carbocyclyl, 3-8 membered saturated or partially unsaturated heterocyclyl, C₆-C₁₀ aryl, 5-12 membered heteroaryl, -NH-CH₂-(3-12 membered saturated or partially unsaturated heterocyclyl), -NH-CH₂-(5-12 membered heteroaryl), -P(O)R_{c1}R_{d1}, -S(O)₂R_{c1}, -S(O)₂NH₂, -S(O)(NH₂)(R_{c1}), -C(O)C(O)₂R_{c1}, - C(O)NHR_{c1}, -NH-C(O)R_{c1}, -C(O)OR_{c1}, -NH-S(O)₂R_{c1}, -N=S(O)R_{c1}R_{d1}, -CH₂-S(O)(NH)(R_{c1}), -NR_{c1}R_{d1}, and 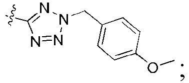 wherein the R_{c1} and R_{d1} are each independently selected from the group consisting of deuterium, C₁-C₄ alkyl, C₂-C₄ alkenyl, C₂-C₄ alkynyl, C₃-C₇ saturated or partially unsaturated carbocyclyl, 3-8 membered saturated or partially unsaturated heterocyclyl, C₆-C₁₀ aryl, and 5-12 membered heteroaryl; or R_{c1}, R_{d1}, and the atom(s) to which they are attached together form a 3-8 membered saturated or partially unsaturated heterocyclyl, or a 5-12 membered heteroaryl; wherein one or more hydrogen atoms on Rₐ may optionally be substituted by substituents selected from the group consisting of: deuterium atom, hydroxyl, halogen, oxo group (=O), 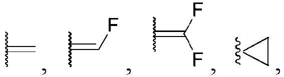 C₁-C₄ alkyl, C₂-C₄ alkenyl, C₂-C₄ alkynyl, S(O)₂CH₃, C₃-C₇ saturated or partially unsaturated carbocyclyl, -C(O)N(C₁-C₄ alkyl)₂, and -N(C₁-C₄ alkyl)₂;
each Rₐ' is independently selected from the group consisting of deuterium, hydroxyl, halogen, amino, nitro, cyano, C₁-C₄ alkyl, C₂-C₄ alkenyl, C₂-C₄ alkynyl, C₂-C₆ ester group, C₁-C₄ alkoxy, C₁-C₄ haloalkyl, C₁-C₄ haloalkoxy, C₁-C₄ amido, C₃-C₇ saturated or partially unsaturated carbocyclyl, 3-12 membered saturated or partially unsaturated heterocyclyl, C₆-C₁₀ aryl, 5-12 membered heteroaryl, -NH-CH₂-(3-12 membered saturated or partially unsaturated heterocyclyl), -NH-CH₂-(5-12 membered heteroaryl), -P(O)R_{c}R₄, -S(O)₂R_{c}, -S(O)₂NH₂, -S(O)(NH₂)(R_{c}), - C(O)C(O)₂R_{c}, -C(O)NHR_{c}, -C(O)NR_{c}R_{d}, -NH-C(O)R_{c}, -OC(O)R_{c}, -C(O)OR_{c}, - C(O)O(C₁-C₄ alkylene)R_{c}, -NH-S(O)₂R_{c}, -C(O)NH(C₁-C₄ alkylene)R_{c}, -N=S(O)R_{c}R_{d}, -CH₂-S(O)(NH)(R_{c}), -NR_{c}R_{d}, and 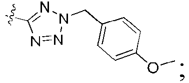
wherein R_{c} and R_{d} are each independently selected from the group consisting of deuterium, C₁-C₄ alkyl, C₁-C₄ alkoxy, C₂-C₄ alkenyl, C₂-C₄ alkynyl, C₃-C₇ saturated or partially unsaturated carbocyclyl, 3-8 membered saturated or partially unsaturated heterocyclyl, C₆-C₁₀ aryl, 8-12 membered fused bicyclic ring, 5-12 membered heteroaryl, -N(C₁-C₄ alkyl)₂, -CONH₂, and -CON(C₁-C₄ alkyl)₂; or R_{c}, R_{d}, and the nitrogen atom to which they are attached together form a 3-8 membered saturated or partially unsaturated heterocyclyl or a 5-12 membered heteroaryl; wherein one or more hydrogen atoms on Rₐ' may optionally be substituted by substituents selected from the group consisting of deuterium atom, hydroxyl, halogen, amino, cyano, carboxyl, oxo group (=O), 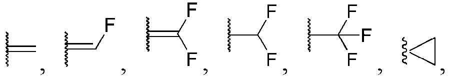 C₁-C₄ alkyl, C₁-C₄ haloalkyl, C₁-C₄ haloalkoxy, C₂-C₄ alkynyl, C₁-C₄ alkoxy, HO-(C₁-C₄ alkylene)-, C₁-C₄ alkylthio, C₂-C₄ alkenyl, C₂-C₆ ester group, S(O)₂CH₃, C₃-C₇ saturated or partially unsaturated carbocyclyl, -C(O)N(C₁-C₄ alkyl)₂, -N(C₁-C₄ alkyl)₂, and C₆-C₁₀ aryl;
n is 0, 1, 2, 3, or 4;
each R_{b} is independently selected from the group consisting of deuterium, hydroxyl, halogen, amino, nitro, oxo group (=O), C₁-C₄ alkyl, C₂-C₄ alkenyl, C₂-C₄ alkynyl, C₁-C₄ alkoxy, C₁-C₄ haloalkyl, C₁-C₄ haloalkoxy, -NH-(C₁-C₄ alkyl), -NH-(C₁-C₄ alkyl-hydroxy), and 4-7 membered heterocyclyl; or two R_{b} groups, together with the carbon atom to which they are attached, form a C₃-C₇ saturated or partially unsaturated carbocyclyl, a 3-8 membered saturated or partially unsaturated heterocyclyl, a C₆-C₁₀ aryl, or a 5-12 membered heteroaryl.

R₁₁ is selected from the group consisting of amino, C₁-C₁₀ alkyl, C₁-C₁₀ alkoxy, C₂-C₁₀ alkenyl, C₂-C₁₀ alkynyl, C₃-C₇ saturated or partially unsaturated carbocyclyl, 3-12 membered saturated or partially unsaturated heterocyclyl, -C₂-C₄ alkynylene-3-12 membered saturated or partially unsaturated heterocyclyl, -O-3-12 membered saturated or partially unsaturated heterocyclyl, -C₆-C₁₀ aryl, -C₂-C₄ alkenylene-C₆-C₁₀ aryl, 5-12 membered heteroaryl, 8-12 membered fused bicyclic ring, -NH-CH₂-3-12 membered saturated or partially unsaturated heterocyclyl, and -NH-CH₂-5-12 membered heteroaryl; wherein one or more hydrogen atoms on R₁₁ may optionally be substituted by substituents selected from the group consisting of deuterium atom, hydroxyl, halogen, amino, carboxylic acid, oxo group (=O), 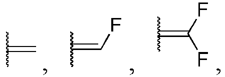 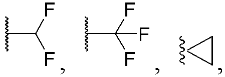 C₁-C₄ haloalkyl, C₁-C₄ haloalkoxy, C₁-C₄ alkyl, C₂-C₄ alkynyl, C₁-C₄ alkoxy, C₁-C₄ alkylthio, C₂-C₄ alkenyl, C₂-C₆ ester group, S(O)₂CH₃, C₃-C₇ saturated or partially unsaturated carbocyclyl, -C(O)N(C₁-C₄ alkyl)₂, -N(C₁-C₄ alkyl)₂, C₆-C₁₀ aryl, and 3-12 membered saturated or partially unsaturated heterocyclyl;
m is 0, 1, 2, 3, or 4;
p is 0, 1, 2, or 3;
with the proviso that, when Rₐ is methyl, n is 1, and m is 0, -Y₁-Y₂-R₁ is not a group selected from: OH, NH₂, -C(O)NH₂,

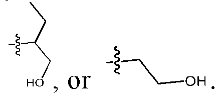

In some embodiments, ring A is a C₆-C₁₀ aromatic ring or a 5-12 membered heteroaromatic ring.

In another preferred embodiment, ring A is a benzene ring, pyrimidine ring, or pyridine ring.

In some embodiments, R₁ is selected from the group consisting of C₁-C₅ alkyl, C₁-C₅ alkylamino, C₁-C₅ alkoxy, C₁-C₅ haloalkyl, C₃-C₆ saturated or partially unsaturated carbocyclyl, 4-7 membered saturated or partially unsaturated heterocyclyl, C₆-C₁₀ aryl, 5-10 membered heteroaryl, C₁-C₄ alkyl-C₃-C₆ saturated or partially unsaturated carbocyclyl, C₁-C₄ alkyl-4-7 membered saturated or partially unsaturated heterocyclyl, C₁-C₄ alkyl-C₆-C₁₀ aryl, and C₁-C₄ alkyl-5-10 membered heteroaryl; wherein said alkyl, haloalkyl, alkylamino, carbocyclyl, heterocyclyl, aryl, and heteroaryl are each independently optionally substituted with 0-4 substituents selected from the group consisting of deuterium, hydroxyl, halogen, oxo group (=O), carboxyl, mercapto, C₁-C₄ alkyl, C₂-C₄ acyl, C₁-C₄ alkoxy, C₁-C₄ alkylthio, C₃-C₆ saturated or partially unsaturated carbocyclyl, 4-7 membered saturated or partially unsaturated heterocyclyl, C₆-C₁₀ aryl, and C₅-C₁₀ heteroaryl.

In another preferred embodiment, said R₂ is selected from the group consisting of: H, C₁-C₄ alkyl, S(=O)₂R₁₁, C(O)R₁₁, C(O)(C₀-C₄ alkylene)R₁₁, 4-7 membered saturated or partially unsaturated heterocyclyl, and 5-7 membered heteroaryl.

In another preferred embodiment, said R₂ is selected from the group consisting of: H, C₁-C₄ alkyl, S(=O)₂R₁₁, C(O)R₁₁, S(=O)₂(CH₂)R₁₁, C(O)(CH₂)R₁₁, 4-7 membered saturated or partially unsaturated heterocyclyl, and 5-7 membered heteroaryl.

In another preferred embodiment, Y₁ and Y₂ are chemical bonds, and R₁ is selected from the group consisting of 4-7 membered saturated or partially unsaturated heterocyclyl, 5-10 membered heteroaryl, C₁-C₄ alkyl-4-7 membered saturated or partially unsaturated heterocyclyl, and C₁-C₄ alkyl-5-10 membered heteroaryl.

In another preferred embodiment, Y is 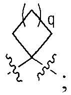 wherein q is 0, 1, 2, or 3, and R₁ is not H.

In another preferred embodiment, R₁ has a structure selected from the group consisting of:

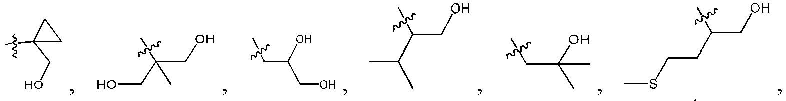

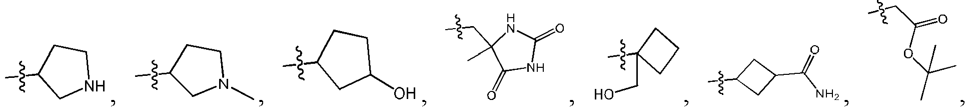

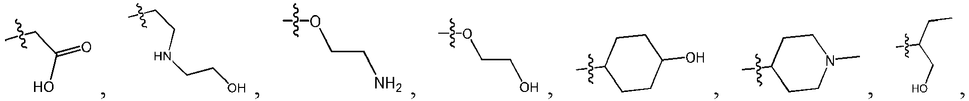

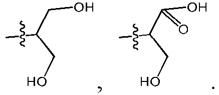

In some embodiments, X₁ is NH;
X₂ is CH₂, S, or NH.

In some embodiments, the compound has a structure represented by formula (IA), formula (IVA), or formula (IIA): 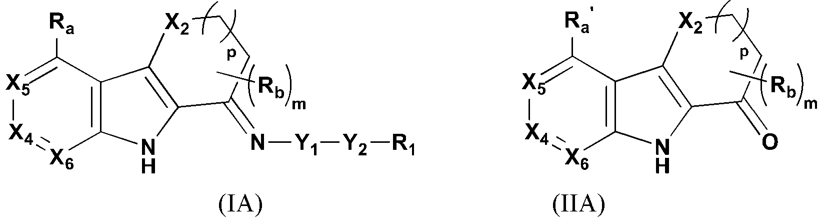 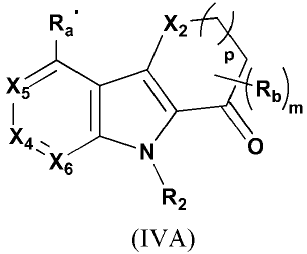
wherein the definitions of Y₁, Y₂, R₁, R₂, Rₐ, Rₐ', R_{b}, X₂, m, and p are as described above;
X₄, X₅ and X₆ are each independently selected from the group consisting of CRₐ₁ and N; wherein Rₐ₁ is selected from the group consisting of H, D, hydroxyl, halogen, amino, nitro, C₁-C₄ alkyl, C₂-C₄ alkenyl, and C₂-C₄ alkynyl.

In another preferred embodiment, Rₐ has a structure selected from the group consisting of C₁-C₄ alkyl, C₁-C₄ alkoxy, -C(O)OR_{c1}, and -NR_{c1}R_{d1}; wherein R_{c1} and R_{d1} are as defined above.

In another preferred embodiment, Rₐ' has a structure selected from the group consisting of deuterium, hydroxyl, halogen, amino, nitro, cyano, C₁-C₄ alkyl, C₂-C₆ ester group, C₁-C₄ haloalkyl, C₃-C₇ saturated or partially unsaturated carbocyclyl, 3-12 membered saturated or partially unsaturated heterocyclyl, C₆-C₁₀ aryl, 5-12 membered heteroaryl, -NH-CH₂-(3-12 membered saturated or partially unsaturated heterocyclyl), -NH-CH₂-(5-12 membered saturated or partially unsaturated heteroaryl), -P(O)R_{c}R_{d}, -S(O)₂R_{c}, -S(O)₂NH₂, -S(O)(NH₂)(R_{c}), -NH-S(O)₂R_{c}, - N=S(O)R_{c}R_{d}, -CH₂-S(O)(NH)(R_{c}), -C(O)NHR_{c}, -C(O)NR_{c}R_{d}, -C(O)OR_{c}, - C(O)O(CH₂)R_{c}, -C(O)NH(C₁-C₄ alkylene)R_{c}, and -NR_{c}R_{d}; wherein R_{c} and R_{d} are as defined above;
wherein one or more hydrogen atoms on Rₐ₁, Rₐ, and Rₐ' may optionally be substituted by substituents selected from the group consisting of: deuterium atom, hydroxyl, halogen, amino, cyano, carboxylic acid, oxo group (=O), 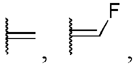 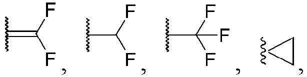 C₁-C₄ **alkyl,** C₂-C₄ alkenyl, C₂-C₄ alkynyl, C₁-C₄ alkoxy, HO-(C₁-C₄ alkylene)-, C₁-C₄ alkylthio, C₂-C₄ alkenyl, C₂-C₆ ester group, S(O)₂CH₃, C₃-C₇ saturated or partially unsaturated carbocyclyl, -C(O)N(C₁-C₄ alkyl)₂, -N(C₁-C₄ alkyl)₂, and C₆-C₁₀ aryl.

In another preferred embodiment, said R_{c} and R_{d} are each independently selected from the group consisting of deuterium, C₁-C₄ alkyl, C₁-C₄ alkoxy, C₂-C₄ alkenyl, C₂-C₄ alkynyl, C₃-C₇ saturated or partially unsaturated carbomonocyclyl, C₃-C₇ saturated or partially unsaturated carbobicyclyl (bridged), C₃-C₇ saturated or partially unsaturated carbospirocyclyl, 3-8 membered saturated or partially unsaturated heteromonocyclyl, 3-8 membered saturated or partially unsaturated heterobicyclyl (bridged), 3-8 membered saturated or partially unsaturated heterospirocyclyl, C₆-C₁₀ aryl, benzo-fused C₅-C₈ carbocyclyl, benzo-fused 5-8 membered heterocyclyl, benzo-fused 5-7 membered heteroaryl, 5-7 membered heteroaryl-fused C₅-C₇ carbocyclyl, 5-7 membered heteroaryl-fused 5-7 membered heterocyclyl, C₄-C₇ carbocycle-fused 5-7 membered heterocycle, 5-12 membered heteroaryl, -N(C₁-C₄ alkyl)₂, -CONH₂, and -CON(C₁-C₄ alkyl)₂; or R_{c}, R_{d}, and the nitrogen atom to which they are attached together form a 3-8 membered saturated or partially unsaturated heterocyclyl or a 5-12 membered heteroaryl.

In another preferred embodiment, said R₁₁ is selected from the group consisting of: amino, C₁-C₁₀ alkyl, C₁-C₁₀ alkoxy, C₂-C₁₀ alkenyl, C₂-C₁₀ alkynyl, C₃-C₇ saturated or partially unsaturated carbomonocyclyl, C₃-C₇ saturated or partially unsaturated bridged carbocyclyl, C₃-C₇ saturated or partially unsaturated spiro carbocyclyl, 3-8 membered saturated or partially unsaturated heteromonocyclyl, 3-8 membered saturated or partially unsaturated bridged heterocyclyl, 3-8 membered saturated or partially unsaturated spiro heterocyclyl, -O-3-12 membered saturated or partially unsaturated heterocyclyl, -C₀-C₄ alkenylene-C₆-C₁₀ aryl, 5-12 membered heteroaryl, benzo-fused C₅-C₈ carbocyclyl, benzo-fused 5-8 membered heterocyclyl, benzo-fused 5-7 membered heteroaryl, 5-7 membered heteroaryl-fused C₅-C₇ carbocyclyl, 5-7 membered heteroaryl-fused 5-7 membered heterocyclyl, C₄-C₇ carbocycle-fused 5-7 membered heterocycle, -NH-CH₂-3-12 membered saturated or partially unsaturated heterocyclyl, and -NH-CH₂-5-12 membered heteroaryl.

In some embodiments, the compound has a structure represented by formula (IIIA) or formula (IVA): 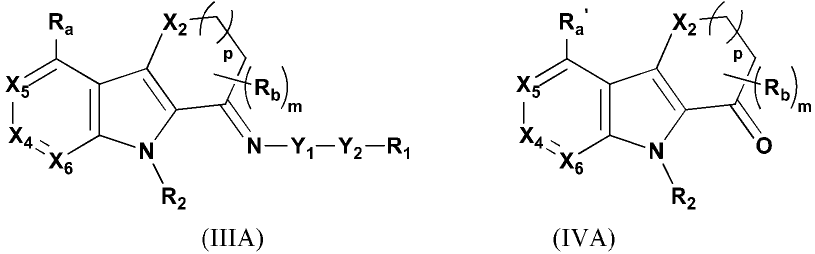 wherein the definitions of Y₁, Y₂, R₁, R₂, Rₐ, Rₐ', R_{b}, X₂, X₄, X₅, X₆, m, and p are as described above.

In some embodiments, the compound has a structure selected from the group consisting of:

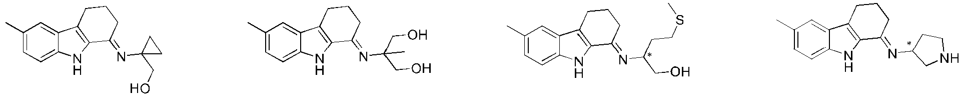

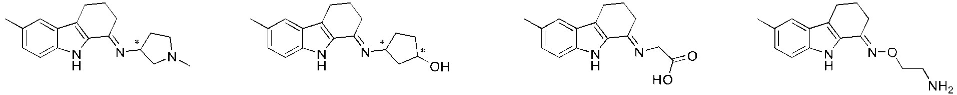

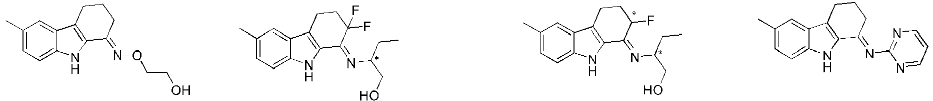

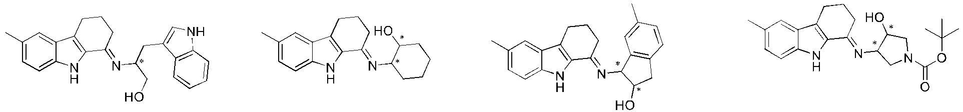

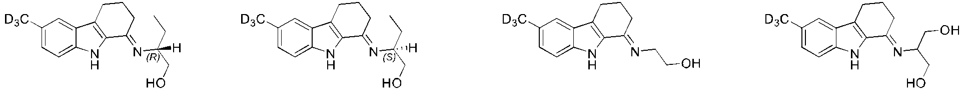

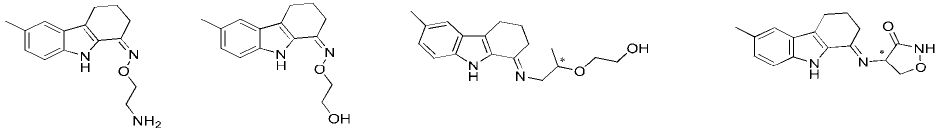

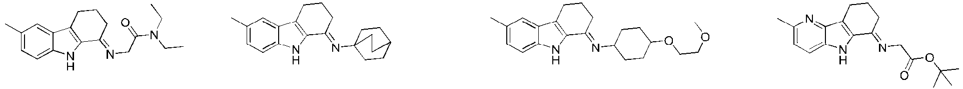

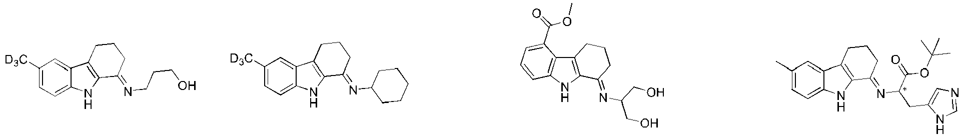

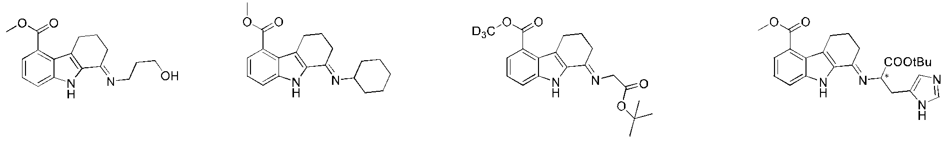

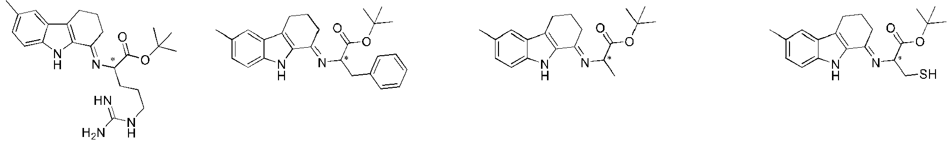

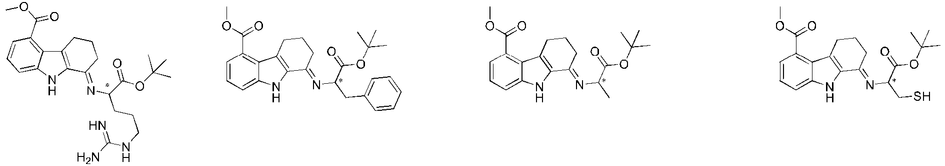

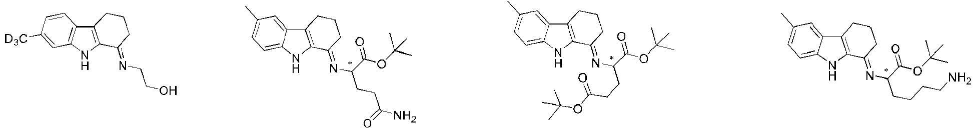

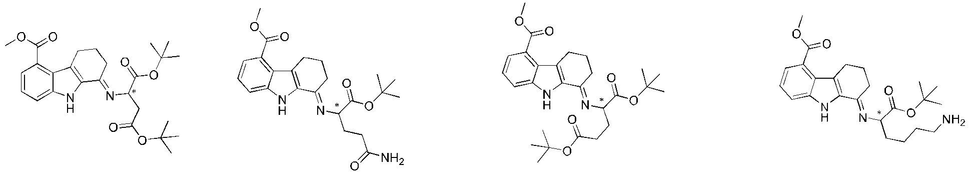

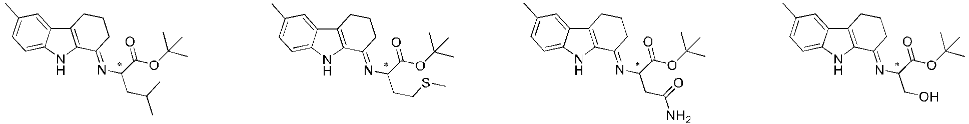

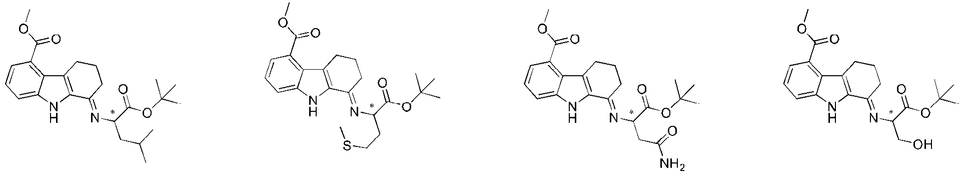

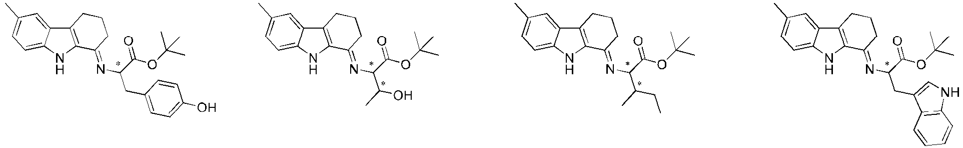

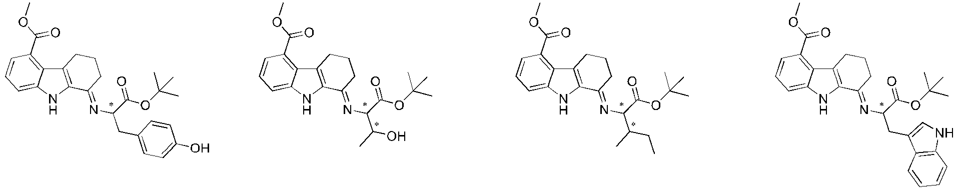

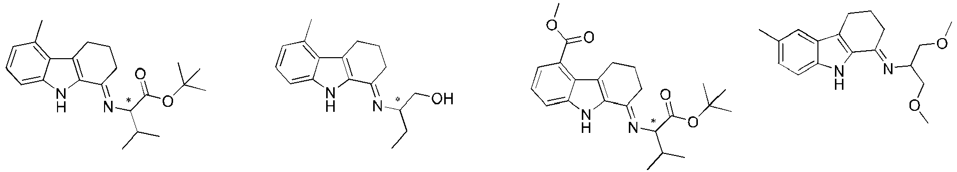

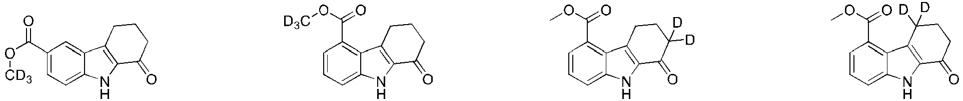

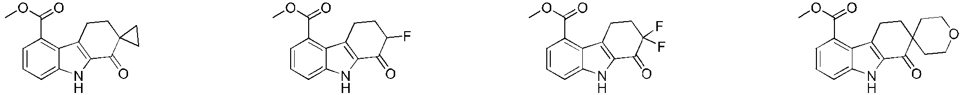

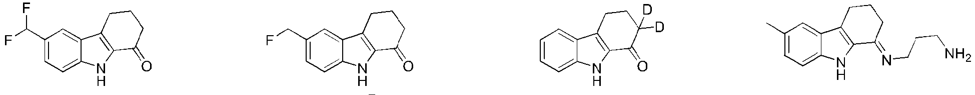

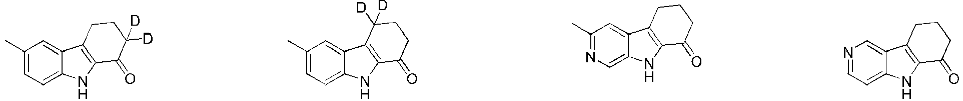

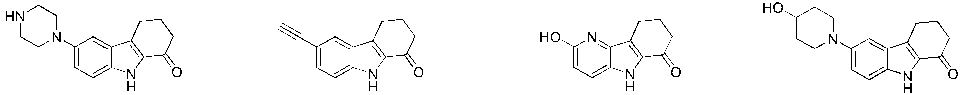

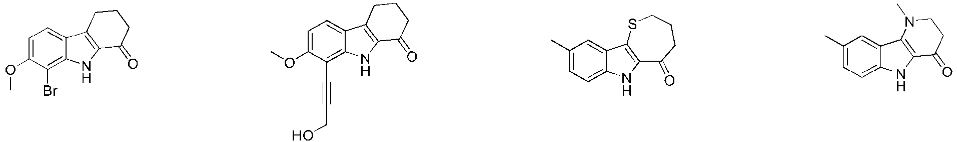

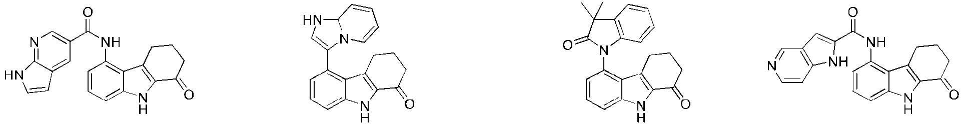

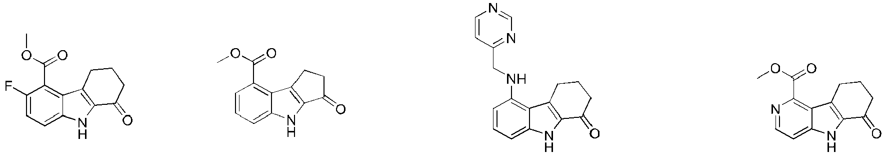

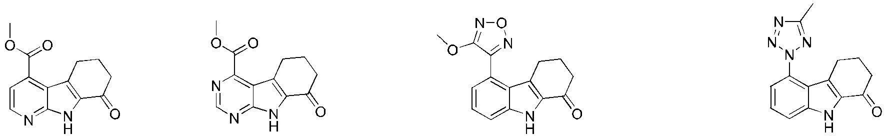

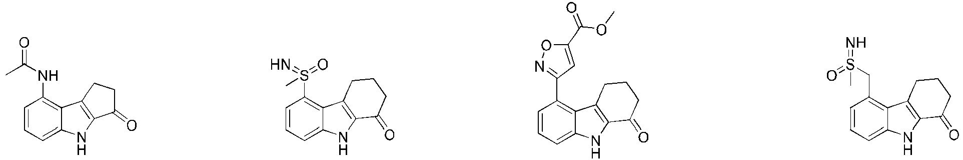

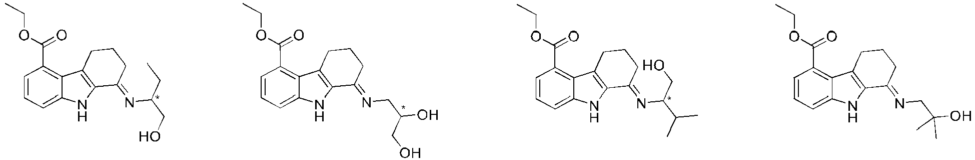

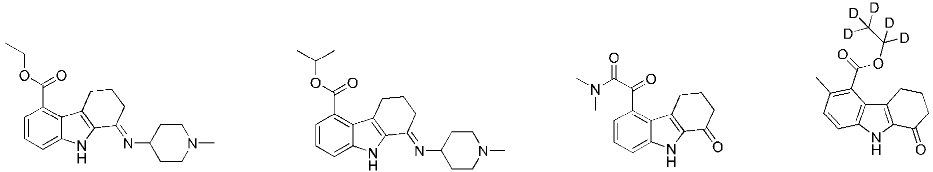

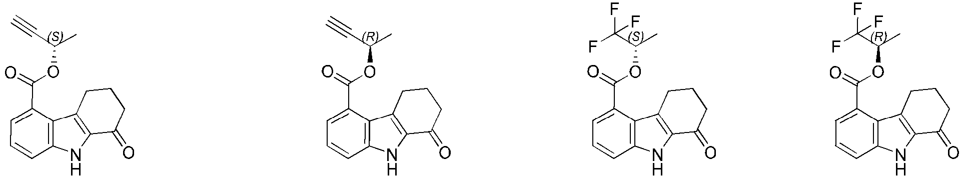

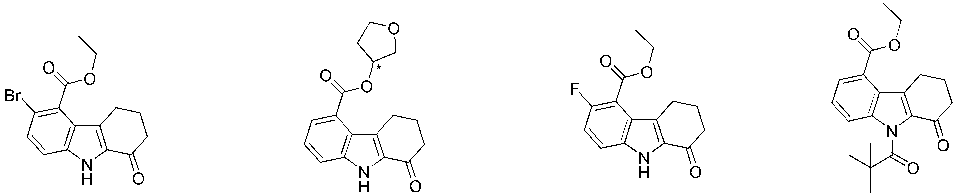

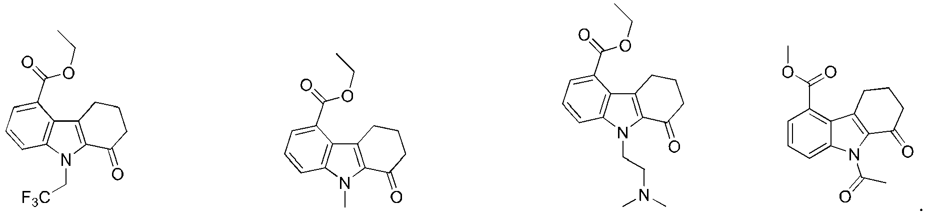

In some embodiments, the compound has a structure selected from the group consisting of:

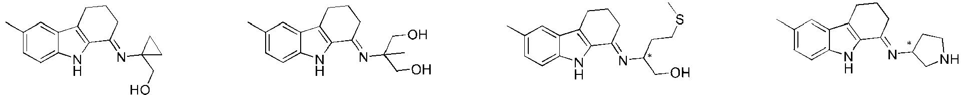

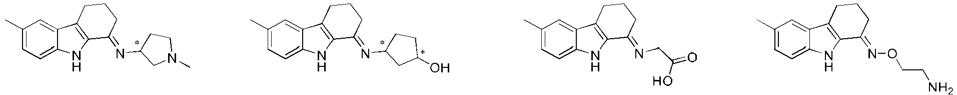

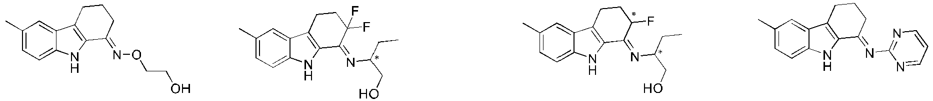

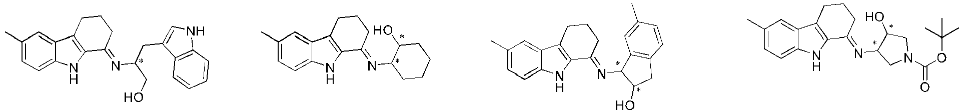

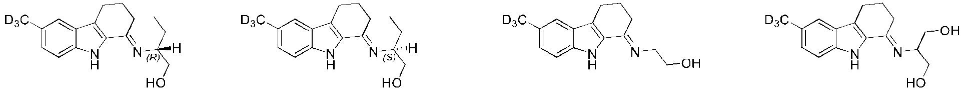

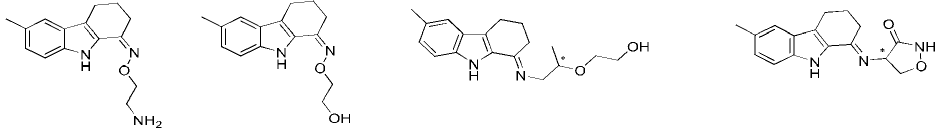

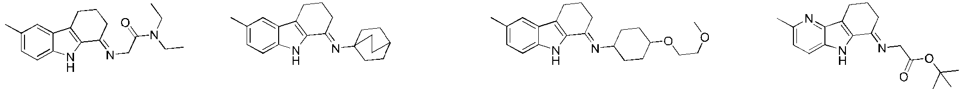

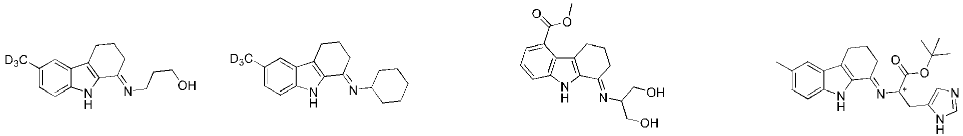

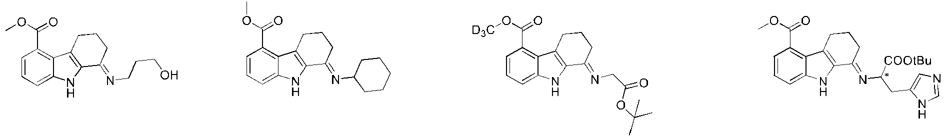

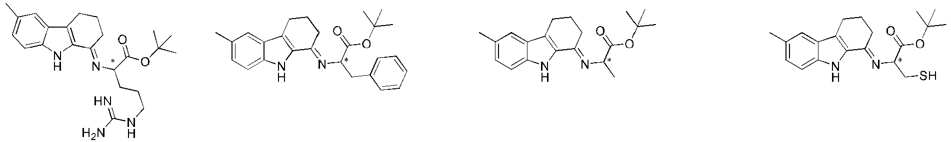

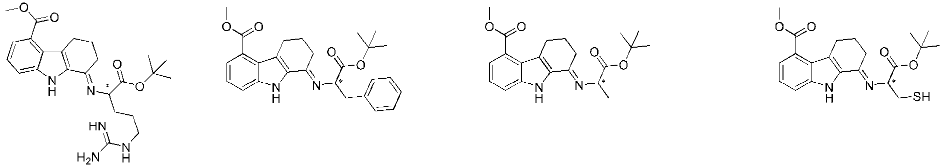

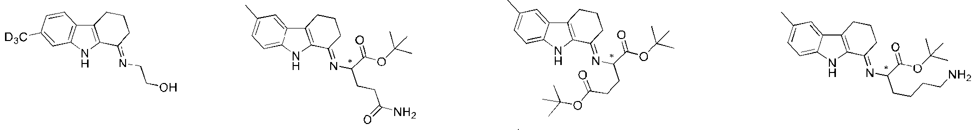

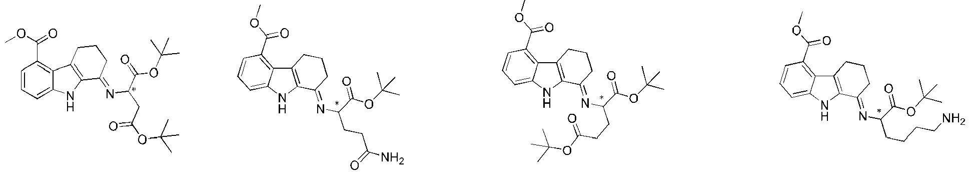

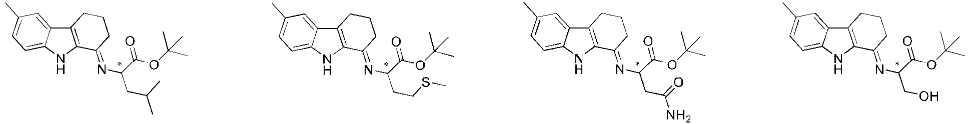

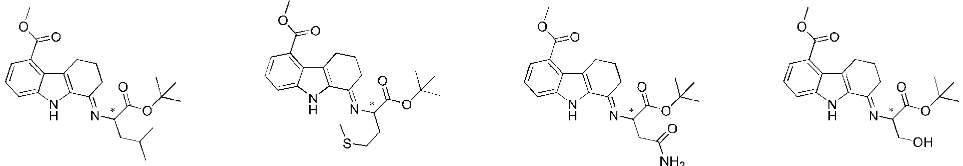

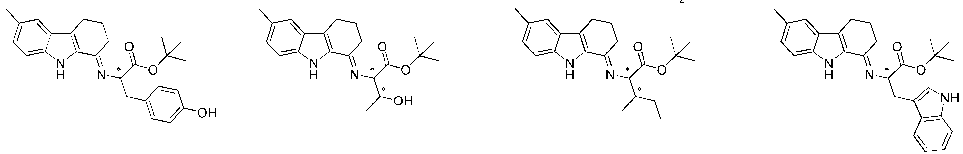

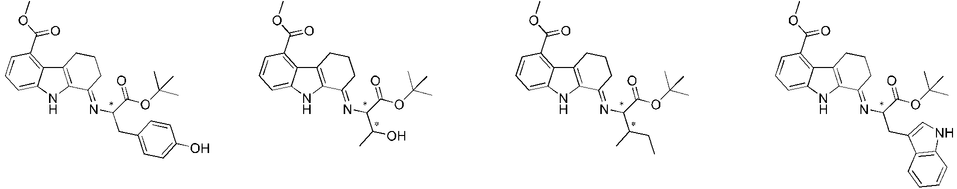

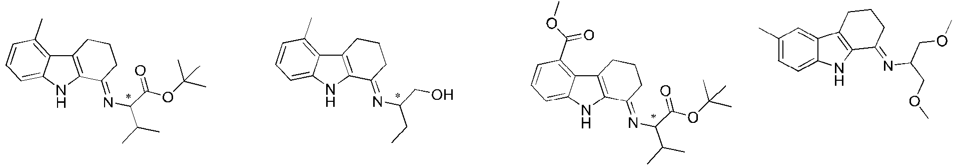

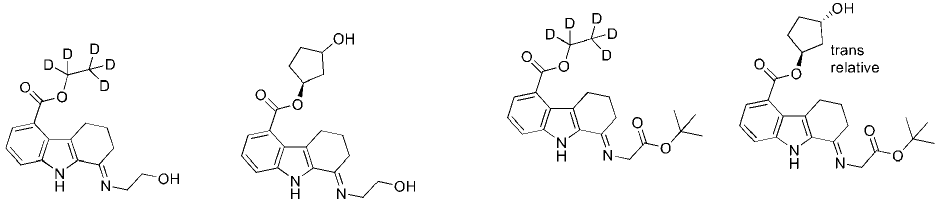

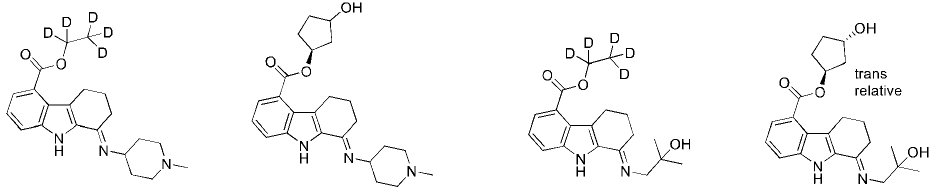

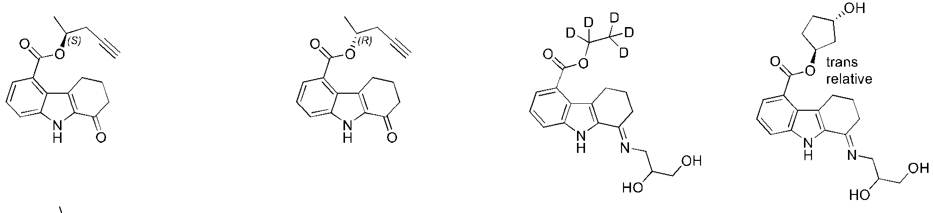

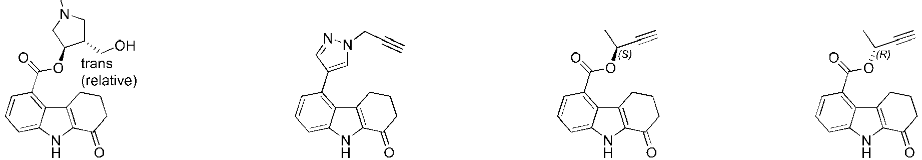

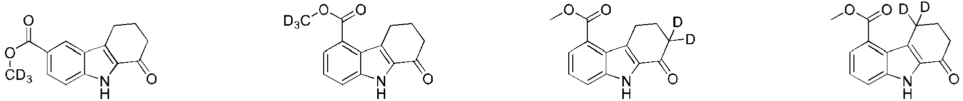

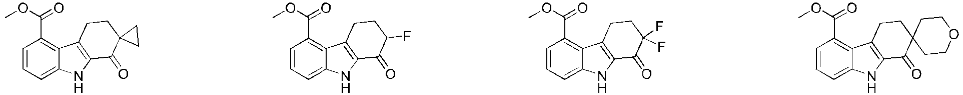

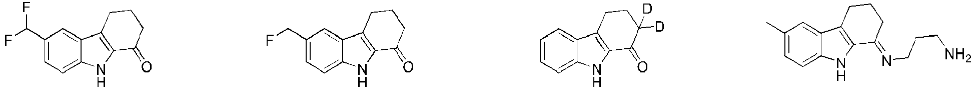

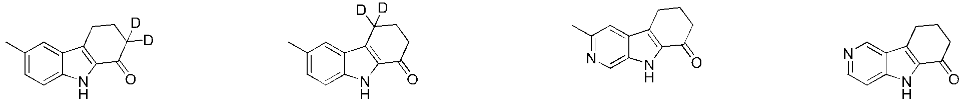

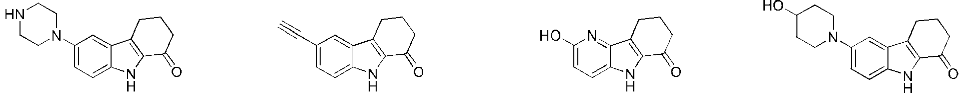

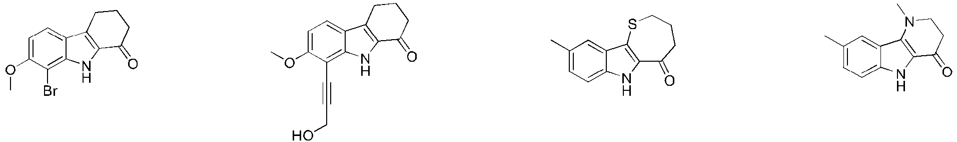

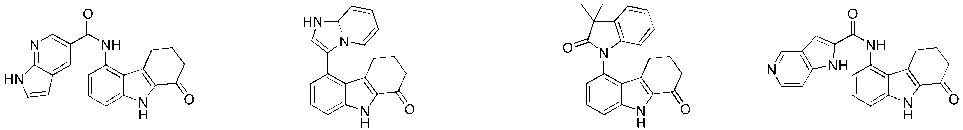

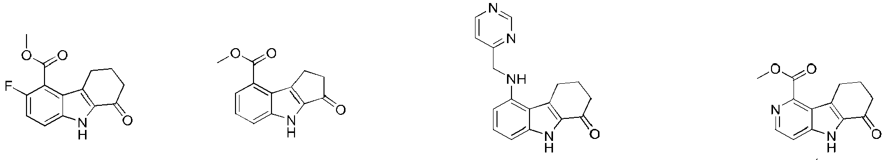

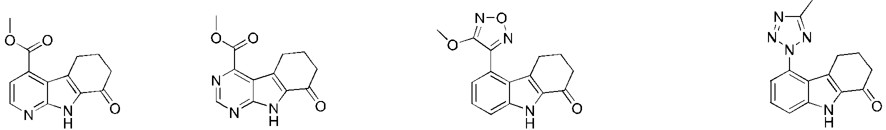

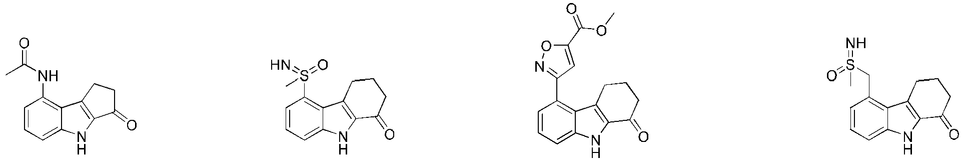

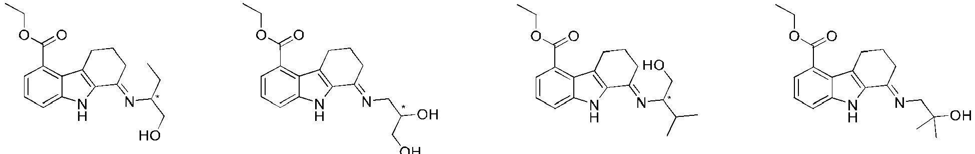

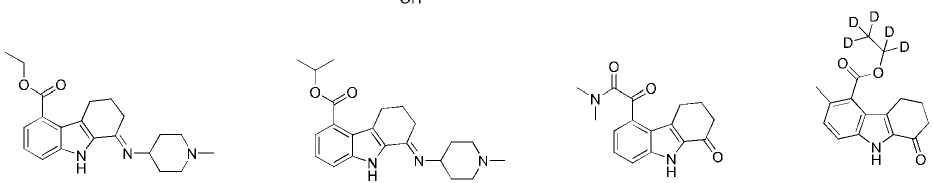

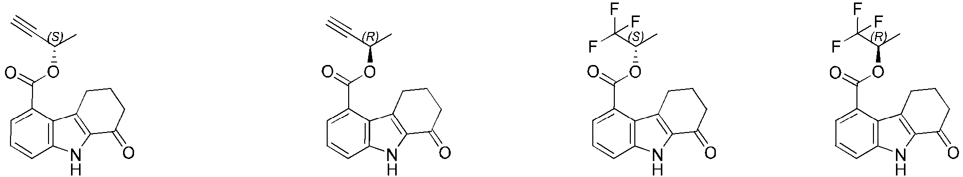

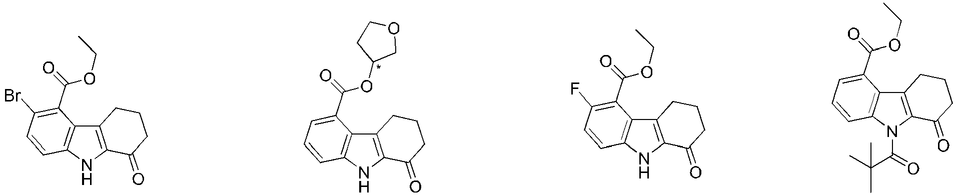

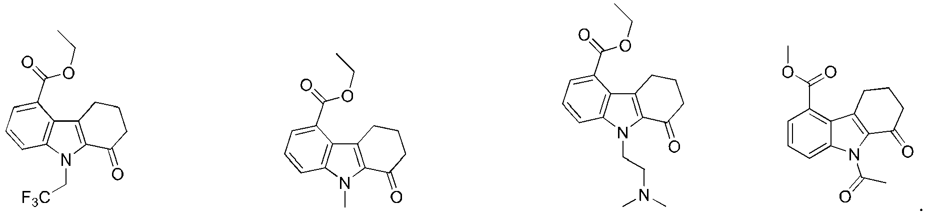

In some embodiments, the compound has a structure selected from those described in the examples.

Another aspect of the present disclosure relates to a method for preparing a compound represented by general formula (II) or a pharmaceutically acceptable salt thereof, the method comprising the following steps: 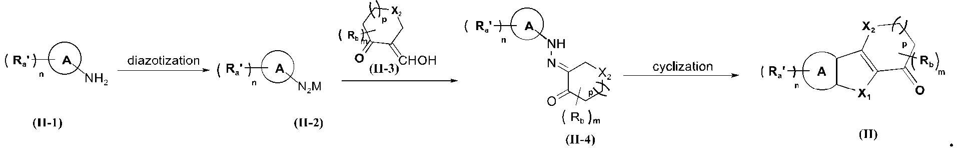
a compound represented by general formula **(II-1)** is subjected to diazotization to obtain a compound represented by general formula (II-2), which is then reacted with a compound represented by general formula (II-3) or an alkali metal salt thereof to obtain a compound represented by general formula (II-4). finally, a cyclization reaction is carried out to obtain the compound represented by general formula (II) or a pharmaceutically acceptable salt thereof.
wherein:
   M is a halogen or BF₄;
   ring A, Rₐ', n, R_{b}, m, p, X₁ and X₂ are as defined **in** general formula (II).

The compound represented by general formula (II-3) or an alkali metal salt thereof may be replaced by a compound represented by general formula (II-3a) or an alkali metal salt thereof: 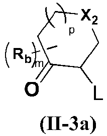 wherein L is an aldehyde group or a carboxyl group.

Another aspect of the present disclosure relates to a method for preparing a compound represented by general formula (II) or a pharmaceutically acceptable salt thereof, the method comprising the following steps:

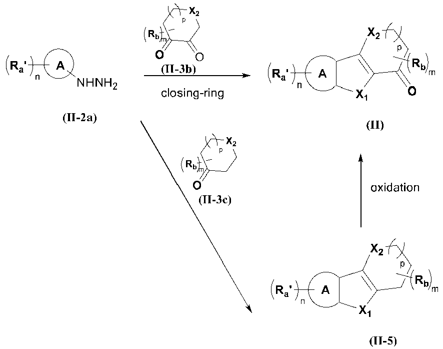

A compound represented by general formula (II-2a) or an acid salt thereof undergoes a cyclization reaction with a compound represented by general formula (II-3b) to yield the compound represented by general formula (II) or a pharmaceutically acceptable salt thereof; alternatively, the compound represented by general formula (II-2a) may react with a compound represented by general formula (II-3c) to yield a compound represented by general formula (II-5), which is then subjected to an oxidation reaction to yield the compound represented by general formula (II) or a pharmaceutically acceptable salt thereof.
wherein:
ring A, Rₐ', n, R_{b}, m, p, X₁, and X₂ are as defined **in** general formula (II).

Another aspect of the present disclosure relates to a method for preparing a compound represented by general formula (I) or a pharmaceutically acceptable salt thereof, the method comprising the following steps:

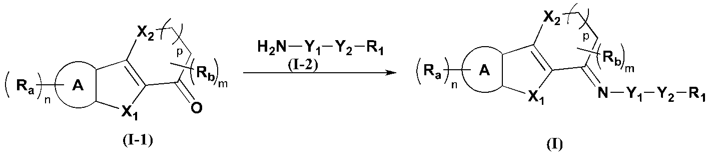

A compound represented by general formula (I-1) undergoes a condensation reaction with a compound represented by general formula (I-2) to yield the compound represented by general formula (I) or a pharmaceutically acceptable salt thereof.
wherein:
ring A, Rₐ, n, R_{b}, m, p, X₁, X₂, Y₁, Y₂, and R₁ are as defined in general formula (I).

In another aspect of the present invention, a pharmaceutical composition is provided, characterized by comprising:
(1) the compound according to the first aspect of the present invention, its stereoisomer, geometric isomer, hydrate, solvate, prodrug, or pharmaceutically acceptable salt thereof; and optionally (2) a pharmaceutically acceptable carrier, excipient, or other active pharmaceutical ingredients.

In another preferred embodiment, the pharmaceutical composition further comprises a second therapeutic component, and the second therapeutic component is selected from the group consisting of: PARP (poly ADP-ribose polymerase) inhibitors, proteasome inhibitors, kinase inhibitors, DNA methyltransferase inhibitors, histone deacetylase inhibitors, nucleoside drugs that interfere with nucleic acid metabolism in tumor cells/viruses/bacteria, chemotherapeutic agents that inhibit tumor cell DNA replication such as platinum-based drugs, paclitaxel and its derivatives, drugs and treatment regimens that inhibit the uptake and metabolism of glucose, amino acids, fatty acids, etc., or other therapeutic components and treatment regimens that can produce synergistic antitumor effects with the aforementioned tetrahydrocarbazole compounds.

In another preferred embodiment, the PARP inhibitor is Olaparib, Rucaparib, Niraparib, Talazoparib, Fluzoparib, Pamiparib, or Saruparib; the proteasome inhibitor is Ixazomib or Bortezomib; the kinase inhibitor is a MEK inhibitor; the MEK inhibitor is Cobimetinib; the DNA methyltransferase inhibitor is SGI-1027; the histone deacetylase inhibitor is Trichostatin A; the nucleoside analog is 6-Mercaptopurine (6-MP); the chemotherapeutic agent is Cisplatin or Cabazitaxel; the metabolic inhibitor is the GLUT1/GLUT3 inhibitor KL-11743 or BAY-876; and the metabolic treatment regimen is intermittent fasting.

In a third aspect of the present invention, a use is provided for the compound according to the first aspect of the present invention, its stereoisomer, geometric isomer, hydrate, solvate, prodrug, or pharmaceutically acceptable salt thereof, or the pharmaceutical composition according to the second aspect of the present invention, characterized in that it is used for the manufacture of a medicament for treating and/or preventing cancer.

In another preferred embodiment, the cancer is selected from the group consisting of: liver cancer, lung cancer, breast cancer, pancreatic cancer, gastric cancer, colorectal cancer, kidney cancer, prostate cancer, cervical cancer, ovarian cancer, anal and perianal cancer, vulvar cancer, vaginal cancer, penile cancer, head and neck cancer, glioma, skin cancer, mesothelioma, muscle cancer, leukemia, lymphoma, and cancers and related lesions caused by oncogenic viruses such as herpesvirus, adenovirus, hepatitis virus, retrovirus, and the papovavirus family.

In some embodiments, the treatment and/or prevention of cancer comprises: administering the compound represented by formula (I) or (II) according to the first aspect of the present invention, its stereoisomer, geometric isomer, hydrate, solvate, prodrug, or pharmaceutically acceptable salt thereof, or the pharmaceutical composition according to the second aspect of the present invention, in combination with a component selected from the group consisting of: PARP inhibitors, proteasome inhibitors, MEK inhibitors, DNA methyltransferase inhibitors, histone deacetylase inhibitors, nucleoside analogs that interfere with nucleic acid metabolism in tumor cells, viruses, and bacteria, chemotherapeutic agents that inhibit tumor cell DNA replication such as platinum-based drugs, paclitaxel and its derivatives, drugs and treatment regimens that inhibit the uptake and metabolism of glucose, amino acids, fatty acids, etc., or other therapeutic components and treatment regimens that can produce synergistic antitumor effects with the compound of formula (I) or (II).

In some embodiments, the PARP inhibitor is Olaparib, Rucaparib, Niraparib, Talazoparib, Fluzoparib, Pamiparib, or Saruparib; the proteasome inhibitor is Ixazomib or Bortezomib; the kinase inhibitor is Cobimetinib; the DNA methyltransferase inhibitor is SGI-1027; the histone deacetylase inhibitor is Trichostatin A; the nucleoside analog is 6-Mercaptopurine (6-MP); the chemotherapeutic agent is Cisplatin or Cabazitaxel; the metabolic inhibitor is the GLUT1/GLUT3 inhibitor KL-11743 or BAY-876; and the metabolic treatment regimen is intermittent fasting.

It should be understood that within the scope of the present invention, the various technical features described above and those specifically described below (e.g., in the examples) can be combined with each other to form new or preferred technical solutions. For the sake of brevity, these combinations are not exhaustively detailed herein.

### Detailed Description

### Terms

As used herein, the term "alkyl" includes straight-chain or branched alkyl groups. For example, "C₁-C₆ alkyl" denotes a straight-chain or branched alkyl group having 1, 2, 3, 4, 5, or 6 carbon atoms, such as methyl, ethyl, n-propyl, isopropyl, cyclopropyl, n-butyl, isobutyl, tert-butyl, cyclobutyl, n-pentyl, isopentyl, neopentyl, and the like.

As used herein, the term "alkenyl" includes straight-chain or branched alkenyl groups. For example, "C₂-C₆ alkenyl" refers to a straight-chain or branched alkenyl group having 2, 3, 4, 5, or 6 carbon atoms, such as vinyl, allyl, 1-propenyl, isopropenyl, 1-butenyl, 2-butenyl, or similar groups.

As used herein, the term "alkynyl" includes straight-chain or branched alkynyl groups. For example, "C₂-C₆ alkynyl" refers to a straight-chain or branched alkynyl group having 2, 3, 4, 5, or 6 carbon atoms, such as ethynyl, propynyl, butynyl, or similar groups.

As used herein, the term "carbocyclyl" refers to a cyclic, saturated or partially unsaturated aliphatic hydrocarbon group having a specified number of carbon atoms. For example, "C₃-C₁₀ carbocyclyl" denotes a cyclic, saturated or partially unsaturated aliphatic hydrocarbon group having 3, 4, 5, 6, 7, 8, 9, or 10 carbon atoms. It can be monocyclic, such as cyclopropyl, cyclobutyl, cyclopentyl, cyclohexyl, or similar groups. "Carbocyclyl" can also be bicyclic or polycyclic, for example, fused (i.e., having multiple rings fused together), bridged, or spiro forms.

As used herein, the term "alkylamino" refers to an amino group substituted by alkyl group(s). For example, "C₁-C₆ alkylamino" refers to an amino group substituted by C₁-C₆ alkyl group(s), which can be mono- or di-substituted; for instance, methylamino, ethylamino, propylamino, isopropylamino, butylamino, isobutylamino, tert-butylamino, dimethylamino, diethylamino, dipropylamino, diisopropylamino, dibutylamino, diisobutylamino, di-tert-butylamino, and the like.

As used herein, the term "alkoxy" refers to a group having an alkyl-O- structure. For example, "C₁-C₆ alkoxy" denotes a straight-chain or branched alkoxy group having 1, 2, 3, 4, 5, or 6 carbon atoms, including methoxy, ethoxy, propoxy, isopropoxy, butoxy, isobutoxy, tert-butoxy, sec-butoxy, pentoxy, neopentoxy, and the like.

As used herein, the term "alkylthio" refers to a group having an alkyl-S-structure. For example, "C₁-C₆ alkylthio" denotes a straight-chain or branched alkylthio group having 1, 2, 3, 4, 5, or 6 carbon atoms, including methylthio, ethylthio, propylthio, isopropylthio, butylthio, isobutylthio, tert-butylthio, sec-butylthio, pentylthio, neopentylthio, and the like.

As used herein, the term "haloalkyl" represents an alkyl group in which one or more hydrogen atoms are replaced by halogen atoms, wherein alkyl is as defined above.

As used herein, the term "haloalkoxy" represents an alkoxy group in which one or more hydrogen atoms are replaced by halogen atoms, wherein alkoxy is as defined above.

As used herein, the term "carbonyl" represents a group containing a specified number of carbon atoms and having an alkyl-C(=O)- or alkyl-C(=O)-alkyl- structure.

As used herein, the terms "heterocyclyl" or "heterocycloalkyl" refer to a saturated or partially saturated cyclic group having a specified number of ring atoms (e.g., 3-10 ring atoms), wherein 1-4 atoms are heteroatoms selected from N, S, and O. It can be monocyclic, or bicyclic or polycyclic, for example, fused (i.e., having multiple rings fused together), bridged, or spiro forms. Specific examples include oxetanyl, azetidinyl, tetrahydro-2H-pyranyl, piperidinyl, tetrahydrofuranyl, morpholinyl, pyrrolidinyl, and the like.

As used herein, the terms "aryl", "aromatic ring", or "arene" refer to a cyclic group with aromaticity. For example, the term "C₆-C₁₀ aryl" denotes an aryl group having 6-10 carbon atoms, such as phenyl or naphthyl, and similar groups. "Aryl" can also be a fused aryl group.

As used herein, the terms "heteroaryl" or "heteroaromatic ring" refer to a cyclic aromatic group wherein 1-3 atoms are heteroatoms selected from the group consisting of N, S, and O; for example, the term "5-12 membered heteroaryl" refers to a heteroaryl group having 5-12 ring atoms, which can be monocyclic or fused. Specific examples include furyl, thienyl, pyrrolyl, thiazolyl, thiadiazolyl, oxazolyl, oxadiazolyl, imidazolyl, pyrazolyl, pyridyl, pyrimidinyl, 1,4-dioxinyl, 2H-1,2-oxazinyl, 4H-1,2-oxazinyl, 6H-1,2-oxazinyl, 4H-1,3-oxazinyl, 6H-1,3-oxazinyl, 4H-1,4-oxazinyl, pyridazinyl, pyrazinyl, 1,2,3-triazinyl, 1,2,4-triazinyl, 1,3,5-triazinyl, 1,2,4,5-tetrazinyl, oxepinyl, thiepinyl, azepinyl, 1,3-diazepinyl, azocinyl, benzofuranyl, benzoisofuranyl, benzothiophenyl, indolyl, benzoxazolyl, benzimidazolyl, indazolyl, benzotriazolyl, quinolinyl, isoquinolinyl, pyridopyrazolyl, pyridopyrrolyl, pyrimidinopyrazolyl, pyrimidinopyrrolyl, pyridazinopyrazolyl, pyridazinopyrrolyl, acridinyl, phenanthridinyl, benzopyridazinyl, phthalazinyl, quinazolinyl, quinoxalinyl, phenazinyl, pteridinyl, purinyl, naphthyridinyl, and the like.

As used herein, the term "fused bicyclic ring" refers to a bicyclic structure formed by the fusion of two rings. Preferably, one ring is a benzene ring or a 5-6 membered heteroaromatic ring, and the other ring is a partially saturated 5-8 membered carbocycle or a partially saturated 5-8 membered heterocycle, but the overall fused bicyclic system is non-aromatic. The fused bicyclic ring may be connected to other groups at any possible position. Examples of fused bicyclic rings include, but are not limited to, for instance: 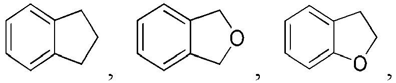 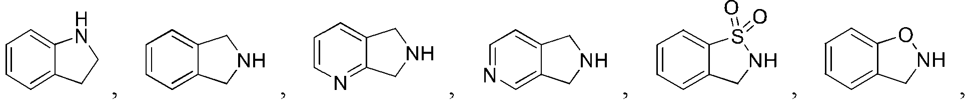 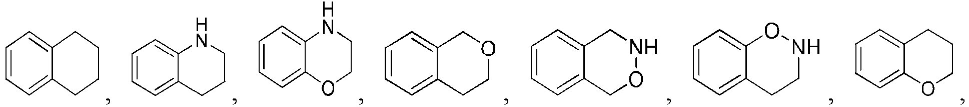 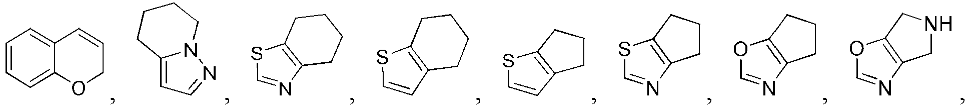 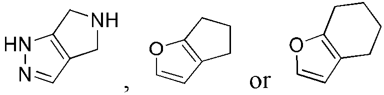 etc.

As used herein, terms such as "benzo-fused C₅-C₈ carbocyclyl", "benzo-fused 5-8 membered heterocyclyl", and "benzo-fused 5-7 membered heteroaryl" have similar definitions, referring to a fused ring system formed by a benzene ring fused with a C₅-C₈ carbocyclyl, a 5-8 membered heterocyclyl, or a 5-7 membered heteroaryl, respectively. Terms such as "5-7 membered heteroaryl-fused C₅-C₇ carbocyclyl", "5-7 membered heteroaryl-fused 5-7 membered heterocyclyl", and "C₄-C₇ carbocycle-fused 5-7 membered heterocycle" have similar definitions, referring to a fused ring system formed by a 5-7 membered heteroaryl fused with a C₅-C₈ carbocyclyl, a 5-8 membered heterocyclyl, or a 5-7 membered heteroaryl, respectively. The term "C₄-C₇ carbocycle-fused 5-7 membered heterocycle" refers to a fused ring system formed by a C₄-C₇ carbocycle fused with a 5-7 membered heterocycle.

As used herein, "halogen" or "halo" refers to F, Cl, Br, and I. Preferably, the halogen or halo atom is selected from F, Cl, and Br. "Halo-" or "halogenated" refers to substitution with atoms selected from F, Cl, Br, and I.

Unless otherwise specified, the structural formulas described in the present invention are intended to include all isomeric forms (e.g., enantiomers, diastereomers, and geometric isomers or conformational isomers): for example, R and S configurations at asymmetric centers, (Z) and (E) isomers of double bonds, etc. Thus, both individual stereochemical isomers of the compounds of the invention and mixtures of their enantiomers, diastereomers, or geometric isomers (or conformational isomers) fall within the scope of the invention.

As used herein, the term "tautomer" refers to structural isomers of differing energy that can interconvert via a low energy barrier. For example, proton tautomers (i.e., prototropic tautomers) include interconversion via proton transfer, such as 1H-indazole and 2H-indazole. Valence tautomers include interconversion via reorganization of some bonding electrons.

As used herein, the term "solvate" refers to a complex formed by coordination of a compound of the present invention with solvent molecules in a specific ratio.

As used herein, the term "hydrate" refers to a complex formed by coordination of a compound of the present invention with water.

As used herein, "*" indicates a chiral center.

After extensive and in-depth research, the inventors have unexpectedly discovered a class of tetrahydrocarbazole compounds that exhibit significantly enhanced inhibitory effects on the proliferation of human tumor cell lines, with IC50 values of several compounds reaching below 0.2 µM.

Abbreviations:
Bu = Butyl
tBu = tert-Butyl
Boc = tert-Butoxycarbonyl
DMF = N,N-Dimethylformamide
DMSO = Dimethyl sulfoxide
Et = Ethyl
HATU=O-(7-Azabenzotriazol-1-yl)-N,N,N',N'-tetramethyluronium hexafluorophosphate
Me = Methyl
PMB = 4-Methoxybenzyl
Tf = Triflyl (Trifluoromethanesulfonyl)
TFA = Trifluoroacetic acid
TMS = Trimethylsilyl

**The main advantages of the present invention are:**
1. The tetrahydrocarbazole compounds of the present invention exhibit significantly enhanced inhibitory effects on the proliferation of human tumor cell lines, with IC₅₀ values of several compounds reaching below 0.2 µM.
2. The tetrahydrocarbazole compounds of the present invention demonstrate favorable safety profiles and low toxicity, and the resulting pharmaceutical preparations are suitable for oral administration.
3. When used in combination with other targeted therapies or chemotherapeutic agents, the tetrahydrocarbazole compounds of the present invention can achieve synergistic efficacy, demonstrating the potential to substantially prolong the survival of cancer patients and even cure certain types of cancer.

The embodiments of the present invention will be explained below with reference to specific examples. Those skilled in the art will understand that the following examples are provided solely to illustrate the invention and should not be construed as limiting the scope of the invention. For any technical details or conditions not specified in the examples, the techniques or conditions described in the literature of the field or the product manuals shall be followed. Reagents or instruments for which the manufacturer is not indicated are conventional products that can be obtained commercially.

### Example

### Example 1: Synthesis of Compound E01

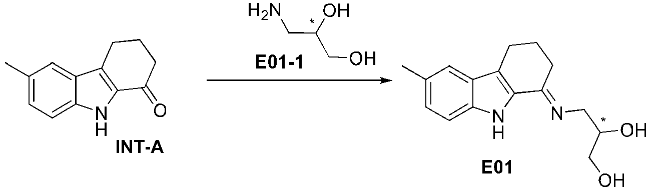

Compound INT-A (199.3 mg, 1.000 mmol, synthesized according to the method in patent WO2006065480) in xylene (5 mL) and compound E01-1 (455.6 mg, 5 mmol, racemate) was stirred at 130 °C for 2 hours, then heated to 165 °C and stirred for additional 4 hours. After the reaction mixture was cooled to room temperature, the precipitated solid was filtered and purified twice with ethanol to yield a white solid E01 (134.3 mg, 49.31%, racemate). ESI/MS (m/z) 273.0 [M+H]⁺.¹H NMR (500 MHz, DMSO-*d*₆) δ 10.80 (s, 1H), 7.28 (s, 1H), 7.25 (d, *J =* 8.3 Hz, 1H), 6.96 (dd, *J =* 8.3, 0.8 Hz, 1H), 4.52 (s, 2H), 3.78-3.74 (m, 1H), 3.53-3.48 (m, 2H), 3.45-3.40 (m, 2H), 2.76 (t, *J=* 5.9 Hz, 2H), 2.57-2.54 (m, 2H), 2.36 (s, 3H), 2.00-1.94 (m, 2H).

### Example 2: Synthesis of Compound E02

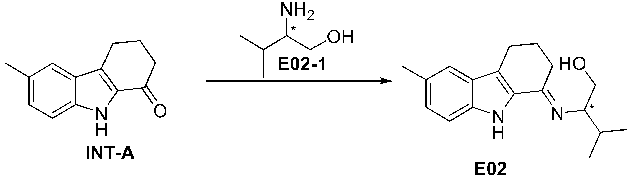

Compound INT-A (199.3 mg, 1.000 mmol) in xylene (5 mL) and compound E02-1 (257.8 mg, 2.5 mmol, racemate) was stirred at 130 °C for 2 hours, then heated to 165 °C and stirred for additional 4 hours. After the reaction mixture was cooled to room temperature, the solvent was removed by vacuum concentration. The mixture was dissolved in a small amount of ethanol, and a large amount of water was added. The precipitated solid was collected by filtration and dried under vacuum to afford compound E02, pale-yellow solid, (130.5 mg, 45.89%, racemate). ESI/MS (m/z) 285.0 [M+H]⁺.¹H NMR (500 MHz, DMSO-*d*₆) δ 10.61 (s, 1H), 7.29 (d, *J =* 8.5 Hz, 1H), 7.27 (s, 1H), 6.96 (d, *J =* 8.3 Hz, 1H), 4.35 (s, 1H), 3.65-3.61 (m, 1H), 3.51-3.48 (m, 1H), 3.43-3.38 (m, 1H), 2.76 (t, *J=* 6.0 Hz, 2H), 2.68-2.63 (m, 1H), 2.55-2.52 (m, 1H), 2.36 (s, 3H), 1.99-1.93 (m, 2H), 1.91-1.83 (m, 1H), 0.90 (dd, *J=* 6.7, 2.2 Hz, 6H).

### Example 3: Synthesis of Compound E03

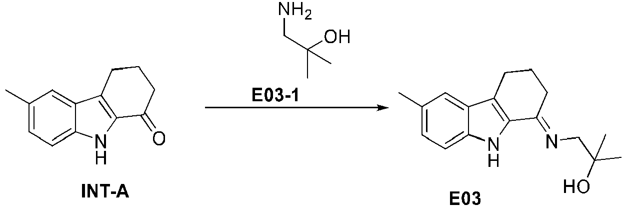

Compound INT-A (199.3 mg, 1.000 mmol) in toluene (5 mL) and compound E03-1 (267.2 mg, 3.000 mmol) was stirred at 130 °C for 5 hours. After the reaction mixture was cooled to room temperature, the solvent was removed by vacuum concentration. The mixture was dissolved in a small amount of ethanol, and a large amount of water was added. The precipitated solid was collected by filtration and dried under vacuum to afford a pale-yellow solid compound E03 (200.7 mg, 74.23%). ESI/MS (m/z) 271.0 [M+H]⁺.¹ H NMR (500 MHz, DMSO-*d*₆) δ 10.79 (s, 1H), 7.29 (s, 1H), 7.27 (d, *J =* 8.3 Hz, 1H), 6.98 (dd, *J =* 8.3, 1.1 Hz, 1H), 4.29 (s, 1H), 3.27 (s, 2H), 2.76 (t, *J =* 6.0 Hz, 2H), 2.52 (d, *J =* 5.8 Hz, 2H), 2.36 (s, 3H), 2.00-1.95 (m, 2H), 1.19 (s, 6H).

### Example 4: Synthesis of Compound E04-R

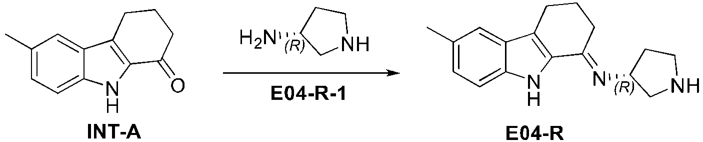

Under nitrogen atmosphere, compound E04-R-1 (300 mg, 3.483 mmol) was added to compound INT-A (231 mg, 1.161 mmol) in toluene (2 mL) at room temperature. The reaction mixture was heated to 140 °C and stirred overnight. After cooling to room temperature, the mixture was concentrated under reduced pressure and washed with n-hexane (3 × 10 mL) to afford a brown solid compound E04-R (140 mg, 41.12%). ESI/MS (m/z): 268.3 [M+H]⁺.¹H NMR (400 MHz, DMSO-*d*₆): δ 10.80-10.55 (m, 1H), 7.25 (d, *J =* 8.3 Hz, 2H), 6.95 (dd, *J =* 8.1, 1.8 Hz, 1H), 4.16 (dq, *J =* 7.6, 5.2 Hz, 1H), 3.01 (dt, *J =* 11.5, 5.7 Hz, 1H), 2.97-2.91 (m, 1H), 2.84 (ddd, *J =* 10.8, 7.9, 6.0 Hz, 2H), 2.75 (t, *J =* 6.1 Hz, 2H), 2.65 (dd, *J =* 10.8, 5.1 Hz, 1H), 2.60-2.55 (m, 2H), 2.35 (s, 3H), 1.98 (dt, *J =* 12.9, 6.4 Hz, 3H), 1.59 (ddt, *J* = 12.9, 8.2, 5.6 Hz, 1H).

### Example 5: Synthesis of Compound E05-R

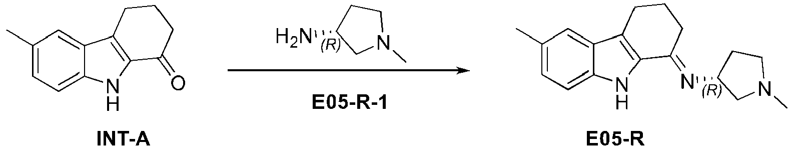

Compound E05-R-1 (151 mg, 1.506 mmol) was added to a toluene (5 mL) solution of compound INT-A (100 mg, 0.502 mmol) at room temperature. The reaction mixture was stirred at 140 °C overnight, then cooled to room temperature and concentrated under reduced pressure. The resulting crude product was purified by column chromatography on triethylamine-treated silica gel (eluent: methanol containing 1% triethylamine) to afford solid E05-R (25 mg, crude). This solid was then dissolved in n-hexane (5 mL) and concentrated to dryness to obtain compound E05-R, pale-yellow solid (19.4 mg, 13.01%). ESI/MS(m/z): 282.15 [M+H]⁺.¹H NMR (300 MHz, DMSO-*d*₆) δ 10.65 (s, 1H), 7.22-7.14 (m, 2H), 6.91-6.83 (m, 1H), 4.20 (dq, *J =* 12.0, 5.1 Hz, 1H), 2.76-2.71 (m, 1H), 2.70-2.64 (m, 2H), 2.47 (t, *J =* 6.8 Hz, 4H), 2.39-2.32 (m, 1H), 2.27 (s, 3H), 2.19 (s, 3H), 2.15 -2.02 (m, 1H), 1.88 (p, *J =* 6.0 Hz, 2H), 1.56 (dq, *J =* 12.2, 7.0 Hz, 1H).

### Example 6: Synthesis of Compound E06-SR

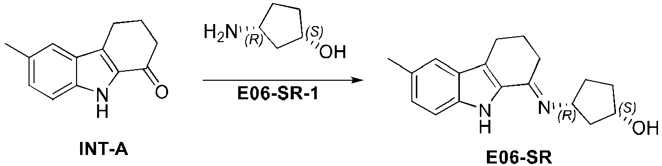

At room temperature, tetraethyl titanate (114.48 mg, 0.502 mmol) was added to compound INT-A (50 mg, 0.251 mmol) and compound E06-SR-1 (27.92 mg, 0.276 mmol) in toluene (3 mL). The reaction mixture was stirred at 100 °C for 3 hours, then cooled to room temperature and concentrated to dryness under reduced pressure. The crude product was purified by reverse-phase preparative chromatography (mobile phase A: water; mobile phase B: methanol) to afford a reddish-brown solid, compound E06-SR (5.7 mg, 7.49%). ESI/MS(m/z): 283.15 [M+H]⁺.¹H NMR (400 MHz, DMSO-*d*₆) δ 10.72 (s, 1H), 7.29-7.23 (m, 2H), 6.95 (dd, *J =* 8.4, 1.5 Hz, 1H), 4.16 (p, *J =* 5.8 Hz, 1H), 4.05 (p, *J =* 7.1 Hz, 1H), 2.75 (t, *J =* 6.0 Hz, 2H), 2.55 (td, *J =* 6.0, 3.7 Hz, 2H), 2.38 (s, 3H), 2.15 (dt, *J =* 13.3, 6.9 Hz, 1H), 1.95 (p, *J =* 6.3 Hz, 2H), 1.81 (ddd, *J =* 15.7, 7.0, 2.9 Hz, 2H), 1.76-1.63 (m, 2H), 1.50 (dt, *J =* 13.0, 6.5 Hz, 1H).

### Example 7: Synthesis of Compound E07

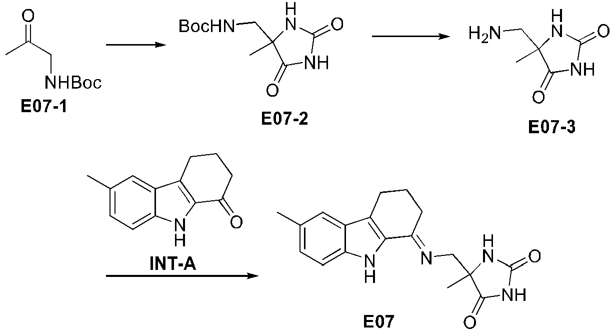

At room temperature, a solution of compound E07-1 (800 mg, 4.619 mmol) in ethanol (4 mL) was added to of ammonium chloride (0.99 g, 18.476 mmol) and ammonium carbonate (6.66 g, 69.285 mmol) in water (4 mL). The reaction mixture was stirred at room temperature for 15 minutes, followed by the addition of trimethylsilyl cyanide (2.06 g, 20.785 mmol). After stirred at 65 °C for 24 hours, the mixture was extracted with ethyl acetate (3 × 100 mL). The organic layers were combined, washed with brine (3 × 50 mL), dried over anhydrous sodium sulfate, and concentrated under reduced pressure to afford a yellow solid compound E07-2 (600 mg, 53.40%). ESI/MS (m/z): 244.15 [M+H]⁺.

At room temperature, trifluoroacetic acid (3 mL) was added to a solution of compound E07-2 (550 mg, 2.261 mmol) **in** dichloromethane (5 mL). The reaction mixture was stirred at room temperature for 3 hours and then concentrated directly to dryness. The residue was purified by reverse-phase preparative chromatography (mobile phase A: water containing 20 mmol/L formic acid; mobile phase B: acetonitrile) to yield a brown solid compound E07-3 (280 mg, 86.52%). ESI/MS (m/z): 144.15 [M+H]⁺.

At room temperature, tetraethyl titanate (114 mg, 0.502 mmol) was added to a toluene (4 mL) solution containing compound E07-3 (50 mg, 0.251 mmol) and compound INT-A (108 mg, 0.753 mmol). The reaction mixture was stirred at 80 °C for 4 hours, then cooled to room temperature. The flitrate was directly collected, washed with toluene (3 × 2 mL) and ethanol (3 mL), and dried to afford an off-white solid compound E07 (35.2 mg, 43.24%). ESI/MS (m/z): 325.20 [M+H]⁺.¹H NMR (400 MHz, DMSO-*d*₆): δ 10.49 (s, 1H), 7.83 (s, 1H), 7.32-7.27 (m, 2H), 6.99 (dd, *J* = 8.4, 1.5 Hz, 1H), 3.64-3.50 (m, 2H), 2.77 (t, *J =* 6.0 Hz, 2H), 2.59 (dt, *J =* 16.3, 6.2 Hz, 1H), 2.46 (d, *J =* 6.1 Hz, 1H), 2.36 (s, 3H), 1.97 (p, *J =* 6.3 Hz, 2H), 1.35 (s, 3H).

### Example 8: Synthesis of Compound E08

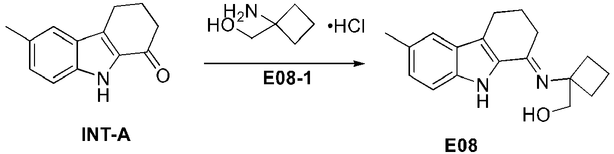

At room temperature, tetraethyl titanate (228.97 mg, 1.004 mmol) was added to a toluene (4 mL) solution containing compound INT-A (100 mg, 0.502 mmol) and compound E08-1 (101.53 mg, 1.004 mmol). The reaction mixture was stirred at 100 °C for 3 hours, then cooled to room temperature and concentrated under reduced pressure. The resulting crude product was washed with ethyl acetate (10 mL), n-hexane (10 mL), and acetonitrile (10 mL) to afford a pale-yellow solid, compound E08 (19.5 mg, 12.85%). ESI/MS(m/z): 283.15 [M+H]⁺.¹H NMR (400 MHz, DMSO-*d*₆)*:* δ 10.68 (s, 1H), 7.24 (d, *J =* 6.8 Hz, 2H), 6.93 (d, *J =* 8.5 Hz, 1H), 4.88 (s, 1H), 3.55 (s, 2H), 2.73 (t, *J =* 5.7 Hz, 2H), 2.53 (s, 2H), 2.35 (s, 3H), 2.33-2.25 (m, 2H), 2.25-2.16 (m, 2H), 1.91 (q, *J =* 10.6, 7.9 Hz, 2H), 1.68 (q, *J =* 8.7 Hz, 2H).

### Example 9: Synthesis of Compound E09

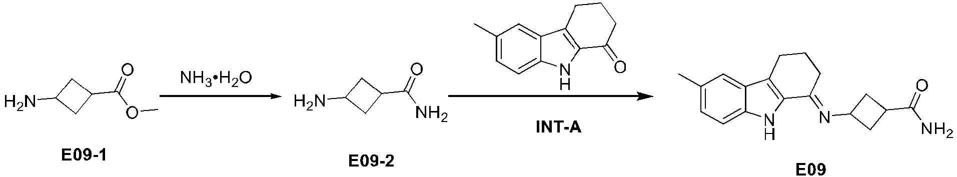

At room temperature, aqueous ammonia (3 mL) was added to compound E09-1 (250 mg, 1.936 mmol). The mixture was stirred at room temperature overnight and then concentrated under reduced pressure. The residue was purified by reverse-phase preparative chromatography (mobile phase A: water containing 5 mmol/L ammonium bicarbonate; mobile phase B: acetonitrile) to afford a white solid, compound E09-2. ESI/MS (m/z): 115.35 [M+H]⁺.

At room temperature, tetraethyl titanate (228.97 mg, 1.004 mmol) was added to compound INT-A (100 mg, 0.502 mmol) and compound E09-2 (143.22 mg, 1.255 mmol) in toluene (6 mL). The reaction mixture was stirred at 100 °C overnight and then filtered. The filter cake was washed with toluene (5 mL), ethyl acetate (5 mL), and n-hexane (10 mL). The solid was then dissolved in dichloromethane (3 × 50 mL) and filtered. The filtrate was concentrated under reduced pressure, and the resulting solid was washed with n-hexane (10 mL) to afford a reddish-brown-yellow solid, compound E09 (19.4 mg, 12.79%). ESI/MS (m/z): 296.15 [M+H]⁺. ¹H NMR (400 MHz, DMSO-*d*₆): δ 12.83 (d, *J =* 46.6 Hz, 1H), 12.52 (d, *J =* 16.3 Hz, 1H), 7.52 (s, 1H), 7.43-7.33 (m, 2H), 7.31 (d, *J =* 8.5 Hz, 1H), 6.90 (t, *J =* 4.8 Hz, 1H), 4.65-4.49 (m, 1H), 3.03-2.89 (m, 4H), 2.80 (h, *J =* 8.2, 7.5 Hz, 1H), 2.60 (dd, *J =* 8.4, 3.9 Hz, 1H), 2.56 (t, *J =* 8.5 Hz, 2H), 2.39 (s, 3H), 2.09 (p, *J =* 6.2 Hz, 2H).

### Example10: Synthesis of Compound E10

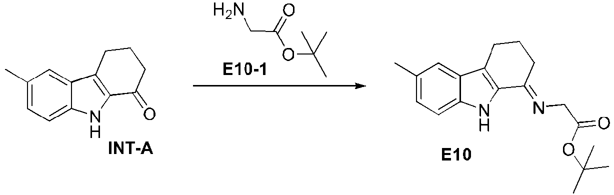

Compound INT-A (149.4 mg, 0.750 mmol) and compound E10-1 (393.5 mg, 3.000 mmol) in toluene (5 mL) was stirred at 130 °C for 6 hours. After cooling to room temperature, the mixture was concentrated. The precipitate was collected by filtration and washed with ethanol to afford a white solid, compound E10 (95.4 mg, 40.72%). ESI/MS (m/z): 313.0 [M+H]⁺. ¹H NMR (500 MHz, DMSO-*d*₆) δ 10.93 (s, 1H), 7.29 (s, 1H), 7.25 (d, *J =* 8.3 Hz, 1H), 6.98 (dd, *J =* 8.3, 1.0 Hz, 1H), 4.27 (s, 2H), 2.78 (t, *J =* 6.0 Hz, 2H), 2.52 (d, *J =* 6.1 Hz, 2H), 2.36 (s, 3H), 1.99-1.93 (m, 2H), 1.44 (s, 9H).

### Example 11: Synthesis of Compound E11

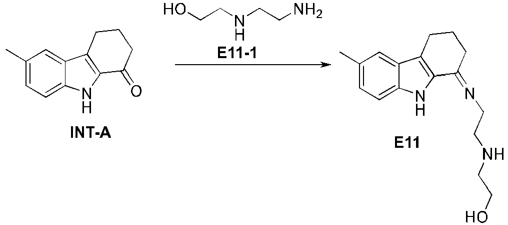

Compound INT-A (99.6 mg, 0.500 mmol) and compound E11-1 (156.2 mg, 1.500 mmol) in toluene (5 mL) was stirred at 130 °C for 4 hours. After the reaction mixture cooled to room temperature, the solvent was removed by vacuum concentration. The residue was dissolved in a small amount of ethanol, then a large amount of water was added. The precipitate was collected by filtration and dried to afford a pale green solid, compound E11 (59.7 mg, 41.84%). ESI/MS (m/z): 286.0 [M+H]⁺. ¹H NMR (500 MHz, DMSO-*d*₆) δ 10.78 (s, 1H), 7.28 (s, 1H), 7.25 (d, *J =* 8.3 Hz, 1H), 6.96 (d, *J =* 8.3 Hz, 1H), 4.45 (s, 1H), 3.52 (t, *J =* 6.2 Hz, 2H), 3.47 (t, *J =* 5.8 Hz, 2H), 2.83 (t, *J =* 6.3 Hz, 2H), 2.76 (t, *J =* 6.0 Hz, 2H), 2.64 (t, *J =* 5.8 Hz, 2H), 2.56 (t, *J =* 6.2 Hz, 2H), 2.35 (s, 3H), 2.00-1.95 (m, 2H).

### Example 12: Synthesis of Compound E12

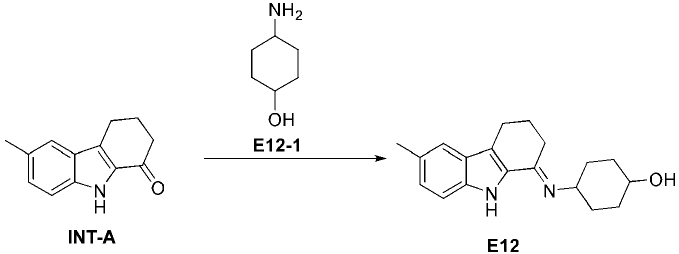

Compound INT-A (99.6 mg, 0.500 mmol) and compound E12-1 (172.8 mg, 1.500 mmol) in toluene (5 mL) was stirred at 135 °C for 6 hours. After the reaction mixture cooled to room temperature, the precipitate was collected by filtration and washed with ethanol to afford a white solid, compound E12 (56.2 mg, 37.79%). ESI/MS (m/z): 297.0 [M+H]⁺. ¹H NMR (500 MHz, DMSO-*d*₆) δ 10.65 (s, 1H), 7.26-7.23 m, 2H), 6.95-6.92 (m, 1H), 4.54 (d, *J =* 4.0 Hz, 1H), 3.56-3.49 (m, 1H), 3.49-3.43 (m, 1H), 2.75 (t, *J =* 5.9 Hz, 2H), 2.60-2.56 (m, 2H), 2.35 (s, 3H), 1.99-1.94 (m, 2H), 1.91- 1.86 (m, 2H), 1.69-1.63 (m, 2H), 1.52-1.42 (m, 2H), 1.35-1.27 (m, 2H).

### Example 13: Synthesis of Compound E13

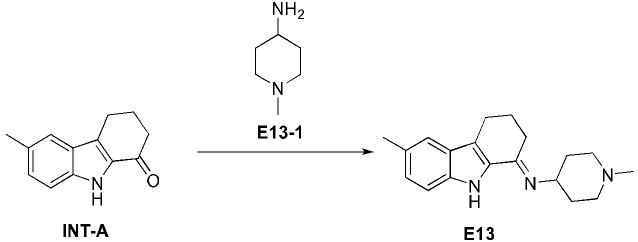

Compound INT-A (99.6 mg, 0.500 mmol) and compound E13-1 (285.5 mg, 2.500 mmol) in toluene (5 mL) was stirred at 135 °C for 3 hours. The resulting solid obtained by concentrating the reaction mixture was purified twice with ethanol to afford a white solid, compound E13 (89.3 mg, 60.45%). ESI/MS (m/z): 296.0 [M+H]⁺. ¹H NMR (500 MHz, DMSO-*d*₆) δ 10.69 (s, 1H), 7.27-7.25 (m, 2H), 6.96-6.93 (m, 1H), 3.58-3.50 (m, 1H), 2.83-2.78 (m, 2H), 2.76 (t, *J =* 6.0 Hz, 2H), 2.62-2.57 (m, 2H), 2.35 (s, 3H), 2.19 (s, 3H), 2.05-1.94 (m, 4H), 1.71-1.60 (m, 4H).

### Example 14: Synthesis of Compound E14

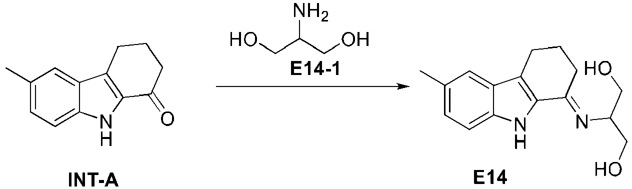

Compound INT-A (199.3 mg, 1.000 mmol) and compound E14-1 (273.3 mg, 3 mmol) in xylene (4 mL) was stirred at 165 °C for 3 hours. The reaction mixture was concentrated under vacuum, dissolved in a small amount of ethanol, and then added to a large volume of water. The resulting turbid solution was subjected to vacuum removal of ethanol. The precipitated solid was washed with a small amount of ethyl acetate to afford a pale-yellow solid, compound E14 (84.5 mg, 31.03%). ESI/MS (m/z): 273.2 [M+H]⁺. ¹H NMR (500 MHz, DMSO-*d*₆) δ 10.76 (s, 1H), 7.28 (s, 1H), 7.25 (d, *J =* 8.3 Hz, 1H), 6.95 (d, *J =* 8.3 Hz, 1H), 4.49 (t, *J =* 5.2 Hz, 2H), 3.83-3.78 (m, 1H), 3.63-3.59 (m, 2H), 3.43-3.38 (m, 2H), 2.76 (t, *J =* 5.4 Hz, 2H), 2.64 (t, *J =* 5.6 Hz, 2H), 2.35 (s, 3H), 1.98-1.93 (m, 2H).

### Example 15: Synthesis of Compound E15

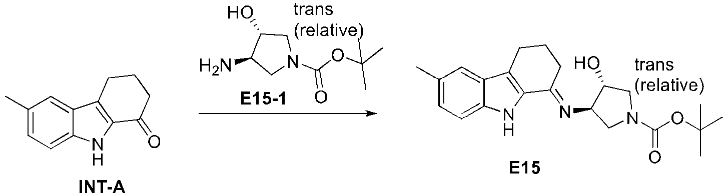

Compound INT-A (199.3 mg, 1.000 mmol) and compound E15-1 (378.0 mg, 2.500 mmol) in toluene (5 mL) was stirred at 130 °C for 2 hours. After the reaction mixture was cooled to room temperature, the solvent was removed by vacuum concentration. The residue was washed with acetonitrile to afford a white solid, compound E15 (245.4 mg, 63.99%). ESI/MS (m/z): 384.1 [M+H]⁺.

### Example 16: Synthesis of Compound E16

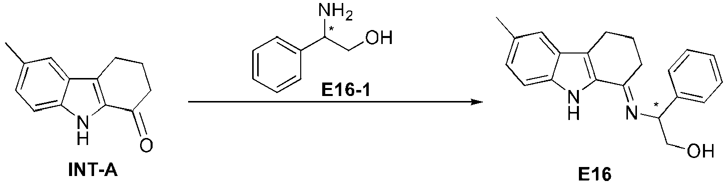

Under nitrogen atmosphere, compound E16-1 (138 mg, 1.004 mmol) was added to compound INT-A (100 mg, 0.502 mmol) and tetraethyl titanate (229 mg, 1.004 mmol) in toluene (3 mL) at room temperature. The reaction mixture was stirred at 80 °C overnight, then cooled to room temperature and concentrated. The crude product was purified by reverse-phase preparative chromatography (mobile phase A: water; mobile phase B: acetonitrile) to afford a yellow solid, compound E16 (21.5 mg, 13.14%). ESI/MS (m/z): 319.10 [M+H]⁺. ¹H NMR (400 MHz, DMSO-*d*₆): δ 10.82 (s, 1H), 7.54-7.48 (m, 2H), 7.31 (q, *J =* 6.6, 5.6 Hz, 4H), 7.22 (dd, *J =* 8.4, 6.2 Hz, 1H), 6.98 (dd, *J =* 8.3, 1.7 Hz, 1H), 4.85 (dd, *J =* 7.6, 5.0 Hz, 1H), 4.73 (t, *J =* 5.8 Hz, 1H), 3.74-3.57 (m, 2H), 2.83-2.71 (m, 3H), 2.48-2.40 (m, 1H), 2.36 (s, 3H), 2.03-1.86 (m, 2H).

### Example 17: Synthesis of Compound E17

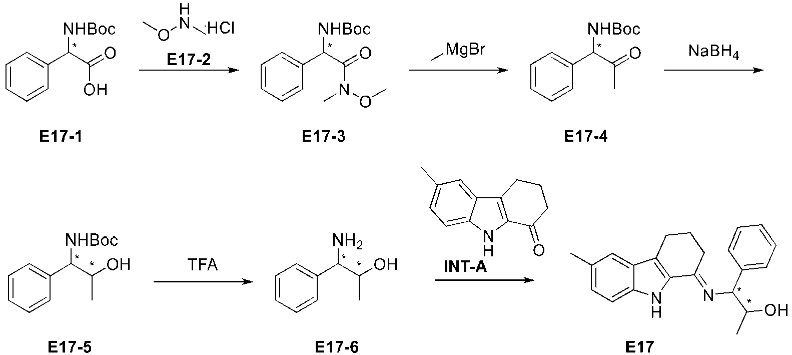

At room temperature, HOBT (1-hydroxybenzotriazole, 1.61 g, 11.938 mmol) and EDCI (1-(3-dimethylaminopropyl)-3-ethylcarbodiimide hydrochloride, 2.29 g, 11.938 mmol) were added to compound E17-1 (2 g, 7.959 mmol) in in DMF (N,N-dimethylformamide, 20 mL). The reaction mixture was stirred at room temperature for one hour, then triethylamine (3.22 g, 31.836 mmol) and compound E17-2 (1.16 g, 11.938 mmol)were added and stirred for 2 hours. The mixture was diluted with distilled water and extracted with ethyl acetate. The combined organic layers were washed with brine, dried over anhydrous sodium sulfate, and concentrated to dryness. The crude product was purified by silica gel column chromatography (petroleum ether: ethyl acetate = 1:1) to afford a white solid, compound E17-3 (2.21 g, 94.33%). ESI/MS (m/z): 295.2 [M+H]⁺.

Under a nitrogen atmosphere, 1 M methylmagnesium bromide in tetrahydrofuran (10.19 mL) was slowly added dropwise to compound E17-3 (1 g, 3.397 mmol) in tetrahydrofuran (8 mL) at 0 °C. The reaction mixture was stirred at room temperature for 2 hours, quenched with saturated ammonium chloride solution, and extracted with ethyl acetate. The combined organic layers were washed with brine, dried over anhydrous sodium sulfate, and concentrated to dryness to afford a colorless oily compound E17-4 (730 mg, 86.19%). ESI/MS (m/z): 250.2 [M+H]⁺.

Under a nitrogen atmosphere, NaBH₄ (sodium borohydride, 221.54 mg, 5.856 mmol) was added to compound E17-4 (730 mg, 2.928 mmol) in methanol (10 mL) at 0 °C. The reaction mixture was stirred at room temperature for 1 hour. The reaction was quenched with distilled water and extracted with ethyl acetate. The combined organic layers were washed with brine, dried over anhydrous sodium sulfate, and concentrated to afford compound E17-5 (680 mg, 92.40%). ESI/MS (m/z): 252.2 [M+H]⁺.

At room temperature, trifluoroacetic acid (1.5 mL, 20.195 mmol) was slowly added dropwise to compound E17-5 (670 mg, 2.666 mmol) in dichloromethane (15 mL). The reaction mixture was stirred at room temperature for 2 hours and then concentrated to dryness. The residue was purified by reverse-phase preparative chromatography (mobile phase A: water containing 10 mmol/L ammonium bicarbonate; mobile phase B: acetonitrile) to afford a white solid, compound E17-6 (350 mg, 86.83%). ESI/MS (m/z): 152.2 [M+H]⁺.

At room temperature, compound INT-A (150 mg, 0.753 mmol) was added to compound E17-6 (227.66 mg, 1.506 mmol) in toluene (8 mL). The reaction mixture was stirred at 130 °C for 5 hours, concentrated directly, and purified by reverse-phase preparative chromatography (mobile phase A: water; mobile phase B: acetonitrile) to afford a white solid, compound E17 (19.6 mg, 7.83%). ESI/MS (m/z): 333.20 [M+H]⁺. ¹H NMR (400 MHz, DMSO-*d*₆): δ 10.86 (s, 1H), 7.43 (d, *J =* 7.4 Hz, 2H), 7.29 (q, *J =* 7.6, 7.1 Hz, 4H), 7.19 (t, *J =* 7.3 Hz, 1H), 6.99 (d, *J =* 8.2 Hz, 1H), 4.57 (t, *J =* 5.8 Hz, 2H), 3.93-4.04 (m, 1H), 2.65-2.81 (m, 3H), 2.36 (s, 3H), 2.24-2.33 (m, 1H), 1.90-2.01 (m, 1H), 1.79-1.90 (m, 1H). 1.05 (d, *J =* 6.2 Hz, 3H).

### Example 18: Synthesis of Compound E18

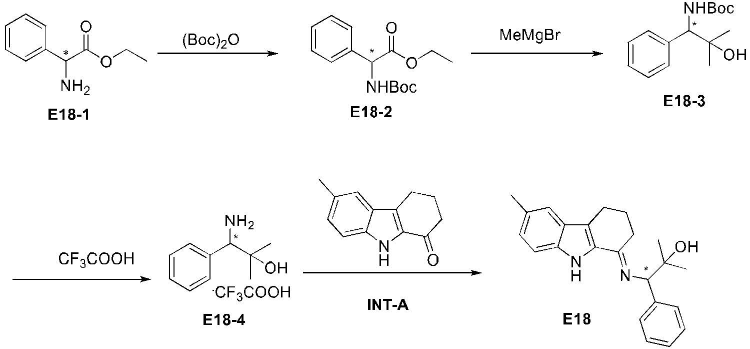

At room temperature, (Boc)₂O (di-tert-butyl dicarbonate, 3.04 g, 13.950 mmol) was added dropwise to compound E18-1 (2 g, 11.160 mmol) and triethylamine (3.39 g, 33.480 mmol) in dichloromethane (15 mL). The reaction mixture was stirred at room temperature for 2 hours, then concentrated directly to dryness. The crude product was purified by silica gel column chromatography (petroleum ether: ethyl acetate = 5:1) to afford a colorless oily compound E18-2 (2.5 g, 76.19%). ESI/MS (m/z): 280.25 [M+H]⁺.

Under a nitrogen atmosphere, 3.4 M solution of methylmagnesium bromide in tetrahydrofuran (4.21 mL, 14.320 mmol) was added dropwise to a tetrahydrofuran (15 mL) solution of compound E18-2 (1 g, 3.580 mmol) at room temperature. The reaction mixture was stirred at room temperature for 2 hours, then quenched with water and extracted with dichloromethane (3 × 30 mL). The combined organic layers were washed with brine (2 × 30 mL), dried over anhydrous sodium sulfate, and concentrated to dryness. The residue was purified by silica gel column chromatography (petroleum ether: ethyl acetate = 1: 1) to afford a yellow solid, compound E18-3 (800 mg, 80.00%). ESI/MS (m/z): 266.25 [M+H]⁺.

At room temperature, trifluoroacetic acid (5 mL) was added dropwise to a dichloromethane (5 mL) solution of compound E18-3 (1 g, 3.769 mmol). The reaction mixture was stirred at room temperature for 1 hour, then concentrated directly to dryness to afford the crude compound E18-4 (1 g). ESI/MS (m/z): 166.25 [M+H]⁺.

Under a nitrogen atmosphere, glacial acetic acid (12.06 mg, 0.201 mmol) was added dropwise to compound INT-A (200 mg, 1.004 mmol) and compound E18-4 (331.71 mg, 2.008 mmol) in toluene (3 mL) at room temperature. The reaction mixture was stirred at 120 °C overnight, then cooled to room temperature and concentrated to dryness. The residue was washed with n-hexane (3 × 5 mL), and the crude product was purified by reverse-phase preparative chromatography (mobile phase A: water; mobile phase B: acetonitrile) to afford a yellow solid, compound E18 (16.8 mg, 4.61%). ESI/MS (m/z): 347.10 [M+H]⁺. ¹H NMR (400 MHz, DMSO-*d*₆): δ 11.01 (s, 1H), 7.40 (d, *J =* 7.5 Hz, 2H), 7.32-7.27 (m, 2H), 7.24 (t, *J =* 7.4 Hz, 2H), 7.18 (t, *J =* 7.1 Hz, 1H), 7.01 (dd, *J =* 8.0, 1.6 Hz, 1H), 4.48 (s, 1H), 4.43 (s, 1H), 2.76-2.66 (m, 2H), 2.66- 2.59 (m, 1H), 2.36 (s, 3H), 2.17-2.06 (m, 1H), 1.90 (s, 1H), 1.82 (s, 1H), 1.13 (s, 3H), 1.00 (s, 3H).

### Example 19: Synthesis of Compound E19

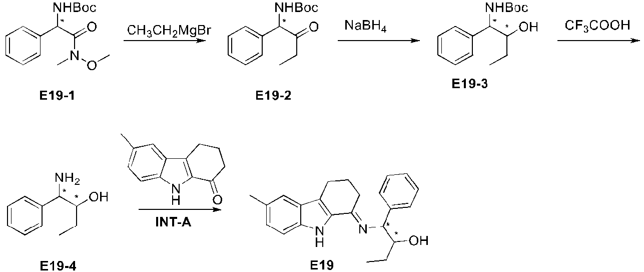

Under a nitrogen atmosphere, a 3.4 M solution of ethylmagnesium bromide in tetrahydrofuran (3.00 mL, 10.191 mmol) was slowly added dropwise to compound E19-1 (1 g, 3.397 mmol) in tetrahydrofuran (8 mL) at 0 °C. The reaction mixture was stirred at room temperature for 2 hours, then quenched with saturated ammonium chloride solution and extracted with ethyl acetate (3 × 30 mL). The combined organic layers were washed with brine (3 × 20 mL), dried over anhydrous sodium sulfate, filtered, and concentrated under vacuum to afford a colorless oily compound E19-2 (710 mg, 79.36%). ESI/MS (m/z): 264.2 [M+H]⁺.

Under a nitrogen atmosphere, NaBH₄ (sodium borohydride, 203 mg, 5.392 mmol) was added to compound E19-2 (710 mg, 2.696 mmol) in methanol (10 mL) at 0 °C. The reaction mixture was stirred at room temperature for 1 hour. The reaction was quenched with water (10 mL) at 0 °C and extracted with ethyl acetate (3 × 20 mL). The combined organic layers were washed with brine (3 × 10 mL), dried over anhydrous sodium sulfate, filtered, and concentrated under vacuum to afford compound E19-3 (650 mg, 90.85%). ESI/MS (m/z): 266.2 [M+H].

At room temperature, trifluoroacetic acid (1.5 mL, 20.195 mmol) was added dropwise to a dichloromethane (15 mL) solution of compound E19-3 (640 mg, 2.412 mmol). The reaction mixture was stirred at room temperature for 2 hours, concentrated under vacuum, and purified by reverse-phase preparative chromatography (mobile phase A: water containing 10 mmol/L ammonium bicarbonate; mobile phase B: acetonitrile) to afford a white solid, compound E19-4 (350 mg, 87.82%). ESI/MS (m/z): 166.2 [M+H]⁺.

Under a nitrogen atmosphere, compound INT-A (150 mg, 0.753 mmol) was added to a toluene (8 mL) solution of compound E19-4 (248 mg, 1.506 mmol) at room temperature. The reaction mixture was stirred at 130 °C for 5 hours, cooled to room temperature, and concentrated to dryness under vacuum. The crude product was purified by reverse-phase preparative chromatography (mobile phase A: water; mobile phase B: acetonitrile) to afford an off-white solid, compound E19 (30.7 mg, 11.77%). ESI/MS (m/z): 347.25 [M+H]⁺. ¹H NMR (400 MHz, DMSO-*d*₆): δ 10.84 (s, 1H), 7.43 (d, *J =* 7.4 Hz, 2H), 7.28 (q, *J =* 7.7 Hz, 4H), 7.18 (t, *J =* 7.2 Hz, 1H), 6.99 (d, *J =* 8.2 Hz, 1H), 4.60 (d, *J =* 6.1 Hz, 1H), 4.48 (d, *J =* 5.1 Hz, 1H), 3.67-3.77 (m, 1H), 2.64-2.84 (m, 3H), 2.36 (s, 3H), 2.24-2.31 (m, 1H), 1.89-2.01 (m, 1H), 1.79-1.89 (m, 1H), 1.44-1.57 (m, 1H), 1.20-1.35 (m, 1H), 0.88 (t, *J =* 7.3 Hz, 3H).

### Example 20: Synthesis of Compound E20

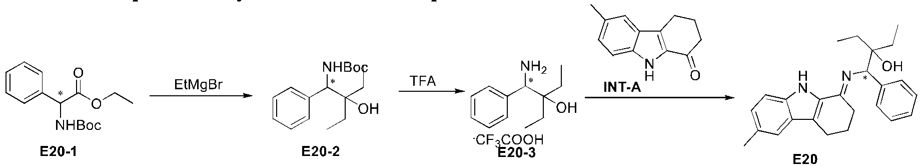

Under a nitrogen atmosphere, a 3.4 M solution of ethylmagnesium bromide in tetrahydrofuran (4.21 mL, 14.320 mmol) was added dropwise to a tetrahydrofuran (15 mL) solution of compound E20-1 (1 g, 3.580 mmol, racemate) at room temperature. The reaction mixture was stirred at room temperature for 2 hours, then quenched with water and extracted with dichloromethane (3 × 30 mL). The combined organic layers were washed with brine (2 × 30 mL), dried over anhydrous sodium sulfate, and concentrated under reduced pressure. The crude product was purified by silica gel column chromatography (petroleum ether:ethyl acetate = 1:1) to afford a yellow solid, compound E20-2 (900 mg, 81.40%, racemate). ESI-MS (m/z): 294.25 [M+H]⁺.

At room temperature, trifluoroacetic acid (5 mL) was added dropwise to a dichloromethane (5 mL) solution of compound E20-2 (900 mg, 0.003 mmol, racemate). The reaction mixture was stirred at room temperature for 1 hour and then concentrated directly to dryness to afford the crude compound E20-3 (1.1 g, racemate). ESI-MS (m/z): 194.25 [M+H]⁺.

Under a nitrogen atmosphere, glacial acetic acid (12 mg, 0.201 mmol) was added dropwise to a toluene (3 mL) solution of compound E20-3 (388 mg, 2.008 mmol, racemate) and compound INT-A (200 mg, 1.004 mmol) at room temperature. The reaction mixture was stirred at 120 °C overnight and then concentrated directly to dryness. The resulting crude product was washed with n-hexane (3 × 5 mL) and purified by reverse-phase preparative chromatography (mobile phase A: water; mobile phase B: acetonitrile) to afford a yellow solid, compound E20 (37.2 mg, 9.90%, racemate). ESI-MS (m/z): 375.15 [M+H]⁺. ¹H NMR (400 MHz, DMSO-*d*₆): δ 11.11 (s, 1H), 7.42 (d, *J =* 7.5 Hz, 2H), 7.28 (t, *J =* 4.0 Hz, 2H), 7.24 (t, *J=* 7.3 Hz, 2H), 7.18 (t, *J =* 7.2 Hz, 1H), 7.02 (d, *J =* 8.4 Hz, 1H), 4.55 (s, 1H), 4.38 (s, 1H), 2.81-2.59 (m, 3H), 2.37 (s, 3H), 2.11 (dt, *J =* 17.1, 4.6 Hz, 1H), 1.98-1.76 (m, 2H), 1.53-1.38 (m, 2H), 1.16 (h, *J=* 7.0 Hz, 2H), 0.83 (t, *J =* 7.4 Hz, 3H), 0.77 (t, *J =* 7.5 Hz, 3H).

### Example 21: Synthesis of Compound E21

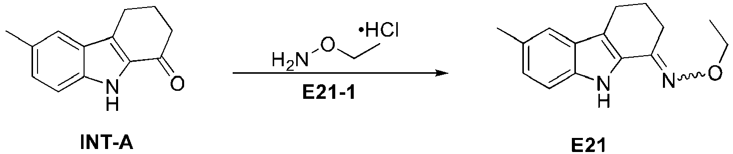

Under a nitrogen atmosphere, E21-1 (293 mg, 3.012 mmol) was added to a toluene (20 mL) solution of compound INT-A (200 mg, 1.004 mmol) at room temperature. The reaction mixture was stirred at 80 °C overnight, cooled to room temperature, and filtered. The filter cake was washed with toluene (15 mL). The filtrate was concentrated to dryness under vacuum, dissolved in n-hexane (60 mL), and filtered. The filtrate was concentrated to dryness under vacuum to afford a yellow-green solid, compound E21 (127.5 mg, 36.64%, mixture of E and Z isomers). ESI/MS (m/z): 243.3 [M+H]⁺.

### Example22: Synthesis of Compound E22

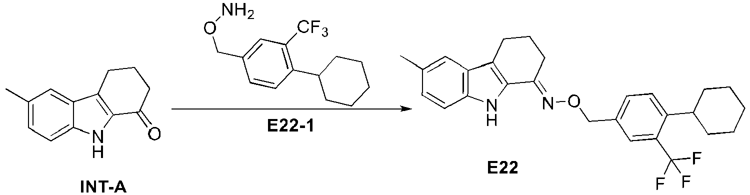

At room temperature, E22-1 (137 mg, 0.502 mmol) was added to a toluene (10 mL) solution of compound INT-A (100 mg, 0.502 mmol). The reaction mixture was stirred at 80 °C overnight, cooled to room temperature, and concentrated to dryness under reduced pressure. Purification by silica gel column chromatography (n-hexane) afforded a pale yellow solid, compound E22 (61 mg, 16.18%). ESI/MS (m/z): 455.3 [M+H]⁺. ¹H NMR (400 MHz, DMSO-*d*₆) δ 10.92 (d, *J =* 41.3 Hz, 1H), 7.72-7.64 (m, 2H), 7.60 (t, *J =* 7.9 Hz, 1H), 7.45-7.18 (m, 2H), 7.01 (ddd, *J =* 39.8, 8.3, 1.6 Hz, 1H), 5.23 (s, 2H), 2.88-2.66 (m, 4H), 2.47 (t, *J =* 6.1 Hz, 1H), 2.37 (d, *J =* 13.1 Hz, 3H), 1.90 (p, *J =* 7.0, 6.3 Hz, 2H), 1.85-1.76 (m, 2H), 1.69 (d, *J=* 13.3 Hz, 3H), 1.51 (q, *J =* 12.3 Hz, 2H), 1.34 (q, *J =* 10.9, 9.3 Hz, 3H).

### Example 23: Synthesis of Compound E23

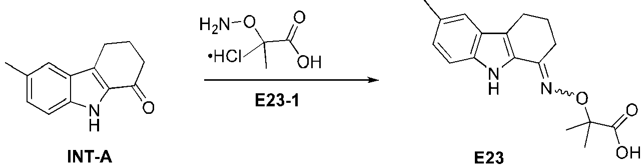

Under a nitrogen atmosphere, compound E23-1 (312 mg, 2.008 mmol) was added to a toluene (20 mL) solution of compound INT-A (200 mg, 1.004 mmol) at room temperature. The reaction mixture was stirred at 80 °C overnight and cooled to room temperature. The mixture was filtered, and the filter cake was washed with toluene (5 mL) and n-hexane (20 mL). The solid was then dissolved in dichloromethane (100 mL) and filtered. The filtrate was concentrated to dryness under vacuum to afford a yellow-green solid, compound E23 (30 mg, 10.11%, mixture of Z and E isomers). ESI/MS (m/z): 301.3 [M+H]⁺.

### Example 24: Synthesis of Compound E24

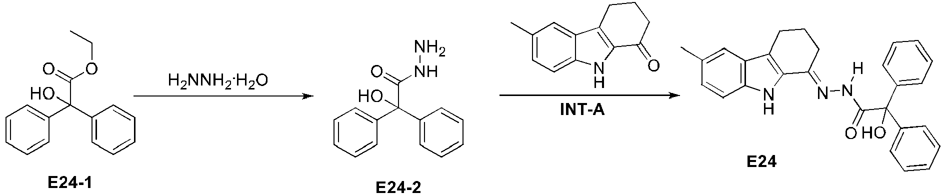

At room temperature, hydrazine hydrate (390 mg, 7.804 mmol) was slowly added to a methanol (15 mL) solution of compound E24-1 (1 g, 3.902 mmol). The reaction mixture was stirred at 65 °C for 2 hours and then cooled to room temperature. After concentration under vacuum, the residue was purified by silica gel column chromatography (petroleum ether:ethyl acetate = 1:4) to afford a white solid, compound E24-2 (420 mg, 44.43%). ESI/MS (m/z): 243.1 [M+H]⁺.

Under a nitrogen atmosphere, acetic acid (9 mg, 0.151 mmol) was added to a toluene (8 mL) solution of compound E24-2 (182 mg, 0.753 mmol) and compound INT-A (150 mg, 0.753 mmol) at room temperature. The reaction mixture was stirred at 100 °C overnight and cooled to room temperature. The mixture was filtered, and the filter cake was washed with toluene (30 mL), diethyl ether (30 mL), and methanol (30 mL). The solid was dried under vacuum to yield a yellow solid, compound E24 (28 mg, 8.97%). ESI/MS (m/z): 424.20 [M+H]⁺. ¹H NMR (400 MHz, DMSO-*d*₆): δ 11.06 (s, 1H), 10.41 (s, 1H), 7.46 (d, *J =* 7.1 Hz, 4H), 7.37 (s, 1H), 7.36 (s, 2H), 7.34 (s, 1H), 7.32 (s, 1H), 7.31 (d, 1H), 7.28 (s, 1H), 7.25 (d, 1H), 7.11 (s, 1H), 6.97 (d, *J* = 8.4 Hz, 1H), 2.78 (t, 2H), 2.67 (t, 2H), 2.35 (s, 3H), 1.99 (p, 2H).

### Example25: Synthesis of Compound E25

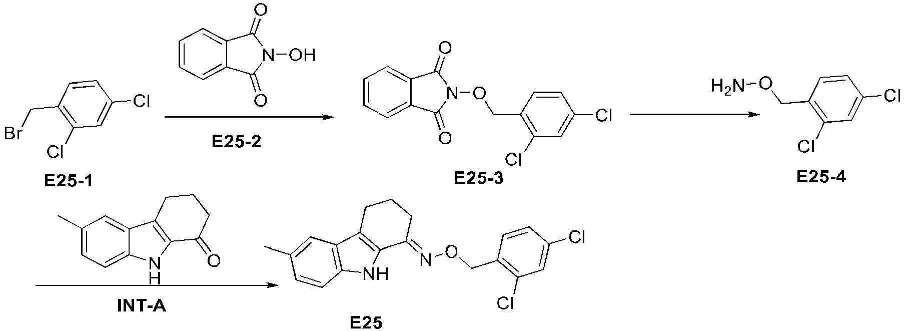

At room temperature, sodium hydroxide (120 mg, 3.001 mmol) was added to a DMF (5 mL) solution of E25-1 (600 mg, 2.501 mmol) and compound E25-2 (408 mg, 2.501 mmol). The reaction mixture was stirred at room temperature overnight. The mixture was quenched with water at 0 °C, , and the solid was collected by filtration and washed with water (3 × 5 mL) to afford a white solid, compound E25-3 (700 mg, 86.89%). ESI/MS (m/z): 322.1 [M+H]⁺.

At room temperature, hydrazine hydrate (62 mg, 1.242 mmol) was added to a methanol (5.5 mL) solution of compound E25-3 (200 mg, 0.621 mmol). The reaction mixture was stirred at 70 °C for 6 hours and then cooled to room temperature. The mixture was filtered and washed with methanol (3 × 5 mL). The filtrate was collected and concentrated under vacuum. Diethyl ether (10 mL) was added, and the mixture was stirred at room temperature for 30 minutes. After filtration, the filter cake was washed with diethyl ether (2 × 5 mL). The filtrate was collected and concentrated under vacuum to afford a white liquid, compound E25-4 (75 mg, 62.90%). ESI/MS (m/z): 192.1 [M+H]⁺.

Under a nitrogen atmosphere, tetraethyl titanate (103 mg, 0.451 mmol) was added to a toluene (2 mL) solution of compound E25-4 (75 mg, 0.391 mmol) and compound INT-A (60 mg, 0.300 mmol) at room temperature. The reaction mixture was stirred at 80 °C for 3 hours, cooled to room temperature, and concentrated under vacuum. The residue was extracted with ethyl acetate (3 × 30 mL). The combined organic layers were washed with brine (2 × 20 mL), dried over anhydrous sodium sulfate, and concentrated under vacuum. The crude product was purified by reverse-phase preparative chromatography (mobile phase A: water; mobile phase B: acetonitrile) to afford a yellow oily compound E25 (17 mg, 15.61%). ESI/MS (m/z): 372.9 [M+H]⁺.¹H NMR (400 MHz, DMSO-*d*₆): δ 10.90 (d, *J =* 37.7 Hz, 1H), 7.64 (dd, *J =* 8.6, 2.0 Hz, 1H), 7.55-7.17 (m, 4H), 7.01 (ddd, *J =* 41.6, 8.4, 1.7 Hz, 1H), 5.27 (d, *J =* 9.9 Hz, 2H), 2.78 (ddt, *J =* 17.1, 12.0, 6.0 Hz, 4H), 2.37 (d, *J =* 14.3 Hz, 3H), 1.93 (q, *J* = 7.1, 6.3 Hz, 2H).

### Example26: Synthesis of Compound E26

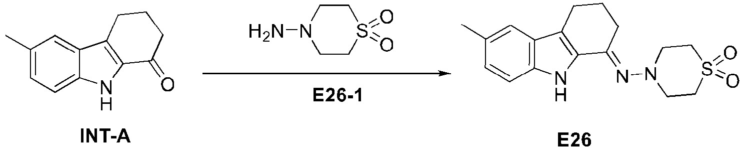

Under a nitrogen atmosphere, acetic acid (12 mg, 0.201 mmol) was added to a toluene (10 mL) solution of compound INT-A (200 mg, 1.004 mmol) and compound E26-1 (301 mg, 2.008 mmol) at room temperature. The reaction mixture was stirred at 100 °C overnight and then cooled to room temperature. The precipitated solid was collected by filtration and washed with toluene (15 mL), diethyl ether (15 mL), n-hexane (15 mL), and methanol (15 mL). After drying, a yellow solid, compound E26 (85.3 mg, 25.64%), was obtained. ESI/MS (m/z): 332.15 [M+H]⁺. ¹H NMR (300 MHz, DMSO-*d*₆): δ 10.94 (s, 1H), 7.29 (s, 1H), 7.25 (d, *J =* 8.3 Hz, 1H), 7.00 (dd, *J =* 8.4, 1.3 Hz, 1H), 3.34 (s, 4H), 3.31 (s, 4H), 2.78 (t, *J =* 5.9 Hz, 4H), 2.37 (s, 3H), 1.95 (p, *J =* 6.0 Hz, 2H).

### Example27: Synthesis of Compound E27

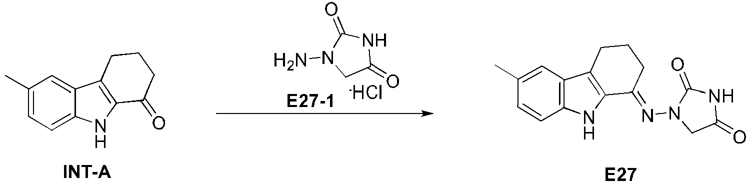

Under a nitrogen atmosphere, compound E27-1 (228 mg, 1.506 mmol) was added to a toluene (3 mL) solution of compound INT-A (150 mg, 0.753 mmol) at room temperature. The reaction mixture was stirred at 140 °C overnight and then cooled to room temperature. The mixture was concentrated under reduced pressure, and the crude product was purified by silica gel column chromatography (petroleum ether:ethyl acetate = 1:1) to give a yellow solid. The solid was further washed with n-hexane (3 × 5 mL) to afford a yellow solid, compound E27 (15 mg, 6.72%). ESI/MS (m/z): 297.10 [M+H]⁺. ¹H NMR (400 MHz, DMSO-*d*₆): δ 11.14 (s, 1H), 11.11 (s, 1H), 7.32 (s, 1H), 7.26 (d, *J =* 8.3 Hz, 1H), 7.02 (dd, *J =* 8.4, 1.7 Hz, 1H), 4.29 (s, 2H), 2.81 (t, *J =* 5.9 Hz, 2H), 2.74 (t, *J=* 6.3 Hz, 2H), 2.37 (s, 3H), 1.96 (dd, *J=* 12.8, 6.5 Hz, 2H).

### Example 28: Synthesis of Compound E28

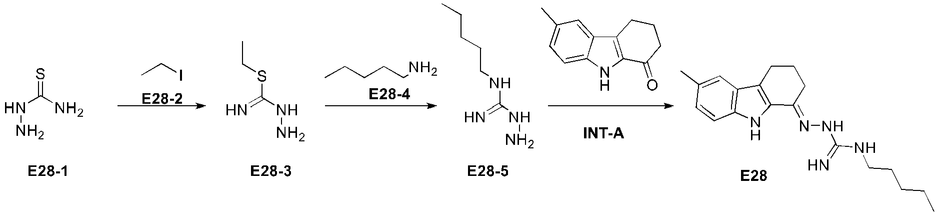

At 57 °C, a solution of compound E28-2 (5.13 g, 32.920 mmol) was added dropwise to an ethanol (38 mL) solution of compound E28-1 (2 g, 21.947 mmol). The reaction mixture was stirred at 57 °C for 7 hours, then cooled to -20 °C. A small amount of n-hexane was added, and the precipitated solid was collected by filtration and washed with diethyl ether (4 × 10 mL) to afford a white solid, compound E28-3 (2.4 g, 91.75%). ESI/MS (m/z): 120.15 [M+H]⁺.

At 60 °C, compound E28-4 (2.81 mL, 24.163 mmol) was added dropwise to a methanol (15 mL) solution of compound E28-3 (2.4 g, 3.580 mmol). The reaction mixture was stirred at 60 °C overnight and then cooled to room temperature. After concentration under reduced pressure, the crude product was purified by reverse-phase preparative chromatography (mobile phase A: water containing 10 mmol/L ammonium bicarbonate; mobile phase B: acetonitrile) to afford a brownish-yellow oily compound E28-5 (1.9 g, 65.43%). ESI/MS (m/z): 145.05 [M+H]⁺.

At room temperature, compound E28-5 (289.53 mg, 2.008 mmol) was added dropwise to a toluene (10 mL) solution of compound INT-A (200 mg, 1.004 mmol) and tetraethyl titanate (420.89 µL, 2.008 mmol). The reaction mixture was stirred at 100 °C for 3 hours and then cooled to room temperature. The mixture was concentrated under reduced pressure, and the crude product was sequentially washed with toluene (2 × 10 mL), diethyl ether (3 × 10 mL), n-hexane (3 × 10 mL), and water (2 × 10 mL) to afford a brown solid, compound E28 (151.0 mg, 46.22%). ESI/MS (m/z): 326.25 [M+H]⁺. ¹H NMR (400 MHz, DMSO-*d*₆): δ 10.98 (s, 1H), 10.43 (s, 1H), 7.94 (s, 2H), 7.36-7.24 (m, 2H), 7.04 (dd, *J =* 8.4, 1.7 Hz, 1H), 3.34 (s, 1H), 3.31 (s, 1H), 2.79 (t, *J =* 6.0 Hz, 2H), 2.62 (t, *J =* 6.3 Hz, 2H), 2.37 (s, 3H), 2.01 (p, *J* = 6.0 Hz, 2H), 1.59 (p, *J =* 7.2 Hz, 2H), 1.41-1.27 (m, *J =* 6.5, 5.9 Hz, 4H), 0.94-0.86 (m, 3H).

### Example 29: Synthesis of Compound B10 and B11

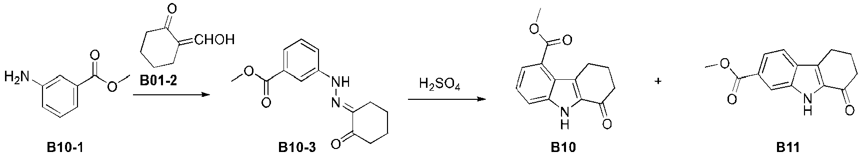

At 0 °C, a solution of sodium nitrite (457 mg, 6.616 mmol) in water (10 mL) was added to a mixture of compound B10-1 (500 mg, 3.308 mmol) in water (30 mL) and concentrated hydrochloric acid (1.5 mL). Then, a solution of compound B01-2 (417 mg, 3.308 mmol) and sodium acetate (597 mg, 7.278 mmol) in a mixture of methanol (10 mL) and water (10 mL) was added. The reaction mixture was stirred at 0 °C for 30 minutes, filtered, and the solid was washed with water (3 × 20 mL) to afford a yellow solid, compound B10-3 (400 mg, 46.46%). ESI/MS (m/z): 261.1 [M+H]⁺.

A solution of compound B10-3 (100 mg, 0.384 mmol), concentrated sulfuric acid (0.2 mL), and water (2 mL) was stirred at 65 °C for 30 minutes, then cooled to room temperature. The pH was adjusted to 8 using potassium hydroxide, and the mixture was extracted with ethyl acetate (3 × 10 mL). The combined organic layers were washed with brine (10 mL), dried over anhydrous sodium sulfate, and concentrated to dryness. The crude product was purified by silica gel column chromatography (petroleum ether:ethyl acetate = 5:1) and further purified by reverse-phase preparative chromatography (mobile phase A: water containing 10 mmol/L ammonium bicarbonate; mobile phase B: acetonitrile) to afford a white solid, compound B10 (26.1 mg, 27.93%) and compound B11 (30.8 mg, 32.96%).

Compound B10: ESI/MS (m/z): 244.1 [M+H]⁺. ¹H NMR (400 MHz, DMSO-*d*₆) δ 12.01 (s, 1H), 7.66 (d, *J =* 8.3 Hz, 1H), 7.60 (d, *J =* 7.3 Hz, 1H), 7.37 (*t, J =* 7.8 Hz, 1H), 3.90 (s, 3H), 3.08 (t, *J =* 5.8 Hz, 2H), 2.63-2.54 (m, 2H), 2.11 (p, *J =* 5.4 Hz, 2H).

Compound B11: ESI/MS (m/z): 244.1 [M+H]⁺.¹H NMR (400 MHz, DMSO-*d*₆) δ 11.88 (s, 1H), 8.06 (s, 1H), 7.78 (d, *J =* 8.4 Hz, 1H), 7.66 (dd, *J =* 8.4, 1.4 Hz, 1H), 3.88 (s, 3H), 2.97 (t, *J =* 5.8 Hz, 2H), 2.66-2.55 (m, 2H), 2.23-2.12 (m, 2H).

### Example 30: Synthesis of Compound E29

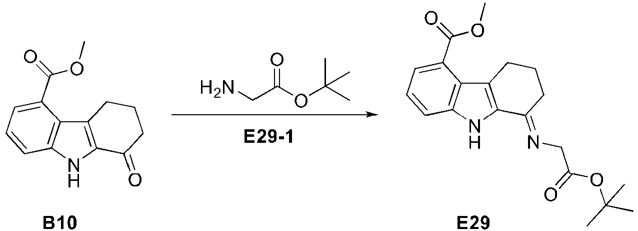

Under a nitrogen atmosphere, compound E29-1 (216 mg, 1.644 mmol) was added to a toluene (4 mL) solution of compound B10 (100 mg, 0.411 mmol) at room temperature. The reaction mixture was stirred at 140 °C overnight and then cooled to room temperature. After concentration under reduced pressure, the crude product was purified by reverse-phase preparative chromatography (mobile phase A: water; mobile phase B: acetonitrile) to afford a yellow solid, compound E29 (39.5 mg, 26.15%). ESI/MS (m/z): 357.2 [M+H]⁺. ¹H NMR (400 MHz, DMSO-*d*₆) δ 11.52 (s, 1H), 7.64 (d, *J =* 8.1 Hz, 1H), 7.51 (dd, *J =* 7.4, 1.0 Hz, 1H), 7.25-7.20 (m, 1H), 4.32 (s, 2H), 3.87 (s, 3H), 2.96 (t, *J =* 6.0 Hz, 2H), 2.54 (t, *J =* 6.0 Hz, 2H), 1.93 (p, *J =* 6.2 Hz, 2H), 1.45 (s, 9H).

### Example 31: Synthesis of Compound E30

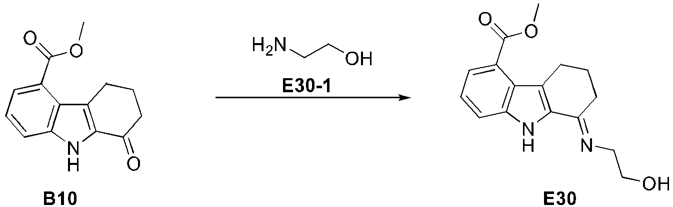

Under a nitrogen atmosphere at room temperature, E30-1 (226 mg, 3.700 mmol) was added to a toluene (5 mL) solution of compound B10 (180 mg, 0.740 mmol). The reaction mixture was stirred at 130 °C under nitrogen for 6 hours. After cooling to room temperature, the mixture was concentrated under reduced pressure. The crude product was washed with ethanol (3 × 5 mL), and the resulting solid was dried under vacuum to afford an orange-yellow solid, compound E30 (38.7 mg, 17.59%). ESI/MS (m/z): 287.2 [M+H]⁺. ¹H NMR (400 MHz, DMSO-*d*₆) δ 11.39 (s, 1H), 7.63 (dd, *J =* 8.1, 0.9 Hz, 1H), 7.49 (dd, *J =* 7.4, 0.9 Hz, 1H), 7.25-7.15 (m, 1H), 4.57 (s, 1H), 3.87 (s, 3H), 3.71 (t, *J =* 6.2 Hz, 2H), 3.58 (t, *J =* 6.2 Hz, 2H), 2.94 (t, *J =* 6.0 Hz, 2H), 2.58 (t, *J =* 6.0 Hz, 2H), 1.93 (p, *J =* 6.2 Hz, 2H).

### Example 32: Synthesis of Compound B01

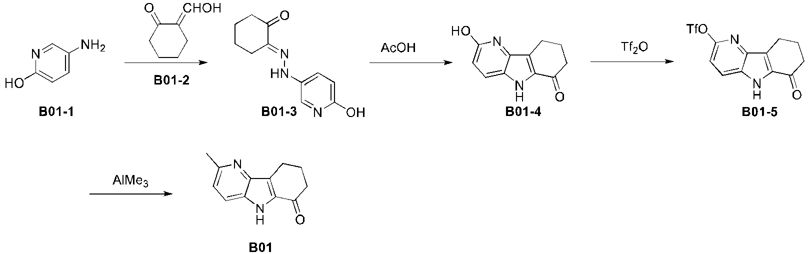

At 0 °C, an aqueous solution (20 mL) of sodium nitrite (1.25 g, 18.162 mmol) was slowly added to a mixture of compound B01-1 (1 g, 9.081 mmol) and concentrated hydrochloric acid (2.70 mL, 88.903 mmol) in water (83 mL). The reaction mixture was stirred at 0 °C for 0.5 hours, followed by the slow addition of a solution of compound B01-2 (1.15 g, 9.081 mmol) and sodium acetate (1.64 g, 19.978 mmol) in a mixture of methanol and water (80 mL, 1:1). The reaction was continued at 0 °C for another 0.5 hours. The pH was adjusted to 8 with a saturated aqueous sodium bicarbonate solution, and the mixture was concentrated under reduced pressure. The crude product was purified by silica gel column chromatography (dichloromethane:methanol = 5:1) to afford a yellow solid, compound B01-3 (400 mg, 20.09%). ESI/MS (m/z): 220.1 [M+H]⁺.

At room temperature, acetic acid (12 mL, 209.359 mmol) and concentrated sulfuric acid (1.20 mL, 22.508 mmol) were added to a methanol (3 mL) solution of compound B01-3. The reaction mixture was stirred at 65 °C for 2 hours and then cooled to room temperature. The pH was adjusted to 8 with a saturated aqueous sodium bicarbonate solution, and the mixture was concentrated under reduced pressure. The crude product was purified by silica gel column chromatography (dichloromethane:methanol = 5:1) to afford a yellow solid, compound B01-4 (300 mg, 43.42%). ESI/MS (m/z): 203.1 [M+H]⁺.

At room temperature, pyridine (587 mg, 7.420 mmol) and trifluoromethanesulfonic anhydride (935 mg, 4.452 mmol) were added to a mixture of compound B01-4 (300 mg, 1.484 mmol) **in** dichloromethane (10 mL), and the mixture was stirred for 2 hours. After concentration under reduced pressure, the crude product was purified by silica gel column chromatography (petroleum ether:ethyl acetate = 1:1) to afford a yellow solid, compound B01-5 (210 mg, 42.35%). ESI/MS (m/z): 334.8 [M+H]⁺.

Under a nitrogen atmosphere at 0 °C, trimethylaluminum (226 mg, 3.143 mmol) was slowly added to a mixture of compound B01-5 (150 mg, 0.449 mmol) and Pd(PPh₃)₄ (52 mg, 0.045 mmol) in tetrahydrofuran (5 mL). The reaction mixture was refluxed at 80 °C for 1 hour. After concentration under reduced pressure, the crude product was purified by silica gel column chromatography (petroleum ether:ethyl acetate = 1:1) to afford a yellow solid, compound B01 (70 mg, 77.90%). ESI/MS (m/z): 200.9 [M+H]⁺. ¹H NMR (400 MHz, DMSO-*d*₆) δ 11.69 (s, 1H), 7.68 (d, *J =* 8.5 Hz, 1H), 7.17 (d, *J =* 8.5 Hz, 1H), 2.98 (t, *J =* 6.0 Hz, 2H), 2.64-2.57 (m, 2H), 2.55 (s, 3H), 2.16 (p, *J =* 6.1 Hz, 2H).

### Example 33: Synthesis of Compound B02

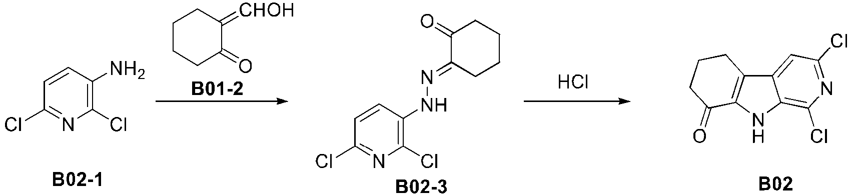

At 0 °C, sodium nitrite (423 mg, 6.134 mmol) was added to an aqueous solution (12.5 mL) of compound B02-1 (500 mg, 3.067 mmol) and concentrated hydrochloric acid (1.25 mL). The reaction mixture was stirred at 0 °C for 10 minutes, followed by the addition of a solution of B01-2 (387 mg, 3.067 mmol) and sodium acetate (554 mg, 6.747 mmol) in a mixture of methanol (12.5 mL) and water (12.5 mL). The reaction was stirred at room temperature for 30 minutes. The mixture was filtered, and the filter cake was collected and washed with water (3 × 30 mL) to afford a yellow solid, compound B02-3 (300 mg, 35.94%). ESI/MS (m/z): 272.2 [M+H]⁺.

At room temperature, concentrated hydrochloric acid (0.5 mL) was added to a methanol (5 mL) solution of compound B02-3 (300 mg, 1.102 mmol). The reaction mixture was stirred at 65 °C overnight and then cooled to room temperature. After concentration to dryness, the crude product was purified by reverse-phase preparative chromatography (mobile phase A: water containing 10 mmol/L ammonium bicarbonate; mobile phase B: acetonitrile) to afford a white solid, compound B02 (4.7 mg, 1.65%). ESI/MS (m/z): 255.0 [M+H]⁺. ¹H NMR (400 MHz, DMSO-*d*₆) δ 12.75 (s, 1H), 7.90-7.81 (m, 1H), 2.94 (td, *J =* 6.0, 2.6 Hz, 2H), 2.65 (t, *J =* 6.3 Hz, 2H), 2.17 (p, *J =* 6.1 Hz, 2H).

### Example 34: Synthesis of Compound B03

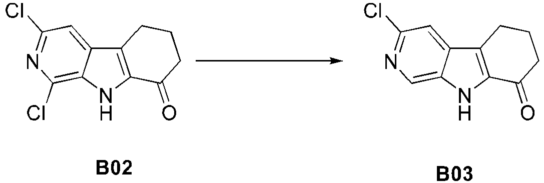

At 0 °C under a nitrogen atmosphere, ammonium formate (60 mg, 0.952 mmol) was added to a mixture of compound B02 (60 mg, 0.235 mmol) and 10% Pd/C (24 mg, 0.0226 mmol) in ethyl acetate (5 mL). The reaction mixture was stirred at room temperature under nitrogen overnight and then filtered. The filter cake was washed with ethyl acetate (2 × 10 mL). The filtrate was concentrated to dryness and purified by reverse-phase preparative chromatography (mobile phase A: water containing 10 mmol/L ammonium bicarbonate; mobile phase B: acetonitrile) to afford a pale yellow solid, compound B03 (1.5 mg, 2.86%). ESI/MS (m/z): 221.0 [M+H]⁺. ¹H NMR (400 MHz, DMSO-*d*₆) δ 12.25 (s, 1H), 8.60 (s, 1H), 7.89-7.72 (m, 1H), 2.95 (t, *J =* 6.0 Hz, 2H), 2.70-2.60 (m, 2H), 2.17 (p, *J =* 6.2 Hz, 2H).

### Example 35: Synthesis of Compound B04

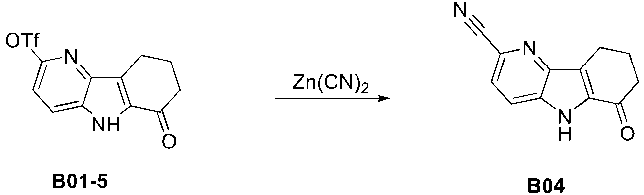

Under a nitrogen atmosphere at room temperature, zinc cyanide (176 mg, 1.496 mmol) was added to a DMF (4 mL) solution of compound B01-5 (250 mg, 0.748 mmol) (Tf: trifluoromethanesulfonyl) and Pd(PPh₃)₄ (87 mg, 0.075 mmol). The reaction mixture was stirred at 130 °C for 4 hours under nitrogen, then cooled to room temperature and quenched with water (40 mL). The mixture was extracted with ethyl acetate (3 × 15 mL). The combined organic layers were washed with brine (20 mL), dried over anhydrous sodium sulfate, filtered, and concentrated under reduced pressure. The crude product was purified by reverse-phase preparative chromatography (mobile phase A: water containing 0.1% formic acid; mobile phase B: acetonitrile) to afford a pale yellow solid, compound B04 (29.8 mg, 18.79%). ESI/MS (m/z): 212.1 [M+H]⁺. ¹H NMR (400 MHz, DMSO-*d*₆) δ 12.42 (s, 1H), 7.98 (d, *J =* 8.6 Hz, 1H), 7.84 (d, *J =* 8.6 Hz, 1H), 3.02 (t, *J =* 6.0 Hz, 2H), 2.72-2.64 (m, 2H), 2.26-2.16 (m, 2H).

### Example 36: Synthesis of Compound B05

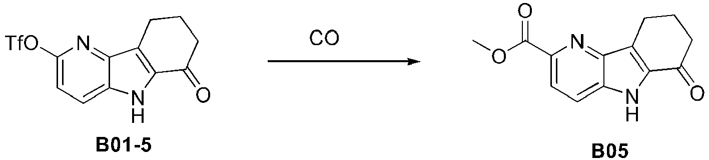

At room temperature, triethylamine (0.19 mL, 1.347 mmol) was added to a methanol (20 mL) solution of compound B01-5 (150 mg, 0.449 mmol) (Tf: trifluoromethanesulfonyl) and the catalyst Pd(dppf)Cl₂CH₂Cl₂ (73 mg, 0.090 mmol). Under a carbon monoxide atmosphere, the reaction mixture was stirred at 80 °C overnight. After concentration under reduced pressure, the crude product was purified by silica gel column chromatography (petroleum ether:ethyl acetate) to afford a pale yellow solid, compound B05 (70 mg, 63.87%). ESI/MS (m/z): 245.0 [M+H]⁺. ¹H NMR (400 MHz, DMSO-d6): δ 12.19 (s, 1H), 8.02 (d, *J =* 8.7 Hz, 1H), 7.92 (d, *J =* 8.7 Hz, 1H), 3.91 (s, 3H), 3.05 (t, *J=* 5.7 Hz, 2H), 2.66 (t, *J=* 6.2 Hz, 2H), 2.21 (p, *J =* 5.8 Hz, 2H).

### Example 37: Synthesis of Compound B06

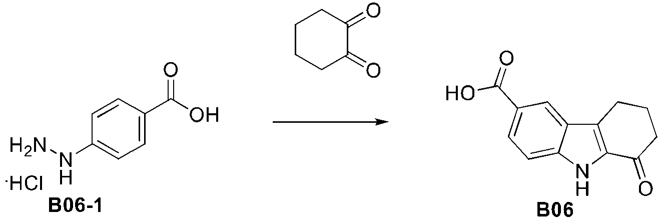

At room temperature, concentrated hydrochloric acid (0.10 mL) was added to an aqueous solution (4.00 mL) of compound B06-1 (50 mg, 0.265 mmol) and 1,2-cyclohexanedione (60 mg, 0.530 mmol). The reaction mixture was stirred at 120 °C overnight, cooled to room temperature, and filtered. The resulting solid was washed with water (3 × 20 mL). The crude product was purified by reverse-phase preparative chromatography (mobile phase A: water containing 10 mmol/L ammonium bicarbonate; mobile phase B: acetonitrile) to afford a light brown solid, compound B06 (22.4 mg, 35.83%). ESI/MS (m/z): 230.1 [M+H]⁺. ¹H NMR (400 MHz, DMSO-*d*₆) δ 11.95 (s, 1H), 8.35 (s, 1H), 7.89 (d, *J =* 8.7 Hz, 1H), 7.45 (d, *J =* 8.7 Hz, 1H), 3.01 (t, *J=* 5.8 Hz, 2H), 2.59 (t, *J =* 6.2 Hz, 2H), 2.18 (q, *J =* 5.7 Hz, 2H).

### Example 38: Synthesis of Compound B07

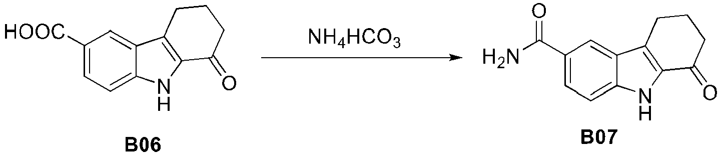

At room temperature, triethylamine (397 mg, 3.927 mmol) and 2-(7-azabenzotriazol-1-yl)-N,N,N',N'-tetramethyluronium hexafluorophosphate (746.42 mg, 1.963 mmol) were added to a DMF (10 mL) solution of compound B06 (300 mg, 1.309 mmol) and ammonium bicarbonate (310 mg, 3.927 mmol). The reaction mixture was stirred at room temperature for 2 hours, quenched with saturated ammonium chloride solution, and extracted with ethyl acetate. The combined organic layers were dried over anhydrous sodium sulfate and concentrated under reduced pressure. The crude product was purified by silica gel column chromatography (petroleum ether:ethyl acetate = 1:1) to afford a gray solid, compound B07 (150 mg, 50.22%). ESI/MS (m/z): 226.9 [M-H]⁻. ¹H NMR (400 MHz, DMSO-*d*₆): δ 11.82 (s, 1H), 8.31 (s, 1H), 7.92 (s, 1H), 7.84 (s, 1H), 7.40 (d, *J =* 8.7 Hz, 1H), 7.20 (s, 1H), 2.99 (t, *J =* 5.9 Hz, 2H), 2.64-2.55 (m, 2H), 2.19 (q, *J =* 6.0 Hz, 2H).

### Example 39: Synthesis of Compound B08

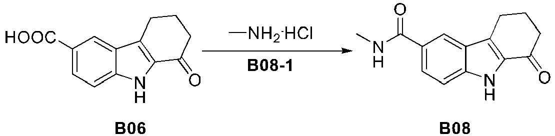

At room temperature, N,N-diisopropylethylamine (1.14 mL, 6.545 mmol) and 2-(7-azabenzotriazol-1-yl)-N,N,N',N'-tetramethyluronium hexafluorophosphate (597 mg, 1.571 mmol) were added to a DMF (3 mL) solution of compound B06 (300 mg, 1.309 mmol) and B08-1 (106 mg, 1.571 mmol). The reaction mixture was stirred at room temperature for 2 hours. After quenching with water, the mixture was extracted with ethyl acetate (3 × 10 mL). The combined organic layers were concentrated under reduced pressure, and the crude product was purified by silica gel column chromatography (dichloromethane:methanol = 10:1) to afford a brown solid, compound B08 (180 mg, 56.77%). ESI/MS (m/z): 243.0 [M+H]⁺. ¹H NMR (400 MHz, DMSO-*d*₆): δ 11.84 (s, 1H), 8.39 (d, *J =* 4.4 Hz, 1H), 8.25 (s, 1H), 7.83 (dd, *J* = 8.7, 1.4 Hz, 1H), 7.43 (d, *J =* 8.7 Hz, 1H), 2.99 (t, *J =* 5.9 Hz, 2H), 2.82 (d, *J =* 4.5 Hz, 3H), 2.66-2.55 (m, 2H), 2.18 (p, *J =* 5.9 Hz, 2H).

### Example 40: Synthesis of Compound B09

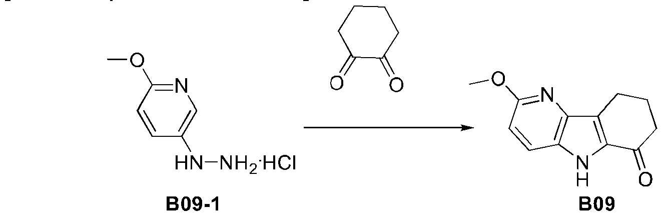

At room temperature, concentrated hydrochloric acid (2.40 mL, 78.989 mmol) was added to an aqueous solution (100 mL) of compound B09-1 (1.2 g, 6.833 mmol) and 1,2-cyclohexanedione (0.92 g, 8.200 mmol). The reaction mixture was stirred at 120 °C overnight and then concentrated under reduced pressure. The crude product was purified by silica gel column chromatography (petroleum ether:ethyl acetate = 1:1) to afford a yellow solid, compound B09 (400 mg, 27.07%). ESI/MS (m/z): 217.1 [M+H]⁺. ¹H NMR (400 MHz, DMSO-*d*₆) δ 11.46 (s, 1H), 7.73 (d, *J =* 8.9 Hz, 1H), 6.79 (d, *J =* 8.9 Hz, 1H), 3.91 (s, 3H), 2.94 (t, *J =* 6.0 Hz, 2H), 2.63-2.54 (m, 2H), 2.14 (p, *J =* 6.2 Hz, 2H).

### Example 41: Synthesis of Compound B12 and B13

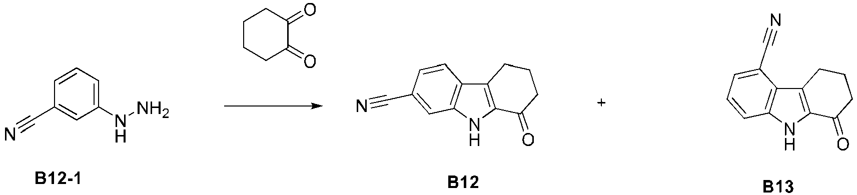

At room temperature, concentrated hydrochloric acid (2.8 mL) was added dropwise to an aqueous solution (130 mL) of compound B12-1 (1 g, 3.979 mmol) and 1,2-cyclohexanedione (0.89 g, 7.958 mmol). The reaction mixture was stirred at 120 °C overnight. After cooling to room temperature, the mixture was filtered, and the solid was collected and washed with water (2 × 100 mL). The resulting solid was purified by reverse-phase preparative chromatography (mobile phase A: water containing 0.1% formic acid; mobile phase B: acetonitrile) to afford a mixture of pale yellow solids, compounds B12 and B13 (97.1 mg, 11.57%). ESI/MS (m/z): 211.2 [M+H]⁺.

### Example 42: Synthesis of Compound B14

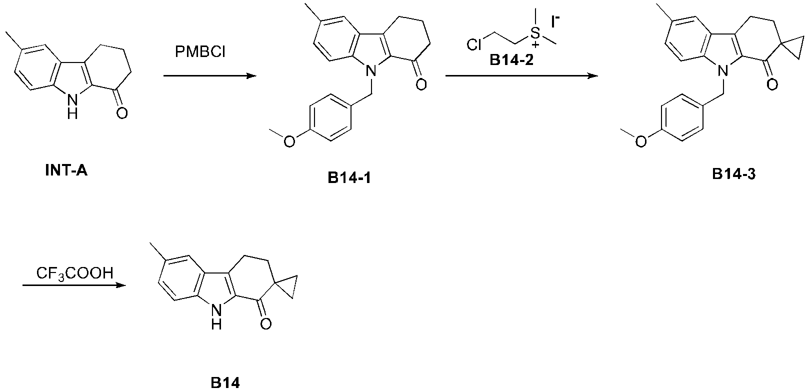

At 0 °C, p-methoxybenzyl chloride (PMBCl, 472 mg, 3.011 mmol) was added to a DMF (8 mL) solution of compound INT-A (500 mg, 2.509 mmol) and sodium hydride (66 mg, 2.760 mmol). The reaction mixture was stirred at room temperature for 4 hours, and the pH was adjusted to 7 using 2 M dilute hydrochloric acid. The mixture was extracted with ethyl acetate (3 × 50 mL), washed with brine (100 mL), dried over anhydrous sodium sulfate, and filtered. The filtrate was concentrated under reduced pressure, and the crude product was purified by silica gel column chromatography to afford a yellow oily compound B14-1 (650 mg, 81.10%). ESI/MS (m/z): 320.1 [M+H]⁺.

At room temperature, compound B14-2 (295 mg, 1.17 mmol) was added to a DMF (25 mL) solution of sodium hydride (188 mg, 7.825 mmol) and compound B14-1 (500 mg, 1.565 mmol). The reaction mixture was stirred at room temperature for 3 hours, then quenched with water (10 mL). The mixture was extracted with ethyl acetate (40 mL), washed with brine (20 mL), dried over anhydrous sodium sulfate, and concentrated under reduced pressure. The crude product was purified by silica gel column chromatography (petroleum ether:ethyl acetate = 6:1) to afford a brown solid, compound B14-3 (44 mg, 8.14%). ESI/MS (m/z): 346.2 [M+H]⁺.

At room temperature, trifluoroacetic acid (3 mL) was added to a dichloromethane (3 mL) solution of compound B14-3 (44 mg, 0.127 mmol). The reaction mixture was stirred at room temperature for 3 hours and then concentrated. The residue was purified by reverse-phase preparative chromatography (mobile phase A: water containing 10 mmol/L ammonium bicarbonate; mobile phase B: acetonitrile) to afford a white solid, compound B14 (3.9 mg, 13.59%). ESI/MS (m/z): 226.2 [M+H]⁺. ¹H NMR (400 MHz, DMSO-*d*₆) δ 11.43 (s, 1H), 7.46 - 7.43 (m, 1H), 7.29 (dd, *J=* 8.4, 0.7 Hz, 1H), 7.13 (dd, *J =* 8.5, 1.6 Hz, 1H), 2.95 (t, *J =* 6.2 Hz, 2H), 2.38 (d, *J* = 0.8 Hz, 3H), 2.04 (t, *J =* 6.2 Hz, 2H), 1.12 (q, *J =* 3.5 Hz, 2H), 0.83 (q, *J =* 3.5 Hz, 2H).

### Example 43: Synthesis of Compound B15

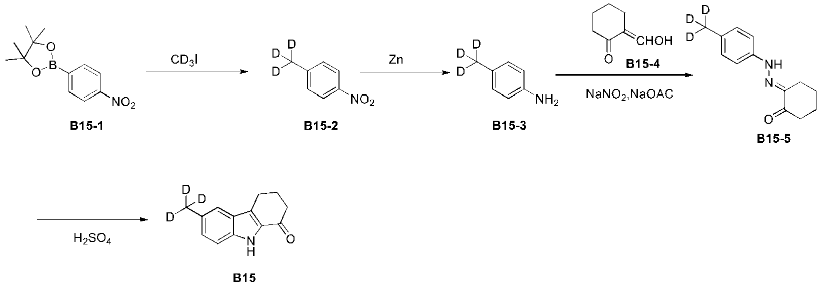

Under a nitrogen atmosphere at room temperature, deuterated iodomethane (6.98 g, 48.180 mmol) and the catalyst Pd(AMPHOS)₂Cl₂ (1.14 g, 1.606 mmol) were added to a pivalic alcohol (60 mL) solution of compound B15-1 (4 g, 16.060 mmol) and potassium tert-butoxide (3.60 g, 32.120 mmol). The reaction mixture was stirred at 60 °C under nitrogen overnight. After cooling to room temperature, the reaction was quenched with water (50 mL). The mixture was extracted with ethyl acetate (3 × 100 mL), and the combined organic layers were washed with brine (100 mL), dried over anhydrous sodium sulfate, filtered, and concentrated under reduced pressure. The crude product was purified by silica gel column chromatography (petroleum ether:ethyl acetate = 5:1) to afford a yellow solid, compound B15-2 (1.6 g, 71.08%).

At 0 °C, zinc powder (5.97 g, 91.328 mmol) was added to an ethanol (30 mL) solution of compound B15-2 (1.6 g, 11.416 mmol) and glacial acetic acid (6.54 mL, 114.160 mmol). The reaction mixture was stirred at room temperature for 1 hour, then quenched with water. The mixture was extracted with ethyl acetate (3 × 100 mL), and the combined organic layers were washed with brine, dried over anhydrous sodium sulfate, filtered, and concentrated to afford a yellow oily compound B15-3 (1.3 g, 82.69%). ESI-MS (m/z): 111.1 [M+H]⁺.

At 0 °C, concentrated hydrochloric acid (3.05 mL) was added to an aqueous solution (45.5 mL) of compound B15-3 (1.3 g, 11.800 mmol). Then, an aqueous solution (10 mL) of sodium nitrite (1.63 g, 23.600 mmol) was added to the above mixture. Finally, a solution of B15-4 (1.49 g, 11.800 mmol) and sodium acetate (2.13 g, 25.960 mmol) in a mixture of water (45.5 mL) and methanol (45.5 mL) was added. The reaction mixture was stirred at room temperature for 1 hour. The mixture was filtered, and the solid was washed with water (50 mL), collected, and concentrated to dryness to afford a red solid, compound B15-5 (2.1 g, 81.15%). ESI/MS (m/z): 220.1 [M+H]⁺.

At room temperature, concentrated sulfuric acid (4 mL) was added to a methanol (40 mL) solution of compound B15-5 (2 g, 9.120 mmol). The reaction mixture was stirred at 60 °C for 1 hour and then cooled to room temperature. The pH was adjusted to 7 using a saturated aqueous sodium bicarbonate solution. The mixture was extracted with ethyl acetate (3 × 150 mL), and the combined organic layers were washed with brine (200 mL), dried over anhydrous sodium sulfate, filtered, and concentrated under reduced pressure. The crude product was purified by reverse-phase preparative chromatography (mobile phase A: water containing 10 mmol/L ammonium bicarbonate; mobile phase B: acetonitrile) to afford a pink solid, compound B15 (369.2 mg, 20.01%). ESI/MS (m/z): 203.2 [M+H]⁺. ¹H NMR (400 MHz, DMSO-*d*₆) δ 11.44 (s, 1H), 7.43 (d, *J* = 1.7 Hz, 1H), 7.29 (dd, *J =* 8.4, 0.7 Hz, 1H), 7.13 (dd, *J* = 8.4, 1.7 Hz, 1H), 2.91 (t, *J =* 6.0 Hz, 2H), 2.54 (m, 2H), 2.18-2.10 (m, 2H).

### Example 44: Using a method similar to that described in Example 1, the following compounds were obtained.

| compound | **Structural formula** | **Analyze data** |
|---|---|---|
| **E31** | 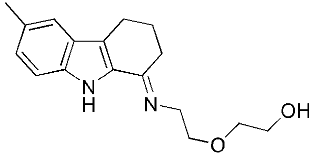 | ESI/MS(m/z): 287.2 [M+H]⁺.¹H NMR (400 MHz, DMSO-*d*₆) δ 10.81 (s, 1H), 7.27 (d, *J* = 1.7 Hz 1H), 7.25 (d, *J =* 8.3 Hz, 1H), 6.95 (dd, *J* = 8.3, 1.7 Hz, 1H), 4.61 (s, 1H), 3.74-3.68 (m, 2H), 3.63 (t, *J =* 6.0 Hz, 2H), 3.53-3.45 (m, 4H), 2.76 (t, *J =* 6.0 Hz, 2H), 2.57 (t, *J =* 6.3 Hz, 2H), 2.35 (s, 3H), 1.97 (p, *J =* 6.2 Hz, 2H). |
| **E32** | 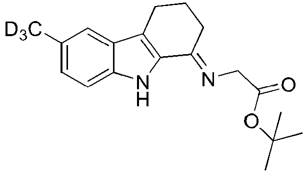 | ESI/MS(m/z): 316.1 [M+H]⁺.¹H NMR (500 MHz, DMSO-*d*₆) δ 11.00 (s, 1H), 7.36 (s, 1H), 7.31 (d, *J* = 8.3 Hz, 1H), 7.04 (dd, *J* = 8.3, 1.6 Hz, 1H), 4.34 (s, 2H), 2.84 (t, *J =* 6.0 Hz, 2H), 2.60-2.58 (m, 2H), 2.07-1.98 (m, 2H), 1.50 (s, 9H). |
| **E33** | 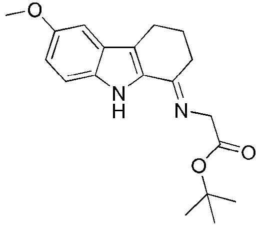 | ESI/MS(m/z): 329.1 [M+H]⁺.¹H NMR (400 MHz, DMSO-*d*₆) δ 10.93 (s, 1H), 7.25 (d, *J* = 8.8 Hz, 1H), 6.99 (d, *J=* 2.2 Hz, 1H), 6.80 (dd, *J =* 8.8, 2.4 Hz, 1H), 4.27 (s, 2H), 3.76 (s, 3H), 2.78 (*t, J =* 5.9 Hz, 2H), 2.53 (t, *J =* 5.9 Hz, 2H), 1.97 (p, *J =* 5.9 Hz, 2H), 1.44 (s, 9H). |
| **E34** | 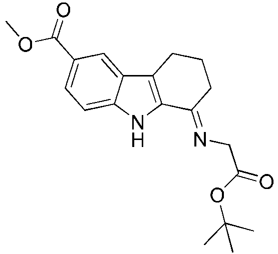 | ESI/MS(m/z): 357.2 [M+H]⁺.¹H NMR (400 MHz, DMSO-*d*₆) δ 11.56 (s, 1H), 8.28-8.21 (m, 1H), 7.79 (m, *J* = 8.7, 1.6 Hz, 1H), 7.44 (d, *J* = 8.6 Hz, 1H), 4.31 (s, 2H), 3.85 (s, 3H), 2.87 (t, *J =* 6.0 Hz, 2H), 2.56 (t, *J* = 6.0 Hz, 2H), 2.04-1.94 (m, 2H), 1.45 (s, 9H). |
| **E35** | 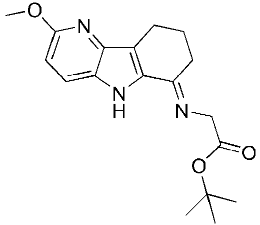 | ESI/MS(m/z): 330.2 [M+H]⁺.¹H NMR (400 MHz, DMSO-*d*₆) δ 11.18 (s, 1H), 7.64 (d, *J* = 8.7 Hz, 1H), 6.62 (d, *J* = 8.7 Hz, 1H), 4.29 (s, 2H), 3.87 (s, 3H), 2.81 (t, *J=* 6.0 Hz, 2H), 2.57-2.52 (m, 2H), 1.96 (p, *J =* 6.4 Hz, 2H), 1.45 (s, 9H). |
| **E36** | 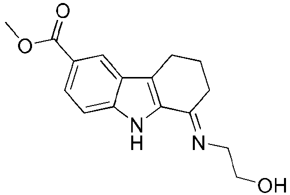 | ESI/MS(m/z): 287.1 [M+H]⁺.¹H NMR (400 MHz, DMSO-*d*₆) δ 11.43 (s, 1H), 8.22 (s, 1H), 7.77 (d, *J* = 8.6 Hz, 1H), 7.43 (d, *J* = 8.6 Hz, 1H), 4.57 (s, 1H), 3.84 (s, 3H), 3.70 (t, *J=* 5.8 Hz, 2H), 3.58 (t, *J =* 6.1 Hz, 2H), 2.85 (t, *J =* 5.4 Hz, 2H), 2.61 (t, *J =* 5.7 Hz, 2H), 2.06-1.93 (m, 2H). |
| **E37** | 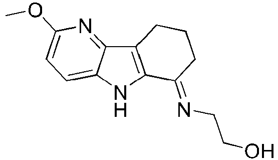 | ESI/MS (m/z):260.1[M+H]⁺.¹H NMR (400 MHz, DMSO-*d*₆) δ 11.07 (s, 1H), 7.63 (d, *J* = 8.7 Hz, 1H), 6.60 (d, *J* = 8.7 Hz, 1H), 4.55 (s, 1H), 3.86 (s, 3H), 3.70 (t, *J =* 6.2 Hz, 2H), 3.57 (t, *J =* 6.2 Hz, 2H), 2.80 (t, *J =* 5.8 Hz, 2H), 2.59 (*t, J =* 6.0 Hz, 2H), 2.02-1.88 (m, 2H). |
| **E38** | 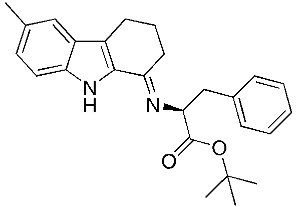 | ESI/MS(m/z): 403.3 [M+H]⁺.¹H NMR (400 MHz, DMSO-*d*₆) δ 10.84 (s, 1H), 7.31-7.26 (m, 4H), 7.26-7.21 (m, 2H), 7.19-7.14 (m, 1H), 6.99 (dd, *J=* 8.4, 1.7 Hz, 1H), 4.54 (dd, *J =* 7.9, 6.1 Hz, 1H), 3.24 (dd, *J* = 13.4, 6.0 Hz, 1H), 3.00 (dd, *J* = 13.4, 7.9 Hz, 1H), 2.75-2.67 (m, 2H), 2.49-2.42 (m, 1H), 2.35 (s, 3H), 2.32-2.23 (m, 1H), 1.92-1.71 (m, 2H), 1.34 (s, 9H). |
| **E39** | 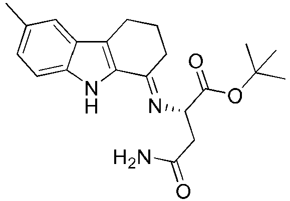 | ESI/MS(m/z): 370.1 [M+H]⁺.¹H NMR (400 MHz, DMSO-*d*₆) δ 10.80 (s, 1H), 7.33 (s, 1H), 7.29 (s, 1H), 7.27 (d, *J =* 8.3 Hz, 1H), 7.01-6.95 (m, 1H), 6.80 (s, 1H), 4.70 (t, *J =* 6.7 Hz, 1H), 2.79-2.75 (m, 2H), 2.67-2.59 (m, 2H), 2.51-2.47 (m, 2H), 2.36 (s, 3H), 1.98 (p, *J =* 6.0 Hz, 2H), 1.38 (s, 9H). |
| **E40** | 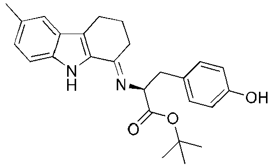 | ESI/MS (m/z): 419.2 [M+H]⁺.¹H NMR (400 MHz, DMSO-*d*₆) δ 10.83 (s, 1H), 9.14 (s, 1H), 7.30 (d, *J =* 8.8 Hz, 1H), 7.27 (s, 1H), 7.04 (d, *J* = 7.2 Hz, 2H), 6.99 (d, *J =* 7.5 Hz, 1H), 6.62 (d, *J =* 7.2 Hz, 2H), 4.44 (s, 1H), 3.14-3.10 (m, 1H), 2.93-2.83 (m, 1H), 2.72 (s, 2H), 2.47-2.21 (m, 2H), 2.36 (s, 3H), 1.82 -1.78 (m, 2H), 1.34 (s, 9H). |
| **E41** | 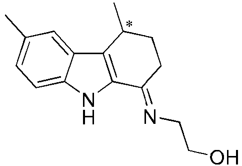 | ESI/MS(m/z): 257.2 [M+H]⁺.¹H NMR (300 MHz, DMSO-*d*₆) δ 10.79 (s, 1H), 7.37 (d, *J =* 1.5 Hz, 1H), 7.25 (d, *J =* 8.3 Hz, 1H), 6.94 (dd, *J* = 8.3, 1.6 Hz, 1H), 4.52 (s, 1H), 3.69 (q, *J =* 5.7, 5.3 Hz, 2H), 3.57 (s, 1H), 3.53 (d, *J =* 2.1 Hz, 1H), 3.17 (h, *J* = 6.5 Hz, 1H), 2.62-2.55 (m, 2H), 2.07-2.01 (m, 1H), 1.81-1.68 (m, 1H), 1.34 (d, *J =* 6.9 Hz, 3H). |

### Example 45: Synthesis of Compound B16

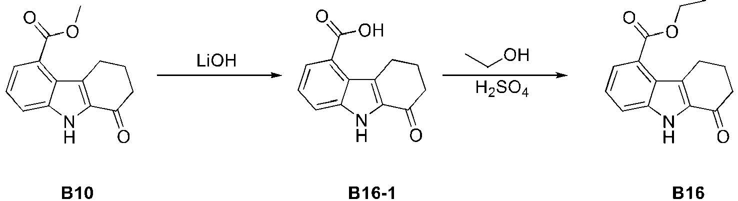

Compound B10 (1.18 g, 4.754 mmol) was dissolved in a mixture of methanol (40 mL) and water (8 mL). Lithium hydroxide (1 g, 3.580 mmol) was added at room temperature. The reaction mixture was stirred at 60 °C for 1.5 hours, cooled to room temperature, and adjusted to pH 7 using 1 M hydrochloric acid. The solution was concentrated to dryness and purified by reverse-phase preparative chromatography (mobile phase A: water; mobile phase B: acetonitrile) to afford a white solid, compound B16-1 (950 mg, 86.22%). ESI-MS (m/z): 230.1 [M+H]⁺.

Compound B16-1 (100 mg, 0.431 mmol) was dissolved in ethanol (2 mL), and concentrated sulfuric acid (100 µL) was added dropwise at room temperature. The reaction mixture was stirred at 70 °C for 24 hours, cooled to room temperature, and quenched with water. The pH was adjusted to 6 using a saturated sodium bicarbonate solution. The mixture was extracted with ethyl acetate (3 × 30 mL). The combined organic layers were washed with brine, dried over anhydrous sodium sulfate, filtered, and concentrated under reduced pressure. The crude product was purified by reverse-phase preparative chromatography (mobile phase A: water; mobile phase B: acetonitrile) to afford a white solid, compound B16 (69.1 mg, 61.50%). ESI-MS (m/z): 258.2 [M+H]⁺. ¹H NMR (400 MHz, DMSO-*d*₆) δ 12.00 (s, 1H), 7.65 (dd, *J=* 8.3, 1.0 Hz, 1H), 7.59 (dd, *J =* 7.3, 1.0 Hz, 1H), 7.37 (dd, *J* = 8.3, 7.3 Hz, 1H), 4.37 (q, *J* = 7.1 Hz, 2H), 3.09 (t, *J =* 6.0 Hz, 2H), 2.57 (t, *J =* 5.9 Hz, 2H), 2.14-2.07 (m, 2H), 1.36 (t, *J =* 7.1 Hz, 3H).

### Example 46: Synthesis of Compound B17

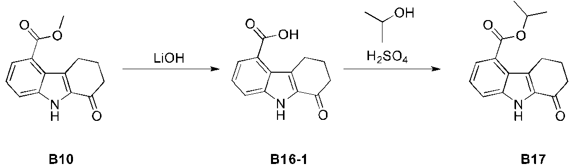

Compound B10 (1.18 g, 4.754 mmol) was dissolved in a mixture of methanol (40 mL) and water (8 mL). Lithium hydroxide (1 g, 3.580 mmol) was added at room temperature. The reaction mixture was stirred at 60 °C for 1.5 hours, cooled to room temperature, and adjusted to pH 7 using 1 M hydrochloric acid. The solution was concentrated to dryness and purified by reverse-phase preparative chromatography (mobile phase A: water; mobile phase B: acetonitrile) to afford a white solid, compound B16-1 (950 mg, 86.22%). ESI-MS (m/z): 230.1 [M+H]⁺.

Compound B16-1 (100 mg, 0.431 mmol) was dissolved in isopropanol (3 mL), and concentrated sulfuric acid (300 µL) was added dropwise at room temperature. The reaction mixture was stirred at 85 °C for 24 hours, cooled to room temperature, and quenched with water. The pH was adjusted to 6 using a saturated sodium bicarbonate solution. The mixture was extracted with ethyl acetate (3 × 30 mL). The combined organic layers were washed with brine, dried over anhydrous sodium sulfate, filtered, and concentrated under reduced pressure. The crude product was purified by reverse-phase preparative chromatography (mobile phase A: water; mobile phase B: acetonitrile) to afford a white solid, compound B17 (32.7 mg, 27.66%). ESI-MS (m/z): 272.0 [M+H]⁺. ¹H NMR (400 MHz, DMSO-*d*₆) *δ* 11.99 (s, 1H), 7.65 (dd, *J =* 8.2*,* 1.1 Hz, 1H), 7.56 (dd, *J =* 7.4, 1.1 Hz, 1H), 7.37 (dd, *J=* 8.3, 7.3 Hz, 1H), 5.22-5.16 (m , 1H), 3.09 (t, *J =* 6.0 Hz, 2H), 2.57 (t, *J =* 7.4 Hz, 2H), 2.14-2.07 (m, 2H), 1.37 (d, *J =* 6.3 Hz, 6H).

### Example47: Synthesis of Compound E42

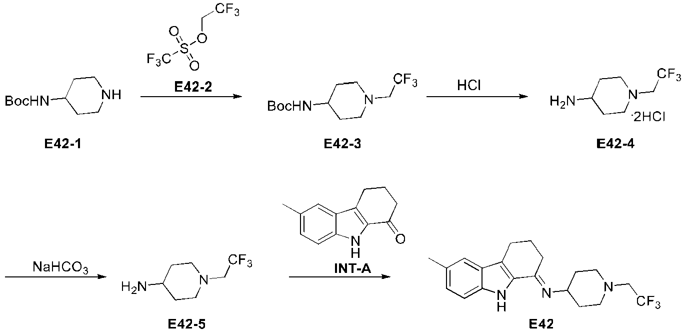

At room temperature, triethylamine (1.52 g, 14.978 mmol) was slowly added dropwise to a tetrahydrofuran (20 mL) solution of compound E42-1 (1.5 g, 7.489 mmol) and compound E42-2 (2.61 g, 11.233 mmol). The reaction mixture was stirred at 70 °C for 4 hours. After cooled to room temperature, the reaction was quenched with water (20 mL) and extracted with ethyl acetate (3 × 20 mL). The combined organic layers were washed with brine (3 × 10 mL), dried over anhydrous sodium sulfate, and concentrated under reduced pressure. The residue was purified by silica gel column chromatography (petroleum ether:ethyl acetate = 4:1) to afford a white solid, compound E42-3 (1.888 g, 89.30%). ESI/MS (m/z): 238.2 [M+H]⁺.

At room temperature, a 4 M hydrogen chloride solution in 1,4-dioxane (5 mL, 20.000 mmol) was slowly added dropwise to a 1,4-dioxane (5 mL) solution of compound E42-3 (600 mg, 2.125 mmol). The mixture was stirred at room temperature for 2 hours. After the reaction, n-hexane (15 mL) was added, and the mixture was filtered. The filter cake was washed with n-hexane (3 × 5 mL). The resulting solid was dried under vacuum to afford a white solid, compound E42-4 (470 mg, crude). ESI/MS (m/z): 183.2 [M+H]⁺.

At room temperature, sodium bicarbonate (323 mg, 3.842 mmol) was added to an aqueous solution (6 mL) of compound E42-4 (420 mg, 1.921 mmol), and the mixture was stirred for 30 minutes. After the reaction, the mixture was concentrated under reduced pressure. The residue was purified by reverse-phase column chromatography (mobile phase A: 10 mmol/L ammonium bicarbonate; mobile phase B: acetonitrile) to afford compound E42-5 (170 mg, 48.58%). ESI/MS (m/z): 183.2 [M+H]⁺.

At room temperature, tetraethyl titanate (206.42 mg, 0.904 mmol) was slowly added dropwise to a toluene (4 mL) solution of compound E42-5 (130.16 mg, 0.678 mmol) and compound INT-A (95 mg, 0.452 mmol). After the addition, the mixture was stirred at 100 °C for 6 hours. The reaction mixture was cooled to room temperature, quenched with water (20 mL), and diluted with dichloromethane (20 mL). The mixture was filtered, and the filter cake was washed with dichloromethane (5 mL). The filtrate was extracted with dichloromethane (20 mL). The organic phase was washed with brine (10 mL), dried over anhydrous sodium sulfate, and concentrated under reduced pressure. The resulting residue was triturated with ethanol (10 mL), filtered, and the filter cake was dried under vacuum to afford an off-white solid, compound E42 (25 mg, 14.76%). ESI/MS (m/z): 364.1 [M+H]⁺.

### Example 48: Synthesis of Compound E43

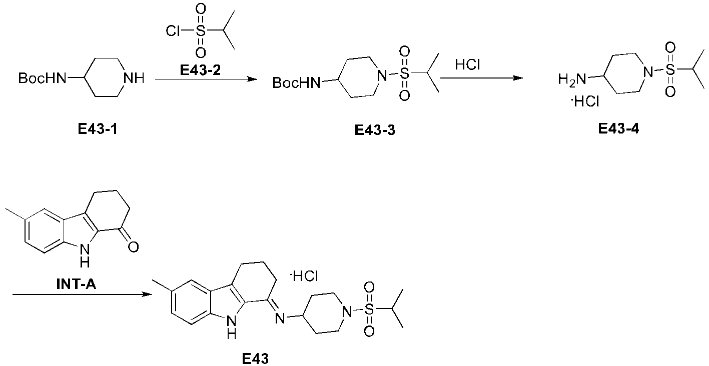

Under a nitrogen atmosphere at 0 °C, N,N-diisopropylethylamine (522 µL, 2.996 mmol) and compound E43-2 (336 µL, 2.996 mmol) were added to a dichloromethane (10 mL) solution of compound E43-1 (600 mg, 2.996 mmol). The mixture was stirred at room temperature overnight. After the reaction, the mixture was diluted with dichloromethane (60 mL) and sequentially washed with 0.1 M hydrochloric acid (30 mL), saturated sodium carbonate solution (30 mL), and brine (30 mL). The organic layer was dried over anhydrous sodium sulfate and concentrated under reduced pressure to afford a white solid, compound E43-3 (560 mg, 61.00%). ESI/MS (m/z): 251.1 [M+H-56]⁺.

At 0 °C, diethyl ether (10 mL) and concentrated hydrochloric acid (1.6 mL) were added to a methanol (1.5 mL) solution of compound E43-3 (550 mg, 1.795 mmol). The mixture was stirred at room temperature overnight, filtered, and the filter cake was washed with diethyl ether (5 mL). After drying, a white solid, compound E43-4 (260 mg, 70.21%), was obtained. ESI/MS (m/z): 207.0 [M+H]⁺.

Under a nitrogen atmosphere at room temperature, tetraethyl titanate (106 mg, 0.464 mmol) was added dropwise to a toluene (5 mL) solution of compound E43-4 (100 mg, 0.309 mmol) and compound INT-A (65 mg, 0.309 mmol). The reaction mixture was stirred at 100 °C for 4 hours, cooled to room temperature, and concentrated under reduced pressure. The residue was quenched with water at room temperature and filtered. The filter cake was washed with DMF (2 × 10 mL). The filtrate was concentrated under reduced pressure, and the residue was triturated with ethanol (4 × 4 mL) to afford a yellow solid, compound E43 (60.3 mg, 44.84%). ESI/MS (m/z): 388.0 [M+H]⁺. ¹H NMR (400 MHz, DMSO-*d*₆) δ 12.70 (s, 1H), 12.43 (d, *J =* 8.4 Hz, 1H), 7.54 (s, 1H), 7.40 (d, *J =* 8.5 Hz, 1H), 7.31 (dd, *J =* 8.6, 1.6 Hz, 1H), 4.39-4.25 (m, 1H), 3.82-3.73 (m, 2H), 3.42-3.34 (m, 1H), 3.14 (t, *J =* 6.3 Hz, 2H), 3.10-3.01 (m, 2H), 2.99 (t, *J =* 6.2 Hz, 2H), 2.39 (s, 3H), 2.18-2.10 (m, 2H), 2.09-2.02 (m, 2H), 1.94-1.82 (m, 2H), 1.25 (d, *J =* 6.8 Hz, 6H).

### Example 49: Synthesis of Compound E44

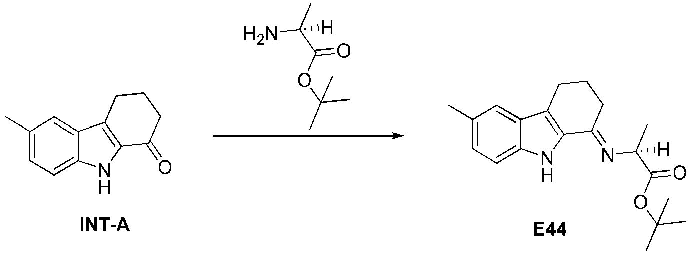

Under a nitrogen atmosphere, a toluene solution (4 mL) of compound INT-A (199.3 mg, 1.000 mmol) and (S)-tert-butyl 2-aminopropanoate (435.6 mg, 3.000 mmol) was stirred at 130 °C for 4 hours. After cooling to room temperature, the mixture was filtered. The filtrate was concentrated under reduced pressure, and the residue was sequentially washed with acetonitrile (2 mL) and a mixture of ethyl acetate and n-hexane (V:V = 1:4, 3 mL) to afford a pink solid, E44 (78.8 mg, 24.14%). ESI/MS (m/z): 327.2 [M+H]⁺. ¹H NMR (500 MHz, DMSO-*d*₆) δ 10.82 (s, 1H), 7.29 (s, 1H), 7.27 (d, *J =* 8.3 Hz, 1H), 6.97 (dd, *J =* 8.3, 1.0 Hz, 1H), 4.42 (q, *J =* 6.6 Hz, 1H), 2.78 *(t, J =* 6.0 Hz, 2H), 2.63-2.53 (m, 2H), 2.36 (s, 3H), 2.00-1.94 (m, 2H), 1.40 (s, 9H), 1.37 (d, *J =* 6.6 Hz, 3H).

### Example 50: Synthesis of Compound E45

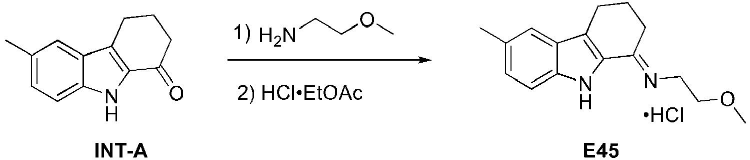

A toluene solution (4 mL) of compound INT-A (199.3 mg, 1.000 mmol) and 2-methoxyethan-1-amine (450.7 mg, 6 mmol) was stirred at 130 °C for 6 hours and then cooled to room temperature. The reaction mixture was concentrated under reduced pressure, and 4 M hydrogen chloride in ethyl acetate (3 mL) was added to the residue. The mixture was filtered and dried to afford a brown solid, compound E45 (115.0 mg, 39.27%). ESI/MS (m/z): 257.1 [M+H]⁺. ¹H NMR (500 MHz, DMSO-*d*₆) δ 12.47 (s, 1H), 12.38 (s, 1H), 7.54 (s, 1H), 7.42 (d, *J =* 8.5 Hz, 1H), 7.32 (dd, *J =* 8.6, 1.3 Hz, 1H), 3.95-3.91 (m, 2H), 3.73 (t, *J =* 5.1 Hz, 2H), 3.32 (s, 3H), 3.07 (t, *J =* 6.2 Hz, 2H), 2.99 (t, *J =* 6.2 Hz, 2H), 2.40 (s, 3H), 2.13-2.08 (m, 2H).

### Example 51: Synthesis of Compound E46

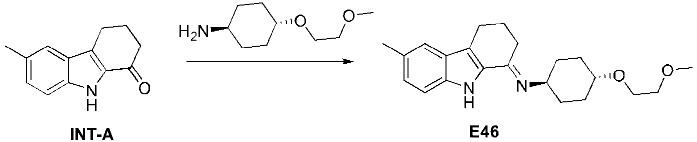

A xylene solution (4 mL) of compound INT-A (199.3 mg, 1.000 mmol) and trans-4-(2-methoxyethoxy)cyclohexanamine (519.8 mg, 3 mmol) was stirred at 150 °C for 5 hours and then cooled to room temperature. The reaction mixture was concentrated under reduced pressure, and n-hexane (10 mL) was added to the residue. The precipitated solid was washed with a small amount of a mixed solvent (ethyl acetate:n-hexane = 1:5) to afford a pale yellow solid, E46 (179.0 mg, 50.50%, trans, relative configuration). ESI/MS (m/z): 355.2 [M+H]⁺. ¹H NMR (500 MHz, DMSO-*d₆*) δ 10.65 (s, 1H), 7.25 (d, *J =* 8.5 Hz, 2H), 6.94 (dd, *J =* 8.3, 1.4 Hz, 1H), 3.60-3.54 (m, 3H), 3.44-3.42 (m, 2H), 3.30-3.28 (m, 1H), 3.25 (s, 3H), 2.75 (t, *J =* 6.0 Hz, 2H), 2.61-2.57 (m, 2H), 2.35 (s, 3H), 2.06-2.02 (m, 2H), 1.99-1.94 (m, 2H), 1.73-1.67 (m, 2H), 1.50-1.42 (m, 2H), 1.34-1.26 (m, 2H).

### Example 52: Synthesis of Compound E47

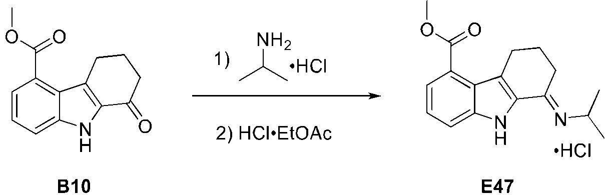

A mixture of compound B10 (243.3 mg, 0.500 mmol), isopropylamine hydrochloride (191.1 mg, 2.000 mmol), and sodium acetate (164.1 mg, 2.000 mmol) in 2-methyltetrahydrofuran (5 mL) was stirred at 95 °C for 3 hours. After cooling to room temperature, the reaction mixture was diluted with ethyl acetate (100 mL). The organic phase was sequentially washed with water (30 mL), dried over anhydrous sodium sulfate, and concentrated under reduced pressure. To the residue, 4 M hydrogen chloride in ethyl acetate (3 mL) was added. The mixture was filtered, and the solid was washed with a small amount of a mixed solvent (ethyl acetate:n-hexane = 1:1) to afford a light brown solid, compound E47 (60.3 mg, 37.59%). ESI/MS (m/z): 285.1 [M+H]⁺. ¹H NMR (500 MHz, DMSO-*d*₆) δ 13.29 (s, 1H), 12.57 (s, 1H), 7.75 (dd, *J* = 8.3, 0.7 Hz, 1H), 7.67-7.64 (m, 1H), 7.55-7.51 (m, 1H), 4.48-4.40 (m, 1H), 3.91 (s, 3H), 3.14-3.08 (m, 4H), 2.11-2.05 (m, 2H), 1.44 (s, 3H), 1.43 (s, 3H).

### Example 53: Synthesis of Compound E48

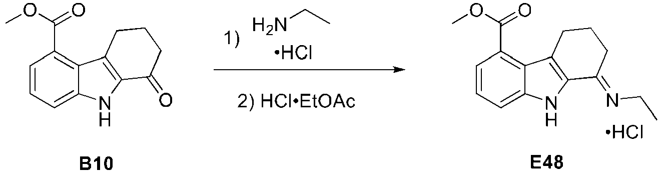

A solution of compound B10 (73.0 mg, 0.300 mmol), ethylamine hydrochloride (98.0 mg, 1.200 mmol), and sodium acetate (98.4 mg, 1.200 mmol) in 2-methyltetrahydrofuran (4 mL) was stirred at 90 °C for 3 hours and then cooled to room temperature. The reaction mixture was diluted with ethyl acetate (100 mL), washed with pure water (20 mL), dried over anhydrous sodium sulfate, filtered, and concentrated under reduced pressure. To the crude product, 4 M hydrogen chloride in ethyl acetate (1 mL) was added. The precipitated solid was collected by filtration and dried to afford a pale-yellow solid, compound E48 (40.8 mg, 44.33%). ESI/MS (m/z): 271.1 [M+H]⁺. ¹H NMR (500 MHz, DMSO-*d*₆) δ 13.11 (s, 1H), 12.64 (s, 1H), 7.76 (dd, *J =* 8.3, 0.8 Hz, 1H), 7.66 (dd, *J =* 7.2*,* 0.8 Hz, 1H), 7.53 (dd, *J =* 8.3, 7.2 Hz, 1H), 3.91 (s, 3H), 3.81 (q, *J =* 7.1 Hz, 2H), 3.12-3.06 (m, 4H), 2.10-2.04 (m, 2H), 1.40 (t, *J =* 7.3 Hz, 3H).

### Example 54: Synthesis of Compound E49

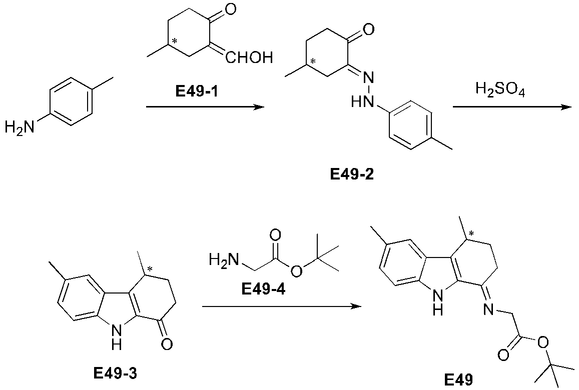

At 0 °C, an aqueous solution (25 mL) of sodium nitrite (1.20 g, 17.406 mmol), a methanol solution (25 mL) of compound E49-1 (1.22 g, 8.703 mmol), and an aqueous solution (25 mL) of sodium acetate (1.57 g, 19.147 mmol) were sequentially added to a mixture of 4-methylaniline (0.93 g, 8.703 mmol) in concentrated hydrochloric acid (5 mL) and water (25 mL). The reaction mixture was stirred at 0 °C for 1 hour and then filtered. The filter cake was washed with water (3 × 50 mL) to afford a yellow solid, compound E49-2 (500 mg, 24.95%). ESI/MS (m/z): 231.1 [M+H]⁺.

A mixture of compound E49-2 (50 mg, 0.217 mmol) and concentrated sulfuric acid (0.2 mL) in methanol (2 mL) was stirred at 65 °C for 4 hours and then cooled to room temperature. The pH was adjusted to 8 with an aqueous potassium hydroxide solution, and the mixture was extracted with ethyl acetate (3 × 10 mL). The combined organic layers were washed with brine (5 mL), dried over anhydrous sodium sulfate, and concentrated under reduced pressure. The residue was purified by reverse-phase preparative chromatography (mobile phase A: water containing 10 mmol/L ammonium bicarbonate; mobile phase B: acetonitrile) to afford a white solid, compound E49-3 (10.3 mg, 22.24%). ESI/MS (m/z): 214.0 [M+H]⁺. ¹H NMR (400 MHz, Methanol-*d*₄) δ 7.51 (s, 1H), 7.30 (d, *J =* 8.5 Hz, 1H), 7.15 (d, *J* = 8.5 Hz, 1H), 3.37 (dt, *J =* 12.5, 6.3 Hz, 1H), 2.73 (ddd, *J =* 14.4, 9.6, 4.4 Hz, 1H), 2.50 (ddd, *J =* 17.2, 6.4, 4.2 Hz, 1H), 2.41 (s, 3H), 2.35 (ddt, *J =* 14.0, 9.5, 4.7 Hz, 1H), 1.98 (dq, *J =* 11.6, 6.1, 5.4 Hz, 1H), 1.48 (d, *J* = 7.0 Hz, 3H).

At room temperature, compound E49-4 (464.97 mg, 3.544 mmol) was added to a toluene (4 mL) solution of compound E49-3 (200 mg, 0.886 mmol). The reaction mixture was stirred at 130 °C under nitrogen for 5 hours. After cooling to room temperature, the mixture was concentrated under reduced pressure. The residue was purified by reverse-phase preparative chromatography (mobile phase A: water; mobile phase B: acetonitrile) to afford a yellow solid, compound E49 (34.4 mg, 10.80%). ESI/MS (m/z): 327.1 [M+H]⁺. ¹H NMR (400 MHz, DMSO-*d*₆) δ 10.93 (s, 1H), 7.39 (s, 1H), 7.26 (d, *J =* 8.3 Hz, 1H), 6.97 (dd, *J =* 8.4, 1.6 Hz, 1H), 4.27 (s, 2H), 3.19 (h, *J* = 6.6 Hz, 1H), 2.59-2.46 (m, 2H), 2.36 (s, 3H), 2.07-1.73 (m, 2H), 1.44 (s, 9H), 1.35 (d, *J =* 6.9 Hz, 3H).

### Example 55: Synthesis of Compound E50

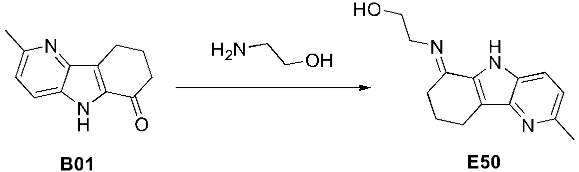

Under a nitrogen atmosphere at room temperature, ethanolamine (116 mg, 1.896 mmol) was added to a toluene (4 mL) solution of compound B01 (100 mg, 0.474 mmol). The reaction mixture was stirred at 130 °C for 4 hours, cooled to room temperature, and concentrated under reduced pressure. The residue was washed with a mixture of n-hexane and ethanol (V:V = 3:1, 3 × 2 mL) to afford a brown solid, compound E50 (27.2 mg, 22.38%). ESI/MS (m/z): 244.1 [M+H]⁺. ¹H NMR (400 MHz, DMSO-*d*₆*)* δ 11.05 (s, 1H), 7.57 (d, *J =* 8.3 Hz, 1H), 7.00 (d, *J =* 8.4 Hz, 1H), 4.56 (s, 1H), 3.70 (t, *J =* 6.3 Hz, 2H), 3.58 (t, *J =* 6.3 Hz, 2H), 2.83 (t, *J =* 6.0 Hz, 2H), 2.61 (t, *J =* 6.3 Hz, 2H), 2.51 (s, 3H), 1.98 (p, *J =* 5.9 Hz, 2H).

### Example56: Synthesis of Compound E51

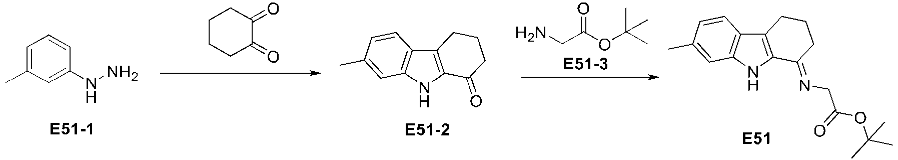

At room temperature, concentrated hydrochloric acid (80 mL) was added to an aqueous solution (2000 mL) of compound E51-1 (20 g, 163.70 mmol) and 1,2-cyclohexanedione (22 g, 196.44 mmol). The reaction mixture was stirred at 120 °C overnight, cooled to room temperature, and filtered. The filter cake was washed with water (2 × 1000 mL). The crude product was purified by reverse-phase preparative chromatography (mobile phase A: water containing 10 mmol/L ammonium bicarbonate and 0.1% ammonia; mobile phase B: acetonitrile) to afford a pale yellow solid, compound E51-2 (268 mg, 0.82%). ESI/MS (m/z): 200.1 [M+H]⁺. ¹H NMR (400 MHz, DMSO-*d*₆) δ 11.53 (s, 1H), 11.44 (s, 1H), 7.53 (s, 1H), 7.20 (d, *J =* 8.4 Hz, 1H), 7.18 (s, 1H), 7.13 (t, *J =* 7.6 Hz, 1H), 6.91 (d, *J =* 8.2 Hz, 1H), 6.78 (d, *J =* 6.9 Hz, 1H), 3.18 (t, *J* = 5.9 Hz, 2H), 2.91 (t, *J =* 5.9 Hz, 2H), 2.61 (s, 2H), 2.57-2.51 (m, 3H), 2.40 (s, 3H), 2.19-2.08 (m, 4H).

At room temperature, compound E51-3 (648 mg, 4.940 mmol) was added to a toluene (3 mL) solution of compound E51-2 (200 mg, 0.988 mmol). The reaction mixture was stirred at 130 °C under nitrogen for 5 hours. After cooling to room temperature, the mixture was concentrated under reduced pressure. The residue was purified by reverse-phase preparative chromatography (mobile phase A: pure water; mobile phase B: acetonitrile) to afford a yellow solid, compound E51 (61.6 mg, 19.28%). ESI/MS (m/z): 313.1 [M+H]⁺. ¹H NMR (400 MHz, DMSO-*d₆*) δ 10.94 (s, 1H), 7.40 (d, *J =* 8.1 Hz, 1H), 7.15 (s, 1H), 6.82 (dd, *J =* 8.1, 1.5 Hz, 1H), 4.27 (s, 2H), 2.78 (t, *J =* 6.0 Hz, 2H), 2.55-2.51 (m, 2H), 2.37 (s, 3H), 1.96 (p, *J =* 6.1 Hz, 2H), 1.44 (s, 9H).

### Example 57: Synthesis of Compound E52

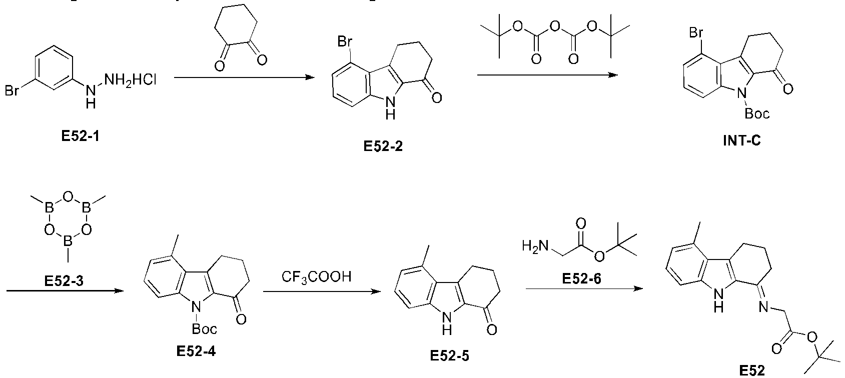

At room temperature, concentrated hydrochloric acid (7 mL) was added dropwise to an aqueous solution (240 mL) of compound E52-1 (10 g, 44.743 mmol) and 1,2-cyclohexanedione (5.5 g, 49.217 mmol). The mixture was stirred at 120 °C overnight. After cooling to room temperature, the mixture was filtered, and the filter cake was collected and washed with water (3 × 200 mL). The resulting solid was dried under vacuum to afford a brown solid, compound E52-2 (12 g, crude). ESI/MS (m/z): 264.1 [M+H]⁺.

To a tetrahydrofuran (20 mL) solution of E52-2 (10 g, 18.931 mmol) and triethylamine (5.8 g, 56.793 mmol), 4-dimethylaminopyridine (2.31 g, 18.931 mmol) and di-tert-butyl dicarbonate (8.3 g, 37.862 mmol) were added. The reaction mixture was stirred at room temperature for 1 hour, quenched with water (50 mL), and extracted with ethyl acetate (3 × 50 mL). The combined organic layers were washed with brine (100 mL), dried over anhydrous sodium sulfate, and concentrated under vacuum. The residue was purified by silica gel column chromatography (petroleum ether:ethyl acetate = 10:1) to afford a pale yellow solid, compound INT-C (1 g, 14.21%). ESI/MS (m/z): 308.0 [M+H-56]⁺.

At room temperature, tetrakis(triphenylphosphine)palladium (310.92 mg, 0.269 mmol) and potassium carbonate (743.69 mg, 5.382 mmol) were added portionwise to a 1,4-dioxane (10 mL) solution of INT-C (1 g, 2.581 mmol) and E52-3 (1.15 mL, 3.876 mmol). The reaction mixture was stirred at 100 °C for 2 hours. After quenching with water at room temperature, the mixture was extracted with dichloromethane (3 × 30 mL). The combined organic layers were washed with brine (30 mL), dried over anhydrous sodium sulfate, and concentrated under reduced pressure. The residue was purified by normal-phase silica gel column chromatography (petroleum ether:ethyl acetate = 5:1) to afford a yellow solid, compound E52-4 (750 mg, 90.23%). ESI/MS (m/z): 244.1 [M+H-56]⁺.

At room temperature, trifluoroacetic acid (1 mL) was added dropwise to a dichloromethane (10 mL) solution of compound E52-4 (100 mg, 0.316 mmol). The reaction mixture was stirred at room temperature for 1 hour and then concentrated directly under reduced pressure. The residue was purified by normal-phase silica gel column chromatography (petroleum ether:ethyl acetate = 5:1) to afford a pale yellow solid, compound E52-5 (22.5 mg, 34.54%). ESI/MS (m/z): 200.2 [M+H]⁺. ¹H NMR (400 MHz, DMSO-*d₆*) δ 11.52 (s, 1H), 7.19 (d, *J =* 8.3 Hz, 1H), 7.13 (dd, *J =* 8.4, 6.9 Hz, 1H), 6.78 (d, *J =* 6.8 Hz, 1H), 3.19 (t, *J =* 6.0 Hz, 2H), 2.62 (s, 3H), 2.54-2.51 (m, 2H), 2.15 (p, *J =* 6.3 Hz, 2H)

At room temperature, E52-6 (474.5 mg, 3.615 mmol) was added to a toluene (3 mL) solution of compound E52-5 (150 mg, 0.723 mmol). The reaction mixture was stirred at 130 °C under nitrogen for 5 hours, cooled to room temperature, and concentrated under reduced pressure. The residue was purified by reverse-phase preparative chromatography (mobile phase A: pure water; mobile phase B: acetonitrile) to afford a yellow solid, compound E52 (28.6 mg, 11.95%). ESI/MS (m/z): 313.1 [M+H]⁺. ¹H NMR (400 MHz, DMSO-*d₆*) δ 11.01 (s, 1H), 7.18 (d, *J =* 8.1 Hz, 1H), 6.98 (dd, *J =* 8.3, 6.9 Hz, 1H), 6.69 (d, *J =* 7.0 Hz, 1H), 4.28 (s, 2H), 3.08 (t, *J =* 6.0 Hz, 2H), 2.59 (s, 3H), 2.49-2.47 (m, 2H), 2.00-1.95 (m, 2H), 1.44 (s, 9H).

### Example 58: Synthesis of Compound E53

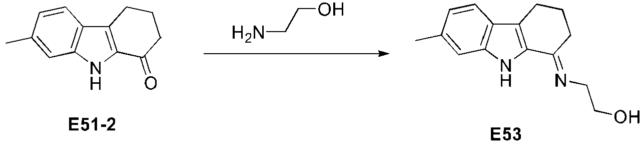

At room temperature, ethanolamine (75.4 mg, 1.235 mmol) was added to a toluene (3 mL) solution of compound E51-2 (100 mg, 0.494 mmol). The reaction mixture was stirred at 130 °C under nitrogen for 5 hours, cooled to room temperature, and concentrated under reduced pressure. The resulting residue was washed with ethanol (3 × 3 mL) to afford a white solid, compound E53 (45.1 mg, 36.67%). ESI/MS (m/z): 243.3 [M+H]⁺. ¹H NMR (300 MHz, DMSO-*d₆*) δ 10.82 (s, 1H), 7.38 (d, *J =* 8.1 Hz, 1H), 7.14 (s, 1H), 6.81 (d, *J =* 8.1 Hz, 1H), 4.54 (t, *J =* 5.2 Hz, 1H), 3.65-3.71 (m, 2H), 3.55 (t, *J =* 6.3 Hz, 2H), 2.76 (t, *J =* 6.0 Hz, 2H), 2.57 (t, *J =* 6.3 Hz, 2H), 2.37 (s, 3H), 1.98 (q, *J =* 6.2 Hz, 2H).

### Example 59: Synthesis of Compound E54

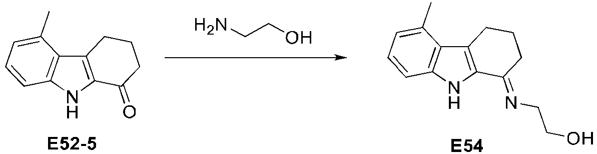

At room temperature, ethanolamine (73.7 mg, 1.206 mmol) was added to a toluene (3 mL) solution of compound E52-5 (100 mg, 0.482 mmol). The reaction mixture was stirred at 130 °C under nitrogen for 5 hours, cooled to room temperature, and concentrated under reduced pressure. The resulting residue was washed with ethanol (2 × 4 mL) to afford a yellow solid, compound E54 (27.6 mg, 22.81%). ESI/MS (m/z): 243.1 [M+H]⁺. ¹H NMR (400 MHz, DMSO-*d₆*) δ 10.88 (s, 1H), 7.17 (d, *J =* 8.2 Hz, 1H), 6.96 (t, *J =* 7.6 Hz, 1H), 6.68 (d, *J =* 7.1 Hz, 1H), 4.53 (t, *J =* 5.3 Hz, 1H), 3.73-3.65 (m, 2H), 3.55 (t, *J =* 6.3 Hz, 2H), 3.06 (t, *J =* 6.0 Hz, 2H), 2.58 (s, 3H), 2.55 (t, *J =* 6.3 Hz, 2H), 1.98 (p, *J =* 6.2 Hz, 2H).

### Example 60: Synthesis of Compound E55

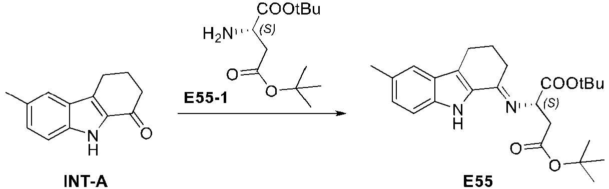

At room temperature, pyrrolidine (51 mg, 0.714 mmol) and glacial acetic acid (9 mg, 0.143 mmol) were added to a toluene (4 mL) solution of compound INT-A (150 mg, 0.714 mmol) and compound E55-1 (513 mg, 1.499 mmol, tBu: tert-butyl). The reaction mixture was stirred at 100 °C under nitrogen for 6 hours. After cooled to room temperature, the mixture was concentrated under reduced pressure. The residue was purified by reverse-phase preparative chromatography (mobile phase A: water; mobile phase B: acetonitrile) to afford a yellow solid, compound E55 (36.9 mg, 14.73%). ESI/MS (m/z): 427.2 [M+H]⁺. ¹H NMR (300 MHz, DMSO-*d*₆) δ 10.83 (s, 1H), 7.30 (s, 1H), 7.27 (d, *J =* 8.3 Hz, 1H), 6.99 (dd, *J =* 8.4, 1.6 Hz, 1H), 4.68 (t, *J* = 7.0 Hz, 1H), 2.90-2.57 (m, 2H), 2.80 (t, *J =* 6.0 Hz, 2H), 2.74-2.65 (m, 2H), 2.36 (s, 3H), 2.00 (p, *J =* 5.2 Hz, 2H), 1.38 (s, 9H), 1.36 (s, 9H).

### Example 61: Synthesis of Compound E56

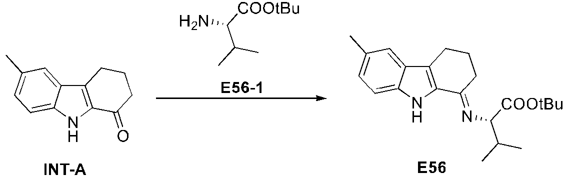

At room temperature, compound E56-1 (608 mg, 3.514 mmol, tBu: tert-butyl) was added to a toluene (4 mL) solution of compound INT-A (200 mg, 1.004 mmol). The reaction mixture was stirred at 140 °C under nitrogen overnight. After cooled to room temperature, the mixture was concentrated under reduced pressure. The residue was purified by reverse-phase preparative chromatography (mobile phase A: water; mobile phase B: acetonitrile) to afford a yellow solid, compound E56 (37.7 mg, 10.43%). ESI/MS (m/z): 355.1 [M+H]⁺. ¹H NMR (300 MHz, DMSO-*d₆*) δ 10.78 (s, 1H), 7.32-7.27 (m, 2H), 7.03-6.94 (m, 1H), 3.94 (d, *J =* 7.1 Hz, 1H), 2.78 (t, *J =* 6.0 Hz, 2H), 2.67-2.55 (m, 1H), 2.44 (d, *J =* 6.3 Hz, 1H), 2.36 (s, 3H), 2.26 (p, *J =* 6.7 Hz, 1H), 1.97 (t, *J =* 6.3 Hz, 2H), 1.40 (s, 9H), 1.00-0.89 (m, 6H).

### Example 62: Synthesis of Compound E57

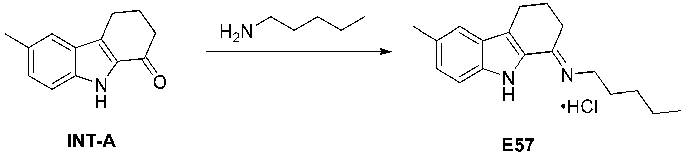

A toluene solution (4 mL) of compound INT-A (199.3 mg, 1.000 mmol) and n-pentylamine (261.5 mg, 3 mmol) was stirred at 135 °C for 3 hours. After cooled to room temperature, the reaction mixture was concentrated under vacuum, and 4 M hydrogen chloride in ethyl acetate (3 mL) was added. The precipitated solid was triturated with a small amount of a mixed solvent (acetonitrile/water = 2:1) to afford a pale yellow solid, compound E57 (157.6 mg, 51.70%). ESI/MS (m/z): 269.1 [M+H]⁺. ¹H NMR (500 MHz, DMSO-*d*₆) δ 12.57 (s, 1H), 12.39 (s, 1H), 7.53 (s, 1H), 7.40 (d, *J =* 8.5 Hz, 1H), 7.31 (d, *J =* 8.6 Hz, 1H), 3.74-3.69 (m, 2H), 3.06 (t, *J =* 6.2 Hz, 2H), 2.98 (t, *J =* 6.1 Hz, 2H), 2.39 (s, 3H), 2.14-2.08 (m, 2H), 1.82-1.76 (m, 2H), 1.41-1.33 (m, 4H), 0.90 (t, *J =* 7.1 Hz, 3H).

### Example 63: Synthesis of Compound E58

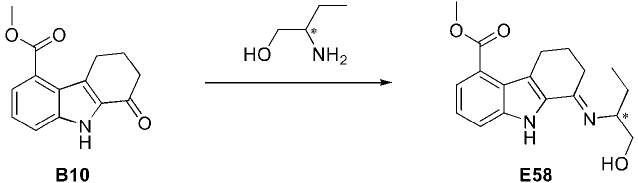

At room temperature, 2-amino-1-butanol (271 mg, 3.045 mmol, racemate) was added to a toluene (3 mL) solution of compound B10 (150 mg, 0.609 mmol). The reaction mixture was stirred at 130 °C for 6 hours. After cooled to room temperature, the mixture was concentrated under reduced pressure. The residue was washed with a mixed solvent of ethyl acetate and n-hexane (ethyl acetate:n-hexane = 1:6, 1 mL) to afford a pale yellow solid, compound E58 (74.8 mg, 39.83%, racemate). ESI/MS (m/z): 315.1 [M+H]⁺. ¹H NMR (400 MHz, DMSO-*d*₆) δ 11.25 (s, 1H), 7.66 (dd, *J =* 8.1, 1.1 Hz, 1H), 7.50 (dd, *J =* 7.4, 1.1 Hz, 1H), 7.20 (dd, *J =* 8.1, 7.4 Hz, 1H), 4.50 (s, 1H), 3.87 (s, 3H), 3.71-3.61 (m, 1H), 3.57-3.48 (m, 1H), 3.41 (t, *J =* 8.6 Hz, 1H), 2.94 (t, *J =* 6.0 Hz, 2H), 2.74-2.52 (m, 2H), 1.98-1.87 (m, 2H), 1.74-1.60 (m, 1H), 1.52-1.37 (m, 1H), 0.83 (t, *J =* 7.4 Hz, 3H)

### Example 64: Synthesis of Compound E59

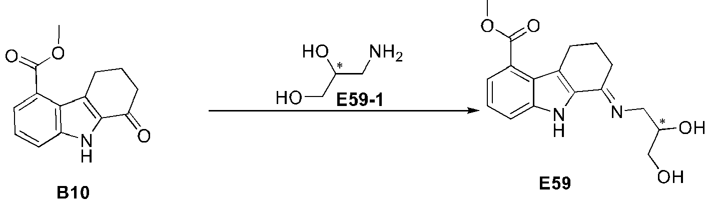

At room temperature, compound E59-1 (468.17 mg, 5.140 mmol, racemate) was added dropwise to a toluene (5 mL) solution of compound B10 (250 mg, 1.028 mmol). Under nitrogen protection, the reaction mixture was stirred at 130 °C for 1 hour. After cooling to room temperature, the mixture was concentrated under reduced pressure. The residue was washed with ethanol (4 × 5 mL) to afford a pale yellow solid, compound E59 (123.4 mg, 37.30%, racemate). ESI/MS (m/z): 317.0 [M+H]⁺. ¹H NMR (400 MHz, DMSO-*d*₆) δ 11.37 (s, 1H), 7.64 (dd, *J =* 8.1, 1.1 Hz, 1H), 7.50 (dd, *J* = 7.4, 1.1 Hz, 1H), 7.21 (t, *J* = 7.8 Hz, 1H), 4.56 (d, *J =* 4.8 Hz, 1H), 4.55-4.48 (m, 1H), 3.87 (s, 3H), 3.84-3.72 (m, *J =* 4.2 Hz, 1H), 3.57-3.49 (m, 2H), 3.47-3.42 (m, 2H), 2.94 (t, *J =* 6.0 Hz, 2H), 2.58-2.54 (m, 2H), 1.96-1.90 (m, 2H).

### Example 65: Synthesis of Compound E60

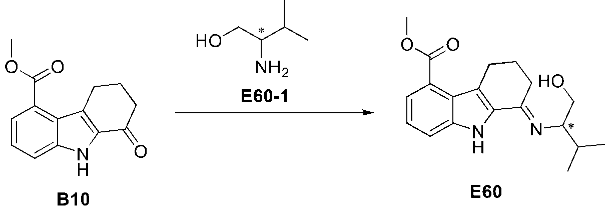

At room temperature, compound E60-1 (318.07 mg, 3.085 mmol, racemate) was added dropwise to a toluene (3 mL) solution of compound B10 (150 mg, 0.617 mmol). The reaction mixture was stirred at 130 °C for 1 day. After cooling to room temperature, the mixture was directly concentrated under reduced pressure. The residue was purified by silica gel column chromatography (petroleum ether:ethyl acetate = 2:1) to afford a white solid, compound E60 (27.6 mg, 12.98%, racemate). ESI/MS (m/z): 329.0 [M+H]⁺. ¹H NMR (400 MHz, DMSO-*d*₆) δ 11.18 (s, 1H), 7.68 (d, *J =* 8.1 Hz, 1H), 7.50 (d, *J =* 7.3 Hz, 1H), 7.20 (t, *J =* 7.7 Hz, 1H), 4.40 (s, 1H), 3.87 (s, 3H), 3.69-3.62 (m, 1H), 3.57-3.38 (m, 2H), 2.94 (t, *J =* 5.9 Hz, 2H), 2.72-2.51 (m, 2H), 1.96 -1.90 (m, 2H), 1.90-1.84 (m, 1H), 0.91 (d, *J =* 6.7 Hz, 6H).

### Example 66: Synthesis of Compound E61

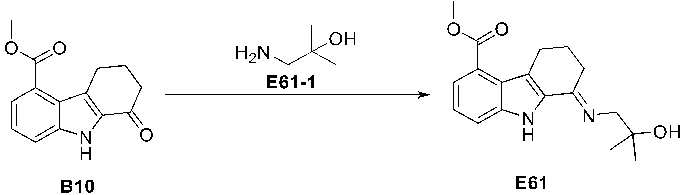

At room temperature, compound E61-1 (269.32 mg, 3.020 mmol) was added dropwise to a toluene (3 mL) solution of compound B10 (150 mg, 0.604 mmol). Under nitrogen protection, the reaction mixture was stirred at 130 °C for 3.5 hours. After cooling to room temperature, the mixture was concentrated under reduced pressure. The residue was washed with a mixture of toluene and n-hexane (toluene:n-hexane = 1:5, 3 × 6 mL) to afford a yellow solid, compound E61 (54.2 mg, 27.10%). ESI/MS (m/z): 315.2 [M+H]⁺. ¹H NMR (400 MHz, DMSO-*d₆*) δ 11.34 (s, 1H), 7.65 (dd, *J =* 8.1, 1.1 Hz, 1H), 7.51 (dd, *J =* 7.4, 1.1 Hz, 1H), 7.22 (dd, *J =* 8.1, 7.4 Hz, 1H), 4.32 (s, 1H), 3.87 (s, 3H), 3.31 (s, 2H), 2.95 (t, *J =* 6.0 Hz, 2H), 2.54-2.51 (m, 2H), 1.96-1.90 (m, 2H), 1.20 (s, 6H).

### Example 67: Synthesis of Compound E62

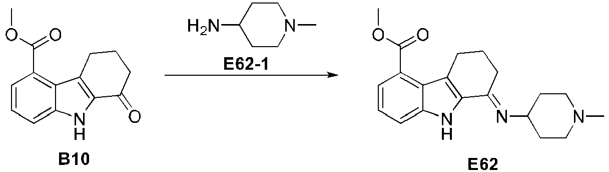

Under a nitrogen atmosphere, E62-1 (347 mg, 3.045 mmol) was added to a toluene (3 mL) solution of B10 (150 mg, 0.609 mmol). The reaction mixture was stirred at 130 °C overnight. After cooling to room temperature, the mixture was concentrated under reduced pressure. The residue was washed with a mixed solvent of ethyl acetate and n-hexane (ethyl acetate: n-hexane = 1:6, 7 mL) to afford a yellow solid, compound E62 (31.1 mg, 14.95%). ESI/MS (m/z): 340.1 [M+H]⁺. ¹H NMR (400 MHz, DMSO-*d*₆) δ 11.25 (s, 1H), 7.66 (dd, *J =* 8.2, 1.1 Hz, 1H), 7.49 (dd, *J =* 7.4, 1.1 Hz, 1H), 7.23-7.15 (m, 1H), 3.87 (d, *J =* 1.0 Hz, 3H), 3.62-3.51 (m, 1H), 2.93 (t, *J =* 6.0 Hz, 2H), 2.87-2.76 (m, 2H), 2.63-2.57 (m, 2H), 2.19 (s, 3H), 2.08-1.97 (m, 2H), 1.92 (t, *J =* 6.2 Hz, 2H), 1.77-1.66 (m, 2H), 1.66-1.56 (m, 2H)

### Example 68: Synthesis of Compound B18

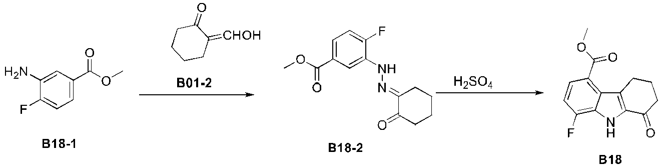

At 0-5 °C, concentrated hydrochloric acid (1.25 mL) and an aqueous solution (5 mL) of sodium nitrite (0.41 g, 5.92 mmol) were sequentially added to an aqueous solution (20 mL) of compound B18-1 (0.5 g, 2.96 mmol). The mixture was stirred for 10 minutes to prepare the diazonium salt solution. To a methanol (10 mL) solution of compound B01-2 (0.45 g, 3.55 mmol), an aqueous solution (5 mL) of sodium acetate (0.53 g, 6.5 mmol) was added. The system was cooled to 0-5 °C, and the previously prepared diazonium salt solution was added dropwise. After stirred for 30 minutes, the mixture was filtered, and the resulting solid was washed with water and dried to afford the crude product B18-2 as an orange solid (0.56 g, 68%).

To an acetonitrile solution (6 mL) of compound B18-2 (0.56 g, 2.0 mmol), 1.8 M sulfuric acid (3 mL) was added. The reaction mixture was stirred at 80 °C for 4 hours. After cooling to room temperature, the mixture was diluted with ethyl acetate (30 mL). The organic phase was washed with brine, dried over anhydrous sodium sulfate, filtered, and concentrated under reduced pressure. The residue was purified by silica gel column chromatography (petroleum ether:ethyl acetate = 10:1) to afford a pink solid, B18 (90 mg, 17.2%). ESI/MS (m/z): 262.0 [M+H]⁺. ¹H NMR (500 MHz, DMSO-*d*₆) δ 12.53 (s, 1H), 7.62 (dd, *J =* 8.2, 4.7 Hz, 1H), 7.22 (dd, *J =* 10.5, 8.2 Hz, 1H), 3.89 (s, 3H), 3.10 (t, *J =* 6.0 Hz, 2H), 2.61-2.59 (m, 2H), 2.142.09 (m, 2H).

### Example 69: Synthesis of Compound B19

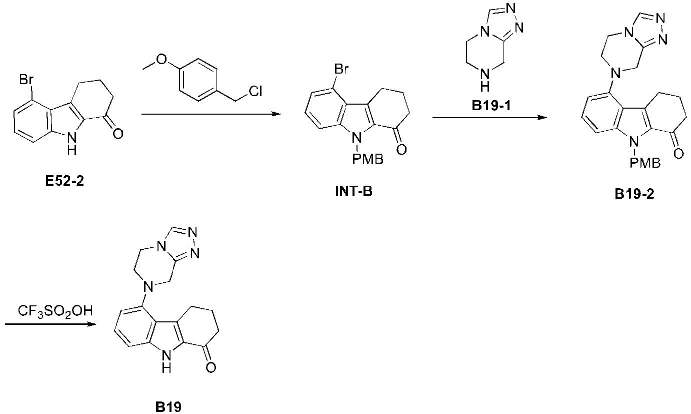

At 0 °C, 4-methoxybenzyl chloride (858 mg, 5.481 mmol) was added to a DMF (5 mL) solution of compound E52-2 (1 g, 3.654 mmol) and 60% sodium hydride (293 mg, 7.308 mmol). The mixture was stirred at room temperature for 1 hour. The reaction was quenched with saturated ammonium chloride solution (50 mL) and extracted with ethyl acetate (3 × 80 mL). The combined organic layers were washed with brine (100 mL), dried over anhydrous sodium sulfate, filtered, and concentrated under reduced pressure. The residue was purified by silica gel column chromatography (petroleum ether:ethyl acetate = 7:1) to afford a yellow solid, compound INT-B (1.3 g, 82.59%; PMB: 4-methoxybenzyl). ESI/MS (m/z): 386.1 [M+H]⁺.

Under a nitrogen atmosphere, compound B19-1 (99.3 mg, 0.800 mmol), (2-dicyclohexylphosphino-2',6'-diisopropoxy-1,1'-biphenyl)(2-amino-1,1'-biphenyl-2-yl)palladium(II) methanesulfonate (33.5 mg, 0.040 mmol), 2-dicyclohexylphosphino-2',6'-diisopropoxy-1,1'-biphenyl (18.7 mg, 0.040 mmol), and sodium tert-butoxide (76.9 mg, 0.800 mmol) were sequentially added to a mixture of compound INT-B (153.7 mg, 0.400 mmol) and 1,4-dioxane (4 mL). The reaction mixture was stirred at 100 °C for 3 hours. After cooling to room temperature, the mixture was diluted with ethyl acetate (100 mL), filtered through Celite, and concentrated under reduced pressure to afford the crude compound B19-2. To the crude compound B19-2, a mixture of trifluoromethanesulfonic acid (0.15 mL) and trifluoroacetic acid (3 mL) was added, and the mixture was stirred at room temperature for 10 minutes. The reaction mixture was diluted with ethyl acetate (100 mL) and sequentially washed with saturated sodium bicarbonate solution (30 mL) and brine (30 mL). The organic layer was dried over anhydrous sodium sulfate and concentrated under reduced pressure. The residue was purified by reverse-phase silica gel column chromatography (10-50% acetonitrile/water) to afford a yellow solid, compound B19 (38.3 mg, 31.15%). ESI/MS (m/z): 308.0 [M+H]⁺. ¹H NMR (500 MHz, DMSO-*d*₆) δ 11.69 (s, 1H), 8.53 (s, 1H), 7.21 (t, *J =* 7.8 Hz, 1H), 7.14 (d, *J =* 8.2 Hz, 1H), 6.71 (d, *J =* 7.4 Hz, 1H), 4.36 (s, 2H), 4.21 (t, *J =* 5.5 Hz, 2H), 3.63-3.45 (m, 2H), 3.14 (t, *J =* 5.8 Hz, 2H), 2.55-2.52 (m, 2H), 2.10-2.04 (m, 2H).

### Example 70: Synthesis of Compound B20

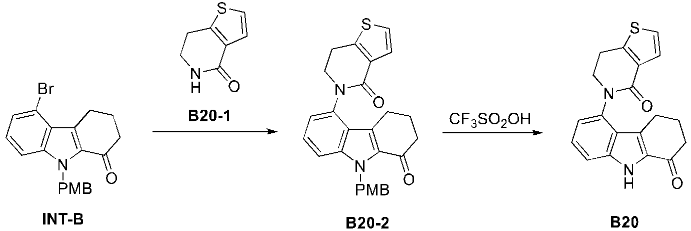

Under a nitrogen atmosphere, B20-1 (64.5 mg, 0.400 mmol), tris(dibenzylideneacetone)dipalladium (18.3 mg, 0.020 mmol), 4,5-bis(diphenylphosphino)-9,9-dimethylxanthene (11.6 mg, 0.020 mmol), and sodium tert-butoxide (48.0 mg, 0.500 mmol) were sequentially added to a mixture of compound INT-B (76.9 mg, 0.200 mmol; PMB: 4-methoxybenzyl) and 1,4-dioxane (2 mL). The mixture was stirred at 110 °C for 8 hours. After cooling to room temperature, the reaction mixture was diluted with ethyl acetate (100 mL), filtered through Celite, and concentrated under vacuum to afford the crude compound B20-2. To the crude compound B20-2, a mixture of trifluoromethanesulfonic acid (0.06 mL) and trifluoroacetic acid (3 mL) was added, and the mixture was stirred at room temperature for 20 minutes. The reaction mixture was diluted with ethyl acetate (100 mL) and sequentially washed with saturated sodium bicarbonate solution (30 mL) and brine (30 mL). The organic layer was dried over anhydrous sodium sulfate and concentrated under reduced pressure. The residue was purified by reverse-phase silica gel column chromatography (10-70% acetonitrile/water) to afford a white solid, compound B20 (22.4 mg, 33.29%). ESI/MS (m/z): 337.0 [M+H]⁺. ¹H NMR (500 MHz, CDCl₃) δ 8.95 (s, 1H), 7.53 (d, *J* = 5.2 Hz, 1H), 7.36 (d, *J =* 2.7 Hz, 1H), 7.35 (s, 1H), 7.17 (d, *J =* 5.2 Hz, 1H), 7.02-6.99 (m, 1H), 4.26-4.20 (m, 1H), 4.00-3.95 (m, 1H), 3.40-3.33 (m, 1H), 3.26-3.20 (m, 1H), 3.06-3.00 (m, 1H), 2.96-2.90 (m, 1H), 2.62-2.56 (m, 2H), 2.22-2.12 (m, 2H).

### Example 71: Synthesis of Compound B21

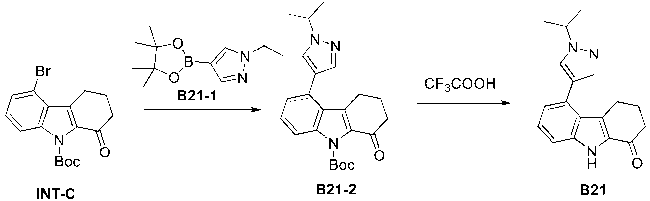

Under a nitrogen atmosphere, compound B21-1 (94.4 mg, 0.400 mmol), [1,1'-bis(diphenylphosphino)ferrocene]palladium(II) dichloride dichloromethane complex (8.0 mg, 0.010 mmol), and potassium carbonate (55.3 mg, 0.400 mmol) were sequentially added to a mixture of compound INT-C (72.8 mg, 0.200 mmol; Boc: tert-butoxycarbonyl) and 1,4-dioxane (3 mL). The mixture was stirred at 80 °C for 3 hours. After cooling to room temperature, the reaction mixture was diluted with ethyl acetate (100 mL), filtered through Celite, and concentrated under reduced pressure to afford the crude compound B21-2. To the crude compound B21-2, a mixture of trifluoroacetic acid (0.3 mL) and dichloromethane (3 mL) was added, and the mixture was stirred at room temperature for 10 minutes. The reaction mixture was diluted with ethyl acetate (100 mL) and sequentially washed with saturated sodium bicarbonate solution (30 mL) and brine (30 mL). The organic layer was dried over anhydrous sodium sulfate and concentrated under reduced pressure. The residue was purified by reverse-phase silica gel column chromatography (10-70% acetonitrile/water) to afford a pale yellow solid, compound B21 (34.0 mg, 57.95%). ESI/MS (m/z): 294.1 [M+H]⁺. ¹H NMR (500 MHz, DMSO-*d*₆) δ 11.67 (s, 1H), 7.96 (s, 1H), 7.59 (s, 1H), 7.33 (d, *J =* 8.1 Hz, 1H), 7.29-7.24 (m, 1H), 6.93 (d, *J =* 6.9 Hz, 1H), 4.60-4.52 (m, 1H), 2.73 (t, *J =* 5.9 Hz, 2H), 2.53-2.51 (m, 2H), 2.03-1.97 (m, 2H), 1.49 (s, 3H), 1.47 (s, 3H).

### Example 72: Synthesis of Compound B22

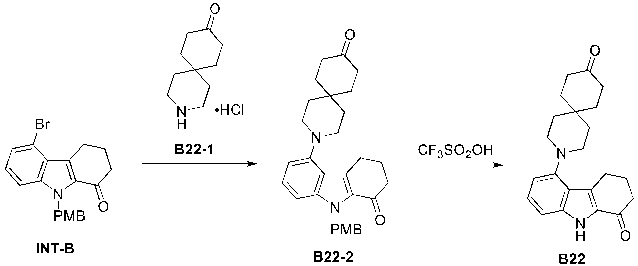

Under a nitrogen atmosphere, compound B22-1 (163.0 mg, 0.800 mmol), (2-dicyclohexylphosphino-2',6'-diisopropoxy-1,1'-biphenyl)(2-amino-1,1'-biphenyl-2-yl)palladium(II) methanesulfonate (33.5 mg, 0.040 mmol), 2-dicyclohexylphosphino-2',6'-diisopropoxy-1,1'-biphenyl (18.7 mg, 0.040 mmol), and sodium tert-butoxide (153.8 mg, 1.600 mmol) were sequentially added to a mixture of compound INT-B (153.7 mg, 0.400 mmol) and 1,4-dioxane (4 mL). The mixture was stirred at 100 °C for 3 hours. After cooling to room temperature, the reaction mixture was diluted with ethyl acetate (100 mL), filtered through Celite, and concentrated under vacuum to afford the crude compound B22-2. To the crude compound B22-2, a mixture of trifluoromethanesulfonic acid (0.15 mL) and trifluoroacetic acid (3 mL) was added, and the mixture was stirred at room temperature for 10 minutes. The reaction mixture was diluted with ethyl acetate (100 mL) and sequentially washed with saturated sodium bicarbonate solution (30 mL) and brine (30 mL). The organic layer was dried over anhydrous sodium sulfate and concentrated under reduced pressure. The residue was purified by reverse-phase silica gel column chromatography (10-70% acetonitrile/water) to afford a white solid, compound B22 (8.0 mg, 5.71%). ESI/MS (m/z): 351.1 [M+H]⁺. ¹H NMR (500 MHz, DMSO-*d*₆) δ 11.54 (s, 1H), 7.15 (t, *J =* 7.9 Hz, 1H), 7.02 (d, *J =* 8.2 Hz, 1H), 6.60 (d, *J =* 7.5 Hz, 1H), 3.23 (t, *J =* 5.8 Hz, 2H), 3.00 (s, 4H), 2.55-2.52 (m, 2H), 2.30 (t, *J* = 6.7 Hz, 4H), 2.13-2.09 (m, 2H), 1.82-1.73 (m, 8H).

### Example 73: Synthesis of Compound B23

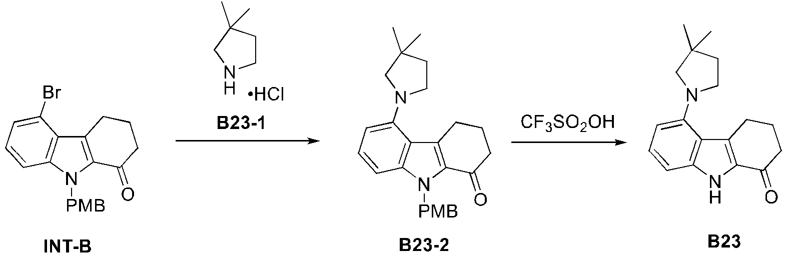

Under a nitrogen atmosphere, compound B23-1 (39.7 mg, 0.400 mmol), (2-dicyclohexylphosphino-2',6'-diisopropoxy-1,1'-biphenyl)(2-amino-1,1'-biphenyl-2-yl)palladium(II) methanesulfonate (16.7 mg, 0.020 mmol), 2-dicyclohexylphosphino-2',6'-diisopropoxy-1,1'-biphenyl (9.3 mg, 0.020 mmol), and sodium tert-butoxide (76.9 mg, 0.800 mmol) were sequentially added to a mixture of compound INT-B (76.9 mg, 0.200 mmol) and 1,4-dioxane (2 mL). The mixture was stirred at 90 °C for 3 hours. After cooling to room temperature, the reaction mixture was diluted with ethyl acetate (100 mL), filtered through Celite, and concentrated under reduced pressure to afford the crude compound B23-2. To the crude compound B23-2, a mixture of trifluoromethanesulfonic acid (0.15 mL) and trifluoroacetic acid (3 mL) was added, and the mixture was stirred at room temperature for 20 minutes. The reaction mixture was diluted with ethyl acetate (100 mL) and sequentially washed with saturated sodium bicarbonate solution (30 mL) and brine (30 mL). The organic layer was dried over anhydrous sodium sulfate and concentrated under reduced pressure. The residue was purified by reverse-phase silica gel column chromatography (10-50% acetonitrile/water) to afford a yellow solid, compound B23 (7.4 mg, 13.10%). ESI/MS (m/z): 283.1 [M+H]⁺. ¹H NMR (500 MHz, DMSO-*d*₆) δ 11.48 (s, 1H), 7.08 (t, *J =* 7.9 Hz, 1H), 6.90 (d, *J =* 8.1 Hz, 1H), 6.44 (d, *J =* 7.6 Hz, 1H), 3.30-2.26 (m, 2H), 3.18 (t, *J =* 5.8 Hz, 2H), 2.97 (s, 2H), 2.53-2.51 (m, 2H), 2.10-2.05 (m, 2H), 1.72 (t, *J =* 7.0 Hz, 2H), 1.15 (s, 6H).

### Example 74: Synthesis of Compound B24

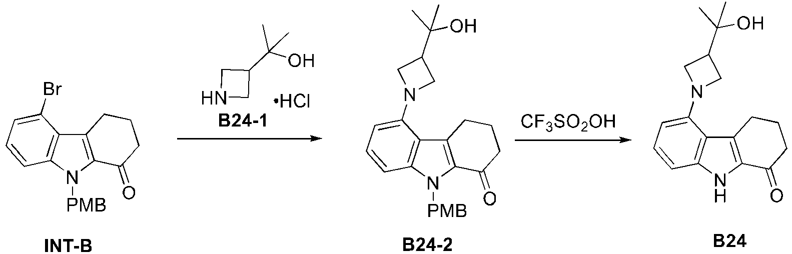

Under a nitrogen atmosphere, compound B24-1 (121.3 mg, 0.800 mmol), (2-dicyclohexylphosphino-2',6'-diisopropoxy-1,1'-biphenyl)(2-amino-1,1'-biphenyl-2-yl)palladium(II) methanesulfonate (33.5 mg, 0.040 mmol), 2-dicyclohexylphosphino-2',6'-diisopropoxy-1,1'-biphenyl (18.7 mg, 0.040 mmol), and sodium tert-butoxide (153.8 mg, 1.600 mmol) were sequentially added to a mixture of compound INT-B (153.7 mg, 0.400 mmol) and 1,4-dioxane (4 mL). The mixture was stirred at 100 °C for 3 hours. After cooling to room temperature, the reaction mixture was diluted with ethyl acetate (100 mL), filtered through Celite, and concentrated under vacuum to afford the crude compound B24-2. To the crude compound B24-2, a mixture of trifluoromethanesulfonic acid (0.15 mL) and trifluoroacetic acid (3 mL) was added, and the mixture was stirred at room temperature for 10 minutes. The reaction mixture was diluted with ethyl acetate (100 mL) and sequentially washed with saturated sodium bicarbonate solution (30 mL) and brine (30 mL). The organic layer was dried over anhydrous sodium sulfate and concentrated under reduced pressure. The residue was purified by reverse-phase silica gel column chromatography (10-40% acetonitrile/water) to afford a yellow solid, compound B24 (46.5 mg, 38.96%). ESI/MS (m/z): 299.1 [M+H]⁺. ¹H NMR (500 MHz, DMSO-*d*₆) δ 11.43 (s, 1H), 7.07 (t, *J =* 7.9 Hz, 1H), 6.78 (d, *J =* 8.1 Hz, 1H), 6.06 (d, *J =* 7.6 Hz, 1H), 4.37 (s, 1H), 3.92 (t, *J =* 7.8 Hz, 2H), 3.84 (t, *J =* 7.0 Hz, 2H), 3.08 (t, *J =* 5.8 Hz, 2H), 2.70-2.64 (m, 1H), 2.49-2.47 (m, 2H), 2.11-2.05 (m, 2H), 1.09 (s, 6H).

### Example 75: Synthesis of Compound B25

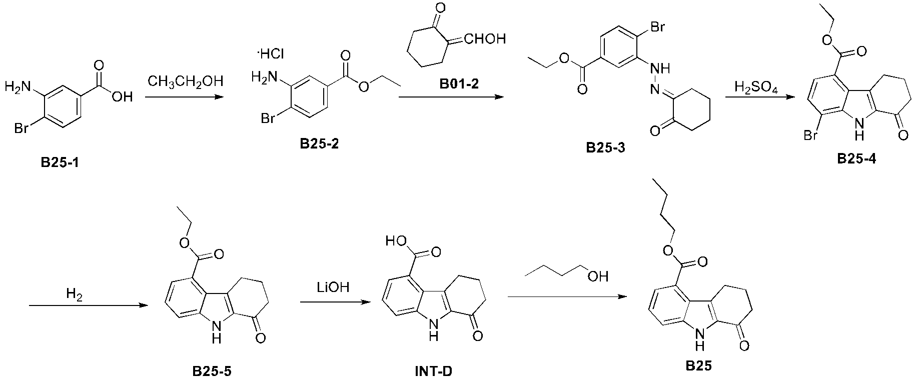

Under an ice bath, thionyl chloride (110 g, 0.93 mol) was added dropwise to a mixture of compound B25-1 (25.0 g, 0.12 mol) in anhydrous ethanol (250 mL). The reaction was heated under reflux for 24 hours. After cooling to room temperature, ethyl acetate (100 mL) was added, and the mixture was filtered. The filter cake was washed with ethyl acetate and dried to afford a white solid, compound B25-2 (30.1 g, 92.7%).

At 0-5 °C, concentrated hydrochloric acid (18 mL) and an aqueous solution (30 mL) of sodium nitrite (4.2 g, 61 mmol) were sequentially added to an aqueous solution (150 mL) of compound B25-2 (11.5 g, 41 mmol). The mixture was stirred for 10 minutes to prepare the diazonium salt solution. To a methanol (100 mL) solution of compound B01-2 (11.4 g, 90 mmol), an aqueous solution (80 mL) of sodium acetate (7.4 g, 90 mmol) was added. The system was cooled to 0-5 °C, and the previously prepared diazonium salt solution was added dropwise. Stirring was continued for 30 minutes. After the reaction, the mixture was filtered, and the filter cake was washed with water and dried to afford the crude product B25-3 as an orange solid (15 g, 100%).

To an acetonitrile (300 mL) solution of compound B25-3 (15 g, 41 mmol), 1.8 M sulfuric acid (60 mL) was added. The reaction was stirred at 80 °C for 4 hours. After cooling to room temperature, the mixture was diluted with ethyl acetate (200 mL). The organic phase was washed with brine, dried over anhydrous sodium sulfate, filtered, and concentrated under reduced pressure. The residue was purified by silica gel column chromatography (petroleum ether:ethyl acetate = 10:1) to afford an orange-red solid, B25-4 (6.4 g, 46%).

Under a nitrogen atmosphere, a mixture of methanol (42 mL) and ethyl acetate (42 mL) was added to a reaction flask containing compound B25-4 (4.2 g, 12.5 mmol) and palladium on carbon (10% w/w, 0.21 g). The atmosphere was replaced with hydrogen three times using a hydrogen balloon. Triethylamine (1.3 g, 12.5 mmol) was added, and the mixture was stirred at room temperature for 4 hours. After the reaction, the mixture was diluted with ethyl acetate (100 mL) and filtered through Celite to remove the palladium catalyst. The filtrate was sequentially washed with 0.5 M hydrochloric acid and brine, dried over anhydrous sodium sulfate, filtered, and concentrated under reduced pressure to afford the crude product B25-5 as a yellow solid (2.9 g, 90%).

At room temperature, an aqueous solution (10 mL) of lithium hydroxide (590 mg, 24.5 mmol) was added to a methanol (50 mL) solution of B25-5 (1.26 g, 4.9 mmol). The mixture was stirred at 65 °C for 1 hour. After the reaction, the mixture was cooled to 0-5 °C, and the pH was adjusted to 3 with 3 M hydrochloric acid. The mixture was directly filtered, and the filter cake was washed with water and dried to afford a white solid, INT-D (1.1 g, 98%).

Two drops of concentrated sulfuric acid were added to a mixture of compound INT-D (50 mg, 0.22 mmol) in n-butanol (0.5 mL). The reaction was stirred at 110 °C for 1 hour. After cooling to room temperature, the mixture was diluted with ethyl acetate (20 mL). The organic phase was washed with brine, dried over anhydrous sodium sulfate, filtered, and concentrated under reduced pressure. The residue was purified by silica gel column chromatography (ethyl acetate:petroleum ether = 1:10) to afford a white solid, compound B25 (50 mg, 79.6%). MS m/z: 286.1 [M+H]⁺. ¹H NMR (500 MHz, DMSO-*d*₆) δ 12.00 (s, 1H), 7.65 (dd, *J =* 8.3, 0.9 Hz, 1H), 7.59 (dd, *J =* 7.3, 0.9 Hz, 1H), 7.39-7.36 (m, 1H), 4.33 (t, *J =* 6.7 Hz, 2H), 3.09 (t, *J =* 6.0 Hz, 2H), 2.59-2.56 (m, 2H), 2.14-2.09 (m, 2H), 1.76-1.70 (m, 2H), 1.47-1.40 (m, 2H), 0.95 (t, *J =* 7.4 Hz, 3H).

### Example 76: Synthesis of Compound B26

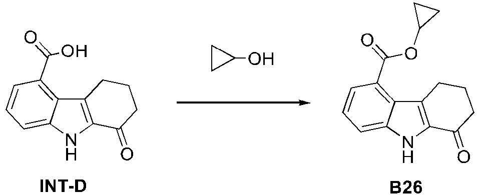

To a mixture of compound INT-D (50 mg, 0.22 mmol) in dichloromethane (1 mL), cyclopropanol (51 mg, 0.88 mmol), 4-dimethylaminopyridine (26.9 mg, 0.22 mmol), and N,N'-dicyclohexylcarbodiimide (91 mg, 0.44 mmol) were added. The reaction mixture was stirred at room temperature for 2 hours. The mixture was diluted with ethyl acetate (30 mL) and sequentially washed with 0.5 M hydrochloric acid (10 mL) and brine. The organic layer was dried over anhydrous sodium sulfate, filtered, and concentrated under reduced pressure. The residue was purified by silica gel column chromatography (ethyl acetate:petroleum ether = 1:5) to afford a white solid, compound B26 (51 mg, 86.1%). ESI/MS (m/z): 270.0 [M+H]⁺. ¹H NMR (500 MHz, DMSO-*d*₆) δ 12.01 (s, 1H), 7.66 (dd, *J =* 8.3, 0.9 Hz, 1H), 7.56 (dd, *J =* 7.3, 0.9 Hz, 1H), 7.37-7.34 (m, 1H), 4.38-4.34 (m, 1H), 3.09 (t, *J =* 6.0 Hz, 2H), 2.62-2.55 (m, 2H), 2.14-2.09 (m, 2H), 0.84-0.82 (m, 4H).

### Example 77: Synthesis of Compound B27

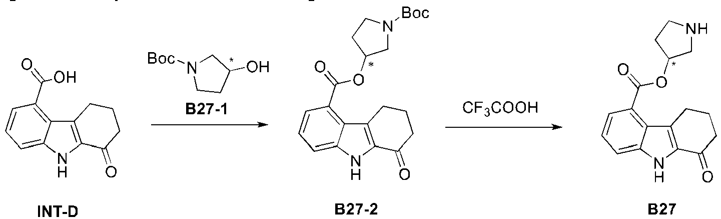

Under a nitrogen atmosphere at 0 °C, diethyl azodicarboxylate (153 mg, 0.88 mmol) was added to a mixture of compound INT-D (100 mg, 0.44 mmol), compound B27-1 (racemate, 165 mg, 0.88 mmol, Boc: tert-butoxycarbonyl), and triphenylphosphine (231 mg, 0.88 mmol) in tetrahydrofuran (1 mL). The reaction mixture was stirred at room temperature for 6 hours. After the reaction, the mixture was diluted with ethyl acetate (50 mL) and washed with brine. The organic layer was dried over anhydrous sodium sulfate, filtered, and concentrated under reduced pressure. The residue was purified by silica gel column chromatography (ethyl acetate:petroleum ether = 1:6) to afford a white solid, compound B27-2 (250 mg, 100%, racemate).

At room temperature, trifluoroacetic acid (0.25 mL) was added to a dichloromethane (1 mL) solution of compound B27-2 (250 mg). The mixture was stirred at room temperature for 1 hour and then concentrated directly. The residue was purified by silica gel column chromatography (dichloromethane:methanol = 15:1) to afford a white solid, compound B27 (89 mg, 67.8%, racemate). ESI/MS (m/z): 299.0 [M+H]⁺. ¹H NMR (500 MHz, DMSO-*d*₆) δ 11.99 (br, 1H), 7.65 (d, *J =* 8.0 Hz, 1H), 7.60 (d, *J =* 7.2 Hz, 1H), 7.28-7.35 (m, 1H), 5.38-5.35 (m, 1H), 3.12-3.08 (m, 3H), 2.97-2.92 (m, 2H), 2.84-2.79 (m, 1H), 2.59-2.56 (m, 2H), 2.14-2.03 (m, 3H), 1.90-1.86 (m, 1H).

### Example 78: Synthesis of Compound B28

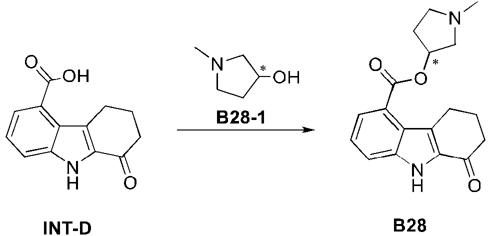

Under a nitrogen atmosphere at 0 °C, diethyl azodicarboxylate (76 mg, 0.44 mmol) was added to a mixture of compound INT-D (50 mg, 0.22 mmol), compound B28-1 (44 mg, 0.44 mmol, racemate), and triphenylphosphine (115 mg, 0.44 mmol) in tetrahydrofuran (1 mL). The reaction mixture was stirred at room temperature for 6 hours. After the reaction, the mixture was diluted with ethyl acetate (30 mL) and washed with brine. The organic layer was dried over anhydrous sodium sulfate, filtered, and concentrated under reduced pressure. The residue was purified by silica gel column chromatography (dichloromethane:methanol = 15:1) to afford a white solid, compound B28 (31 mg, 45.1%, racemate). ESI/MS (m/z): 313.0 [M+H]⁺. ¹H NMR (500 MHz, DMSO-*d*₆) δ 12.00 (s, 1H), 7.66 (d, *J =* 8.2 Hz, 1H), 7.59 (d, *J =* 7.1 Hz, 1H), 7.39-7.36 (m, 1H), 5.39-5.35 (m, 1H), 3.12-3.10 (m, 2H), 2.84-2.76 (m, 3H), 2.59-2.56 (m, 2H), 2.43-2.30 (m, 5H), 2.14-2.09 (m, 2H), 1.96-1.90 (m, 1H).

### Example79: Synthesis of Compound B29

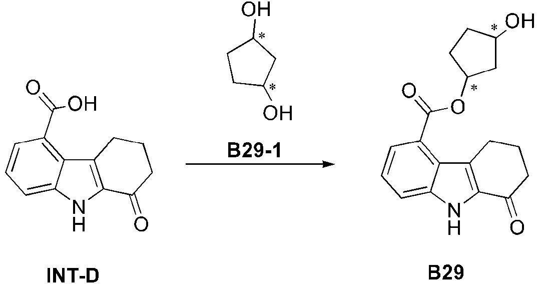

At room temperature, 4-dimethylaminopyridine (26.9 mg, 0.22 mmol) and N,N'-dicyclohexylcarbodiimide (91 mg, 0.44 mmol) were added to a mixture of compound INT-D (50 mg, 0.22 mmol) and compound B29-1 (44 mg, 0.44 mmol) in dichloromethane (1 mL). The reaction mixture was stirred at room temperature for 2 hours. The mixture was diluted with ethyl acetate (30 mL) and sequentially washed with 0.5 M hydrochloric acid (10 mL) and brine. The organic layer was dried over anhydrous sodium sulfate, filtered, and concentrated under reduced pressure. The residue was purified by silica gel column chromatography (ethyl acetate:petroleum ether = 1:6) to afford a white solid, compound B29 (26 mg, 37.7%, mixture of optical isomers). ESI/MS (m/z): 314.0 [M+H]⁺. ¹H NMR (500 MHz, DMSO-*d*₆) δ 11.98 (s, 1H), 7.66-7.63 (m, 1H), 7.60-7.54 (m, 1H), 7.39-7.34 (m, 1H), 5.45-5.23 (m, 1H), 4.69-4.66 (m, 1H), 4.32-4.14 (m, 1H), 3.12-3.07 (m, 2H), 2.59-2.56 (m, 2H), 2.37-2.19 (m, 1H), 2.14-2.09(m, 2H), 2.06-1.90 (m, 3H), 1.81-1.54 (m, 2H).

### Example 80: Synthesis of Compound B30

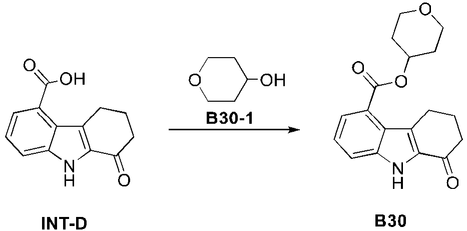

Under a nitrogen atmosphere at 0 °C, diethyl azodicarboxylate (76 mg, 0.44 mmol) was added to a mixture of compound INT-D (50 mg, 0.22 mmol), compound B30-1 (45 mg, 0.44 mmol), and triphenylphosphine (115 mg, 0.44 mmol) in tetrahydrofuran (1 mL). The reaction mixture was stirred at room temperature for 6 hours. After the reaction, the mixture was diluted with ethyl acetate (30 mL) and washed with brine. The organic layer was dried over anhydrous sodium sulfate, filtered, and concentrated under reduced pressure. The residue was purified by silica gel column chromatography (ethyl acetate:petroleum ether = 1:6) to afford a white solid, compound B30 (48 mg, 69.7%). ESI/MS (m/z): 314.1 [M+H]⁺. ¹H NMR (500 MHz, DMSO-*d*₆) δ 12.00 (s, 1H), 7.66 (d, *J =* 8.3 Hz, 1H), 7.63 (d, *J =* 7.3 Hz, 1H), 7.38 (t, *J =* 7.8 Hz, 1H), 5.21-5.13 (m, 1H), 3.91-3.87 (m, 2H), 3.56-3.52 (m, 2H), 3.10 (t, *J =* 6.0 Hz, 2H), 2.59-2.56 (m, 2H), 2.14-2.09 (m, 2H), 2.04-2.02 (m, 2H), 1.77-1.70 (m, 2H).

### Example 81: Synthesis of Compound B31

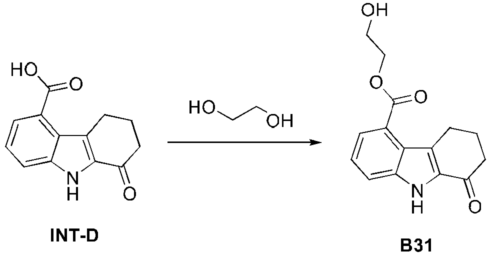

At room temperature, two drops of concentrated sulfuric acid were added to a mixture of compound INT-D (50 mg, 0.22 mmol) in ethylene glycol (0.5 mL). The reaction mixture was stirred at 110 °C for 1 hour. After the reaction, the mixture was diluted with ethyl acetate (30 mL) and washed with brine. The organic layer was dried over anhydrous sodium sulfate, filtered, and concentrated under reduced pressure. The residue was purified by silica gel column chromatography (ethyl acetate:petroleum ether = 1:3) to afford a white solid, compound B31 (28 mg, 46.6%). ESI/MS m/z: 274.0 [M+H]⁺.¹H NMR (500 MHz, DMSO-*d*₆) δ 12.00 (s, 1H), 7.67-7.64 (m, 2H), 7.40-7.36 (m, 1H), 4.93 (t, *J =* 5.5 Hz, 1H), 4.35-4.33 (m, 2H), 3.76-3.73 (m, 2H), 3.12-3.09 (m, 2H), 2.59-2.56 (m, 2H), 2.13-2.08 (m, 2H).

### Example 82: Synthesis of Compound B32

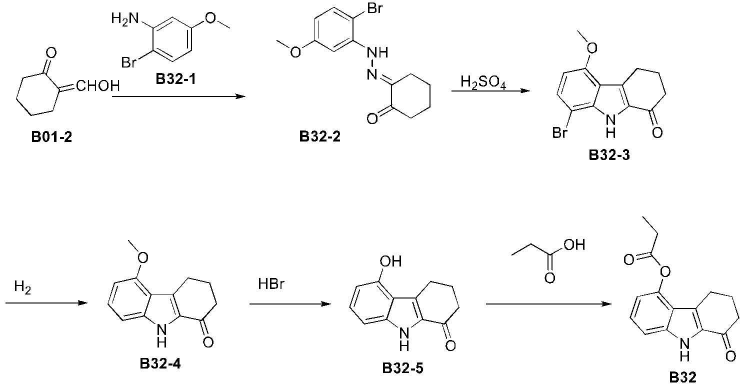

At 0-5 °C, concentrated hydrochloric acid (4.2 mL) and an aqueous solution (10 mL) of sodium nitrite (1.4 g, 20 mmol) were sequentially added to an aqueous solution (30 mL) of compound B32-1 (2.0 g, 10 mmol). The mixture was stirred for 10 minutes to prepare the diazonium salt solution. To a methanol (20 mL) solution of compound B01-2 (1.9 g, 15 mmol), an aqueous solution (20 mL) of sodium acetate (1.8 g, 22 mmol) was added. The system was cooled to 0-5 °C, and the previously prepared diazonium salt solution was added dropwise. After stirred for 30 minutes, the mixture was filtered, and the filter cake was washed with water and dried to afford the crude product B32-2 as a reddish-brown solid (3.0 g, 100%).

At room temperature, 1.8 M sulfuric acid (17 mL) was added to an acetonitrile (30 mL) solution of compound B32-2 (3.0 g, 20 mmol). The reaction mixture was stirred at 80 °C for 4 hours. After cooling to room temperature, the mixture was diluted with ethyl acetate (50 mL) and washed with brine. The organic layer was dried over anhydrous sodium sulfate, filtered, and concentrated under reduced pressure. The residue was purified by silica gel column chromatography (petroleum ether:ethyl acetate = 12:1) to afford an orange-red solid, compound B32-3 (1.1 g, 38.4%).

Under a nitrogen atmosphere, a mixture of methanol (15 mL) and ethyl acetate (15 mL) was added to a reaction flask containing compound B32-3 (1.1 g, 3.7 mmol) and palladium on carbon (10% w/w, 55 mg). The atmosphere was replaced with hydrogen three times using a hydrogen balloon. Triethylamine (0.38 g, 3.7 mmol) was injected, and the mixture was stirred at room temperature for 4 hours. The reaction mixture was diluted with ethyl acetate (50 mL) and filtered through Celite. The filtrate was sequentially washed with 0.5 M hydrochloric acid and brine, dried over anhydrous sodium sulfate, filtered, and concentrated under reduced pressure to afford the crude product B32-4 as a brownish-yellow solid (600 mg, 75%).

A mixture of compound B32-4 (500 mg, 2.32 mmol) in 48% hydrobromic acid (10 mL) was stirred at 105 °C for 1 hour. After cooling to room temperature, the mixture was diluted with water (10 mL) and extracted with ethyl acetate (20 mL × 3). The combined organic layers were washed with brine, dried over anhydrous sodium sulfate, filtered, and concentrated under reduced pressure to afford the crude product B32-5 as a brown solid (400 mg, 85.7%).

At room temperature, 4-dimethylaminopyridine (24 mg, 0.2 mmol) and N,N'-dicyclohexylcarbodiimide (62 mg, 0.3 mmol) were added to a mixture of compound B32-5 (40 mg, 0.2 mmol) and propionic acid (18 mg, 0.24 mmol) in dichloromethane (1 mL). The reaction mixture was stirred at room temperature for 2 hours. The mixture was diluted with ethyl acetate (30 mL) and sequentially washed with 0.5 M hydrochloric acid and brine. The organic layer was dried over anhydrous sodium sulfate, filtered, and concentrated under reduced pressure. The residue was purified by silica gel column chromatography (ethyl acetate:petroleum ether = 1:6) to afford a white solid, compound B32 (26 mg, 50.5%). ESI/MS (m/z): 258.0 [M+H]⁺.¹H NMR (500 MHz, DMSO-*d*₆) δ 11.81 (s, 1H), 7.30-7.26 (m, 2H), 6.81-6.78 (m, 1H), 2.93 (t, *J* = 6.0 Hz, 2H), 2.72 (q, *J =* 7.5 Hz, 2H), 2.55-2.52 (m, 2H), 2.16-2.11 (m, 2H), 1.20 (t, *J =* 7.5 Hz, 3H).

### Example 83: Synthesis of Compound B33

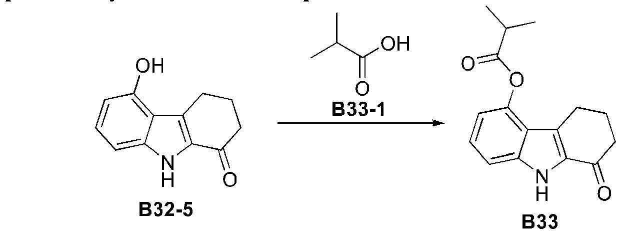

At room temperature, 4-dimethylaminopyridine (24 mg, 0.2 mmol) and N,N'-dicyclohexylcarbodiimide (62 mg, 0.3 mmol) were added to a mixture of compound B32-5 (40 mg, 0.2 mmol) and compound B33-1 (21 mg, 0.24 mmol) in dichloromethane (1 mL). The reaction mixture was stirred for 2 hours. The mixture was diluted with ethyl acetate (30 mL) and sequentially washed with 0.5 M hydrochloric acid and brine. The organic layer was dried over anhydrous sodium sulfate, filtered, and concentrated under reduced pressure. The residue was purified by silica gel column chromatography (ethyl acetate:petroleum ether = 1:6) to afford a white solid, compound B33 (20 mg, 36.9%).ESI/MS (m/z) 272.0 [M+H]⁺.¹H NMR (500 MHz, DMSO-*d*₆) δ 11.82 (s, 1H), 7.30-7.26 (m, 2H), 6.80-6.76 (m, 1H), 2.97-2.91 (m, 3H), 2.56-2.54 (m, 2H), 2.17-2.12 (m, 2H), 1.31 (d, *J =* 7.0 Hz, 6H).

### Example 84: Synthesis of Compound B34

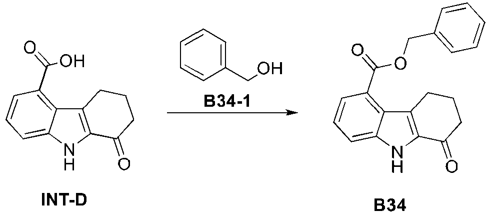

At room temperature, 4-dimethylaminopyridine (26.9 mg, 0.22 mmol) and N,N'-dicyclohexylcarbodiimide (91 mg, 0.44 mmol) were added to a mixture of compound INT-D (50 mg, 0.22 mmol) and compound B34-1 (47 mg, 0.44 mmol) in dichloromethane (1 mL). The reaction mixture was stirred at room temperature for 2 hours. The mixture was diluted with ethyl acetate (30 mL) and sequentially washed with 0.5 M hydrochloric acid (10 mL) and brine. The organic layer was dried over anhydrous sodium sulfate, filtered, and concentrated under reduced pressure. The residue was purified by silica gel column chromatography (ethyl acetate:petroleum ether = 1:6) to afford a white solid, compound B34 (53 mg, 75.4%). ESI/MS (m/z): 320.1 [M+H]⁺.¹H NMR (500 MHz, DMSO-*d*₆) δ 12.01 (s, 1H), 7.66 (dd, *J* = 9.9, 7.8 Hz, 2H), 7.51 (d, *J =* 7.2 Hz, 2H), 7.43 (t, *J =* 7.3 Hz, 2H), 7.41-7.35 (m, 2H), 5.41 (s, 2H), 3.04 (t, *J =* 6.0 Hz, 2H), 2.58-2.53 (m, 2H), 2.12-2.03 (m, 2H).

### Example 85: Synthesis of Compound B35

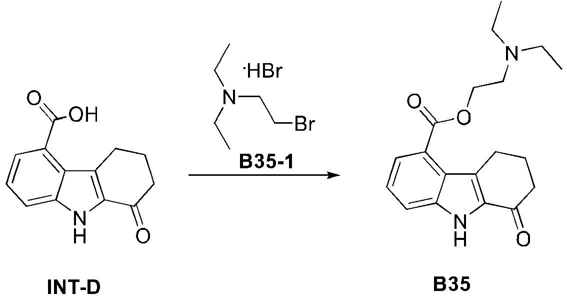

At room temperature, potassium carbonate (91 mg, 0.66 mmol) was added to a mixture of compound INT-D (50 mg, 0.22 mmol) and compound B35-1 (86 mg, 0.33 mmol) in N,N-dimethylformamide (1 mL). The reaction mixture was stirred at room temperature for 2 hours. After the reaction, the mixture was diluted with ethyl acetate (30 mL) and washed with brine. The organic layer was dried over sodium sulfate, filtered, and concentrated under reduced pressure. The residue was purified by silica gel column chromatography (dichloromethane:methanol = 15:1) to afford a white solid, compound B35 (17 mg, 23.5%). ESI/MS (m/z): 329.1 [M+H]⁺.¹H NMR (500 MHz, DMSO-*d*₆) δ 12.01 (s, 1H), 7.67-7.61 (m, 2H), 7.38 (t, *J =* 7.8 Hz, 1H), 4.38 (s, 2H), 3.12 (t, *J =* 5.9 Hz, 2H), 2.79 (s, 2H), 2.53-2.52 (m, 6H), 2.14-2.09 (m, 2H), 0.99 (s, 6H).

### Example 86: Synthesis of Compound B36

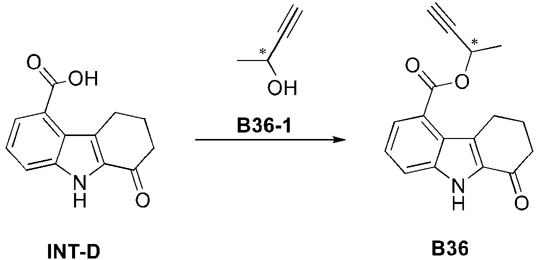

At room temperature, 4-dimethylaminopyridine (26.9 mg, 0.22 mmol) and N,N'-dicyclohexylcarbodiimide (91 mg, 0.44 mmol) were added to a mixture of compound INT-D (50 mg, 0.22 mmol) and compound B36-1 (31 mg, 0.44 mmol, racemate) in dichloromethane (1 mL). The reaction mixture was stirred at room temperature for 2 hours. The mixture was diluted with ethyl acetate (30 mL) and sequentially washed with 0.5 M hydrochloric acid (10 mL) and brine. The organic layer was dried over anhydrous sodium sulfate, filtered, and concentrated under reduced pressure. The residue was purified by silica gel column chromatography (ethyl acetate:petroleum ether = 1:6) to afford a white solid, compound B36 (43 mg, 69.5%, racemate). ESI/MS (m/z): 282.0 [M+H]⁺.¹H NMR (500 MHz, DMSO-*d*₆) δ 12.04 (s, 1H), 7.69 (d, *J =* 8.2 Hz, 1H), 7.61 (d, *J =* 7.3 Hz, 1H), 7.39 (t, *J =* 7.8 Hz, 1H), 5.69-5.64 (m, 1H), 3.63 (d, *J =* 2.1 Hz, 1H), 3.13 (t, *J =* 6.0 Hz, 2H), 2.60-2.57 (m, 2H), 2.15-2.11 (m, 2H), 1.60 (d, *J =* 6.7 Hz, 3H).

### Example 87: Synthesis of Compound B37

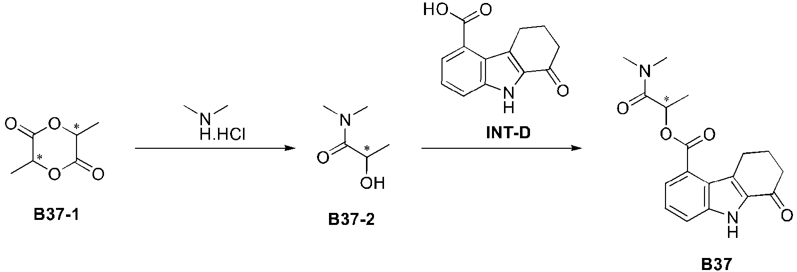

Under a nitrogen atmosphere at 0 °C, sodium hydride (0.32 g, 8 mmol) was added to a tetrahydrofuran (20 mL) mixture of dimethylamine hydrochloride (0.33 g, 4 mmol), and the mixture was stirred for 15 minutes. Compound B37-1 (0.3 g, 2 mmol) was added, and the reaction was stirred at room temperature overnight. The mixture was diluted with ethyl acetate (20 mL), filtered, and concentrated under reduced pressure to afford the crude product B37-2 as a pale yellow liquid (0.35 g, 74.7%, racemate).

At room temperature, 4-dimethylaminopyridine (26.9 mg, 0.22 mmol) and N,N'-dicyclohexylcarbodiimide (91 mg, 0.44 mmol) were added to a mixture of compound INT-D (50 mg, 0.22 mmol) and compound B37-2 (260 mg, 2.2 mmol) in dichloromethane (1 mL). The reaction mixture was stirred at room temperature for 2 hours. The mixture was diluted with ethyl acetate (30 mL) and sequentially washed with 0.5 M hydrochloric acid (10 mL) and brine. The organic layer was dried over anhydrous sodium sulfate, filtered, and concentrated under reduced pressure. The residue was purified by silica gel column chromatography (ethyl acetate:petroleum ether = 1:4) to afford a white solid, compound B37 (20 mg, 27.7%, racemate). ESI/MS (m/z): 329.0 [M+H]⁺.¹H NMR (500 MHz, DMSO-*d*₆) δ 12.01 (s, 1H), 7.68 (d, *J =* 3.7 Hz, 1H), 7.67 (d, *J =* 2.8 Hz, 1H), 7.39 (t, *J =* 7.8 Hz, 1H), 5.66 (q, *J =* 6.7 Hz, 1H), 3.20-3.15 (m, 1H), 3.10-3.04 (m, 4H), 2.88 (s, 3H), 2.59-2.56 (m, 2H), 2.13-2.08 (m, 2H), 1.48 (d, *J =* 6.7 Hz, 3H).

### Example 88: Synthesis of Compound B38

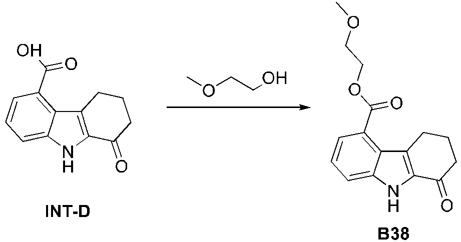

At room temperature, two drops of concentrated sulfuric acid were added to a mixture of compound INT-D (50 mg, 0.22 mmol) in ethylene glycol monomethyl ether (0.5 mL). The reaction mixture was stirred at 120 °C for 2 hours. The mixture was diluted with ethyl acetate (20 mL) and washed with brine. The organic layer was dried over anhydrous sodium sulfate, filtered, and concentrated under reduced pressure. The residue was purified by silica gel column chromatography (ethyl acetate:petroleum ether = 1:5) to afford a white solid, compound B38 (46 mg, 72.8%). ESI/MS m/z: 288.1 [M+H]⁺.¹H NMR (500 MHz, DMSO-*d*₆) δ 12.00 (s, 1H), 7.67 (dd, *J =* 8.3, 0.7 Hz, 1H), 7.63-7.58 (m, 1H), 7.40-7.37 (m, 1H), 4.46-4.44 (m, 2H), 3.70-3.69 (m, 2H), 3.32 (s, 3H), 3.10 (t, *J =* 6.0 Hz, 2H), 2.59-2.57 (m, 2H), 2.14-2.09 (m, 2H).

### Example 89: Synthesis of Compound B39

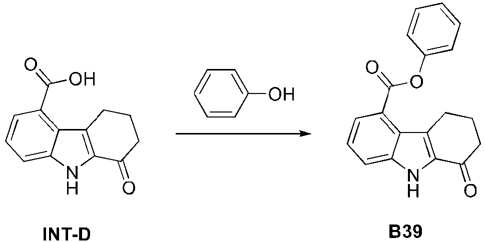

At room temperature, 4-dimethylaminopyridine (26.9 mg, 0.22 mmol) and N,N'-dicyclohexylcarbodiimide (91 mg, 0.44 mmol) were added to a mixture of compound INT-D (50 mg, 0.22 mmol) and phenol (41.4 mg, 0.44 mmol) in dichloromethane (1 mL). The reaction mixture was stirred for 2 hours. The mixture was diluted with ethyl acetate (30 mL) and sequentially washed with 0.5 M hydrochloric acid (10 mL) and brine. The organic layer was dried over anhydrous sodium sulfate, filtered, and concentrated under reduced pressure. The residue was purified by silica gel column chromatography (ethyl acetate:petroleum ether = 1:6) to afford a white solid, compound B39 (51 mg, 75.9%). ESI/MS (m/z): 306.0 [M+H]⁺.¹H NMR (500 MHz, DMSO-*d*₆) δ 12.11 (s, 1H), 7.89 (dd, *J =* 7.3, 0.9 Hz, 1H), 7.76 (dd, *J =* 8.3, 0.9 Hz, 1H), 7.53-7.50 (m, 2H), 7.48-7.45 (m, 1H), 7.37-7.33 (m, 3H), 3.15 (t, *J =* 6.0 Hz, 2H), 2.59-2.57 (m, 2H), 2.13-2.08 (m, 2H).

### Example 90: Synthesis of Compound B40

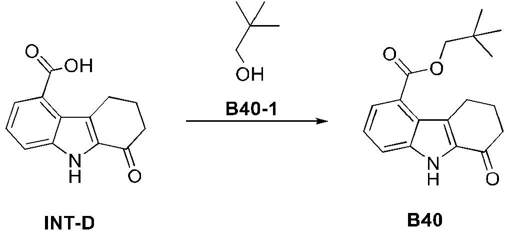

At room temperature, 4-dimethylaminopyridine (26.9 mg, 0.22 mmol) and N,N'-dicyclohexylcarbodiimide (91 mg, 0.44 mmol) were added to a mixture of compound INT-D (50 mg, 0.22 mmol) and compound B40-1 (39 mg, 0.44 mmol) in dichloromethane (1 mL). The reaction mixture was stirred for 2 hours. The mixture was diluted with ethyl acetate (30 mL) and sequentially washed with 0.5 M hydrochloric acid (10 mL) and brine. The organic layer was dried over anhydrous sodium sulfate, filtered, and concentrated under reduced pressure. The residue was purified by silica gel column chromatography (ethyl acetate:petroleum ether = 1:6) to afford a white solid, compound B40 (35 mg, 53.1%). ESI/MS (m/z): 300.0 [M+H]⁺.¹H NMR (500 MHz, DMSO-*d*₆) δ 12.01 (s, 1H), 7.67 (dd, *J =* 8.3, 0.8 Hz, 1H), 7.64 (dd, *J =* 7.3, 0.8 Hz, 1H), 7.41-7.38 (m, 1H), 4.05 (s, 2H), 3.10 (t, *J =* 6.0 Hz, 2H), 2.59-2.56 (m, 2H), 2.13-2.08 (m, 2H), 1.02 (s, 9H).

### Example91: Synthesis of Compound B41

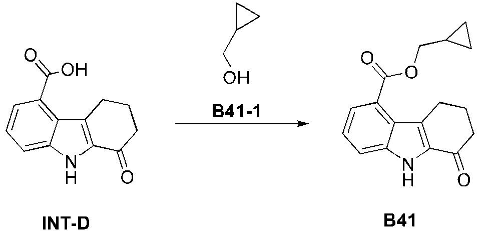

At room temperature, 4-dimethylaminopyridine (26.9 mg, 0.22 mmol) and N,N'-dicyclohexylcarbodiimide (91 mg, 0.44 mmol) were added to a mixture of compound INT-D (50 mg, 0.22 mmol) and compound B41-1 (32 mg, 0.44 mmol) in dichloromethane (1 mL), and the mixture was stirred at room temperature for 2 hours. The reaction mixture was diluted with ethyl acetate (30 mL) and sequentially washed with 0.5M hydrochloric acid aqueous solution (10 mL) and saturated sodium chloride aqueous solution. It was then dried over anhydrous sodium sulfate, filtered, and concentrated under reduced pressure. The residue was purified by silica gel column chromatography (ethyl acetate:petroleum ether = 1:6) to afford compound B41 as a white solid (53 mg, 85%). ESI/MS (m/z) 284.0 [M+H]⁺. ¹H NMR (500 MHz, DMSO-*d*₆) δ 11.99 (s, 1H), 7.67-7.63 (m, 1H), 7.60 (d, *J =* 7.2 Hz, 1H), 7.40-7.35 (m, 1H), 4.17 (d, *J =* 7.3 Hz, 2H), 3.11 (t, *J =* 6.0 Hz, 2H), 2.57-2.54 (m, 2H), 2.14-2.09 (m, 2H), 1.29-1.25 (m, 1H), 0.61-0.58 (m, 2H), 0.40-0.37 (m, 2H).

### Example 92: Synthesis of Compound B42

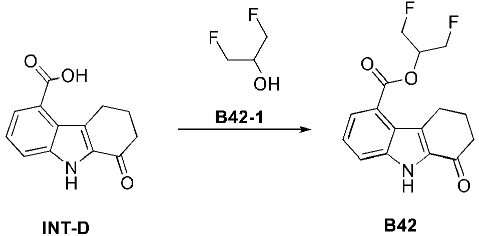

At room temperature, 4-dimethylaminopyridine (26.9 mg, 0.22 mmol) and N,N'-dicyclohexylcarbodiimide (91 mg, 0.44 mmol) were added to a mixture of compound INT-D (50 mg, 0.22 mmol) and compound B42-1 (40 mg, 0.44 mmol) in dichloromethane (1 mL). The mixture was stirred for 2 hours. The reaction mixture was diluted with ethyl acetate (30 mL) and sequentially washed with 0.5M hydrochloric acid aqueous solution (10 mL) and saturated sodium chloride aqueous solution. It was then dried over anhydrous sodium sulfate, filtered, and concentrated under reduced pressure. The residue was purified by silica gel column chromatography (ethyl acetate:petroleum ether = 1:6) to afford compound B42 as a white solid (27 mg, 39.9%). ESI/MS (m/z) 308.0 [M+H]⁺. ¹H NMR (500 MHz, DMSO-*d*₆) δ 12.06 (s, 1H), 7.71-7.67 (m, 2H), 7.43-7.39 (m, 1H), 5.64-5.52 (m, 1H), 4.91-4.82 (m, 2H), 4.81-4.72 (m, 2H), 3.12 (t, *J =* 6.0 Hz, 2H), 2.60-2.57 (m, 2H), 2.15-2.09 (m, 2H).

### Example 93: Synthesis of Compound B43

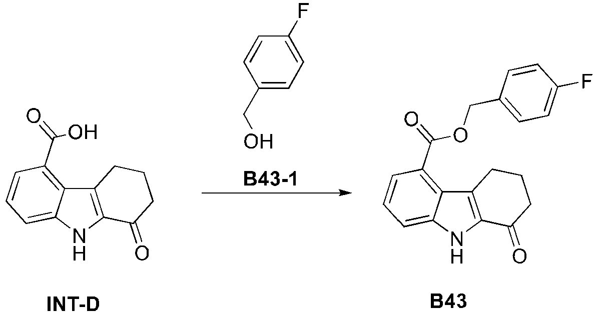

At room temperature, 4-dimethylaminopyridine (26.9 mg, 0.22 mmol) and N,N'-dicyclohexylcarbodiimide (91 mg, 0.44 mmol) were added to a mixture of compound INT-D (50 mg, 0.22 mmol) and compound B43-1 (47 mg, 0.44 mmol) in dichloromethane (1 mL). The mixture was stirred for 2 hours. The reaction mixture was diluted with ethyl acetate (30 mL) and sequentially washed with 0.5M hydrochloric acid aqueous solution (10 mL) and saturated sodium chloride aqueous solution. It was then dried over anhydrous sodium sulfate, filtered, and concentrated under reduced pressure. The residue was purified by silica gel column chromatography (ethyl acetate:petroleum ether = 1:6) to afford compound B43 as a white solid (45 mg, 60.6%). ESI/MS (m/z) 338.0 [M+H]⁺.¹H NMR (500 MHz, DMSO-*d*₆) δ 12.01 (s, 1H), 7.66 (d, *J =* 8.3 Hz, 1H), 7.63 (d, *J =* 7.3 Hz, 1H), 7.59-7.56 (m, 2H), 7.39-7.36 (m, 1H), 7.27-7.23 (m, 2H), 5.39 (s, 2H), 3.02 (t, *J =* 6.0 Hz, 2H), 2.57-2.54 (m, 2H), 2.09-2.04 (m, 2H).

### Example 94: Synthesis of Compound B44

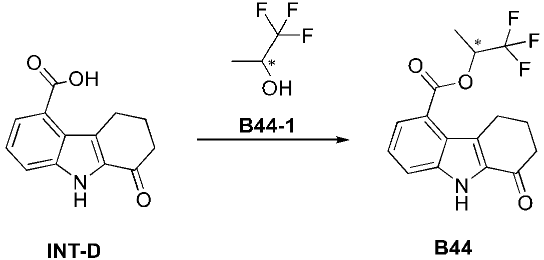

At room temperature, 4-dimethylaminopyridine (26.9 mg, 0.22 mmol) and N,N'-dicyclohexylcarbodiimide (91 mg, 0.44 mmol) were added to a mixture of compound INT-D (50 mg, 0.22 mmol) and compound B44-1 (50 mg, 0.44 mmol, racemate) in dichloromethane (1 mL). The mixture was stirred for 2 hours. The reaction mixture was diluted with ethyl acetate (30 mL) and sequentially washed with 0.5M hydrochloric acid aqueous solution (10 mL) and saturated sodium chloride aqueous solution. It was then dried over anhydrous sodium sulfate, filtered, and concentrated under reduced pressure. The residue was purified by silica gel column chromatography (ethyl acetate:petroleum ether = 1:6) to afford compound B44 as a white solid (44 mg, 61.5%, racemate). ESI/MS (m/z) 326.0 [M+H]⁺.¹H NMR (500 MHz, DMSO-*d*₆) 12.10 (s, 1H), 7.73 (d, *J =* 8.3 Hz, 1H), 7.67 (d, *J =* 7.2 Hz, 1H), 7.47-7.39 (m, 1H), 5.82-5.73 (m, 1H), 3.14-3.06 (m, 2H), 2.62-2.57 (m, 2H), 2.15-2.10 (m, 2H), 1.55 (d, *J =* 6.6 Hz, 3H).

### Example95: Synthesis of Compound B45

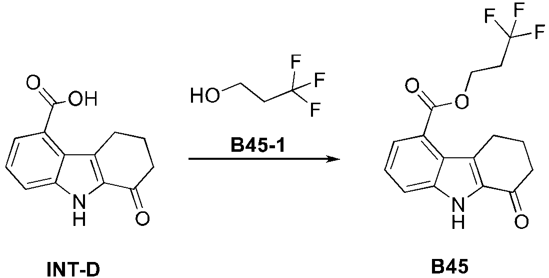

To a mixture of compound INT-D (50 mg, 0.22 mmol) and compound B45-1 (47 mg, 0.44 mmol) in dichloromethane (1 mL) were added 4-dimethylaminopyridine (26.9 mg, 0.22 mmol) and N,N'-dicyclohexylcarbodiimide (91 mg, 0.44 mmol). The mixture was stirred at room temperature for 2 hours. The reaction mixture was diluted with ethyl acetate (30 mL), then sequentially washed with 0.5M hydrochloric acid aqueous solution (10 mL) and saturated sodium chloride aqueous solution. The organic layer was dried over anhydrous sodium sulfate, filtered, and concentrated under reduced pressure. The residue was purified by silica gel column chromatography (ethyl acetate:petroleum ether = 1:6) to afford compound B45 as a white solid (42 mg, 58.7%). ESI/MS (m/z) 326.0 [M+H]⁺. ¹H NMR (500 MHz, DMSO-*d*₆) δ 12.03 (s, 1H), 7.68 (dd, *J =* 8.3, 0.6 Hz, 1H), 7.62 (d, *J =* 7.3 Hz, 1H), 7.42-7.37 (m, 1H), 4.55 (t, *J =* 6.0 Hz, 2H), 3.11 (t, *J =* 6.0 Hz, 2H), 2.91-2.78 (m, 2H), 2.61-2.56 (m, 2H), 2.17-2.06 (m, 2H).

### Example96: Synthesis of Compound B46

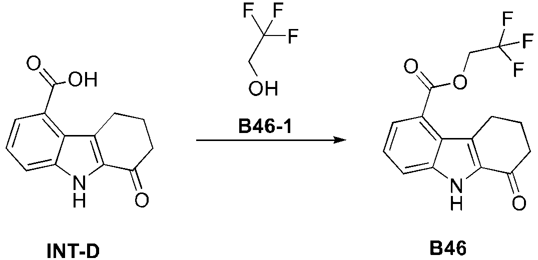

To a mixture of compound INT-D (50 mg, 0.22 mmol) and compound B46-1 (44 mg, 0.44 mmol) in dichloromethane (1 mL) were added 4-dimethylaminopyridine (26.9 mg, 0.22 mmol) and N,N'-dicyclohexylcarbodiimide (91 mg, 0.44 mmol). The mixture was stirred at room temperature for 2 hours. The reaction mixture was diluted with ethyl acetate (30 mL), then sequentially washed with 0.5M hydrochloric acid aqueous solution (10 mL) and saturated sodium chloride aqueous solution. The organic layer was dried over anhydrous sodium sulfate, filtered, and concentrated under reduced pressure. The residue was purified by silica gel column chromatography (ethyl acetate:petroleum ether = 1:6) to afford compound B46 as a white solid (59 mg, 86.2%). ESI/MS (m/z) 312.0 [M+H]⁺.¹H NMR (500 MHz, DMSO-*d*₆) δ 12.11 (s, 1H), 7.74 (dd, *J =* 8.3, 0.8 Hz, 1H), 7.69 (dd, *J =* 7.3, 0.7 Hz, 1H), 7.44-7.41 (m, 1H), 5.06 (q, *J =* 9.1 Hz, 2H), 3.11 (t, *J =* 6.0 Hz, 2H), 2.60-2.58 (m, 2H), 2.14-2.09 (m, 2H).

### Example97: Synthesis of Compound B47

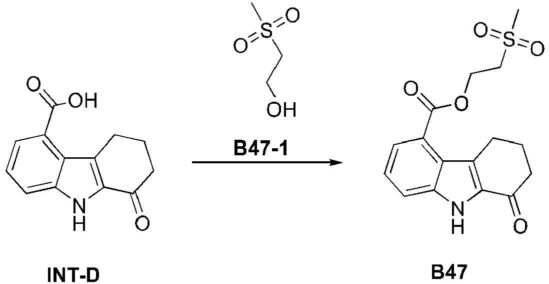

To a mixture of compound INT-D (50 mg, 0.22 mmol) and compound B47-1 (55 mg, 0.44 mmol) in dichloromethane (1 mL) were added 4-dimethylaminopyridine (26.9 mg, 0.22 mmol) and N,N'-dicyclohexylcarbodiimide (91 mg, 0.44 mmol). The mixture was stirred at room temperature for 2 hours. The reaction mixture was diluted with ethyl acetate (30 mL), then sequentially washed with 0.5M hydrochloric acid aqueous solution and saturated sodium chloride aqueous solution. The organic layer was dried over anhydrous sodium sulfate, filtered, and concentrated under reduced pressure. The residue was purified by silica gel column chromatography (ethyl acetate:petroleum ether = 1:1) to afford compound B47 as a white solid (17 mg, 23.0%). ESI/MS (m/z) 336.0 [M+H]⁺. ¹H NMR (500 MHz, DMSO-*d*₆) δ 12.03 (s, 1H), 7.69 (d, *J* = 3.2 Hz, 1H), 7.67 (d, *J =* 2.3 Hz, 1H), 7.39 (t, *J =* 7.8 Hz, 1H), 4.69 (t, *J =* 5.9 Hz, 2H), 3.70 (t, *J =* 5.9 Hz, 2H), 3.13 (t, *J =* 6.0 Hz, 2H), 3.07 (s, 3H), 2.60-2.57 (m, 2H), 2.14-2.09 (m, 2H).

### Example 98: Synthesis of Compound B48

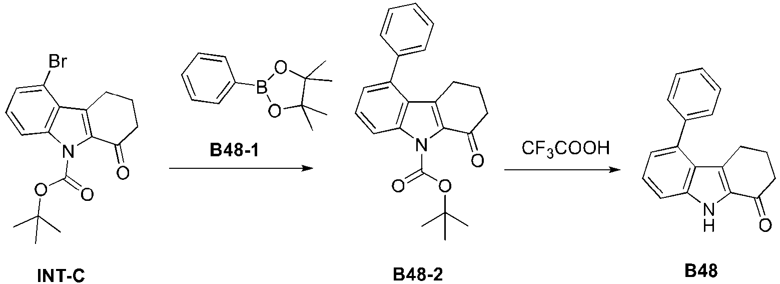

Under nitrogen protection, to a mixture of compound INT-C (109.3 mg, 0.300 mmol) and 1,4-dioxane (3 mL) were sequentially added compound B48-1 (110.2 mg, 0.540 mmol), [1,1'-bis(diphenylphosphino)ferrocene]dichloropalladium(II) dichloromethane complex (12.2 mg, 0.015 mmol), and potassium carbonate (82.9 mg, 0.600 mmol). The mixture was stirred at 80 °C for 3 hours. After cooling to room temperature, the reaction mixture was diluted with ethyl acetate (100 mL), and the resulting mixture was filtered through celite. The filtrate was concentrated under reduced pressure to afford crude compound B48-2. To the crude compound B48-2 was added a mixture of trifluoroacetic acid (0.3 mL) and dichloromethane (3 mL), and the mixture was stirred at room temperature for 10 minutes. The reaction mixture was diluted with ethyl acetate (100 mL), then sequentially washed with saturated sodium bicarbonate aqueous solution (30 mL) and saturated sodium chloride aqueous solution (30 mL). The organic layer was dried over anhydrous sodium sulfate. After filtration and concentration under reduced pressure, the crude product was purified by reverse-phase silica gel column chromatography (50-90% acetonitrile/water) to afford compound B48 as a light yellow solid (41.0 mg, 52.30%). ESI/MS (m/z) 262.1 [M+H]⁺.¹H NMR (500 MHz, DMSO-*d*₆) δ 11.77 (s, 1H), 7.49-7.45 (m, 2H), 7.45-7.40 (m, 4H), 7.35-7.31 (m, 1H), 6.93 (dd, *J =* 7.0, 0.8 Hz, 1H), 2.4-2.47 (m, 2H), 2.40 (t, *J=* 6.0 Hz, 2H), 1.95-1.88 (m, 2H).

### Example 99: Synthesis of Compound B49

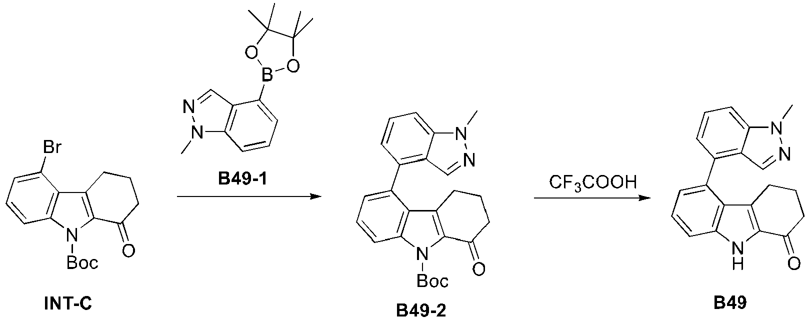

Under nitrogen protection, to a mixture of compound INT-C (72.8 mg, 0.200 mmol) and 1,4-dioxane (3 mL) were sequentially added compound B49-1 (61.9 mg, 0.240 mmol), [1,1'-bis(diphenylphosphino)ferrocene]dichloropalladium(II) dichloromethane complex (8.0 mg, 0.010 mmol), and potassium carbonate (41.5 mg, 0.300 mmol). The mixture was stirred at 80 °C for 3 hours. After cooling to room temperature, the reaction mixture was diluted with ethyl acetate (100 mL), and the resulting mixture was filtered through celite. The filtrate was concentrated under vacuum to afford crude compound B49-2. To the crude compound B49-2 was added a mixture of trifluoroacetic acid (0.3 mL) and dichloromethane (3 mL), and the mixture was stirred at room temperature for 10 minutes. The reaction mixture was diluted with ethyl acetate (100 mL), then sequentially washed with saturated sodium bicarbonate aqueous solution (30 mL) and saturated sodium chloride aqueous solution (30 mL). The organic layer was dried over anhydrous sodium sulfate. After filtration and concentration under reduced pressure, the crude product was purified by reverse-phase silica gel column chromatography (10-70% acetonitrile/water) to afford compound B49 as a light yellow solid (30.0 mg, 47.56%). ESI/MS (m/z) 316.1 [M+H]⁺.MR (500 MHz, DMSO-*d*₆) δ 11.82 (s, 1H), 7.68 (d, *J =* 8.5 Hz, 1H), 7.66 (s, 1H), 7.52-7.47 (m, 2H), 7.40-7.37 (m, 1H), 7.11 (d, *J=* 6.9 Hz, 1H), 7.07 (d, *J=* 7.0 Hz, 1H), 4.10 (s, 3H), 2.44 (t, *J =* 5.8 Hz, 2H), 2.27 (br, 1H), 2.06-1.90 (m, 1H), 1.81 (br, 2H).

### Example 100: Synthesis of Compound B50

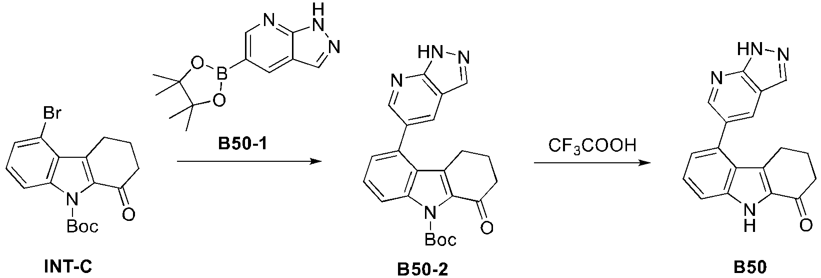

Under nitrogen protection, to a mixture of compound INT-C (109.3 mg, 0.300 mmol) and 1,4-dioxane (3 mL) were sequentially added B50-1 (220.6 mg, 0.900 mmol), [1,1'-bis(diphenylphosphino)ferrocene]dichloropalladium(II) dichloromethane complex (12.2 mg, 0.015 mmol), and potassium carbonate (124.4 mg, 0.900 mmol). The mixture was stirred at 80 °C for 8 hours. After cooling to room temperature, the reaction mixture was diluted with ethyl acetate (100 mL), and the resulting mixture was filtered through celite. The filtrate was concentrated under vacuum to afford crude compound B50-2. To the crude compound B50-2 was added a mixture of trifluoroacetic acid (0.3 mL) and dichloromethane (3 mL), and the mixture was stirred at room temperature for 10 minutes. The reaction mixture was diluted with ethyl acetate (100 mL), then sequentially washed with saturated sodium bicarbonate aqueous solution (30 mL) and saturated sodium chloride aqueous solution (30 mL). The organic layer was dried over anhydrous sodium sulfate. After filtration and concentration under reduced pressure, the crude product was purified by reverse-phase silica gel column chromatography (10-50% acetonitrile/water) to afford compound B50 as a light yellow solid (13.0 mg, 14.33%). ESI/MS (m/z) 303.0 [M+H]⁺.¹H NMR (500 MHz, DMSO-*d*₆) δ 13.76 (s, 1H), 11.84 (s, 1H), 8.60 (d, *J =* 2.0 Hz, 1H), 8.28 (d, *J =* 2.0 Hz, 1H), 8.21 (s, 1H), 7.47 (d, *J=* 8.2 Hz, 1H), 7.40-7.36 (m, 1H), 7.03 (d, *J =* 7.0 Hz, 1H), 2.49-2.47 (m, 2H), 2.38 (t, *J =* 6.0 Hz, 2H), 1.93-1.87 (m, 2H).

### Example 101: Synthesis of Compound B51

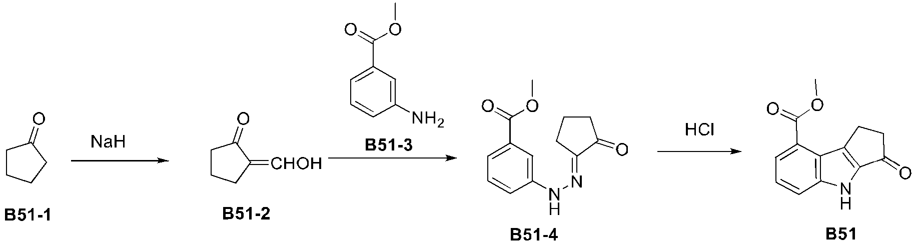

At 0 °C, to a solution of compound B51-1 (2 g, 23.776 mmol) and ethyl formate (3.52 g, 47.552 mmol) in toluene (40 mL) was added 60% sodium hydride (0.86 g, 35.664 mmol). The mixture was stirred at room temperature for 2 hours. After the reaction was complete, concentrated hydrochloric acid was added to adjust the pH to 1, and the mixture was extracted with ethyl acetate (3 × 40 mL). The combined organic phases were washed with saturated sodium chloride aqueous solution (40 mL), dried over anhydrous sodium sulfate, filtered, and concentrated under reduced pressure. The residue was purified by silica gel column chromatography (petroleum ether:ethyl acetate = 1:1) to afford compound B51-2 as a brown oil (2.5 g, 73.05%). ESI/MS (m/z): 113.1 [M+H]⁺.

At 0 °C, to a solution of compound B51-3 (2.31 g, 15.284 mmol) in concentrated hydrochloric acid (5.7 mL) and water (80 mL) was slowly added a solution of sodium nitrite (2.11 g, 30.568 mmol) in water (30 mL). Then, at 0 °C, a solution of compound B51-2 (2.2 g, 15.284 mmol) and sodium acetate (2.76 g, 33.625 mmol) in methanol (80 mL) and water (80 mL) was slowly added to the above mixture. The reaction was stirred at room temperature for 1 hour. The mixture was filtered, and the filter cake was washed with water (50 mL) and dried to afford compound B51-4 as a red solid (4.1 g, 54.46%). ESI/MS (m/z): 247.1 [M+H]⁺.

At room temperature, to a solution of compound B51-4 (3.6 g, 7.309 mmol) in methanol (100 mL) was added concentrated hydrochloric acid (3.5 mL). The mixture was stirred at 60 °C overnight. After cooling to room temperature, the reaction mixture was adjusted to pH = 7 with saturated sodium carbonate aqueous solution and extracted with ethyl acetate (3 × 150 mL). The combined organic phases were washed with saturated sodium chloride aqueous solution (150 mL), dried over anhydrous sodium sulfate, filtered, and concentrated under reduced pressure. The residue was purified by reverse-phase preparative chromatography (mobile phase A: water (0.1% formic acid); mobile phase B: acetonitrile) to afford compound B51 as a yellow solid (33.8 mg, 1.97%). ESI/MS (m/z): 230.1 [M+H]⁺.

¹H NMR (400 MHz, DMSO-*d*₆) δ 12.06 (s, 1H), 7.80 (d, *J =* 7.3 Hz, 1H), 7.72 (d, *J* = 8.3 Hz, 1H), 7.45 (t, *J =* 7.8 Hz, 1H), 3.92 (s, 3H), 3.26-3.21 (m, 2H), 2.91-2.84 (m, 2H).

### Example 102: Synthesis of Compound B52

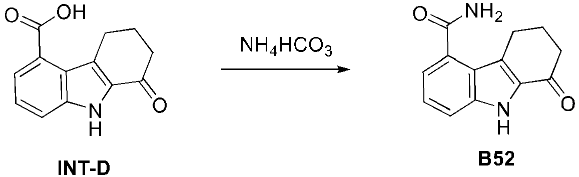

To a mixture of compound INT-D (100 mg, 0.431 mmol) and ammonium bicarbonate (102 mg, 1.293 mmol) in N,N-dimethylformamide (4 mL) were added triethylamine (180 µL, 1.293 mmol) and 2-(7-aza-1H-benzotriazol-1-yl)-1,1,3,3-tetramethyluronium hexafluorophosphate (197 mg, 0.517 mmol). The mixture was stirred at room temperature for 2 hours. After the reaction was complete, water (5 mL) was added, and the mixture was filtered. The filter cake was washed with water (2 × 2 mL) and purified by reverse-phase preparative chromatography (mobile phase A: water (10 mmol/L ammonium bicarbonate); mobile phase B: acetonitrile) to afford compound B52 as a white solid (9.1 mg, 8.97%). ESI/MS (m/z): 229.1 [M+H]⁺.¹H NMR (400 MHz, DMSO-*d*₆) δ 11.77 (s, 1H), 7.84 (s, 1H), 7.47 (dd, *J =* 8.3, 1.0 Hz, 1H), 7.41 (s, 1H), 7.29 (dd, *J =* 8.3, 7.1 Hz, 1H), 7.17 (dd, *J =* 7.1, 1.1 Hz, 1H), 2.99 (t, *J =* 6.0 Hz, 2H), 2.54 (t, *J =* 6.4 Hz, 2H), 2.10 (p, *J =* 6.1 Hz, 2H).

### Example 103: Synthesis of Compound B53

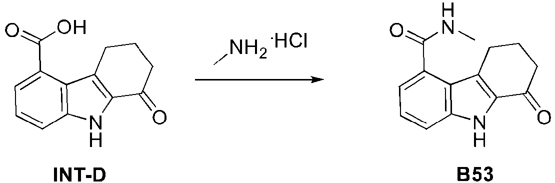

At room temperature, to a solution of compound INT-D (130 mg, 0.561 mmol) and methylamine hydrochloride (45 mg, 0.673 mmol) in N,N-dimethylformamide (4 mL) were added 2-(7-aza-1H-benzotriazol-1-yl)-1,1,3,3-tetramethyluronium hexafluorophosphate (256 mg, 0.673 mmol) and N,N-diisopropylethylamine (488 µL, 2.805 mmol). The mixture was stirred for 2 hours. After the reaction was complete, water (10 mL) was added, and the mixture was extracted with ethyl acetate (3 × 20 mL). The combined organic phases were washed with saturated sodium chloride aqueous solution (10 mL), dried over anhydrous sodium sulfate, filtered, and concentrated under reduced pressure. The residue was purified by reverse-phase preparative chromatography (mobile phase A: water (0.1% formic acid); mobile phase B: acetonitrile) to afford compound B53 as a white solid (52.6 mg, 38.44%). ESI/MS (m/z): 243.2 [M+H]⁺.¹H NMR (300 MHz, DMSO-*d*₆) δ 11.80 (s, 1H), 8.31 (q, *J* = 4.4 Hz, 1H), 7.47 (dd, *J =* 8.4, 1.0 Hz, 1H), 7.30 (dd, *J =* 8.3, 7.1 Hz, 1H), 7.11 (dd, *J* = 7.1, 1.0 Hz, 1H), 2.90 (t, *J =* 6.0 Hz, 2H), 2.80 (d, *J =* 4.6 Hz, 3H), 2.54 (t, *J =* 6.3 Hz, 2H), 2.16-2.03 (m, 2H).

### Example 104: Synthesis of Compound B54

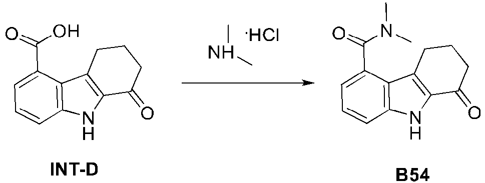

At room temperature, to a solution of compound INT-D (130 mg, 0.561 mmol) and dimethylamine hydrochloride (55 mg, 0.673 mmol) in N,N-dimethylformamide (4 mL) were added 2-(7-aza-1H-benzotriazol-1-yl)-1,1,3,3-tetramethyluronium hexafluorophosphate (256 mg, 0.673 mmol) and N,N-diisopropylethylamine (488 µL, 2.805 mmol). The mixture was stirred for 2 hours. After the reaction was complete, water (15 mL) was added, and the mixture was extracted with ethyl acetate (3 × 20 mL). The combined organic phases were washed with saturated sodium chloride aqueous solution (15 mL), dried over anhydrous sodium sulfate, filtered, and concentrated under reduced pressure. The residue was purified by reverse-phase preparative chromatography (mobile phase A: water (0.1% formic acid); mobile phase B: acetonitrile) to afford compound B54 as a white solid (97.7 mg, 67.49%). ESI/MS (m/z): 257.1 [M+H]⁺.¹H NMR (400 MHz, DMSO-*d*₆) δ 11.82 (s, 1H), 7.43 (dd, *J =* 8.4, 1.0 Hz, 1H), 7.32 (dd, *J =* 8.4, 7.0 Hz, 1H), 6.93 (dd, *J =* 7.1, 1.0 Hz, 1H), 3.06 (s, 3H), 2.75 (s, 3H), 2.74-2.71 (m, 2H), 2.59-2.53 (m, 2H), 2.11 (p, *J =* 6.1 Hz, 2H).

### Example 105: Synthesis of Compound B55

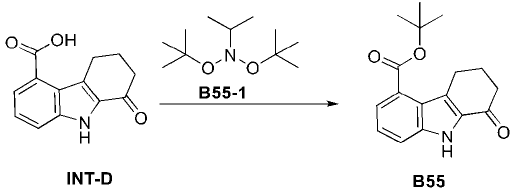

At 100 °C, a solution of compound B55-1 (1.1 g, 5.547 mmol) in toluene was added dropwise to a solution of compound INT-D (200 mg, 0.860 mmol) in toluene (10 mL). The mixture was stirred for 1 hour. After cooling to room temperature, the reaction was quenched with water (100 mL) and extracted with ethyl acetate (3 × 50 mL). The combined organic phases were washed with saturated sodium chloride aqueous solution (30 mL), dried over anhydrous sodium sulfate, and concentrated under reduced pressure. The residue was purified by silica gel column chromatography (petroleum ether:ethyl acetate = 5:1) to afford compound B55 as a white solid (10 mg, 4.00%). ESI/MS (m/z): 286.2 [M+H]⁺.

¹H NMR (400 MHz, DMSO-*d*₆) δ 11.94 (s, 1H), 7.60 (d, *J =* 8.2 Hz, 1H), 7.48 (d, *J* = 7.3 Hz, 1H), 7.34 (dd, *J =* 8.3, 7.3 Hz, 1H), 3.08 (t, *J =* 6.0 Hz, 2H), 2.57 (t, *J =* 6.4 Hz, 2H), 2.12 (p, *J =* 6.2 Hz, 2H), 1.59 (s, 9H).

### Example 106: Synthesis of Compound B56

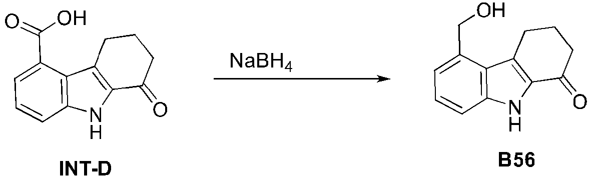

At 0 °C, a solution of N,N'-carbonyldiimidazole (418.48 mg, 2.580 mmol) in tetrahydrofuran was added dropwise to a solution of compound INT-D (200 mg, 0.860 mmol) in tetrahydrofuran (10 mL). The mixture was stirred at room temperature for 1 hour. Then, a solution of sodium borohydride (48.82 mg, 1.290 mmol) in water (1 mL) was added at room temperature, and the mixture was stirred for an additional 1 hour before being quenched with water. The mixture was extracted with dichloromethane (2 × 30 mL). The combined organic phases were washed with saturated sodium chloride aqueous solution (20 mL), dried over anhydrous sodium sulfate, and directly concentrated under reduced pressure. The residue was purified by silica gel column chromatography (petroleum ether:ethyl acetate = 1:1) to afford compound B56 as a white solid. ESI/MS (m/z): 216.2 [M+H]⁺.¹H NMR (400 MHz, DMSO-*d₆*) δ 11.58 (s, 1H), 7.29 (d, *J =* 8.3 Hz, 1H), 7.22 (d, *J =* 8.3, 6.9 Hz, 1H), 7.04 (dd, J = 6.9, 1.1 Hz, 1H), 5.18 (t, *J* = 5.4 Hz, 1H), 4.84 (d, *J =* 5.4 Hz, 2H), 3.17 (t, *J =* 6.0 Hz, 2H), 2.55-2.51 (m, 2H), 2.16-2.10 (m, 2H).

### Example 107: Synthesis of Compound B57

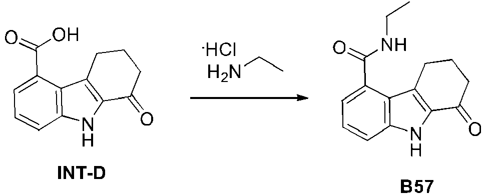

At room temperature, to a solution of compound INT-D (130 mg, 0.561 mmol) and ethylamine hydrochloride (55 mg, 0.673 mmol) in N,N-dimethylformamide (4 mL) were added 2-(7-aza-1H-benzotriazol-1-yl)-1,1,3,3-tetramethyluronium hexafluorophosphate (256 mg, 0.673 mmol) and N,N-diisopropylethylamine (488 µL, 2.805 mmol). The mixture was stirred for 2 hours. The reaction was quenched with water (15 mL) and extracted with ethyl acetate (3 × 20 mL). The combined organic phases were washed with saturated sodium chloride aqueous solution (15 mL), dried over anhydrous sodium sulfate, filtered, and concentrated under reduced pressure. The residue was purified by reverse-phase preparative chromatography (mobile phase A: water (0.1% formic acid); mobile phase B: acetonitrile) to afford compound B57 as a white solid (89.9 mg, 62.23%). ESI/MS (m/z): 257.1 [M+H]⁺. ¹H NMR (400 MHz, DMSO-*d*₆) δ 11.78 (s, 1H), 8.36 (t, *J=* 5.5 Hz, 1H), 7.47 (d, *J=* 8.3 Hz, 1H), 7.30 (t, *J* = 7.7 Hz, 1H), 7.10 (d, *J =* 7.0 Hz, 1H), 3.32-3.26 (m, 2H), 2.92 (t, *J=* 6.0 Hz, 2H), 2.54 (t, *J =* 6.3 Hz, 2H), 2.09 (p, *J =* 6.1 Hz, 2H), 1.15 (t, *J =* 7.2 Hz, 3H).

### Example108: Synthesis of Compound B58

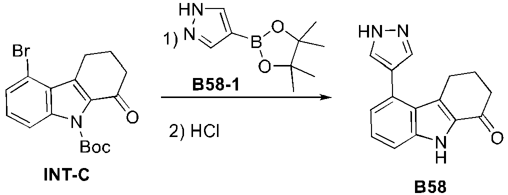

Under a nitrogen atmosphere, to a solution of INT-C (200 mg, 0.538 mmol) and compound B58-1 (209 mg, 1.076 mmol) in 1,4-dioxane (8 mL) and water (1.6 mL) were added cesium carbonate (351 mg, 1.076 mmol) and [1,1'-bis(diphenylphosphino)ferrocene]dichloropalladium(II) dichloromethane complex (66 mg, 0.081 mmol). The reaction mixture was stirred at 100 °C overnight. After cooling to room temperature, the reaction was quenched with water (30 mL) and extracted with ethyl acetate (3 × 20 mL). The combined organic phases were washed with saturated sodium chloride aqueous solution (3 × 20 mL), dried over anhydrous sodium sulfate, and concentrated under reduced pressure. The residue was purified by silica gel column chromatography (petroleum ether:ethyl acetate = 1:9) to afford the intermediate compound. The intermediate was redissolved in a 4M hydrogen chloride solution in 1,4-dioxane (8 mL, 263.302 mmol) and stirred at room temperature for 2 hours. The reaction mixture was directly concentrated under reduced pressure and purified by reverse-phase preparative chromatography (mobile phase A: water (0.1% formic acid); mobile phase B: acetonitrile) to afford compound B58 as a white solid (26.7 mg, 19.55%). ESI/MS (m/z): 252.2 [M+H]⁺. ¹H NMR (400 MHz, DMSO-*d*₆) δ 12.97 (s, 1H), 11.67 (s, 1H), 7.92 (s, 1H), 7.66 (s, 1H), 7.34 (dd, *J =* 8.3, 1.1 Hz, 1H), 7.27 (dd, *J =* 8.3, 7.0 Hz, 1H), 6.93 (dd, *J =* 7.1, 1.1 Hz, 1H), 2.71 (t, *J =* 5.9 Hz, 2H), 2.52-2.49 (m, 2H), 2.00 (p, *J =* 6.2 Hz, 2H).

### Example 109: Synthesis of Compound B59

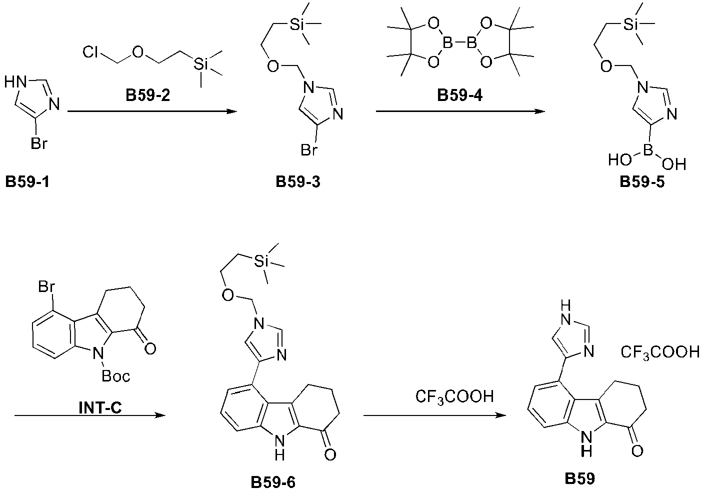

Under a nitrogen atmosphere at 0 °C, to a solution of B59-1 (3 g, 20.41 mmol) and compound B59-2 (6.8 g, 40.85 mmol) in tetrahydrofuran (50 mL) was added 60% sodium hydride (1.6 g, 40.82 mmol). The mixture was stirred at room temperature for 2 hours, quenched with water (50 mL), and extracted with ethyl acetate (3 × 50 mL). The combined organic phases were washed with saturated sodium chloride aqueous solution (3 × 50 mL), dried over anhydrous sodium sulfate, and concentrated under reduced pressure. The residue was purified by silica gel column chromatography (petroleum ether:ethyl acetate = 3:1) to afford compound B59-3 as a colorless oil (2.67 g, 37.27%). ESI/MS (m/z): 277.0 [M+H]⁺.

Under a nitrogen atmosphere at room temperature, to a solution of compound B59-3 (2.5 g, 9.02 mmol) and compound B59-4 (5.7 g, 22.54 mmol) in 1,4-dioxane (60 mL) were added potassium acetate (2.7 g, 27.06 mmol) and [1,1'-bis(diphenylphosphino)ferrocene]dichloropalladium(II) dichloromethane complex (1.5 g, 1.80 mmol). The reaction mixture was stirred at 90 °C overnight. After cooling to room temperature, the mixture was filtered, and the filter cake was washed with 1,4-dioxane (3 × 10 mL). The filtrate was concentrated under reduced pressure, and the residue was purified by reverse-phase preparative chromatography (mobile phase A: water (0.1% formic acid); mobile phase B: acetonitrile) to afford compound B59-5 as a brown-yellow solid (1.6 g, 70.96%). ESI/MS (m/z): 243.1 [M+H]⁺.

Under a nitrogen atmosphere at room temperature, to a solution of compound B59-5 (496.4 mg, 2.05 mmol) and compound INT-C (300 mg, 0.82 mmol) in 1,4-dioxane (10 mL) and water (2 mL) were added cesium carbonate (534.4 mg, 1.64 mmol) and [1,1'-bis(diphenylphosphino)ferrocene]dichloropalladium(II) dichloromethane complex (98.00 mg, 0.12 mmol). The reaction mixture was stirred at 100 °C overnight. After cooling to room temperature, the reaction was quenched with water (20 mL) and extracted with ethyl acetate (3 × 20 mL). The combined organic phases were washed with saturated sodium chloride aqueous solution (3 × 20 mL), dried over anhydrous sodium sulfate, and concentrated under reduced pressure. The residue was purified by silica gel column chromatography (petroleum ether:ethyl acetate = 1:2) to afford compound B59-6 as a brown-yellow oil (220 mg, 70.32%). ESI/MS (m/z): 382.3 [M+H]⁺.

At room temperature, trifluoroacetic acid (2 mL) was added dropwise to a solution of compound B59-6 (200 mg, 0.52 mmol) in dichloromethane (8 mL). The mixture was stirred for 5 hours. The reaction mixture was directly concentrated under reduced pressure, and the residue was purified by reverse-phase preparative chromatography (mobile phase A: water (0.05% trifluoroacetic acid); mobile phase B: acetonitrile) to afford compound B59 as a brown-yellow solid (61.5 mg, 32.13%). ESI/MS (m/z): 252.1 [M+H]⁺.¹H NMR (400 MHz, DMSO-*d6*) δ 14.70 (s, 2H), 12.01 (s, 1H), 9.18 (d, *J =* 1.3 Hz, 1H), 7.88 (d, *J =* 1.3 Hz, 1H), 7.58 (dd, *J =* 8.4, 0.9 Hz, 1H), 7.42 (dd, *J =* 8.4, 7.2 Hz, 1H), 7.17 (dd, *J =* 7.2, 1.0 Hz, 1H), 2.62 (t, *J =* 5.9 Hz, 2H), 2.58-2.52 (m, 2H), 2.08-1.99 (m, *J =* 5.9 Hz, 2H).

### Example 110: Synthesis of Compound B60

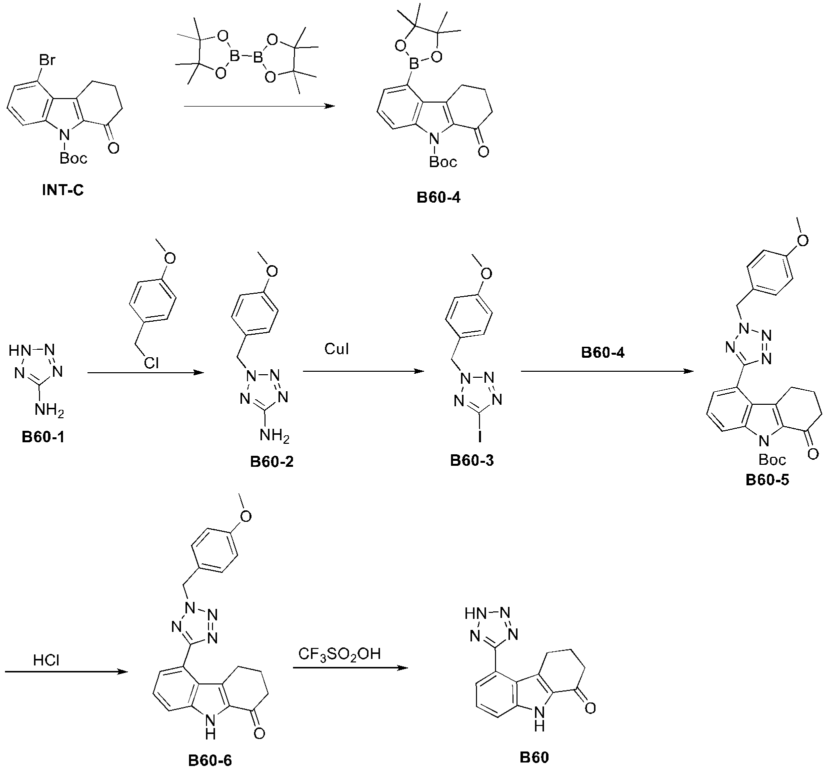

At room temperature, 4-methoxybenzyl chloride (13.81 g, 88.162 mmol) was added portionwise to a mixture of compound B60-1 (5 g, 8.775 mmol) and potassium carbonate (16.25 g, 117.550 mmol) in N,N-dimethylformamide (100 mL). The reaction mixture was stirred at 80 °C overnight. After cooling to room temperature, the reaction was quenched with water (30 mL) and extracted with dichloromethane (2 × 50 mL). The combined organic phases were washed with saturated sodium chloride aqueous solution (50 mL), dried over anhydrous sodium sulfate, and directly concentrated under reduced pressure. The residue was purified by silica gel column chromatography (petroleum ether:ethyl acetate = 1:3) to afford compound B60-2 as a white solid (4.1 g, 32.16%). ESI/MS (m/z): 206.2 [M+H]⁺.

At room temperature, diiodomethane (2.85 g, 10.645 mmol) and isoamyl nitrite (5.67 g, 48.385 mmol) were added portionwise to a solution of compound B60-2 (2 g, 9.677 mmol) and copper(I) iodide (184.31 mg, 0.968 mmol) in tetrahydrofuran (100 mL). The reaction mixture was stirred at 70 °C for 2 hours. After cooling to room temperature, the reaction was quenched with water (30 mL) and extracted with dichloromethane (2 × 100 mL). The combined organic phases were washed with sodium thiosulfate aqueous solution (50 mL), dried over anhydrous sodium sulfate, and concentrated under reduced pressure. The residue was purified by silica gel column chromatography (petroleum ether:ethyl acetate = 5:1) to afford compound B60-3 as a yellow solid (700 mg, 21.59%). ESI/MS (m/z): 316.9 [M+H]⁺.

Under a nitrogen atmosphere at room temperature, bis(pinacolato)diboron (1.71 g, 6.727 mmol), [1,1'-bis(diphenylphosphino)ferrocene]dichloropalladium(II) dichloromethane complex (438.35 mg, 0.538 mmol), and potassium acetate (792.17 mg, 8.073 mmol) were added to a solution of compound INT-C (1 g, 2.691 mmol) in 1,4-dioxane (40 mL). The mixture was stirred at 90 °C overnight. After cooling to room temperature, the mixture was filtered through celite, and the filter cake was washed with dichloromethane (3 × 30 mL). The filtrate was concentrated under vacuum, and the residue was purified by normal-phase silica gel column chromatography (petroleum ether:ethyl acetate = 5:1) to afford compound B60-4 as a yellow solid (570 mg, 46.36%). ESI/MS (m/z): 412.2 [M+H]⁺.

At room temperature, [1,1'-bis(diphenylphosphino)ferrocene]dichloropalladium(II) dichloromethane complex (196.27 mg, 0.241 mmol) and potassium carbonate (665.97 mg, 4.819 mmol) were added portionwise to a solution of compound B60-3 (800 mg, 2.409 mmol) and compound B60-4 (1.6 g, 3.624 mmol) in 1,4-dioxane (10 mL) and water (3 mL). The reaction mixture was stirred at 100 °C for 2 hours. After cooling to room temperature, the reaction was quenched with water and extracted with dichloromethane (3 × 30 mL). The combined organic phases were washed with saturated sodium chloride aqueous solution (30 mL), dried over anhydrous sodium sulfate, and concentrated under reduced pressure. The residue was purified by silica gel column chromatography (petroleum ether:ethyl acetate = 1:1) to afford compound B60-5 as a yellow solid (660 mg, 57.85%). ESI/MS (m/z): 418.2 [M+H-56]⁺.

At room temperature, 4M hydrogen chloride in 1,4-dioxane (1 mL) was added to compound B60-5 (50 mg, 0.106 mmol). The reaction mixture was stirred at 80 °C for 30 minutes. After cooling to room temperature, the pH was adjusted to 8 with sodium hydroxide solution, and the mixture was extracted with ethyl acetate (3 × 30 mL). The combined organic phases were washed with saturated sodium chloride aqueous solution (30 mL), dried over anhydrous sodium sulfate, and concentrated under reduced pressure. The residue was purified by silica gel column chromatography (petroleum ether:ethyl acetate = 1:1) to afford compound B60-6 as a white solid (31.6 mg, 77.66%).ESI/MS (m/z): 374.0 [M+H]⁺.¹H NMR (400 MHz, DMSO-*d₆*) δ 11.94 (s, 1H), 7.58 (dd, *J =* 8.0, 1.3 Hz, 1H), 7.47-7.45 (m, 2H), 7.44-7.39 (m, 2H), 7.02-6.96 (m, 2H), 5.95 (s, 2H), 3.76 (s, 3H), 2.60 (t, *J =* 6.0 Hz, 2H), 2.53-2.51 (m, 2H), 1.98-1.88 (m, 2H).

At room temperature, trifluoromethanesulfonic acid (0.5 mL) was added dropwise to a solution of compound B60-6 (130 mg, 0.338 mmol) in trifluoroacetic acid (5 mL). The mixture was stirred for 30 minutes. The reaction mixture was directly concentrated under reduced pressure, and the residue was purified by reverse-phase preparative chromatography (mobile phase A: water (0.1% formic acid); mobile phase B: acetonitrile) to afford compound B60 as a white solid (34.8 mg, 40.36%).ESI/MS (m/z): 252.2 [M-H]⁻.¹H NMR (400 MHz, DMSO-*d₆*) δ 12.04 (s, 1H), 7.64 (dd, *J =* 8.3, 1.0 Hz, 1H), 7.46 (dd, *J =* 8.3, 7.2 Hz, 1H), 7.35 (dd, *J =* 7.2, 1.0 Hz, 1H), 2.70 (t, *J =* 6.0 Hz, 2H), 2.58-2.52 (m, 2H), 2.09-1.99 (m, 2H).

### Example 111: Synthesis of Compound B61

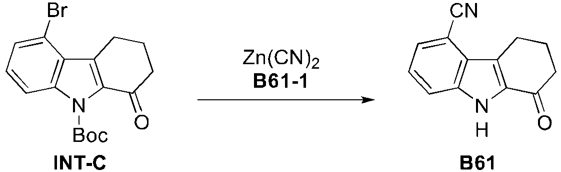

Under a nitrogen atmosphere at room temperature, 1,1'-bis(diphenylphosphino)ferrocene (72.7 mg, 0.132 mmol), zinc powder (4.3 mg, 0.066 mmol), and tris(dibenzylideneacetone)dipalladium (60.5 mg, 0.066 mmol) were added to a solution of INT-C (120 mg, 0.329 mmol) and compound B61-1 (77.4 mg, 0.659 mmol) in N-methylpyrrolidone. The reaction mixture was stirred at 120 °C overnight. After cooling to room temperature, the reaction was quenched with water (6 mL) and extracted with ethyl acetate (3 × 15 mL). The combined organic phases were washed with saturated brine (3 × 10 mL), dried over anhydrous sodium sulfate, and concentrated under reduced pressure. The residue was purified by reverse-phase preparative chromatography (mobile phase A: water (0.1% formic acid); mobile phase B: acetonitrile) to afford compound B61 as a white solid (45.6 mg, 65.93%). ESI/MS (m/z): 211.0 [M+H]⁺.¹H NMR (400 MHz, DMSO-*d6*) δ 12.24 (s, 1H), 7.74 (d, *J* = 8.4 Hz, 1H), 7.63 (d, *J* = 7.2 Hz, 1H), 7.44 (t, *J =* 7.8 Hz, 1H), 3.18 (t, *J =* 6.0 Hz, 2H), 2.62 (t, *J =* 6.4 Hz, 2H), 2.21 (p, *J =* 6.2 Hz, 2H).

### Example 112: Synthesis of Compound B62

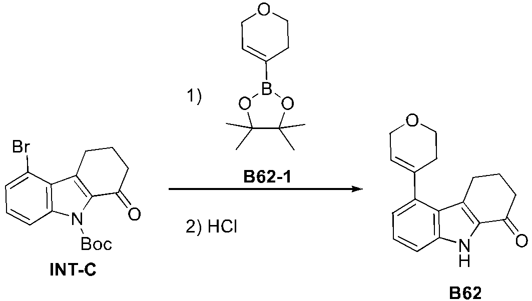

Under a nitrogen atmosphere, to a solution of compound INT-C (200 mg, 0.538 mmol) and compound B62-1 (226 mg, 1.076 mmol) in 1,4-dioxane (8 mL) and water (1.6 mL) were added cesium carbonate (351 mg, 1.076 mmol) and [1,1'-bis(diphenylphosphino)ferrocene]dichloropalladium(II) dichloromethane complex (66 mg, 0.081 mmol). The reaction mixture was stirred at 100 °C overnight. After cooling to room temperature, the reaction was quenched with water (30 mL) and extracted with ethyl acetate (3 × 20 mL). The combined organic phases were washed with saturated sodium chloride aqueous solution (3 × 20 mL), dried over anhydrous sodium sulfate, and concentrated under reduced pressure. The residue was purified by silica gel column chromatography (petroleum ether:ethyl acetate = 2:1) to afford the intermediate compound. The intermediate was redissolved in a 4M hydrogen chloride solution in 1,4-dioxane (8 mL, 263.302 mmol) and stirred at room temperature for 2 hours. The reaction mixture was directly concentrated under reduced pressure and purified by reverse-phase preparative chromatography (mobile phase A: water (0.1% formic acid); mobile phase B: acetonitrile) to afford compound B62 as a white solid (59.9 mg, 40.72%). ESI/MS (m/z): 268.2 [M+H]⁺.¹H NMR (400 MHz, DMSO-*d6*) δ 11.66 (s, 1H), 7.32 (dd, *J =* 8.3, 1.1 Hz, 1H), 7.24 (dd, *J =* 8.3, 7.0 Hz, 1H), 6.84 (dd, *J =* 7.1, 1.1 Hz, 1H), 5.72-5.70 (m, 1H), 4.24 (q, *J =* 2.7 Hz, 2H), 3.88 (t, *J* = 5.4 Hz, 2H), 2.95 (t, *J =* 6.0 Hz, 2H), 2.53 (t, *J =* 6.3 Hz, 2H), 2.43-2.39 (m, 2H), 2.15-2.05 (m, 2H).

### Example113: Synthesis of Compound B63

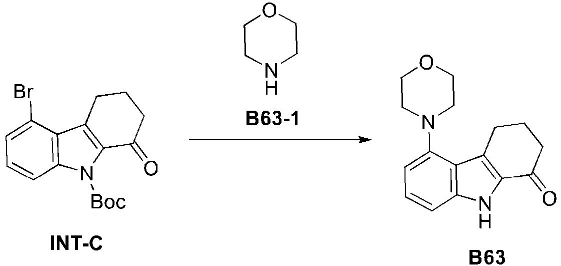

Under a nitrogen atmosphere at room temperature, to a solution of compound INT-C (200 mg, 0.538 mmol) and sodium tert-butoxide (155 mg, 1.614 mmol) in 1,4-dioxane (5 mL) were added (2-dicyclohexylphosphino-2',6'-diisopropoxy-1,1'-biphenyl)(2-amino-1,1'-biphenyl-2-yl)palladium(II) methanesulfonate (90 mg, 0.108 mmol), 2-dicyclohexylphosphino-2',6'-diisopropoxybiphenyl (50 mg, 0.108 mmol), and compound B63-1 (141 mg, 1.614 mmol). The reaction mixture was stirred at 100 °C for 3 hours. After cooling to room temperature, the reaction was quenched with water (50 mL) and extracted with ethyl acetate (3 × 30 mL). The combined organic phases were washed with saturated sodium chloride aqueous solution (10 mL), dried over anhydrous sodium sulfate, filtered, and concentrated under reduced pressure. The residue was purified by reverse-phase preparative chromatography (mobile phase A: water (10 mmol/L ammonium bicarbonate); mobile phase B: acetonitrile) to afford compound B63 as a light yellow solid (34.8 mg, 44.30%). ESI/MS (m/z): 271.3 [M+H]⁺.¹H NMR (400 MHz, DMSO-*d*₆) δ 11.65 (s, 1H), 7.22 (t, *J =* 7.8 Hz, 1H), 7.11 (d, *J =* 8.2 Hz, 1H), 6.63 (d, *J* = 7.5 Hz, 1H), 3.86 (t, *J =* 4.4 Hz, 4H), 3.25 (t, *J =* 5.8 Hz, 2H), 3.08-2.98 (m, 4H), 2.58-2.56 (m, 2H), 2.16 (p, *J =* 5.8 Hz, 2H).

### Example 114: Synthesis of Compound B64

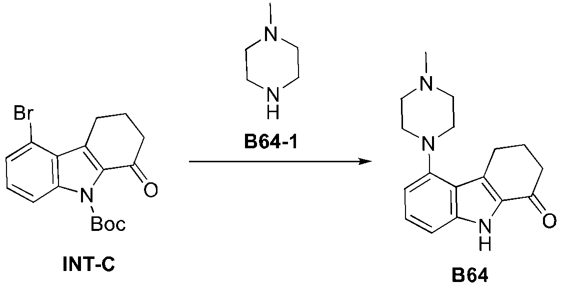

Under a nitrogen atmosphere at room temperature, to a solution of compound INT-C (200 mg, 0.538 mmol) and sodium tert-butoxide (155 mg, 1.614 mmol) in 1,4-dioxane (5 mL) were added (2-dicyclohexylphosphino-2',6'-diisopropoxy-1,1'-biphenyl)(2-amino-1,1'-biphenyl-2-yl)palladium(II) methanesulfonate (90 mg, 0.108 mmol), 2-dicyclohexylphosphino-2',6'-diisopropoxybiphenyl (50 mg, 0.108 mmol), and compound B64-1 (162 mg, 1.614 mmol). The reaction mixture was stirred at 100 °C overnight. After cooling to room temperature, the reaction was quenched with water (60 mL) and extracted with ethyl acetate (3 × 40 mL). The combined organic phases were washed with saturated sodium chloride aqueous solution (20 mL), dried over anhydrous sodium sulfate, filtered, and concentrated under reduced pressure. The residue was purified by reverse-phase preparative chromatography (mobile phase A: water (10 mmol/L ammonium bicarbonate); mobile phase B: acetonitrile) to afford compound B64 as a yellow solid (28.9 mg, 46.13%). ESI/MS (m/z): 284.1 [M+H]⁺.¹H NMR (400 MHz, DMSO-*d₆*) δ 11.56 (s, 1H), 7.15 (t, *J =* 7.9 Hz, 1H), 7.05-7.01 (m, 1H), 6.56 (d, *J =* 7.4 Hz, 1H), 3.34(s, 2H), 3.20 (t, *J =* 5.9 Hz, 2H), 2.98-2.95 (m, 4H), 2.57-2.51 (m, 4H), 2.26 (s, 3H), 2.14-2.07 (m, 2H).

### Example 115: Synthesis of Compound B65

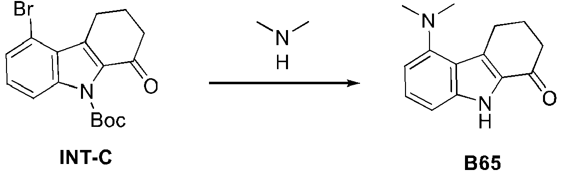

Under a nitrogen atmosphere at room temperature, to a solution of compound INT-C (200 mg, 0.538 mmol) and sodium tert-butoxide (155 mg, 1.614 mmol) in 1,4-dioxane (5 mL) were added (2-dicyclohexylphosphino-2',6'-diisopropoxy-1,1'-biphenyl)(2-amino-1,1'-biphenyl-2-yl)palladium(II) methanesulfonate (90 mg, 0.108 mmol), 2-dicyclohexylphosphino-2',6'-diisopropoxybiphenyl (50 mg, 0.108 mmol), and a 2M solution of dimethylamine in tetrahydrofuran (1.345 mL, 2.690 mmol). The reaction mixture was stirred at 100 °C overnight. After cooling to room temperature, the reaction was quenched with water (70 mL) and extracted with ethyl acetate (3 × 50 mL). The combined organic phases were washed with saturated sodium chloride aqueous solution (30 mL), dried over anhydrous sodium sulfate, filtered, and concentrated under reduced pressure. The residue was purified by reverse-phase preparative chromatography (mobile phase A: water (10 mmol/L ammonium bicarbonate); mobile phase B: acetonitrile) to afford compound B65 as a light yellow solid (41.2 mg, 34.29%). ESI/MS (m/z): 229.2 [M+H]⁺.¹H NMR (400 MHz, DMSO-*d*₆) δ 11.53 (s, 1H), 7.21-7.07 (m, 1H), 6.99 (d, *J =* 7.5 Hz, 1H), 6.53 (d, *J =* 7.2 Hz, 1H), 3.20 (t, *J* = 5.9 Hz, 2H), 2.76 (s, 6H), 2.55-2.52 (m, 2H), 2.10-2.07 (m , 2H).

### Example116: Synthesis of Compound B66

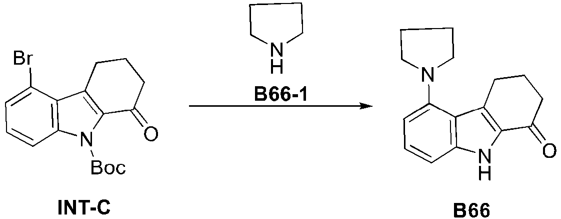

Under a nitrogen atmosphere at room temperature, to a solution of compound INT-C (200 mg, 0.538 mmol) and sodium tert-butoxide (155 mg, 1.614 mmol) in 1,4-dioxane (5 mL) were added (2-dicyclohexylphosphino-2',6'-diisopropoxy-1,1'-biphenyl)(2-amino-1,1'-biphenyl-2-yl)palladium(II) methanesulfonate (90 mg, 0.108 mmol), 2-dicyclohexylphosphino-2',6'-diisopropoxybiphenyl (50 mg, 0.108 mmol), and compound B66-1 (115 mg, 1.614 mmol). The reaction mixture was stirred at 100 °C for 3 hours. After cooling to room temperature, the reaction was quenched with water (60 mL) and extracted with ethyl acetate (3 × 35 mL). The combined organic phases were washed with saturated sodium chloride aqueous solution (30 mL), dried over anhydrous sodium sulfate, filtered, and concentrated under reduced pressure. The residue was purified by reverse-phase preparative chromatography (mobile phase A: water (10 mmol/L ammonium bicarbonate); mobile phase B: acetonitrile) to afford compound B66 as a light yellow solid (85.2 mg, 77.31%). ESI/MS (m/z): 255.2 [M+H]⁺.¹H NMR (300 MHz, DMSO-*d*₆) δ 11.52 (s, 1H), 7.10 (t, *J* = 7.9 Hz, 1H), 6.94 (d, *J =* 8.3, 0.8 Hz, 1H), 6.48 (d, *J =* 7.6, 0.9 Hz, 1H), 3.19-3.17 (m, 2H), 3.15-3.11 (m, 4H), 2.56-2.51 (m, 2H), 2.07 (p, *J =* 6.1 Hz, 2H), 1.90 (p, *J =* 3.1 Hz, 4H).

### Example117: Synthesis of Compound B67

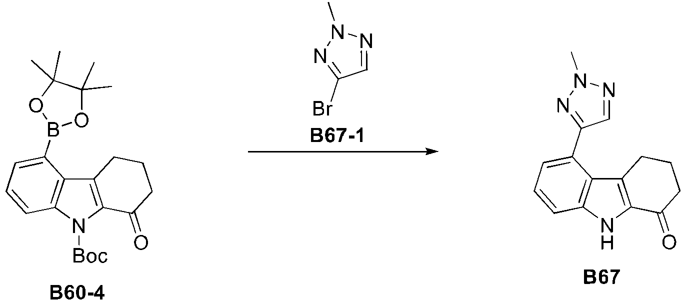

Under a nitrogen atmosphere, to a solution of compound B60-4 (564 mg, 1.234 mmol) and compound B67-1 (100 mg, 0.617 mmol) in 1,4-dioxane (8 mL) and water (1.6 mL) were added cesium carbonate (402 mg, 1.234 mmol) and [1,1'-bis(diphenylphosphino)ferrocene]dichloropalladium(II) dichloromethane complex (75 mg, 0.093 mmol). The reaction mixture was stirred at 100 °C overnight. After cooling to room temperature, the reaction was quenched with water (20 mL) and extracted with ethyl acetate (3 × 20 mL). The combined organic phases were washed with saturated sodium chloride aqueous solution (20 mL), dried over anhydrous sodium sulfate, and concentrated under reduced pressure. The residue was purified by reverse-phase chromatography (mobile phase A: water (10 mmol/L ammonium bicarbonate); mobile phase B: acetonitrile) to afford compound B67 as a white solid (62.7 mg, 37.99%). ESI/MS (m/z): 267.2 [M+H]⁺.¹H NMR (400 MHz, DMSO-*d*₆) δ 11.82 (s, 1H), 8.00 (s, 1H), 7.47 (dd, *J =* 8.4, 1.0 Hz, 1H), 7.35 (dd, *J* = 8.3*,* 7.1 Hz, 1H), 7.15 (dd, *J =* 7.1, 1.0 Hz, 1H), 4.25 (s, 3H), 2.75 (t, *J =* 6.0 Hz, 2H), 2.58-2.51 (m, 2H), 2.07-1.97 (m, 2H).

### Example118: Synthesis of Compound B68

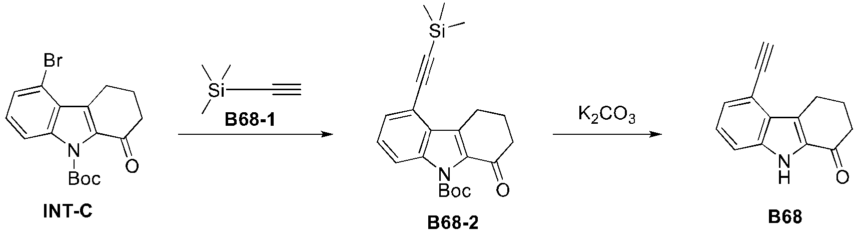

Under a nitrogen atmosphere, triethylamine (3 mL), bis(triphenylphosphine)palladium(II) chloride (96.2 mg, 0.137 mmol), copper(I) iodide (26.1 mg, 0.137 mmol), and triphenylphosphine (71.9 mg, 0.274 mmol) were added to a solution of compound INT-C (500 mg, 1.373 mmol) and compound B68-1 (1.4 g, 13.73 mmol) in tetrahydrofuran (10 mL). The reaction mixture was stirred at 70 °C for 4 hours. After cooling to room temperature, the reaction was quenched with water (30 mL) and extracted with ethyl acetate (3 × 30 mL). The combined organic phases were washed with saturated sodium chloride aqueous solution (3 × 10 mL), dried over anhydrous sodium sulfate, and concentrated under reduced pressure. The residue was purified by silica gel column chromatography (petroleum ether:ethyl acetate = 10:1) to afford compound B68-2 as a light yellow solid (310 mg, 59.17%). ESI/MS (m/z): 326.1 [M+H-56]⁺.

At room temperature, potassium carbonate (72.5 mg, 0.524 mmol) was added to a solution of compound B68-2 (100 mg, 0.262 mmol) in methanol (6 mL). The reaction mixture was stirred at room temperature for 1 hour and then filtered. The filter cake was washed with methanol (3 × 5 mL), and the filtrate was concentrated under reduced pressure. The residue was purified by silica gel column chromatography (petroleum ether:ethyl acetate = 4:1) to afford compound B68 as a white solid (31.3 mg, 57.09%). ESI/MS (m/z): 210.2 [M+H]⁺.¹H NMR (400 MHz, DMSO-d6) δ 11.84 (s, 1H), 7.44 (dd, *J =* 8.2, 1.1 Hz, 1H), 7.27 (t, *J =* 7.7 Hz, 1H), 7.21 (dd, *J* = 7.2*,* 1.1 Hz, 1H), 4.43 (s, 1H), 3.25 (t, *J =* 6.1 Hz, 2H), 2.56 (t, *J* =6.1 Hz, 2H), 2.16 (p, *J =* 6.1 Hz, 2H).

### Example 119: Synthesis of Compound B69

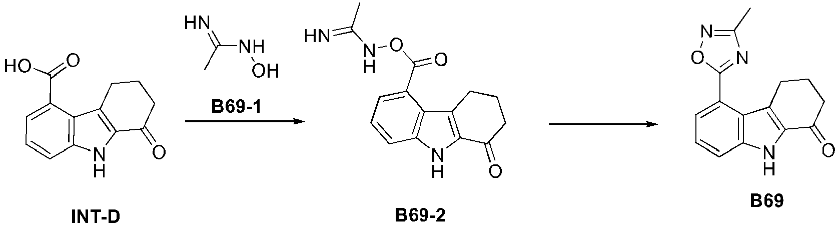

At room temperature, to a solution of compound INT-D (200 mg, 0.860 mmol) and compound B69-1 (127 mg, 1.720 mmol) in N,N-dimethylformamide (5 mL) were added 2-(7-aza-1H-benzotriazol-1-yl)-1,1,3,3-tetramethyluronium hexafluorophosphate (491 mg, 0.673 mmol) and N,N-diisopropylethylamine (334 mg, 2.580 mmol). The mixture was stirred for 30 minutes. The reaction was quenched with water (15 mL) and extracted with ethyl acetate (3 × 20 mL). The combined organic phases were washed with saturated sodium chloride aqueous solution (15 mL), dried over anhydrous sodium sulfate, filtered, and concentrated under reduced pressure. The residue was purified by reverse-phase preparative chromatography (mobile phase A: water (10 mmol/L ammonium bicarbonate); mobile phase B: acetonitrile) to afford compound B69-2 as a white solid (160 mg, 65.19%). ESI/MS (m/z): 286 [M+H]⁺.

A solution of compound B69-2 (140 mg, 0.491 mmol) in toluene (6 mL) was stirred at 120 °C overnight. After cooling to room temperature, the mixture was concentrated under reduced pressure. The residue was purified by reverse-phase preparative chromatography (mobile phase A: water (0.1% formic acid); mobile phase B: acetonitrile) to afford compound B69 as a white solid (8.8 mg, 6.68%). ESI/MS (m/z): 268.1 [M+H]⁺.¹H NMR (400 MHz, DMSO-*d*₆) δ 12.17 (s, 1H), 7.74 (dd, *J* = 4.8*,* 1.0 Hz, 1H), 7.73-7.71 (m, 1H), 7.48 (dd, *J =* 8.2, 7.4 Hz, 1H), 3.07 (t, *J* = 6.0 Hz, 2H), 2.65-2.55 (m, 2H), 2.47 (s, 3H), 2.16-2.06 (m, 2H).

### Example 120: Synthesis of Compound B70

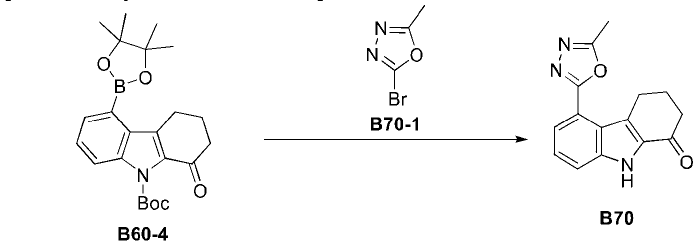

Under a nitrogen atmosphere, to a solution of compound B60-4 (549 mg, 1.228 mmol) and compound B70-1 (100 mg, 0.614 mmol) in 1,4-dioxane (8 mL) and water (1.6 mL) were added cesium carbonate (400 mg, 1.228 mmol) and [1,1'-bis(diphenylphosphino)ferrocene]dichloropalladium(II) dichloromethane complex (75 mg, 0.093 mmol). The reaction mixture was stirred at 100 °C overnight. After cooling to room temperature, the mixture was diluted with water (20 mL) and extracted with ethyl acetate (3 × 20 mL). The combined organic phases were washed with saturated sodium chloride aqueous solution (20 mL), dried over anhydrous sodium sulfate, and concentrated under reduced pressure. The residue was purified by reverse-phase chromatography (mobile phase A: water (10 mmol/L ammonium bicarbonate); mobile phase B: acetonitrile) to afford compound B70 as a light yellow solid (5.3 mg, 3.09%). ESI/MS (m/z): 268.1 [M+H]⁺.¹H NMR (400 MHz, DMSO-*d*₆) δ 12.08 (s, 1H), 7.67 (dd, *J =* 8.3, 1.0 Hz, 1H), 7.58 (dd, *J =* 7.3, 1.0 Hz, 1H), 7.45 (dd, *J =* 8.3*,* 7.3 Hz, 1H), 3.07 (t, *J =* 6.0 Hz, 2H), 2.63 (s, 3H), 2.60-2.54 (m, 2H), 2.17-2.04 (m, 2H).

### Example 121: Synthesis of Compound B71

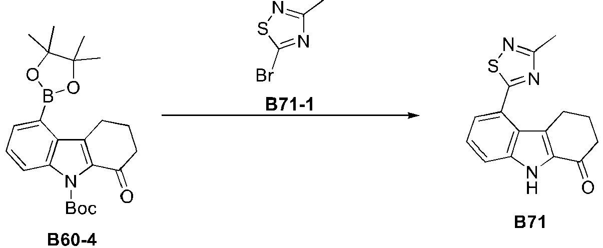

Under a nitrogen atmosphere, to a solution of compound B60-4 (499 mg, 1.118 mmol) and compound B71-1 (100 mg, 0.559 mmol) in 1,4-dioxane (8 mL) and water (1.6 mL) were added cesium carbonate (364 mg, 1.118 mmol) and [1,1'-bis(diphenylphosphino)ferrocene]dichloropalladium(II) dichloromethane complex (68 mg, 0.084 mmol). The reaction mixture was stirred at 100 °C overnight. After cooling to room temperature, the mixture was diluted with water (20 mL) and extracted with ethyl acetate (3 × 20 mL). The combined organic phases were washed with saturated sodium chloride aqueous solution (20 mL), dried over anhydrous sodium sulfate, and concentrated under reduced pressure. The residue was purified by reverse-phase preparative chromatography (mobile phase A: water (0.1% formic acid); mobile phase B: acetonitrile) to afford compound B71 as a light yellow solid (76.2 mg, 45.79%).¹H NMR (400 MHz, DMSO-*d6*) δ 12.09 (s, 1H), 7.65 (dd, *J =* 7.2, 2.1 Hz, 1H), 7.45-7.41 (m, 1H), 7.41-7.38 (m, 1H), 2.86 (t, *J =* 6.0 Hz, 2H), 2.71 (s, 3H), 2.59-2.53 (m, 2H), 2.11-2.01 (m, 2H).

### Example122: Synthesis of Compound B72

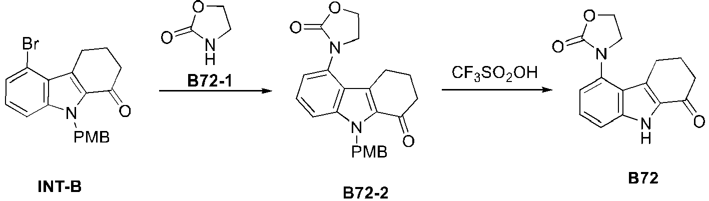

Under a nitrogen atmosphere at room temperature, to a solution of compound INT-B (200 mg, 0.464 mmol) and sodium tert-butoxide (134 mg, 1.392 mmol) in toluene (6 mL) were added tris(dibenzylideneacetone)dipalladium (85.1 mg, 0.093 mmol), 4,5-bis(diphenylphosphino)-9,9-dimethylxanthene (54 mg, 0.093 mmol), and compound B72-1 (121.3 mg, 1.392 mmol). The reaction mixture was stirred at 110 °C for 2 hours. After cooling to room temperature, the reaction was quenched with water (60 mL) and extracted with ethyl acetate (3 × 60 mL). The combined organic phases were washed with saturated sodium chloride aqueous solution (50 mL), dried over anhydrous sodium sulfate, and concentrated under reduced pressure. The residue was purified by silica gel column chromatography (petroleum ether:ethyl acetate = 4:1) to afford compound B72-2 as a light yellow solid (160 mg, 45.55%). ESI/MS (m/z): 391.1 [M+H]⁺.

At room temperature, trifluoromethanesulfonic acid (0.3 mL) was added to a solution of compound B72-2 (140 mg, 0.185 mmol) in trifluoroacetic acid (3 mL). The mixture was stirred for 10 minutes. After the reaction was complete, the mixture was directly concentrated under reduced pressure, and the residue was purified by reverse-phase preparative chromatography (mobile phase A: water (0.1% formic acid); mobile phase B: acetonitrile) to afford compound B72 as a pale yellow solid (42.5 mg, 84.81%). ESI/MS (m/z): 271.2 [M+H]⁺.¹H NMR (400 MHz, DMSO-*d*₆) δ 11.82 (s, 1H), 7.39 (d, *J =* 8.5 Hz, 1H), 7.32 (t, *J =* 7.8 Hz, 1H), 7.07 (d, *J =* 7.2 Hz, 1H), 4.54 (t, *J =* 7.8 Hz, 2H), 4.05 (t, *J =* 7.9 Hz, 2H), 3.00 (t, *J =* 6.0 Hz, 2H), 2.58-2.53 (m, 2H), 2.18-2.09 (m, 2H).

### Example 123: Synthesis of Compound B73

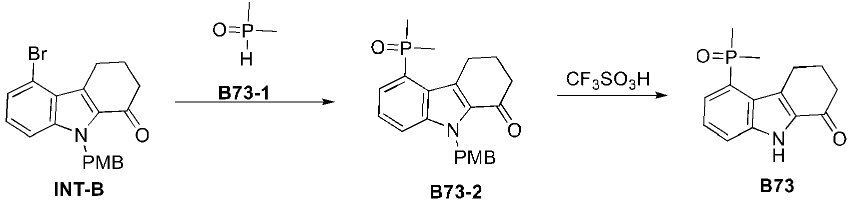

Under a nitrogen atmosphere, to a solution of compound INT-B (200 mg, 0.520 mmol) and compound B73-1 (122 mg, 1.560 mmol) in ethanol (8 mL) were added tricyclohexylphosphonium tetrafluoroborate (38 mg, 0.104 mmol) and (2'-amino-[1,1'-biphenyl]-2-yl)(tricyclohexylphosphine)palladium(II) methanesulfonate (68 mg, 0.104 mmol). The reaction mixture was stirred at 100 °C overnight. After cooling to room temperature, the mixture was filtered, and the filter cake was washed with ethanol (5 mL). The combined filtrates were concentrated under reduced pressure, and the residue was purified by reverse-phase preparative chromatography (mobile phase A: water (0.1% formic acid); mobile phase B: acetonitrile) to afford compound B73-2 as a white solid (100 mg, 48.86%). ESI/MS (m/z): 382.1 [M+H]⁺.

At room temperature, trifluoromethanesulfonic acid (200 µL) was added to a solution of compound B73-2 (80 mg, 0.203 mmol) in trifluoroacetic acid (2 mL). The mixture was stirred for 10 minutes. The reaction mixture was directly concentrated under reduced pressure, and the residue was purified by reverse-phase preparative chromatography (mobile phase A: water (10 mmol/L ammonium bicarbonate); mobile phase B: acetonitrile) to afford compound B73 as a white solid (22.4 mg, 41.93%). ESI/MS (m/z): 262.2 [M+H]⁺.¹H NMR (400 MHz, DMSO-*d*₆) δ 12.02 (s, 1H), 7.66-7.61 (m, 1H), 7.41-7.37 (m, 1H), 7.37-7.34 (m, 1H), 3.45-3.39 (m, 2H), 2.61-2.55 (m, 2H), 2.17-2.08 (m, 2H), 1.80 (s, 3H), 1.77 (s, 3H).

### Example 124: Synthesis of Compound B74

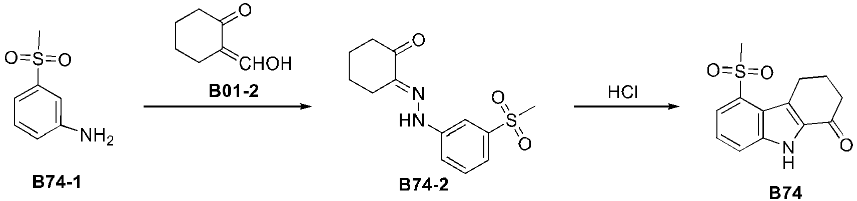

At 0 °C, a solution of sodium nitrite (0.85 g, 12.265 mmol) in water (8 mL) was slowly added to a solution of compound B74-1 (1.4 g, 8.177 mmol) in water (32 mL) and concentrated hydrochloric acid (2.3 mL). Then, at 0 °C, a solution of compound B01-2 (1.27 g, 9.812 mmol) in methanol (32 mL) and a solution of sodium acetate (1.48 g, 17.989 mmol) in water (32 mL) were slowly added dropwise to the above mixture. The mixture was stirred at room temperature for 1 hour, filtered, and the filter cake was washed with water (30 mL) and dried to afford compound B74-2 as a brown solid (1.5 g, 54.44%). ESI/MS (m/z): 281.1 [M+H]⁺.

At room temperature, concentrated hydrochloric acid (5 mL) was added to a solution of compound B74-2 (1.5 g, 4.446 mmol) in methanol (100 mL). The mixture was stirred at 60 °C for 2 hours. After cooling to room temperature, the pH was adjusted to 7 with saturated potassium hydroxide solution. The mixture was extracted with ethyl acetate (3 × 100 mL). The combined organic phases were washed with saturated sodium chloride aqueous solution (100 mL), dried over anhydrous sodium sulfate, filtered, and concentrated under reduced pressure. The residue was purified by reverse-phase preparative chromatography (mobile phase A: water (0.1% formic acid); mobile phase B: acetonitrile) to afford compound B74 as a white solid (52.4 mg, 4.43%). ESI/MS (m/z): 264.1 [M+H]⁺.¹H NMR (400 MHz, DMSO-*d*₆) δ 12.38 (s, 1H), 7.80 (dd, *J = 8.3,* 1.0 Hz, 1H), 7.74 (dd, *J =* 7.4, 1.0 Hz, 1H), 7.49 (t, *J =* 7.9 Hz, 1H), 3.31 (s, 3H), 3.30-3.26 (m, 2H), 2.64-2.57 (m, 2H), 2.24-2.13 (m, 2H).

### Example 125: Synthesis of Compound B75

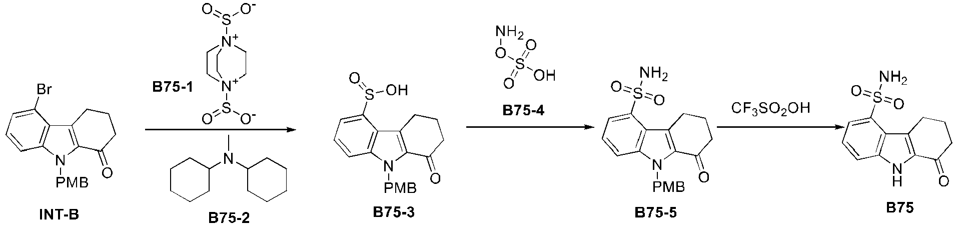

Under a nitrogen atmosphere, dichlorodi-tert-butyl-(4-dimethylaminophenyl)phosphine palladium(II) (98.6 mg, 0.139 mmol) and compound B75-2 (408 mg, 2.088 mmol) were added to a solution of compound INT-B (300 mg, 0.696 mmol) and compound B75-1 (167 mg, 0.696 mmol) in isopropanol (10 mL). The reaction mixture was stirred at 110 °C for 2 hours. After cooling to room temperature, the mixture was concentrated under reduced pressure. The residue was purified by reverse-phase preparative chromatography (mobile phase A: water (0.1% formic acid); mobile phase B: acetonitrile) to afford compound B75-3 as a pale yellow solid (105 mg, 38.32%). ESI/MS (m/z): 370.1 [M+H]⁺.

A solution of sodium acetate (89 mg, 1.080 mmol) in water (6 mL) and compound B75-4 (122 mg, 1.080 mmol) were added to a solution of compound B75-3 (85 mg, 0.216 mmol) in isopropanol (3 mL). The mixture was stirred at room temperature overnight. The reaction mixture was extracted with ethyl acetate (3 × 5 mL). The combined organic phases were washed with saturated sodium chloride aqueous solution (5 mL), dried over anhydrous sodium sulfate, filtered, and concentrated under reduced pressure. The residue was purified by reverse-phase preparative chromatography (mobile phase A: water (0.1% formic acid); mobile phase B: acetonitrile) to afford compound B75-5 as a white solid (56 mg, 59.40%). ESI/MS (m/z): 385.1 [M+H]⁺.

Trifluoromethanesulfonic acid (0.3 mL) was added to a solution of compound B75-5 (50 mg, 0.115 mmol) in trifluoroacetic acid (3 mL). The mixture was stirred at room temperature for 10 minutes. The reaction mixture was concentrated under reduced pressure, and the residue was purified by reverse-phase preparative chromatography (mobile phase A: water (0.1% formic acid); mobile phase B: acetonitrile) to afford compound B75 as a white solid (17.6 mg, 57.59%). ESI/MS (m/z): 265.1 [M+H]⁺.¹H NMR (400 MHz, DMSO-*d6*) δ 12.15 (s, 1H), 7.65 (d, *J =* 8.3 Hz, 1H), 7.62 (d, *J =* 7.3 Hz, 1H), 7.54 (s, 2H), 7.41 (t, *J =* 7.8 Hz, 1H), 3.26 (t, *J = 6.0* Hz, 2H), 2.63-2.54 (m, 2H), 2.19-2.08 (m, 2H).

### Example 126: Synthesis of Compound B76

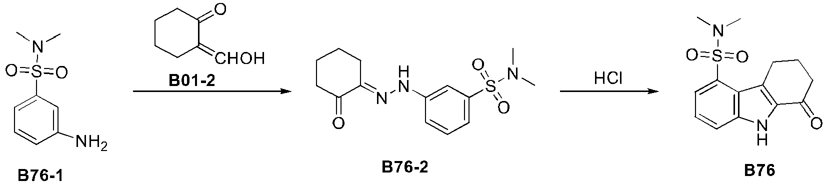

At 0 °C, a solution of sodium nitrite (0.93 g, 13.482 mmol) in water (9 mL) was slowly added to a solution of compound B76-1 (1.8 g, 8.988 mmol) in water (36 mL) and concentrated hydrochloric acid (0.3 mL). Then, at 0 °C, a solution of compound B01-2 (1.40 g, 10.786 mmol) in methanol (36 mL) and a solution of sodium acetate (1.62 g, 19.774 mmol) in water (36 mL) were slowly added to the above mixture. The reaction mixture was stirred at room temperature for 1 hour and then filtered. The filter cake was washed with water (30 mL) and dried to afford compound B76-2 as a brown solid (1.9 g, 59.31%). ESI/MS (m/z): 310.0 [M+H]⁺.

At room temperature, concentrated hydrochloric acid (5 mL) was added to a solution of compound B76-2 (1.55 g, 4.349 mmol) in methanol (100 mL). The mixture was stirred at 60 °C for 2 hours. After cooling to room temperature, the reaction mixture was adjusted to pH 7 with saturated potassium hydroxide solution and extracted with ethyl acetate (3 × 100 mL). The combined organic phases were washed with saturated sodium chloride aqueous solution (100 mL), dried over anhydrous sodium sulfate, filtered, and concentrated under reduced pressure. The residue was purified by reverse-phase preparative chromatography (mobile phase A: water (0.1% formic acid); mobile phase B: acetonitrile) to afford compound B76 as a white solid (76 mg, 5.95%). ESI/MS (m/z): 293.1 [M+H]⁺.¹H NMR (400 MHz, DMSO-*d*₆) δ 12.27 (s, 1H), 7.74 (dd, *J =* 8.2, 1.0 Hz, 1H), 7.53 (dd, *J =* 7.4, 1.0 Hz, 1H), 7.44 (dd, *J=* 8.3, 7.4 Hz, 1H), 3.22 (t, *J=* 6.0 Hz, 2H), 2.83 (s, 6H), 2.63-2.55 (m, 2H), 2.17-2.14 (m, 2H).

### Example 127: Synthesis of Compound B77

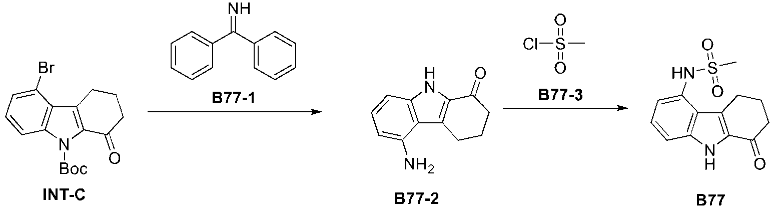

Under a nitrogen atmosphere, to a solution of compound INT-C (1 g, 2.581 mmol) and sodium tert-butoxide (744.1 mg, 7.743 mmol) in 1,4-dioxane (15 mL) were added (2-dicyclohexylphosphino-2',6'-diisopropoxy-1,1'-biphenyl)(2-amino-1,1'-biphenyl-2-yl)palladium(II) methanesulfonate (0.43 g, 0.516 mmol), 2-dicyclohexylphosphino-2',6'-diisopropoxybiphenyl (240.86 mg, 0.516 mmol), and compound B77-1 (1.40 g, 7.743 mmol). The reaction mixture was stirred at 100 °C for 3 hours. After cooling to room temperature, the reaction was quenched with water (200 mL). Then, 5M hydrochloric acid (15 mL) was added to the mixture and stirred for 2 hours. The mixture was extracted with ethyl acetate (3 × 100 mL). The remaining aqueous phase was neutralized to pH 8 with saturated sodium bicarbonate solution and extracted with ethyl acetate (3 × 150 mL). The combined organic phases were washed with saturated sodium chloride aqueous solution (100 mL), dried over anhydrous sodium sulfate, filtered, and concentrated under reduced pressure. The residue was purified by reverse-phase preparative chromatography (mobile phase A: water (10 mmol/L ammonium bicarbonate); mobile phase B: acetonitrile) to afford compound B77-2 as a yellow solid (340 mg, 62.90%). ESI/MS (m/z): 201.0 [M+H]⁺.

At room temperature, compound B77-3 (246.08 mg, 2.148 mmol) was added to a solution of compound B77-2 (150 mg, 0.716 mmol) and pyridine (113.29 mg, 1.432 mmol) in dichloromethane (5 mL). The reaction mixture was stirred at room temperature overnight. The reaction was quenched with water (15 mL) and extracted with ethyl acetate (3 × 15 mL). The combined organic phases were washed with saturated sodium chloride aqueous solution (10 mL), dried over anhydrous sodium sulfate, filtered, and concentrated under reduced pressure. The residue was purified by reverse-phase preparative chromatography (mobile phase A: water (10 mmol/L ammonium bicarbonate); mobile phase B: acetonitrile) to afford compound B77 as a white solid (28.1 mg, 14.00%). ESI/MS (m/z): 279.1 [M+H]⁺.¹H NMR (400 MHz, DMSO-*d*₆) δ 11.69 (s, 1H), 9.29 (s, 1H), 7.31 (dd, *J =* 8.3*,* 1.1 Hz, 1H), 7.26 (dd, *J =* 8.3, 7.2 Hz, 1H), 6.97 (dd, *J* = 7.2, 1.1 Hz, 1H), 3.22 (t, *J =* 6.0 Hz, 2H), 3.03 (s, 3H), 2.56-2.52 (m, 2H), 2.16-2.08 (m, 2H).

### Example128: Synthesis of Compound B78

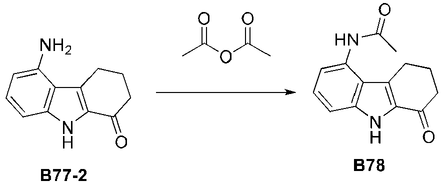

To a solution of compound B77-2 (200 mg, 0.955 mmol) and pyridine (151.06 mg, 1.910 mmol) in dichloromethane (10 mL) was added acetic anhydride (107.23 mg, 1.050 mmol). The reaction mixture was stirred at room temperature overnight. The reaction was quenched with water (20 mL) and extracted with ethyl acetate (3 × 15 mL). The combined organic phases were washed with saturated sodium chloride aqueous solution (15 mL), dried over anhydrous sodium sulfate, filtered, and concentrated under reduced pressure. The residue was purified by reverse-phase preparative chromatography (mobile phase A: water (10 mmol/L ammonium bicarbonate); mobile phase B: acetonitrile) to afford compound B78 as a white solid (42.1 mg, 18.16%). ESI/MS (m/z): 243.2 [M+H]⁺.¹H NMR (400 MHz, DMSO-*d*₆) δ 11.63 (s, 1H), 9.61 (s, 1H), 7.23 (s, 1H), 7.22 (s, 1H), 6.95 (t, *J=* 4.2 Hz, 1H), 3.08 (t, *J=* 6.0 Hz, 2H), 2.56-2.51 (m, 2H), 2.13-2.10 (m, 2H), 2.09 (s, 3H).

### Example129: Synthesis of Compound B79

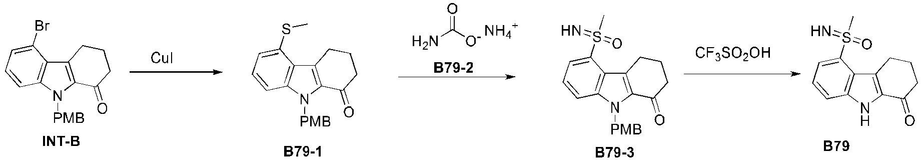

Under a nitrogen atmosphere, copper(I) iodide (55 mg, 0.290 mmol) was added to a solution of compound INT-B (500 mg, 1.161 mmol) and copper(II) acetate (422 mg, 2.322 mmol) in dimethyl sulfoxide (5 mL). The reaction mixture was stirred at 135 °C for 3 hours. After cooling to room temperature, the reaction was quenched with water (20 mL) and extracted with ethyl acetate (3 × 50 mL). The combined organic phases were washed with saturated sodium chloride aqueous solution (100 mL), dried over anhydrous sodium sulfate, filtered, and concentrated under reduced pressure. The residue was purified by silica gel column chromatography (petroleum ether:ethyl acetate = 10: 1) to afford compound B79-1 as a yellow solid (115 mg, 28.19%). ESI/MS (m/z): 352.1 [M+H]⁺.

(Diacetoxyiodo)benzene (165 mg, 0.512 mmol) was added to a solution of compound B79-1 (100 mg, 0.256 mmol) and compound B79-2 (80 mg, 1.024 mmol) in methanol (5 mL). The reaction mixture was stirred at room temperature for 1 hour. The mixture was directly concentrated under reduced pressure, and the residue was purified by reverse-phase preparative chromatography (mobile phase A: water (0.1% formic acid); mobile phase B: acetonitrile) to afford compound B79-3 as a gray solid (60 mg, 61.26%). ESI/MS (m/z): 383.0 [M+H]⁺.

Trifluoromethanesulfonic acid (100 µL) was added to a solution of compound B79-3 (50 mg, 0.131 mmol) in trifluoroacetic acid (1 mL). The mixture was stirred at room temperature for 10 minutes. The reaction mixture was directly concentrated under reduced pressure, and the residue was purified by reverse-phase preparative chromatography (mobile phase A: water (10 mmol/L ammonium bicarbonate); mobile phase B: acetonitrile) to afford compound B79 as a white solid (24.9 mg, 72.25%). ESI/MS (m/z): 263.1 [M+H]⁺.¹H NMR (400 MHz, DMSO-*d*₆) δ 9.92 (br, 1H), 7.77 (d, *J* = 7.4 Hz, 1H), 7.72 (d, *J=* 8.2 Hz, 1H), 7.45 (t, *J =* 7.8 Hz, 1H), 4.40 (s, 1H), 3.51-3.38 (m, 2H), 3.18 (s, 3H), 2.63-2.55 (m, 2H), 2.21-2.09 (m, 2H).

### Example 130: Synthesis of Compound B80

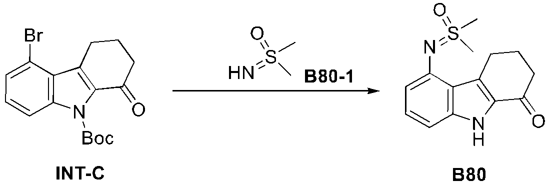

Under a nitrogen atmosphere at room temperature, to a solution of compound INT-C (200 mg, 0.516 mmol) and sodium tert-butoxide (148.81 mg, 1.548 mmol) in 1,4-dioxane (5 mL) were added (2-dicyclohexylphosphino-2',6'-diisopropoxy-1,1'-biphenyl)(2-amino-1,1'-biphenyl-2-yl)palladium(II) methanesulfonate (86.34 mg, 0.103 mmol), 2-dicyclohexylphosphino-2',6'-diisopropoxybiphenyl (48.17 mg, 0.103 mmol), and compound B80-1 (144.22 mg, 1.548 mmol). The reaction mixture was stirred at 100 °C overnight. After cooling to room temperature, the reaction was quenched with water (60 mL). The mixture was extracted with ethyl acetate (3 × 60 mL). The combined organic phases were washed with saturated sodium chloride aqueous solution (30 mL), dried over anhydrous sodium sulfate, filtered, and concentrated under reduced pressure. The residue was purified by reverse-phase preparative chromatography (mobile phase A: water (10 mmol/L ammonium bicarbonate); mobile phase B: acetonitrile) to afford compound B80 as a yellow solid (40.2 mg, 28.04%). ESI/MS (m/z): 277.2 [M+H]⁺.¹H NMR (400 MHz, DMSO-*d*₆) δ 11.30 (s, 1H), 7.05 (dd, *J* = 8.2, 7.5 Hz, 1H), 6.87 (dd, *J =* 8.2, 0.8 Hz, 1H), 6.60 (dd, *J =* 7.5, 0.8 Hz, 1H), 3.26 (s, 6H), 3.19 (t, *J =* 6.0 Hz, 2H), 2.49-2.45 (m, 2H), 2.12-2.07 (m, 2H).

### Example 131: Synthesis of Compound B81

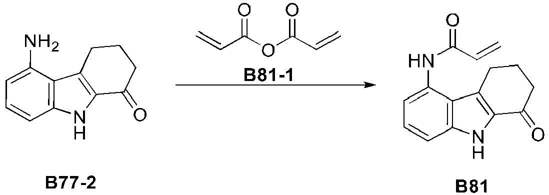

To a solution of compound B77-2 (130 mg, 0.621 mmol) and pyridine (98.19 mg, 1.242 mmol) in dichloromethane (4 mL) was added compound B81-1 (93.93 mg, 0.745 mmol). The reaction mixture was stirred at room temperature overnight. The reaction was quenched with water (10 mL) and extracted with ethyl acetate (3 × 10 mL). The combined organic phases were washed with saturated sodium chloride aqueous solution (10 mL), dried over anhydrous sodium sulfate, filtered, and concentrated under reduced pressure. The residue was purified by reverse-phase preparative chromatography (mobile phase A: water (10 mmol/L ammonium bicarbonate); mobile phase B: acetonitrile) to afford compound B81 as a white solid (26.2 mg, 16.45%). ESI/MS (m/z): 255.2 [M+H]⁺.¹H NMR (400 MHz, DMSO-*d*₆) δ 11.66 (br, 1H), 9.86 (s, 1H), 7.27-7.26 (m, 1H), 7.25 (d, *J =* 1.7 Hz, 1H), 7.04-7.00 (m, 1H), 6.52 (dd, *J=* 17.1, 10.2 Hz, 1H), 6.27 (dd, *J =* 17.1, 2.0 Hz, 1H), 5.78 (dd, *J =* 10.2, 2.1 Hz, 1H), 3.04 (t, *J =* 6.0 Hz, 2H), 2.55-2.51 (m, 2H), 2.13-2.05 (m, 2H).

### Example132: Synthesis of Compound B82

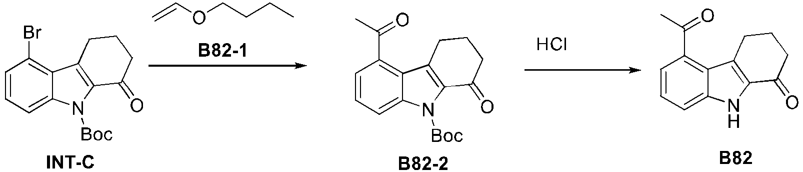

Under a nitrogen atmosphere, to a solution of compound INT-C (200 mg, 0.516 mmol) and potassium carbonate (214.00 mg, 1.548 mmol) in 1,4-dioxane (6 mL) were added tris(dibenzylideneacetone)dipalladium (94.53 mg, 0.103 mmol), 1,3-bis(diphenylphosphino)propane (42.58 mg, 0.103 mmol), and compound B82-1 (155.09 mg, 1.548 mmol). The reaction mixture was stirred at 110 °C for 3 hours. After cooling to room temperature, the reaction was quenched with water (70 mL), and the mixture was extracted with ethyl acetate (3 × 50 mL). The combined organic phases were washed with saturated sodium chloride aqueous solution (30 mL), dried over anhydrous sodium sulfate, filtered, and concentrated under reduced pressure. The residue was purified by silica gel column chromatography (petroleum ether:ethyl acetate = 3: 1) to afford compound B82-2 as a yellow solid (86 mg, 49.62%). ESI/MS (m/z): 328.1 [M+H]⁺.

At room temperature, 4M hydrogen chloride in 1,4-dioxane (1 mL) was added to a solution of compound B82-2 (66 mg, 0.197 mmol) in 1,4-dioxane (3 mL). The mixture was stirred for 1 hour. The reaction mixture was directly concentrated under reduced pressure, and the residue was purified by reverse-phase preparative chromatography (mobile phase A: water (0.1% formic acid); mobile phase B: acetonitrile) to afford compound B82 as a pale yellow solid (11.2 mg, 24.95%). ESI/MS (m/z): 228.2 [M+H]⁺.¹H NMR (300 MHz, DMSO-*d₆*) δ 11.96 (s, 1H), 7.66-7.64 (m, 1H), 7.63-7.61 (m, 1H), 7.38 (dd, *J =* 8.4, 7.2 Hz, 1H), 2.97 (t, *J =* 6.0 Hz, 2H), 2.66 (s, 3H), 2.59-2.53 (m, 2H), 2.12-2.03 (m, 2H).

### Example 133: Synthesis of Compound B83 and B84

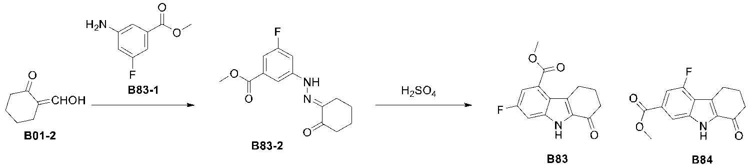

At 0 °C, concentrated hydrochloric acid (1.25 mL) and a solution of sodium nitrite (0.41 g, 5.92 mmol) in water (5 mL) were sequentially added to a solution of compound B83-1 (0.5 g, 2.76 mmol) in water (20 mL). The mixture was stirred for 10 minutes. A solution of compound B01-2 (0.45 g, 3.55 mmol) in methanol (10 mL) was combined with a solution of sodium acetate (0.53 g, 6.5 mmol) in water (5 mL) and cooled to 0 °C. The previously prepared diazonium salt solution was added dropwise to this mixture, and stirring was continued at 0 °C for 30 minutes. Solids precipitated from the system, which were collected by filtration. The filter cake was washed with water and dried to afford compound B83-2 as an orange solid (0.55 g, 66.8%).

A 1.8M sulfuric acid solution (3 mL) was added to a solution of compound B83-2 (0.55 g, 2.0 mmol) in acetonitrile (6 mL). The reaction mixture was stirred at 80 °C for 3 hours. After cooling to room temperature, the mixture was diluted with ethyl acetate (30 mL). The organic phase was washed with saturated sodium chloride aqueous solution, dried over anhydrous sodium sulfate, filtered, and concentrated under reduced pressure. The residue was purified by silica gel column chromatography (petroleum ether:ethyl acetate = 10:1) to afford compound B83 as a white solid (60 mg, 11.5%) and compound B84 as a white solid (38 mg, 7.3%).

Compound **B83:** ESI/MS (m/z) 262.0 [M+H]⁺.¹H NMR (500 MHz, DMSO-*d*₆) δ 12.10 (s, 1H), 7.41 (dd, *J =* 9.9, 2.4 Hz, 1H), 7.38 (dd, *J =* 9.1, 2.4 Hz, 1H), 3.92 (s, 3H), 3.05 (t, *J =* 6.0 Hz, 2H), 2.58-2.56 (m, 2H), 2.13-2.08 (m, 2H).

Compound **B84:** ESI/MS (m/z) 262.0[M+H]⁺.¹H NMR (500 MHz, DMSO-*d*₆) δ 12.34 (s, 1H), 7.88 (d, *J =* 0.8 Hz, 1H), 7.28 (dd, *J=* 11.4, 0.7 Hz, 1H), 3.88 (s, 3H), 3.08 (t, *J =* 6.0 Hz, 2H), 2.62-2.60 (m, 2H), 2.21-2.16 (m, 2H).

### Example 134: Synthesis of Compound B85 and B86

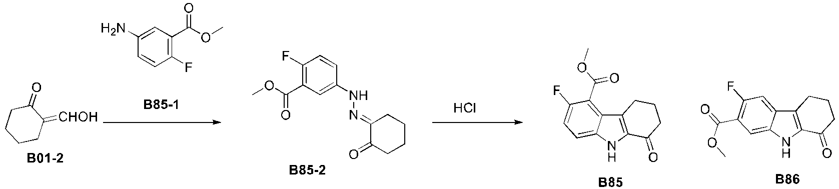

At 0 °C, concentrated hydrochloric acid (1.25 mL) and a solution of sodium nitrite (0.41 g, 5.92 mmol) in water (5 mL) were sequentially added to a solution of compound B85-1 (0.5 g, 2.96 mmol) in water (20 mL). The mixture was stirred for 10 minutes. A solution of compound B01-2 (0.45 g, 3.6 mmol) in methanol (10 mL) was combined with a solution of sodium acetate (0.53 g, 6.5 mmol) in water (5 mL), and the system was cooled to 0 °C. The previously prepared diazonium salt solution was added dropwise to the reaction system, and stirring was continued for 30 minutes. Solids precipitated from the system, which were collected by suction filtration. The filter cake was washed with water and dried to afford crude compound B85-2 as an orange solid (0.38 g, 46.1%).

Concentrated hydrochloric acid (0.2 mL) was added to a solution of compound B85-2 (0.38 g, 1.36 mmol) in methanol (6 mL). The reaction mixture was stirred at 60 °C for 1 hour. After cooling to room temperature, the mixture was extracted with ethyl acetate (30 mL). The organic phase was washed with saturated sodium chloride aqueous solution, dried over anhydrous sodium sulfate, filtered, and concentrated under reduced pressure. The crude product was purified by silica gel column chromatography (petroleum ether:ethyl acetate = 10:1) to afford a mixture. The mixture was separated by reverse-phase preparative chromatography (mobile phase A: water (10 mmol/L ammonium bicarbonate); mobile phase B: acetonitrile) to afford compound B85 as a pale yellow solid (21.9 mg, 73.22%) and compound B86 as a pale yellow solid (8.5 mg, 28.77%).

**B85:** ESI/MS (m/z): 262.2 [M+H]⁺.¹H NMR (400 MHz, DMSO-*d*₆) δ 12.06 (s, 1H), 7.58 (dd, *J=* 9.1, 4.4 Hz, 1H), 7.27 (dd, *J=* 10.4, 9.0 Hz, 1H), 3.94 (s, 3H), 2.81 (t, *J= 6.0* Hz, 2H), 2.62-2.54 (m, 2H), 2.12 (p, *J =* 6.3 Hz, 2H)_{∘}

**B86:** ESI/MS(m/z): 262.2 [M+H]⁺.¹H NMR (400 MHz, DMSO-*d*₆) δ 11.95 (s, 1H), 7.95 (d, *J= 6.0* Hz, 1H), 7.61 (d, *J=* 11.7 Hz, 1H), 3.87 (s, 3H), 2.94 (t, *J=* 6.0 Hz, 2H), 2.64-2.57 (m, 2H), 2.20-2.13 (m, 2H).

### Example 135: Synthesis of Compound B87 and B88

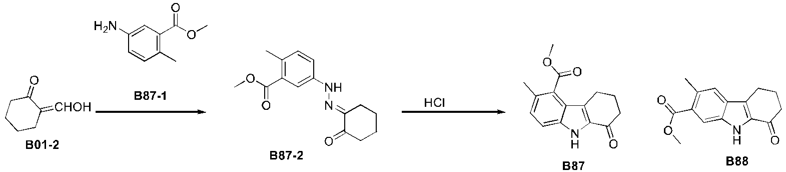

At 0 °C, concentrated hydrochloric acid (1.25 mL) and a solution of sodium nitrite (0.41 g, 6.0 mmol) in water (5 mL) were sequentially added to a solution of compound B87-1 (0.5 g, 3.0 mmol) in water (20 mL). The mixture was stirred for 10 minutes. A solution of compound B01-2 (0.45 g, 3.6 mmol) in methanol (10 mL) was combined with a solution of sodium acetate (0.54 g, 6.6 mmol) in water (5 mL), and the system was cooled to 0 °C. The previously prepared diazonium salt solution was added dropwise to the reaction system, and stirring was continued for 30 minutes. Solids precipitated from the system, which were collected by suction filtration. The filter cake was washed with water and dried to afford crude compound B87-2 as a red solid (0.61 g, 74.2%).

Concentrated hydrochloric acid (0.2 mL) was added to a solution of compound B87-2 (0.61 g, 2.2 mmol) in methanol (6 mL). The reaction was carried out at 60 °C for 1 hour. After cooling to room temperature, ethyl acetate (30 mL) was added. The organic phase was washed with saturated sodium chloride aqueous solution, dried over anhydrous sodium sulfate, filtered, and concentrated under reduced pressure. The crude product was purified by silica gel column chromatography (petroleum ether:ethyl acetate = 10:1) to afford a brown solid mixture. The mixture was separated by reverse-phase preparative chromatography (mobile phase A: water (10 mmol/L ammonium bicarbonate); mobile phase B: acetonitrile) to afford compound B87 as a pale yellow solid (25.2 mg, 72.86%) and compound B88 as a pale yellow solid (12.4 mg, 35.71%).

Compound **B87:** ESI/MS (m/z): 258.2 [M+H]⁺. ¹H NMR (400 MHz, DMSO-*d*₆) δ 11.79 (s, 1H), 7.42 (d, *J =* 8.5 Hz, 1H), 7.20 (d, *J =* 8.5 Hz, 1H), 3.92 (s, 3H), 2.75 (t, *J= 6.0* Hz, 2H), 2.59-2.51 (m, 2H), 2.34 (s, 3H), 2.15-2.06 (m, 2H).

Compound **B88:** ESI/MS(m/z): 258.2 [M+H]⁺.¹H NMR (400 MHz, DMSO-*d*₆) δ 11.79 (s, 1H), 7.95 (s, 1H), 7.58 (s, 1H), 3.84 (s, 3H), 2.94 (t, *J=* 6.0 Hz, 2H), 2.62-2.58 (m, 2H), 2.57 (s, 3H), 2.21-2.12 (m, 2H).

### Example 136: Synthesis of Compound B89 and B90

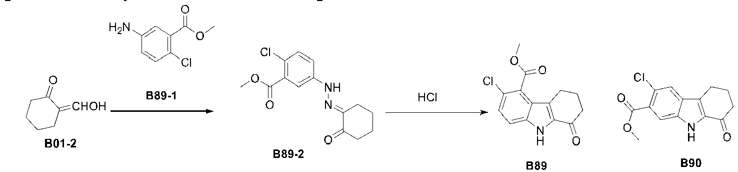

At 0 °C, concentrated hydrochloric acid (1.1 mL) and a solution of sodium nitrite (0.37 g, 5.4 mmol) in water (5 mL) were sequentially added to a solution of compound B89-1 (0.5 g, 2.69 mmol) in water (20 mL). The mixture was stirred for 10 minutes. A solution of compound B01-2 (0.41 g, 3.2 mmol) in methanol (10 mL) was combined with a solution of sodium acetate (0.48 g, 5.9 mmol) in water (5 mL), and the system was cooled to 0 °C. The previously prepared diazonium salt solution was added dropwise to the reaction system, and stirring was continued for 30 minutes. Solids precipitated from the system, which were collected by suction filtration. The filter cake was washed with water and dried to afford crude compound B89-2 as a brown solid (0.56 g, 70.6%).

Concentrated hydrochloric acid (0.2 mL) was added to a solution of compound B89-2 (0.56 g, 1.9 mmol) in methanol (6 mL). The mixture was stirred at 60 °C for 1 hour. After cooling to room temperature, the reaction mixture was diluted with ethyl acetate (30 mL). The organic phase was washed with saturated sodium chloride aqueous solution, dried over anhydrous sodium sulfate, filtered, and concentrated under reduced pressure. The crude product was purified by silica gel column chromatography (petroleum ether:ethyl acetate = 10:1) to afford a brown solid mixture. The mixture was separated by reverse-phase preparative chromatography (mobile phase A: water (10 mmol/L ammonium bicarbonate); mobile phase B: acetonitrile) to afford compound B89 as a pale yellow solid (22.4 mg, 53.57%) and compound B90 as a pale yellow solid (12.3 mg, 29.38%).

Compound **B89** (22.4 mg, 53.57%). ESI/MS (m/z): 278.1 [M+H]⁺.¹H NMR (400 MHz, DMSO-*d*₆) δ 11.97 (s, 1H), 7.53 (d, *J=* 8.9 Hz, 1H), 7.39 (d, *J=* 8.8 Hz, 1H), 3.96 (s, 3H), 2.74 (t, *J=* 6.0 Hz, 2H), 2.63-2.53 (m, 2H), 2.13 (p, *J=* 6.2 Hz, 2H).

Compound **B90**(12.3 mg, 29.38%).ESI/MS(m/z): 278.1 [M+H]⁺.¹H NMR (400 MHz, DMSO-*d*₆) δ 12.08 (s, 1H), 7.91 (s, 1H), 7.87 (s, 1H), 3.87 (s, 3H), 2.96 (t, *J* = 6.0 Hz, 2H), 2.66-2.55 (m, 2H), 2.23-2.12 (m, 2H).

### Example 137: Synthesis of Compound B91 and B92

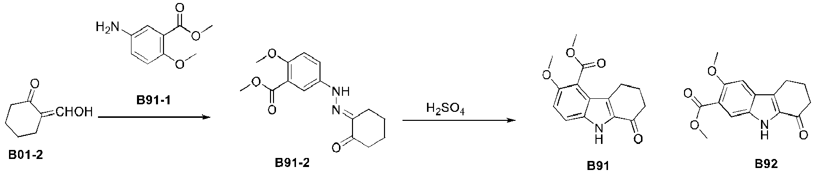

At 0 °C, concentrated hydrochloric acid (1.25 mL) and a solution of sodium nitrite (0.38 g, 5.5 mmol) in water (5 mL) were sequentially added to a solution of compound B91-1 (0.5 g, 2.76 mmol) in water (20 mL). The mixture was stirred for 10 minutes. A solution of compound B01-2 (0.42 g, 3.31 mmol) in methanol (15 mL) was combined with a solution of sodium acetate (0.53 g, 6.1 mmol) in water (10 mL), and the system was cooled to 0 °C. The previously prepared diazonium salt solution was added dropwise to the reaction system, and stirring was continued for 30 minutes. Solids precipitated from the system, which were collected by suction filtration. The filter cake was washed with water and dried to afford compound B91-2 as a reddish-brown solid (0.45 g, 56.1%).

A 1.8 M sulfuric acid solution (2 mL) was added to a solution of compound B91-2 (0.45 g, 1.55 mmol) in acetonitrile (5 mL). The reaction mixture was stirred at 80 °C for 3 hours. After cooling to room temperature, the mixture was diluted with ethyl acetate (30 mL). The organic phase was washed with saturated sodium chloride aqueous solution, dried over anhydrous sodium sulfate, filtered, and concentrated under reduced pressure. The residue was purified by silica gel column chromatography (petroleum ether:ethyl acetate = 5:1) to afford compound B91 as a pale yellow solid (60 mg, 14.2%) and compound B92 as a pale yellow solid (50 mg, 11.8%).

Compound **B91:** ESI/MS (m/z): 262.1 [M+H]⁺.¹H NMR (500 MHz, DMSO-*d*₆) δ 11.73 (s, 1H), 7.49 (d, *J* = 9.1 Hz, 1H), 7.24 (d, *J =* 9.1 Hz, 1H), 3.97 (s, 3H), 3.81 (s, 3H), 2.72 (t, *J =* 6.0 Hz, 2H), 2.58-2.54 (m, 2H), 2.13-2.08 (m, 2H)_{∘} ESI/MS (m/z) 274.0[M+H]⁺.

Compound **B92:** ¹H NMR (500 MHz, DMSO-*d*₆) δ 11.70 (s, 1H), 7.71 (s, 1H), 7.28 (s, 1H), 3.84 (s, 3H), 3.81 (s, 3H), 2.95 (t, *J=* 6.0 Hz, 2H), 2.60-2.57 (m, 2H), 2.19-2.14 (m, 2H).ESI/MS (m/z) 274.0[M+H]⁺.

### Example 138: Synthesis of Compound B93

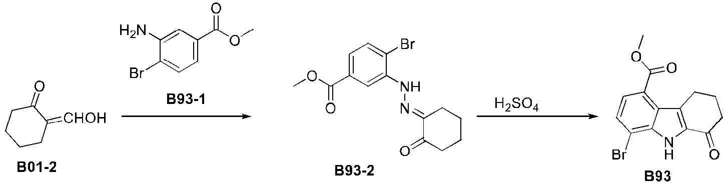

At 0 °C, concentrated hydrochloric acid (1.8 mL) and a solution of sodium nitrite (0.6 g, 8.70 mmol) in water (5 mL) were sequentially added to a solution of compound B93-1 (1.0 g, 4.35 mmol) in water (20 mL). The mixture was stirred for 10 minutes. A solution of compound B01-2 (0.66 g, 5.22 mmol) in methanol (10 mL) was combined with a solution of sodium acetate (0.78 g, 9.57 mmol) in water (10 mL), and the system was cooled to 0 °C. The previously prepared diazonium salt solution was added dropwise to the reaction system, and stirring was continued in an ice bath for 30 minutes. Solids precipitated from the system, which were collected by suction filtration. The filter cake was washed with water and dried to afford crude compound B93-2 as an orange solid (0.91 g, 61.7%).

A 1.8 M sulfuric acid solution (15 mL) was added to a solution of compound B93-2 (0.91 g, 2.68 mmol) in acetonitrile (30 mL). The reaction mixture was stirred at 80 °C for 3 hours. After cooling to room temperature, the mixture was diluted with ethyl acetate (50 mL). The organic phase was washed with saturated sodium chloride aqueous solution, dried over anhydrous sodium sulfate, filtered, and concentrated under reduced pressure. The crude product was purified by silica gel column chromatography (petroleum ether:ethyl acetate = 5:1) to afford compound B93 as an orange-red solid (0.47 g, 54.4%). ESI/MS m/z 322.0 & 323.8 [M+H]⁺.¹H NMR (500 MHz, DMSO-*d*₆) δ12.05 (s, 1H), 7.65 (d, *J =* 7.9 Hz, 1H), 7.50 (d, *J =* 7.9 Hz, 1H), 3.91 (s, 3H), 3.06 (t, *J =* 6.0 Hz, 2H), 2.63-2.59 (m, 2H), 2.15-2.08 (m, 2H).

### Example 139: Synthesis of Compound B94

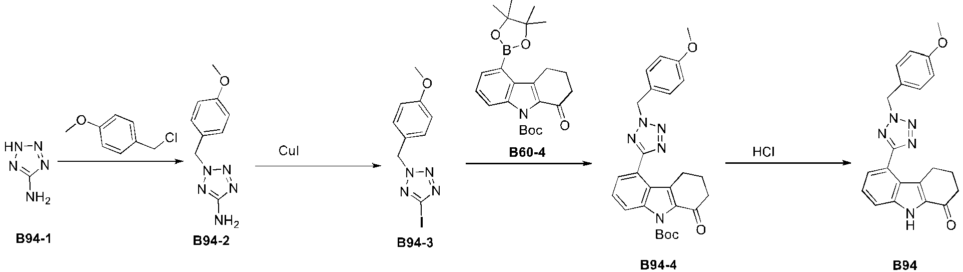

At room temperature, 4-methoxybenzyl chloride (13.81 g, 88.162 mmol) was added portionwise to a solution of compound B94-1 (5 g, 8.775 mmol) and potassium carbonate (16.25 g, 117.550 mmol) in N,N-dimethylformamide (100 mL). The reaction mixture was stirred at 80 °C overnight. After cooling to room temperature, the reaction was quenched with water (30 mL) and extracted with dichloromethane (2 × 50 mL). The combined organic phases were washed with saturated sodium chloride aqueous solution (50 mL), dried over anhydrous sodium sulfate, and concentrated under reduced pressure. The residue was purified by silica gel column chromatography (petroleum ether:ethyl acetate = 1:3) to afford compound B94-2 as a white solid (4.1 g, 32.16%). ESI/MS (m/z): 206.2 [M+H]⁺.

At room temperature, diiodomethane (2.85 g, 10.645 mmol) and isoamyl nitrite (5.67 g, 48.385 mmol) were added portionwise to a solution of compound B94-2 (2 g, 9.677 mmol) and copper(I) iodide (184.31 mg, 0.968 mmol) in tetrahydrofuran (100 mL). The reaction mixture was stirred at 70 °C for 2 hours. After cooling to room temperature, the reaction was quenched with water (30 mL) and extracted with dichloromethane (2 × 100 mL). The combined organic phases were washed with sodium thiosulfate solution (50 mL), dried over anhydrous sodium sulfate, and concentrated under reduced pressure. The residue was purified by silica gel column chromatography (petroleum ether:ethyl acetate = 5:1) to afford compound B94-3 as a yellow solid (700 mg, 21.59%). ESI/MS (m/z): 316.9 [M+H]⁺.

At room temperature, [1,1'-bis(diphenylphosphino)ferrocene]dichloropalladium(II) dichloromethane complex (196.27 mg, 0.241 mmol) and potassium carbonate (665.97 mg, 4.819 mmol) were added portionwise to a solution of compound B94-3 (800 mg, 2.409 mmol) and compound B60-4 (1.62 g, 3.624 mmol) in 1,4-dioxane (10 mL) and water (3 mL). The reaction mixture was stirred at 100 °C for 2 hours. After cooling to room temperature, the reaction was quenched with water and extracted with dichloromethane (3 × 30 mL). The combined organic phases were washed with saturated sodium chloride aqueous solution (30 mL), dried over anhydrous sodium sulfate, and concentrated under reduced pressure. The residue was purified by silica gel column chromatography (petroleum ether:ethyl acetate = 1:1) to afford compound B94-4 as a yellow solid (660 mg, 57.85%). ESI/MS (m/z): 418.2 [M+H-56]⁺.

At room temperature, 4M hydrogen chloride in 1,4-dioxane (1 mL) was added to compound B94-4 (50 mg, 0.106 mmol). The reaction mixture was stirred at 80 °C for 30 minutes. After cooling to room temperature, the mixture was basified to pH 8 with sodium hydroxide solution. The mixture was extracted with ethyl acetate (3 × 30 mL). The combined organic phases were washed with saturated sodium chloride aqueous solution (30 mL), dried over anhydrous sodium sulfate, and concentrated under reduced pressure. The residue was purified by silica gel column chromatography (petroleum ether:ethyl acetate = 1:1) to afford compound B94 as a white solid (31.6 mg, 77.66%). ESI/MS (m/z): 374.0 [M+H]⁺.¹H NMR (400 MHz, DMSO-*d₆*) δ 11.94 (s, 1H), 7.58 (dd, *J =* 8.0, 1.3 Hz, 1H), 7.47-7.45 (m, 2H), 7.44-7.39 (m, 2H), 7.02-6.96 (m, 2H), 5.95 (s, 2H), 3.76 (s, 3H), 2.60 (t, *J=* 6.0 Hz, 2H), 2.53-2.51 (m, 2H), 1.98-1.88 (m, 2H).

### Example 140: Synthesis of Compound E63

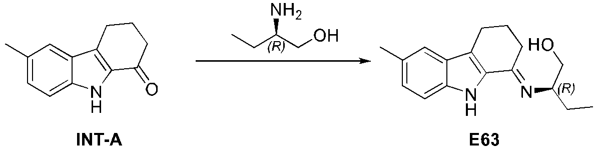

The toluene solution (5 mL) of compound INT-A (498.1 mg, 2.500 mmol) and (R)-(-)-2-amino-1-butanol (668.6 mg, 7.500 mmol) was stirred at 130 °C for 4 hours. After cooling to room temperature, the solvent was removed by concentration under vacuum. The mixture was dissolved in a small amount of ethanol, and then poured into a large volume of water. The precipitated solid was collected by filtration and dried under vacuum to afford compound E63 as a light yellow solid (277.3 mg, 41.02%). ESI/MS (m/z) 271.0 [M+H]⁺.¹H NMR (400 MHz, DMSO-*d₆*) δ 10.70 (s, 1H), 7.28 (s, 1H), 7.27 (d, *J =* 6.1 Hz, 1H), 6.95 (dd, J = 8.5, 1.1 Hz, 1H), 4.49 (s, 1H), 3.67-3.59 (m, 1H), 3.54-3.47 (m, 1H), 3.42-3.37 (m, 1H), 2.76 (t, *J =* 6.0 Hz, 2H), 2.70-2.61 (m, 1H), 2.59-2.51 (m, 1H), 2.36 (s, 3H), 2.01-1.91 (m, 2H), 1.72-1.61 (m, 1H), 1.47-1.36 (m, 1H), 0.82 (t, *J =* 7.4 Hz, 3H).

### Example 141: Synthesis of Compound B80 and E64

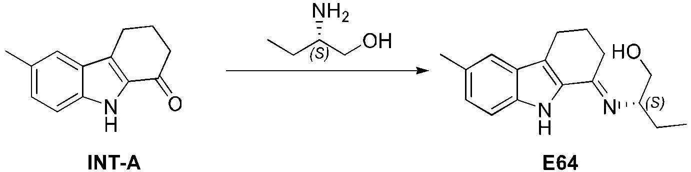

The toluene solution (5 mL) of compound INT-A (498.1 mg, 2.500 mmol) and (S)-(+)-2-amino-1-butanol (668.6 mg, 7.500 mmol) was stirred at 130 °C for 4 hours. After cooling to room temperature, the solvent was removed by concentration under vacuum. The mixture was dissolved in a small amount of ethanol and then poured into a large volume of water. The precipitated solid was collected by filtration and dried under vacuum to afford compound E64 as a light yellow solid (217.0 mg, 32.10%). ESI/MS (m/z) 271.0 [M+H]⁺.¹H NMR (400 MHz, DMSO-*d₆*) δ 10.70 (s, 1H), 7.28 (s, 1H), 7.27 (d, *J =* 6.4 Hz, 1H), 6.97-6.92 (m, 1H), 4.49 (t, *J =* 5.3 Hz, 1H), 3.66-3.59 (m, 1H), 3.53-3.47 (m, 1H), 3.42-3.36 (m, 1H), 2.76 (t, *J =* 5.9 Hz, 2H), 2.70-2.62 (m, 1H), 2.59-2.51 (m, 1H), 2.35 (s, 3H), 2.01-1.93 (m, 2H), 1.72-1.61 (m, 1H), 1.48-1.36 (m, 1H), 0.82 (t, *J=* 7.4 Hz, 3H).

### Example 142: Synthesis of Compound E65

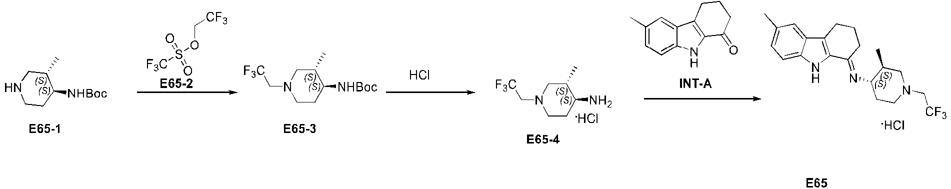

At room temperature, triethylamine (1.27 g, 12.66 mmol) was slowly added dropwise to a solution of compound E65-1 (900 mg, 4.200 mmol) and compound E65-2 (1.95 g, 8.400 mmol) in tetrahydrofuran (15 mL). The reaction mixture was stirred at 60 °C for 3 hours. After cooling to room temperature, the mixture was concentrated under reduced pressure. The residue was purified by reverse-phase preparative chromatography (mobile phase A: water (10 mmol/L ammonium bicarbonate); mobile phase B: acetonitrile) to afford compound E65-3 as a white solid (1.15 g, 91.39%). ESI/MS (m/z): 297.0 [M+H]⁺.

At room temperature, a 4M hydrogen chloride solution in 1,4-dioxane (13.5 mL, 20.000 mmol) was slowly added dropwise to a solution of compound E65-3 (1 g, 3.337 mmol) in 1,4-dioxane (5 mL). The reaction mixture was stirred at room temperature for 2 hours, and then n-hexane (30 mL) was added. The mixture was filtered, and the filter cake was washed with n-hexane (3 × 10 mL). The resulting solid was dried under vacuum to afford compound E65-4 as a white solid (870 mg, crude). This compound was used directly in the next step without further purification. ESI/MS (m/z): 197.1 [M+H]⁺.

At room temperature, tetraethyl titanate (454 mg, 1.906 mmol) was added to a solution of compound E65-4 (452 mg, 1.906 mmol) and compound INT-A (200 mg, 0.953 mmol) in toluene (5 mL). The reaction mixture was stirred at 100 °C for 6 hours. After cooling to room temperature, the mixture was diluted with water (20 mL) and dichloromethane (20 mL), and then filtered. The filter cake was washed with dichloromethane (10 mL). The filtrate was extracted with dichloromethane (20 mL). The organic phase was washed with saturated sodium chloride aqueous solution (10 mL), dried over anhydrous sodium sulfate, and concentrated to dryness. The residue was washed with ethanol (10 mL). The resulting solid was dried under vacuum to afford compound E65 as a yellow solid (85.8 mg, 23.53%). ESI/MS (m/z): 378.1 [M+H]⁺.¹H NMR (400 MHz, DMSO-*d*₆) δ 12.74 (s, 1H), 12.44 (d, *J =* 8.7 Hz, 1H), 7.54 (s, 1H), 7.39 (d, *J=* 8.5 Hz, 1H), 7.32 (d, *J =* 8.6 Hz, 1H), 3.87-3.74 (m, 1H), 3.24 (q, *J =* 10.2 Hz, 2H), 3.16-3.06 (m, 2H), 3.01-2.98 (m, 4H), 2.54-2.48 (m, 1H), 2.40 (s, 3H), 2.28-2.17 (m, 2H), 2.14-2.11 (m, 2H), 2.00-1.87 (m, 2H), 0.88 (d, *J =* 6.0 Hz, 3H).

### Example 143: Synthesis of Compound E66

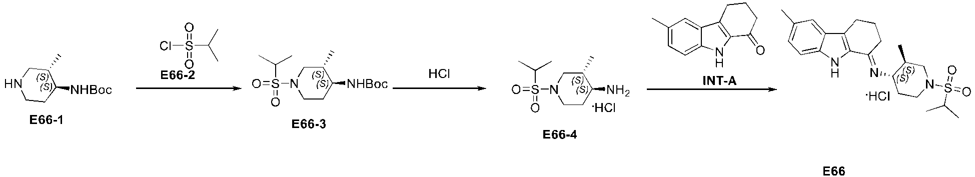

At 0 °C, N,N-diisopropylethylamine (1.6 g, 12.600 mmol) was slowly added dropwise to a solution of compound E66-1 (900 mg, 4.200 mmol) and compound E66-2 (1.2 g, 8.400 mmol) in dichloromethane (15 mL). The reaction mixture was stirred at room temperature overnight. The reaction was quenched with water (30 mL), and the mixture was extracted with dichloromethane (3 × 30 mL). The combined organic phases were washed with saturated sodium chloride aqueous solution (20 mL), dried over anhydrous sodium sulfate, and concentrated under reduced pressure. The residue was purified by silica gel column chromatography (petroleum ether: ethyl acetate = 2:1) to afford compound E66-3 as a light-yellow solid (830 mg, 57.98%). ESI/MS (m/z): 265.1 [M+H-56]⁺.

At room temperature, a 4M hydrogen chloride solution in 1,4-dioxane (14.1 mL, 56.399 mmol) was slowly added dropwise to a solution of compound E66-3 (780 mg, 2.434 mmol) in 1,4-dioxane (3 mL). After stirring for 2 hours, n-hexane (30 mL) was added. The mixture was filtered, and the filter cake was washed with n-hexane (3 × 10 mL). The resulting solid was dried under vacuum to afford compound E66-4 as a white solid (630 mg, crude). ESI/MS (m/z): 221.2 [M+H]⁺.

At room temperature, tetraethyl titanate (344 mg, 1.506 mmol) was slowly added dropwise to a solution of compound E66-4 (387 mg, 1.506 mmol) and compound INT-A (150 mg, 0.753 mmol) in toluene (4 mL). The mixture was heated to 100 °C and stirred for 6 hours. After cooling to room temperature, the reaction mixture was diluted with water (20 mL) and dichloromethane (20 mL), and then filtered. The filter cake was washed with dichloromethane (10 mL). The filtrate was extracted with dichloromethane (20 mL). The combined organic phases were washed with saturated sodium chloride aqueous solution (20 mL), dried over anhydrous sodium sulfate, and concentrated under reduced pressure. The residue was triturated with diethyl ether (10 mL). The resulting solid was dried under vacuum to afford compound E66 as a brownish-yellow solid (89.0 mg, 26.98%). ESI/MS (m/z): 402.1 [M+H]⁺.¹H NMR (400 MHz, DMSO-*d*₆) δ 12.57 (s, 1H), 12.39 (d, *J=* 8.7 Hz, 1H), 7.55 (s, 1H), 7.41 (d, *J=* 8.5 Hz, 1H), 7.33 (d, *J=* 8.5 Hz, 1H), 4.04-4.01 (m, 1H), 3.78-3.71 (m, 2H), 3.41-3.15 (m, 2H), 3.11-3.03 (m, 2H), 3.02-2.95 (m, 2H), 2.75 (t, *J= 12.0* Hz, 1H), 2.40 (s, 3H), 2.19-2.05 (m, 4H), 1.92-1.77 (m, 1H), 1.25 (d, *J* = 6.8 Hz, 6H), 0.92 (d, *J=* 6.5 Hz, 3H).

### Example 144: Synthesis of Compound E67

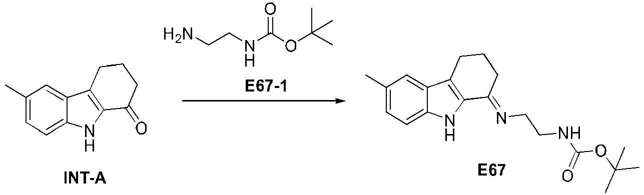

The toluene solution (5 mL) of compound INT-A (199.3 mg, 1.000 mmol) and compound E67-1 (480.7 mg, 3.000 mmol) was stirred at 135 °C for 5 hours. After cooling to room temperature, the mixture was concentrated under vacuum. The crude product was purified by reverse-phase column chromatography (10-90% acetonitrile/water) to afford compound E67 as a white solid (220.0 mg, 64.43%). ESI/MS (m/z) 342.1 [M+H]⁺.¹H NMR (500 MHz, DMSO-*d*₆) δ 10.81 (s, 1H), 7.28 (s, 1H), 7.24 (d, *J =* 8.3 Hz, 1H), 6.96 (d, *J =* 8.3 Hz, 1H), 6.78 (t, *J =* 5.2 Hz, 1H), 3.49 (t, *J=* 6.7 Hz, 2H), 3.26-3.19 (m, 2H), 2.76 (t, *J=* 6.0 Hz, 2H), 2.56-2.52 (m, 2H), 2.36 (s, 3H), 2.00-1.94 (m, 2H), 1.39 (s, 9H).

### Example 145: Synthesis of Compound E68

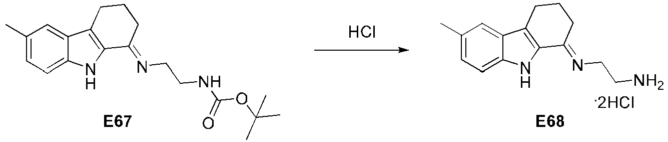

At room temperature, compound E67 (90.0 mg, 0.264 mmol) was added to a 4M hydrogen chloride solution in ethyl acetate (3 mL) and stirred for 10 minutes. Filtration afforded compound E68 as a yellow solid. ESI/MS (m/z) 242.1 [M+H]⁺.¹H NMR (500 MHz, DMSO-*d*₆) δ 12.69 (s, 1H), 12.43 (s, 1H), 8.35 (s, 3H), 7.55 (s, 1H), 7.41 (d, *J* = 8.5 Hz, 1H), 7.33 (d, *J =* 8.5 Hz, 1H), 4.05 (t, *J =* 6.2 Hz, 2H), 3.28-3.33 (m, 2H), 3.07 (t, *J =* 5.6 Hz, 2H), 3.00 (t, *J =* 6.1 Hz, 2H), 2.40 (s, 3H), 2.16-2.11 (m, 2H).

### Example 146: Synthesis of Compound E69

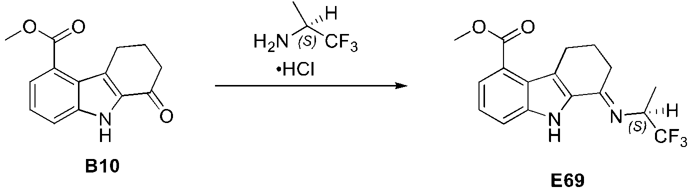

A solution of compound B10 (243.3 mg, 1.000 mmol), (S)-1,1,1-trifluoroisopropylamine hydrochloride (448.6 mg, 3.000 mmol), sodium acetate (98.4 mg, 1.200 mmol), and pyrrolidine (71.1 mg, 1.000 mmol) in 2-methyltetrahydrofuran (5 mL) was stirred at 90 °C for 3 hours. The reaction mixture was diluted with ethyl acetate (100 mL) and washed with pure water (20 mL), then dried over anhydrous sodium sulfate. The organic phase was concentrated under vacuum and purified by reverse-phase silica gel column chromatography (10%-90% acetonitrile/water) to afford compound E69 as a light-yellow solid (34.1 mg, 10.08%). ESI/MS (m/z) 339.1 [M+H]⁺.¹H NMR (500 MHz, DMSO-*d*₆) δ 11.31 (s, 1H), 7.71 (dd, *J =* 8.2, 0.9 Hz, 1H), 7.52 (dd, *J =* 7.3, 0.9 Hz, 1H), 7.26-7.22 (m, 1H), 4.56-4.47 (m, 1H), 3.87 (s, 3H), 3.00-2.94 (m, 2H), 2.76-2.69 (m, 1H), 2.62-2.55 (m, 1H), 2.00-1.92 (m, 2H), 1.29 (d, *J=* 6.6 Hz, 3H).

### Example 147: Synthesis of Compound E70

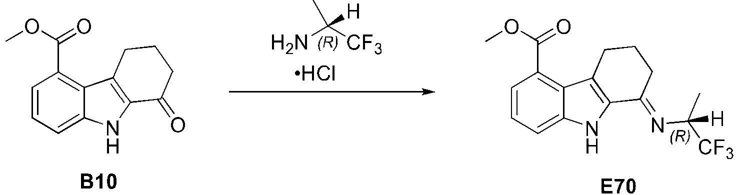

A solution of compound B10 (243.3 mg, 1.000 mmol), (R)-1,1,1-trifluoroisopropylamine hydrochloride (448.6 mg, 3.000 mmol), sodium acetate (98.4 mg, 1.200 mmol), and pyrrolidine (71.1 mg, 1.000 mmol) in 2-methyltetrahydrofuran (5 mL) was stirred at 90 °C for 3 hours. The reaction mixture was diluted with ethyl acetate (100 mL) and washed with pure water (20 mL), then dried over anhydrous sodium sulfate. After concentration under vacuum, the crude product was purified by reverse-phase silica gel column chromatography (acetonitrile: water = 10%-90%) to afford compound E70 as a light-yellow solid (31.4 mg, 9.28%). ESI/MS (m/z) 339.0 [M+H]⁺. ¹H NMR (500 MHz, DMSO-*d*₆) δ 11.31 (s, 1H), 7.71 (d, *J* = 8.0 Hz, 1H), 7.52 (d, *J=* 7.2 Hz, 1H), 7.24 (t, *J=* 7.8 Hz, 1H), 4.55-4.48 (m, 1H), 3.87 (s, 3H), 3.01-2.94 (m, 2H), 2.75-2.69 (m, 1H), 2.62-2.55 (m, 1H), 2.00-1.90 (m, 2H), 1.29 (d, *J =* 6.6 Hz, 3H).

### Example 148: Synthesis of Compound E71

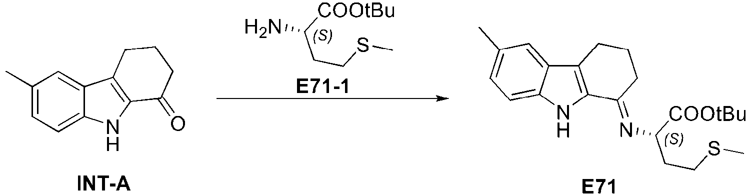

Under a nitrogen atmosphere, compound E71-1 (514 mg, 2.478 mmol) was added to a solution of compound INT-A (200 mg, 0.953 mmol) in toluene (4 mL). The mixture was heated to 140 °C and stirred overnight. After cooling to room temperature, the reaction mixture was concentrated under reduced pressure. The residue was purified by reverse-phase preparative chromatography (mobile phase A: water; mobile phase B: acetonitrile) to afford compound E71 as a reddish-brown yellow solid (50.6 mg, 13.18%). ESI/MS (m/z): 387.2 [M+H]⁺.¹H NMR (400 MHz, DMSO-*d*₆) δ 10.84 (s, 1H), 7.30 (s, 1H), 7.27 (d, *J=* 8.3 Hz, 1H), 6.99 (d, *J=* 8.3 Hz, 1H), 4.42 (dd, *J=* 7.9, 5.0 Hz, 1H), 2.79 (t, *J =* 6.0 Hz, 2H), 2.68-2.59 (m, 1H), 2.53 (t, *J=* 7.2 Hz, 2H), 2.36 (s, 3H), 2.18-2.12 (m, 1H), 2.18-2.07 (m, 2H), 2.06 (s, 3H), 2.01-2.05(m, 1H), 1.98-1.95 (m, 2H), 1.40 (s, 9H).

### Example 149: Synthesis of Compound E72

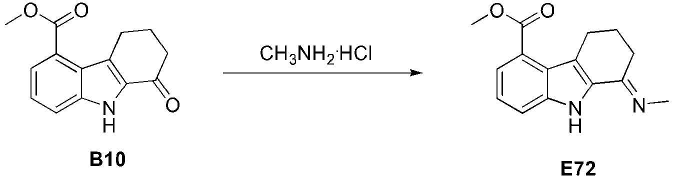

A solution of compound B10 (243.3 mg, 1.000 mmol), methylamine hydrochloride (202.6 mg, 3.000 mmol), and sodium acetate (98.4 mg, 3.000 mmol) in 2-methyltetrahydrofuran (4 mL) was stirred at 90 °C for 4 hours. The reaction mixture was diluted with ethyl acetate (100 mL) and washed with pure water (20 mL), then dried over anhydrous sodium sulfate. The organic phase was concentrated under vacuum, and the residue was washed with acetonitrile (5 mL) to afford compound E72 as a light-yellow solid (71.2 mg, 27.78%). ESI/MS (m/z) 257.1 [M+H]⁺.¹H NMR (500 MHz, DMSO-*d*₆) δ 12.84 (s, 1H), 7.75 (d, *J =* 8.3 Hz, 1H), 7.64 (d, *J =* 6.7 Hz, 1H), 7.52-7.48 (m, 1H), 3.91 (s, 3H), 3.35 (s, 3H), 3.09 (t, *J =* 6.1 Hz, 2H), 3.01 (t, *J* = 6.1 Hz, 2H), 2.08-2.03 (m, 2H).

### Example 150: Synthesis of Compound B95

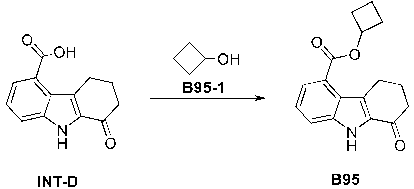

Under a nitrogen atmosphere at 0 °C, diethyl azodicarboxylate (76 mg, 0.44 mmol) was added to a mixture of compound INT-D (50 mg, 0.22 mmol), compound B95-1 (32 mg, 0.44 mmol), and triphenylphosphine (115 mg, 0.44 mmol) in tetrahydrofuran (1 mL). The reaction was warmed to room temperature and stirred for 6 hours. The mixture was diluted with ethyl acetate (30 mL) and washed with saturated sodium chloride aqueous solution. The organic phase was dried over anhydrous sodium sulfate and concentrated under reduced pressure. The residue was purified by silica gel column chromatography (ethyl acetate: petroleum ether = 1:6) to afford compound B95 as a white solid (6 mg, 9.6%). ESI/MS (m/z) 284.0 [M+H]⁺.

### Example 151: Synthesis of Compound B96

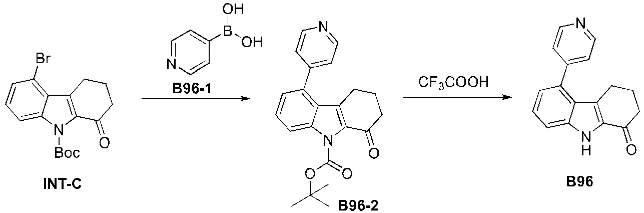

Under a nitrogen atmosphere, compound B96-1 (24.6 mg, 0.200 mmol), [1,1'-bis(diphenylphosphino)ferrocene]dichloropalladium (II) dichloromethane complex (4.0 mg, 0.005 mmol), potassium carbonate (27.6 mg, 0.200 mmol), and water (0.2 mL) were sequentially added to a solution of compound INT-C (36.4 mg, 0.100 mmol) in 1,4-dioxane (0.8 mL). The mixture was heated to 80 °C and stirred for 3 hours. After cooling to room temperature, the reaction mixture was diluted with ethyl acetate (30 mL). The organic phase was washed with saturated sodium chloride aqueous solution, dried over anhydrous sodium sulfate, and concentrated under reduced pressure. The residue was purified by reverse-phase silica gel column chromatography (10-50% acetonitrile/water) to afford compound B96-2. A mixture of compound B96-2, trifluoroacetic acid (0.3 mL), and dichloromethane (3 mL) was stirred at room temperature for 10 minutes. The reaction mixture was diluted with ethyl acetate (100 mL) and sequentially washed with saturated sodium bicarbonate aqueous solution (30 mL) and saturated sodium chloride aqueous solution (30 mL). The organic phase was dried over anhydrous sodium sulfate and concentrated under reduced pressure to afford compound B96 as a white solid (4.2 mg, 16.01%). ESI/MS (m/z) 263.0 [M+H]⁺.

### Example 152: Synthesis of Compound B97

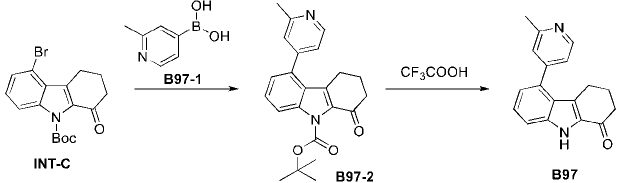

Under a nitrogen atmosphere, compound B97-1 (27.4 mg, 0.200 mmol), [1,1'bis(diphenylphosphino)ferrocene]dichloropalladium(II) dichloromethane complex (4.0 mg, 0.005 mmol), potassium carbonate (27.6 mg, 0.200 mmol), and water (0.2 mL) were sequentially added to a solution of compound INT-C (36.4 mg, 0.100 mmol) in 1,4-dioxane (0.8 mL). The mixture was heated to 80 °C and stirred for 3 hours. After cooling to room temperature, the reaction mixture was diluted with ethyl acetate (30 mL). The organic phase was washed with saturated sodium chloride aqueous solution, dried over anhydrous sodium sulfate, and concentrated under reduced pressure. The residue was purified by reverse-phase silica gel column chromatography (acetonitrile:water = 10-50%) to afford compound B97-2. A mixture of compound B97-2, trifluoroacetic acid (0.3 mL), and dichloromethane (3 mL) was stirred at room temperature for 10 minutes. The reaction mixture was diluted with ethyl acetate (100 mL) and sequentially washed with saturated sodium bicarbonate aqueous solution (30 mL) and saturated sodium chloride aqueous solution (30 mL). The organic phase was dried over anhydrous sodium sulfate and concentrated under reduced pressure to afford compound B97 as a white solid (5.1 mg, 18.46%). ESI/MS (m/z) 277.0 [M+H]⁺.

### Example153: Synthesis of Compound B98

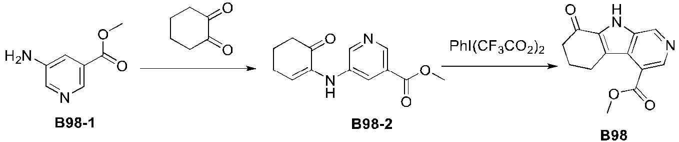

At room temperature, 1,2-cyclohexanedione (740 mg, 6.600 mmol) was added to a solution of compound B98-1 (1 g, 6.572 mmol) in toluene (30 mL). The reaction mixture was stirred at 120 °C overnight, then cooled to room temperature and concentrated under reduced pressure. The residue was purified by reverse-phase preparative chromatography (mobile phase A: water (0.1% formic acid); mobile phase B: acetonitrile) to afford compound B98-2 as a yellow solid (800 mg, 49.43%). ESI/MS (m/z): 246.9 [M+H]⁺.

At room temperature, [bis(trifluoroacetoxy)iodo]benzene (384.17 mg, 0.893 mmol) was added to a solution of compound B98-2 (200 mg, 0.812 mmol) in acetic anhydride (6 mL). The reaction mixture was stirred at room temperature for 1 hour, then quenched with water. The mixture was extracted with ethyl acetate (3 × 30 mL). The combined organic phases were washed with saturated sodium chloride aqueous solution (20 mL), dried over anhydrous sodium sulfate, and concentrated under vacuum. The residue was purified by reverse-phase preparative chromatography (mobile phase A: water (0.1% formic acid); mobile phase B: acetonitrile) to afford compound B98 as a yellow solid (4.2 mg, 2.10%). ESI/MS (m/z): 245.2 [M+H]⁺.¹H NMR (400 MHz, DMSO-*d₆*) δ 12.26 (s, 1H), 8.97 (s, 1H), 8.29 (s, 1H), 3.91 (s, 3H), 3.06-3.01 (m, 2H), 2.69-2.64 (m, 2H), 2.23-2.16 (m, 2H).

### Example 154: Synthesis of Compound B99

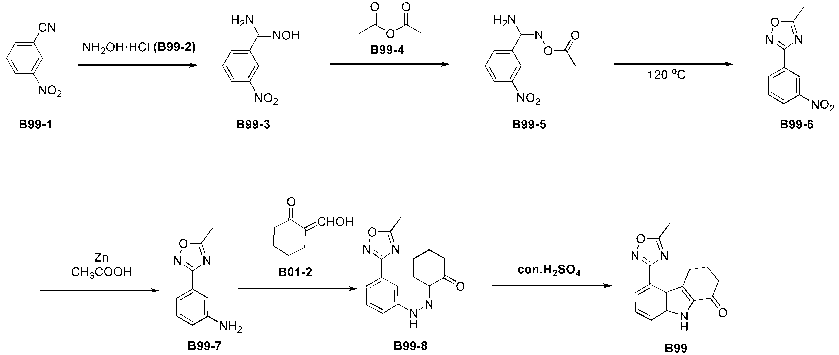

At room temperature, compound B99-2 (1.88 g, 27.004 mmol) was added to a solution of compound B99-1 (2 g, 13.50 mmol) and triethylamine (2.73 g, 27.00 mmol) in ethanol (50 mL). The reaction mixture was stirred at room temperature for 4 hours and then concentrated under reduced pressure. The residue was redissolved in ethyl acetate (100 mL) and sequentially washed with 1N hydrochloric acid aqueous solution (30 mL), saturated sodium bicarbonate aqueous solution (30 mL), and saturated brine (50 mL). The organic phase was dried over anhydrous sodium sulfate and concentrated under vacuum to afford compound B99-3 as a yellow solid (2.2 g, 89.04%). ESI/MS (m/z): 182.0 [M+H]⁺.

At 0 °C, compound B99-4 (1.67 g, 16.40 mmol) was slowly added to a solution of compound B99-3 (2 g, 10.93 mmol) and N,N-diisopropylethylamine (7.06 g, 54.65 mmol) in dichloromethane (50 mL). The mixture was stirred for 1 hour. The reaction mixture was sequentially washed with 1N hydrochloric acid aqueous solution (30 mL), saturated sodium bicarbonate aqueous solution (30 mL), and saturated sodium chloride aqueous solution (80 mL). The organic phase was dried over anhydrous sodium sulfate and concentrated under vacuum to afford compound B99-5 as a white solid (1.9 g, 77.11%). ESI/MS (m/z): 224.0 [M+H]⁺.

A solution of compound B99-5 (1.7 g, 7.54 mmol) in toluene (50 mL) was stirred at 120 °C overnight. After cooling to room temperature, the mixture was concentrated under reduced pressure. The residue was purified by silica gel column chromatography (petroleum ether:ethyl acetate = 3:1) to afford compound B99-6 as a white solid (1.4 g, 87.77%). ESI/MS (m/z): 206.1 [M+H]⁺.

At 0 °C, acetic acid (3.69 g, 61.46 mmol) was added to a solution of compound B99-6 (1.3 g, 6.15 mmol) and zinc powder (2.01 g, 30.73 mmol) in ethanol (50 mL). The reaction mixture was stirred at room temperature for 1 hour and then filtered. The filter cake was washed with ethanol (3 × 10 mL), and the combined filtrates were concentrated under reduced pressure. The residue was purified by silica gel column chromatography (petroleum ether:ethyl acetate = 1:1) to afford compound B99-7 as a white solid (1.05 g, 93.62%). ESI/MS (m/z): 217.1 [M+H+CH₃CN]⁺.

At 0 °C, concentrated hydrochloric acid (2 mL, 65.83 mmol) and a solution of sodium nitrite (718 mg, 10.41 mmol) in water (9 mL) were slowly added to a solution of compound B99-7 (950 mg, 5.21 mmol) in water (72 mL). The reaction mixture was stirred at 0 °C for 0.5 hours. Then, a solution of compound B01-2 (812 mg, 6.25 mmol) in methanol (34 mL) and a solution of sodium acetate (940 mg, 11.45 mmol) in water (72 mL) were added. The mixture was warmed to room temperature and stirred for an additional 2 hours. The mixture was filtered, and the filter cake was washed with water (3 × 15 mL) and dried under vacuum to afford compound B99-8 as a brownish-yellow solid (1.4 g, 87.02%). ESI/MS (m/z): 285.4 [M+H]⁺.

At 0 °C, concentrated sulfuric acid (2 mL, 37.52 mmol) was added to a solution of compound B99-8 (1.3 g, 4.21 mmol) in acetonitrile (40 mL). The reaction mixture was stirred at room temperature for 1 hour and then concentrated under reduced pressure. The residue was purified by reverse-phase chromatography (mobile phase A: water (0.1% formic acid); mobile phase B: acetonitrile) to afford compound B99 as a light yellow solid (89.3 mg, 7.80%). ESI/MS (m/z): 268.0 [M+H]⁺.¹H NMR (400 MHz, DMSO-*d*₆) δ 12.08 (s, 1H), 7.67 (dd, *J =* 8.3, 1.0 Hz, 1H), 7.58 (dd, *J =* 7.3, 1.0 Hz, 1H), 7.45 (dd, *J =* 8.3, 7.3 Hz, 1H), 3.07 (t, *J =* 6.0 Hz, 2H), 2.63 (s, 3H), 2.60-2.54 (m, 2H), 2.17-2.04 (m, 2H).

### Example 155: Synthesis of Compound B100

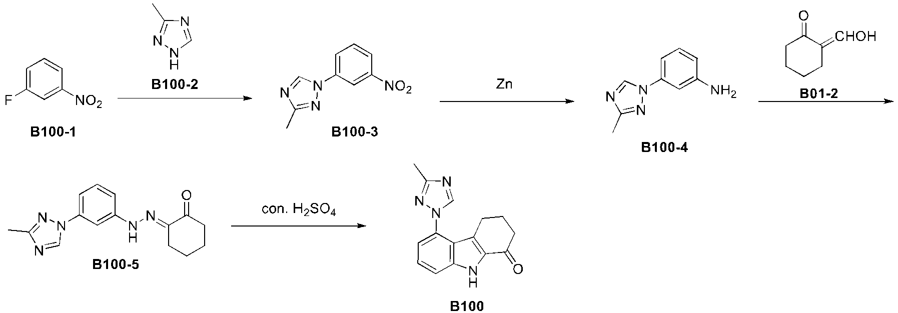

At room temperature, cesium carbonate (5.8 g, 17.709 mmol) was added to a solution of compound B100-1 (1 g, 7.087 mmol) and compound B100-2 (1.77 g, 21.261 mmol) in dimethyl sulfoxide (8 mL). The mixture was heated to 100 °C and stirred overnight. After cooling to room temperature, the reaction was quenched with water (100 mL). The mixture was extracted with ethyl acetate (3 × 80 mL). The combined organic phases were washed with saturated sodium chloride aqueous solution (60 mL), dried over anhydrous sodium sulfate, filtered, and concentrated under reduced pressure. The residue was purified by reverse-phase preparative chromatography (mobile phase A: water (5 mmol ammonium bicarbonate); mobile phase B: acetonitrile) to afford compound B100-3 as a light yellow solid (560 mg, 38.70%). ESI/MS (m/z): 205.0 [M+H]⁺.

At room temperature, acetic acid (1.65 g, 27.430 mmol) was added to a solution of compound B100-3 (560 mg, 2.743 mmol) and zinc powder (0.90 g, 13.715 mmol) in ethanol (8 mL). The reaction mixture was stirred at room temperature for 3 hours. The mixture was filtered, and the solid was washed with ethanol (3 × 20 mL). The filtrate was concentrated under reduced pressure to afford compound B100-4 as a light yellow solid (520 mg, 73.68%). ESI/MS (m/z): 175.3 [M+H]⁺.

At 0 °C, a solution of sodium nitrite (385 mg, 5.580 mmol) in water (4 mL) was slowly added to a solution of compound B100-4 (520 mg, 2.790 mmol) in water (15 mL) and concentrated hydrochloric acid (0.8 mL). The mixture was stirred for 30 minutes. Then, a solution of compound B01-2 (427 mg, 3.348 mmol) in methanol (16 mL) and a solution of sodium acetate (504 mg, 6.038 mmol) in water (16 mL) were slowly added to the above mixture. The reaction was stirred at room temperature for 1 hour. The mixture was filtered, and the filter cake was washed with water (3 × 20 mL) and dried under vacuum to afford compound B100-5 as a yellow solid (300 mg, 34.42%). ESI/MS (m/z): 284.2 [M+H]⁺.

At room temperature, concentrated sulfuric acid (0.5 mL) was added to a solution of compound B100-5 (300 mg, 0.960 mmol) in acetonitrile (10 mL). The reaction mixture was stirred at room temperature for 1 hour and then concentrated under reduced pressure. The residue was purified by reverse-phase preparative chromatography (mobile phase A: water (0.1% formic acid); mobile phase B: methanol) to afford compound B100 as a white solid (11.6 mg, 4.46%). ESI/MS (m/z): 267.2 [M+H]⁺.¹H NMR (400 MHz, DMSO-*d*₆) δ 12.07 (s, 1H), 8.78 (s, 1H), 7.56 (d, *J= 8.3* Hz, 1H), 7.40 (dd, *J =* 8.4, 7.4 Hz, 1H), 7.16 (d, *J =* 7.3 Hz, 1H), 2.55-2.51 (m, 2H), 2.43 (t, *J=* 6.0 Hz, 2H), 2.39 (s, 3H), 2.05-1.95 (m, 2H).

### Example 156: Synthesis of Compound E73

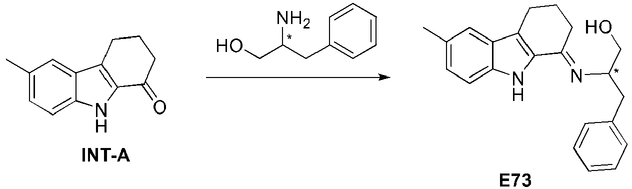

A solution of compound INT-A (199.3 mg, 1.000 mmol) and DL-2-amino-3-phenyl-1-propanol (378.0 mg, 2.500 mmol) in toluene (5 mL) was stirred at 130 °C for 4 hours. After cooling to room temperature, the reaction mixture was concentrated under reduced pressure. The residue was dissolved in a small amount of acetonitrile and then poured into a large volume of water. The precipitated solid was washed with acetonitrile to afford compound E73 as a brown solid (59.7 mg, 41.84%). ESI/MS (m/z) 333.1 [M+H]⁺.¹H NMR (500 MHz, DMSO-*d*₆) δ 10.72 (s, 1H), 7.28 (d, *J=* 8.3 Hz, 1H), 7.24 (s, 1H), 7.22-7.19 (m, 2H), 7.18-7.11 (m, 3H), 6.96 (d, *J=* 8.3 Hz, 1H), 4.60 (t, *J =* 5.3 Hz, 1H), 3.96-3.90 (m, 1H), 3.60-3.55 (m, 1H), 3.51-3.46 (m, 1H), 3.02 (dd, *J=* 13.1, 4.0 Hz, 1H), 2.67-2.62 (m, 3H), 2.43-2.35 (m, 4H), 1.98-1.92 (m, 1H), 1.80-1.74 (m, 1H), 1.62-1.56 (m, 1H).

### Example 157: Synthesis of Compound C01

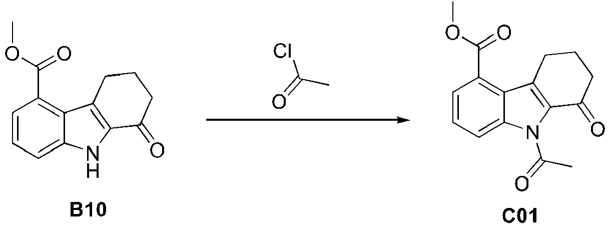

Under a nitrogen atmosphere at 0 °C, 60% sodium hydride (123.31 mg, 3.083 mmol) was added to a solution of compound B10 (300 mg, 1.233 mmol) in dichloromethane (10 mL). The reaction mixture was stirred at 0 °C for 30 minutes, and then a solution of acetyl chloride (125.85 mg, 1.603 mmol) in dichloromethane (1 mL) was added. The mixture was stirred at room temperature for 1 hour. After the reaction was complete, it was quenched with water and extracted with ethyl acetate (3 × 30 mL). The combined organic phases were washed with saturated sodium chloride aqueous solution (20 mL), dried over anhydrous sodium sulfate, and concentrated under reduced pressure. The residue was purified by reverse-phase preparative chromatography (mobile phase A: water (0.1% formic acid); mobile phase B: acetonitrile) to afford compound C01 as a white solid (66.2 mg, 18.65%). ESI/MS (m/z): 286.0 [M+H]⁺.¹H NMR (400 MHz, DMSO-*d₆*) δ 8.20 (dd, *J* = 8.5, 1.1 Hz, 1H), 7.71 (dd, *J=* 7.5, 1.0 Hz, 1H), 7.60 (dd, *J=* 8.5, 7.4 Hz, 1H), 3.93 (s, 3H), 3.03 (t, *J=* 6.0 Hz, 2H), 2.70-2.61 (m, 2H), 2.54 (s, 3H), 2.19-2.08 (m, 2H).

### Example 158: Synthesis of Compound C02

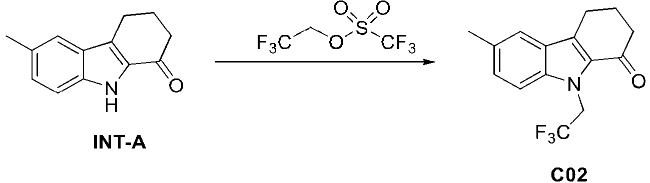

A mixture of compound INT-A (199.3 mg, 1.000 mmol), cesium carbonate (651.6 mg, 2.000 mmol), and 2,2,2-trifluoroethyl trifluoromethanesulfonate (464.2 mg, 2.000 mmol) in N,N-dimethylformamide (4 mL) was stirred at room temperature for 3 hours, then quenched with water (10 mL). The mixture was extracted with ethyl acetate (3 × 20 mL). The organic layer was washed with saturated sodium chloride aqueous solution (4 × 30 mL), dried over anhydrous sodium sulfate, filtered, and concentrated under reduced pressure. The residue was purified by silica gel column chromatography (ethyl acetate:petroleum ether = 1:20) to afford compound C02 as a white solid (216.0 mg, 76.79%). ESI/MS (m/z) 282.0 [M+H]⁺.¹H NMR (500 MHz, DMSO-*d*₆) δ 7.57 (d, *J =* 8.6 Hz, 1H), 7.52 (s, 1H), 7.29 (dd, *J =* 8.6, 1.3 Hz, 1H), 5.48 (q, *J =* 9.1 Hz, 2H), 2.97 (t, *J =* 6.1 Hz, 2H), 2.60-2.57 (m, 2H), 2.41 (s, 3H), 2.15-2.10 (m, 2H).

### Example 159: Synthesis of Compound C03

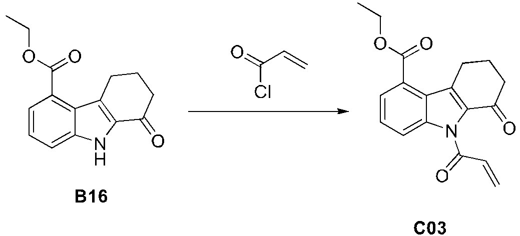

Compound B16 (0.1280 g, 0.50 mmol) was dissolved in tetrahydrofuran (5 mL) under a nitrogen atmosphere. The solution was cooled to -5 °C, and 60% sodium hydride (0.0597 g, 1.49 mmol) was added. After stirring for an additional 10 minutes, acryloyl chloride (0.0675 g, 0.75 mmol) was added to the mixture. The reaction was stirred at -5 °C for 5 hours. Upon completion, the reaction was quenched with saturated ammonium chloride solution and extracted with dichloromethane (30 mL). The organic phase was washed with water (10 mL × 2), dried over anhydrous sodium sulfate, filtered, and concentrated. The residue was purified by silica gel column chromatography (ethyl acetate:petroleum ether = 1:4) to afford compound C03 as a white solid (0.0060 g, 0.018 mmol). ESI/MS (m/z) 312.0 [M+H]⁺.¹H NMR (500 MHz, DMSO-*d*₆) δ 8.18 (dd, *J=* 8.4, 1.0 Hz, 1H), 7.74 (dd, *J=* 7.4, 1.0 Hz, 1H), 7.64-7.59 (m, 1H), 6.68-6.61 (m, 1H), 6.33 (dd, *J =* 16.9, 1.3 Hz, 1H), 5.91 (dd, *J =* 6.3, 1.3 Hz, 1H), 4.40 (q, *J=* 7.2 Hz, 2H), 3.07 (t, *J=* 6.0 Hz, 2H), 2.63 (t, *J=* 10.0, 5.7 Hz, 2H), 2.20-2.10 (m, 2H), 1.37 (t, *J =* 7.1 Hz, 3H).

### Example 160: Synthesis of Compound C04

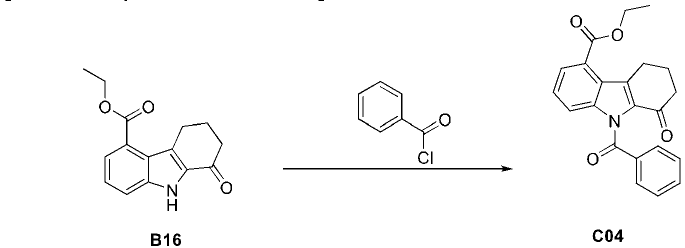

Compound B16 (0.1280 g, 0.50 mmol) was dissolved in tetrahydrofuran (5 mL) under a nitrogen atmosphere. The solution was cooled to -5 °C, and 60% sodium hydride (0.0597 g, 1.49 mmol) was added. After stirring for an additional 10 minutes, benzoyl chloride (0.1049 g, 0.75 mmol) was added to the mixture. The reaction was stirred at room temperature for 5 hours. Upon completion, the reaction was quenched with saturated ammonium chloride solution and extracted with dichloromethane (30 mL). The organic phase was washed with water (10 mL × 2), dried over anhydrous sodium sulfate, filtered, and concentrated. The residue was purified by silica gel column chromatography (ethyl acetate:petroleum ether = 1:4) to afford compound C04 as a white solid (0.053 g, 0.15 mmol). ESI/MS (m/z) 362.0 [M+H]⁺.¹H NMR (500 MHz, DMSO-*d*₆) δ 7.88-7.84 (m, 1H), 7.78-7.74 (m, 1H), 7.66 (t, *J=* 7.4 Hz, 1H), 7.63-7.60 (m, 2H), 7.60-7.55 (m, 1H), 7.50 (t, *J=* 7.7 Hz, 2H), 4.43 (q, *J=* 7.1 Hz, 2H), 3.13 (t, *J =* 6.0 Hz, 2H), 2.49-2.45 (m, 2H), 2.18-2.09 (m, 2H), 1.40 (t, *J =* 7.1 Hz, 3H).

### Example 161: Synthesis of Compound C05

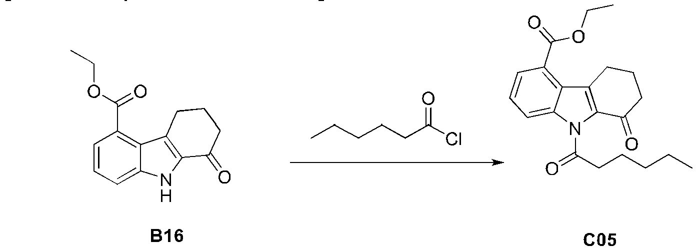

Compound B16 (0.1280 g, 0.50 mmol) was dissolved in dichloromethane (5 mL) and cooled to -5 °C. 60% sodium hydride (0.0597 g, 1.49 mmol) was added, and the mixture was stirred for 10 minutes. Hexanoyl chloride (0.1004 g, 0.75 mmol) was then added. The reaction mixture was stirred at room temperature for 5 hours, quenched with saturated ammonium chloride solution, and extracted with dichloromethane (30 mL). The organic phase was washed with water (10 mL × 2), dried over anhydrous sodium sulfate, filtered, and concentrated. The residue was purified by preparative thin-layer chromatography (ethyl acetate:petroleum ether = 1:4) to afford compound C05 as a light yellow oil (0.0870 g, 0.24 mmol). ESI/MS (m/z) 356.1 [M+H]⁺.¹H NMR (500 MHz, DMSO-*d*₆) δ 8.09 (dd, *J* = 8.5 Hz, *J =* 1.0 Hz, 1H), 7.71 (dd, *J=* 7.4 Hz, *J=* 1.0 Hz , 1H), 7.60 (dd, *J =* 8.5, 7.4 Hz, 1H), 4.40 (q, *J* = 7.1 Hz, 2H), 3.06 (t, *J=* 6.0 Hz, 2H), 2.83 (t, *J=* 7.4 Hz, 2H), 2.69-2.62 (m, 2H), 2.18-2.11 (m, 2H), 1.67-1.59 (m, 2H), 1.37 (t, *J=* 7.1 Hz, 3H), 1.26-1.19 (m, 4H), 0.85-0.78 (m, 3H).

### Example 162: Synthesis of Compound C06

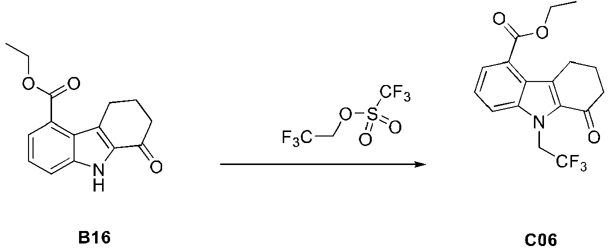

Compound B16 (0.1280 g, 0.50 mmol) was dissolved in N,N-dimethylformamide (3 mL), and 2,2,2-trifluoroethyl trifluoromethanesulfonate (0.2309 g, 0.99 mmol) and cesium carbonate (0.4862 g, 1.49 mmol) were added. The reaction mixture was stirred at room temperature for 2 hours, then quenched with saturated ammonium chloride solution and extracted with dichloromethane (30 mL). The organic phase was washed with water (10 mL × 2), dried over anhydrous sodium sulfate, filtered, and concentrated. The residue was purified by silica gel column chromatography (ethyl acetate: dichloromethane = 1:1) to afford compound C06 as a white solid (0.1140 g, 0.31 mmol). ESI/MS (m/z) 340.0 [M+H]⁺.¹H NMR (500 MHz, DMSO-*d*₆) δ 7.99 (d, *J =* 8.5 Hz, 1H), 7.65 (dd, *J =* 7.3, 0.9 Hz, 1H), 7.57-7.51 (m, 1H), 5.64 (q, *J* = 9.0 Hz, 2H), 4.39 (q, *J=* 7.1 Hz, 2H), 3.08 (t, *J=* 6.1 Hz, 2H), 2.67-2.53 (m, 2H), 2.14-2.05 (m, 2H), 1.37 (t, *J =* 7.1 Hz, 3H).

### Example163: Synthesis of Compound C07

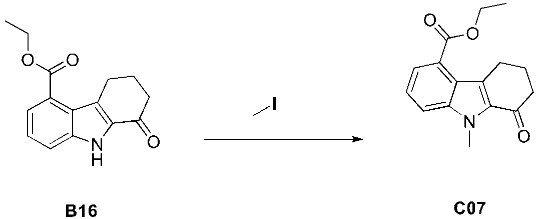

Compound B16 (0.1280 g, 0.50 mmol) was dissolved in tetrahydrofuran (5 mL) under a nitrogen atmosphere. The solution was cooled to -5 °C, and sodium hydride (0.0597 g, 1.49 mmol) was added. After stirring for 10 minutes, iodomethane (0.1103 g, 0.75 mmol) was added, and the reaction mixture was stirred at -5 °C for 5 hours. The reaction was quenched with saturated ammonium chloride solution and extracted with dichloromethane (30 mL). The combined organic phases were washed with water (10 mL × 2), dried over anhydrous sodium sulfate, and concentrated under reduced pressure. The residue was purified by silica gel column chromatography (ethyl acetate:petroleum ether = 1:4) to afford compound C07 as a white solid (0.0650 g, 0.24 mmol). ESI/MS (m/z) 272.1 [M+H]⁺.¹H NMR (500 MHz, DMSO-*d*₆) δ 7.82 (d, *J=* 8.4 Hz, 1H), 7.59 (d, *J=* 7.2 Hz, 1H), 7.48-7.44 (m, 1H), 4.38 (q, *J=* 7.1 Hz, 2H), 4.06 (s, 3H), 3.06 (t, *J =* 6.0 Hz, 2H), 2.62-2.56 (m, 2H), 2.12-2.04 (m, 2H), 1.36 (t, *J =* 7.1 Hz, 3H).

### Example 164: Synthesis of Compound C08

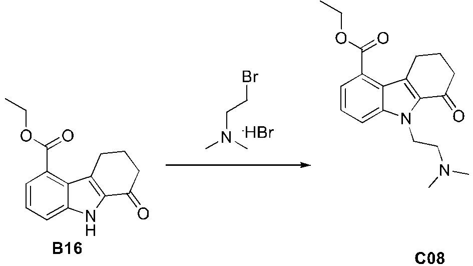

Compound B16 (0.1280 g, 0.50 mmol) was dissolved in tetrahydrofuran (5 mL) under a nitrogen atmosphere and cooled to -5 °C. Sodium hydride (0.0597 g, 1.49 mmol) was added, and the mixture was stirred for 10 minutes. Then, N,N-dimethylaminoethyl bromide hydrobromide (0.1738 g, 0.75 mmol) was added, and the reaction mixture was stirred at -5 °C for 5 hours. The reaction was quenched with saturated ammonium chloride solution and extracted with dichloromethane (30 mL). The organic phase was washed with water (10 mL × 2), dried over anhydrous sodium sulfate, and concentrated under vacuum. The residue was purified by preparative thin-layer chromatography (dichloromethane:methanol = 20:1) to afford compound C08 as a white solid (0.0080 g, 0.23 mmol). ESI/MS (m/z) 329.1 [M+H]⁺;¹H NMR (500 MHz, DMSO-*d*₆) δ 7.83 (d, *J=* 8.4 Hz, 1H), 7.57 (d, *J=* 7.2 Hz, 1H), 7.47-7.43 (m, 1H), 4.66 (t, *J =* 7.0 Hz, 2H), 4.37 (q, *J =* 7.1 Hz, 2H), 3.05 (t, *J =* 6.0 Hz, 2H), 2.62-2.57 (m, 2H), 2.49-2.46 (m, 2H), 2.19 (s, 6H), 2.11-2.03 (m, 2H), 1.36 (t, *J =* 7.1 Hz, 3H).

### Example 165: Synthesis of Compound B103

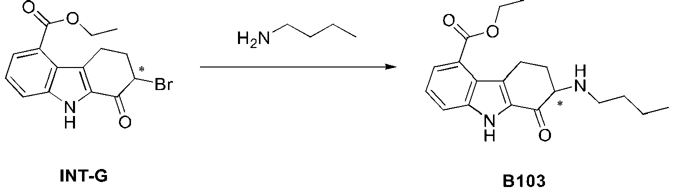

At room temperature, n-butylamine (22 mg, 0.30 mmol) was added to a solution of compound INT-G (50 mg, 0.15 mmol, racemate) in N,N-dimethylformamide (1 mL). The reaction mixture was stirred for 2 hours and then diluted with ethyl acetate (20 mL). The organic phase was washed with water (10 mL × 2), dried over anhydrous sodium sulfate, and concentrated under vacuum. The residue was purified by reverse-phase preparative chromatography (acetonitrile/water/trifluoroacetic acid system) to afford the trifluoroacetate salt of compound B103 as a white solid (11 mg, 0.024 mmol, racemate). ESI/MS (m/z) 329.1 [M+H]⁺;¹H NMR (500 MHz, DMSO-*d₆*) δ 12.36 (s, 1H), 9.05 (d, *J =* 69.9 Hz, 2H), 7.70 (dd, *J =* 8.4, 1.0 Hz, 1H), 7.67 (dd, *J* = 7.2, 1.0 Hz, 1H), 7.46 (dd, *J=* 8.4, 7.3 Hz, 1H), 4.54 (d, *J=* 13.4 Hz, 1H), 4.44-4.32 (m, 2H), 3.43-3.39 (m, 1H), 3.24-3.16 (m, 1H), 3.13-3.00 (m, 2H), 2.65-2.57 (m, 1H), 2.22-2.10 (m, 1H), 1.77-1.58 (m, 2H), 1.43-1.34 (m, 5H), 0.94 (t, *J=* 7.3 Hz, 3H).

### Example 166: Synthesis of Compound B104

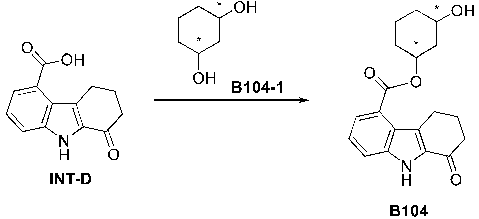

At room temperature, N,N'-dicyclohexylcarbodiimide (160.0 mg, 0.774 mmol) and 4-dimethylaminopyridine (63.0 mg, 0.516 mmol) were added to a mixture of compound INT-D (120.0 mg, 0.516 mmol) and compound B104-1 (72.0 mg, 0.619 mmol) in dichloromethane (4 mL) and N,N-dimethylformamide (4 mL). The reaction mixture was stirred at room temperature for 4 hours and then concentrated under vacuum. The residue was purified by reverse-phase chromatography (mobile phase A: water (0.1% formic acid); mobile phase B: acetonitrile) to afford compound B104 as a white solid (16.2 mg, 9.46%). ESI/MS (m/z): 328.2 [M+H]⁺.

### Example167: Synthesis of Compound B105

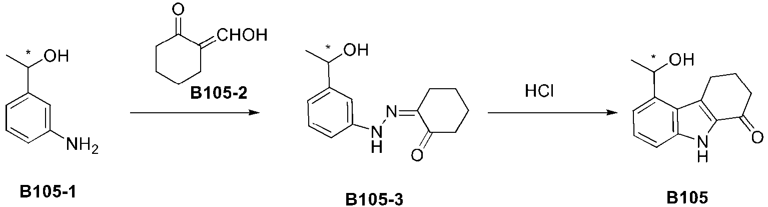

At 0 °C, a solution of sodium nitrite (2.0 g, 29.158 mmol) in water (17 mL) was slowly added dropwise to a solution of compound B105-1 (2.0 g, 14.579 mmol) in water (60 mL) and concentrated hydrochloric acid (4.3 mL). The mixture was stirred at 0 °C for 30 minutes. Then, a solution of compound B105-2 (2.23 g, 17.495 mmol) in methanol (70 mL) and a solution of sodium acetate (2.63 g, 32.074 mmol) in water (70 mL) were slowly added to the above mixture. The reaction was stirred at room temperature for 1 hour. The resulting mixture was extracted with ethyl acetate (3 × 80 mL). The combined organic phases were washed with saturated sodium chloride aqueous solution (60 mL), dried over anhydrous sodium sulfate, and concentrated under vacuum. The residue was purified by reverse-phase preparative chromatography (mobile phase A: water (0.1% formic acid); mobile phase B: acetonitrile) to afford compound B105-3 as a light yellow solid (430 mg, 11.63%). ESI/MS (m/z): 247.3 [M+H]⁺.

At room temperature, concentrated hydrochloric acid (0.15 mL) was added to a solution of compound B105-3 (200 mg, 0.788 mmol) in acetonitrile (3 mL). The reaction mixture was stirred at room temperature for 2 hours and then concentrated under vacuum. The residue was purified by reverse-phase preparative chromatography (mobile phase A: water (0.1% formic acid); mobile phase B: acetonitrile) to afford compound B105 as a white solid (24.2 mg, 13.29%). ESI/MS (m/z): 230.2 [M+H]⁺.¹H NMR (400 MHz, DMSO-*d*₆) δ 11.61 (s, 1H), 7.26 (d, *J =* 1.9 Hz, 1H), 7.23 (d, *J =* 8.2 Hz, 1H), 7.17 (dd, *J =* 6.5, 1.8 Hz, 1H), 5.35-5.28 (m, 1H), 5.18 (d, *J=* 4.0 Hz, 1H), 3.22-3.07 (m, 2H), 2.56-2.51 (m, 2H), 2.19-2.11 (m, 2H), 1.42 (d, *J =* 6.4 Hz, 3H).

### Example 168: Synthesis of Compound B106

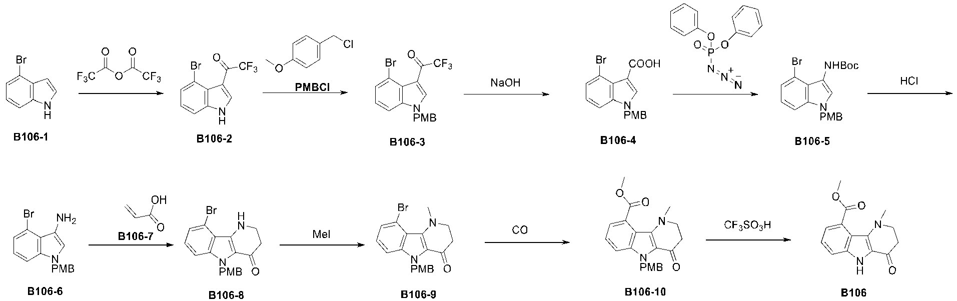

At 0 °C, trifluoroacetic anhydride (13.9 g, 66.323 mmol) was slowly added to a solution of compound B106-1 (10 g, 51.008 mmol) in N,N-dimethylformamide (100 mL). The reaction mixture was stirred at room temperature for 20 hours, diluted with water (100 mL), and extracted with ethyl acetate (3 × 300 mL). The combined organic phases were washed with saturated sodium chloride aqueous solution (300 mL), dried over anhydrous sodium sulfate, and concentrated under vacuum. The residue was purified by silica gel column chromatography (petroleum ether:ethyl acetate = 5:1) to afford compound B106-2 as a pink solid (9 g, 59.81%). ESI/MS (m/z): 292.1 [M+H]⁺.

At 0 °C, 60% sodium hydride (1.83 g, 45.762 mmol) was added to a solution of compound B106-2 (9 g, 30.508 mmol) in N,N-dimethylformamide (100 mL). After stirring for 30 minutes, p-methoxybenzyl chloride (9.56 g, 61.016 mmol, PMBCl) was added. The reaction mixture was stirred at room temperature for 4 hours, diluted with water (150 mL), and extracted with ethyl acetate (3 × 200 mL). The combined organic phases were washed with saturated sodium chloride aqueous solution (150 mL), dried over anhydrous sodium sulfate, and concentrated under vacuum. The residue was purified by silica gel column chromatography (petroleum ether:ethyl acetate = 6:1) to afford compound B106-3 as a brown oil (10 g, 72.36%). ESI/MS (m/z): 412.0 [M+H]⁺.

At 0 °C, sodium hydroxide (19.9 g, 497.325 mmol) and water (50 mL) were added to a solution of compound B106-3 (10 g, 19.893 mmol) in ethanol (120 mL). The mixture was heated to 60 °C and stirred for 5 hours. After cooling to room temperature, the pH was adjusted to 5 with dilute hydrochloric acid, and the mixture was extracted with ethyl acetate (3 × 100 mL). The combined organic phases were dried over anhydrous sodium sulfate and concentrated under vacuum. The residue was purified by silica gel column chromatography (petroleum ether:ethyl acetate = 1:2) to afford compound B106-4 as a yellow solid (7.5 g, 95.25%). ESI/MS (m/z): 362.0 [M+H]⁺.

At room temperature, diphenylphosphoryl azide (6.2 g, 22.446 mmol) was slowly added to a solution of compound B106-4 (7.5 g, 20.405 mmol) and triethylamine (4.1 g, 40.810 mmol) in tetrahydrofuran (130 mL). The reaction mixture was stirred at room temperature for 24 hours and concentrated under vacuum. tert-Butanol (100 mL) was added to the residue, and the mixture was stirred at 85 °C for 24 hours. After cooling to room temperature, the mixture was concentrated under vacuum. The residue was purified by reverse-phase chromatography (mobile phase A: water (0.1% formic acid); mobile phase B: acetonitrile) to afford compound B106-5 as a brown solid (2.5 g, 26.70%). ESI/MS (m/z): 375.8 [M+H-56]⁺.

At room temperature, a 4M hydrogen chloride solution in 1,4-dioxane (50 mL) was added to compound B106-5 (2.5 g, 5.448 mmol). The reaction mixture was stirred at room temperature for 2 hours and concentrated under vacuum to afford compound B106-6 as a gray solid (1.7 g, 87.61%). ESI/MS (m/z): 330.8 [M+H]⁺.

At room temperature, compound B106-7 (688 mg, 9.546 mmol) was added to a solution of compound B106-6 (1.7 g, 4.773 mmol) in water (100 mL). The mixture was heated to 100 °C and stirred overnight. After cooling to room temperature, the mixture was filtered. The filter cake was washed with water (10 mL) and purified by reverse-phase chromatography (mobile phase A: water (0.1% formic acid); mobile phase B: acetonitrile) to afford compound B106-8 as a yellow solid (200 mg, 10.33%). ESI/MS (m/z): 385.1 [M+H]⁺.

At 0 °C, 60% sodium hydride (30 mg, 0.740 mmol) was added to a solution of compound B106-8 (200 mg, 0.493 mmol) in N,N-dimethylformamide (6 mL). After stirring for 30 minutes, iodomethane (70 mg, 0.493 mmol) was added. The reaction mixture was stirred at room temperature for 2 hours, quenched with water (30 mL), and extracted with ethyl acetate (3 × 30 mL). The combined organic phases were washed with saturated sodium chloride aqueous solution (20 mL), dried over anhydrous sodium sulfate, and concentrated under vacuum to afford compound B106-9 as a yellow solid (210 mg, 92.78%). ESI/MS (m/z): 399.1 [M+H]⁺.

In a high-pressure autoclave, [1,1'-bis(diphenylphosphino)ferrocene]dichloropalladium(II) dichloromethane complex (37 mg, 0.046 mmol) was added to a solution of compound B106-9 (210 mg, 0.458 mmol) and triethylamine (231 mg, 2.290 mmol) in methanol (15 mL). Carbon monoxide was introduced to 10 atm, and the reaction mixture was stirred at 80 °C overnight. After cooling to room temperature, the mixture was concentrated under vacuum. The residue was purified by silica gel column chromatography (petroleum ether:ethyl acetate = 1:1) to afford compound B106-10 as a yellow oil (90 mg, 50.94%). ESI/MS (m/z): 378.9 [M+H]⁺.

At room temperature, trifluoromethanesulfonic acid (0.3 mL) was added to a solution of compound B106-10 (80 mg, 0.207 mmol) in trifluoroacetic acid (3 mL). The reaction mixture was stirred at room temperature for 10 minutes and concentrated under vacuum. The residue was purified by reverse-phase chromatography (mobile phase A: water (0.1% formic acid); mobile phase B: acetonitrile) to afford compound B106 as a yellow solid (21.1 mg, 38.96%). ESI/MS (m/z): 259.1 [M+H]⁺.¹H NMR (400 MHz, DMSO-*d₆*) δ 11.57 (s, 1H), 7.55 (dd, *J =* 8.1, 1.0 Hz, 1H), 7.40 (dd, *J* = 7.4, 1.0 Hz, 1H), 7.13-7.07 (m, 1H), 3.82 (s, 3H), 2.99 (s, 3H), 2.95 (t, *J =* 7.2 Hz, 2H), 2.68 (t, *J =* 7.2 Hz, 2H).

### Example 169: Synthesis of Compound B107

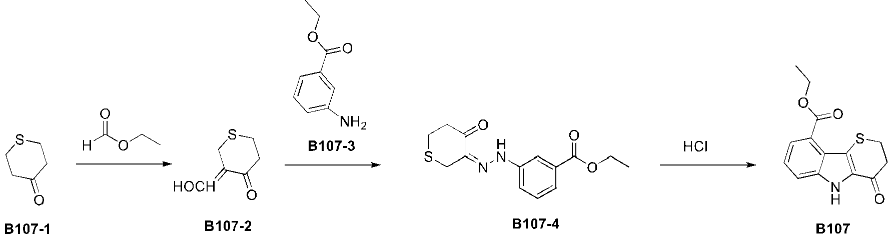

At 0 °C, ethyl formate (3.8 g, 51.645 mmol) was added to a solution of compound B107-1 (1 g, 8.607 mmol) and sodium methoxide (1.4 g, 25.822 mmol) in 1,4-dioxane (30 mL). The mixture was stirred for 2 hours. At 0 °C, the reaction mixture was adjusted to pH 6 with a citric acid aqueous solution and extracted with ethyl acetate (3 × 60 mL). The combined organic phases were washed with saturated sodium chloride aqueous solution (30 mL), dried over anhydrous sodium sulfate, and concentrated under vacuum. The residue was purified by silica gel column chromatography (petroleum ether:ethyl acetate = 1:1) to afford compound B107-2 as a brown oil (1.5 g, 52.33%). ESI/MS (m/z): 145.0 [M+H]⁺.

At 0 °C, a solution of sodium nitrite (0.67 g, 9.686 mmol) in water (5 mL) was slowly added to a solution of compound B107-3 (0.8 g, 4.843 mmol) in concentrated hydrochloric acid (3 mL) and water (30 mL). The mixture was stirred for 30 minutes. Then, at 0 °C, a solution of compound B107-2 (1.40 g, 9.686 mmol) in methanol (30 mL) and a solution of sodium acetate (0.87 g, 10.655 mmol) in water (30 mL) were slowly added to the above mixture. The reaction was stirred at room temperature for 2 hours and extracted with ethyl acetate (3 × 80 mL). The combined organic phases were washed with saturated sodium chloride aqueous solution (60 mL), dried over anhydrous sodium sulfate, filtered, and concentrated under vacuum to afford compo und B107-4 as a brown oil (0.8 g, crude). ESI/MS (m/z): 293.1 [M+H]⁺.

At room temperature, concentrated hydrochloric acid (250 µL) was added to a solution of compound B107-4 (650 mg, crude) in acetonitrile (5 mL). The reaction mixture was stirred at room temperature for 1 hour and concentrated under vacuum. The residue was purified by reverse-phase chromatography (mobile phase A: water (0.1% formic acid); mobile phase B: acetonitrile) to afford compound B107 as a yellow solid (16.3 mg, 3.6%). ESI/MS (m/z): 276.1 [M+H]⁺.¹H NMR (400 MHz, DMSO-*d*₆) δ 12.23 (s, 1H), 7.67 (dd, *J=* 8.4, 1.1 Hz, 1H), 7.58 (dd, *J=* 7.3, 1.1 Hz, 1H), 7.41 (dd, *J* = 8.2, 7.4 Hz, 1H), 4.38 (q, *J=* 7.1 Hz, 2H), 3.27-3.17 (m, 2H), 2.89-2.78 (m, 2H), 1.37 (t, *J =* 7.1 Hz, 3H).

### Example 170: Synthesis of Compound B108

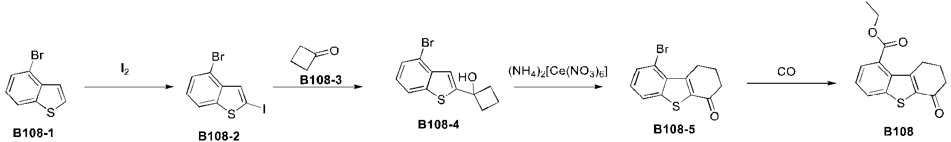

At -78 °C, a 2M solution of lithium diisopropylamide in tetrahydrofuran (2.4 mL) was added dropwise to a solution of compound B108-1 (500 mg, 2.346 mmol) in tetrahydrofuran (13 mL). The reaction mixture was stirred at -78 °C for 30 minutes. A solution of iodine (1.19 g, 4.692 mmol) in tetrahydrofuran (3 mL) was then added dropwise to the system, and stirring was continued for 1 hour. The reaction was warmed to room temperature, quenched with water (30 mL), and extracted with dichloromethane (2 × 30 mL). The combined organic phases were washed with saturated sodium chloride aqueous solution (20 mL), dried over anhydrous sodium sulfate, and concentrated under vacuum to afford compound B108-2 as a light yellow solid (600 mg, 72.42%).

At -78 °C, a 2.5M solution of n-butyllithium in hexane (623.0 µL) was added dropwise to a solution of compound B108-2 (500 mg, 1.416 mmol) in tetrahydrofuran (10 mL). The mixture was stirred for 1 hour. Then, compound B108-3 (119.1 mg, 1.699 mmol) was added dropwise to the reaction mixture, and stirring was continued for 1 hour. The reaction was warmed to room temperature, quenched with water (30 mL), and extracted with dichloromethane (3 × 30 mL). The combined organic phases were washed with saturated sodium chloride aqueous solution (20 mL), dried over anhydrous sodium sulfate, and concentrated under vacuum. The residue was purified by silica gel column chromatography (petroleum ether:ethyl acetate = 5:1) to afford compound B108-4 as a brown oil (250 mg, 46.45%).

At 0 °C, ceric ammonium nitrate (868.6 mg, 1.578 mmol) was added to a solution of compound B108-4 (240 mg, 0.631 mmol) in acetonitrile (1 mL) and water (1 mL). The mixture was stirred for 30 minutes. The reaction was warmed to room temperature, quenched with water (30 mL), and extracted with dichloromethane (3 × 30 mL). The combined organic phases were washed with saturated sodium chloride aqueous solution (10 mL), dried over anhydrous sodium sulfate, and concentrated under vacuum. The residue was purified by silica gel column chromatography (petroleum ether:ethyl acetate = 4:1) to afford compound B108-5 as a yellow solid (150 mg, 68.69%). ESI/MS (m/z): 280.8 [M+H]⁺.

In a high-pressure autoclave, [1,1'-bis(diphenylphosphino)ferrocene]dichloropalladium(II) dichloromethane complex (33.0 mg, 0.041 mmol) and triethylamine (409.6 mg, 4.050 mmol) were added to a solution of compound B108-5 (140 mg, 0.405 mmol) in ethanol (20 mL). Carbon monoxide was introduced to 10 atm, and the reaction mixture was heated to 80 °C and stirred overnight. The mixture was concentrated under vacuum, and the residue was purified by reverse-phase chromatography (mobile phase A: water (10 mmol/L ammonium bicarbonate); mobile phase B: acetonitrile) to afford compound B108 as a white solid (57.3 mg, 50.67%). ESI/MS (m/z): 274.9 [M+H]⁺.¹H NMR (400 MHz, DMSO-*d₆*) δ 8.27 (dd, *J=* 8.1, 1.2 Hz, 1H), 7.70 (dd, *J =* 7.3, 1.1 Hz, 1H), 7.62 (dd, *J=* 8.1, 7.3 Hz, 1H), 4.47-4.37 (m, 2H), 2.96 (t, *J=* 6.0 Hz, 2H), 2.73-2.66 (m, 2H), 2.21-2.11 (m, 2H), 1.37 (t, *J =* 7.1 Hz, 3H).

### Example 171: Synthesis of Compound B109

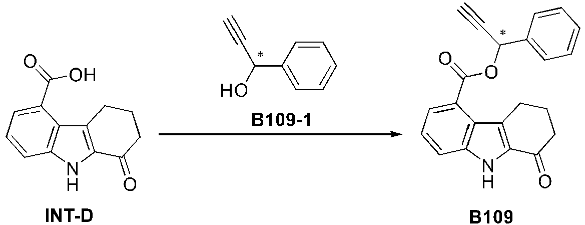

At room temperature, N,N'-dicyclohexylcarbodiimide (135.0 mg, 0.654 mmol) and 4-dimethylaminopyridine (64.0 mg, 0.523 mmol) were added to a mixture of compound INT-D (100 mg, 0.436 mmol) and compound B109-1 (115.3 mg, 0.872 mmol) in dichloromethane (3 mL) and N,N-dimethylformamide (3 mL). The reaction mixture was stirred at room temperature for 2 hours, then quenched with water (30 mL) and extracted with dichloromethane (2 × 30 mL). The combined organic phases were washed with saturated sodium chloride aqueous solution (20 mL), dried over anhydrous sodium sulfate, and concentrated under vacuum. The residue was purified by reverse-phase chromatography (mobile phase A: water (0.1% formic acid); mobile phase B: acetonitrile) to afford compound B109 as a white solid (60.5 mg, 40.11%, racemate). ESI/MS (m/z): 344.1 [M+H]⁺.¹H NMR (400 MHz, DMSO-*d₆*) δ 12.06 (s, 1H), 7.72-7.60 (m, 4H), 7.51-7.43 (m, 3H), 7.42-7.35 (m, 1H), 6.75 (s, 1H), 3.94-3.90 (m, 1H), 3.08-3.00 (m, 2H), 2.60-2.53 (m, 2H), 2.13-2.00 (m, 2H).

### Example172: Synthesis of Compound B110

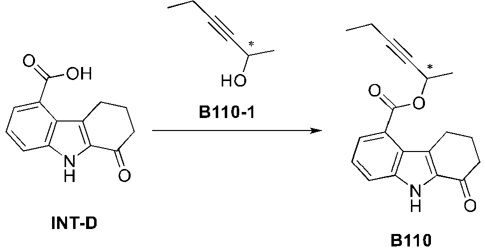

At room temperature, N,N'-dicyclohexylcarbodiimide (270.0 mg, 1.308 mmol) and compound B110-1 (171.3 mg, 1.744 mmol) were added to a mixture of compound INT-D (200 mg, 0.872 mmol) and 4-dimethylaminopyridine (127.9 mg, 1.046 mmol) in dichloromethane (10 mL) and N,N-dimethylformamide (3 mL). The reaction mixture was stirred at room temperature for 3 hours, then quenched with water (30 mL) and extracted with dichloromethane (3 × 40 mL). The combined organic phases were washed with saturated sodium chloride aqueous solution (30 mL), dried over anhydrous sodium sulfate, and concentrated under vacuum. The residue was purified by reverse-phase chromatography (mobile phase A: water (10 mmol/L ammonium bicarbonate); mobile phase B: acetonitrile) to afford compound B110 as a white solid (75.6 mg, 27.95%, racemate). ESI/MS (m/z): 310.1 [M+H]⁺.¹H NMR (400 MHz, DMSO-*d₆*) δ 12.03 (s, 1H), 7.66 (dd, *J =* 8.3, 1.1 Hz, 1H), 7.58 (dd, *J= 7.3,* 1.1 Hz, 1H), 7.42-7.34 (m, 1H), 5.71-5.62 (m, 1H), 3.15-3.09 (m, 2H), 2.61-2.55 (m, 2H), 2.30-2.20 (m, 2H), 2.16-2.06 (m, 2H), 1.56 (d, *J =* 6.6 Hz, 3H), 1.07 (t, *J* = 7.5 Hz, 3H).

### Example 173: Synthesis of Compound B111

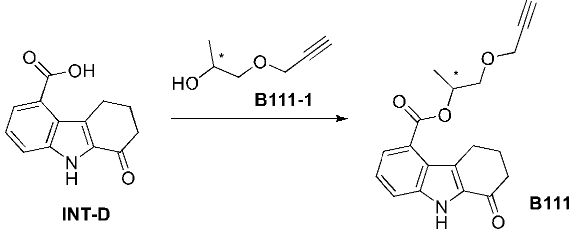

At room temperature, N,N'-dicyclohexylcarbodiimide (202.5 mg, 0.981 mmol) and compound B111-1 (149.4 mg, 1.308 mmol) were added to a mixture of compound INT-D (150 mg, 0.654 mmol) and 4-dimethylaminopyridine (95.9 mg, 0.785 mmol) in dichloromethane (10 mL) and N,N-dimethylformamide (3 mL). The reaction mixture was stirred at room temperature overnight, then quenched with water (30 mL) and extracted with dichloromethane (3 × 40 mL). The combined organic phases were washed with saturated sodium chloride aqueous solution (30 mL), dried over anhydrous sodium sulfate, and concentrated under vacuum. The residue was purified by reverse-phase chromatography (mobile phase A: water (0.1% formic acid); mobile phase B: acetonitrile) to afford compound B111 as a white solid (26.4 mg, 12.33%, racemate). ESI/MS (m/z): 326.0 [M+H]⁺.¹H NMR (400 MHz, DMSO-*d*₆) δ 12.00 (s, 1H), 7.65 (d, *J =* 8.3 Hz, 1H), 7.58 (d, *J =* 7.5 Hz, 1H), 7.41-7.33 (m, 1H), 5.33-5.24 (m, 1H), 4.24-4.18 (m, 2H), 3.70-3.64 (m, 2H), 3.50-3.44 (m, 1H), 3.14-3.06 (m, 2H), 2.61-2.54 (m, 2H), 2.16-2.09 (m, 2H), 1.33 (d, *J =* 6.4 Hz, 3H).

### Example 174: Synthesis of Compound B112

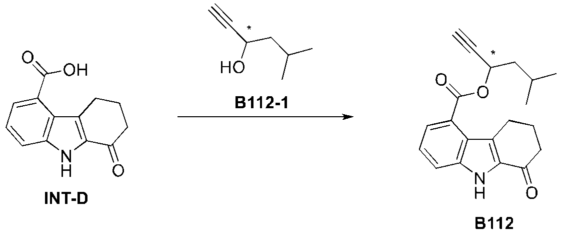

At room temperature, 4-dimethylaminopyridine (126.1 mg, 1.032 mmol) and N,N'-dicyclohexylcarbodiimide (266.3 mg, 1.290 mmol) were added to a mixture of compound INT-D (200 mg, 0.860 mmol) and compound B112-1 (193.0 mg, 1.720 mmol) in dichloromethane (5 mL) and N,N-dimethylformamide (5 mL). The reaction mixture was stirred at room temperature for 2 hours, then quenched with water (30 mL) and extracted with dichloromethane (3 × 30 mL). The combined organic phases were washed with saturated sodium chloride aqueous solution (30 mL), dried over anhydrous sodium sulfate, and concentrated under vacuum. The residue was purified by reverse-phase chromatography (mobile phase A: water (10 mmol/L ammonium bicarbonate); mobile phase B: methanol) to afford compound B112 as a white solid (54.2 mg, 19.19%, racemate). ESI/MS (m/z): 324.2 [M+H]⁺.¹H NMR (400 MHz, DMSO-*d₆*) δ 12.02 (s, 1H), 7.69 (d, *J =* 8.3 Hz, 1H), 7.61 (d, *J =* 7.3 Hz, 1H), 7.43-7.35 (m, 1H), 5.67-5.58 (m, 1H), 3.66-3.63 (m, 1H), 3.13 (t, *J =* 6.0 Hz, 2H), 2.59 (t, *J= 6.5* Hz, 2H), 2.18-2.07 (m, 2H), 1.90-1.80 (m, 2H), 1.81-1.70 (m, 1H), 0.96 (d, *J* = 5.7 Hz, 6H).

### Example 175: Synthesis of Compound B113

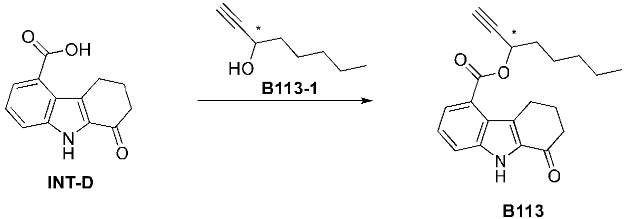

At room temperature, 4-dimethylaminopyridine (126.1 mg, 1.032 mmol) and N,N'-dicyclohexylcarbodiimide (266.3 mg, 1.290 mmol) were added to a mixture of compound INT-D (200 mg, 0.860 mmol) and compound B113-1 (227.4 mg, 1.720 mmol) in dichloromethane (5 mL) and N,N-dimethylformamide (5 mL). The reaction mixture was stirred at room temperature for 2 hours, then quenched with water (30 mL) and extracted with dichloromethane (3 × 30 mL). The combined organic phases were washed with saturated sodium chloride aqueous solution (30 mL), dried over anhydrous sodium sulfate, and concentrated under vacuum. The residue was purified by reverse-phase chromatography (mobile phase A: water (10 mmol/L ammonium bicarbonate); mobile phase B: acetonitrile) to afford compound B113 as a white solid (56.1 mg, 19.27%, racemate). ESI/MS (m/z): 338.2 [M+H]⁺.¹H NMR (400 MHz, DMSO-*d₆*) δ 12.02 (s, 1H), 7.69 (dd, *J =* 8.3, 1.0 Hz, 1H), 7.60 (dd, *J =* 7.3, 1.0 Hz, 1H), 7.39 (dd, *J=* 8.3, 7.3 Hz, 1H), 5.61-5.56 (m, 1H), 3.62 (d, *J* = 2.1 Hz, 1H), 3.12 (t, *J=* 6.0 Hz, 2H), 2.62-2.54 (m, 2H), 2.15-2.08 (m, 2H), 1.95-1.81 (m, 2H), 1.53-1.44 (m, 2H), 1.36-1.25 (m, 4H), 0.90-0.84 (m, 3H).

### Example 176: Synthesis of Compound B114

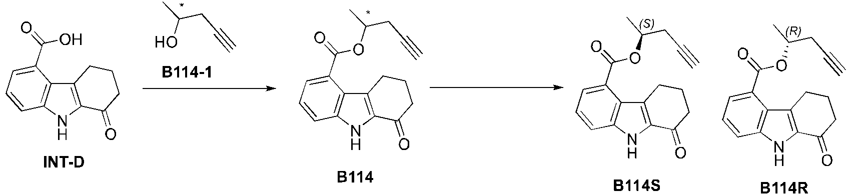

At room temperature, 4-dimethylaminopyridine (126.1 mg, 1.032 mmol) and N,N'-dicyclohexylcarbodiimide (266.3 mg, 1.290 mmol) were added to a mixture of compound INT-D (200 mg, 0.860 mmol) and compound B114-1 (144.7 mg, 1.720 mmol) in dichloromethane (5 mL) and N,N-dimethylformamide (5 mL). The reaction mixture was stirred at room temperature for 5 hours, then quenched with water (30 mL) and extracted with dichloromethane (3 × 30 mL). The combined organic phases were washed with saturated sodium chloride aqueous solution (30 mL), dried over anhydrous sodium sulfate, and concentrated under vacuum. The residue was purified by reverse-phase chromatography (mobile phase A: water (10 mmol/L ammonium bicarbonate); mobile phase B: acetonitrile) to afford compound B114 as a white solid (21 mg, 8.26%, racemate). ESI/MS (m/z): 296.1 [M+H]⁺.¹H NMR (400 MHz, DMSO-*d₆*) δ 12.00 (s, 1H), 7.66 (dd, *J=* 8.3, 1.0 Hz, 1H), 7.61 (dd, *J=* 7.3, 1.0 Hz, 1H), 7.40-7.34 (m, 1H), 5.24-5.15 (m, 1H), 3.12 (t, *J =* 6.0 Hz, 2H), 2.95-2.92 (m, 1H), 2.68-2.63 (m, 2H), 2.60-2.54 (m, 2H), 2.15-2.08 (m, 2H), 1.43 (d, *J=* 6.3 Hz, 3H). Compound B114 can be resolved into compound B114R and compound B114S via chiral separation. Alternatively, compound B114R and compound B114S can also be synthesized separately from their corresponding chiral starting materials.

### Example 177: Synthesis of Compound B115

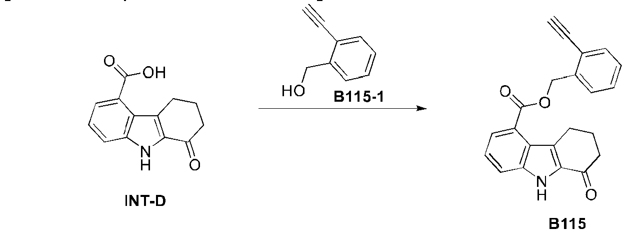

At room temperature, 4-dimethylaminopyridine (126.1 mg, 1.032 mmol) and N,N'-dicyclohexylcarbodiimide (266.3 mg, 1.290 mmol) were added to a mixture of compound INT-D (200 mg, 0.860 mmol) and compound B115-1 (227.4 mg, 1.720 mmol) in dichloromethane (5 mL) and N,N-dimethylformamide (5 mL). The reaction mixture was stirred at room temperature for 2 hours, then quenched with water (30 mL) and extracted with dichloromethane (3 × 30 mL). The combined organic phases were washed with saturated sodium chloride aqueous solution (30 mL), dried over anhydrous sodium sulfate, and concentrated under vacuum. The residue was purified by reverse-phase chromatography (mobile phase A: water (0.1% formic acid); mobile phase B: methanol) to afford compound B115 as a white solid (56.6 mg, 18.82%). ESI/MS (m/z): 344.1 [M+H]⁺.¹H NMR (400 MHz, DMSO-*d₆*) δ 12.02 (s, 1H), 7.70-7.68 (m, 1H), 7.68-7.65 (m, 1H), 7.61-7.57 (m, 2H), 7.50-7.45 (m, 1H), 7.43-7.36 (m, 2H), 5.51 (s, 2H), 4.51 (s, 1H), 3.05 (t, *J =* 6.0 Hz, 2H), 2.58-2.52 (m, 2H), 2.12-2.03 (m, 2H).

### Example 178: Synthesis of Compound B116

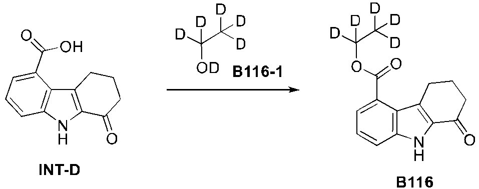

At room temperature, N,N'-dicyclohexylcarbodiimide (133.1 mg, 0.645 mmol) and compound B116-1 (44.8 mg, 0.860 mmol) were added to a mixture of compound INT-D (100 mg, 0.430 mmol) and 4-dimethylaminopyridine (63.1 mg, 0.516 mmol) in dichloromethane (10 mL) and N,N-dimethylformamide (3 mL). The reaction mixture was stirred at room temperature overnight, then quenched with water (30 mL) and extracted with dichloromethane (3 × 40 mL). The combined organic phases were washed with saturated sodium chloride aqueous solution (30 mL), dried over anhydrous sodium sulfate, and concentrated under vacuum. The residue was purified by reverse-phase chromatography (mobile phase A: water (10 mmol/L ammonium bicarbonate); mobile phase B: acetonitrile) to afford compound B116 as a white solid (23.5 mg, 20.72%). ESI/MS (m/z): 263.2 [M+H]⁺.¹H NMR (400 MHz, DMSO-*d*₆) δ 11.99 (s, 1H), 7.65 (dd, *J= 8.3,* 1.1 Hz, 1H), 7.58 (dd, *J=* 7.3, 1.0 Hz, 1H), 7.36 (dd, *J=* 8.3, 7.3 Hz, 1H), 3.12-3.04 (m, 2H), 2.61-2.53 (m, 2H), 2.13-2.07 (m, 2H).

### Example 179: Synthesis of Compound B117

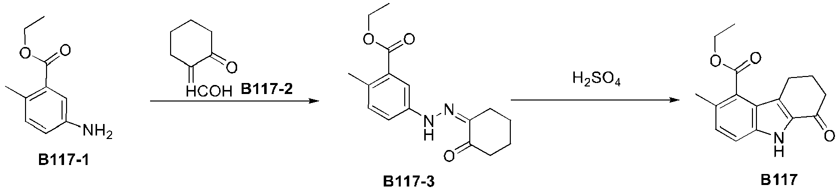

At 0 °C, a solution of sodium nitrite (1.15 g, 16.740 mmol) in water (10 mL) was slowly added to a solution of compound B117-1 (2 g, 11.160 mmol) in water (50 mL) and concentrated hydrochloric acid (4.9 mL, 55.912 mmol). The reaction mixture was stirred for 10 minutes, followed by the slow addition of a solution of compound B117-2 (1.76 g, 13.392 mmol) in methanol (60 mL) and a solution of sodium acetate (2.01 g, 24.552 mmol) in water (40 mL). The reaction was continued at 0 °C for 2 hours and then filtered. The filter cake was washed with water (40 mL) and dried under vacuum to afford compound B117-3 as an orange solid (3.2 g, 86.72%). ESI/MS (m/z): 288.9 [M+H]⁺.

At 0 °C, 10% dilute sulfuric acid (33 mL) was added to a solution of compound B117-3 (3 g, 9.072 mmol) in acetonitrile (100 mL). The mixture was heated to 75 °C and stirred for 4 hours. After cooling to room temperature, the pH was adjusted to 7 with saturated sodium carbonate solution. The mixture was concentrated under vacuum and extracted with ethyl acetate (3 × 100 mL). The combined organic phases were washed with saturated sodium chloride aqueous solution (100 mL), dried over anhydrous sodium sulfate, and concentrated under vacuum. The residue was purified by silica gel column chromatography (petroleum ether:ethyl acetate = 5:1) to afford compound B117 as a yellow solid (180.3 mg, 7.13%). ESI/MS (m/z): 272.1 [M+H]⁺.¹H NMR (400 MHz, DMSO-*d₆*) δ 11.79 (s, 1H), 7.41 (d, *J =* 8.5 Hz, 1H), 7.20 (d, *J =* 8.6 Hz, 1H), 4.40 (q, *J =* 7.1 Hz, 2H), 2.77 (t, *J =* 6.0 Hz, 2H), 2.55 (t, *J* = 6.4 Hz, 2H), 2.34 (s, 3H), 2.15-2.07 (m, 2H), 1.35 (t, *J =* 7.1 Hz, 3H).

### Example 180: Synthesis of Compound B118 and B125

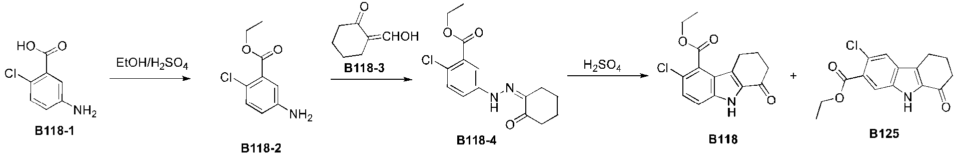

At 0 °C, concentrated sulfuric acid (3 mL) was added to a solution of compound B118-1 (10 g, 58.282 mmol) in ethanol (100 mL). The mixture was heated to 80 °C and stirred for 24 hours. After cooling to room temperature, the reaction mixture was concentrated under vacuum. The residue was adjusted to pH 8 with saturated sodium carbonate solution and extracted with ethyl acetate (3 × 150 mL). The combined organic phases were washed with saturated sodium chloride aqueous solution (100 mL), dried over anhydrous sodium sulfate, and concentrated under vacuum to afford compound B118-2 as a black solid (9.5 g, 80.02%). ESI/MS (m/z): 200.0 [M+H]⁺.

At 0 °C, a solution of sodium nitrite (0.76 g, 11.046 mmol) in water (10 mL) was slowly added to a solution of compound B118-2 (1.5 g, 7.364 mmol) in water (40 mL) and concentrated hydrochloric acid (3.7 mL, 36.820 mmol). The reaction mixture was stirred for 10 minutes, followed by the slow addition of a solution of compound B118-3 (1.16 g, 8.837 mmol) in methanol (60 mL) and a solution of sodium acetate (1.33 g, 16.201 mmol) in water (30 mL). The reaction was continued at 0 °C for 2 hours and then filtered. The filter cake was washed with water (30 mL) and dried under vacuum to afford compound B118-4 as an orange solid (1.8 g, 63.34%). ESI/MS (m/z): 309.1 [M+H]⁺.

At 0 °C, 10% dilute sulfuric acid (15 mL) was added to a solution of compound B118-4 (1.8 g, 4.664 mmol) in acetonitrile (50 mL). The mixture was heated to 75 °C and stirred for 4 hours. After cooling to room temperature, the pH was adjusted to 7 with saturated sodium carbonate solution. The mixture was concentrated under vacuum and extracted with ethyl acetate (3 × 100 mL). The combined organic phases were washed with saturated sodium chloride aqueous solution (100 mL), dried over anhydrous sodium sulfate, and concentrated under vacuum. The residue was purified by reverse-phase chromatography (mobile phase A: water (0.1% formic acid); mobile phase B: acetonitrile) to afford compound B118 as a white solid (69.8 mg) and compound B125 (11.8 mg).

Compound B118: ESI/MS (m/z): 292.1 [M+H]⁺.¹H NMR (400 MHz, DMSO-*d₆*) δ 12.13 (s, 1H), 7.52 (d, *J=* 8.9 Hz, 1H), 7.38 (d, *J=* 8.9 Hz, 1H), 4.44 (q, *J* = 7.1 Hz, 2H), 2.77 (t, *J =* 6.0 Hz, 2H), 2.58 (t, 2H), 2.19-2.08 (m, 2H), 1.36 (t, *J =* 7.1 Hz, 3H).

Compound B125: ESI/MS (m/z): 292.1 [M+H]⁺.¹H NMR (400 MHz, DMSO-*d*₆) δ 12.06 (s, 1H), 7.91 (s, 1H), 7.85 (s, 1H), 4.34 (q, *J=* 7.1 Hz, 2H), 2.96 (t, *J=* 6.0 Hz, 2H), 2.61 (t, *J=* 6.3 Hz, 2H), 2.22-2.11 (m, 2H), 1.34 (t, *J =* 7.1 Hz, 3H).

### Example 181: Synthesis of Compound B119

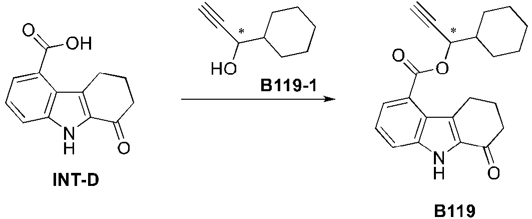

At room temperature, 4-dimethylaminopyridine (94.6 mg, 0.774 mmol) and N,N'-dicyclohexylcarbodiimide (199.7 mg, 0.968 mmol) were added to a mixture of compound INT-D (150 mg, 0.645 mmol) and compound B119-1 (89.2 mg, 0.645 mmol) in dichloromethane (5 mL) and N,N-dimethylformamide (5 mL). The reaction mixture was stirred at room temperature overnight and then concentrated under vacuum. The residue was purified by reverse-phase chromatography (mobile phase A: water (10 mmol/L ammonium bicarbonate); mobile phase B: acetonitrile) to afford compound B119 as a white solid (25.6 mg, 11.34%, racemate). ESI/MS (m/z): 350.1 [M+H]⁺.¹H NMR (400 MHz, DMSO-*d₆*) δ 12.04 (s, 1H), 7.69 (dd, *J=* 8.3, 1.0 Hz, 1H), 7.61 (dd, *J =* 7.3, 1.0 Hz, 1H), 7.40 (dd, *J =* 8.3, 7.3 Hz, 1H), 5.44 (dd, *J=* 5.9, 2.2 Hz, 1H), 3.62 (d, *J =* 2.1 Hz, 1H), 3.12 (t, *J =* 6.0 Hz, 2H), 2.62-2.54 (m, 2H), 2.15-2.06 (m, 2H), 1.95-1.79 (m, 3H), 1.78-1.69 (m, 2H), 1.68-1.59 (m, 1H), 1.32-1.09 (m, 5H).

### Example182: Synthesis of Compound B120

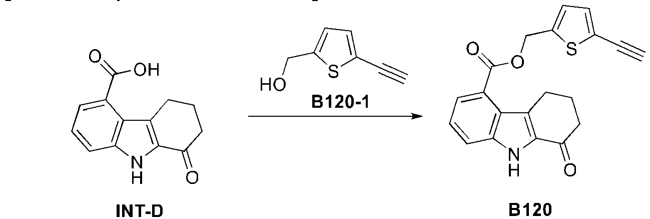

At room temperature, N,N'-dicyclohexylcarbodiimide (202.5 mg, 0.981 mmol) and compound B120-1 (89.2 mg, 0.645 mmol) were added to a mixture of compound INT-D (150 mg, 0.654 mmol) and 4-dimethylaminopyridine (95.9 mg, 0.785 mmol) in dichloromethane (10 mL) and N,N-dimethylformamide (3 mL). The reaction mixture was stirred at room temperature overnight, then quenched with water (30 mL) and extracted with dichloromethane (3 × 40 mL). The combined organic phases were washed with saturated sodium chloride aqueous solution (30 mL), dried over anhydrous sodium sulfate, and concentrated under vacuum. The residue was purified by reverse-phase chromatography (mobile phase A: water (0.1% formic acid); mobile phase B: acetonitrile) to afford compound B120 as an off-white solid (10.3 mg, 4.56%). ESI/MS (m/z): 350.2 [M+H]⁺.¹H NMR (400 MHz, DMSO-*d*₆) δ 12.02 (s, 1H), 7.67 (d, *J= 8.3* Hz, 1H), 7.61 (d, *J=* 7.3 Hz, 1H), 7.41-7.34 (m, 1H), 7.30 (d, *J* = 3.7 Hz, 1H), 7.24 (d, *J* = 3.7 Hz, 1H), 5.54 (s, 2H), 4.59 (s, 1H), 3.10-3.02 (m, 2H), 2.60-2.53 (m, 2H), 2.14-2.03 (m, 2H).

### Example 183: Synthesis of Compound B121

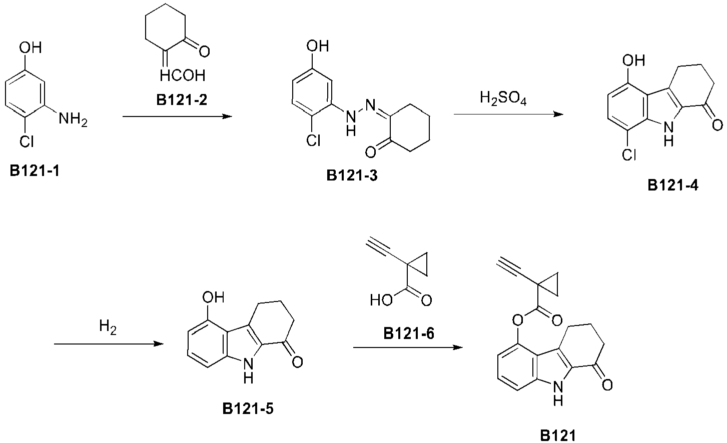

At 0 °C, a solution of sodium nitrite (1.44 g, 20.895 mmol) in water (10 mL) was slowly added to a solution of compound B121-1 (2 g, 13.930 mmol) in water (50 mL) and concentrated hydrochloric acid (6.1 mL, 69.650 mmol). The reaction mixture was stirred for 10 minutes, followed by the slow addition of a solution of compound B121-2 (2.19 g, 16.716 mmol) in methanol (60 mL) and a solution of sodium acetate (2.51 g, 30.646 mmol) in water (40 mL). The mixture was stirred at 0 °C for an additional 2 hours, then filtered. The filter cake was washed with water (40 mL) and dried under vacuum to afford compound B121-3 as a gray solid (2.8 g, 62.04%). ESI/MS (m/z): 252.9 [M+H]⁺.

At 0 °C, 10% dilute sulfuric acid (33 mL) was added to a solution of compound B121-3 (2.6 g, 8.025 mmol) in acetonitrile (100 mL). The mixture was heated to 75 °C and stirred for 4 hours. After cooling to room temperature, the pH was adjusted to 7 with saturated sodium carbonate solution. The mixture was concentrated under vacuum and extracted with ethyl acetate (3 × 100 mL). The combined organic phases were washed with saturated sodium chloride aqueous solution (100 mL), dried over anhydrous sodium sulfate, and concentrated under vacuum. The residue was purified by silica gel column chromatography (petroleum ether:ethyl acetate = 5:1) to afford compound B121-4 as a gray solid (1.2 g, 63.45%). ESI/MS (m/z): 235.9 [M+H]⁺.

Under a nitrogen atmosphere, triethylamine (689 mg, 6.807 mmol) and methanol (40 mL) were added to a mixture of compound B121-4 (1.15 mg, 4.538 mmol) and 10% palladium on carbon (724 mg, 0.681 mmol) in ethyl acetate (40 mL). The reaction mixture was purged three times with hydrogen and stirred at room temperature for 3 hours. The mixture was filtered, and the filter cake was washed with ethyl acetate (20 mL). The filtrate was concentrated under vacuum, and the residue was purified by silica gel column chromatography (petroleum ether:ethyl acetate = 1:1) to afford compound B121-5 as a yellow solid (650 mg, 64.77%). ESI/MS (m/z): 201.9 [M+H]⁺.

At room temperature, N,N'-dicyclohexylcarbodiimide (202 mg, 0.977 mmol) was added to a solution of compound B121-5 (180 mg, 0.814 mmol) and compound B121-6 (108 mg, 0.977 mmol) in dichloromethane (10 mL). The reaction mixture was stirred at room temperature for 3 hours, then quenched with water (20 mL) and extracted with ethyl acetate (3 × 30 mL). The combined organic phases were washed with saturated sodium chloride aqueous solution (30 mL), dried over anhydrous sodium sulfate, and concentrated under vacuum. The residue was purified by reverse-phase chromatography (mobile phase A: water (0.1% formic acid); mobile phase B: acetonitrile) to afford compound B121 as a white solid (60.6 mg, 25.15%). ESI/MS (m/z): 294.2 [M+H]⁺.

### Example184: Synthesis of Compound B122

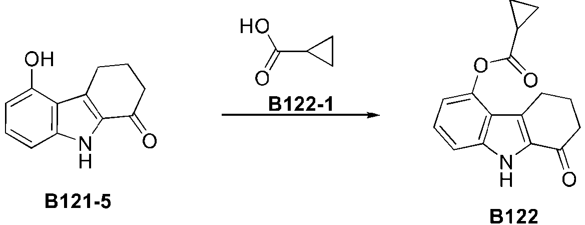

At room temperature, N,N'-dicyclohexylcarbodiimide (202 mg, 0.977 mmol) was added to a mixture of compound B121-5 (180 mg, 0.814 mmol) and compound B122-1 (84 mg, 0.977 mmol) in dichloromethane (10 mL). The reaction mixture was stirred at room temperature for 3 hours, then quenched with water (20 mL) and extracted with ethyl acetate (3 × 30 mL). The combined organic phases were washed with saturated sodium chloride aqueous solution (30 mL), dried over anhydrous sodium sulfate, and concentrated under vacuum. The residue was purified by reverse-phase chromatography (mobile phase A: water (0.1% formic acid); mobile phase B: acetonitrile) to afford compound B122 as a white solid (73.9 mg, 33.48%). ESI/MS (m/z): 270.4 [M+H]⁺.¹H NMR (400 MHz, DMSO-*d*₆) δ 11.82 (s, 1H), 7.30-7.24 (m, 2H), 6.79 (dd, *J=* 6.0, 2.4 Hz, 1H), 2.95 (t, *J=* 6.0 Hz, 2H), 2.55 (t, *J=* 6.3 Hz, 2H), 2.20-2.09 (m, 2H), 2.06-1.95 (m, 1H), 1.15-1.09 (m, 2H), 1.09-1.04 (m, 2H).

### Example 185: Synthesis of Compound B123

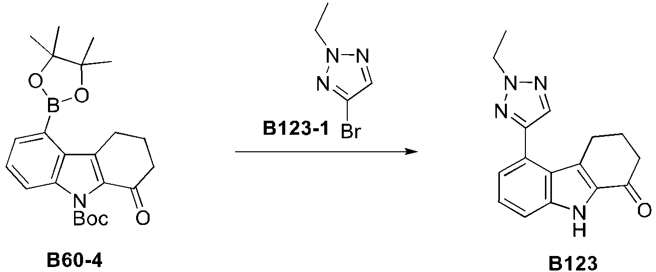

Under a nitrogen atmosphere, cesium carbonate (334 mg, 1.02 mmol) and [1,1'-bis(diphenylphosphino)ferrocene]dichloropalladium(II) dichloromethane complex (83 mg, 0.10 mmol) were added to a solution of compound B60-4 (400 mg, 0.77 mmol) and compound B123-1 (90 mg, 0.51 mmol) in 1,4-dioxane (8 mL) and water (1.6 mL). The mixture was heated to 100 °C and stirred overnight. After cooling to room temperature, the reaction was quenched with water (20 mL) and extracted with ethyl acetate (3 × 20 mL). The combined organic phases were washed with saturated sodium chloride aqueous solution (20 mL), dried over anhydrous sodium sulfate, and concentrated under vacuum. The residue was purified by reverse-phase chromatography (mobile phase A: water (10 mmol/L ammonium bicarbonate); mobile phase B: acetonitrile) to afford compound B123 as a white solid (28.9 mg, 20.08%). ESI/MS (m/z): 281.1 [M+H]⁺.¹H NMR (400 MHz, DMSO-*d*₆) δ 11.82 (s, 1H), 8.00 (s, 1H), 7.47 (dd, *J=* 8.3, 1.0 Hz, 1H), 7.35 (dd, *J=* 8.3, 7.1 Hz, 1H), 7.16 (dd, *J* = 7.2, 1.0 Hz, 1H), 4.54 (q, *J=* 7.3 Hz, 2H), 2.76 (t, *J=* 6.0 Hz, 2H), 2.56-2.51 (m, 2H), 2.06-2.98 (m, 2H), 1.53 (t, *J =* 7.3 Hz, 3H).

### Example 186: Synthesis of Compound B124

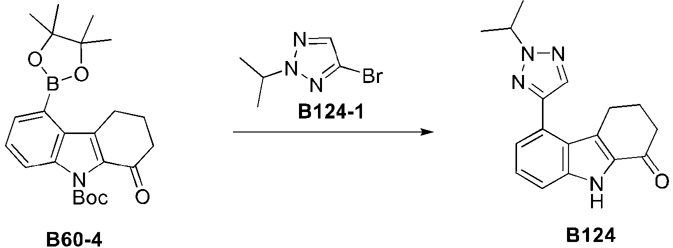

Under a nitrogen atmosphere, cesium carbonate (463 mg, 1.42 mmol) and [1,1'-bis(diphenylphosphino)ferrocene]dichloropalladium(II) dichloromethane complex (77 mg, 0.10 mmol) were added to a solution of compound B60-4 (321 mg, 0.71 mmol) and compound B124-1 (90 mg, 0.47 mmol) in 1,4-dioxane (8 mL) and water (2 mL). The mixture was heated to 100 °C and stirred overnight. After cooling to room temperature, the reaction was quenched with water (20 mL) and extracted with ethyl acetate (3 × 20 mL). The combined organic phases were washed with saturated sodium chloride aqueous solution (20 mL), dried over anhydrous sodium sulfate, and concentrated under vacuum. The residue was purified by reverse-phase chromatography (mobile phase A: water (10 mmol/L ammonium bicarbonate); mobile phase B: acetonitrile) to afford compound B124 as a white solid (50.2 mg, 35.94%). ESI/MS (m/z): 295.2 [M+H]⁺.¹H NMR (400 MHz, DMSO-*d*₆) δ 11.81 (s, 1H), 8.00 (s, 1H), 7.47 (dd, *J=* 8.3, 1.0 Hz, 1H), 7.35 (dd, *J=* 8.3, 7.1 Hz, 1H), 7.17 (dd, *J =* 7.1, 1.0 Hz, 1H), 4.96-4.84 (m, 1H), 2.77 (t, *J =* 6.0 Hz, 2H), 2.56-2.51 (m, 2H), 2.07-1.97 (m, 2H), 1.57 (d, *J=* 6.7 Hz, 6H).

### Example 187: Synthesis of Compound B126

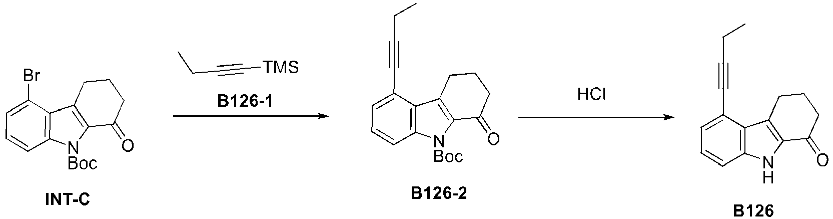

Under a nitrogen atmosphere, copper(I) iodide (5.1 mg, 0.027 mmol), palladium(II) acetate (6.1 mg, 0.027 mmol), 1,1'-bis(diphenylphosphino)ferrocene (14.9 mg, 0.027 mmol), and cesium carbonate (175.3 mg, 0.538 mmol) were added to a solution of compound INT-C (100 mg, 0.269 mmol) and compound B126-1 (101.9 mg, 0.807 mmol) in N,N-dimethylacetamide (4 mL). The mixture was heated to 80 °C and stirred for 2 hours. After cooling to room temperature, the reaction was quenched with water (80 mL) and extracted with ethyl acetate (3 × 30 mL). The combined organic phases were washed with saturated sodium chloride aqueous solution (20 mL), dried over anhydrous sodium sulfate, and concentrated under vacuum. The residue was purified by silica gel column chromatography (petroleum ether:ethyl acetate = 4:1) to afford compound B126-2 as a brown oil (80 mg, 65.74%). ESI/MS (m/z): 338.3 [M+H]⁺.

At room temperature, a 4M hydrogen chloride solution in ethyl acetate (3 mL) was added to compound B126-2 (70 mg, 0.155 mmol). The mixture was stirred for 30 minutes and then concentrated under vacuum. The residue was purified by reverse-phase chromatography (mobile phase A: water (0.1% formic acid); mobile phase B: acetonitrile) to afford compound B126 as a white solid (7.7 mg, 20.84%). ESI/MS (m/z): 238.3 [M+H]⁺.¹H NMR (400 MHz, DMSO-*d*₆) δ 11.74 (s, 1H), 7.36 (d, *J* = 8.2 Hz, 1H), 7.22 (dd, *J=* 8.3, 7.2 Hz, 1H), 7.07 (d, *J=* 7.1 Hz, 1H), 3.30-3.18 (m, 2H), 2.58-2.53 (m, 2H), 2.52-2.47 (m, 2H), 2.21-2.10 (m, 2H), 1.22 (t, *J* = 7.5 Hz, 3H).

### Example 188: Synthesis of Compound B127

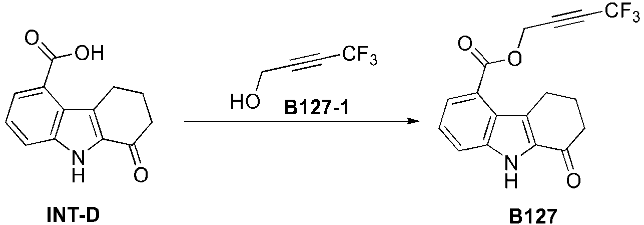

At 0 °C, diisopropyl azodicarboxylate (130.5 mg, 0.645 mmol) was added dropwise to a mixture of compound INT-D (100 mg, 0.430 mmol), compound B127-1 (58.70 mg, 0.473 mmol), and triphenylphosphine (169.2 mg, 0.645 mmol) in tetrahydrofuran (4 mL). The reaction mixture was stirred at room temperature for 30 minutes, then quenched with water (20 mL) and extracted with dichloromethane (3 × 20 mL). The combined organic phases were washed with saturated sodium chloride aqueous solution (10 mL), dried over anhydrous sodium sulfate, and concentrated under vacuum. The residue was purified by reverse-phase chromatography (mobile phase A: water (10 mmol/L ammonium bicarbonate); mobile phase B: acetonitrile) to afford compound B127 as a white solid (14.8 mg, 2.33%). ESI/MS (m/z): 336.2 [M+H]⁺.¹H NMR (400 MHz, DMSO-*d*₆) δ 12.08 (s, 1H), 7.71 (dd, *J =* 8.2, 1.0 Hz, 1H), 7.65 (dd, *J=* 7.4, 1.1 Hz, 1H), 7.40 (dd, *J=* 8.3, 7.3 Hz, 1H), 5.33-5.26 (m, 2H), 3.14-3.05 (m, 2H), 2.63-2.54 (m, 2H), 2.16-2.04 (m, 2H).

### Example 189: Synthesis of Compound B128

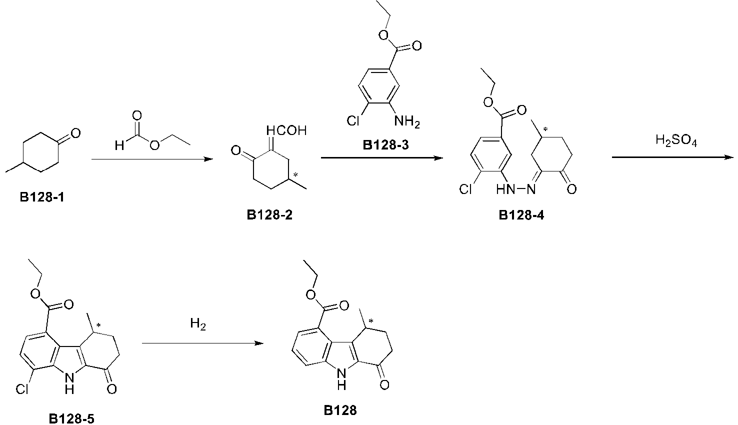

At 0 °C, a solution of ethyl formate (1.19 g, 16.046 mmol) in methanol (0.5 mL) was added to a mixture of 60% sodium hydride (482 mg, 12.034 mmol) and compound B128-1 (900 mg, 8.023 mmol) in toluene (30 mL). The reaction mixture was stirred at room temperature for 1 hour, then adjusted to pH 3 with dilute hydrochloric acid and extracted with ethyl acetate (3 × 150 mL). The combined organic phases were washed with saturated sodium chloride aqueous solution (100 mL), dried over anhydrous sodium sulfate, and concentrated under vacuum. The residue was purified by silica gel column chromatography (petroleum ether:ethyl acetate = 5:1) to afford compound B128-2 as a colorless oil (900 mg, 76.82%). ESI/MS (m/z): 141.0 [M+H]⁺.

At 0 °C, a solution of sodium nitrite (0.49 g, 7.168 mmol) in water (10 mL) was slowly added to a solution of compound B128-3 (1 g, 4.779 mmol) in water (20 mL) and concentrated hydrochloric acid (2.07 mL, 23.895 mmol). The reaction mixture was stirred for 10 minutes, followed by the slow addition of a solution of compound B128-2 (0.84 g, 5.735 mmol) in methanol (40 mL) and a solution of sodium acetate (0.86 g, 10.514 mmol) in water (15 mL). The mixture was stirred at 0 °C for an additional 2 hours and then filtered. The filter cake was washed with water (40 mL) and dried under vacuum to afford compound B128-4 as a red solid (1 g, 48.62%). ESI/MS (m/z): 322.9 [M+H]⁺.

At 0 °C, 10% dilute sulfuric acid (10 mL) was added to a solution of compound B128-4 (1 g, 2.323 mmol) in acetonitrile (50 mL). The mixture was heated to 75 °C and stirred for 4 hours. After cooling to room temperature, the pH was adjusted to 7 with saturated sodium carbonate solution. The mixture was concentrated under vacuum and extracted with ethyl acetate (3 × 100 mL). The combined organic phases were washed with saturated sodium chloride aqueous solution (100 mL), dried over anhydrous sodium sulfate, and concentrated under vacuum. The residue was purified by silica gel column chromatography (petroleum ether:ethyl acetate = 5:1) to afford compound B128-5 as a red solid (260 mg, 32.54%). ESI/MS (m/z): 305.9 [M+H]⁺.

Under a nitrogen atmosphere, triethylamine (135 mg, 1.338 mmol) and methanol (10 mL) were added to a mixture of compound B128-5 (230 mg, 0.669 mmol) and 10% palladium on carbon (14 mg, 0.013 mmol) in ethyl acetate (10 mL). The reaction system was purged three times with hydrogen and stirred at room temperature for 4 hours. The mixture was filtered, and the filter cake was washed with ethyl acetate (20 mL). The filtrate was concentrated under vacuum, and the residue was purified by reverse-phase chromatography (mobile phase A: water (0.1% formic acid); mobile phase B: acetonitrile) to afford compound B128 as a white solid (38.2 mg, 20.91%, racemate). ESI/MS (m/z): 272.2 [M+H]⁺.¹H NMR (400 MHz, DMSO-*d*₆) δ 12.00 (s, 1H), 7.65 (dd, *J =* 8.3, 1.0 Hz, 1H), 7.59 (dd, *J =* 7.3, 1.0 Hz, 1H), 7.37 (dd, *J=* 8.3, 7.3 Hz, 1H), 4.37 (q, *J =* 7.1 Hz, 2H), 3.30-3.22 (m, 1H), 2.77-2.66 (m, 1H), 2.58-2.51 (m, 1H), 2.48-2.39 (m, 1H), 2.39-2.28 (m, 1H), 1.37 (t, *J =* 7.1 Hz, 3H), 1.14 (d, *J=* 6.4 Hz, 3H).

### Example 190: Synthesis of Compound B129

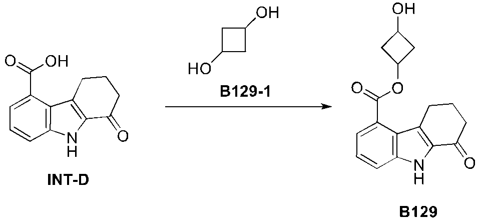

At room temperature, N,N'-dicyclohexylcarbodiimide (133 mg, 0.645 mmol) and 4-dimethylaminopyridine (53 mg, 0.430 mmol) were added to a mixture of compound INT-D (100 mg, 0.430 mmol) and compound B129-1 (46 mg, 0.516 mmol) in dichloromethane (3 mL) and N,N-dimethylformamide (3 mL). The reaction mixture was stirred at room temperature for 4 hours and then concentrated under vacuum. The residue was purified by reverse-phase chromatography (mobile phase A: water (0.1% formic acid); mobile phase B: acetonitrile) to afford compound B129 as a white solid (12.2 mg, 9.33%, mixture of cis-trans isomers). ESI/MS (m/z): 300.1 [M+H]⁺.

### Example 191: Synthesis of Compound B130

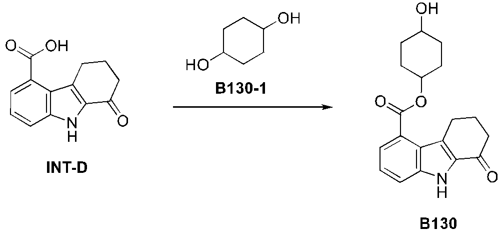

At room temperature, N,N'-dicyclohexylcarbodiimide (133 mg, 0.645 mmol) and 4-dimethylaminopyridine (53 mg, 0.430 mmol) were added to a solution of compound INT-D (100 mg, 0.430 mmol) and compound B130-1 (60 mg, 0.516 mmol) in dichloromethane (3 mL) and N,N-dimethylformamide (3 mL). The reaction mixture was stirred at room temperature for 4 hours and then concentrated under vacuum. The residue was purified by reverse-phase chromatography (mobile phase A: water (0.1% formic acid); mobile phase B: acetonitrile) to afford compound B130 as a white solid (11.1 mg, 7.81%, mixture of cis-trans isomers). ESI/MS (m/z): 328.2 [M+H]⁺.

### Example 192: Synthesis of Compound B131

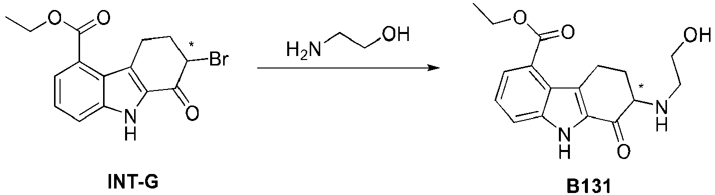

At room temperature, ethanolamine (34.9 mg, 0.571 mmol) was added to a solution of compound INT-G (100.0 mg, 0.277 mmol, racemate) in N,N-dimethylformamide (2 mL). The mixture was stirred for 2 hours, then diluted with ethyl acetate (100 mL) and washed with water (50 mL × 5). The organic phase was dried over anhydrous sodium sulfate and concentrated under vacuum. A 4M hydrogen chloride solution in ethyl acetate (2 mL) was added to the residue, precipitating a yellow solid. The yellow solid was purified by reverse-phase column chromatography (acetonitrile/water = 10%-50%) to afford compound B131 as a pale yellow solid (4.370 mg, 4.64%, racemate). ESI/MS (m/z): 317.1 [M+H]⁺.

### Example 193: Synthesis of Compound B132

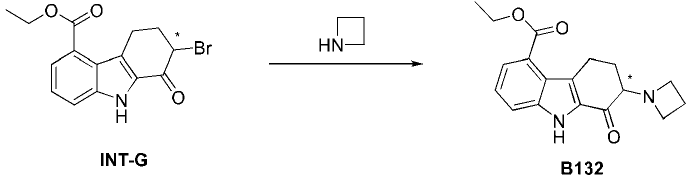

At room temperature, azetidine (31.6 mg, 0.553 mmol) was added to a solution of compound INT-G (100.0 mg, 0.277 mmol, racemate) in N,N-dimethylformamide (4 mL). The mixture was stirred for 10 minutes, then diluted with ethyl acetate (100 mL) and washed with water (50 mL × 5). The organic phase was dried over anhydrous sodium sulfate and concentrated under vacuum. A 4M hydrogen chloride solution in ethyl acetate (2 mL) was added to the residue, precipitating a yellow solid. The solid was dried under vacuum to afford the hydrochloride salt of compound B132 (33.3 mg, 32.39%, racemate). ESI/MS (m/z) 313.1 [M+H]⁺.

### Example194: Synthesis of Compound B134

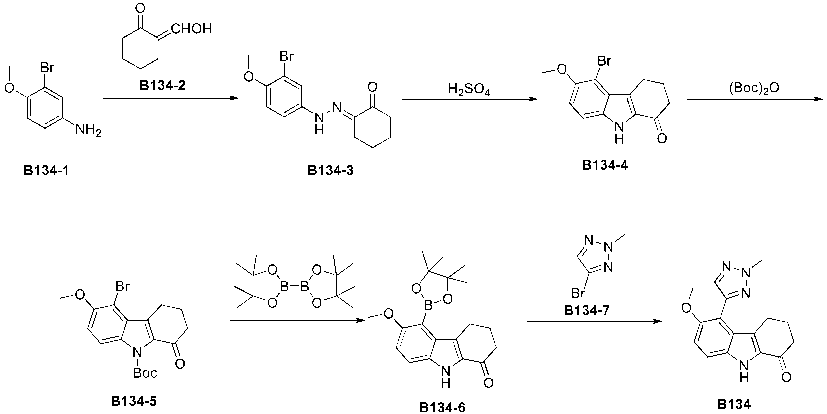

At 5 °C, concentrated hydrochloric acid (4.2 mL) and a solution of sodium nitrite (1.37 g, 19.8 mmol) in water (5 mL) were sequentially added to a solution of compound B134-1 (2.0 g, 9.9 mmol) in water (20 mL). The mixture was stirred for 10 minutes. A solution of compound B134-2 (1.87 g, 14.58 mmol) in methanol (15 mL) was combined with a solution of sodium acetate (2.03 g, 24.75 mmol) in water (10 mL) and cooled to 5 °C. The previously prepared diazonium salt solution was added dropwise to this mixture, and stirring was continued for 30 minutes. The mixture was filtered by suction, and the filter cake was washed with water and dried under vacuum to afford crude compound B134-3 as a red solid (2.1 g, 68.2%). ESI/MS (m/z): 311.0 [M+H]⁺.

At room temperature, 1.8 M sulfuric acid (11 mL) was added to a solution of compound B134-3 (2.1 g, 6.75 mmol) in acetonitrile (30 mL). The mixture was heated to 80 °C and stirred for 4 hours. After cooling to room temperature, the reaction mixture was diluted with ethyl acetate (50 mL). The organic phase was washed with saturated sodium chloride aqueous solution, dried over anhydrous sodium sulfate, and concentrated under vacuum. The residue was purified by silica gel column chromatography (petroleum ether:ethyl acetate = 5:1) to afford compound B134-4 as a brown solid (0.77 g, 38.8%). ESI/MS (m/z): 293.9 [M+H]⁺.

At room temperature, 4-dimethylaminopyridine (207.7 mg, 1.70 mmol) and di-tert-butyl dicarbonate (1.48 g, 6.80 mmol) were added to a solution of compound B134-4 (1.0 g, 3.40 mmol) in tetrahydrofuran (10 mL). The mixture was stirred for 1 hour, then quenched with water (10 mL) and extracted with ethyl acetate (15 mL × 3). The combined organic phases were washed with saturated sodium chloride aqueous solution, dried over anhydrous sodium sulfate, and concentrated under vacuum. The residue was purified by silica gel column chromatography (petroleum ether:ethyl acetate = 5:1) to afford compound B134-5 as a brown solid (240.0 mg, 17.9%). ESI/MS (m/z): 416.0 [M+Na]⁺.

Under a nitrogen atmosphere, bis(pinacolato)diboron (263.4 mg, 1.12 mmol), [1,1'-bis(diphenylphosphino)ferrocene]dichloropalladium(II) dichloromethane complex (41.1 mg, 0.05 mmol), and potassium acetate (199.1 mg, 2.03 mmol) were added to a solution of compound B134-5 (400.0 mg, 1.01 mmol) in N,N-dimethylformamide (8 mL). The mixture was heated to 120 °C and stirred overnight. After cooling to room temperature, the reaction was quenched with water (20 mL) and extracted with ethyl acetate (25 mL × 3). The combined organic phases were washed with saturated sodium chloride aqueous solution, dried over anhydrous sodium sulfate, and concentrated under vacuum. The residue was purified by silica gel column chromatography (petroleum ether:ethyl acetate = 3:1) to afford compound B134-6 as a white solid (230.0 mg, 66.4%). ESI/MS (m/z): 342.1 [M+H]⁺.

Under a nitrogen atmosphere, [1,1'-bis(diphenylphosphino)ferrocene]dichloropalladium(II) dichloromethane complex (24.9 mg, 0.031 mmol) and potassium carbonate (170.1 mg, 1.23 mmol) were added to a solution of compound B134-6 (210.0 mg, 0.62 mmol) and compound B134-7 (99.7 mg, 0.62 mmol) in 1,4-dioxane (4 mL) and water (0.8 mL). The mixture was heated to 100 °C and stirred for 2 hours. The reaction was quenched with water (10 mL) and extracted with ethyl acetate (15 mL × 3). The combined organic phases were washed with saturated sodium chloride aqueous solution (10 mL), dried over anhydrous sodium sulfate, and concentrated under vacuum. The residue was purified by silica gel column chromatography (petroleum ether:ethyl acetate = 3:1) to afford compound B134 as a light yellow solid (90.0 mg, 49.3%). ESI/MS (m/z) 297.0 [M+H]⁺.¹H NMR (500 MHz, DMSO-*d*₆) δ 11.59 (s, 1H), 7.82 (s, 1H), 7.45 (d, *J =* 9.0 Hz, 1H), 7.25 (d, *J =* 9.1 Hz, 1H), 4.23 (s, 3H), 3.72 (s, 3H), 2.47 (dd, *J =* 7.3, 5.5 Hz, 2H), 2.33 (t, *J =* 6.0 Hz, 2H), 1.98-1.90 (m, 2H).

### Example 195: Synthesis of Compound B135

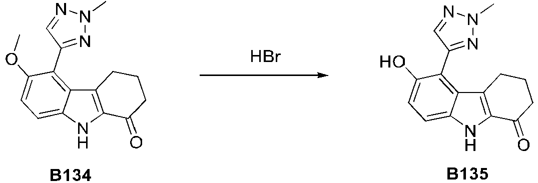

Under a nitrogen atmosphere, compound B134 (100.0 mg, 0.34 mmol) was added to a 48% hydrobromic acid aqueous solution (1.5 mL). The mixture was heated to 120 °C and stirred for 2 hours. After cooling to room temperature, the reaction was quenched with saturated sodium chloride aqueous solution (10 mL) and extracted with ethyl acetate (20 mL × 3). The combined organic phases were washed with saturated sodium chloride aqueous solution (20 mL), dried over anhydrous sodium sulfate, and concentrated under vacuum. The residue was purified by reverse-phase preparative chromatography (acetonitrile/water = 10-60%) to afford compound B135 as a pale-yellow solid (37.0 mg, 38.8%). ESI/MS (m/z): 283.1 [M+H]⁺.¹H NMR (500 MHz, DMSO-*d*₆) δ 11.47 (s, 1H), 9.01 (s, 1H), 7.81 (s, 1H), 7.30 (d, *J =* 8.8 Hz, 1H), 7.01 (d, *J =* 8.8 Hz, 1H), 4.22 (s, 3H), 2.45 (dd, *J =* 7.4, 5.5 Hz, 2H), 2.38 (t, *J =* 6.0 Hz, 2H), 1.93 (p, *J =* 6.3 Hz, 2H).

### Example 196: Synthesis of Compound B136

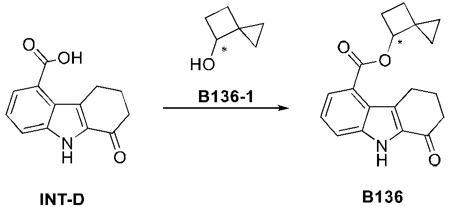

At room temperature, 4-dimethylaminopyridine (31.7 mg, 0.26 mmol) and 1-(3-dimethylaminopropyl)-3-ethylcarbodiimide hydrochloride (75.0 mg, 0.39 mmol) were added to a mixture of compound INT-D (60.0 mg, 0.26 mmol) and compound B136-1 (30.8 mg, 0.31 mmol) in dichloromethane (1 mL). The reaction mixture was stirred for 2 hours, then diluted with ethyl acetate (30 mL) and sequentially washed with 0.5M hydrochloric acid aqueous solution (10 mL) and saturated sodium chloride aqueous solution (10 mL). The organic phase was dried over anhydrous sodium sulfate and concentrated under vacuum. The residue was purified by silica gel column chromatography (ethyl acetate: petroleum ether = 1:5) to afford compound B136 as a white solid (55.0 mg, 67.9%, racemate). ESI/MS (m/z): 310.0 [M+H]⁺.¹H NMR (500 MHz, DMSO-*d*₆) δ 12.00 (s, 1H), 7.66 (dd, *J =* 8.2, 1.0 Hz, 1H), 7.60 (dd, *J =* 7.3, 1.0 Hz, 1H), 7.37 (dd, *J =* 8.3, 7.3 Hz, 1H), 5.35 (t, *J =* 6.7 Hz, 1H), 3.07 (qt, *J* = 17.2, 6.1 Hz, 2H), 2.60-2.51 (m, 3H), 2.34-2.27 (m, 1H), 2.15-1.99 (m, 4H), 0.79-0.75 (m, 1H), 0.73-0.69 (m, 1H), 0.60-0.56 (m, 1H), 0.51-0.47 (m, 1H).

### Example 197: Synthesis of Compound B137

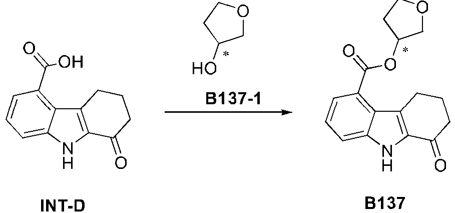

At room temperature, 4-dimethylaminopyridine (26.4 mg, 0.22 mmol) and 1-(3-dimethylaminopropyl)-3-ethylcarbodiimide hydrochloride (83.6 mg, 0.44 mmol) were added to a mixture of compound INT-D (50.0 mg, 0.22 mmol) and compound B137-1 (38.4 mg, 0.44 mmol) in dichloromethane (1 mL). The reaction mixture was stirred for 2 hours, then diluted with ethyl acetate (30 mL) and sequentially washed with 0.5M hydrochloric acid aqueous solution (10 mL) and saturated sodium chloride aqueous solution (10 mL). The organic phase was dried over anhydrous sodium sulfate and concentrated under vacuum. The residue was purified by silica gel column chromatography (ethyl acetate: petroleum ether = 1:6) to afford compound B137 as a white solid (47.0 mg, 72%, racemate). ESI/MS (m/z): 300.1 [M+H]⁺.¹H NMR (500 MHz, DMSO-*d*₆) δ 12.00 (s, 1H), 7.66 (dd, *J =* 8.3, 1.0 Hz, 1H), 7.60 (dd, *J =* 7.3, 1.0 Hz, 1H), 7.37 (dd, *J =* 8.3, 7.3 Hz, 1H), 5.53-5.50 (m, 1H), 3.95-3.85 (m, 3H), 3.79 (td, *J =* 8.4, 4.5 Hz, 1H), 3.10 (dd, *J =* 6.8, 5.4 Hz, 2H), 2.57 (dd, *J =* 7.3, 5.6 Hz, 2H), 2.33-2.25 (m, 1H), 2.11 (p, *J =* 6.0 Hz, 3H).

### Example 198: Synthesis of Compound B138

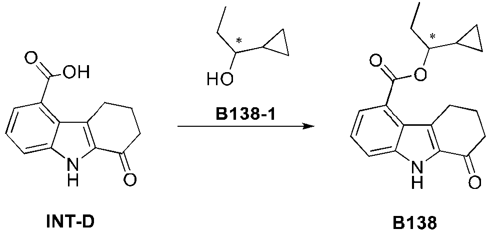

At room temperature, 4-dimethylaminopyridine (26.4 mg, 0.22 mmol) and 1-(3-dimethylaminopropyl)-3-ethylcarbodiimide hydrochloride (83.6 mg, 0.44 mmol) were added to a mixture of compound INT-D (50.0 mg, 0.22 mmol) and compound B138-1 (43.7 mg, 0.44 mmol) in dichloromethane (1 mL). The reaction mixture was stirred for 12 hours, then diluted with ethyl acetate (30 mL) and sequentially washed with 0.5M hydrochloric acid aqueous solution (10 mL) and saturated sodium chloride aqueous solution (20 mL). The organic phase was dried over anhydrous sodium sulfate and concentrated under vacuum. The residue was purified by reverse-phase preparative chromatography (acetonitrile/water = 10-60%) to afford compound B138 as a pale-yellow solid (41.7 mg, 61.4%, racemate). ESI/MS (m/z): 312.1 [M+H]⁺.¹H NMR (500 MHz, DMSO-*d*₆) δ 11.98 (s, 1H), 7.65 (dd, *J =* 8.4, 1.0 Hz, 1H), 7.58 (dd, *J=* 7.3, 1.0 Hz, 1H), 7.38 (dd, *J =* 8.3, 7.3 Hz, 1H), 4.47-4.43 (m, 1H), 3.10 (t, *J =* 6.0 Hz, 2H), 2.57 (dd, *J =* 7.6, 5.5 Hz, 2H), 2.17-2.05 (m, 2H), 1.87-1.75 (m, 2H), 1.19-1.10 (m, 1H), 0.98 (t, *J =* 7.4 Hz, 3H), 0.64-0.57 (m, 1H), 0.56-0.47 (m, 1H), 0.460.38 (m, 2H).

### Example 199: Synthesis of Compound B139

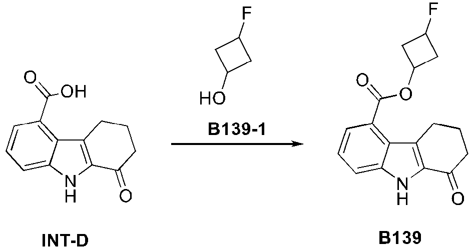

At room temperature, 4-dimethylaminopyridine (26.4 mg, 0.22 mmol) and 1-(3-dimethylaminopropyl)-3-ethylcarbodiimide hydrochloride (83.6 mg, 0.44 mmol) were added to a mixture of compound INT-D (50.0 mg, 0.22 mmol) and compound B139-1 (43.7 mg, 0.44 mmol) in dichloromethane (1 mL). The reaction mixture was stirred for 2 hours, then diluted with ethyl acetate (30 mL) and sequentially washed with 0.5M hydrochloric acid aqueous solution (10 mL) and saturated sodium chloride aqueous solution (20 mL). The organic phase was dried over anhydrous sodium sulfate and concentrated under vacuum. The residue was purified by reverse-phase preparative chromatography (acetonitrile/water = 10-60%) to afford compound B139 as a pale-yellow solid (39.0 mg, 59.3%, mixture of cis-trans isomers). ESI/MS (m/z): 302.1 [M+H]⁺.

### Example 200: Synthesis of Compound B140

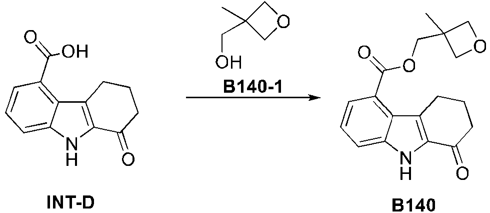

At room temperature, 4-dimethylaminopyridine (26.4 mg, 0.22 mmol) and 1-(3-dimethylaminopropyl)-3-ethylcarbodiimide hydrochloride (83.6 mg, 0.44 mmol) were added to a mixture of compound INT-D (50.0 mg, 0.22 mmol) and compound B140-1 (44.6 mg, 0.44 mmol) in dichloromethane (1 mL). The reaction mixture was stirred for 12 hours, then diluted with ethyl acetate (30 mL) and sequentially washed with 0.5M hydrochloric acid aqueous solution (10 mL) and saturated sodium chloride aqueous solution (20 mL). The organic phase was dried over anhydrous sodium sulfate and concentrated under vacuum. The residue was purified by reverse-phase preparative chromatography (acetonitrile/water = 10-60%) to afford compound B140 as a white solid (35.0 mg, 51.2%). ESI/MS (m/z) 314.0 [M+H]⁺.¹H NMR (500 MHz, DMSO-*d*₆) δ 12.01 (s, 1H), 7.67 (dd, *J =* 8.3, 1.0 Hz, 1H), 7.65 (dd, *J =* 7.3, 1.0 Hz, 1H), 7.39 (dd, *J =* 8.3, 7.3 Hz, 1H), 4.52 (d, *J =* 5.8 Hz, 2H), 4.44 (s, 2H), 4.33 (d, *J* = 5.9 Hz, 2H), 3.10 (t, *J =* 6.0 Hz, 2H), 2.58 (dd, *J =* 7.3, 5.5 Hz, 2H), 2.11 (p, *J =* 6.3 Hz, 2H), 1.36 (s, 3H).

### Example 201: Synthesis of Compound B141

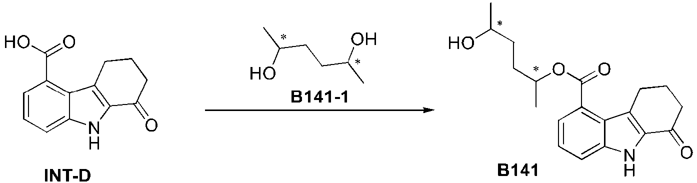

At room temperature, compound B141-1 (73.5 mg, 0.622 mmol), 4-dimethylaminopyridine (38.0 mg, 0.311 mmol), and N,N'-dicyclohexylcarbodiimide (128.3 mg, 0.622 mmol) were added to a mixture of compound INT-D (75.0 mg, 0.311 mmol) in dichloromethane (2 mL). The reaction mixture was heated to 45 °C and stirred for 6 hours. After cooling to room temperature, the mixture was diluted with dichloromethane (100 mL). The organic phase was sequentially washed with water (30 mL) and saturated sodium chloride aqueous solution (30 mL), dried over anhydrous sodium sulfate, and concentrated under vacuum. The residue was purified by normal-phase silica gel column chromatography (methanol:dichloromethane = 1:50) to afford a crude white solid. The crude product was further purified by reverse-phase column chromatography (acetonitrile:water = 10%-50%) to afford compound B141 as a white solid (47.0 mg, 43.43%). ESI/MS (m/z): 330.1 [M+H]⁺.¹H NMR (500 MHz, DMSO-*d*₆) δ 11.98 (s, 1H), 7.64 (dd, *J* = 8.3, 1.1 Hz, 1H), 7.57 (ddd, *J =* 7.3, 2.4, 1.0 Hz, 1H), 7.37 (dd, *J =* 8.3, 7.3 Hz, 1H), 5.16-5.07 (m, 1H), 4.41 (dd, *J* = 4.7, 1.5 Hz, 1H), 3.65-3.56 (m, *J =* 5.9 Hz, 1H), 3.11-3.07 (m, 2H), 2.57 (dd, *J =* 7.4, 5.6 Hz, 2H), 2.11 (p, *J* = 6.1 Hz, 2H), 1.85-1.59 (m, 2H), 1.48-1.36 (m, 2H), 1.34 (dd, *J* = 6.3, 1.3 Hz, 3H), 1.05 (d, *J =* 6.1 Hz, 3H).

### Example 202: Synthesis of Compound B142

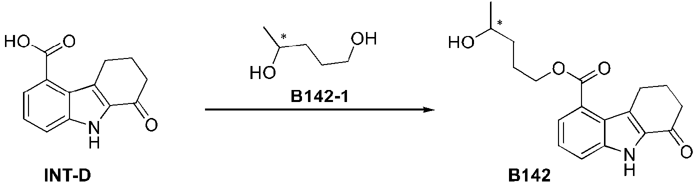

At room temperature, compound B142-1 (43.2 mg, 0.414 mmol), 4-dimethylaminopyridine (12.7 mg, 0.104 mmol), and N,N'-dicyclohexylcarbodiimide (85.5 mg, 0.414 mmol) were added to a mixture of compound INT-D (50.0 mg, 0.207 mmol) in dichloromethane (1.5 mL). The reaction mixture was stirred at 45 °C for 2 hours. After cooling to room temperature, the mixture was diluted with dichloromethane (100 mL). The organic phase was sequentially washed with water (30 mL) and saturated sodium chloride aqueous solution (30 mL), dried over anhydrous sodium sulfate, and concentrated under vacuum. The residue was purified by normal-phase silica gel column chromatography (methanol:dichloromethane = 1:50) to afford a crude white solid. The crude product was further purified by reverse-phase column chromatography (acetonitrile:water = 10%-50%) to afford compound B142 as a white solid (36.5 mg, 54.07%, racemate). ESI/MS (m/z) 316.1 [M+H]⁺.¹H NMR (500 MHz, DMSO-*d*₆) δ 11.99 (s, 1H), 7.65 (dd, *J =* 8.3, 1.0 Hz, 1H), 7.58 (dd, *J=* 7.3, 1.0 Hz, 1H), 7.37 (dd, *J =* 8.3, 7.3 Hz, 1H), 4.43 (d, *J =* 4.7 Hz, 1H), 4.36-4.28 (m, 2H), 3.69-3.61 (m, 1H), 3.08 (t, *J=* 6.0 Hz, 2H), 2.57 (dd, *J =* 7.3, 5.6 Hz, 2H), 2.11 (p, *J =* 6.2 Hz, 2H), 1.88-1.79 (m, 1H), 1.79-1.69 (m, 1H), 1.47-1.41 (m, 2H), 1.07 (d, *J =* 6.1 Hz, 3H).

### Example 203: Synthesis of Compound B145

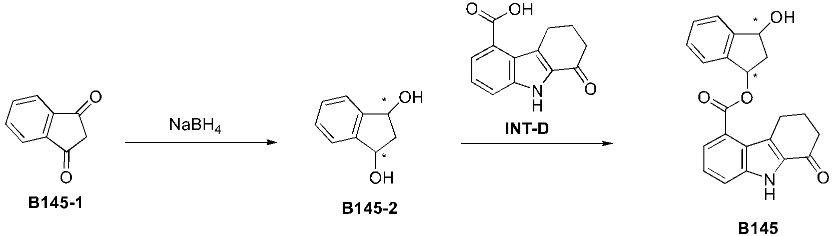

At room temperature, sodium borohydride (388 mg, 10.263 mmol) was added to a solution of compound B145-1 (500 mg, 3.421 mmol) in dichloromethane (10 mL) and water (10 mL). The mixture was stirred for 8 hours and then filtered. The filter cake was washed with dichloromethane (15 mL) and dried under vacuum to afford compound B145-2 as a yellow solid (300 mg, 55.47%).

At room temperature, N,N'-dicyclohexylcarbodiimide (133 mg, 0.645 mmol) and 4-dimethylaminopyridine (53 mg, 0.430 mmol) were added to a mixture of compound INT-D (100 mg, 0.430 mmol) and compound B145-2 (68 mg, 0.430 mmol) in dichloromethane (3 mL) and N,N-dimethylformamide (3 mL). The reaction mixture was stirred at room temperature for 4 hours and then concentrated under vacuum. The residue was purified by reverse-phase chromatography (mobile phase A: water (0.1% formic acid); mobile phase B: acetonitrile) to afford compound B145 as a white solid (10.6 mg, 6.73%, mixture of cis-trans isomers). ESI/MS (m/z): 362.2 [M+H]⁺.¹H NMR (400 MHz, DMSO-*d*₆) δ 12.02 (s, 1H), 7.71-7.60 (m, 2H), 7.47 (d, *J =* 7.4 Hz, 1H), 7.45-7.39 (m, 2H), 7.41-7.31 (m, 2H), 6.35-6.18 (m, 1H), 5.60 (d, *J* = 5.9 Hz, 1H), 5.15-4.96 (m, 1H), 3.20-3.10 (m, 2H), 3.10-3.03 (m, 1H), 2.61-2.53 (m, 2H), 2.19-2.02 (m, 2H), 2.01-1.90 (m, 1H).

### Example 204: Synthesis of Compound B146

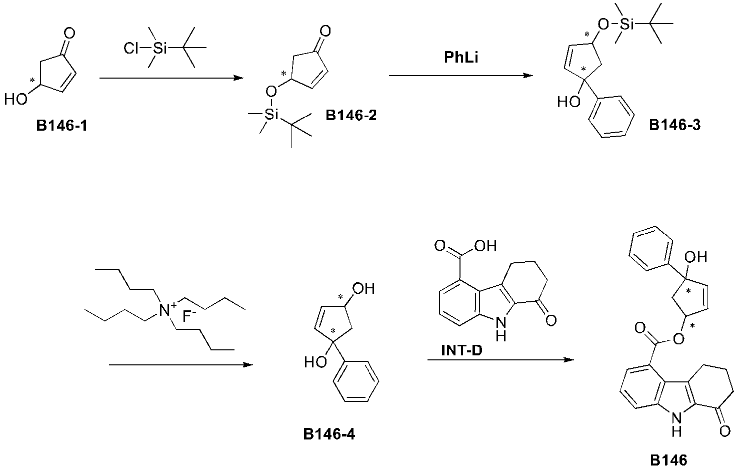

At 0 °C, triethylamine (6.19 g, 61.16 mmol) was added to a solution of compound B146-1 (2 g, 20.39 mmol) and 4-dimethylaminopyridine (1.25 g, 10.19 mmol) in dichloromethane (40 mL). The reaction mixture was stirred at 0 °C for 10 minutes, and then tert-butyldimethylsilyl chloride (4.61 g, 30.58 mmol) was added. The mixture was stirred at room temperature for an additional 3 hours and then concentrated under vacuum. The residue was purified by silica gel column chromatography (petroleum ether:ethyl acetate = 10:1) to afford compound B146-2 as a white solid (1.97 g, 19.28%). ESI/MS (m/z): 213.1 [M+H]⁺.

At -70 °C under a nitrogen atmosphere, a 1.9 M solution of phenyllithium in dibutyl ether (2.97 mL, 5.65 mmol) was slowly added to a solution of copper(I) iodide (538 mg, 2.83 mmol) in tetrahydrofuran (10 mL). The mixture was stirred for 1 hour, and then compound B146-2 (300 mg, 1.41 mmol) was added. The reaction was stirred at 0 °C for 3 hours, then quenched with saturated ammonium chloride aqueous solution (20 mL) and extracted with ethyl acetate (3 × 20 mL). The combined organic phases were washed with saturated sodium chloride aqueous solution (30 mL), dried over anhydrous sodium sulfate, and concentrated under vacuum. The residue was purified by silica gel column chromatography (petroleum ether:ethyl acetate = 9:1) to afford compound B146-3 as a light yellow oil (295 mg, 71.89%). ESI/MS (m/z): 273.1 [M+H-H₂O]⁺.

At 0 °C, tetrabutylammonium fluoride (1.22 g, 4.65 mmol) was added to a solution of compound B146-3 (270 mg, 0.93 mmol) in tetrahydrofuran (10 mL). The reaction mixture was stirred at room temperature for 3 hours, then quenched with water (10 mL). The mixture was extracted with ethyl acetate (3 × 20 mL), and the combined organic phases were washed with saturated sodium chloride aqueous solution (60 mL), dried over anhydrous sodium sulfate, and concentrated under vacuum. The residue was purified by silica gel column chromatography (petroleum ether:ethyl acetate = 1:19) to afford compound B146-4 as a white solid (110 mg, 54.40%). ESI/MS (m/z): 159.1 [M+H-H₂O]⁺.

At room temperature, N,N'-dicyclohexylcarbodiimide (142 mg, 0.69 mmol) and 4-dimethylaminopyridine (67 mg, 0.55 mmol) were added to a mixture of compound INT-D (107 mg, 0.46 mmol) in dichloromethane (3 mL) and N,N-dimethylformamide (3 mL), followed by the addition of compound B146-4 (100 mg, 0.46 mmol). The reaction mixture was stirred at room temperature for 4 hours, then quenched with water (20 mL) and extracted with ethyl acetate (3 × 20 mL). The combined organic phases were washed with saturated sodium chloride aqueous solution (30 mL), dried over anhydrous sodium sulfate, and concentrated under vacuum. The residue was purified by reverse-phase chromatography (mobile phase A: water (10 mmol/L ammonium bicarbonate); mobile phase B: acetonitrile) to afford compound B146 as a white solid (34.1 mg, 18.96%, mixture of cis-trans isomers). ESI/MS (m/z): 388.2 [M+H]⁺.¹H NMR (400 MHz, DMSO-*d*₆) δ 12.01 (s, 1H), 7.67 (dd, *J =* 8.3, 1.0 Hz, 1H), 7.63 (dd, *J =* 7.4, 1.0 Hz, 1H), 7.41 (d, *J =* 1.3 Hz, 1H), 7.39-7.32 (m, 4H), 7.27-7.21 (m, 1H), 6.16 (dd, *J =* 5.5, 1.9 Hz, 1H), 6.12 (dd, *J =* 5.4, 1.2 Hz, 1H), 5.99-5.93 (m, 1H), 5.81 (s, 1H), 3.16-3.08 (m, 2H), 2.82 (dd, *J =* 14.1, 7.4 Hz, 1H), 2.62-2.54 (m, 2H), 2.27 (dd, *J =* 14.0, 4.7 Hz, 1H), 2.16-2.06 (m, 2H).

### Example 205: Synthesis of Compound B147

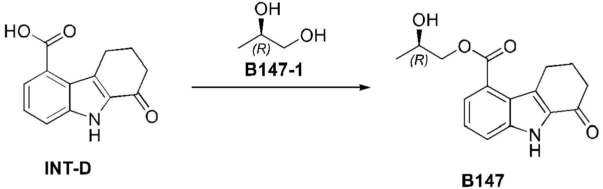

At room temperature, to a mixture of compound INT-D (90.0 mg, 0.373 mmol) in dichloromethane (2 mL) were added compound B147-1 (56.8 mg, 0.746 mmol), 4-dimethylaminopyridine (22.8 mg, 0.186 mmol), and N,N'-dicyclohexylcarbodiimide (153.9 mg, 0.746 mmol). The reaction mixture was heated to 45°C and stirred for 2 hours. After cooling to room temperature, the mixture was diluted with dichloromethane (100 mL). The resulting organic phase was washed sequentially with water (30 mL) and saturated sodium chloride solution (30 mL), dried over anhydrous sodium sulfate, and concentrated under vacuum. The residue was purified by normal-phase silica gel column chromatography (methanol:dichloromethane = 1:30) to give a crude white solid. Further purification by reverse-phase column chromatography (acetonitrile:water = 10%-50%) afforded compound B147 (51.4 mg, 47.13% yield) as a white solid. ESI/MS (m/z): 288.0 [M+H]⁺.¹H NMR (500 MHz, DMSO-*d*₆) δ 11.98 (s, 1H), 7.66 (d, *J =* 3.1 Hz, 1H), 7.64 (d, *J =* 2.0 Hz, 1H), 7.37 (t, *J* = 7.7 Hz, 1H), 4.94 (d, *J =* 5.0 Hz, 1H), 4.17 (d, *J* = 3.2 Hz, 1H), 4.16 (d, *J =* 1.7 Hz, 1H), 4.03-3.94 (m, 1H), 3.10 (t, *J =* 6.0 Hz, 2H), 2.57 (dd, *J* = 7.3, 5.5 Hz, 2H), 2.10 (p, *J =* 6.1 Hz, 2H), 1.15 (d, *J =* 6.4 Hz, 3H).

### Example 206: Synthesis of Compound B148

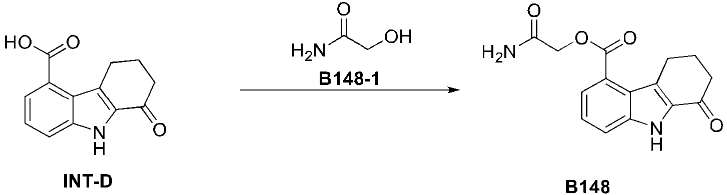

At room temperature, to a mixture of compound INT-D (70.0 mg, 0.290 mmol) in dichloromethane (2 mL) were added compound B148-1 (28.3 mg, 0.377 mmol), N,N-diisopropylethylamine (112.5 mg, 0.870 mmol), and N,N,N',N'-tetramethyl-O-(7-azabenzotriazol-1-yl)uronium hexafluorophosphate (165.5 mg, 0.435 mmol). The reaction mixture was heated to 45°C and stirred for 48 hours. After cooling to room temperature, the mixture was filtered by suction. The resulting filter cake was triturated with acetonitrile (2 mL) at 45°C for 24 hours and then filtered by suction. The filter cake was dried under vacuum to afford compound B148 (27.9 mg, 31.80% yield) as a white solid. ESI/MS (m/z): 287.0 [M+H]⁺.¹H NMR (500 MHz, DMSO-*d*₆) δ 12.00 (s, 1H), 7.72 (d, *J =* 7.2 Hz, 1H), 7.68 (d, *J =* 8.2 Hz, 1H), 7.57 (s, 1H), 7.39 (t, *J* = 7.8 Hz, 1H), 7.26 (s, 1H), 4.71 (s, 2H), 3.10 (t, *J =* 6.1 Hz, 2H), 2.59-2.54 (m, 2H), 2.09 (p, *J =* 6.2 Hz, 2H).

### Example 207: Synthesis of Compound B149

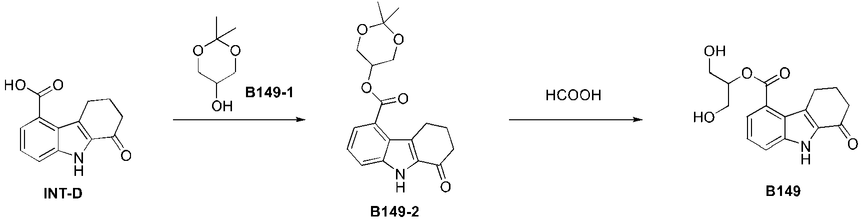

At room temperature, to a mixture of compound INT-D (50.0 mg, 0.207 mmol) in dichloromethane (1.5 mL) were added compound B149-1 (54.8 mg, 0.414 mmol) and 4-dimethylaminopyridine (12.7 mg, 0.104 mmol). After stirring uniformly, N,N'-dicyclohexylcarbodiimide (85.5 mg, 0.414 mmol) was added, and the mixture was stirred at 45 °C for 5 hours. After cooling to room temperature, the reaction mixture was diluted with dichloromethane (100 mL). The organic phase was washed with water (30 mL) and saturated sodium chloride solution (30 mL), dried over anhydrous sodium sulfate, and concentrated under vacuum. The residue was purified by normal-phase silica gel column chromatography (methanol:dichloromethane = 1:30) to afford crude compound B149-2 as a white solid. ESI/MS (m/z): 344.1 [M+H]⁺.

At room temperature, to a solution of compound B149-2 in acetonitrile (8 mL) were added formic acid (28.6 mg, 0.621 mmol) and water (2 mL). The mixture was heated to 45 °C and stirred for 6 hours. After cooling to room temperature, the reaction mixture was concentrated under vacuum. The residue was purified by reverse-phase column chromatography (acetonitrile:water = 10% to 50%) to afford compound B149 (20.0 mg, 0.062 mmol) as a white solid. ESI/MS (m/z): 304.1 [M+H]⁺.

### Example 208: Synthesis of Compound B150

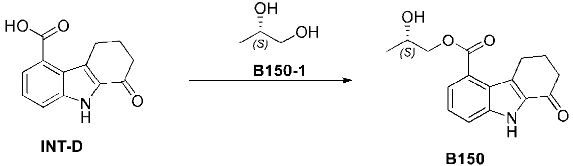

At room temperature, to a mixture of compound INT-D (90.0 mg, 0.373 mmol) in dichloromethane (2 mL) were added compound B150-1 (56.8 mg, 0.746 mmol), 4-dimethylaminopyridine (22.8 mg, 0.186 mmol), and N,N'-dicyclohexylcarbodiimide (153.9 mg, 0.746 mmol). The reaction mixture was heated to 45 °C and stirred for 2 hours. After cooling to room temperature, the mixture was diluted with dichloromethane (100 mL). The resulting organic phase was washed sequentially with water (30 mL) and saturated sodium chloride solution (30 mL), dried over anhydrous sodium sulfate, and concentrated under vacuum. The residue was purified by normal-phase silica gel column chromatography (methanol:dichloromethane = 1:30) to give a crude white solid. Further purification by reverse-phase column chromatography (acetonitrile:water = 10% to 50%) afforded compound B150 (54.7 mg, 56.55% yield) as a white solid. ESI/MS (m/z): 288.0 [M+H]⁺.¹H NMR (500 MHz, DMSO-*d*₆) δ 11.98 (s, 1H), 7.67-7.65 (m, 1H), 7.65-7.63 (m, 1H), 7.37 (dd, *J* = 8.2, 7.4 Hz, 1H), 4.93 (d, *J* = 5.0 Hz, 1H), 4.18-4.15 (m, 2H), 4.02-3.94 (m, 1H), 3.10 (t, *J =* 6.0 Hz, 2H), 2.57 (dd, *J =* 7.3, 5.5 Hz, 2H), 2.13-2.08 (m, 2H), 1.15 (d, *J =* 6.3 Hz, 3H).

### Example 209: Synthesis of Compound B151

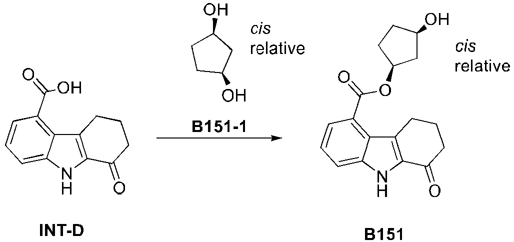

At room temperature, to a mixture of compound INT-D (50 mg, 0.21 mmol), compound B151-1 (42.3 mg, 0.41 mmol), and dichloromethane (1 mL) were added 4-dimethylaminopyridine (12.7 mg, 0.10 mmol) and 1-(3-dimethylaminopropyl)-3-ethylcarbodiimide hydrochloride (79.4 mg, 0.41 mmol). The reaction mixture was stirred for 4 hours, then diluted with dichloromethane (30 mL). The resulting organic phase was washed sequentially with 0.5 M hydrochloric acid solution (10 mL) and saturated sodium chloride solution (10 mL), dried over anhydrous sodium sulfate, and concentrated under vacuum. The residue was purified by reverse-phase preparative chromatography (acetonitrile/water = 10-45%) to afford compound B151 (28.0 mg, 40.97%, cis isomer) as a white solid. ESI/MS (m/z): 314.0 [M+H]⁺.¹H NMR (500 MHz, DMSO-*d*₆) δ 11.97 (s, 1H), 7.64 (dd, *J =* 8.3, 1.0 Hz, 1H), 7.58 (dd, *J =* 7.3, 1.1 Hz, 1H), 7.36 (t, *J =* 7.8 Hz, 1H), 5.27-5.22 (m, 1H), 4.68 (d, *J =* 3.8 Hz, 1H), 4.18-4.13 (m, 1H), 3.10 (t, *J =* 6.0 Hz, 2H), 2.58-2.56 (m, 2H), 2.36-2.30 (m, 1H), 2.13-2.08 (m, 2H), 2.05-1.98 (m, 1H), 1.96-1.90 (m, 1H), 1.81-1.74 (m, 1H), 1.71-1.64 (m, 2H).

### Example 210: Synthesis of Compound B152

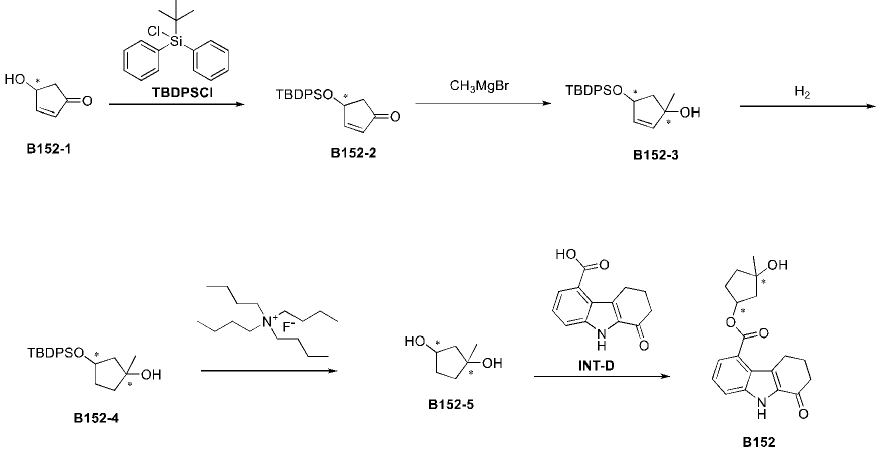

At room temperature, to a solution of compound B152-1 (2 g, 20.4 mmol) in dichloromethane (30 mL) were added triethylamine (3.09 g, 30.6 mmol), 4-dimethylaminopyridine (124 mg, 1.02 mmol), and tert-butyldiphenylsilyl chloride (6.16 g, 22.43 mmol). The reaction mixture was stirred at room temperature for 3 hours, then diluted with dichloromethane (90 mL). The resulting organic phase was washed with saturated sodium chloride solution (30 mL × 3), dried over anhydrous sodium sulfate, and concentrated under vacuum. The residue was purified by silica gel column chromatography (petroleum ether:ethyl acetate = 6:1) to afford compound B152-2 (5.30 g, 12.340 mmol) as a white solid. ESI/MS (m/z): 337.1 [M+H]⁺.

At -20 °C, to a solution of compound B152-2 (3.90 g, 9.08 mmol) in tetrahydrofuran (80 mL) was added dropwise 1 M methylmagnesium bromide in hexane (9.1 mL, 9.1 mmol). The mixture was stirred for 1 hour, then slowly warmed to 10 °C and stirred for an additional 4 hours. The reaction was quenched with saturated ammonium chloride solution (300 mL), and the mixture was extracted with ethyl acetate (150 mL × 2). The combined organic phase was dried over anhydrous sodium sulfate and concentrated under vacuum to give crude compound B152-3 (2.10 g, 5.55 mmol). ESI/MS (m/z): 375.1 [M+Na]⁺.

Under a nitrogen atmosphere, to a mixture of compound B152-3 (1.50 g, 3.86 mmol) and 10% palladium on carbon (100 mg) was slowly added methanol (15 mL). The system was purged with hydrogen three times. The reaction mixture was stirred at 25 °C for 5 hours and then filtered. The filtrate was concentrated under vacuum to afford compound B152-4 (1.44 g, 2.17 mmol) as a colorless oil. ESI/MS (m/z): 377.1 [M+Na]⁺.

Under nitrogen protection, to a solution of compound B152-4 (1.44 g, 2.17 mmol) in tetrahydrofuran (5 mL) was added tetrabutylammonium fluoride (1.66 g, 6.37 mmol). The mixture was stirred at room temperature for 5 hours, then concentrated under vacuum. The residue was purified by silica gel column chromatography (ethyl acetate 100%) to give compound B152-5 (260 mg, 2.24 mmol) as a pale yellow liquid. ¹H NMR (500 MHz, DMSO-d₆) δ 4.49 (d, J = 5.0 Hz, 1H), 4.31 (s, 1H), 4.06-4.02 (m, 1H), 1.82-1.68 (m, 3H), 1.66-1.58 (m, 1H), 1.57-1.53 (m, 1H), 1.44-1.39 (m, 1H), 1.15 (s, 3H).

At room temperature, to a mixture of compound B152-5 (72 mg, 0.62 mmol) and dichloromethane (3 mL) were added compound INT-D (100 mg, 0.41 mmol), 1-(3-dimethylaminopropyl)-3-ethylcarbodiimide hydrochloride (159 mg, 0.83 mmol), and 4-dimethylaminopyridine (152 mg, 1.24 mmol). The reaction mixture was stirred at room temperature for 15 hours, then diluted with ethyl acetate (30 mL) and washed with water (10 mL × 3). The organic phase was dried over anhydrous sodium sulfate and concentrated under vacuum. The residue was purified by silica gel column chromatography (petroleum ether:ethyl acetate = 1:1) to afford compound B152 (19 mg, 0.057 mmol, mixture of cis/trans isomers) as a white solid. ESI/MS (m/z): 328.0 [M+H]⁺.¹H NMR (500 MHz, DMSO-*d*₆) δ 11.97 (s, 1H), 7.64 (dd, *J =* 8.2, 1.0 Hz, 1H), 7.58 (dd, *J =* 7.3, 1.0 Hz, 1H), 7.36 (dd, *J* = 8.3, 7.3 Hz, 1H), 5.33-5.24 (m, 1H), 4.48 (s, 1H), 3.14-3.05 (m, 2H), 2.61-2.53 (m, 2H), 2.19-2.06 (m, 4H), 2.03-1.96 (m, 1H), 1.90-1.77 (m, 2H), 1.57-1.51 (m, 8.2 Hz, 1H), 1.26 (s, 3H).

### Example 211: Synthesis of Compound B153

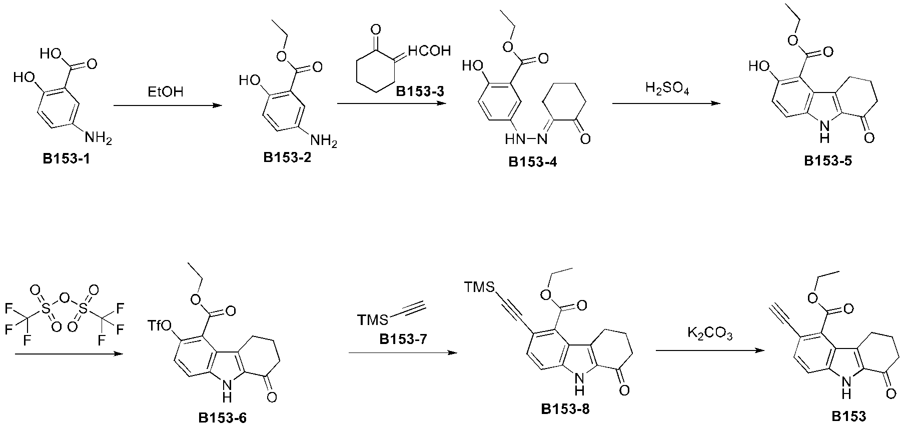

At 0 °C, to a solution of compound B153-1 (5 g, 32.651 mmol) in ethanol (200 mL) was added concentrated sulfuric acid (10 mL). The reaction mixture was heated to 80 °C and stirred for 24 hours. After cooling to room temperature, the mixture was concentrated under vacuum and neutralized to pH = 8 with saturated sodium carbonate solution. The mixture was extracted with ethyl acetate (3 × 200 mL). The combined organic phase was washed with saturated sodium chloride solution (100 mL), dried over anhydrous sodium sulfate, and concentrated under vacuum to afford compound B153-2 (3.5 g, 56.80% yield) as a brown solid. ESI/MS (m/z): 182.1 [M+H]⁺.

At 0 °C, to a solution of compound B153-2 (1.8 g, 9.537 mmol) in water (30 mL) and concentrated hydrochloric acid (4.14 mL, 47.685 mmol) was added a solution of sodium nitrite (0.99 g, 14.306 mmol) in water (12 mL). The mixture was stirred for 10 minutes. At 0 °C, a solution of compound B153-3 (1.50 g, 11.444 mmol) in methanol (72 mL) and a solution of sodium acetate (1.72 g, 20.981 mmol) in water (30 mL) were added dropwise to the above system. The reaction was allowed to warm to room temperature and stirred for an additional 2 hours. The mixture was filtered, and the filter cake was washed with water (40 mL) and dried under vacuum to give compound B153-4 (2 g, 63.57% yield) as a red solid. ESI/MS (m/z): 290.9 [M+H]⁺.

At 0 °C, to a solution of compound B153-4 (1.8 g, 5.456 mmol) in acetonitrile (50 mL) was added 10% dilute sulfuric acid (10 mL). The reaction mixture was heated to 75 °C and stirred for 6 hours. After cooling to room temperature, the mixture was neutralized to pH = 7 with saturated sodium carbonate solution. The mixture was extracted with ethyl acetate (3 × 100 mL). The combined organic phase was washed with saturated sodium chloride solution (100 mL), dried over anhydrous sodium sulfate, and concentrated under vacuum. The residue was purified by silica gel column chromatography (petroleum ether: ethyl acetate = 1:4) to afford compound B153-5 (400 mg, 24.14% yield) as a brown solid. ESI/MS (m/z): 274.1 [M+H]⁺.

At 0 °C, to a solution of compound B153-5 (300 mg, 0.988 mmol) and pyridine (390.7 mg, 4.940 mmol) in dichloromethane (10 mL) was added trifluoromethanesulfonic anhydride (362.4 mg, 1.284 mmol). The reaction mixture was stirred at room temperature for 3 hours and then concentrated under vacuum. The residue was purified by silica gel column chromatography (petroleum ether: ethyl acetate = 1:1) to afford compound B153-6 (200 mg, 46.45% yield) as a yellow solid. ESI/MS (m/z): 406.1 [M+H]⁺.

At room temperature, to a solution of compound B153-6 (180 mg, 0.333 mmol) and bis(triphenylphosphine)palladium(II) chloride (6.41 mg, 0.006 mmol) in N,N-dimethylformamide (5 mL) were added triethylamine (101.1 mg, 0.999 mmol) and compound B153-7 (327.1 mg, 3.330 mmol). The reaction mixture was stirred at 80 °C overnight. After cooling to room temperature, the reaction was quenched with water (30 mL). The mixture was extracted with dichloromethane (3 × 30 mL). The combined organic phase was washed with saturated sodium chloride solution (30 mL), dried over anhydrous sodium sulfate, and concentrated under vacuum. The residue was purified by silica gel column chromatography (petroleum ether:ethyl acetate = 1:1) to afford compound B153-8 (100 mg, 84.94% yield) as a yellow oil. ESI/MS (m/z): 353.9 [M+H]⁺.

At room temperature, to a solution of compound B153-8 (90 mg, 0.249 mmol) in methanol (5 mL) was added potassium carbonate (68.9 mg, 0.498 mmol). The reaction mixture was stirred at room temperature for 0.5 hour and then concentrated under vacuum. The residue was purified by reverse-phase chromatography (mobile phase A: water (0.1% formic acid), mobile phase B: methanol) to afford compound B153 (25.4 mg, 36.22% yield) as a pale yellow solid. ESI/MS (m/z): 282.2 [M+H]⁺.

¹H NMR (400 MHz, DMSO-*d*₆) δ 12.13 (s, 1H), 7.50 (d, *J =* 8.6 Hz, 1H), 7.42 (d, *J =* 8.6 Hz, 1H), 4.41 (q, *J =* 7.1 Hz, 2H), 4.20 (s, 1H), 2.79 (t, *J =* 6.0 Hz, 2H), 2.62-2.54 (m, 2H), 2.18-2.08 (m, 2H), 1.36 (t, *J =* 7.1 Hz, 3H).

### Example 212: Synthesis of Compound B154

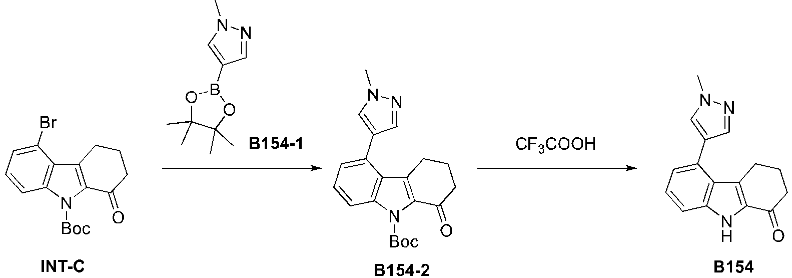

Under a nitrogen atmosphere, to a solution of compound INT-C (100 mg, 0.27 mmol) in a mixture of 1,4-dioxane (2 mL) and water (0.5 mL) were sequentially added compound B154-1 (86 mg, 0.41 mmol), [1,1'-bis(diphenylphosphino)ferrocene]palladium(II) dichloride dichloromethane complex (11 mg, 0.014 mmol), and potassium carbonate (76 mg, 0.55 mmol). The reaction mixture was heated to 80 °C and stirred for 3 hours. After cooling to room temperature, the mixture was diluted with ethyl acetate (30 mL), filtered through a pad of Celite, and the filtrate was concentrated under vacuum to afford crude compound B154-2.

To compound B154-2 were added trifluoroacetic acid (0.3 mL) and dichloromethane (3 mL). The mixture was stirred at room temperature for 2 hours, then diluted with ethyl acetate (30 mL). The organic phase was washed sequentially with saturated sodium bicarbonate solution (10 mL) and saturated sodium chloride solution (10 mL), dried over anhydrous sodium sulfate, and concentrated under vacuum. The residue was purified by reverse-phase silica gel column chromatography (acetonitrile/water: 10-50%) to afford compound B154 (18 mg, 0.063 mmol) as a pale yellow solid. ESI/MS (m/z): 266.0 [M+H]⁺.¹H NMR (500 MHz, DMSO-*d*₆) δ 11.69 (s, 1H), 7.90 (s, 1H), 7.59 (s, 1H), 7.34 (dd, *J =* 8.3, 1.0 Hz, 1H), 7.27 (dd, *J =* 8.3, 7.1 Hz, 1H), 6.91 (dd, *J =* 7.1, 1.0 Hz, 1H), 3.92 (s, 3H), 2.74 (t, *J =* 6.0 Hz, 2H), 2.53-2.51 (m, 2H), 2.01 (p, *J =* 6.1 Hz, 2H).

### Example 213: Synthesis of Compound B155

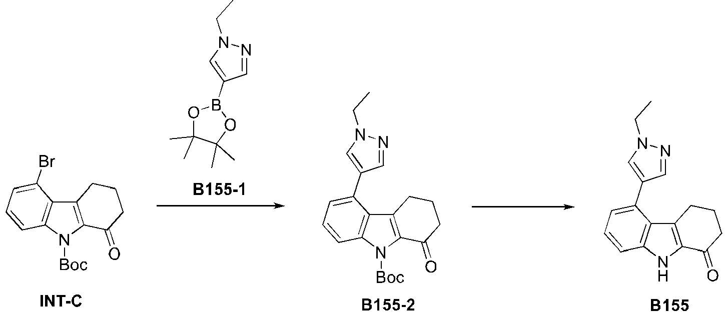

Under nitrogen protection, to a mixture of compound INT-C (100 mg, 0.27 mmol) in 1,4-dioxane (2 mL) and water (0.5 mL) were sequentially added compound B155-1 (92 mg, 0.41 mmol), [1,1'-bis(diphenylphosphino)ferrocene]palladium(II) dichloride dichloromethane complex (11 mg, 0.014 mmol), and potassium carbonate (76 mg, 0.55 mmol). The reaction mixture was heated to 80 °C and stirred for 3 hours. After cooling to room temperature, the mixture was diluted with ethyl acetate (30 mL), filtered through celite, and the filtrate was concentrated under vacuum to obtain crude compound B155-2.

The crude compound B155-2 was then treated with a mixture of trifluoroacetic acid (0.3 mL) and dichloromethane (3 mL), and stirred at room temperature for 2 hours. The reaction mixture was diluted with ethyl acetate (30 mL), sequentially washed with saturated sodium bicarbonate solution (10 mL) and saturated sodium chloride solution (10 mL), dried over anhydrous sodium sulfate, and concentrated under vacuum. The residue was purified by reverse-phase preparative liquid chromatography (acetonitrile/water: 10-50%) to afford compound B155 (13 mg, 0.048 mmol) as a pale yellow solid. ESI/MS (m/z): 280.1 [M+H]⁺.¹H NMR (500 MHz, DMSO-*d*₆) δ 11.69 (s, 1H), 7.94 (s, 1H), 7.60 (s, 1H), 7.34 (d, *J =* 8.2 Hz, 1H), 7.27 (t, *J =* 7.7 Hz, 1H), 6.92 (d, *J* = 7.0 Hz, 1H), 4.21 (q, *J* = 7.2 Hz, 2H), 2.73 (t, *J* = 5.9 Hz, 2H), 2.53-2.51 (m, 2H), 2.01 (p, *J =* 6.0 Hz, 2H), 1.44 (t, *J =* 7.2 Hz, 3H).

### Example 214: Synthesis of Compound B156

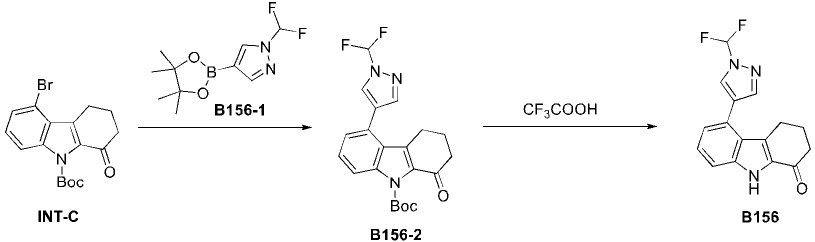

Under a nitrogen atmosphere, to a mixture of compound INT-C (100 mg, 0.27 mmol) in 1,4-dioxane (2 mL) and water (0.5 mL) were sequentially added compound B156-1 (101 mg, 0.41 mmol), [1,1'-bis(diphenylphosphino)ferrocene]palladium(II) dichloride dichloromethane complex (11 mg, 0.014 mmol), and potassium carbonate (76 mg, 0.55 mmol). The reaction mixture was heated to 80 °C and stirred for 3 hours. After cooling to room temperature, the mixture was diluted with ethyl acetate (30 mL), filtered through Celite, and the filtrate was concentrated under vacuum to afford crude compound B156-2.

To the crude compound B156-2 was added a mixture of trifluoroacetic acid (0.3 mL) and dichloromethane (3 mL). The reaction mixture was stirred at room temperature for 2 hours, then diluted with ethyl acetate (30 mL). The organic phase was washed sequentially with saturated sodium bicarbonate solution (10 mL) and saturated sodium chloride solution (10 mL), dried over anhydrous sodium sulfate, and concentrated under vacuum. The residue was purified by reverse-phase silica gel column chromatography (acetonitrile/water: 10-50%) to afford compound B156 (23 mg, 0.074 mmol) as a pale yellow solid. ESI/MS (m/z): 302.0 [M+H]⁺.¹H NMR (500 MHz, DMSO-*d*₆) δ 11.79 (s, 1H), 8.45 (s, 1H), 8.00 (s, 1H), 7.89 (t, *J* = 59.2 Hz, 1H), 7.42 (dd, *J =* 8.3, 0.9 Hz, 1H), 7.32 (dd, *J =* 8.4, 7.1 Hz, 1H), 7.00 (dd, *J =* 7.1, 0.9 Hz, 1H), 2.68 (t, *J =* 6.0 Hz, 2H), 2.52 (dd, *J =* 6.7, 4.9 Hz, 2H), 2.02 (p, *J=* 6.2 Hz, 2H).

### Example 215: Synthesis of Compound B157

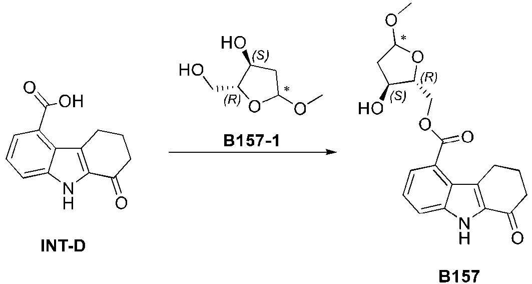

At room temperature, to a mixture of compound INT-D (50.0 mg, 0.21 mmol) and compound B157-1 (64.1 mg, 0.41 mmol) in dichloromethane (1 mL) were added 4-dimethylaminopyridine (12.7 mg, 0.10 mmol) and 1-(3-dimethylaminopropyl)-3-ethylcarbodiimide hydrochloride (79.4 mg, 0.41 mmol). The reaction mixture was stirred for 4 hours, then diluted with dichloromethane (30 mL). The resulting organic phase was washed sequentially with 0.5 M hydrochloric acid solution (10 mL) and saturated sodium chloride solution (10 mL), dried over anhydrous sodium sulfate, and concentrated under vacuum. The residue was purified by preparative thin-layer chromatography (petroleum ether: ethyl acetate = 1:1) to afford compound B157 (25.0 mg, 28.2%, racemate) as a white solid. ESI/MS (m/z): 360.0 [M+H]⁺.

### Example 216: Synthesis of Compound B158

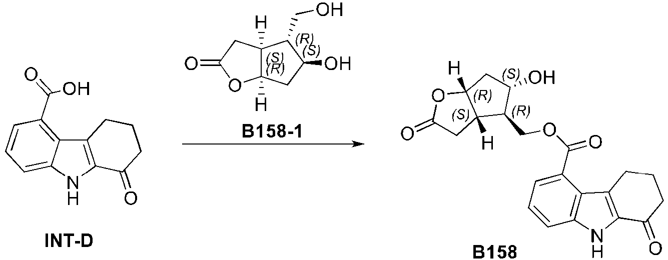

At room temperature, to a mixture of compound INT-D (60.0 mg, 0.25 mmol) and compound B158-1 (64.2 mg, 0.37 mmol) in dichloromethane (1 mL) were added 4-dimethylaminopyridine (15.2 mg, 0.12 mmol) and N,N'-diisopropylcarbodiimide (62.7 mg, 0.50 mmol). The reaction mixture was stirred for 6 hours, then diluted with dichloromethane (30 mL). The resulting organic phase was washed sequentially with 0.5 M hydrochloric acid solution (10 mL) and saturated sodium chloride solution (10 mL), dried over anhydrous sodium sulfate, and concentrated under vacuum. The residue was purified by preparative thin-layer chromatography (dichloromethane: methanol = 20:1) to afford compound B158 (38.5 mg, 39.2% yield) as a white solid. ESI/MS (m/z): 384.1 [M+H]⁺. ¹H NMR (500 MHz, DMSO-*d*₆) δ 12.01 (s, 1H), 7.67 (dd, *J =* 8.3, 1.0 Hz, 1H), 7.62 (dd, *J =* 7.3, 1.0 Hz, 1H), 7.40-7.37 (m, 1H), 5.02 (d, *J =* 4.4 Hz, 1H), 4.93 (td, *J* = 7.1, 2.8 Hz, 1H), 4.37 (dd, *J=* 11.2, 5.8 Hz, 1H), 4.23 (dd, *J* = 11.2, 6.8 Hz, 1H), 4.05-3.98 (m, 1H), 3.10 (t, *J =* 6.1 Hz, 2H), 2.85 (dd, *J =* 17.9, 10.3 Hz, 1H), 2.77-2.69 (m, 1H), 2.59-2.56 (m, 2H), 2.44 (dd, *J =* 17.9, 2.5 Hz, 1H), 2.35-2.30 (m, 1H), 2.15-2.08 (m, 3H), 1.79 (ddd, *J =* 14.4, 5.8, 2.8 Hz, 1H).

### Example 217: Synthesis of Compound B159

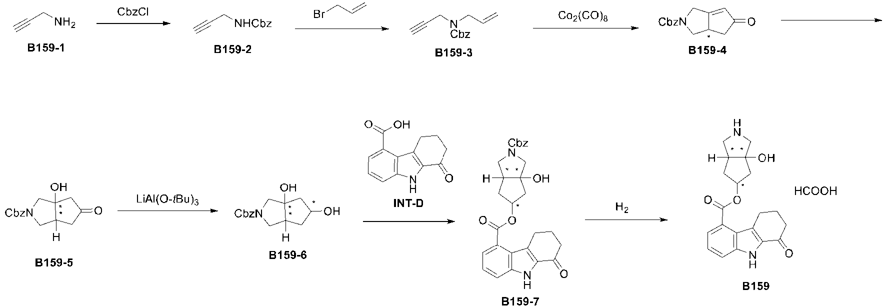

At 0 °C, to a solution of compound B159-1 (4 g, 72.62 mmol) and triethylamine (22.05 g, 217.87 mmol) in dichloromethane (60 mL) was added benzyl chloroformate (24.93 g, 72.61 mmol). The reaction mixture was stirred at room temperature overnight. Water (100 mL) was added, and the mixture was extracted with dichloromethane (3 × 100 mL). The combined organic phase was washed with saturated sodium chloride solution (200 mL), dried over anhydrous sodium sulfate, and concentrated under vacuum. The residue was purified by silica gel column chromatography (petroleum ether: ethyl acetate = 4:1) to afford compound B159-2 (3.5 g, 17.58% yield) as a colorless oil. ESI/MS (m/z): 190.0 [M+H]⁺.

At 0 °C, to a solution of compound B159-2 (3.5 g, 18.50 mmol) and allyl bromide (2.69 g, 22.20 mmol) in tetrahydrofuran (100 mL) was added 60% sodium hydride (888 mg, 22.20 mmol). The reaction was warmed to room temperature and stirred for 4 hours. The mixture was cooled to 0 °C, quenched with water (50 mL), and extracted with ethyl acetate (3 × 60 mL). The combined organic phase was washed with saturated sodium chloride solution (50 mL), dried over anhydrous sodium sulfate, and concentrated under vacuum. The residue was purified by silica gel column chromatography (petroleum ether: ethyl acetate = 9:1) to afford compound B159-3 (3.2 g, 69.42% yield) as a colorless oil. ESI/MS (m/z): 230.3 [M+H]⁺.

Under a nitrogen atmosphere at 0 °C, to a solution of compound B159-3 (3.2 g, 13.96 mmol) and tetramethylthiourea (923 mg, 6.98 mmol) in toluene (70 mL) was added dicobalt octacarbonyl (1.19 g, 3.49 mmol). The reaction mixture was stirred at 80 °C under a carbon monoxide atmosphere (1 atm) for 3 hours. After cooling to room temperature, the mixture was filtered. The filter cake was washed with toluene (70 mL), and the combined filtrate was concentrated under vacuum. The residue was purified by silica gel column chromatography (petroleum ether: ethyl acetate = 1:1) to afford compound B159-4 (1.2 g, 32.75% yield) as a pale yellow solid. ESI/MS (m/z): 258.3 [M+H]⁺.

Under a nitrogen atmosphere, to a mixture of copper(I) chloride (45 mg, 0.46 mmol), sodium tert-butoxide (44 mg, 0.46 mmol), S-(-)-1,1'-binaphthyl-2,2'-diylbis(diphenylphosphine) (285 mg, 0.46 mmol), and bis(pinacolato)diboron (1.51 g, 5.94 mmol) in 2-methyltetrahydrofuran (50 mL) was added compound B159-4 (1.2 g, 4.57 mmol). The mixture was stirred at room temperature for 2 hours. Methanol (293 mg, 9.14 mmol) was added, and stirring was continued for 0.5 hours. The mixture was cooled to 0 °C, and sodium hydroxide (183 mg, 4.57 mmol) and hydrogen peroxide (313 mg, 9.14 mmol) were added. The reaction was warmed to room temperature and stirred for 1 hour. The mixture was cooled to 0 °C, quenched with saturated sodium thiosulfate solution (10 mL), and extracted with ethyl acetate (3 × 60 mL). The combined organic phase was washed with saturated sodium chloride solution (80 mL), dried over anhydrous sodium sulfate, and concentrated under vacuum. The residue was purified by silica gel column chromatography (petroleum ether: ethyl acetate = 10: 1) to afford compound B159-5 (720 mg, 56.07% yield) as a pale yellow oil. ESI/MS (m/z): 276.1 [M+H]⁺.

At 0 °C, to a solution of compound B159-5 (720 mg, 2.62 mmol) in tetrahydrofuran (10 mL) was added dropwise 1 M lithium tri-tert-butoxyaluminum hydride in tetrahydrofuran (5.23 mL, 5.23 mmol). The reaction was warmed to room temperature and stirred for 2 hours. The mixture was cooled to 0 °C, quenched with saturated ammonium chloride solution, and extracted with ethyl acetate (3 × 30 mL). The combined organic phase was washed with saturated sodium chloride solution (50 mL), dried over anhydrous sodium sulfate, and concentrated under vacuum. The residue was purified by silica gel column chromatography (dichloromethane: methanol = 6:1) to afford compound B159-6 (520 mg, 71.70% yield) as a colorless oil. ESI/MS (m/z): 278.0 [M+H]⁺.

At room temperature, to a mixture of compound INT-D (279 mg, 1.20 mmol), N,N'-dicyclohexylcarbodiimide (371 mg, 1.80 mmol), and 4-dimethylaminopyridine (176 mg, 1.44 mmol) in dichloromethane (5 mL) were added N,N-dimethylformamide (5 mL) and compound B159-6 (350 mg, 1.20 mmol). The reaction mixture was stirred at room temperature overnight, cooled to 0 °C, quenched with water, and extracted with ethyl acetate (3 × 30 mL). The combined organic phase was washed with saturated sodium chloride solution (50 mL), dried over anhydrous sodium sulfate, and concentrated under vacuum. The residue was purified by silica gel column chromatography (petroleum ether:ethyl acetate = 1:3) to afford compound B159-7 (280 mg, 47.80% yield) as a white solid. ESI/MS (m/z): 489.2 [M+H]⁺.

Under a nitrogen atmosphere, to a solution of compound B159-7 (150 mg, 0.30 mmol) in methanol (10 mL) was added 10% palladium on carbon (75 mg, 0.07 mmol). The reaction vessel was purged with hydrogen three times and stirred at room temperature for 2 hours. The mixture was filtered, and the filter cake was washed with methanol (10 mL). The combined filtrate was concentrated under vacuum. The residue was purified by reverse-phase chromatography (mobile phase A: water (0.1% formic acid), mobile phase B: acetonitrile) to afford compound B159 (17.8 mg, 15.08% yield) as a white solid. ESI/MS (m/z): 355.1 [M+H]⁺.¹H NMR (400 MHz, DMSO-*d*₆) δ 12.03 (s, 1H), 8.37 (s, 1H), 7.66 (dd, *J =* 8.3, 1.0 Hz, 1H), 7.58 (dd, *J =* 7.4, 1.0 Hz, 1H), 7.37 (dd, *J =* 8.3, 7.3 Hz, 1H), 6.40 (s, 1H), 5.44 (p, *J =* 6.3 Hz, 1H), 3.40-3.28 (m, 1H), 3.18-3.11 (m, 1H), 3.10 (t, *J =* 6.0 Hz, 2H), 3.06-2.99 (m, 1H), 2.91-2.81 (m, 1H), 2.61-2.54 (m, 2H), 2.49-2.38 (m, 2H), 2.34-2.24 (m, 1H), 2.16-2.12 (m, 1H), 2.12-2.06 (m, 2H), 1.72-1.64 (m, 1H).

### Example 218: Synthesis of Compound B160

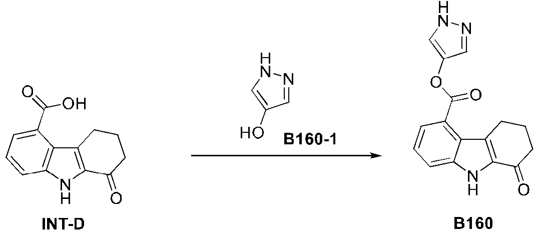

At room temperature, to a mixture of compound INT-D (150 mg, 0.652 mmol) and compound B160-1 (82.3 mg, 0.978 mmol) in dichloromethane (5 mL) were added 1-(3-dimethylaminopropyl)-3-ethylcarbodiimide hydrochloride (101.3 mg, 0.652 mmol) and 4-dimethylaminopyridine (159.4 mg, 1.304 mmol). The reaction mixture was stirred at room temperature overnight, then quenched with water (2 mL). The mixture was concentrated under vacuum, and the residue was purified by reverse-phase chromatography (mobile phase A: water (0.1% formic acid), mobile phase B: acetonitrile) to afford compound B160 (35.3 mg, 18.32% yield) as a white solid. ESI/MS (m/z): 296.1 [M+H]⁺.¹H NMR (400 MHz, DMSO-*d*₆) δ 12.93 (s, 1H), 12.12 (s, 1H), 8.02 (s, 1H), 7.80 (dd, *J =* 7.4, 1.0 Hz, 1H), 7.78-7.58 (m, 2H), 7.44 (dd, *J =* 8.4, 7.3 Hz, 1H), 3.14 (t, *J=* 6.0 Hz, 2H), 2.62-2.54 (m, 2H), 2.17-2.08 (m, 2H).

### Example 219: Synthesis of Compound B161

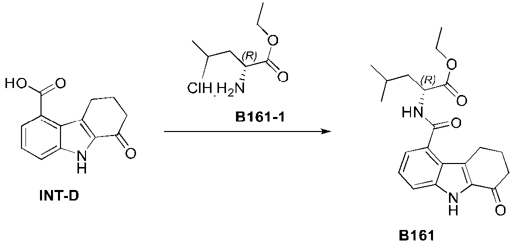

At room temperature, to a mixture of compound INT-D (180.0 mg, 0.746 mmol) in dichloromethane (3.5 mL) were added 1-ethyl-3-(3-dimethylaminopropyl)carbodiimide hydrochloride (171.6 mg, 0.895 mmol) and 4-dimethylaminopyridine (54.7 mg, 0.448 mmol). The reaction mixture was cooled to 5 °C and set aside for use.

Separately, at room temperature, to a solution of pyridine (76.7 mg, 0.970 mmol) in dichloromethane (0.5 mL) was added compound B161-1 (175.2 mg, 0.895 mmol). After stirring for 15 minutes, this solution was slowly added dropwise to the precooled reaction mixture. The combined mixture was stirred at 10 °C for 2 hours, then diluted with ethyl acetate (100 mL). The resulting organic phase was washed sequentially with water (30 mL) and saturated sodium chloride solution (30 mL), dried over anhydrous sodium sulfate, and concentrated under vacuum. The residue was triturated with ethyl acetate (5 mL) and filtered. The filter cake was dried under vacuum to afford compound B161 (53.5 mg) as a white solid. ESI/MS (m/z): 371.1 [M+H]⁺.¹H NMR (500 MHz, DMSO-*d*₆) δ 11.80 (s, 1H), 8.76 (d, *J =* 7.7 Hz, 1H), 7.50 (d, *J =* 8.2 Hz, 1H), 7.33 (dd, *J =* 8.4, 7.1 Hz, 1H), 7.15 (d, *J =* 7.0 Hz, 1H), 4.52-4.46 (m, 1H), 4.17-4.11 (m, 2H), 2.95-2.84 (m, 2H), 2.57-2.52 (m, 2H), 2.12-2.03 (m, 2H), 1.80-1.71 (m, 2H), 1.61-1.54 (m, 1H), 1.22 (t, *J =* 7.1 Hz, 3H), 0.94 (dd, *J =* 6.4, 3.0 Hz, 6H).

### Example 220: Synthesis of Compound B162

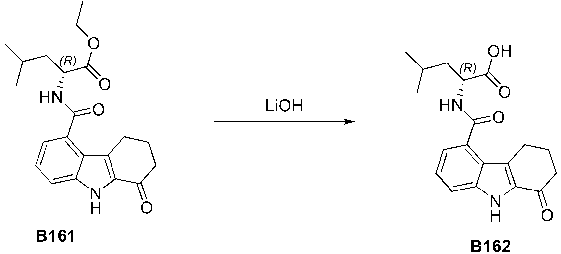

At room temperature, to a solution of compound B161 (50 mg, 0.135 mmol) in tetrahydrofuran (2 mL) was added a saturated aqueous lithium hydroxide solution (2 mL). The reaction mixture was stirred for 1 hour, then acidified to pH = 1 with 3 M hydrochloric acid solution and diluted with ethyl acetate (100 mL). The resulting mixture was washed with water (30 mL), dried over anhydrous sodium sulfate, and concentrated under vacuum. The residue was purified by reverse-phase column chromatography (acetonitrile/water) to afford compound B162 (26.9 mg) as a white solid. ESI/MS (m/z): 343.1 [M+H]⁺.¹H NMR (500 MHz, DMSO-*d*₆) δ 12.56 (s, 1H), 11.78 (s, 1H), 8.62 (d, *J =* 7.9 Hz, 1H), 7.49 (d, *J =* 8.3 Hz, 1H), 7.32 (dd, *J =* 8.3, 7.1 Hz, 1H), 7.17-7.14 (m, 1H), 4.47-4.41 (m, 1H), 2.99-2.92 (m, 1H), 2.91-2.83 (m, 1H), 2.57-2.51 (m, 2H), 2.11-2.02 (m, 2H), 1.80-1.69 (m, 2H), 1.61-1.55 (m, 1H), 0.94 (d, *J =* 6.5 Hz, 6H).

### Example 221: Synthesis of Compound B163

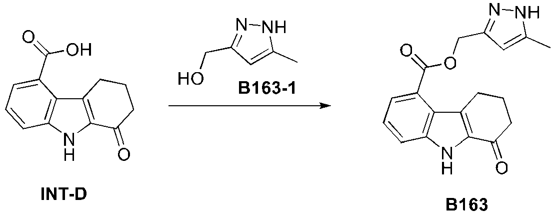

At room temperature, compound B163-1 (108.5 mg, 0.968 mmol) was added to a mixture of compound INT-D (150 mg, 0.645 mmol), 1-(3-dimethylaminopropyl)-3-ethylcarbodiimide hydrochloride (100.2 mg, 0.645 mmol), and N,N-dimethylaminopyridine (157.7 mg, 1.290 mmol) in dichloromethane (10 mL), and the mixture was stirred overnight. The reaction mixture was diluted with dichloromethane (40 mL), washed sequentially with water (3 × 20 mL) and saturated sodium chloride solution (30 mL), dried over anhydrous sodium sulfate, and concentrated under vacuum. The residue was purified by reverse-phase chromatography (mobile phase A: water (0.1% formic acid), mobile phase B: acetonitrile) to afford compound B163 as a white solid (57.6 mg, 57.61%). ESI/MS (m/z): 324.1 [M+H]⁺. ¹H NMR (400 MHz, DMSO-*d*₆) δ 12.56 (s, 1H), 12.01 (s, 1H), 7.66 (dd, *J =* 8.3, 1.0 Hz, 1H), 7.59 (dd, *J =* 7.2, 1.0 Hz, 1H), 7.36 (dd, *J* = 8.3, 7.3 Hz, 1H), 6.12 (s, 1H), 5.28 (s, 2H), 3.05 (t, *J =* 6.0 Hz, 2H), 2.60-2.53 (m, *J =* 7.3, 5.5 Hz, 2H), 2.22 (s, 3H), 2.13-2.03 (m, 2H).

### Example 222: Synthesis of Compound B164

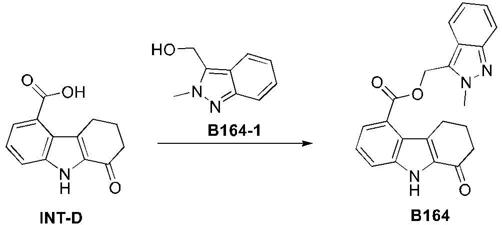

At room temperature, compound B164-1 (157.0 mg, 0.968 mmol) was added to a mixture of compound INT-D (150 mg, 0.645 mmol), 1-(3-dimethylaminopropyl)-3-ethylcarbodiimide hydrochloride (100.2 mg, 0.645 mmol), and N,N-dimethylaminopyridine (157.7 mg, 1.290 mmol) in dichloromethane (10 mL). The mixture was stirred overnight. The reaction was quenched with water (30 mL) and extracted with dichloromethane (3 × 30 mL). The combined organic layers were washed with saturated sodium chloride solution (40 mL), dried over anhydrous sodium sulfate, and concentrated under vacuum. The residue was purified by reverse-phase chromatography (mobile phase A: water (0.1% ammonia water + 10 mmol/L ammonium bicarbonate), mobile phase B: acetonitrile) to afford compound B164 as a white solid (59.7 mg, 24.78%). ESI/MS (m/z): 374.2 [M+H]⁺.¹H NMR (400 MHz, DMSO-*d*₆) δ 12.00 (s, 1H), 7.88 (dt, *J =* 8.3, 1.1 Hz, 1H), 7.66 (dd, *J =* 8.3, 1.0 Hz, 1H), 7.66-7.60 (m, 1H), 7.64-7.58 (m, 1H), 7.35 (dd, *J =* 8.3, 7.3 Hz, 1H), 7.30-7.24 (m,1H), 7.14-7.08 (m, 1H), 5.90 (s, 2H), 4.27 (s, 3H), 2.97 (t, *J =* 6.0 Hz, 2H), 2.56-2.49 (m, 2H), 2.05-1.94 (m, 2H).

### Example 223: Synthesis of Compound B165

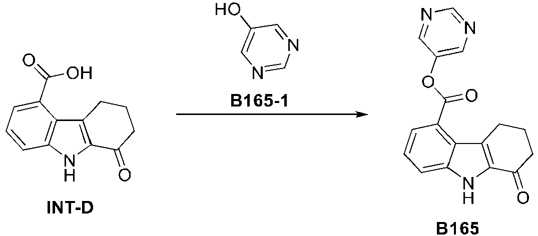

At room temperature, 1-(3-dimethylaminopropyl)-3-ethylcarbodiimide hydrochloride (101.3 mg, 0.652 mmol) and N,N-dimethylaminopyridine (159.4 mg, 1.304 mmol) were added to a mixture of compound INT-D (150 mg, 0.652 mmol) and compound B165-1 (94.0 mg, 0.978 mmol) in dichloromethane (5 mL). The mixture was stirred overnight. The reaction mixture was concentrated under vacuum, and the residue was purified by reverse-phase chromatography (mobile phase A: water (0.1% formic acid), mobile phase B: acetonitrile) to afford compound B165 as a pale yellow solid (61.1 mg, 30.48%). ESI/MS (m/z): 308.0 [M+H]⁺.¹H NMR (400 MHz, DMSO-*d*₆) δ 12.18 (s, 1H), 9.19 (s, 1H), 9.01 (s, 2H), 7.99 (d, *J =* 7.3 Hz, 1H), 7.81 (d, *J =* 8.2 Hz, 1H), 7.54-7.43 (m, 1H), 3.17 (t, *J =* 6.0 Hz, 2H), 2.66-2.53 (m, 2H), 2.17-2.05 (m, 2H).

### Example 224: Synthesis of Compound B166

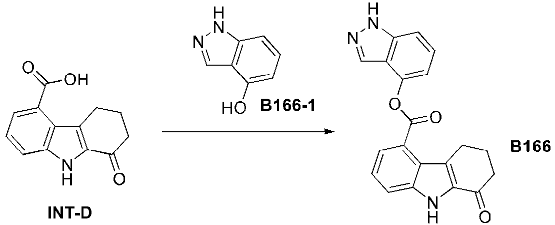

At room temperature, 1-(3-dimethylaminopropyl)-3-ethylcarbodiimide hydrochloride (101.3 mg, 0.652 mmol) and N,N-dimethylaminopyridine (159.4 mg, 1.304 mmol) were added to a mixture of compound INT-D (150 mg, 0.652 mmol) and compound B166-1 (131.3 mg, 0.978 mmol) in dichloromethane (5 mL). The mixture was stirred overnight. The reaction mixture was concentrated under vacuum, and the residue was purified by reverse-phase chromatography (mobile phase A: water (0.1% formic acid), mobile phase B: acetonitrile) to afford compound B166 as a pale yellow solid (63.1 mg, 28.01%). ESI/MS (m/z): 346.1 [M+H]⁺.¹H NMR (400 MHz, DMSO-*d*₆) δ 13.35 (s, 1H), 12.15 (s, 1H), 8.09 (s, 1H), 8.01 (dd, *J =* 7.4, 1.0 Hz, 1H), 7.80 (dd, *J =* 8.3, 1.0 Hz, 1H), 7.55-7.48 (m, 2H), 7.44 (dd, *J =* 8.4, 7.4 Hz, 1H), 7.09 (d, *J =* 7.3 Hz, 1H), 3.16 (t, *J =* 6.0 Hz, 2H), 2.61-2.54 (m, 2H), 2.15-2.05 (m, 2H).

### Example 225: Synthesis of Compound B167

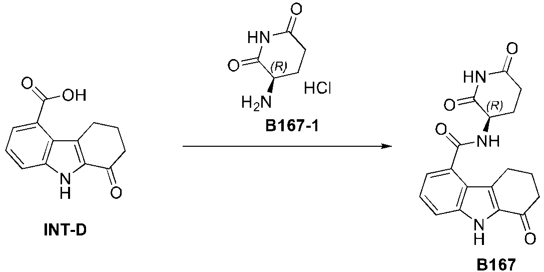

At room temperature, N,N-dimethylformamide (3 mL), N,N'-dicyclohexylcarbodiimide (161.5 mg, 0.782 mmol), and N,N-dimethylaminopyridine (119.6 mg, 0.978 mmol) were added to a mixture of compound INT-D (150 mg, 0.652 mmol) and compound B167-1 (161.1 mg, 0.978 mmol) in dichloromethane (3 mL). The mixture was stirred overnight. The reaction mixture was concentrated under vacuum, and the residue was purified by reverse-phase chromatography (mobile phase A: water (0.1% formic acid), mobile phase B: acetonitrile) to afford compound B167 as a white solid (64.8 mg, 29.27%). ESI/MS (m/z): 340.1 [M+H]⁺.¹H NMR (400 MHz, DMSO-*d*₆) δ 11.82 (s, 1H), 10.85 (s, 1H), 8.71 (d, *J =* 8.4 Hz, 1H), 7.51 (dd, *J =* 8.3, 1.0 Hz, 1H), 7.34 (dd, *J =* 8.4, 7.1 Hz, 1H), 7.21 (dd, *J =* 7.1, 1.0 Hz, 1H), 4.85-4.74 (m, 1H), 3.08-2.88 (m, 2H), 2.88-2.76 (m, 1H), 2.62-2.57 (m, 1H), 2.56-2.52 (m, 2H), 2.20-2.00 (m, 4H).

### Example 226: Synthesis of Compound B168

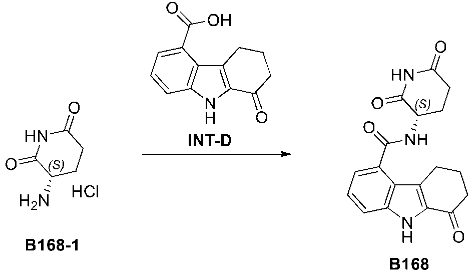

At room temperature, N,N-dimethylformamide (5 mL) and compound B168-1 (159.3 mg, 0.968 mmol) were added to a mixture of compound INT-D (150 mg, 0.645 mmol), N,N'-dicyclohexylcarbodiimide (199.7 mg, 0.968 mmol), and N,N-dimethylaminopyridine (94.6 mg, 0.774 mmol) in dichloromethane (5 mL). The mixture was stirred overnight. The reaction was quenched with water (30 mL) and extracted with ethyl acetate (30 mL × 3). The combined organic layers were washed with saturated sodium chloride solution (40 mL), dried over anhydrous sodium sulfate, and concentrated under vacuum. The residue was purified by reverse-phase chromatography (mobile phase A: water (0.1% formic acid), mobile phase B: acetonitrile) to afford compound B168 as a white solid (35.0 mg, 15.99%). ESI/MS (m/z): 340.1 [M+H]⁺.¹H NMR (400 MHz, DMSO-*d*₆) δ 11.83 (s, 1H), 10.86 (s, 1H), 8.71 (d, *J =* 8.4 Hz, 1H), 7.52 (dd, *J =* 8.3, 1.0 Hz, 1H), 7.34 (dd, *J =* 8.3, 7.1 Hz, 1H), 7.21 (dd, *J =* 7.1, 1.0 Hz, 1H), 4.84-4.75 (m, 1H), 3.09-2.99 (m, 1H), 2.97-2.88 (m, 1H), 2.87-2.77 (m, 1H), 2.62-2.55 (m, 1H), 2.55-2.52 (m, 2H), 2.20-2.11 (m, 1H), 2.11-2.07 (m, 2H), 2.07-2.01 (m, 1H).

### Example 227: Synthesis of Compound B169

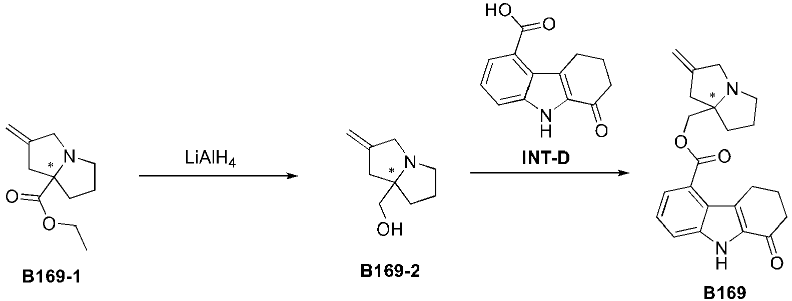

At 0°C, a 2M solution of lithium aluminum hydride in tetrahydrofuran (2.56 mL, 5.122 mmol) was added to a solution of compound B169-1 (500 mg, 2.561 mmol) in tetrahydrofuran (10 mL). The reaction mixture was stirred at room temperature for 2 hours and then quenched sequentially with water (0.5 mL), 10% aqueous sodium hydroxide solution (1 mL), and water (1.5 mL). The mixture was filtered, and the filter cake was washed with tetrahydrofuran (3 × 10 mL). The combined filtrates were concentrated under vacuum, and the residue was purified by silica gel column chromatography (dichloromethane (1% triethylamine): ethyl acetate = 1:1) to afford compound B169-2 as a light-yellow oil (110 mg, 28.04%). ESI/MS (m/z): 154.0 [M+H]⁺.

At room temperature, N,N-dimethylformamide (3 mL) and compound B169-2 (100 mg, 0.653 mmol) were added to a mixture of compound INT-D (151.7 mg, 0.653 mmol), N,N-dimethylaminopyridine (159.5 mg, 1.306 mmol), and 1-(3-dimethylaminopropyl)-3-ethylcarbodiimide hydrochloride (101.3 mg, 0.653 mmol) in dichloromethane (3 mL). The reaction mixture was stirred at room temperature overnight, diluted with water (20 mL), and extracted with ethyl acetate (3 × 20 mL). The combined organic layers were washed with saturated sodium chloride solution (40 mL), dried over anhydrous sodium sulfate, and concentrated under vacuum. The residue was purified by reverse-phase chromatography (mobile phase A: water (10 mmol/L ammonium bicarbonate), mobile phase B: acetonitrile) to afford compound B169 as a white solid (18.5 mg, 7.78%, racemate). ESI/MS (m/z): 365.2 [M+H]⁺.¹H NMR (400 MHz, DMSO-*d*₆) δ 12.00 (s, 1H), 7.66 (dd, *J =* 8.3, 1.1 Hz, 1H), 7.61 (dd, *J =* 7.3, 1.1 Hz, 1H), 7.37 (dd, *J =* 8.4, 7.2 Hz, 1H), 4.90 (dq, *J =* 7.8, 1.9 Hz, 2H), 4.07 (d, *J =* 10.7 Hz, 1H), 4.00 (d, *J=* 10.7 Hz, 1H), 3.57 (dt, *J =* 14.1, 1.6 Hz, 1H), 3.20 (dq, *J =* 14.2, 1.9 Hz, 1H), 3.11 (t, *J =* 6.0 Hz, 2H), 3.04-2.96 (m, 1H), 2.62-2.53 (m, 4H), 2.38 (d, *J =* 15.8 Hz, 1H), 2.15-2.06 (m, 2H), 1.98-1.82 (m, 2H), 1.82-1.64 (m, 2H).

### Example 228: Synthesis of Compound B170

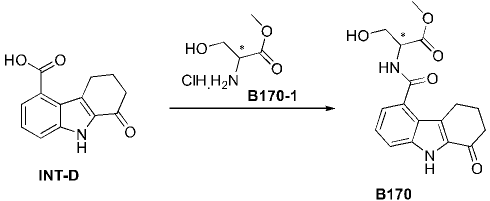

At room temperature, compound B170-1 (125.7 mg, 0.808 mmol), N,N-diisopropylethylamine (278.5 mg, 2.150 mmol), and N,N,N',N'-tetramethyl-O-(7-azabenzotriazol-1-yl)uronium hexafluorophosphate (307.3 mg, 0.808 mmol) were added to a mixture of compound INT-D (130.0 mg, 0.539 mmol) in dichloromethane (3 mL). The reaction mixture was stirred at room temperature for 1 hour and then filtered. The resulting filter cake was stirred at room temperature in a mixed solution of methanol (2 mL) and water (1 mL) for 3 hours, followed by suction filtration. The filter cake was dried under vacuum to afford compound B170 as a white solid (152.1 mg, racemate). ESI/MS (m/z): 331.0 [M+H]⁺.¹H NMR (500 MHz, DMSO-*d*₆) δ 11.81 (s, 1H), 8.55 (d, *J =* 7.4 Hz, 1H), 7.51 (dd, *J =* 8.4, 0.9 Hz, 1H), 7.33 (dd, *J = 8.3,* 7.1 Hz, 1H), 7.22 (dd, *J* = 7.2, 0.9 Hz, 1H), 5.00 (t, *J =* 6.2 Hz, 1H), 4.55 (dt, *J* = 7.5, 5.3 Hz, 1H), 3.80 (t, *J= 5.7* Hz, 2H), 3.69 (s, 3H), 2.96-2.88 (m, 2H), 2.56-2.52 (m, 2H), 2.12-2.05 (m, 2H).

### Example 229: Synthesis of Compound B171

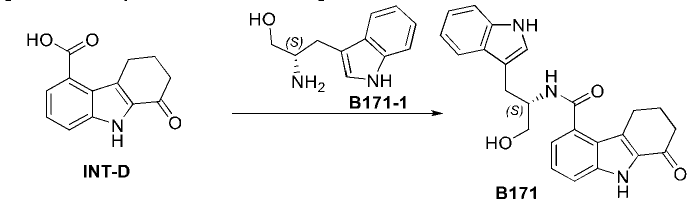

At room temperature, compound B171-1 (47.3 mg, 0.249 mmol), N,N-diisopropylethylamine (80.3 mg, 0.622 mmol), and N,N,N',N'-tetramethyl-O-(7-azabenzotriazol-1-yl)uronium hexafluorophosphate (118.7 mg, 0.311 mmol) were added to a mixture of compound INT-D (50.0 mg, 0.207 mmol) in dichloromethane (1.5 mL). The reaction mixture was stirred for 1 hour and then filtered. The resulting filter cake was slurried at room temperature in a mixed solution of methanol (1.5 mL) and water (0.5 mL) for 3 hours, followed by suction filtration. The filter cake was dried under vacuum to afford compound B171 as a white solid (62.36 mg). ESI/MS (m/z): 402.0 [M+H]⁺.¹H NMR (500 MHz, DMSO-*d*₆) δ 11.72 (s, 1H), 10.77 (s, 1H), 8.11 (d, *J* = 8.4 Hz, 1H), 7.65 (d, *J=* 7.9 Hz, 1H), 7.44 (d, *J =* 8.3 Hz, 1H), 7.34 (d, *J =* 8.0 Hz, 1H), 7.26 (dd, *J* = 8.3, 7.1 Hz, 1H), 7.16 (d, *J =* 2.3 Hz, 1H), 7.06 (t, *J =* 7.7 Hz, 1H), 7.03 (d, *J =* 7.1 Hz, 1H), 6.98 (t, *J =* 7.4 Hz, 1H), 4.80 (t, *J =* 5.7 Hz, 1H), 4.34-4.26 (m, 1H), 3.63-3.55 (m, 1H), 3.52-3.45 (m, 1H), 3.06 (dd, *J =* 14.5, 5.5 Hz, 1H), 2.88 (dd, *J =* 14.5, 8.4 Hz, 1H), 2.75 (dt, *J =* 17.2, 6.0 Hz, 1H), 2.56 (dt, *J =* 17.2, 6.1 Hz, 1H), 2.49-2.46 (m, 2H), 1.99-1.90 (m, 2H).

### Example 230: Synthesis of Compound B172

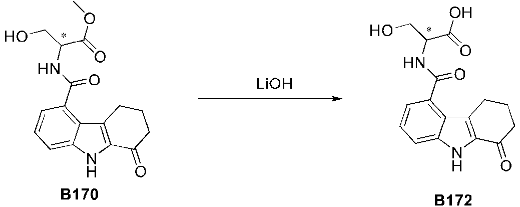

At room temperature, a solution of lithium hydroxide (29.0 mg, 1.210 mmol) in water (1.2 mL) was added to a solution of compound B170 (80.0 mg, 0.242 mmol) in tetrahydrofuran (1.2 mL). The reaction mixture was stirred at room temperature for 1 hour and then quenched with a 3M aqueous hydrochloric acid solution. The mixture was filtered, and the filter cake was dried under vacuum to afford compound B172 as a pale yellow solid (64.2 mg, racemate). ESI/MS (m/z): 317.0 [M+H]⁺.¹H NMR (500 MHz, DMSO-*d*₆) δ 12.65 (br, 1H), 11.80 (s, 1H), 8.35 (d, *J =* 7.7 Hz, 1H), 7.51 (d, *J =* 8.3 Hz, 1H), 7.33 (dd, *J* = 8.3, 7.1 Hz, 1H), 7.23 (d, *J =* 7.0 Hz, 1H), 4.93 (br, 1H), 4.51-4.46 (m, 1H), 3.80 (d, *J =* 5.1 Hz, 2H), 2.94 (t, *J =* 6.1 Hz, 2H), 2.56-2.52 (m, 2H), 2.11-2.03 (m, 2H).

### Example 231: Synthesis of Compound B173

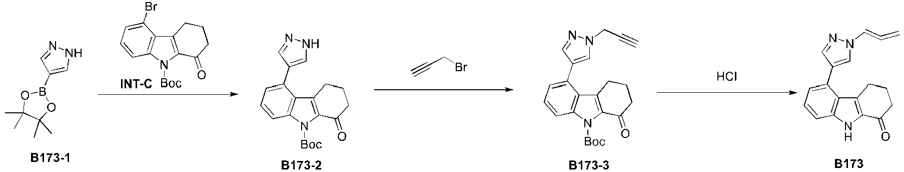

Under a nitrogen atmosphere, a solution of compound INT-C (200 mg, 0.52 mmol) in 1,4-dioxane (3 mL) was sequentially treated with water (0.75 mL), compound B173-1 (151 mg, 0.78 mmol), [1,1'-bis(diphenylphosphino)ferrocene]dichloropalladium(II) dichloromethane complex (21 mg, 0.026 mmol), and potassium carbonate (144 mg, 1.04 mmol). The mixture was heated to 80 °C and stirred for 4 hours. After cooling to room temperature, the reaction mixture was diluted with ethyl acetate (30 mL), washed sequentially with saturated aqueous sodium bicarbonate solution (10 mL) and saturated aqueous sodium chloride solution (10 mL), dried over anhydrous sodium sulfate, and concentrated under vacuum. The residue was purified by reverse-phase preparative liquid chromatography (acetonitrile/water: 10-40%) to afford compound B173-2 as a yellow oil (95 mg, 0.17 mmol). ESI/MS (m/z): 352.0 [M+H]⁺.

At room temperature, a mixture of compound B173-2 (85 mg, 0.15 mmol) in acetonitrile (2 mL) was treated with 3-bromopropyne (43 mg, 0.22 mmol) and cesium carbonate (97 mg, 0.3 mmol), then heated to 80 °C and stirred for 4 hours. The reaction mixture was diluted with ethyl acetate (30 mL), washed sequentially with water (10 mL) and saturated aqueous sodium chloride solution (10 mL), dried over anhydrous sodium sulfate, and concentrated under vacuum. The residue was purified by reverse-phase preparative liquid chromatography (acetonitrile/water: 10-80%) to afford compound B173-3 as a white solid (65 mg, 0.15 mmol). ESI/MS (m/z): 390.0 [M+H]⁺.

At room temperature, concentrated hydrochloric acid (0.2 mL) was added to a solution of compound B173-3 (65 mg, 0.15 mmol) in methanol (2 mL), and the mixture was stirred for 3 hours. The reaction mixture was concentrated under vacuum, and the residue was purified by reverse-phase preparative liquid chromatography (acetonitrile/water: 10-50%) to afford compound B173 as a white solid (6 mg, 0.02 mmol). ESI/MS (m/z): 290.0 [M+H]⁺.¹H NMR (500 MHz, DMSO-*d*₆) δ 11.73 (s, 1H), 8.00 (s, 1H), 7.79 (s, 1H), 7.56 (t, *J =* 6.5 Hz, 1H), 7.37 (dd, *J =* 8.2, 1.0 Hz, 1H), 7.29 (dd, *J =* 8.3, 7.1 Hz, 1H), 6.96 (dd, *J =* 7.0, 1.0 Hz, 1H), 5.77 (d, *J =* 6.5 Hz, 2H), 2.72 (t, *J =* 6.0 Hz, 2H), 2.55-2.51 (m, 2H), 2.05-1.97 (m, 2H).

### Example 232: Synthesis of Compound B174

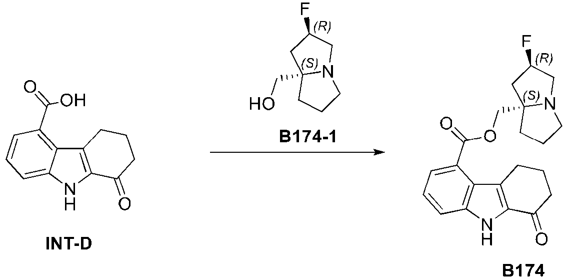

At room temperature, 4-dimethylaminopyridine (12.7 mg, 0.10 mmol) and 1-(3-dimethylaminopropyl)-3-ethylcarbodiimide hydrochloride (59.6 mg, 0.31 mmol) were added to a mixture of compound INT-D (50.0 mg, 0.21 mmol) and compound B174-1 (49.5 mg, 0.31 mmol) in dichloromethane (1 mL). The mixture was stirred for 6 hours. The reaction mixture was diluted with dichloromethane (30 mL), washed sequentially with 0.5M aqueous hydrochloric acid solution (10 mL) and saturated aqueous sodium chloride solution (10 mL), dried over anhydrous sodium sulfate, and concentrated under vacuum. The residue was purified by preparative thin-layer chromatography (dichloromethane: methanol = 20:1) to afford compound B174 as a white solid (22.0 mg, 27.8%). ESI/MS (m/z): 371.1 [M+H]⁺.¹H NMR (500 MHz, DMSO-*d*₆) δ 12.00 (s, 1H), 7.66 (dd, *J =* 8.2, 1.0 Hz, 1H), 7.61 (d, *J =* 7.3 Hz, 1H), 7.40-7.37 (m, 1H), 5.30 (d, *J =* 54.0 Hz, 1H), 4.10 -4.07(dz, 2H), 3.15-3.08 (m, 5H), 2.87 (s, 1H), 2.58-2.56 (m, 2H), 2.13-2.00 (m, 5H), 1.91-1.81 (m, 3H).

### Example 233: Synthesis of Compound B175

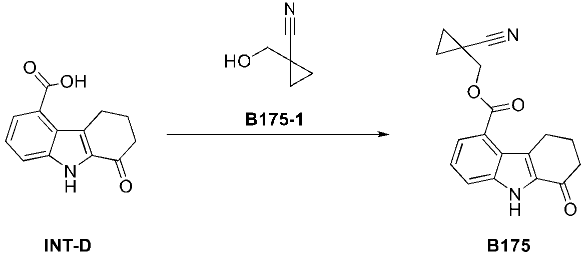

At room temperature, 4-dimethylaminopyridine (12.7 mg, 0.10 mmol) and 1-(3-dimethylaminopropyl)-3-ethylcarbodiimide hydrochloride (59.6 mg, 0.31 mmol) were added to a mixture of compound INT-D (50.0 mg, 0.21 mmol) and compound B175-1 (30.2 mg, 0.31 mmol) in dichloromethane (1 mL). The mixture was stirred at room temperature for 6 hours. The reaction mixture was diluted with dichloromethane (30 mL), washed sequentially with 0.5M aqueous hydrochloric acid solution (10 mL) and saturated aqueous sodium chloride solution (10 mL), dried over anhydrous sodium sulfate, and concentrated under vacuum. The residue was purified by preparative thin-layer chromatography (petroleum ether:ethyl acetate = 1:1) to afford compound B175 as a white solid (39.2 mg, 58.3%). ESI/MS (m/z): 309.0 [M+H]⁺.¹H NMR (500 MHz, DMSO-*d*₆) δ 12.05 (s, 1H), 7.70 (dd, *J =* 8.3, 1.0 Hz, 1H), 7.67 (dd, *J =* 7.3, 1.0 Hz, 1H), 7.42 (dd, *J =* 8.3, 7.3 Hz, 1H), 4.39 (s, 2H), 3.14 (t, *J =* 6.0 Hz, 2H), 2.58 (dd, *J =* 7.3, 5.5 Hz, 2H), 2.12 (p, *J =* 6.2 Hz, 2H), 1.45-1.39 (m, 2H), 1.29-1.24 (m, 2H).

### Example 234: Synthesis of Compound B176

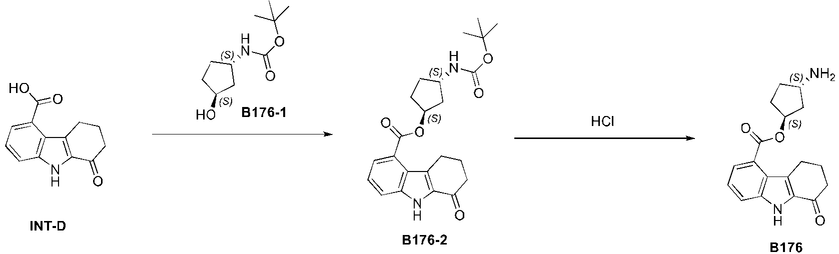

At room temperature, 4-dimethylaminopyridine (18.5 mg, 0.15 mmol) and 1-(3-dimethylaminopropyl)-3-ethylcarbodiimide hydrochloride (75.4 mg, 0.39 mmol) were added to a mixture of compound INT-D (73.0 mg, 0.30 mmol) and compound B176-1 (73.1 mg, 0.36 mmol) in dichloromethane (1.5 mL). The mixture was stirred overnight. The reaction mixture was diluted with dichloromethane (30 mL), washed sequentially with 0.5M aqueous hydrochloric acid solution (10 mL) and saturated aqueous sodium chloride solution (10 mL), dried over anhydrous sodium sulfate, and concentrated under vacuum. The residue was purified by silica gel column chromatography (petroleum ether:ethyl acetate = 3:1) to afford compound B176-2 as a white solid (68 mg, 51.8%). ESI/MS (m/z): 435.1 [M+Na]⁺.

At room temperature, a 4M hydrogen chloride solution in 1,4-dioxane (0.5 mL) was added to a solution of compound B176-2 (60 mg, 0.14 mmol) in dichloromethane (1 mL), and the mixture was stirred overnight. The reaction mixture was concentrated under vacuum, and the residue was washed with dichloromethane and filtered. The resulting filter cake was dried under vacuum to afford the hydrochloride salt of compound B176 as a white solid (42.5 mg, 83.8%). ESI/MS (m/z): 313.1 [M+H]⁺.¹H NMR (500 MHz, DMSO-*d*₆) δ 12.01 (s, 1H), 8.17 (s, 3H), 7.66 (dd, *J =* 8.2, 1.0 Hz, 1H), 7.58 (dd, *J =* 7.3, 1.0 Hz, 1H), 7.38-7.35 (m, 1H), 5.47-5.43 (m, 1H), 3.76-3.73 (mz, 1H), 3.09 (t, *J =* 6.0 Hz, 2H), 2.59-2.56 (m, 2H), 2.35-2.27 (m, 1H), 2.27-2.21 (m, 1H), 2.20-2.09 (m, 4H), 1.93-1.81 (m, 1H), 1.76-1.69 (m, 1H).

### Example 235: Synthesis of Compound B177

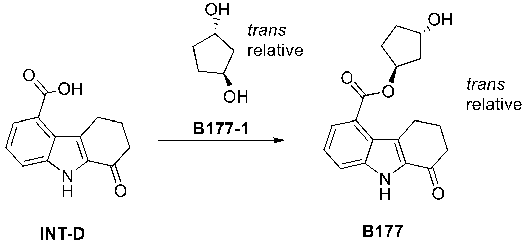

At room temperature, 4-dimethylaminopyridine (26.9 mg, 0.22 mmol) and N,N'-dicyclohexylcarbodiimide (91 mg, 0.44 mmol) were added to a mixture of compound INT-D (50 mg, 0.22 mmol) and compound B177-1 (44 mg, 0.44 mmol) in dichloromethane (1 mL). The mixture was stirred for 2 hours. The reaction mixture was diluted with ethyl acetate (30 mL), washed sequentially with 0.5M aqueous hydrochloric acid solution (10 mL) and saturated aqueous sodium chloride solution (10 mL), dried over anhydrous sodium sulfate, and concentrated under vacuum. The residue was purified by silica gel column chromatography (ethyl acetate:petroleum ether = 1:6) to afford compound B177 as an off-white solid (26 mg, 37.7%). ESI/MS (m/z): 314.0 [M+H]⁺.¹H NMR (400 MHz, DMSO-*d*₆) δ 11.98 (s, 1H), 7.64 (dd, *J =* 8.3, 1.0 Hz, 1H), 7.54 (dd, *J =* 7.3, 1.0 Hz, 1H), 7.35 (dd, *J =* 8.3, 7.3 Hz, 1H), 5.46-5.39 (m, 1H), 4.67 (d, *J =* 3.8 Hz, 1H), 4.37-4.26 (m, 1H), 3.08 (t, *J =* 6.0 Hz, 2H), 2.61-2.53 (m, 2H), 2.29-2.15 (m, 1H), 2.14-2.07 (m, 2H), 2.06-1.97 (m, 2H), 1.96-1.88 (m, 1H), 1.81-1.71 (m, 1H), 1.61-1.52 (m, 1H).

### Example 236: Synthesis of Compound B178

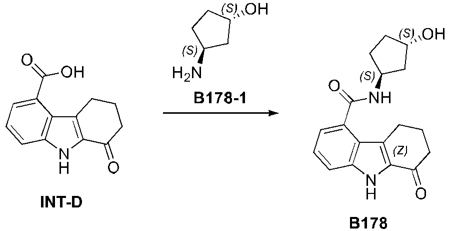

At room temperature, 1-(3-dimethylaminopropyl)-3-ethylcarbodiimide hydrochloride (140.2 mg, 0.903 mmol) was added to a mixture of compound INT-D (210 mg, 0.903 mmol), compound B178-1 (91.4 mg, 0.903 mmol), and N,N-dimethylaminopyridine (220.7 mg, 1.806 mmol) in dichloromethane (10 mL). The mixture was stirred overnight. The reaction was quenched with water (30 mL) and extracted with ethyl acetate (30 mL × 3). The combined organic layers were washed with saturated sodium chloride solution (40 mL), dried over anhydrous sodium sulfate, and concentrated under vacuum. The residue was purified by reverse-phase chromatography (mobile phase A: water (10 mmol/L ammonium bicarbonate), mobile phase B: acetonitrile) to afford compound B178 as a white solid (22.0 mg, 7.80%). ESI/MS (m/z): 313.1 [M+H]⁺.¹H NMR (400 MHz, DMSO-*d*₆) δ 11.77 (s, 1H), 8.33 (d, *J =* 7.5 Hz, 1H), 7.46 (dd, *J =* 8.3, 1.0 Hz, 1H), 7.29 (dd, *J =* 8.4, 7.1 Hz, 1H), 7.08 (dd, *J =* 7.1, 1.0 Hz, 1H), 4.58-4.51 (m, 1H), 4.45 (p, *J=* 7.4 Hz, 1H), 4.21 (s, 1H), 2.92 (t, *J =* 6.0 Hz, 2H), 2.58-2.51 (m, 2H), 2.18-2.05 (m, 3H), 1.96-1.84 (m, 2H), 1.74-1.65 (m, 1H), 1.56-1.42 (m, 2H).

### Example 237: Synthesis of Compound B179

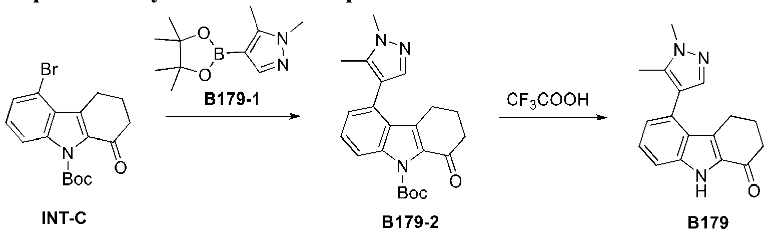

Under a nitrogen atmosphere, a mixture of compound INT-C (100 mg, 0.27 mmol) in 1,4-dioxane (2 mL) and water (0.5 mL) was sequentially treated with compound B179-1 (90 mg, 0.40 mmol), [1,1'-bis(diphenylphosphino)ferrocene]dichloropalladium(II) dichloromethane complex (11 mg, 0.014 mmol), and potassium carbonate (76 mg, 0.55 mmol). The mixture was heated to 80 °C and stirred for 3 hours. After cooling to room temperature, the reaction mixture was diluted with ethyl acetate (30 mL). The resulting mixture was filtered through celite, and the filtrate was concentrated under vacuum to afford the crude compound B179-2. Trifluoroacetic acid (0.3 mL) and dichloromethane (3 mL) were added to compound B179-2, and the mixture was stirred at room temperature for 2 hours. The reaction mixture was diluted with ethyl acetate (30 mL), washed sequentially with saturated aqueous sodium bicarbonate solution (10 mL) and saturated aqueous sodium chloride solution (10 mL), dried over anhydrous sodium sulfate, and concentrated under vacuum. The residue was purified by reverse-phase preparative liquid chromatography (acetonitrile/water: 10-50%) to afford compound B179 as a white solid (41 mg, 0.14 mmol). ESI/MS (m/z): 280.1 [M+H]⁺.¹H NMR (500 MHz, DMSO-*d*₆) δ 11.67 (s, 1H), 7.40 (s, 1H), 7.35 (dd, *J =* 8.3, 1.0 Hz, 1H), 7.29 (dd, *J =* 8.3, 7.0 Hz, 1H), 6.80 (dd, *J =* 7.0, 1.0 Hz, 1H), 3.82 (s, 3H), 2.55 (t, *J* = 6.0 Hz, 2H), 2.49-2.46 (m, 2H), 2.18 (s, 3H), 1.97 (p, *J =* 6.2 Hz, 2H).

### Example 238: Synthesis of Compound B180

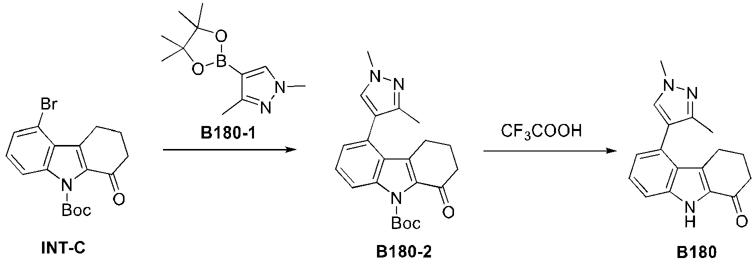

Under a nitrogen atmosphere, a mixture of compound INT-C (100 mg, 0.27 mmol) in 1,4-dioxane (2 mL) and water (0.5 mL) was sequentially treated with compound B180-1 (90 mg, 0.40 mmol), [1,1'-bis(diphenylphosphino)ferrocene]dichloropalladium(II) dichloromethane complex (11 mg, 0.014 mmol), and potassium carbonate (76 mg, 0.55 mmol). The mixture was heated to 80 °C and stirred for 3 hours. After cooling to room temperature, the reaction mixture was diluted with ethyl acetate (30 mL), filtered through celite, and the filtrate was concentrated under vacuum to afford compound B180-2. Trifluoroacetic acid (0.3 mL) and dichloromethane (3 mL) were added to compound B180-2, and the mixture was stirred at room temperature for 2 hours. The reaction mixture was diluted with ethyl acetate (30 mL), washed sequentially with saturated aqueous sodium bicarbonate solution (10 mL) and saturated aqueous sodium chloride solution (10 mL), dried over anhydrous sodium sulfate, and concentrated under vacuum. The residue was purified by reverse-phase preparative liquid chromatography (acetonitrile/water: 10-50%) to afford compound B180 as a white solid (33 mg, 0.12 mmol). ESI/MS (m/z): 280.1 [M+H]⁺.¹H NMR (500 MHz, DMSO-*d*₆) δ 11.66 (s, 1H), 7.66 (s, 1H), 7.35 (dd, *J =* 8.3, 1.0 Hz, 1H), 7.28 (dd, *J =* 8.3, 7.0 Hz, 1H), 6.83 (dd, *J =* 7.1, 1.0 Hz, 1H), 3.83 (s, 3H), 2.54 (t, *J =* 6.0 Hz, 2H), 2.50-2.46 (m, 2H), 2.04 (s, 3H), 1.98 (p, *J =* 6.1 Hz, 2H).

### Example 239: Synthesis of Compound B181

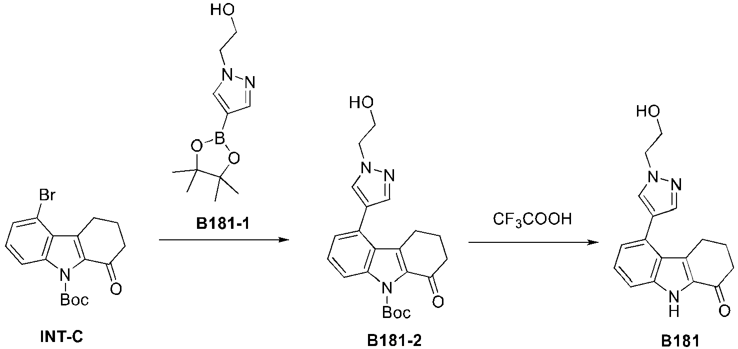

Under a nitrogen atmosphere, a mixture of compound INT-C (100 mg, 0.27 mmol) in 1,4-dioxane (2 mL) and water (0.5 mL) was sequentially treated with compound B181-1 (95 mg, 0.40 mmol), [1,1'-bis(diphenylphosphino)ferrocene]dichloropalladium(II) dichloromethane complex (11 mg, 0.014 mmol), and potassium carbonate (76 mg, 0.55 mmol). The mixture was heated to 80 °C and stirred for 3 hours. After cooling to room temperature, the reaction mixture was diluted with ethyl acetate (30 mL). The resulting mixture was filtered through celite, and the filtrate was concentrated under vacuum to afford the crude compound B181-2. Trifluoroacetic acid (0.3 mL) and dichloromethane (3 mL) were added to compound B181-2, and the mixture was stirred at room temperature for 2 hours. The reaction mixture was diluted with ethyl acetate (30 mL), washed sequentially with saturated aqueous sodium bicarbonate solution (10 mL) and saturated aqueous sodium chloride solution (10 mL), dried over anhydrous sodium sulfate, and concentrated under vacuum. The residue was purified by reverse-phase preparative liquid chromatography (acetonitrile/water: 10-50%) to afford compound B181 as a yellow solid (29 mg, 0.1 mmol). ESI/MS (m/z): 296.0 [M+H]⁺.¹H NMR (500 MHz, DMSO-*d*₆) δ 11.68 (s, 1H), 7.90 (s, 1H), 7.61 (s, 1H), 7.33 (dd, *J =* 8.3, 1.1 Hz, 1H), 7.27 (dd, *J =* 8.3, 7.0 Hz, 1H), 6.92 (dd, *J =* 7.0, 1.1 Hz, 1H), 4.91 (d, *J* = 5.3 Hz, 1H), 4.22 (t, *J =* 5.7 Hz, 2H), 3.80 (q, *J =* 5.5 Hz, 2H), 2.75 (t, *J =* 6.0 Hz, 2H), 2.52 (d, *J =* 5.9 Hz, 2H), 2.05-1.94 (m, 2H).

### Example 240: Synthesis of Compound B182

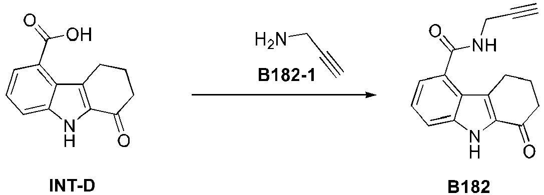

At room temperature, compound B182-1 (13.6 mg, 0.25 mmol), diisopropylethylamine (58.9 mg, 0.44 mmol), and 2-(7-azabenzotriazol-1-yl)-N,N,N',N'-tetramethyluronium hexafluorophosphate (93.6 mg, 0.25 mmol) were added to a mixture of compound INT-D (54 mg, 0.22 mmol) in N,N-dimethylformamide (1 mL). The mixture was stirred for 1 hour. The reaction was quenched with water (5 mL) and extracted with ethyl acetate (10 mL × 3). The combined organic layers were washed with saturated sodium chloride solution (10 mL), dried over anhydrous sodium sulfate, and concentrated under vacuum. The residue was purified by reverse-phase preparative chromatography (acetonitrile/water = 10-35%) to afford compound B182 as a white solid (34.4 mg, 54.8%). ESI/MS (m/z): 267.0 [M+H]⁺.¹H NMR (500 MHz, DMSO-*d*₆) δ 11.81 (s, 1H), 8.83 (t, *J =* 5.6 Hz, 1H), 7.50 (dd, *J =* 8.3, 1.0 Hz, 1H), 7.31 (dd, *J =* 8.3, 7.1 Hz, 1H), 7.12 (dd, *J =* 7.2, 1.0 Hz, 1H), 4.07 (dd, *J =* 5.6, 2.5 Hz, 2H), 3.14 (t, *J =* 2.5 Hz, 1H), 2.93 (t, *J =* 6.0 Hz, 2H), 2.55 (dd, *J =* 7.3, 5.5 Hz, 2H), 2.13-2.05 (m, 2H).

### Example 241: Synthesis of Compound B183

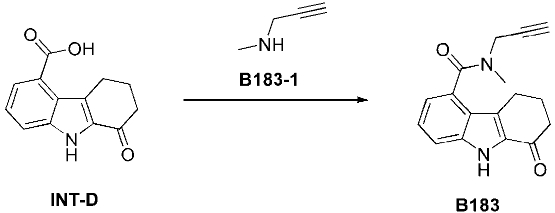

At room temperature, compound B183-1 (17.0 mg, 0.25 mmol), diisopropylethylamine (58.9 mg, 0.44 mmol), and 2-(7-azabenzotriazol-1-yl)-N,N,N',N'-tetramethyluronium hexafluorophosphate (93.6 mg, 0.25 mmol) were added to a mixture of compound INT-D (54 mg, 0.22 mmol) in N,N-dimethylformamide (1 mL). The mixture was stirred for 1 hour. The reaction was quenched with water (5 mL) and extracted with ethyl acetate (10 mL × 3). The combined organic layers were washed with saturated sodium chloride solution (10 mL), dried over anhydrous sodium sulfate, filtered, and concentrated under vacuum. The residue was purified by reverse-phase preparative chromatography (acetonitrile/water = 10-35%) to afford compound B183 as a white solid (56.5 mg, 85.6%). ESI/MS (m/z): 281.0 [M+H]⁺.¹H NMR (500 MHz, DMSO-*d*₆) δ 11.85 (s, 1H), 7.46 (ddd, *J* = 8.4, 4.6, 0.9 Hz, 1H), 7.34 (dd, *J* = 8.4, 7.0 Hz, 1H), 6.95 (ddd, *J* = 10.4, 7.1, 0.9 Hz, 1H), 4.40 (s, 1H), 3.93 (s, 1H), 3.30 (d, *J =* 2.7 Hz, 1H), 3.10 (s, 1H), 2.82-2.76 (m, 4H), 2.58-2.52 (m, 2H), 2.10 (h, *J =* 5.5, 4.8 Hz, 2H).

### Example 242: Synthesis of Compound B184

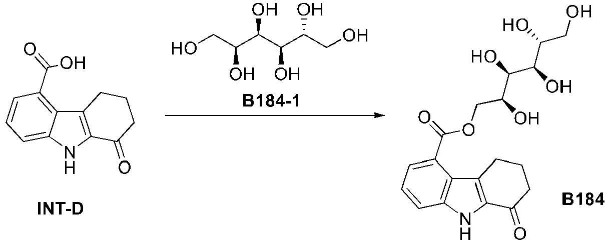

At room temperature, 4-dimethylaminopyridine (12.7 mg, 0.10 mmol) and 1-(3-dimethylaminopropyl)-3-ethylcarbodiimide hydrochloride (59.6 mg, 0.31 mmol) were added to a mixture of compound INT-D (50.0 mg, 0.21 mmol) and compound B184-1 (56.0 mg, 0.31 mmol) in dichloromethane (1 mL). The mixture was stirred for 4 hours. The reaction mixture was diluted with dichloromethane (30 mL), washed sequentially with 0.5M aqueous hydrochloric acid solution (10 mL) and saturated aqueous sodium chloride solution (10 mL), dried over anhydrous sodium sulfate, and concentrated under vacuum. The residue was purified by reverse-phase preparative chromatography (acetonitrile/water = 10-35%) to afford compound B184 as a brown solid (31.5 mg, 36.9%). ESI/MS (m/z): 394.1 [M+H]⁺.

### Example 243: Synthesis of Compound B185

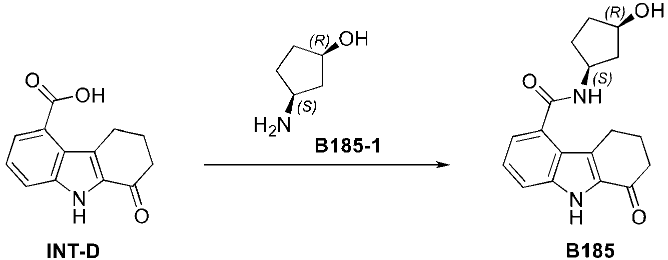

At room temperature, N,N-dimethylformamide (3 mL), 1-(3-dimethylaminopropyl)-3-ethylcarbodiimide (135.0 mg, 0.870 mmol), and N,N-dimethylaminopyridine (212.5 mg, 1.740 mmol) were added to a mixture of compound INT-D (200 mg, 0.870 mmol) and compound B185-1 (88.0 mg, 0.870 mmol) in dichloromethane (6 mL). The mixture was stirred overnight. The reaction was quenched with water (30 mL) and extracted with dichloromethane (3 × 30 mL). The combined organic layers were washed with saturated sodium chloride solution (20 mL), dried over anhydrous sodium sulfate, and concentrated under vacuum. The residue was purified by reverse-phase chromatography (mobile phase A: water (0.1% formic acid), mobile phase B: acetonitrile) to afford compound B185 as a white solid (64.8 mg, 23.85%). ESI/MS (m/z): 313.2 [M+H]⁺.¹H NMR (400 MHz, DMSO-*d*₆) δ 11.71 (s, 1H), 8.22 (d, *J =* 7.5 Hz, 1H), 7.39 (dd, *J =* 8.4, 1.0 Hz, 1H), 7.22 (dd, *J =* 8.4, 7.1 Hz, 1H), 7.02 (dd, *J =* 7.1, 1.0 Hz, 1H), 4.57 (d, *J =* 4.2 Hz, 1H), 4.21-4.09 (m, 1H), 4.08-4.00 (m, 1H), 2.85 (t, *J =* 6.0 Hz, 2H), 2.51-2.44 (m, 2H), 2.16-2.08 (m, 1H), 2.07-1.97 (m, 2H), 1.89-1.78 (m, 1H), 1.71-1.60 (m, 2H), 1.57-1.49 (m, 1H), 1.48-1.39 (m, 1H).

### Example 244: Synthesis of Compound B186

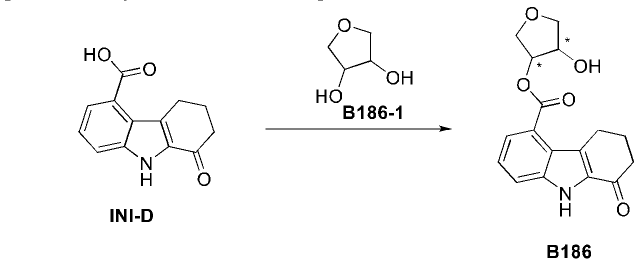

At room temperature, N,N-dimethylformamide (5 mL), N,N'-dicyclohexylcarbodiimide (215.4 mg, 1.044 mmol), and N,N-dimethylaminopyridine (159.4 mg, 1.305 mmol) were added to a mixture of compound INT-D (200 mg, 0.870 mmol) and compound B186-1 (135.8 mg, 1.305 mmol) in dichloromethane (5 mL). The mixture was stirred for 12 hours. The reaction was quenched with water (30 mL) and extracted with dichloromethane (3 × 30 mL). The combined organic layers were washed with saturated sodium chloride solution (20 mL), dried over anhydrous sodium sulfate, and concentrated under vacuum. The residue was purified by reverse-phase chromatography (mobile phase A: water (0.1% formic acid), mobile phase B: acetonitrile) to afford compound B186 as a white solid (42.7 mg, 15.57%). ESI/MS (m/z): 316.2 [M+H]⁺.¹H NMR (400 MHz, DMSO-*d*₆) δ 11.99 (s, 1H), 7.71 (dd, *J =* 7.2, 1.0 Hz, 1H), 7.66 (dd, *J =* 8.4, 1.1 Hz, 1H), 7.38 (dd, *J =* 8.3, 7.3 Hz, 1H), 5.38-5.30 (m, 2H), 4.49-4.39 (m, 1H), 4.08 (dd, *J =* 9.9, 5.5 Hz, 1H), 3.95-3.84 (m, 2H), 3.56 (dd, *J* = 8.7, 6.1 Hz, 1H), 3.19-3.09 (m, 2H), 2.61-2.53 (m, 2H), 2.15-2.05 (m, 2H).

### Example 245: Synthesis of Compound B187

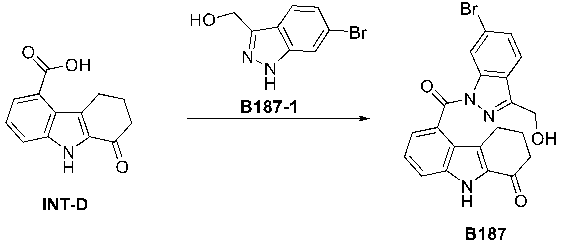

At room temperature, O-(7-azabenzotriazol-1-yl)-N,N,N',N'-tetramethyluronium hexafluorophosphate (372.1 mg, 0.978 mmol) and N,N-diisopropylethylamine (253.0 mg, 1.956 mmol) were added to a mixture of compound INT-D (150 mg, 0.652 mmol) and compound B187-1 (222.2 mg, 0.978 mmol) in N,N-dimethylformamide (10 mL). The mixture was heated to 60°C and stirred overnight. After cooling to room temperature, the reaction was quenched with water (30 mL) and extracted with dichloromethane (3 × 30 mL). The combined organic layers were washed with saturated sodium chloride solution (30 mL), dried over anhydrous sodium sulfate, and concentrated under vacuum. The residue was purified by reverse-phase chromatography (mobile phase A: water (10 mmol/L ammonium bicarbonate + 0.05% ammonia water), mobile phase B: acetonitrile) to afford compound B187 as a white solid (21.5 mg, 7.52%). ESI/MS (m/z): 437.8 [M-H]⁻.¹H NMR (400 MHz, DMSO-*d*₆) δ 11.99 (s, 1H), 8.65 (d, *J =* 1.6 Hz, 1H), 8.01 (d, *J* = 8.5 Hz, 1H), 7.71 (dd, *J =* 8.5, 1.7 Hz, 1H), 7.65 (dd, *J =* 8.1, 1.2 Hz, 1H), 7.43 (dd, *J =* 8.1, 7.2 Hz, 1H), 7.39 (dd, *J =* 7.2, 1.2 Hz, 1H), 5.60 (t, *J =* 5.9 Hz, 1H), 4.70 (d, *J =* 5.8 Hz, 2H), 2.53-2.50 (d, *J =* 6.5 Hz, 2H), 2.49-2.46(s, 2H), 2.03-1.93 (m, 2H).

### Example 246: Synthesis of Compound B188

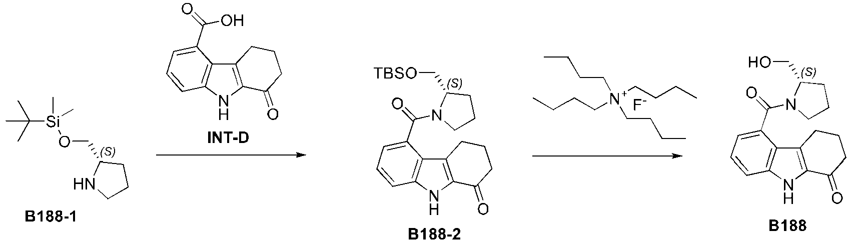

At room temperature, N,N-dimethylformamide (5 mL), N,N-dimethylaminopyridine (113.4 mg, 0.928 mmol), and 1-(3-dimethylaminopropyl)-3-ethylcarbodiimide hydrochloride (108.1 mg, 0.696 mmol) were added to a mixture of compound INT-D (161.9 mg, 0.696 mmol) and compound B188-1 (100 mg, 0.464 mmol) in dichloromethane (5 mL). The mixture was stirred overnight at room temperature. The reaction was quenched with water (20 mL) and extracted with ethyl acetate (3 × 30 mL). The combined organic layers were washed with saturated sodium chloride solution (50 mL), dried over anhydrous sodium sulfate, and concentrated under vacuum. The residue was purified by silica gel column chromatography (petroleum ether:ethyl acetate = 1:2) to afford compound B188-2 as a white solid (160 mg, 80.79%). ESI/MS (m/z): 427.2 [M+H]⁺.

At room temperature, a 1M solution of tetrabutylammonium fluoride in tetrahydrofuran (1.74 mL, 1.740 mmol) was added to a solution of compound B188-2 (150 mg, 0.348 mmol) in tetrahydrofuran (10 mL). The reaction mixture was stirred at room temperature for 2 hours, diluted with water (20 mL), and extracted with ethyl acetate (3 × 30 mL). The combined organic layers were washed with saturated sodium chloride solution (30 mL), dried over anhydrous sodium sulfate, and concentrated under vacuum. The residue was purified by reverse-phase chromatography (mobile phase A: water (10 mmol/L ammonium bicarbonate), mobile phase B: acetonitrile) to afford compound B188 as a white solid (31.9 mg, 29.34%). ESI/MS (m/z): 313.2 [M+H]⁺.¹H NMR (400 MHz, DMSO-*d*₆) δ 11.81 (s, 1H), 7.45-7.40 (m, 1H), 7.34-7.28 (m, 1H), 7.02-6.96 (m, 1H), 4.94-4.54 (m, 1H), 4.24-3.52 (m, 2H), 3.53-3.45 (m, 1H), 3.14-2.95 (m, 2H), 2.92-2.70 (m, 2H), 2.64-2.52 (m, 2H), 2.18-2.03 (m, 2H), 2.00-1.92 (m, 2H), 1.91-1.63 (m, 2H).

### Example 247: Synthesis of Compound B189

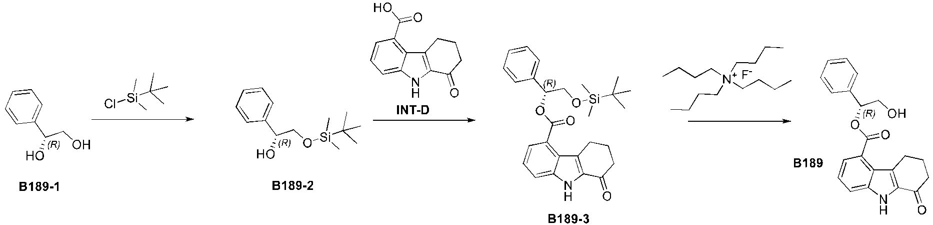

At 0°C, triethylamine (1.10 g, 10.857 mmol) and N,N-dimethylaminopyridine (0.09 g, 0.724 mmol) were added to a solution of compound B189-1 (1 g, 7.238 mmol) and tert-butyldimethylchlorosilane (1.31 g, 7.962 mmol) in dichloromethane (10 mL). The reaction mixture was stirred at room temperature for 3 hours, quenched with water (30 mL), and extracted with dichloromethane (3 × 30 mL). The combined organic layers were washed with saturated sodium chloride solution (30 mL), dried over anhydrous sodium sulfate, and concentrated under vacuum to afford compound B189-2 as a yellow oil (1.6 g, crude).

At room temperature, N,N-dimethylformamide (5 mL), 1-(3-dimethylaminopropyl)-3-ethylcarbodiimide (135.0 mg, 0.870 mmol), and N,N-dimethylaminopyridine (212.5 mg, 1.740 mmol) were added to a mixture of compound B189-2 (696.3 mg, 2.610 mmol) and compound INT-D (200 mg, 0.870 mmol) in dichloromethane (5 mL). The reaction mixture was stirred overnight at room temperature, quenched with water, and extracted with dichloromethane (3 × 30 mL). The combined organic layers were washed with saturated sodium chloride solution (30 mL), dried over anhydrous sodium sulfate, and concentrated under vacuum. The residue was purified by silica gel column chromatography (petroleum ether:ethyl acetate = 1:3) to afford compound B189-3 as a pale yellow solid (160 mg, 39.67%). ESI/MS (m/z): 464.3 [M+H]⁺.

At room temperature, a 1M solution of tetrabutylammonium fluoride in tetrahydrofuran (1.6 mL, 1.620 mmol) was added to a solution of compound B189-3 (150 mg, 0.324 mmol) in tetrahydrofuran (5 mL). The reaction mixture was stirred at room temperature for 12 hours, quenched with water (30 mL), and extracted with dichloromethane (3 × 30 mL). The combined organic layers were washed with saturated sodium chloride solution (20 mL), dried over anhydrous sodium sulfate, and concentrated under vacuum. The residue was purified by reverse-phase chromatography (mobile phase A: water (10 mmol/L ammonium bicarbonate), mobile phase B: acetonitrile) to afford compound B189 as a white solid (38.4 mg, 33.97%). ESI/MS (m/z): 350.0 [M+H]⁺.¹H NMR (400 MHz, DMSO-*d*₆) δ 7.65 (dd, *J* = 8.3, 1.1 Hz, 1H), 7.58 (dd, *J =* 7.3, 1.0 Hz, 1H), 7.48-7.43 (m, 2H), 7.40-7.33 (m, 3H), 7.31-7.26 (m, 1H), 5.73 (s, 1H), 4.97 (t, *J =* 5.8 Hz, 1H), 4.42-4.36 (m, 2H), 3.04-2.95 (m, 2H), 2.59-2.51 (m, 2H), 2.11-2.01 (m, 2H).

### Example 248: Synthesis of Compound B190

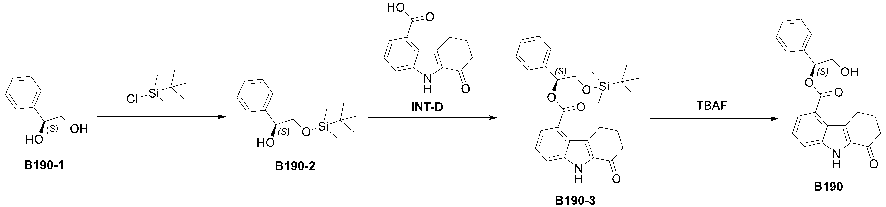

At 0°C, triethylamine (1.10 g, 10.857 mmol) was added to a solution of compound B190-1 (1.0 g, 7.238 mmol) and N,N-dimethylaminopyridine (0.09 g, 0.724 mmol) in dichloromethane (20 mL). To the above solution, tert-butyldimethylchlorosilane (1.20 g, 7.962 mmol) was then added. The reaction mixture was stirred at room temperature for 3 hours, diluted with water (40 mL), and extracted with dichloromethane (3 × 30 mL). The combined organic layers were washed with saturated sodium chloride solution (50 mL), dried over anhydrous sodium sulfate, and concentrated under vacuum to afford compound B190-2 as a yellow oil (1.5 g, crude). ESI/MS (m/z): 251.0 [M-H]⁻.

At room temperature, N,N-dimethylformamide (5 mL) and compound B190-2 (339.3 mg, 1.290 mmol) were added to a mixture of compound INT-D (200 mg, 0.860 mmol), 1-(3-dimethylaminopropyl)-3-ethylcarbodiimide hydrochloride (133.6 mg, 0.860 mmol), and N,N-dimethylaminopyridine (210.2 mg, 1.720 mmol) in dichloromethane (5 mL). The reaction mixture was stirred overnight at room temperature, quenched with water (20 mL), and extracted with dichloromethane (3 × 30 mL). The combined organic layers were washed with saturated sodium chloride solution (30 mL), dried over anhydrous sodium sulfate, and concentrated under vacuum. The residue was purified by silica gel column chromatography (petroleum ether:ethyl acetate = 4:1) to afford compound B190-3 as a pale yellow solid (200 mg, 50.14%). ESI/MS (m/z): 464.2 [M+H]⁺.

At room temperature, a 1M solution of tetrabutylammonium fluoride in tetrahydrofuran (2.0 mL, 2.03 mmol) was added to a solution of compound B190-3 (190 mg, 0.406 mmol) in tetrahydrofuran (10 mL). The reaction mixture was stirred at room temperature for 2 hours, diluted with water (20 mL), and extracted with ethyl acetate (3 × 30 mL). The combined organic layers were washed with saturated sodium chloride solution (30 mL), dried over anhydrous sodium sulfate, and concentrated under vacuum. The residue was purified by reverse-phase chromatography (mobile phase A: water (10 mmol/L ammonium bicarbonate), mobile phase B: acetonitrile) to afford compound B190 as a white solid (31.8 mg, 22.43%). ESI/MS (m/z): 350.0 [M+H]⁺.¹H NMR (400 MHz, DMSO-*d*₆) δ 11.93 (s, 1H), δ 7.65 (dd, *J =* 8.3, 1.1 Hz, 1H), 7.58 (dd, *J =* 7.3, 1.0 Hz, 1H), 7.49-7.42 (m, 2H), 7.40-7.33 (m, 3H), 7.32-7.26 (m, 1H), 5.73 (s, 1H), 4.97 (t, *J =* 5.8 Hz, 1H), 4.42-4.37 (m, 2H), 3.05-2.95 (m, 2H), 2.58-2.52 (m, 2H), 2.12-2.01 (m, 2H).

### Example 249: Synthesis of Compound B191

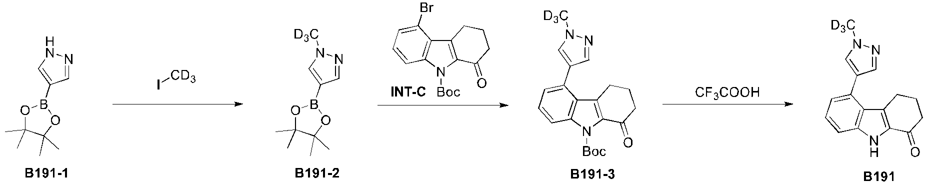

At room temperature, deuterated iodomethane (224 mg, 1.5 mmol) and cesium carbonate (671 mg, 2.06 mmol) were added to a solution of compound B191-1 (200 mg, 1.03 mmol) in N,N-dimethylformamide (1 mL). The mixture was stirred for 15 hours. The reaction mixture was diluted with ethyl acetate (30 mL), washed sequentially with water (10 mL) and saturated sodium chloride solution (10 mL), dried over anhydrous sodium sulfate, and concentrated under vacuum. The residue was purified by silica gel column chromatography (petroleum ether:ethyl acetate = 3:1) to afford compound B191-2 as a colorless oil (140 mg, 0.43 mmol). ESI/MS (m/z): 212.1 [M+H]⁺.

Under a nitrogen atmosphere, a mixture of compound INT-C (100 mg, 0.27 mmol) in 1,4-dioxane (2 mL) was sequentially treated with water (0.5 mL), compound B191-2 (140 mg, 0.43 mmol), [1,1'-bis(diphenylphosphino)ferrocene]dichloropalladium(II) dichloromethane complex (11 mg, 0.014 mmol), and potassium carbonate (76 mg, 0.55 mmol). The mixture was heated to 80 °C and stirred for 3 hours. After cooling to room temperature, the reaction mixture was diluted with ethyl acetate (30 mL), filtered through celite, and the filtrate was concentrated under vacuum to afford the crude compound B191-3. Trifluoroacetic acid (0.3 mL) and dichloromethane (3 mL) were added to compound B191-3, and the mixture was stirred for 2 hours. The reaction mixture was diluted with ethyl acetate (30 mL), washed sequentially with saturated aqueous sodium bicarbonate solution (10 mL) and saturated aqueous sodium chloride solution (10 mL), dried over anhydrous sodium sulfate, and concentrated under vacuum. The residue was purified by reverse-phase preparative liquid chromatography (acetonitrile/water: 10-40%) to afford compound B191 as a yellow solid (7.1 mg, 0.025 mmol). ESI/MS (m/z): 269.1 [M+H]⁺.¹H NMR (500 MHz, DMSO-*d*₆) δ 11.68 (s, 1H), 7.89 (d, *J =* 0.8 Hz, 1H), 7.59 (d, *J =* 0.8 Hz, 1H), 7.34 (dd, *J =* 8.3, 1.1 Hz, 1H), 7.26 (dd, *J =* 8.3, 7.0 Hz, 1H), 6.91 (dd, *J =* 7.0, 1.1 Hz, 1H), 2.74 (t, *J =* 5.9 Hz, 2H), 2.55-2.51 (m, 2H), 2.04-1.97 (m, 2H).

### Example 250: Synthesis of Compound B192

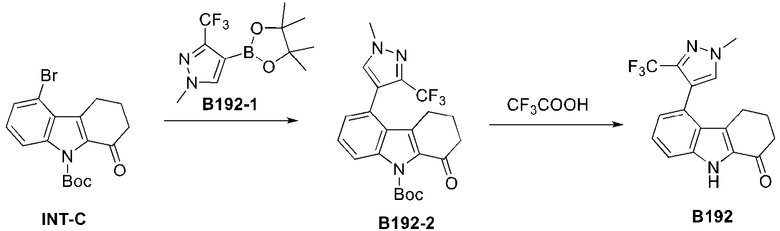

Under a nitrogen atmosphere, a mixture of compound INT-C (100 mg, 0.27 mmol) in 1,4-dioxane (2 mL) and water (0.5 mL) was sequentially treated with compound B192-1 (110 mg, 0.40 mmol), [1,1'-bis(diphenylphosphino)ferrocene]dichloropalladium(II) dichloromethane complex (11 mg, 0.014 mmol), and potassium carbonate (76 mg, 0.55 mmol). The reaction mixture was heated to 80 °C and stirred for 3 hours, then cooled to room temperature. The mixture was diluted with ethyl acetate (30 mL), filtered through celite, and the filtrate was concentrated under vacuum to afford compound B192-2. Trifluoroacetic acid (0.3 mL) and dichloromethane (3 mL) were added to compound B192-2, and the mixture was stirred at room temperature for 2 hours. The reaction mixture was diluted with ethyl acetate (30 mL), washed sequentially with saturated aqueous sodium bicarbonate solution (10 mL) and saturated aqueous sodium chloride solution (10 mL), dried over anhydrous sodium sulfate, and concentrated under vacuum. The residue was purified by reverse-phase preparative liquid chromatography (acetonitrile/water: 10-40%) to afford compound B192 as a yellow solid (38 mg, 0.11 mmol). ESI/MS (m/z): 334.0 [M+H]⁺.¹H NMR (500 MHz, DMSO-*d*₆) δ 11.77 (s, 1H), 8.04 (s, 1H), 7.43 (d, *J =* 8.3 Hz, 1H), 7.30 (dd, *J =* 8.4, 7.1 Hz, 1H), 6.90 (d, *J =* 7.0 Hz, 1H), 4.01 (s, 3H), 2.50-2.46 (m, 2H), 2.40 (t, *J =* 5.9 Hz, 2H), 1.98 (p, *J =* 6.1 Hz, 2H).

### Example 251: Synthesis of Compound B193

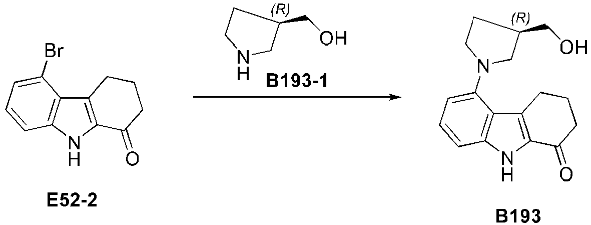

Under a nitrogen atmosphere, a solution of compound E52-2 (100 mg, 0.27 mmol) in 1,4-dioxane (2 mL) was sequentially treated with compound B193-1 (40 mg, 0.40 mmol), 2-dicyclohexylphosphino-2',6'-diisopropoxy-1,1'-biphenyl (14 mg, 0.03 mmol), (2-dicyclohexylphosphino-2',6'-diisopropoxy-1,1'-biphenyl)[2-(2'-amino-1,1'-biphenyl)]palladium(II) methanesulfonate (25 mg, 0.03 mmol), and sodium tert-butoxide (155 mg, 1.62 mmol). The mixture was heated to 90 °C and stirred for 3 hours. After cooling to room temperature, the reaction mixture was diluted with ethyl acetate (30 mL), washed sequentially with saturated aqueous sodium bicarbonate solution (10 mL) and saturated aqueous sodium chloride solution (10 mL), dried over anhydrous sodium sulfate, and concentrated under vacuum. The residue was purified by reverse-phase preparative liquid chromatography (acetonitrile/water: 10-35%) to afford compound B193 as a yellow solid (11 mg, 0.038 mmol). ESI/MS (m/z): 285.1 [M+H]⁺.¹H NMR (500 MHz, DMSO-*d*₆) δ 11.49 (s, 1H), 7.10 (t, *J =* 7.9 Hz, 1H), 6.94 (d, *J =* 8.1 Hz, 1H), 6.48 (d, *J =* 7.5 Hz, 1H), 4.66 (s, 1H), 3.48-3.39 (m, 2H), 3.20-3.14 (m, 4H), 3.11-3.06 (m, 1H), 3.00 (dd, *J =* 9.6, 5.4 Hz, 1H), 2.54-2.51 (m, 2H), 2.43-2.35 (m, 1H), 2.07 (p, *J =* 6.0 Hz, 2H), 2.03-1.97 (m, 1H), 1.62-1.55 (m, 1H).

### Example 252: Synthesis of Compound B194

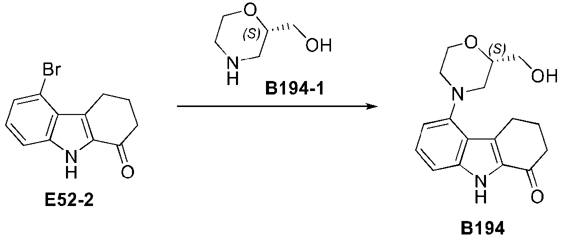

Under a nitrogen atmosphere, a solution of compound E52-2 (100 mg, 0.27 mmol) in 1,4-dioxane (2 mL) was sequentially treated with compound B194-1 (47 mg, 0.40 mmol), 2-dicyclohexylphosphino-2',6'-diisopropoxy-1,1'-biphenyl (14 mg, 0.03 mmol), (2-dicyclohexylphosphino-2',6'-diisopropoxy-1,1'-biphenyl)[2-(2'-amino-1,1'-biphenyl)]palladium(II) methanesulfonate (25 mg, 0.03 mmol), and sodium tert-butoxide (155 mg, 1.62 mmol). The mixture was heated to 90 °C and stirred for 3 hours. After cooling to room temperature, the reaction mixture was diluted with ethyl acetate (30 mL), washed sequentially with saturated aqueous sodium bicarbonate solution (10 mL) and saturated aqueous sodium chloride solution (10 mL), dried over anhydrous sodium sulfate, and concentrated under vacuum. The residue was purified by reverse-phase preparative liquid chromatography (acetonitrile/water: 10-35%) to afford compound B194 as a yellow solid (13 mg, 0.043 mmol). ESI/MS (m/z): 301.1 [M+H]⁺.¹H NMR (500 MHz, DMSO-*d*₆) δ 11.58 (s, 1H), 7.17 (t, *J =* 7.8 Hz, 1H), 7.05 (d, *J =* 8.2 Hz, 1H), 6.57 (d, *J =* 7.4 Hz, 1H), 4.74 (t, *J=* 5.8 Hz, 1H), 3.98-3.92 (m, 1H), 3.77 (td, *J=* 11.4, 2.5 Hz, 1H), 3.74-3.69 (m, 1H), 3.53-3.48 (m, 1H), 3.42-3.37 (m, 1H), 3.36-3.33 (m, 1H), 3.26-3.17 (m, 2H), 3.13 (dd, *J =* 11.9, 2.2 Hz, 1H), 2.79 (td, *J =* 11.6, 3.1 Hz, 1H), 2.55-2.51 (m, 2H), 2.46 (dd, *J =* 11.7, 9.9 Hz, 1H), 2.17-2.04 (m, 2H).

### Example 253: Synthesis of Compound B195

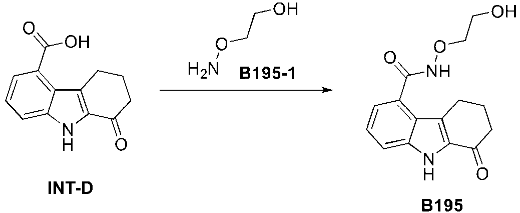

At room temperature, compound B195-1 (25.9 mg, 0.34 mmol), diisopropylethylamine (58.9 mg, 0.44 mmol), and 2-(7-azabenzotriazol-1-yl)-N,N,N',N'-tetramethyluronium hexafluorophosphate (93.6 mg, 0.25 mmol) were added to a mixture of compound INT-D (54 mg, 0.22 mmol) in N,N-dimethylformamide (1 mL). The mixture was stirred for 1 hour. The reaction was quenched with water (5 mL) and extracted with ethyl acetate (10 mL × 3). The combined organic layers were washed with saturated sodium chloride solution, dried over anhydrous sodium sulfate, and concentrated under vacuum. The residue was purified by preparative thin-layer chromatography (dichloromethane:methanol = 20:1) to afford compound B195 as a white solid (37.6 mg, 56.0%). ESI/MS (m/z): 289.0 [M+H]⁺.¹H NMR (500 MHz, DMSO-*d*₆) δ 11.86 (s, 1H), 11.55 (s, 1H), 7.52 (dd, *J =* 8.4, 0.9 Hz, 1H), 7.31 (dd, *J =* 8.3, 7.1 Hz, 1H), 7.14 (dd, *J =* 7.1, 1.0 Hz, 1H), 4.78 (t, *J =* 5.8 Hz, 1H), 3.97 (t, *J =* 5.0 Hz, 2H), 3.65 (q, *J =* 5.2 Hz, 2H), 2.93 (t, *J =* 6.0 Hz, 2H), 2.56 (dd, *J =* 7.3, 5.6 Hz, 2H), 2.15-2.08 (m, 2H).

### Example 254: Synthesis of Compound B196

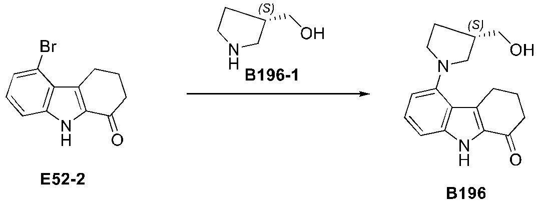

Under a nitrogen atmosphere, a solution of compound E52-2 (100 mg, 0.27 mmol) in 1,4-dioxane (2 mL) was sequentially treated with compound B196-1 (40 mg, 0.40 mmol), 2-dicyclohexylphosphino-2',6'-diisopropoxy-1,1'-biphenyl (14 mg, 0.03 mmol), (2-dicyclohexylphosphino-2',6'-diisopropoxy-1,1'-biphenyl)[2-(2'-amino-1,1'-biphenyl)]palladium(II) methanesulfonate (25 mg, 0.03 mmol), and sodium tert-butoxide (155 mg, 1.62 mmol). The mixture was heated to 90 °C and stirred for 3 hours. After cooling to room temperature, the reaction mixture was diluted with ethyl acetate (30 mL). The resulting mixture was washed sequentially with saturated aqueous sodium bicarbonate solution (10 mL) and saturated aqueous sodium chloride solution (10 mL), dried over anhydrous sodium sulfate, and concentrated under vacuum. The residue was purified by reverse-phase preparative liquid chromatography (acetonitrile/water: 10-35%) to afford compound B196 as a yellow solid (20 mg, 0.069 mmol). ESI/MS (m/z): 285.0 [M+H]⁺.¹H NMR (500 MHz, DMSO-*d*₆) δ 11.49 (s, 1H), 7.10 (t, *J =* 7.9 Hz, 1H), 6.94 (d, *J =* 8.1 Hz, 1H), 6.48 (d, *J* = 7.5 Hz, 1H), 4.65 (t, *J* = 5.2 Hz, 1H), 3.48-3.38 (m, 2H), 3.17 (q, *J* = 6.7, 6.1 Hz, 4H), 3.11-3.06 (m, 1H), 3.00 (dd, *J =* 9.5, 5.4 Hz, 1H), 2.55-2.51 (m, 2H), 2.44-2.35 (m, 1H), 2.07 (p, *J =* 6.2 Hz, 2H), 2.04-1.96 (m, 1H), 1.62-1.56 (m, 1H).

### Example 255: Synthesis of Compound B197

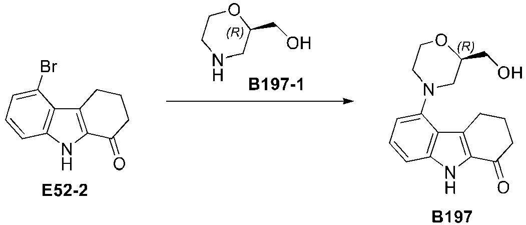

Under a nitrogen atmosphere, a solution of compound E52-2 (100 mg, 0.27 mmol) in 1,4-dioxane (2 mL) was sequentially treated with compound B197-1 (47 mg, 0.40 mmol), 2-dicyclohexylphosphino-2',6'-diisopropoxy-1,1'-biphenyl (14 mg, 0.03 mmol), (2-dicyclohexylphosphino-2',6'-diisopropoxy-1,1'-biphenyl)[2-(2'-amino-1,1'-biphenyl)]palladium(II) methanesulfonate (25 mg, 0.03 mmol), and sodium tert-butoxide (155 mg, 1.62 mmol). The mixture was heated to 90 °C and stirred for 3 hours. After cooling to room temperature, the reaction mixture was diluted with ethyl acetate (30 mL). The resulting organic phase was washed sequentially with saturated aqueous sodium bicarbonate solution (10 mL) and saturated aqueous sodium chloride solution (10 mL), dried over anhydrous sodium sulfate, and concentrated under vacuum. The residue was purified by reverse-phase preparative liquid chromatography (acetonitrile/water: 10-35%) to afford compound B197 as a yellow solid (19 mg, 0.064 mmol). ESI/MS (m/z): 301.1 [M+H]⁺.¹H NMR (500 MHz, DMSO-*d*₆) δ 11.59 (s, 1H), 7.17 (t, *J =* 7.9 Hz, 1H), 7.06 (d, *J =* 8.2 Hz, 1H), 6.57 (d, *J* = 7.3 Hz, 1H), 4.75 (t, *J =* 5.8 Hz, 1H), 3.97-3.92 (m, 1H), 3.77 (td, *J* = 11.2, 2.4 Hz, 1H), 3.74-3.69 (m, 1H), 3.55-3.47 (m, 1H), 3.43-3.37 (m, 1H), 3.35 (dd, *J =* 12.1, 2.3 Hz, 1H), 3.27-3.15 (m, 2H), 3.12 (dd, *J =* 11.7, 2.2 Hz, 1H), 2.79 (td, *J =* 11.6, 3.1 Hz, 1H), 2.55-2.51 (m, 2H), 2.46 (dd, *J =* 11.8, 10.0 Hz, 1H), 2.17-2.05 (m, 2H).

### Example 256: Synthesis of Compound B198

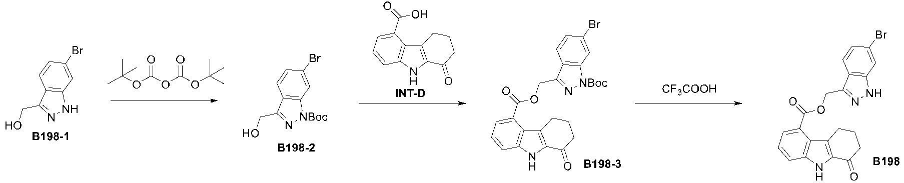

At room temperature, di-tert-butyl dicarbonate (384.5 mg, 1.762 mmol) was added to a solution of compound B198-1 (400 mg, 1.762 mmol) and N,N-dimethylaminopyridine (21.5 mg, 0.176 mmol) in tetrahydrofuran (10 mL). The reaction mixture was stirred at room temperature for 1 hour, quenched with water (10 mL), and extracted with dichloromethane (3 × 30 mL). The combined organic layers were washed with saturated sodium chloride solution (20 mL), dried over anhydrous sodium sulfate, and concentrated under vacuum. The residue was purified by silica gel column chromatography (petroleum ether:ethyl acetate = 5:1) to afford compound B198-2 as a white solid (300 mg, 52.05%). ESI/MS (m/z): 327.0 [M+H]⁺.

At room temperature, 1-(3-dimethylaminopropyl)-3-ethylcarbodiimide hydrochloride (87.8 mg, 0.565 mmol) and N,N-dimethylaminopyridine (138.2 mg, 1.130 mmol) were added to a solution of compound B198-2 (277.5 mg, 0.848 mmol) and compound INT-D (130 mg, 0.565 mmol) in N,N-dimethylformamide (5 mL). The reaction mixture was stirred overnight at room temperature, quenched with water (20 mL), and extracted with dichloromethane (3 × 20 mL). The combined organic layers were washed with saturated sodium chloride solution (20 mL), dried over anhydrous sodium sulfate, and concentrated under vacuum. The residue was purified by silica gel column chromatography (petroleum ether:ethyl acetate = 3:1) to afford compound B198-3 as a yellow solid (100 mg, 32.85%). ESI/MS (m/z): 540.4 [M+H]⁺.

At room temperature, trifluoroacetic acid (0.5 mL) was added dropwise to a solution of compound B198-3 (95 mg, 0.173 mmol) in dichloromethane (5 mL). The reaction mixture was stirred at room temperature for 1 hour and concentrated under vacuum. The residue was purified by reverse-phase chromatography (mobile phase A: water (0.1% formic acid), mobile phase B: acetonitrile) to afford compound B198 as a white solid (22.7 mg, 29.92%). ESI/MS (m/z): 440.0 [M+H]⁺.¹H NMR (400 MHz, DMSO-*d*₆) δ 13.34 (s, 1H), 12.01 (s, 1H), 7.88 (d, *J =* 8.6 Hz, 1H), 7.81 (d, *J =* 1.7 Hz, 1H), 7.65 (dd, *J =* 8.3, 1.1 Hz, 1H), 7.59 (dd, *J =* 7.4, 1.0 Hz, 1H), 7.38-7.29 (m, 2H), 5.74 (s, 2H), 3.00 (t, *J* = 6.0 Hz, 2H), 2.59-2.51 (m, 2H), 2.02 (p, *J =* 6.2 Hz, 2H).

### Example 257: Synthesis of Compound B199

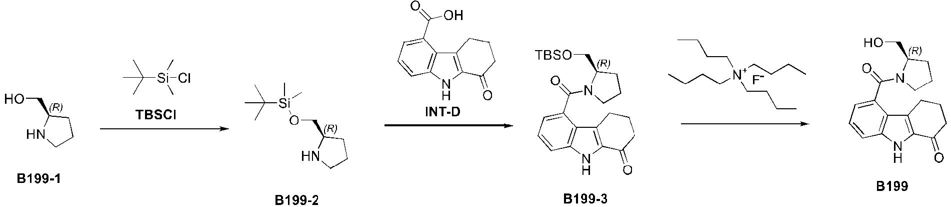

At 0°C, triethylamine (2.0 g, 19.772 mmol) and tert-butyldimethylchlorosilane (1.49 g, 9.886 mmol) were added to a solution of compound B199-1 (1 g, 9.886 mmol) and N,N-dimethylaminopyridine (0.12 g, 0.989 mmol) in dichloromethane (30 mL). The mixture was stirred at room temperature overnight. The reaction mixture was diluted with water (40 mL) and extracted with dichloromethane (3 × 30 mL). The combined organic layers were washed with saturated sodium chloride solution (50 mL), dried over anhydrous sodium sulfate, and concentrated under vacuum to afford compound B199-2 as a pale yellow oil (2.1 g, crude).

At room temperature, compound INT-D (374.8 mg, 1.740 mmol) was added to a solution of compound B199-2 (200 mg, 0.870 mmol), N,N-dimethylaminopyridine (212.5 mg, 1.740 mmol), and 1-(3-dimethylaminopropyl)-3-ethylcarbodiimide hydrochloride (135.0 mg, 0.870 mmol) in dichloromethane (5 mL) and N,N-dimethylformamide (5 mL). The reaction mixture was stirred overnight at room temperature, diluted with water (20 mL), and extracted with ethyl acetate (3 × 20 mL). The combined organic layers were washed with saturated sodium chloride solution (40 mL), dried over anhydrous sodium sulfate, and concentrated under vacuum. The residue was purified by silica gel column chromatography (petroleum ether:ethyl acetate = 1:2) to afford compound B199-3 as a pale yellow solid (150 mg, 40.42%). ESI/MS (m/z): 427.1 [M+H]⁺.

At room temperature, a 1M solution of tetrabutylammonium fluoride in tetrahydrofuran (1.72 mL, 1.725 mmol) was added to a solution of compound B199-3 (150 mg, 0.345 mmol) in tetrahydrofuran (10 mL). The mixture was stirred for 3 hours. The reaction mixture was diluted with water (20 mL) and extracted with ethyl acetate (3 × 30 mL). The combined organic layers were washed with saturated sodium chloride solution (40 mL), dried over anhydrous sodium sulfate, and concentrated under vacuum. The residue was purified by reverse-phase chromatography (mobile phase A: water (10 mmol/L ammonium bicarbonate), mobile phase B: acetonitrile) to afford compound B199 as a white solid (34.7 mg, 32.24%). ESI/MS (m/z): 313.2 [M+H]⁺.¹H NMR (400 MHz, DMSO-*d*₆) δ 11.81 (s, 1H), 7.43 (dt, *J* = 8.3, 1.2 Hz, 1H), 7.32 (ddd, *J* = 8.4, 7.0, 1.9 Hz, 1H), 7.02-6.97 (m, 1H), 4.98-4.54 (m, 1H), 4.25-3.57 (m, 2H), 3.56-3.45 (m, 1H), 3.14-2.95 (m, 2H), 2.92-2.70 (m, 2H), 2.64-2.52 (m, 2H), 2.19-2.04 (m, 2H), 2.00-1.92 (m, 2H), 1.91-1.62 (m, 2H).

### Example 258: Synthesis of Compound B200

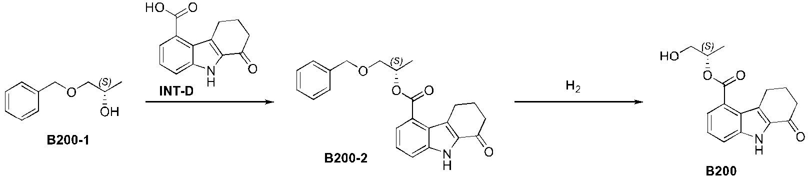

At room temperature, compound B200-1 (124.6 mg, 0.749 mmol) and 4-dimethylaminopyridine (114.5 mg, 0.937 mmol) were added to a mixture of compound INT-D (150.0 mg, 0.625 mmol) in dichloromethane (5 mL). After stirring uniformly, N,N'-dicyclohexylcarbodiimide (193.3 mg, 0.937 mmol) was added to the above solution. The mixture was heated to 40 °C and stirred overnight. The reaction mixture was diluted with ethyl acetate (100 mL), washed sequentially with water (30 mL × 3) and saturated sodium chloride solution (30 mL), dried over anhydrous sodium sulfate, and concentrated under vacuum. The residue was purified by normal-phase preparative thin-layer chromatography (methanol/dichloromethane) and reverse-phase preparative chromatography (acetonitrile/water) to afford compound B200-2 (120 mg). ESI/MS (m/z): 400.1 [M+Na]⁺.

Under a nitrogen atmosphere, 10% palladium on carbon (16.9 mg, 0.016 mmol) and trifluoroacetic acid (0.3 mL) were added to a solution of compound B200-2 (60.0 mg, 0.053 mmol) in ethyl acetate (3 mL). The reaction system was purged with hydrogen four times and stirred at room temperature for 1 hour. The mixture was filtered through celite, and the filter cake was washed with ethyl acetate. The combined filtrates were concentrated under vacuum, and the residue was purified by reverse-phase preparative liquid chromatography (acetonitrile/water) to afford compound B200 as a white solid (6 mg). ESI/MS (m/z): 288.0 [M+H]⁺.

### Example 259: Synthesis of Compound B201

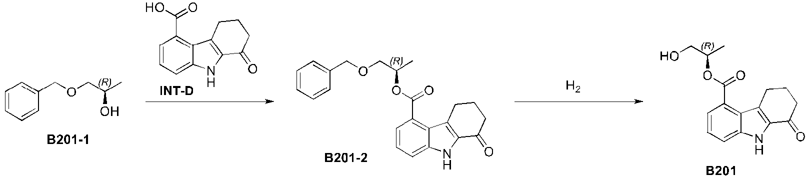

At room temperature, compound B201-1 (124.6 mg, 0.749 mmol) and 4-dimethylaminopyridine (114.5 mg, 0.937 mmol) were added to a mixture of compound INT-D (150.0 mg, 0.625 mmol) in dichloromethane (5 mL). After stirring uniformly, N,N'-dicyclohexylcarbodiimide (193.3 mg, 0.937 mmol) was added to the above solution. The mixture was heated to 40 °C and stirred overnight. The reaction mixture was diluted with ethyl acetate (100 mL), washed sequentially with water (30 mL × 3) and saturated sodium chloride solution (30 mL), dried over anhydrous sodium sulfate, and concentrated under vacuum. The residue was purified by normal-phase preparative thin-layer chromatography (methanol/dichloromethane) and reverse-phase preparative chromatography (acetonitrile/water) to afford compound B201-2 (133 mg). ESI/MS (m/z): 400.1 [M+Na]⁺.

Under a nitrogen atmosphere, 10% palladium on carbon (16.9 mg, 0.016 mmol) and trifluoroacetic acid (0.3 mL) were added to a solution of compound B201-2 (60.0 mg, 0.053 mmol) in ethyl acetate (3 mL). The reaction system was purged with hydrogen four times and stirred at room temperature for 1 hour. The mixture was filtered through celite, and the filter cake was washed with ethyl acetate. The combined filtrates were concentrated under vacuum, and the residue was purified by reverse-phase preparative liquid chromatography (acetonitrile/water) to afford compound B201 as a white solid (27.3 mg). ESI/MS (m/z): 288.0 [M+H]⁺.

### Example 260: Synthesis of Compound B202

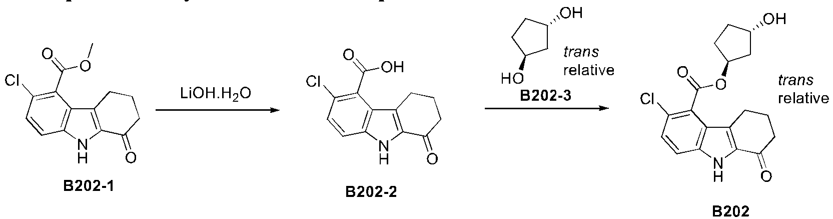

At room temperature, lithium hydroxide monohydrate (576.1 mg, 14.400 mmol) was added to a mixture of compound B202-1 (400 mg, 1.440 mmol) in methanol (10 mL) and water (2.5 mL). The mixture was heated to 60 °C and stirred for 72 hours. After cooling to room temperature, the reaction mixture was adjusted to pH 3 with dilute aqueous hydrochloric acid and extracted with dichloromethane (3 × 30 mL). The combined organic layers were washed with saturated sodium chloride solution (20 mL), dried over anhydrous sodium sulfate, and concentrated under vacuum. The residue was purified by silica gel column chromatography (dichloromethane:methanol = 3:1) to afford compound B202-2 as a yellow solid (300 mg, 78.99%). ESI/MS (m/z): 264.0 [M+H]⁺.

At 0 °C, triphenylphosphine (596.8 mg, 2.274 mmol) was added to a mixture of compound B202-2 (200 mg, 0.758 mmol) and compound B202-3 (154.9 mg, 1.516 mmol) in tetrahydrofuran (10 mL). The mixture was stirred for 5 minutes. Diethyl azodicarboxylate (396.3 mg, 2.274 mmol) was then added to the above reaction mixture, and stirring was continued at room temperature overnight. The reaction was quenched with water (20 mL) and extracted with ethyl acetate (3 × 20 mL). The combined organic layers were washed with saturated sodium chloride solution (20 mL), dried over anhydrous sodium sulfate, and concentrated under vacuum. The residue was purified by reverse-phase chromatography (mobile phase A: water (10 mmol/L ammonium bicarbonate), mobile phase B: acetonitrile) to afford compound B202 as a white solid (9.5 mg, 3.60%). ESI/MS (m/z): 346.0 [M-H]⁻.

### Example 261: Synthesis of Compound B203

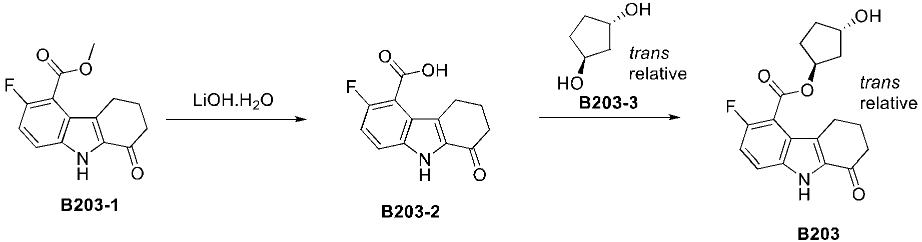

At room temperature, lithium hydroxide monohydrate (381.07 mg, 9.080 mmol) was added to a mixture of compound B203-1 (500 mg, 1.816 mmol) in methanol (5 mL) and water (5 mL). The mixture was heated to 60 °C and stirred for 4 hours. After cooling to room temperature, the reaction mixture was adjusted to pH 2 with dilute aqueous hydrochloric acid and extracted with dichloromethane (3 × 30 mL). The combined organic layers were washed with saturated sodium chloride solution (20 mL), dried over anhydrous sodium sulfate, and concentrated under vacuum. The residue was purified by silica gel column chromatography (dichloromethane:methanol = 3:1) to afford compound B203-2 as a yellow solid (420 mg, 93.53%). ESI/MS (m/z): 493.1 [2M-H]⁻.

At room temperature, compound B203-3 (153.1 mg, 1.498 mmol) was added to a solution of compound B203-2 (260 mg, 0.999 mmol), N,N-dimethylaminopyridine (146.5 mg, 1.199 mmol), and N,N'-dicyclohexylcarbodiimide (309.2 mg, 1.498 mmol) in N,N-dimethylformamide (8 mL). The reaction mixture was stirred overnight at room temperature, quenched with water (20 mL), and extracted with ethyl acetate (3 × 30 mL). The combined organic layers were washed with saturated sodium chloride solution (40 mL), dried over anhydrous sodium sulfate, and concentrated under vacuum. The residue was purified by reverse-phase chromatography (mobile phase A: water (10 mmol/L ammonium bicarbonate), mobile phase B: acetonitrile) to afford compound B203 as a white solid (26.5 mg, 8.01%). ESI/MS (m/z): 332.0 [M+H]⁺.¹H NMR (400 MHz, DMSO-*d*₆) δ 12.04 (s, 1H), 7.56 (dd, *J =* 9.0, 4.4 Hz, 1H), 7.25 (dd, *J =* 10.2, 8.9 Hz, 1H), 5.53-5.44 (m, 1H), 4.69 (s, 1H), 4.31-4.26 (m, 1H), 2.83 (t, *J* = 6.0 Hz, 2H), 2.62-2.53 (m, 2H), 2.31 -2.18 (m, 1H), 2.18-2.08 (m, 2H), 2.07-1.93 (m, 2H), 1.93-1.84 (m, 1H), 1.81-1.69 (m, 1H), 1.62-1.50 (m, 1H).

### Example 262: Synthesis of Compound B204

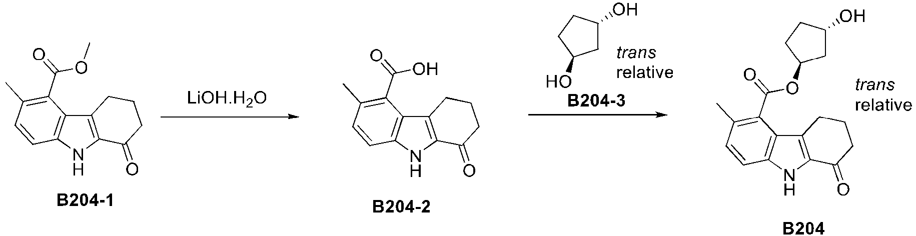

At room temperature, lithium hydroxide monohydrate (186.2 mg, 7.775 mmol) was added to a mixture of compound B204-1 (400 mg, 1.555 mmol) in methanol (5 mL) and water (5 mL). The mixture was heated to 60 °C and stirred for 72 hours. After cooling to room temperature, the reaction mixture was adjusted to pH 2 with dilute aqueous hydrochloric acid and extracted with dichloromethane (3 × 30 mL). The combined organic layers were washed with saturated sodium chloride solution (40 mL), dried over anhydrous sodium sulfate, and concentrated under vacuum. The residue was purified by silica gel column chromatography (dichloromethane: methanol = 3:1) to afford compound B204-2 as a pale-yellow solid (420 mg, 93.53%). ESI/MS (m/z): 244.1 [M+H]⁺.

At 0 °C, diethyl azodicarboxylate (404.0 mg, 2.319 mmol) was slowly added to a mixture of compound B204-2 (190 mg, 0.773 mmol), triphenylphosphine (608.4 mg, 2.319 mmol), and compound B204-3 (158.0 mg, 1.546 mmol) in tetrahydrofuran (8 mL). The reaction mixture was stirred overnight at room temperature, diluted with water (20 mL), and extracted with ethyl acetate (3 × 30 mL). The combined organic layers were washed with saturated sodium chloride solution (40 mL), dried over anhydrous sodium sulfate, and concentrated under vacuum. The residue was purified by reverse-phase chromatography (mobile phase A: water (10 mmol/L ammonium bicarbonate), mobile phase B: acetonitrile) to afford compound B204 as a white solid (40.5 mg, 16.00%). ESI/MS (m/z): 328.2 [M+H]⁺.

### Example 263: Synthesis of Compound C11

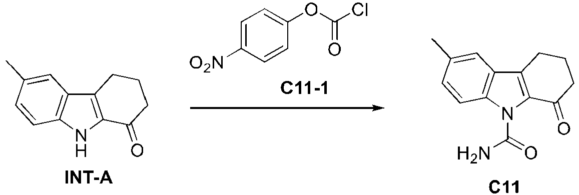

At 5°C, 60% sodium hydride (0.0601 g, 1.5 mmol) was added to a mixture of compound INT-A (0.100 g, 0.5 mmol) in tetrahydrofuran (5 mL), and the mixture was stirred for 5 minutes. A solution of compound C11-1 (201 mg, 0.750 mmol) in tetrahydrofuran (1 mL) was added dropwise to the reaction mixture, and stirring was continued for 0.5 hours. The reaction was quenched with water (10 mL) and extracted with ethyl acetate (3 × 10 mL). The combined organic layers were dried over anhydrous sodium sulfate and concentrated under vacuum. Ammonium bicarbonate (0.159 g, 2.0 mmol) was added to the residue in N,N-dimethylformamide (2 mL), and the mixture was heated to 50°C and stirred for 1 hour. After cooling to room temperature, the reaction mixture was diluted with water (10 mL) and extracted with ethyl acetate (10 mL × 3). The combined organic layers were dried over anhydrous sodium sulfate and concentrated under vacuum. The residue was purified by silica gel column chromatography (petroleum ether:ethyl acetate = 4:1) to afford compound C11 as a white solid (0.0311 g, 0.13 mmol). ESI/MS (m/z): 243.0 [M+H]⁺.¹H NMR (500 MHz, DMSO-*d₆*) δ 8.63 (br, 1H), 8.00 (d, *J =* 8.6 Hz, 1H), 7.60 (br, 1H), 7.54 (s, 1H), 7.32 (d, *J =* 8.5 Hz, 1H), 2.99 (t, *J =* 6.0 Hz, 2H), 2.70-2.61 (m, 2H), 2.42 (s, 3H), 2.19-2.09 (m, 2H).

### Example 264: Synthesis of Compound C12

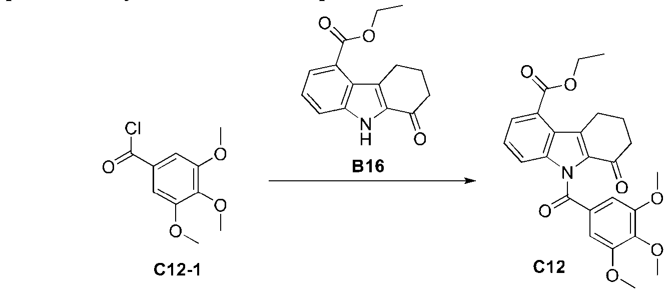

At 5°C, a solution of compound C12-1 (100 mg, 0.434 mmol) was slowly added dropwise to a mixture of compound B16 (93.9 mg, 0.347 mmol), 4-dimethylaminopyridine (26.5 mg, 0.217 mmol), and triethylamine (131.6 mg, 1.302 mmol) in dichloromethane (5 mL). The reaction mixture was stirred at room temperature for 1 hour and then concentrated under vacuum. The residue was purified by reverse-phase chromatography (mobile phase A: water (0.1% formic acid), mobile phase B: acetonitrile) to afford compound C12 as a white solid (24.8 mg, 12.62%). ESI/MS (m/z): 452.2 [M+H]⁺.¹H NMR (400 MHz, DMSO-*d₆*) δ 7.85 (d, *J =* 8.2 Hz, 1H), 7.75 (d, *J =* 7.1 Hz, 1H), 7.57 (dd, *J =* 8.5, 7.4 Hz, 1H), 6.95 (s, 2H), 4.42 (q, *J* = 7.1 Hz, 2H), 3.78-3.74 (m, 9H), 3.18-3.07 (m, 2H), 2.56-2.51 (m, 2H), 2.20-2.07 (m, 2H), 1.39 (t, *J =* 7.1 Hz, 3H).

### Example 265: Synthesis of Compound C13

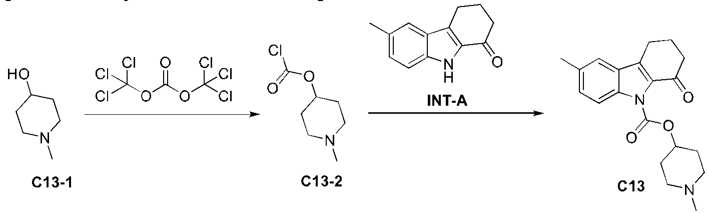

At 0°C, triphosgene (1.29 g, 4.341 mmol) was added to a solution of compound C13-1 (1 g, 8.682 mmol) and pyridine (1.40 mL, 17.364 mmol) in dichloromethane (10 mL). The reaction mixture was stirred at room temperature overnight. The mixture was filtered, and the resulting solid was washed with dichloromethane (10 mL) and dried under vacuum to afford compound C13-2 as a white solid (1 g, 64.84%).

At room temperature, 60% sodium hydride (20 mg, 0.828 mmol) was added to a solution of compound INT-A (150 mg, 0.753 mmol) in N,N-dimethylformamide (5 mL). The reaction mixture was stirred at room temperature for 30 minutes, and then compound C13-2 (200 mg, 1.129 mmol) was added. Stirring was continued overnight. The reaction was quenched with water (60 mL) and extracted with ethyl acetate (3 × 50 mL). The combined organic layers were washed with saturated sodium chloride solution (40 mL), dried over anhydrous sodium sulfate, and concentrated under vacuum. The residue was purified by reverse-phase preparative column chromatography (mobile phase A: water (0.1% formic acid), mobile phase B: acetonitrile) to afford the formate salt of compound C13 as a colorless oil (38.5 mg, 13.23%). ESI/MS (m/z): 341.2 [M+H]⁺.¹H NMR (400 MHz, DMSO-*d₆*) δ 8.19 (s, 1H), 7.83 (d, *J =* 8.5 Hz, 1H), 7.58 (s, 1H), 7.38 (dd, *J =* 8.6, 1.7 Hz, 1H), 4.95-4.89 (m, 1H), 2.96 (t, *J =* 6.0 Hz, 2H), 2.77-2.66 (m, 2H), 2.65-2.57 (m, 2H), 2.42 (s, 3H), 2.39 (d, *J =* 9.5 Hz, 2H), 2.28 (s, 3H), 2.16 (p, *J =* 6.2 Hz, 2H), 2.04-1.97 (m, 2H), 1.85-1.76 (m, 2H).

### Example 266: Synthesis of Compound C14

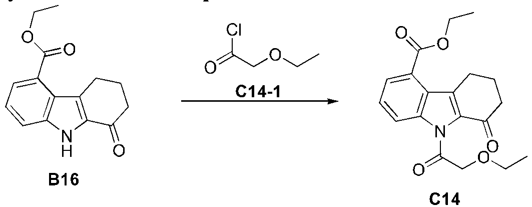

At 0°C, triethylamine (116.4 mg, 1.149 mmol) and compound C14-1 (70.5 mg, 0.575 mmol) were added to a solution of compound B16 (100 mg, 0.383 mmol) and N,N-dimethylaminopyridine (23.4 mg, 0.192 mmol) in dichloromethane (4 mL). The reaction mixture was stirred at room temperature for 1 hour, quenched with water (30 mL), and extracted with dichloromethane (3 × 40 mL). The combined organic layers were washed with saturated sodium chloride solution (30 mL), dried over anhydrous sodium sulfate, and concentrated under vacuum. The residue was purified by reverse-phase chromatography (mobile phase A: water (0.1% formic acid), mobile phase B: acetonitrile) to afford compound C14 as a white solid (11.9 mg, 8.84%). ESI/MS (m/z): 344.3 [M+H]⁺.¹H NMR (400 MHz, DMSO-*d₆*) δ 8.15 (d, *J =* 8.3 Hz, 1H), 7.73 (d, *J =* 7.4 Hz, 1H), 7.65-7.57 (m, 1H), 4.50 (s, 2H), 4.40 (q, *J =* 7.1 Hz, 2H), 3.27 (q, *J =* 7.0 Hz, 2H), 3.08-3.02 (m, 2H), 2.70-2.61 (m, 2H), 2.19-2.08 (m, 2H), 1.37 (t, *J =* 7.1 Hz, 3H), 1.00 (t, *J =* 7.0 Hz, 3H).

### Example 267: Synthesis of Compound C15

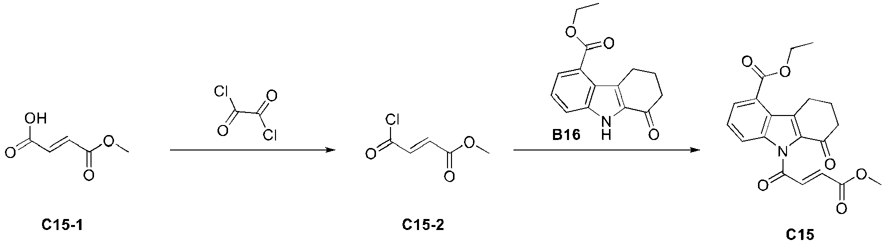

At room temperature, oxalyl chloride (585 mg, 4.61 mmol) was added to a solution of compound C15 (500 mg, 3.84 mmol) and N,N-dimethylformamide (50 µL) in dichloromethane (10 mL). The reaction mixture was stirred at room temperature for 3 hours and then concentrated under vacuum to afford compound C15-2 as a pale yellow oil (350 mg, 59.47%).

At 0°C, triethylamine (175 mg, 1.73 mmol) was added to a mixture of compound B16 (150 mg, 0.58 mmol) and 4-dimethylaminopyridine (35 mg, 0.29 mmol) in dichloromethane (10 mL). After stirring for 10 minutes, compound C15-2 (176 mg, 1.15 mmol) was added. The reaction mixture was warmed to room temperature and stirred for 2 hours, then concentrated under vacuum. The residue was purified by reverse-phase chromatography (mobile phase A: water (0.1% formic acid), mobile phase B: acetonitrile) to afford compound C15 as a pale yellow solid (88.2 mg, 41.29%). ESI/MS (m/z): 370.1 [M+H]⁺.¹H NMR (400 MHz, DMSO-*d₆*) δ 8.26 (dd, *J =* 8.5, 1.0 Hz, 1H), 7.75 (dd, *J =* 7.5, 1.0 Hz, 1H), 7.64 (dd, *J =* 8.5, 7.5 Hz, 1H), 7.27 (d, *J =* 15.4 Hz, 1H), 6.80 (d, *J =* 15.5 Hz, 1H), 4.40 (q, *J =* 7.1 Hz, 2H), 3.75 (s, 3H), 3.07 (t, *J =* 6.0 Hz, 2H), 2.69-2.62 (m, 2H), 2.21-2.12 (m, 2H), 1.37 (t, *J =* 7.1 Hz, 3H).

### Example 268: Synthesis of Compound C16

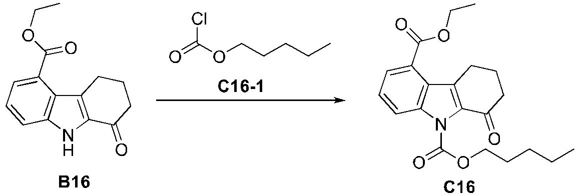

At 0°C, triethylamine (174.5 mg, 1.725 mmol) and compound C16-1 (129.9 mg, 0.862 mmol) were added to a mixture of compound B16 (150 mg, 0.575 mmol) and N,N-dimethylaminopyridine (35.1 mg, 0.287 mmol) in dichloromethane (4 mL). The reaction mixture was stirred at room temperature for 1 hour, quenched with water (20 mL), and extracted with dichloromethane (3 × 20 mL). The combined organic layers were washed with saturated sodium chloride solution (20 mL), dried over anhydrous sodium sulfate, and concentrated under vacuum. The residue was purified by reverse-phase chromatography (mobile phase A: water (0.1% formic acid), mobile phase B: acetonitrile) to afford compound C16 as a colorless oil (26.5 mg, 12.40%). ESI/MS (m/z): 372.3 [M+H]⁺.¹H NMR (400 MHz, DMSO-*d₆*) δ 8.12 (dd, *J* = 8.4, 1.0 Hz, 1H), 7.73 (dd, *J =* 7.4, 1.0 Hz, 1H), 7.62 (dd, *J =* 8.5, 7.5 Hz, 1H), 4.43-4.37 (m, 2H), 4.37-4.32 (m, 2H), 3.07-2.98 (m, 2H), 2.70-2.58 (m, 2H), 2.17-2.05 (m, 2H), 1.79-1.64 (m, 2H), 1.40-1.35 (m, 3H), 1.35-1.29 (m, 4H), 0.93-0.83 (m, 3H).

### Example 269: Synthesis of Compound C17

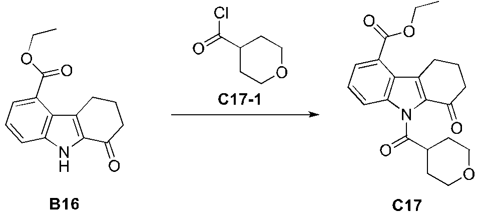

At 0°C, triethylamine (175 mg, 1.73 mmol) was added to a mixture of compound B16 (150 mg, 0.58 mmol) and 4-dimethylaminopyridine (35 mg, 0.29 mmol) in dichloromethane (10 mL). The reaction mixture was stirred at 0°C for 10 minutes, and then compound C17-1 (128 mg, 0.86 mmol) was added. The mixture was warmed to room temperature and stirred for 2 hours, then concentrated under vacuum. The residue was purified by reverse-phase chromatography (mobile phase A: water (0.1% formic acid), mobile phase B: acetonitrile) to afford compound C17 as a white solid (91.8 mg, 43.14%). ESI/MS (m/z): 370.3 [M+H]⁺.¹H NMR (400 MHz, DMSO-*d₆*) δ 7.94 (dd, *J =* 8.5, 1.0 Hz, 1H), 7.71 (dd, *J =* 7.4, 1.0 Hz, 1H), 7.59 (dd, *J =* 8.5, 7.4 Hz, 1H), 4.40 (q, *J =* 7.1 Hz, 2H), 3.87-3.79 (m, 2H), 3.31-324 (m, 2H), 3.23-3.14 (m, 1H), 3.08 (t, *J =* 6.0 Hz, 2H), 2.72-2.62 (m, 2H), 2.21-2.12 (m, 2H), 1.70-1.61 (m, 4H), 1.37 (t, *J =* 7.1 Hz, 3H).

### Example 270: Synthesis of Compound C18

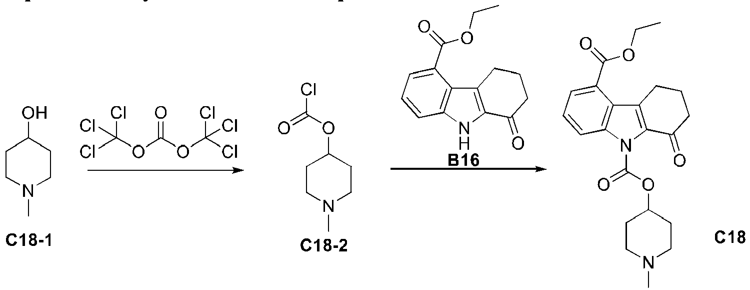

At room temperature, triphosgene (2.58 g, 8.682 mmol) was added to a solution of compound C18-1 (500 mg, 3.421 mmol) in acetonitrile (17 mL). The reaction mixture was stirred at room temperature for 5 hours and then filtered. The resulting filter cake was washed with n-hexane (3 × 10 mL) and dried under vacuum to afford compound C18-2 as a white solid (500 mg, crude).

At room temperature, triethylamine (232.7 mg, 2.298 mmol) and compound C18-2 (204.2 mg, 1.149 mmol) were added to a solution of compound B16 (200 mg, 0.766 mmol) and N,N-dimethylaminopyridine (46.8 mg, 0.383 mmol) in dichloromethane (8 mL). The reaction mixture was stirred at room temperature for 5 hours and then concentrated under vacuum. The residue was purified by reverse-phase chromatography (mobile phase A: water (0.1% formic acid), mobile phase B: acetonitrile) to afford the formate salt of compound C18 as a white solid (109.4 mg, 31.60%). ESI/MS (m/z): 399.1 [M+H]⁺.¹H NMR (400 MHz, DMSO-*d₆*) δ 8.27 (s, 1H), 8.14 (dd, *J =* 8.4,1.1 Hz, 1H), 7.72 (dd, *J =* 7.4, 1.1 Hz, 1H), 7.61 (dd, *J =* 8.5, 7.4 Hz, 1H), 5.03-4.95 (m, 1H), 4.40 (q, *J =* 7.1 Hz, 2H), 3.04 (t, *J =* 5.9 Hz, 2H), 2.79-2.69 (m, 2H), 2.68-2.61 (m, 2H), 2.50-2.42 (m, 2H), 2.32 (s, 3H), 2.18-2.07 (m, 2H), 2.09-2.00 (m, 2H), 1.90-1.79 (m, 2H), 1.37 (t, *J =* 7.1 Hz, 3H).

### Example 271: Synthesis of Compound C19

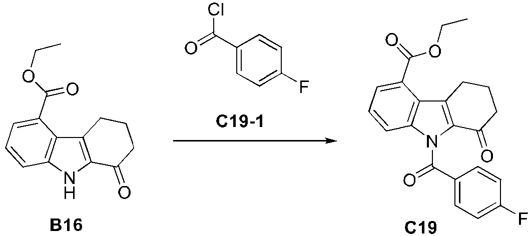

At 0°C, triethylamine (174.5 mg, 1.725 mmol) and compound C19-1 (136.7 mg, 0.862 mmol) were added to a mixture of compound B16 (150 mg, 0.575 mmol) and N,N-dimethylaminopyridine (35.1 mg, 0.287 mmol) in dichloromethane (4 mL). The reaction mixture was stirred at room temperature for 1 hour, quenched with water (25 mL), and extracted with dichloromethane (3 × 30 mL). The combined organic layers were washed with saturated sodium chloride solution (25 mL), dried over anhydrous sodium sulfate, and concentrated under vacuum. The residue was purified by reverse-phase chromatography (mobile phase A: water (0.1% formic acid), mobile phase B: acetonitrile) to afford compound C19 as a white solid (48.7 mg, 22.26%). ESI/MS (m/z): 380.2 [M+H]⁺.¹H NMR (400 MHz, DMSO-*d₆*) δ 7.88 (dd, *J =* 8.5*,* 1.0 Hz, 1H), 7.76 (dd, *J =* 7.4, 1.0 Hz, 1H), 7.74-7.71 (m, 1H), 7.71-7.68 (m, 1H), 7.58 (dd, *J =* 8.5*,* 7.4 Hz, 1H), 7.36-7.28 (m, 2H), 4.42 (q, *J =* 7.1 Hz, 2H), 3.18-3.06 (m, 2H), 2.50-2.45 (m, 2H), 2.19-2.09 (m, 2H), 1.39 (t, *J =* 7.1 Hz, 3H).

### Example 272: Synthesis of Compound C20

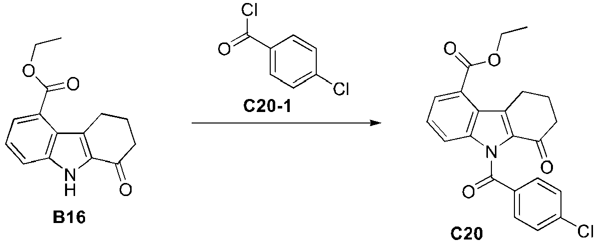

At room temperature, triethylamine (174.5 mg, 1.725 mmol) and compound C20-1 (150.9 mg, 0.862 mmol) were added to a mixture of compound B16 (150 mg, 0.575 mmol) and N,N-dimethylaminopyridine (35.1 mg, 0.287 mmol) in dichloromethane (5 mL). The reaction mixture was stirred at room temperature for 2 hours and then concentrated under vacuum. The residue was purified by reverse-phase chromatography (mobile phase A: water (0.1% formic acid), mobile phase B: acetonitrile) to afford compound C20 as a white solid (70.6 mg, 30.93%). ESI/MS (m/z): 396.1 [M+H]⁺.¹H NMR (400 MHz, DMSO-*d₆*) δ 7.93 (d, *J =* 8.4 Hz, 1H), 7.77 (d, *J =* 7.4 Hz, 1H), 7.63 (d, *J =* 8.3 Hz, 2H), 7.61-7.56 (m, 1H), 7.54 (d, *J =* 8.3 Hz, 2H), 4.47-4.39 (m, 2H), 3.13 (t, *J =* 6.0 Hz, 2H), 2.50-2.44 (m, 2H), 2.18-2.09 (m, 2H), 1.40 (t, *J =* 7.1 Hz, 3H).

### Example 273: Synthesis of Compound C21

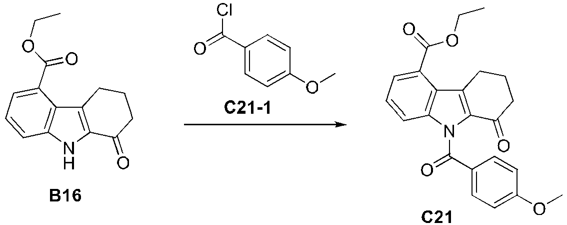

At room temperature, N,N-dimethylaminopyridine (42 mg, 0.345 mmol) and triethylamine (209 mg, 2.070 mmol) were added to a mixture of compound B16 (180 mg, 0.690 mmol) and compound C21-1 (177 mg, 1.035 mmol) in dichloromethane (10 mL). The reaction mixture was stirred at room temperature for 1 hour and then concentrated under vacuum. The residue was purified by reverse-phase chromatography (mobile phase A: water (0.1% formic acid), mobile phase B: acetonitrile) to afford compound C21 as a white solid (83.4 mg, 30.73%). ESI/MS (m/z): 392.2 [M+H]⁺.¹H NMR (400 MHz, DMSO-*d₆*) δ 7.76-7.68 (m, 2H), 7.60 (d, *J* = 8.5 Hz, 2H), 7.53 (dd, *J* = 8.5, 7.3 Hz, 1H), 7.03 (d, *J =* 9.1 Hz, 2H), 4.42 (q, *J =* 7.1 Hz, 2H), 3.84 (s, 3H), 3.13 (t, *J =* 6.0 Hz, 2H), 2.55-2.51 (m, 2H), 2.20-2.09 (m, 2H), 1.39 (t, *J =* 7.1 Hz, 3H).

### Example 274: Synthesis of Compound C22

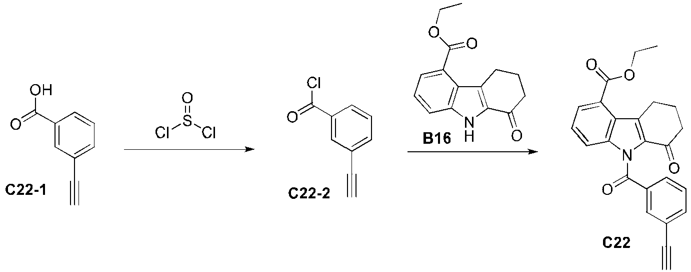

At room temperature, thionyl chloride (5 mL) was added to compound C22-1 (500 mg, 3.421 mmol). The reaction mixture was stirred at 50 °C for 2 hours and then concentrated under vacuum to afford compound C22-2 as a yellow oil (500 mg, crude).

At room temperature, triethylamine (775.6 mg, 7.660 mmol) and compound C22-2 (313.3 mg, 1.149 mmol) were added to a mixture of compound B16 (200 mg, 0.766 mmol) and N,N-dimethylaminopyridine (46.8 mg, 0.383 mmol) in dichloromethane (8 mL). The reaction mixture was stirred at room temperature for 5 hours and then concentrated under vacuum. The residue was purified by reverse-phase chromatography (mobile phase A: water (0.1% formic acid), mobile phase B: acetonitrile) to afford compound C22 as a white solid (86.4 mg, 29.10%). ESI/MS (m/z): 386.1 [M+H]⁺.¹H NMR (400 MHz, DMSO-*d₆*) δ 7.94 (dd, *J =* 8.5*,* 1.0 Hz, 1H), 7.78 (dd, *J =* 7.4, 1.1 Hz, 1H), 7.76-7.72 (m, 1H), 7.69 (d, *J =* 1.8 Hz, 1H), 7.63-7.57 (m, 2H), 7.54-7.46 (m, 1H), 4.43 (q, *J =* 7.1 Hz, 2H), 4.33 (s, 1H), 3.13 (t, *J =* 5.9 Hz, 2H), 2.50-2.42 (m, 2H), 2.17-2.09 (m, 2H), 1.39 (t, *J =* 7.1 Hz, 3H).

### Example 275: Synthesis of Compound C23

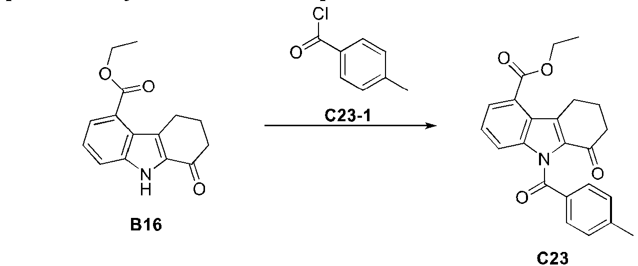

At 0°C, triethylamine (175 mg, 1.73 mmol) was added to a solution of compound B16 (150 mg, 0.58 mmol) and 4-dimethylaminopyridine (35 mg, 0.29 mmol) in dichloromethane (10 mL). The reaction mixture was stirred at 0°C for 10 minutes, and then compound C23-1 (133 mg, 0.86 mmol) was added. The mixture was stirred at room temperature for 2 hours and then concentrated under vacuum. The residue was purified by reverse-phase chromatography (mobile phase A: water (10 mmol/L ammonium bicarbonate), mobile phase B: methanol) to afford compound C23 as a white solid (83.8 mg, 38.36%). ESI/MS (m/z): 376.2 [M+H]⁺.¹H NMR (400 MHz, DMSO-*d*₆) δ 7.80 (dd, *J =* 8.5, 1.0 Hz, 1H), 7.75 (dd, *J* = 7.5, 1.0 Hz, 1H), 7.55 (dd, *J* = 8.5, 7.4 Hz, 1H), 7.51 (d, *J =* 7.9 Hz, 2H), 7.30 (d, *J =* 8.0 Hz, 2H), 4.42 (q, *J =* 7.1 Hz, 2H), 3.13 (t, *J =* 6.0 Hz, 2H), 2.49-2.46 (m, 2H), 2.38 (s, 3H), 2.18-2.09 (m, 2H), 1.39 (t, *J =* 7.1 Hz, 3H).

### Example 276: Synthesis of Compound C24

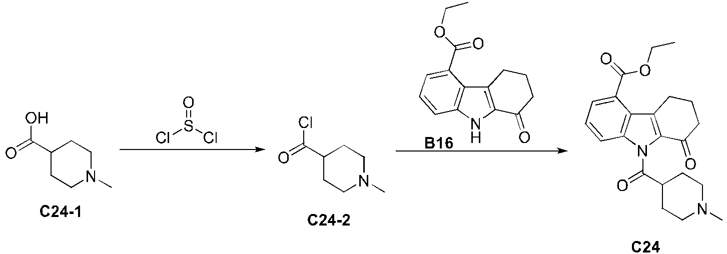

At room temperature, thionyl chloride (5 mL) was added to compound C24-1 (500 mg, 3.492 mmol). The reaction mixture was stirred at 50 °C for 3 hours, cooled to room temperature, and concentrated under vacuum. The residue was obtained as a white solid, compound C24-2 (500 mg, crude).

At room temperature, triethylamine (773.2 mg, 7.640 mmol) and compound C24-2 (188.7 mg, 1.146 mmol) were added to a mixture of compound B16 (200 mg, 0.764 mmol) and N,N-dimethylaminopyridine (46.8 mg, 0.382 mmol) in dichloromethane (8 mL). The reaction mixture was stirred at room temperature overnight and then concentrated under vacuum. The residue was purified by reverse-phase chromatography (mobile phase A: water (10 mmol/L ammonium bicarbonate), mobile phase B: acetonitrile) to afford compound C24 as a white solid (21 mg, 7.06%). ESI/MS (m/z): 383.2 [M+H]⁺.¹H NMR (400 MHz, DMSO-*d₆*) δ 7.93 (dd, *J* = 8.5, 1.0 Hz, 1H), 7.71 (dd, *J =* 7.4, 1.0 Hz, 1H), 7.58 (dd, *J =* 8.5*,* 7.4 Hz, 1H), 4.40 (q, *J =* 7.1 Hz, 2H), 3.08 (t, *J =* 6.0 Hz, 2H), 2.90-2.78 (m, 1H), 2.75-2.69 (m, 2H), 2.69-2.63 (m, 2H), 2.21-2.13 (m, 2H), 2.11 (s, 3H), 1.86-1.75 (m, 2H), 1.74-1.58 (m, 4H), 1.37 (t, *J =* 7.1 Hz, 3H).

### Example277: Synthesis of Compound C25

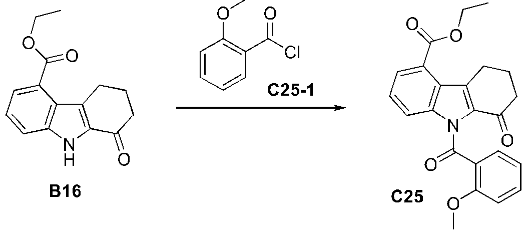

At room temperature, triethylamine (232.7 mg, 2.298 mmol) and compound C25-1 (130.8 mg, 0.766 mmol) were added to a mixture of compound B16 (200 mg, 0.766 mmol) and N,N-dimethylaminopyridine (46.8 mg, 0.383 mmol) in dichloromethane (8 mL). The reaction mixture was stirred at room temperature for 5 hours and then concentrated under vacuum. The residue was purified by reverse-phase chromatography (mobile phase A: water (10 mmol/L ammonium bicarbonate), mobile phase B: acetonitrile) to afford compound C25 as a white solid (25.5 mg, 8.47%). ESI/MS (m/z): 392.2 [M+H]⁺.¹H NMR (400 MHz, DMSO-*d₆*) δ 8.04 (d, *J =* 8.4 Hz, 1H), 7.75 (d, *J =* 7.5 Hz, 1H), 7.59 (dd, *J =* 8.5, 7.4 Hz, 1H), 7.55-7.49 (m, 1H), 7.36 (dd, *J =* 7.7*,* 1.8 Hz, 1H), 7.07 (d, *J =* 8.4 Hz, 1H), 7.02-6.96 (m, 1H), 4.41 (q, *J =* 7.1 Hz, 2H), 3.60 (s, 3H), 3.07 (t, *J =* 6.0 Hz, 2H), 2.42-2.34 (m, 2H), 2.07-1.99 (m, 2H), 1.38 (t, *J =* 7.1 Hz, 3H).

### Example 278: Synthesis of Compound C26

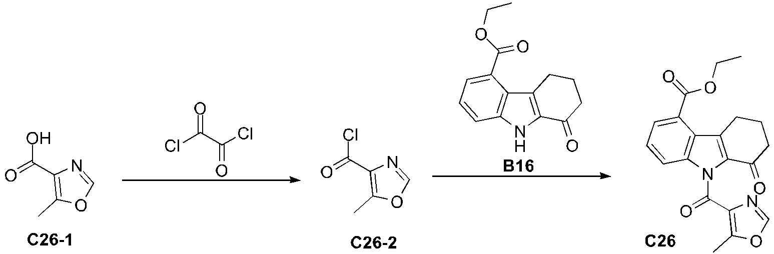

At 0°C, N,N-dimethylformamide (one drop) and oxalyl chloride (399.5 mg, 3.148 mmol) were added dropwise to a solution of compound C26-1 (100 mg, 0.787 mmol) in dichloromethane (3 mL). The reaction mixture was stirred at room temperature for 2 hours and then concentrated under vacuum to afford compound C26-2 as a yellow oil (100 mg, crude).

At 0°C, a solution of compound C26-2 (100 mg, crude) in dichloromethane (0.5 mL) was slowly added dropwise to a mixture of compound B16 (141.7 mg, 0.551 mmol), 4-dimethylaminopyridine (48.1 mg, 0.394 mmol), and triethylamine (238.9 mg, 2.361 mmol) in dichloromethane (5 mL). The reaction mixture was stirred at room temperature for 2 hours, quenched with water (20 mL), and extracted with dichloromethane (3 × 20 mL). The combined organic layers were washed with saturated sodium chloride solution (10 mL), dried over anhydrous sodium sulfate, and concentrated under vacuum. The residue was purified by reverse-phase chromatography (mobile phase A: water (0.1% formic acid), mobile phase B: acetonitrile) to afford compound C26 as a white solid (26.6 mg, 9.17%). ESI/MS (m/z): 367.0 [M+H]⁺.¹H NMR (400 MHz, DMSO-*d₆*) δ 8.28 (s, 1H), 8.03 (dd, *J =* 8.4, 1.0 Hz, 1H), 7.74 (dd, *J =* 7.5, 1.0 Hz, 1H), 7.58 (dd, *J =* 8.4, 7.4 Hz, 1H), 4.41 (q, *J =* 7.1 Hz, 2H), 3.13-3.04 (m, 2H), 2.68 (s, 3H), 2.58-2.51 (m, 2H), 2.19-2.08 (m, 2H), 1.38 (t, *J =* 7.1 Hz, 3H).

### Example 279: Synthesis of Compound C27

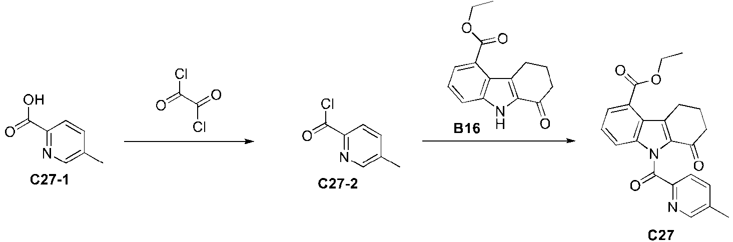

At room temperature, oxalyl chloride (463 mg, 3.6 mmol) was added dropwise to a solution of compound C27-1 (500 mg, 3.6 mmol) and N,N-dimethylformamide (30 µL) in dichloromethane (10 mL). The reaction mixture was stirred at 35 °C for 4 hours and then concentrated under vacuum to afford compound C27-2 as a brownish-yellow oil (450 mg, crude).

At 0 °C, triethylamine (175 mg, 1.73 mmol) was added to a mixture of compound B16 (150 mg, 0.58 mmol) and 4-dimethylaminopyridine (35 mg, 0.29 mmol) in dichloromethane (10 mL). The reaction mixture was stirred at 0 °C for 10 minutes, and then compound C27-2 (135 mg, 0.86 mmol) was added. The mixture was stirred at room temperature for 2 hours and then concentrated under vacuum. The residue was purified by reverse-phase chromatography (mobile phase A: water (10 mmol/L ammonium bicarbonate), mobile phase B: acetonitrile) to afford compound C27 as a white solid (54.5 mg, 24.91%). ESI/MS (m/z): 377.2 [M+H]⁺.¹H NMR (400 MHz, Chloroform-d) δ 8.32 (d, *J =* 8.4 Hz, 1H), 8.27-8.22 (m, 1H), 8.12 (d, *J =* 8.0 Hz, 1H), 7.81 (d, *J =* 7.4 Hz, 1H), 7.70 (dd, *J =* 8.0, 2.1 Hz, 1H), 7.50 (t, *J =* 8.0 Hz, 1H), 4.45 (q, *J =* 7.1 Hz, 2H), 3.23 (t, *J =* 6.0 Hz, 2H), 2.51-2.45 (m, 2H), 2.39 (s, 3H), 2.25-2.17 (m, 2H), 1.45 (t, *J =* 7.1 Hz, 3H).

### Example 280: Synthesis of Compound C28

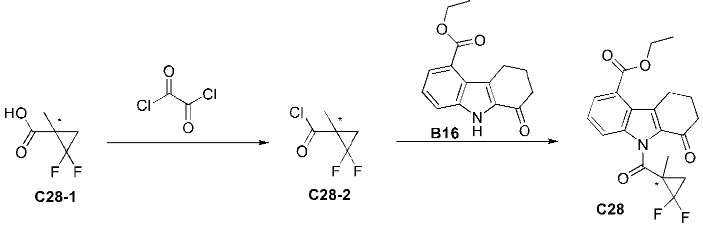

At 0°C, N,N-dimethylformamide (one drop) and oxalyl chloride (373.1 mg, 2.940 mmol) were added dropwise to a solution of compound C28-1 (100 mg, 0.735 mmol, racemate) in dichloromethane (3 mL). The reaction mixture was stirred at room temperature for 2 hours and then concentrated under vacuum to afford compound C28-2 as a yellow oil (100 mg, crude, racemate).

At 0°C, a solution of compound C28-2 (100 mg, crude) in dichloromethane (0.5 mL) was slowly added dropwise to a mixture of compound B16 (159.2 mg, 0.588 mmol), 4-dimethylaminopyridine (44.9 mg, 0.367 mmol), and triethylamine (223.1 mg, 2.205 mmol) in dichloromethane (5 mL). The reaction mixture was stirred at room temperature for 2 hours, quenched with water (20 mL), and extracted with dichloromethane (3 × 20 mL). The combined organic layers were washed with saturated sodium chloride solution (10 mL), dried over anhydrous sodium sulfate, and concentrated under vacuum. The residue was purified by reverse-phase chromatography (mobile phase A: water (0.1% formic acid), mobile phase B: acetonitrile) to afford compound C28 as a white solid (51.6 mg, 18.65%, racemate). ESI/MS (m/z): 376.0 [M+H]⁺.¹H NMR (400 MHz, DMSO-*d₆*) δ 7.84 (d, *J =* 8.4 Hz, 1H), 7.73 (d, *J =* 7.3 Hz, 1H), 7.65-7.57 (m, 1H), 4.40 (q, *J =* 7.1 Hz, 2H), 3.24-3.12 (m, 1H), 3.05-2.93 (m, 1H), 2.86-2.74 (m, 1H), 2.64-2.52 (m, 1H), 2.24-2.14 (m, 2H), 2.14-2.06 (m, 1H), 1.91-1.79 (m, 1H), 1.49 (s, 3H), 1.37 (t, *J =* 7.1 Hz, 3H).

### Example 281: Synthesis of Compound C29

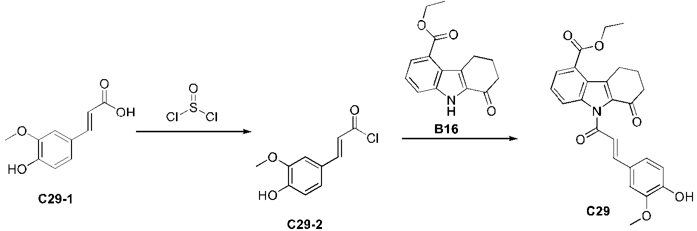

At room temperature, compound C29-1 (500 mg, 3.492 mmol) was added to thionyl chloride (8 mL) and the mixture was heated to 50 °C with stirring for 2 hours. The reaction mixture was concentrated under vacuum to afford compound C29-2 as a yellow solid (500 mg, crude).

At room temperature, triethylamine (232.7 mg, 2.298 mmol) and compound C29-2 (244.5 mg, 1.149 mmol) were added to a mixture of compound B16 (200 mg, 0.766 mmol) and N,N-dimethylaminopyridine (46.8 mg, 0.383 mmol) in dichloromethane (8 mL). The reaction mixture was stirred at room temperature for 5 hours and then concentrated under vacuum. The residue was purified by reverse-phase chromatography (mobile phase A: water (10 mmol/L ammonium bicarbonate), mobile phase B: acetonitrile) to afford compound C29 as a yellow solid (32 mg, 9.60%). ESI/MS (m/z): 434.1 [M+H]⁺.¹H NMR (400 MHz, DMSO-*d₆*) δ 9.65 (br, 1H), 8.13 (dd, *J =* 8.5, 1.0 Hz, 1H), 7.71 (dd, *J =* 7.5, 1.0 Hz, 1H), 7.64 (d, *J* = 15.5 Hz, 1H), 7.58 (dd, *J =* 8.5, 7.4 Hz, 1H), 7.32 (d, *J =* 2.0 Hz, 1H), 7.21 (dd, *J =* 8.3, 2.0 Hz, 1H), 6.89 (d, *J =* 15.6 Hz, 1H), 6.82 (d, *J =* 8.2 Hz, 1H), 4.41 (q, *J =* 7.1 Hz, 2H), 3.81 (s, 3H), 3.10 (t, *J =* 6.0 Hz, 2H), 2.70-2.62 (m, 2H), 2.25-2.14 (m, 2H), 1.38 (t, *J =* 7.1 Hz, 3H).

### Example 282: Synthesis of Compound C30

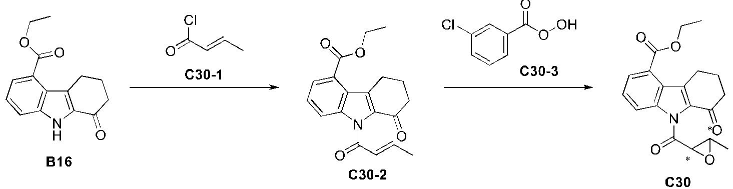

At 0°C, 60% sodium hydride (184 mg, 4.60 mmol) was added to a solution of compound B16 (800 mg, 3.07 mmol) in tetrahydrofuran (30 mL), and the mixture was stirred for 0.5 hours. Compound C30-1 (481 mg, 4.60 mmol) was added to the above reaction mixture, and stirring was continued at room temperature for 2 hours. The reaction was cooled to 0°C, quenched with water (20 mL), and extracted with ethyl acetate (3 × 30 mL). The combined organic layers were washed with saturated sodium chloride solution (50 mL), dried over anhydrous sodium sulfate, and concentrated under vacuum. The residue was purified by silica gel column chromatography (petroleum ether:ethyl acetate = 4:1) to afford compound C30-2 as a white solid (450 mg, 41.05%). ESI/MS (m/z): 326.00 [M+H]⁺.

At room temperature, compound C30-3 (217 mg, 1.26 mmol) was added to a solution of compound C30-2 (300 mg, 0.84 mmol) in chloroform (25 mL). The mixture was heated to 50°C and stirred overnight. After cooling to room temperature, the reaction was quenched with saturated aqueous sodium thiosulfate solution (20 mL) and extracted with dichloromethane (3 × 30 mL). The combined organic layers were washed with saturated sodium chloride solution (50 mL), dried over anhydrous sodium sulfate, and concentrated under vacuum. The residue was purified by reverse-phase chromatography (mobile phase A: water (0.1% formic acid), mobile phase B: acetonitrile) to afford compound C30 as a white solid (15.1 mg, 5.23%). ESI/MS (m/z): 342.0 [M+H]⁺.¹H NMR (400 MHz, DMSO-*d₆*) δ 8.14 (dd, *J =* 8.5, 1.1 Hz, 1H), 7.74 (dd, *J* = 7.5, 1.1 Hz, 1H), 7.64-7.58 (m, 1H), 4.40 (q, *J =* 7.1 Hz, 2H), 3.80 (d, *J =* 1.9 Hz, 1H), 3.28-3.22 (m, 1H), 3.14-2.96 (m, 2H), 2.76-2.67 (m, 1H), 2.66-2.56 (m, 1H), 2.21-2.12 (m, 2H), 1.40-1.35 (m, 3H), 1.32 (d, *J =* 5.1 Hz, 3H).

### Example 283: Synthesis of Compound C31

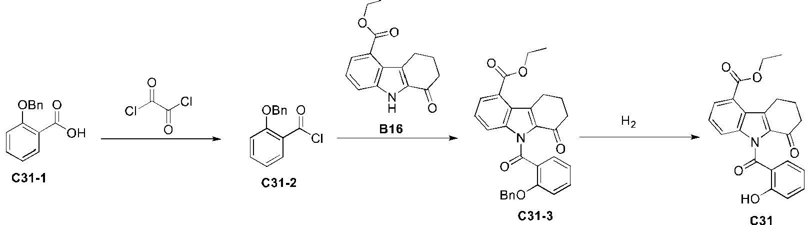

At room temperature, oxalyl chloride (0.97 mL, 11.393 mmol) was added to a solution of compound C31-1 (500 mg, 2.191 mmol) in dichloromethane (10 mL), and the mixture was stirred for 2 hours. The reaction mixture was concentrated under vacuum to afford compound C31-2 as a yellow solid (500 mg, crude).

At room temperature, triethylamine (232.7 mg, 2.298 mmol) and compound C31-2 (283.6 mg, 1.149 mmol) were added to a mixture of compound B16 (200 mg, 0.766 mmol) and N,N-dimethylaminopyridine (46.8 mg, 0.383 mmol) in dichloromethane (10 mL). The reaction mixture was stirred at room temperature for 2 hours and then concentrated under vacuum. The residue was purified by silica gel column chromatography (petroleum ether:ethyl acetate = 1:1) to afford compound C31-3 as a pale yellow solid (160 mg, 44.65%). ESI/MS (m/z): 468.2 [M+H]⁺.

Under a nitrogen atmosphere, a solution of compound C31-3 (140 mg, 0.299 mmol) in methanol (10 mL) and 10% palladium on carbon (14.6 mg) were added to a high-pressure reactor. Hydrogen gas was introduced into the reactor (30 psi), and the mixture was stirred at room temperature for 1 hour. The reaction mixture was concentrated under vacuum, and the residue was purified by reverse-phase chromatography (mobile phase A: water (0.1% formic acid), mobile phase B: acetonitrile) to afford compound C31 as a white solid (30.7 mg, 27.14%). ESI/MS (m/z): 378.2 [M+H]⁺.¹H NMR (400 MHz, DMSO-*d₆*) δ 11.99 (s, 1H), 7.97 (dd, *J =* 7.8, 1.7 Hz, 1H), 7.79-7.74 (m, 1H), 7.65 (dd, *J =* 8.1, 1.1 Hz, 1H), 7.54 (d, *J =* 7.9 Hz, 1H), 7.36-7.31 (m, 2H), 7.24 - 7.19 (m, 1H), 4.34 (t, *J =* 7.1 Hz, 2H), 2.91 (t, *J* = 6.2 Hz, 2H), 2.34 (dd, *J =* 8.6, 3.7 Hz, 2H), 1.96-1.85 (m, 2H), 1.36-1.33 (m, 3H).

### Example 284: Synthesis of Compound C32

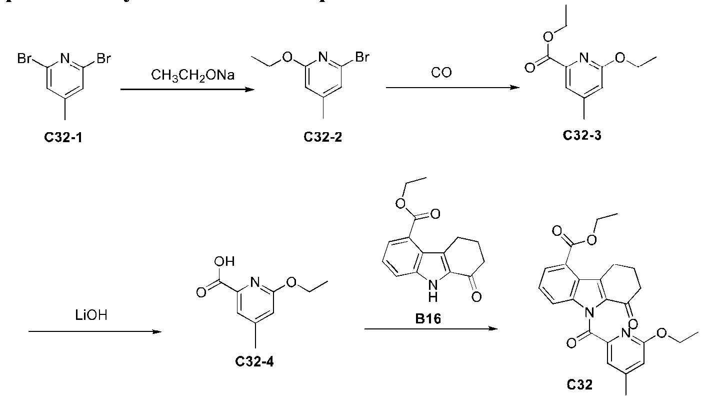

At room temperature, sodium ethoxide (650.9 mg, 9.565 mmol) was added to a solution of compound C32-1 (2 g, 7.971 mmol) in ethanol (10 mL). The reaction mixture was stirred at 80 °C for 1 day, cooled to room temperature, and concentrated under vacuum. The residue was purified by silica gel column chromatography (petroleum ether:ethyl acetate = 10:1) to afford compound C32-2 as a yellow oil (1 g, crude). ESI/MS (m/z): 216.1 [M+H]⁺.

In a high-pressure reactor, [1,1'-bis(diphenylphosphino)ferrocene]dichloropalladium(II) dichloromethane complex (158.5 mg, 0.195 mmol) and triethylamine (2.7 mL, 19.450 mmol) were added to a solution of compound C32-2 (900 mg, 1.945 mmol) in ethanol (25 mL). Carbon monoxide was introduced into the reactor to a pressure of 10 atm, and the mixture was heated to 80 °C and stirred overnight. After cooling to room temperature, the reaction mixture was concentrated under vacuum, and the residue was purified by silica gel column chromatography (petroleum ether:ethyl acetate = 3:1) to afford compound C32-3 as a yellow oil (400 mg, 93.86%). ESI/MS (m/z): 210.2 [M+H]⁺.

At room temperature, lithium hydroxide monohydrate (287.3 mg, 6.846 mmol) was added to a solution of compound C32-3 (500 mg, 2.282 mmol) in acetonitrile (3 mL) and water (3 mL). The reaction mixture was stirred at room temperature for 2 hours and concentrated under vacuum. The residue was diluted with water (3 mL), adjusted to pH 3 with dilute hydrochloric acid, and extracted with dichloromethane (3 × 30 mL). The combined organic layers were washed with saturated sodium chloride solution (20 mL), dried over anhydrous sodium sulfate, and concentrated under vacuum to afford compound C32-4 as a white solid (350 mg, 81.94%). ESI/MS (m/z): 182.1 [M+H]⁺.

At room temperature, 4-(dimethylamino)pyridine N-oxide hydrate (23.9 mg, 0.153 mmol), triethylamine (319.6 µL, 2.298 mmol), and di-tert-butyl dicarbonate (213.2 µL, 0.996 mmol) were added to a solution of compound C32-4 (215.2 mg, 1.149 mmol) and compound B16 (200 mg, 0.766 mmol) in acetonitrile (10 mL). The reaction mixture was stirred overnight at room temperature and concentrated under vacuum. The residue was purified by reverse-phase chromatography (mobile phase A: water (10 mmol/L ammonium bicarbonate), mobile phase B: acetonitrile) to afford compound C32 as a white solid (98.5 mg, 29.89%). ESI/MS (m/z): 421.1 [M+H]⁺.¹H NMR (400 MHz, DMSO-*d₆*) δ 8.12 (dd, *J =* 8.5, 1.0 Hz, 1H), 7.76 (dd, *J =* 7.5, 1.0 Hz, 1H), 7.68-7.66 (m, 1H), 7.61 (dd, *J =* 8.5, 7.4 Hz, 1H), 6.87-6.82 (m, 1H), 4.42 (q, *J =* 7.1 Hz, 2H), 4.02 (q, *J =* 7.0 Hz, 2H), 3.12 (t, *J =* 5.9 Hz, 2H), 2.50-2.45 (m, 2H), 2.40 (s, 3H), 2.17-2.09 (m, 2H), 1.39 (t, *J =* 7.1 Hz, 3H), 1.17 (t, *J =* 7.0 Hz, 3H).

### Example 285: Synthesis of Compound C33

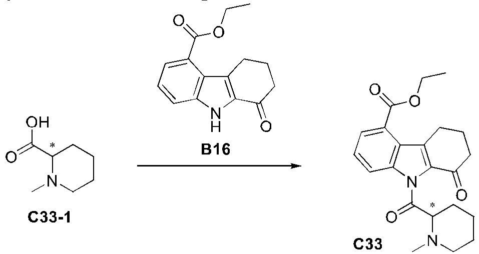

At 0°C, di-tert-butyl dicarbonate (198.2 mg, 0.907 mmol) was slowly added dropwise to a mixture of compound C33-1 (100 mg, 0.698 mmol, racemate), 4-dimethylaminopyridine N-oxide (21.8 mg, 0.140 mmol), triethylamine (212.0 mg, 2.094 mmol), and compound B16 (151.3 mg, 0.558 mmol) in acetonitrile (5 mL). The reaction mixture was stirred at room temperature for 2 hours, quenched with water (50 mL), and extracted with ethyl acetate (3 × 60 mL). The combined organic layers were washed with saturated sodium chloride solution (30 mL), dried over anhydrous sodium sulfate, and concentrated under vacuum. The residue was purified by reverse-phase chromatography (mobile phase A: water (0.1% formic acid), mobile phase B: acetonitrile) to afford compound C33 as a white solid (56.0 mg, 20.53%, racemate). ESI/MS (m/z): 383.2 [M+H]⁺.¹H NMR (400 MHz, DMSO-*d₆*) δ 8.05 (dd, *J =* 8.4, 1.0 Hz, 1H), 7.71 (dd, *J =* 7.4, 1.0 Hz, 1H), 7.59 (dd, *J =* 8.5, 7.4 Hz, 1H), 4.39 (q, *J =* 7.1 Hz, 2H), 3.66-3.55 (m, 1H), 3.09-2.98 (m, 2H), 2.76-2.67 (m, 1H), 2.67-2.60 (m, 2H), 2.27-2.21 (m, 1H), 2.19 (s, 3H), 2.17-2.09 (m, 2H), 1.53-1.40 (m, 5H), 1.36 (t, *J =* 7.1 Hz, 3H), 1.30-1.19 (m, 1H).

### Example 286: Synthesis of Compound C34

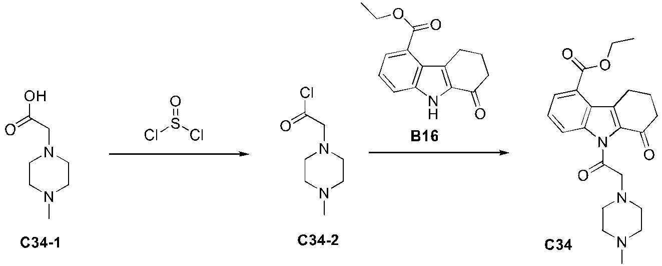

At room temperature, thionyl chloride (451 mg, 3.79 mmol) was added to a solution of compound C34-1 (500 mg, 3.16 mmol) in tetrahydrofuran (5 mL). The mixture was heated to 65 °C and stirred for 3 hours. After cooling to room temperature, the reaction mixture was concentrated under vacuum to afford compound C34-2 as a pale yellow solid (520 mg, crude).

At 0 °C, triethylamine (194 mg, 1.92 mmol) was added to a solution of compound B16 (100 mg, 0.38 mmol) and 4-dimethylaminopyridine (23 mg, 0.19 mmol) in dichloromethane (10 mL). The mixture was stirred for 10 minutes. Compound C34-2 (135 mg, 0.77 mmol) was then added to the above reaction mixture, and stirring was continued at room temperature for 2 hours. The reaction mixture was concentrated under vacuum, and the residue was purified by reverse-phase chromatography (mobile phase A: water (0.1% formic acid), mobile phase B: acetonitrile) to afford the formate salt of compound C34 as a white solid (29.7 mg, 17.32%). ESI/MS (m/z): 398.2 [M+H]⁺.¹H NMR (400 MHz, DMSO-*d₆*) δ 8.23 (dd, *J =* 8.4, 1.0 Hz, 1H), 8.18 (s, 1H), 7.72 (dd, *J =* 7.4, 1.0 Hz, 1H), 7.58 (dd, *J =* 8.4, 7.5 Hz, 1H), 4.39 (q, *J =* 7.1 Hz, 2H), 3.61 (s, 2H), 3.04 (t, *J =* 5.9 Hz, 2H), 2.70-2.63 (m, 2H), 2.30-2.17 (m, 4H), 2.17-2.12 (m, 4H), 2.12-2.11 (m, 2H), 2.11 (s, 3H), 1.37 (t, *J =* 7.1 Hz, 3H).

### Example287: Synthesis of Compound C35

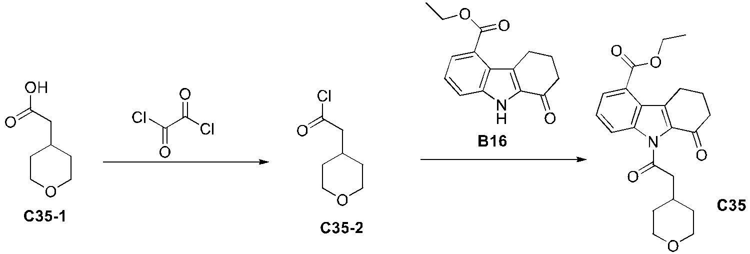

At room temperature, oxalyl chloride (528 mg, 4.16 mmol) was added to a mixture of compound C35-1 (500 mg, 3.47 mmol) and N,N-dimethylformamide (20 µL) in dichloromethane (10 mL). The reaction mixture was stirred at room temperature overnight and then concentrated under vacuum to afford compound C35-2 as a pale yellow solid (520 mg, crude).

At 0 °C, triethylamine (194 mg, 1.92 mmol) was added to a mixture of compound B16 (100 mg, 0.38 mmol) and 4-dimethylaminopyridine (23 mg, 0.19 mmol) in dichloromethane (10 mL). After stirring for 10 minutes, compound C35-2 (125 mg, 0.77 mmol) was added to the above reaction mixture. Stirring was continued at room temperature for 2 hours, and the mixture was then concentrated under vacuum. The residue was purified by reverse-phase chromatography (mobile phase A: water (0.1% formic acid), mobile phase B: acetonitrile) to afford compound C35 as a white solid (32.3 mg, 21.81%). ESI/MS (m/z): 384.3 [M+H]⁺.¹H NMR (400 MHz, DMSO-*d₆*) δ 8.10 (dd, *J =* 8.5, 1.0 Hz, 1H), 7.70 (dd, *J =* 7.5, 1.0 Hz, 1H), 7.60 (dd, *J =* 8.5, 7.4 Hz, 1H), 4.39 (q, *J =* 7.1 Hz, 2H), 3.79-3.71 (m, 2H), 3.29-2.20 (m, 2H), 3.05 (t, *J =* 6.0 Hz, 2H), 2.82 (d, *J =* 6.9 Hz, 2H), 2.70-2.62 (m, 2H), 2.19-2.10 (m, 2H), 2.09-1.97 (m, 1H), 1.54-1.45 (m, 2H), 1.37 (t, *J =* 7.1 Hz, 3H), 1.25-1.11 (m, 2H).

### Example 288: Synthesis of Compound C36

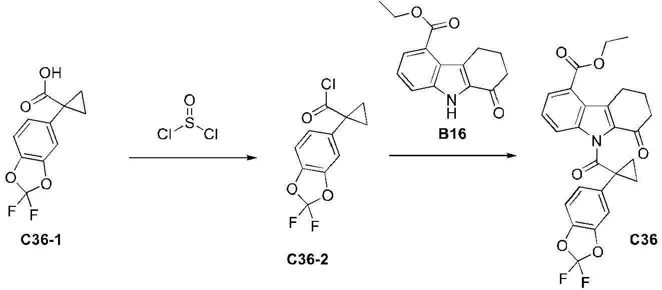

At room temperature, thionyl chloride (1.23 g, 10.33 mmol) was added to a solution of compound C36-1 (500 mg, 2.07 mmol) in toluene (5 mL). The mixture was heated to 65 °C and stirred for 3 hours. After cooling to room temperature, the reaction mixture was concentrated under vacuum to afford compound C36-2 as a pale yellow solid (520 mg, crude).

At 0 °C, triethylamine (194 mg, 1.92 mmol) was added to a mixture of compound B16 (100 mg, 0.38 mmol) and 4-dimethylaminopyridine (23 mg, 0.19 mmol) in dichloromethane (10 mL). The mixture was stirred for 10 minutes. Compound C36-2 (200 mg, 0.77 mmol) was then added to the above reaction mixture, and stirring was continued at room temperature for 2 hours. The reaction mixture was concentrated under vacuum, and the residue was purified by reverse-phase chromatography (mobile phase A: water (0.1% formic acid), mobile phase B: acetonitrile) to afford compound C36 as a white solid (82.4 mg, 44.39%). ESI/MS (m/z): 482.2 [M+H]⁺.¹H NMR (400 MHz, DMSO-*d₆*) δ 7.90 (dd, *J* = 8.4*,* 1.1 Hz, 1H), 7.72 (dd, *J =* 7.4, 1.1 Hz, 1H), 7.60 (dd, *J =* 8.4, 7.4 Hz, 1H), 7.14 (d, *J =* 8.4 Hz, 1H), 6.99 (d, *J =* 1.8 Hz, 1H), 6.77 (dd, *J =* 8.4, 1.8 Hz, 1H), 4.35 (q, *J =* 7.1 Hz, 2H), 2.84 (t, *J =* 5.9 Hz, 2H), 2.44-2.37 (m, 2H), 1.90-1.83 (m, 2H), 1.77-1.68 (m, 2H), 1.62-1.56 (m, 2H), 1.34 (t, *J =* 7.1 Hz, 3H).

### Example 289: Synthesis of Compound C37

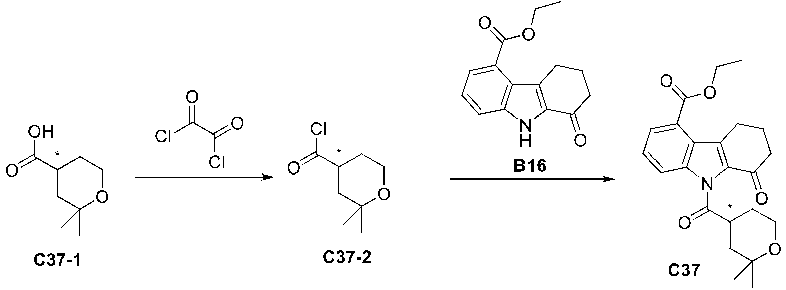

At room temperature, oxalyl chloride (64.6 µL, 0.758 mmol) was added to a solution of compound C37-1 (100 mg, 0.632 mmol, racemate) in dichloromethane (5 mL). The reaction mixture was stirred at room temperature for 2 hours and then concentrated under vacuum to afford compound C37-2 as a yellow oil (120 mg, crude, racemate).

At room temperature, triethylamine (116.3 mg, 1.149 mmol) and compound C37-2 (101.5 mg, 0.575 mmol, racemate) were added to a solution of compound B16 (100 mg, 0.383 mmol) and N,N-dimethylaminopyridine (23.4 mg, 0.192 mmol) in dichloromethane (5 mL). The reaction mixture was stirred at room temperature for 2 hours and then concentrated under vacuum. The residue was purified by reverse-phase chromatography (mobile phase A: water (0.1% formic acid), mobile phase B: acetonitrile) to afford compound C37 as a white solid (14 mg, 9.06%, racemate). ESI/MS (m/z): 398.3 [M+H]⁺.¹H NMR (400 MHz, DMSO-*d₆*) δ 7.96 (d, *J =* 8.5 Hz, 1H), 7.71 (d, *J =* 5.5 Hz, 1H), 7.62-7.55 (m, 1H), 4.40 (q, *J =* 7.0 Hz, 2H), 3.65-3.46 (m, 2H), 3.39-3.29 (m, 1H), 3.09 (t, *J =* 6.0 Hz, 2H), 2.75-2.62 (m, 2H), 2.24-2.07 (m, 2H), 1.67-1.57 (m, 2H), 1.56-1.42 (m, 2H), 1.37 (t, *J =* 7.1 Hz, 3H), 1.09 (d, *J =* 18.1 Hz, 6H).

### Example 290: Synthesis of Compound C38

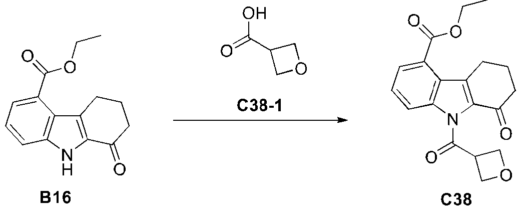

At room temperature, triethylamine (38 mg, 0.374 mmol) and di-tert-butyl dicarbonate (53 mg, 0.243 mmol) were added to a solution of compound B16 (50 mg, 0.187 mmol), 4-dimethylaminopyridine N-oxide (6 mg, 0.037 mmol), and compound C38-1 (23 mg, 0.224 mmol) in acetonitrile (3 mL) and N,N-dimethylformamide (3 mL). The reaction mixture was stirred at room temperature for 12 hours and then concentrated under vacuum. The residue was purified by reverse-phase chromatography (mobile phase A: water (0.1% formic acid), mobile phase B: acetonitrile) to afford compound C38 as a white solid (3.1 mg, 4.83%). ESI/MS (m/z): 342.2 [M+H]⁺.¹H NMR (400 MHz, DMSO-*d₆*) δ 8.29 (d, *J =* 8.4 Hz, 1H), 7.73 (d, *J* = 7.4 Hz, 1H), 7.67-7.59 (m, 1H), 4.75-4.67 (m, 2H), 4.68-4.60 (m, 2H), 4.40 (q, 2H), 4.36-4.30 (m, 1H), 3.04 (t, *J =* 6.0 Hz, 2H), 2.64 (t, *J =* 6.4 Hz, 2H), 2.19-2.08 (m, 2H), 1.36 (t, *J =* 7.1 Hz, 3H).

### Example 291: Synthesis of Compound C39

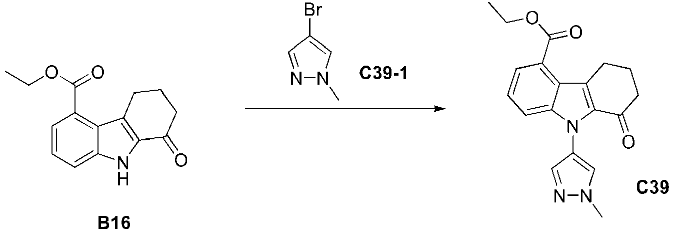

Under a nitrogen atmosphere, compound C39-2 (117.6 mg, 0.731 mmol), potassium phosphate (155.1 mg, 0.731 mmol), copper(I) iodide (139.2 mg, 0.731 mmol), and N,N'-dimethylethylenediamine (64.4 mg, 0.731 mmol) were added to a solution of compound B16 (100.0 mg, 0.365 mmol) in N,N-dimethylformamide (2 mL). The mixture was heated to 130 °C and stirred for 2 hours. After cooling to room temperature, the reaction mixture was diluted with ethyl acetate (100 mL). The resulting organic phase was washed with saturated sodium chloride solution (30 mL), dried over anhydrous sodium sulfate, and concentrated under vacuum. The residue was purified by normal-phase column chromatography (methanol/dichloromethane = 2%) to afford compound C39 as a pale yellow solid (41.5 mg, 31.99%). ESI/MS (m/z): 338.1 [M+H]⁺.¹H NMR (500 MHz, DMSO-*d₆*) δ 7.97 (s, 1H), 7.63 (dd, *J =* 6.9, 1.4 Hz, 1H), 7.53 (d, *J =* 0.7 Hz, 1H), 7.44-7.37 (m, 2H), 4.39 (q, *J =* 7.1 Hz, 2H), 3.92 (s, 3H), 3.11 (t, *J =* 6.0 Hz, 2H), 2.56-2.52 (m, 2H), 2.14-2.08 (m, 2H), 1.37 (t, *J =* 7.1 Hz, 3H).

### Example 292: Synthesis of Compound C40

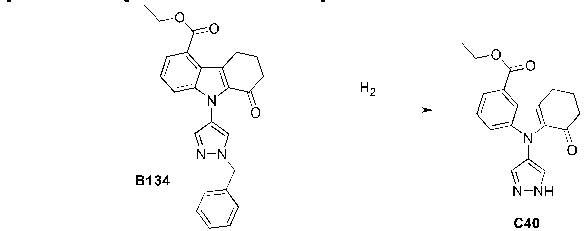

Under a nitrogen atmosphere, 5% palladium hydroxide on carbon (152.8 mg, 0.054 mmol) and a 1M hydrochloric acid solution in ethanol (0.3 mL) were added to a solution of compound B134 (25.0 mg, 0.054 mmol) in ethanol (3 mL). The reaction system was purged with hydrogen four times, heated to 45 °C, and stirred for 1 hour. After cooling to room temperature, the reaction mixture was diluted with ethyl acetate (100 mL) and filtered through celite. The filtrate was concentrated under vacuum, and the residue was purified by normal-phase silica gel column chromatography (methanol/dichloromethane = 2%) to afford compound C40 as a white solid (19.9 mg, 53.72%). ESI/MS (m/z): 324.1 [M+H]⁺.¹H NMR (500 MHz, DMSO-*d₆*) δ 13.07 (s, 1H), 8.00 (s, 1H), 7.63 (dd, *J=* 7.2, 1.1 Hz, 1H), 7.58 (s, 1H), 7.42 (dd, *J =* 8.4, 7.2 Hz, 1H), 7.35 (dd, *J =* 8.4, 1.1 Hz, 1H), 4.39 (q, *J =* 7.1 Hz, 2H), 3.11 (d, *J =* 12.1 Hz, 2H), 2.57-2.52(m, 2H)2.11 (p, *J =* 6.2 Hz, 2H), 1.37 (t, *J* = 7.1 Hz, 3H).

### Example293: Synthesis of Compound C41

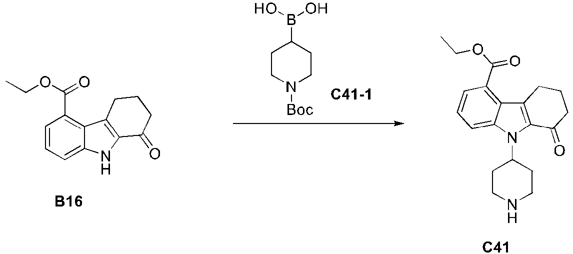

Under a nitrogen atmosphere, compound C41-1 (219.9 mg, 0.960 mmol), copper(II) acetate (87.2 mg, 0.480 mmol), bipyridine (75.0 mg, 0.480 mmol), and sodium carbonate (101.8 mg, 0.960 mmol) were added to a solution of compound B16 (130.0 mg, 0.480 mmol) in toluene (2.5 mL). The mixture was heated to 95 °C and stirred for 5 hours. After cooling to room temperature, the reaction mixture was diluted with ethyl acetate (100 mL). The resulting organic phase was washed with saturated sodium chloride solution (30 mL), dried over anhydrous sodium sulfate, and concentrated under vacuum. The residue was purified by reverse-phase column chromatography (acetonitrile/water = 10%-60%) to afford a yellow liquid compound. To this yellow liquid compound, a 4M hydrogen chloride solution in ethyl acetate (5 mL) was added, and the mixture was stirred at room temperature for 1 hour. Concentration under vacuum afforded the hydrochloride salt of compound C41 (89.0 mg, 44.28%). ESI/MS (m/z): 341.1 [M+H]⁺.¹H NMR (500 MHz, DMSO-*d₆*) δ 9.36 (br, 1H), 9.01 (br, 1H), 8.32 (d, *J =* 8.6 Hz, 1H), 7.53 (d, *J =* 7.2 Hz, 1H), 7.46-7.41 (m, 1H), 5.84 (br, 1H), 4.37 (q, *J =* 7.1 Hz, 2H), 3.44 (d, *J =* 12.6 Hz, 2H), 3.09 (q, *J* = 12.2 Hz, 2H), 2.99 (t, *J =* 6.0 Hz, 2H), 2.85 (qd, *J =* 13.1, 4.2 Hz, 2H), 2.64-2.59 (m, 2H), 2.06 (p, *J =* 6.2 Hz, 2H), 1.92-1.86 (m, 2H), 1.35 (t, *J =* 7.1 Hz, 3H).

### Example 294: Synthesis of Compound C42

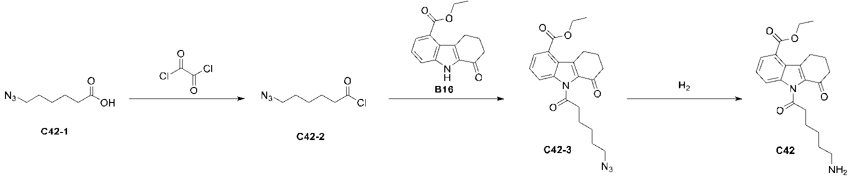

At 0°C, N,N-dimethylformamide (one drop) and oxalyl chloride (760.7 mg, 6.0 mmol) were added dropwise to a solution of compound C42-1 (314.0 mg, 2.0 mmol) in dichloromethane (3 mL). The reaction mixture was stirred at room temperature for 2 hours and then concentrated under vacuum to afford the crude product C42-2 as a yellow liquid (352.0 mg, crude).

At 0°C, compound C42-2 (359.0 mg, 1.95 mmol) was added to a mixture of compound B16 (460.0 mg, 1.77 mmol), 4-dimethylaminopyridine (108.1 mg, 0.89 mmol), and triethylamine (537.3 mg, 5.31 mmol) in dichloromethane (5 mL). The mixture was stirred at room temperature for 2 hours, then quenched with water (20 mL) and extracted with dichloromethane (2 × 20 mL). The combined organic layers were washed with saturated sodium chloride solution, dried over anhydrous sodium sulfate, and concentrated under vacuum. The residue was purified by column chromatography (petroleum ether:ethyl acetate = 4:1) to afford compound C42-3 as a yellow oil (470.0 mg, 63.6%). ESI/MS (m/z): 397.1 [M+H]⁺.

Under a nitrogen atmosphere, 10% palladium on carbon (6 mg) and a 4M hydrogen chloride solution in 1,4-dioxane (0.2 mL) were added to a solution of compound C42-3 (60.0 mg, 0.15 mmol) in methanol (6 mL). The reaction system was purged with hydrogen three times and stirred at room temperature for 30 minutes. The mixture was filtered through celite, and the filtrate was concentrated under vacuum. The residue was purified by reverse-phase preparative chromatography (acetonitrile/water = 10-60%) to afford the hydrochloride salt of compound C42 as a white solid (40.0 mg, 65.0%). ESI/MS (m/z): 371.1 [M+H]⁺.¹H NMR (500 MHz, DMSO-*d*₆) δ 8.10 (dd, *J =* 8.5, 1.0 Hz, 1H), 7.95 (s, 3H), 7.71 (dd, *J =* 7.4, 1.0 Hz, 1H), 7.60 (dd, *J =* 8.5, 7.4 Hz, 1H), 4.40 (q, *J =* 7.1 Hz, 2H), 3.06 (t, *J =* 5.9 Hz, 2H), 2.85 (t, *J =* 7.4 Hz, 2H), 2.77-2.70 (m, 2H), 2.69-2.62 (m, 2H), 2.15 (p, *J =* 6.2 Hz, 2H), 1.67 (p, *J =* 7.5 Hz, 2H), 1.55 (p, *J =* 7.6 Hz, 2H), 1.37 (t, *J =* 7.1 Hz, 3H), 1.35-1.27 (m, 2H).

### Example 295: Synthesis of Compound C43

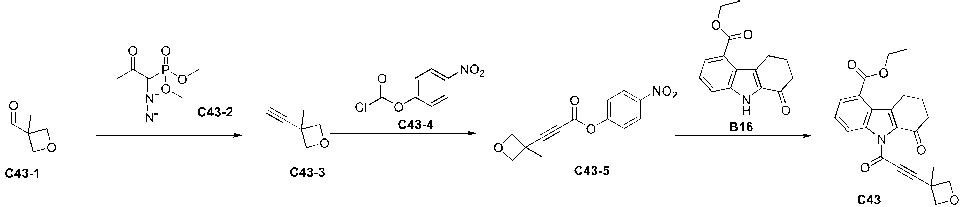

At 0°C, compound C43-2 (11.51 g, 59.930 mmol) was slowly added dropwise to a mixture of compound C43-1 (5 g, 49.942 mmol) and potassium carbonate (13.8 g, 99.884 mmol) in methanol (60 mL). The reaction mixture was stirred overnight at room temperature, quenched with water (150 mL), and extracted with diethyl ether (3 × 100 mL). The combined organic layers were washed with saturated sodium chloride solution (100 mL), dried over anhydrous sodium sulfate, and filtered. The filtrate was subjected to atmospheric distillation at 40°C to remove diethyl ether, yielding compound C43-3 as a colorless oil (3 g, 62.49%). ¹H NMR (400 MHz, Chloroform-d) δ 4.83 (d, J = 5.3 Hz, 2H), 4.39 (d, J = 5.7 Hz, 2H), 2.38 (s, 1H), 1.64 (s, 3H).

Under a nitrogen atmosphere, a solution of compound C43-3 (200 mg, crude) in tetrahydrofuran (10 mL) was cooled to -78°C, and a 2.5M solution of n-butyllithium in hexane (1.08 mL) was slowly added dropwise. The mixture was stirred for 1 hour. A solution of compound C43-4 (629.0 mg, 3.122 mmol) in tetrahydrofuran (4 mL) was then added dropwise to the above reaction mixture, and stirring was continued for 2 hours to afford compound C43-5 (14 mL, crude).

Under a nitrogen atmosphere, a solution of compound B16 (280 mg, 1.034 mmol) in tetrahydrofuran (30 mL) was cooled to -78°C, and a 1M solution of lithium bis(trimethylsilyl)amide in tetrahydrofuran (1.03 mL) was slowly added dropwise. The mixture was stirred for 1 hour. A solution of compound C43-5 (8 mL, crude) in tetrahydrofuran (1 mL) was then added dropwise to the above reaction mixture, and stirring was continued for 1 hour. The reaction was quenched with saturated ammonium chloride solution (30 mL) and extracted with ethyl acetate (3 × 60 mL). The combined organic layers were washed with saturated sodium chloride solution (30 mL), dried over anhydrous sodium sulfate, and concentrated under vacuum. The residue was purified by reverse-phase chromatography (mobile phase A: water (0.1% formic acid), mobile phase B: acetonitrile) to afford compound C43 as a white solid (42.7 mg, 10.79%). ESI/MS (m/z): 380.1 [M+H]⁺.¹H NMR (400 MHz, DMSO-*d₆*) *δ* 8.29 (dd, *J =* 8.5, 1.0 Hz, 1H), 7.77 (dd, *J =* 7.5, 1.0 Hz, 1H), 7.66 (dd, *J =* 8.4, 7.5 Hz, 1H), 4.68-4.62 (m, 2H), 4.44-4.41 (m, 2H), 4.41-4.36 (m, 2H), 3.10-2.99 (m, 2H), 2.71-2.61 (m, 2H), 2.21-2.08 (m, 2H), 1.56 (s, 3H), 1.37 (t, *J =* 7.1 Hz, 3H).

### Example 296: Synthesis of Compound C44

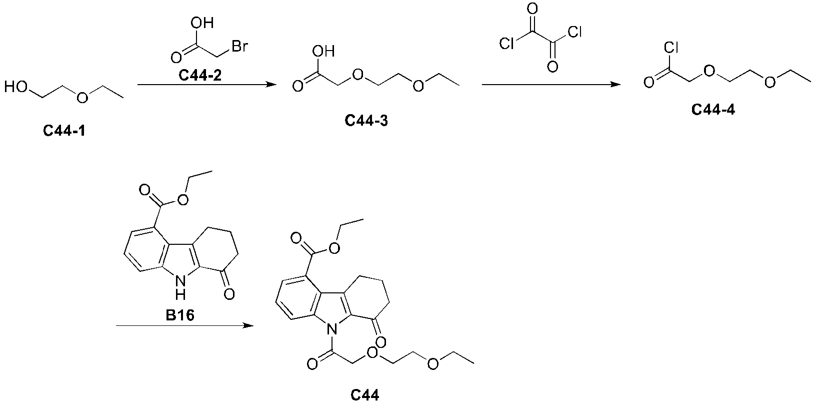

At 0°C, 60% sodium hydride (887.6 mg, 22.192 mmol) was added to a solution of compound C44-1 (500 mg, 5.548 mmol) in tetrahydrofuran (60 mL), and the mixture was stirred for 30 minutes. Compound C44-2 (1.54 g, 11.096 mmol) was then added to the above reaction mixture, and stirring was continued at room temperature overnight. The reaction was cooled to 0°C and quenched with water (10 mL). The mixture was extracted with ethyl acetate (3 × 50 mL), and the combined organic layers were washed with saturated sodium chloride solution (10 mL), dried over anhydrous sodium sulfate, and concentrated under vacuum to afford compound C44-3 as a yellow oil (400 mg, 48.66%). ESI/MS (m/z): 149.1 [M+H]⁺. ¹H NMR (400 MHz, DMSO-d6) δ 12.54 (s, 1H), 4.01 (s, 2H), 3.61-3.54 (m, 2H), 3.50-3.47 (m, 2H), 3.46-3.39 (m, 2H), 1.10 (t, J = 7.0 Hz, 3H).

At room temperature, N,N-dimethylformamide (one drop) and oxalyl chloride (111.4 mg, 0.878 mmol) were added to a solution of compound C44-3 (100 mg, 0.675 mmol) in dichloromethane (3 mL). The reaction mixture was stirred at room temperature for 1 hour and then concentrated under vacuum to afford compound C44-4 as a yellow oil (100 mg, crude).

At room temperature, compound C44-4 (89.8 mg, 0.539 mmol) and triethylamine (163.7 mg, 1.617 mmol) were added to a mixture of compound B16 (140 mg, 0.539 mmol) and N,N-dimethylaminopyridine (32.9 mg, 0.270 mmol) in dichloromethane (5 mL). The reaction mixture was stirred at room temperature for 2 hours and then concentrated under vacuum. The residue was purified by reverse-phase chromatography (mobile phase A: water (0.1% formic acid), mobile phase B: acetonitrile) to afford compound C44 as a colorless oil (22.7 mg, 10.80%). ESI/MS (m/z): 388.2 [M+H]⁺.¹H NMR (400 MHz, DMSO-*d₆*) δ 8.16 (dd, *J =* 8.5, 1.0 Hz, 1H), 7.73 (dd, *J =* 7.5*,* 1.0 Hz, 1H), 7.61 (dd, *J =* 8.5, 7.5 Hz, 1H), 4.55 (s, 2H), 4.40 (q, *J* = 7.1 Hz, 2H), 3.39-3.34 (m, 4H), 3.32-3.27 (m, 2H), 3.04 (t, J = 6.0 Hz, 2H), 2.69-2.61 (m, 2H), 2.19-2.09 (m, 2H), 1.37 (t, *J =* 7.1 Hz, 3H), 1.03 (t, *J =* 7.0 Hz, 3H).

### Example 297: Synthesis of Compound C45

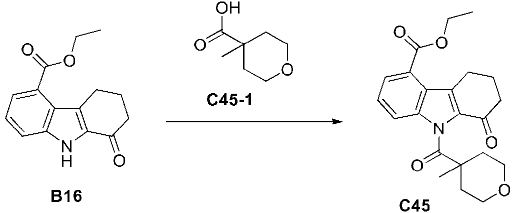

At room temperature, triethylamine (76 mg, 0.746 mmol) and di-tert-butyl dicarbonate (106 mg, 0.485 mmol) were added to a mixture of compound B16 (100 mg, 0.373 mmol), 4-dimethylaminopyridine N-oxide (12 mg, 0.075 mmol), and compound C45-1 (65 mg, 0.448 mmol) in acetonitrile (3 mL) and N,N-dimethylformamide (3 mL). The reaction mixture was stirred at room temperature for 4 hours and then concentrated under vacuum. The residue was purified by reverse-phase chromatography (mobile phase A: water (0.1% formic acid), mobile phase B: acetonitrile) to afford compound C45 as a white solid (10.4 mg, 7.23%). ESI/MS (m/z): 384.2 [M+H]⁺.¹H NMR (400 MHz, DMSO-*d*₆) δ 7.75-7.68 (m, 1H), 7.55 (d, *J* = 6.2 Hz, 2H), 4.40 (q, *J =* 7.1 Hz, 2H), 3.65-3.57 (m, 2H), 3.55-3.45 (m, 2H), 3.11 (t, *J =* 5.9 Hz, 2H), 2.64 (t, *J =* 6.4 Hz, 2H), 2.21-2.09 (m, 2H), 1.94-1.78 (m, 2H), 1.54 (d, *J =* 13.3 Hz, 2H), 1.40 (s, 3H), 1.37 (t, *J =* 7.0 Hz, 3H).

### Example 298: Synthesis of Compound C48

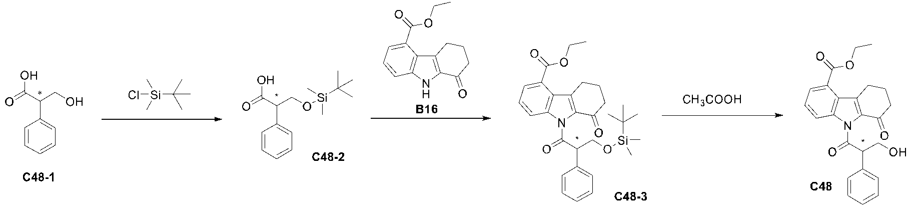

At room temperature, tert-butyldimethylchlorosilane (1.36 g, 9.027 mmol) was added to a solution of compound C48-1 (1 g, 6.018 mmol, racemate) and imidazole (1.23 g, 18.054 mmol) in N,N-dimethylformamide (20 mL). The reaction mixture was stirred overnight at room temperature, quenched with water (50 mL), and extracted with ethyl acetate (3 × 60 mL). The combined organic layers were washed with saturated sodium chloride solution (100 mL), dried over anhydrous sodium sulfate, and concentrated under vacuum. The residue was purified by silica gel column chromatography (petroleum ether:ethyl acetate = 1:1) to afford compound C48-2 as a colorless oil (410 mg, 23%, racemate). ESI/MS (m/z): 281.2 [M+H]⁺.

At 0°C, di-tert-butyl dicarbonate (344.0 mg, 1.576 mmol) was slowly added dropwise to a solution of compound C48-2 (400 mg, 1.212 mmol, racemate), 4-dimethylaminopyridine N-oxide (37.9 mg, 0.242 mmol), triethylamine (368.1 mg, 3.636 mmol), and compound B16 (328.4 mg, 1.212 mmol) in acetonitrile (4 mL). The reaction mixture was stirred at 0°C for 2 hours, quenched with water (30 mL), and extracted with ethyl acetate (3 × 30 mL). The combined organic layers were washed with saturated sodium chloride solution (20 mL), dried over anhydrous sodium sulfate, and concentrated under vacuum. The residue was purified by silica gel column chromatography (petroleum ether:ethyl acetate = 5:1) to afford compound C48-3 as a colorless oil (130 mg, 6.38%, racemate). ESI/MS (m/z): 520.2 [M+H]⁺.

At room temperature, glacial acetic acid (3 mL) was added dropwise to a solution of compound C48-3 (130 mg, 0.077 mmol, racemate) in tetrahydrofuran (1 mL) and water (1 mL). The reaction mixture was stirred overnight at room temperature and then concentrated under vacuum. The residue was purified by reverse-phase chromatography (mobile phase A: water (0.1% formic acid), mobile phase B: acetonitrile) to afford compound C48 as a white solid (10.8 mg, 33.88%, racemate). ESI/MS (m/z): 406.2 [M+H]⁺.¹H NMR (400 MHz, DMSO-*d*₆) δ 8.00 (d, *J =* 8.4 Hz, 1H), 7.65 (d, *J =* 7.4 Hz, 1H), 7.57-7.48 (m, 1H), 7.25-7.11 (m, 5H), 4.95-4.75 (m, 1H), 4.66-4.47 (m, 1H), 4.36 (q, *J =* 7.1 Hz, 2H), 4.10-3.98 (m, 1H), 3.88-3.72 (m, 1H), 3.07-2.88 (m, 2H), 2.74-2.55 (m, 2H), 2.18-1.90 (m, 2H), 1.34 (t, *J =* 7.1 Hz, 3H).

### Example 299: Synthesis of Compound C49

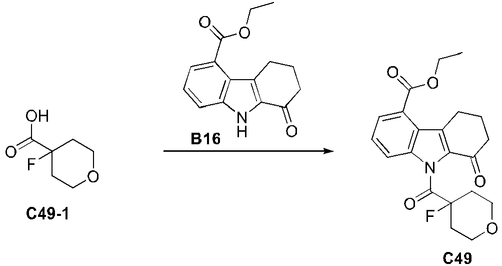

At room temperature, N,N-dimethylformamide (3 mL), 4-(dimethylamino)pyridine N-oxide (24.1 mg, 0.154 mmol), triethylamine (233.9 mg, 2.310 mmol), and di-tert-butyl dicarbonate (218.6 mg, 1.001 mmol) were added to a solution of compound B16 (200 mg, 0.770 mmol) and compound C49-1 (171.2 mg, 1.155 mmol) in acetonitrile (3 mL). The reaction mixture was stirred at room temperature for 1 hour and then concentrated under vacuum. The residue was purified by preparative silica gel thin-layer chromatography (ethyl acetate:petroleum ether = 1: 1) to afford compound C49 as a pale yellow solid (57.3 mg, 18.53%). ESI/MS (m/z): 388.2 [M+H]⁺.¹H NMR (400 MHz, DMSO-*d*₆) δ 7.78-7.76 (m, 1H), 7.76-7.73 (m, 1H), 7.64-7.55 (m, 1H), 4.40 (q, *J =* 7.1 Hz, 2H), 3.90-3.81 (m, 2H), 3.54-3.43 (m, 2H), 3.09 (t, *J =* 6.0 Hz, 2H), 2.68-2.61 (m, 2H), 2.36-2.19 (m, 2H), 2.18-2.11 (m, 2H), 2.10-1.97 (m, 2H), 1.37 (t, *J =* 7.1 Hz, 3H).

### Example 300: Synthesis of Compound C50

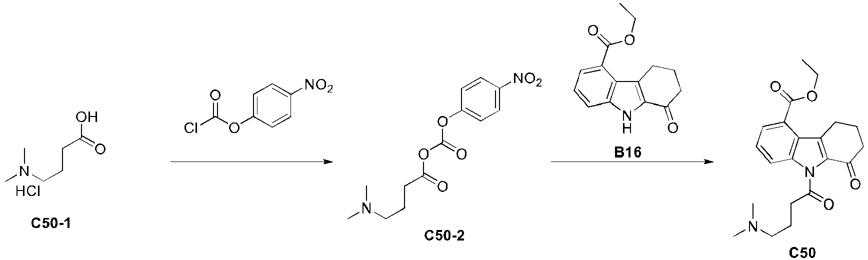

At 0°C, 4-nitrophenyl chloroformate (262.0 mg, 1.3 mmol) was added to a solution of compound C50-1 (167.6 mg, 1.0 mmol) and triethylamine (151.8 mg, 1.5 mmol) in dichloromethane (2 mL). The reaction mixture was stirred overnight at room temperature. The mixture was diluted with dichloromethane (20 mL), washed sequentially with 10% potassium carbonate solution (10 mL) and saturated sodium chloride solution (10 mL), dried over anhydrous sodium sulfate, and concentrated under vacuum to afford the crude product C50-2 as a yellow waxy solid (266.0 mg, 89.8%).

At -65°C, lithium bis(trimethylsilyl)amide (0.38 mL, 0.38 mmol) was added dropwise to a solution of compound B16 (75.0 mg, 0.29 mmol) in tetrahydrofuran (2 mL). The mixture was stirred for 30 minutes. A solution of compound C50-2 (111.5 mg, 0.38 mmol) in tetrahydrofuran (1 mL) was then added dropwise to the above reaction mixture. The temperature was raised to room temperature, and stirring was continued for 1 hour. The reaction was quenched with water (5 mL). The mixture was extracted with ethyl acetate (10 mL × 3), and the combined organic layers were washed with saturated sodium chloride solution, dried over anhydrous sodium sulfate, and concentrated under vacuum. The residue was purified by preparative thin-layer chromatography (dichloromethane:methanol = 20:1), followed by reverse-phase preparative chromatography (acetonitrile/water = 10-50%) to afford compound C50 as a pale yellow solid (3.1 mg, 2.8%). ESI/MS (m/z): 371.1 [M+H]⁺.

### Example 301: Synthesis of Compound C51

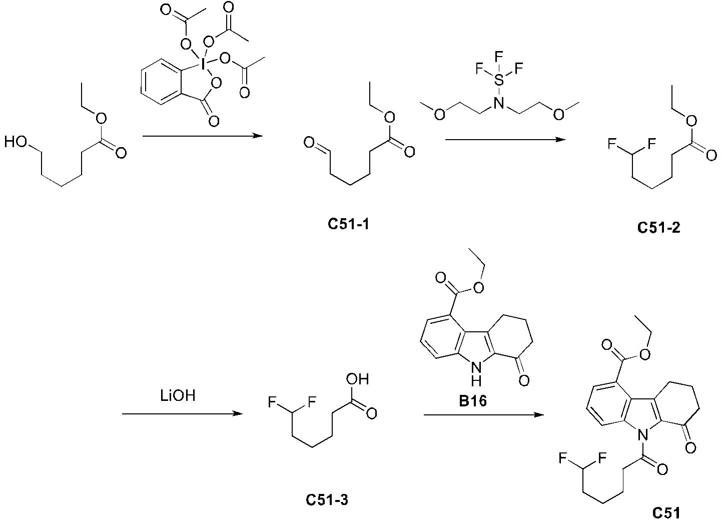

At 0°C, (1,1,1-triacetoxy)-1,1-dihydro-1,2-benziodoxol-3(1H)-one (1.99 g, 4.68 mmol) was added to a solution of ethyl 6-hydroxyhexanoate (500.0 mg, 3.12 mmol) in dichloromethane (5 mL). The reaction mixture was stirred at room temperature for 2 hours. The mixture was diluted with dichloromethane (20 mL), washed sequentially with 10% aqueous sodium thiosulfate solution (10 mL), 10% aqueous potassium carbonate solution (10 mL), and saturated sodium chloride solution (10 mL), dried over anhydrous sodium sulfate, and concentrated under vacuum. The residue was purified by silica gel column chromatography (petroleum ether:ethyl acetate = 10:1) to afford compound C51-1 as a colorless oil (369.0 mg, 71.0%).

At 0°C, bis(2-methoxyethyl)aminosulfur trifluoride (637.2 mg, 2.88 mmol) was added to a solution of compound C51-1 (369.0 mg, 2.22 mmol) in dichloromethane (5 mL). The reaction mixture was stirred overnight at room temperature. The mixture was cooled to 0°C, quenched with 10% aqueous sodium bicarbonate solution (20 mL), and extracted with dichloromethane (20 mL × 2). The combined organic layers were washed with saturated sodium chloride solution (20 mL), dried over anhydrous sodium sulfate, and concentrated under vacuum. The residue was purified by silica gel column chromatography (petroleum ether:ethyl acetate = 10:1) to afford compound C51-2 as a colorless liquid (284.0 mg, 67.6%).

At room temperature, methanol (1 mL) and a solution of anhydrous lithium hydroxide (215.1 mg, 8.98 mmol) in water (1 mL) were added to a solution of compound C51-2 (284.0 mg, 1.50 mmol) in tetrahydrofuran (3 mL). The mixture was stirred overnight. The reaction mixture was diluted with water (10 mL) and washed with petroleum ether. The aqueous phase was adjusted to pH 2 with 4M aqueous hydrochloric acid and extracted with ethyl acetate (10 mL × 2). The combined organic layers were washed with saturated sodium chloride solution (20 mL), dried over anhydrous sodium sulfate, and concentrated under vacuum to afford the crude product C51-3 as a pale yellow oil (235.0 mg, 95%).

At 0°C, N,N-dimethylformamide (one drop) and oxalyl chloride (134.0 mg, 1.06 mmol) were added to a solution of compound C51-3 (56.4 mg, 0.35 mmol) in dichloromethane (1 mL). The reaction mixture was stirred at room temperature for 2 hours and then concentrated under vacuum to afford the acyl chloride intermediate. At 0°C, this acyl chloride intermediate was added to a solution of compound B16 (77.0 mg, 0.29 mmol), 4-dimethylaminopyridine (17.9 mg, 0.15 mmol), and triethylamine (89.0 mg, 0.88 mmol) in dichloromethane (1 mL). The mixture was stirred at room temperature for 2 hours. The reaction was quenched with water (5 mL), and the mixture was extracted with dichloromethane (10 mL × 3). The combined organic layers were washed with saturated sodium chloride solution (10 mL), dried over anhydrous sodium sulfate, and concentrated under vacuum. The residue was purified by reverse-phase preparative chromatography (acetonitrile/water = 10-65%) to afford compound C51 as a white solid (67.6 mg, 57.1%). ESI/MS (m/z): 392.1 [M+H]⁺.¹H NMR (500 MHz, DMSO-*d*₆) δ 8.09 (dd, *J* = 8.5, 1.0 Hz, 1H), 7.70 (dd, *J =* 7.4, 1.0 Hz, 1H), 7.59 (dd, *J* = 8.5, 7.4 Hz, 1H), 6.03 (tt, *J =* 56.9, 4.4 Hz, 1H), 4.39 (q, *J =* 7.1 Hz, 2H), 3.05 (t, *J =* 6.0 Hz, 2H), 2.87 (t, *J =* 7.3 Hz, 2H), 2.66 (dd, *J =* 7.3, 5.7 Hz, 2H), 2.15 (p, *J =* 6.2 Hz, 2H), 1.85-1.75 (m, 2H), 1.71 (p, *J =* 7.5 Hz, 2H), 1.43-1.35 (m, 5H).

### Example 302: Synthesis of Compound C52

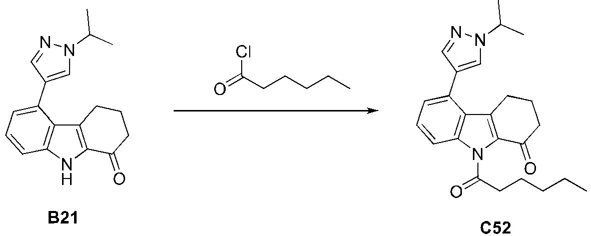

At 0°C, hexanoyl chloride (67 mg, 0.50 mmol) was added to a solution of compound B21 (100 mg, 0.33 mmol), 4-dimethylaminopyridine (81 mg, 0.66 mmol), and triethylamine (100 mg, 0.99 mmol) in dichloromethane (5 mL). The reaction mixture was stirred at 0°C for 2 hours and then diluted with dichloromethane (30 mL). The resulting organic phase was washed with water (10 mL × 2), dried over anhydrous sodium sulfate, and concentrated under vacuum. The residue was purified by reverse-phase preparative chromatography (acetonitrile/water = 20%-75%) to afford compound C52 as a colorless oil (51 mg, 0.13 mmol). ESI/MS (m/z): 392.1 [M+H]⁺.¹H NMR (500 MHz, DMSO-*d*₆) δ 8.01 (s, 1H), 7.87 (dd, *J =* 8.5, 0.9 Hz, 1H), 7.61 (s, 1H), 7.50 (dd, *J =* 8.5, 7.3 Hz, 1H), 7.16 (dd, *J =* 7.3, 1.0 Hz, 1H), 4.57 (p, *J* = 6.7 Hz, 1H), 2.82 (t, *J =* 7.4 Hz, 2H), 2.69 (t, *J =* 5.9 Hz, 2H), 2.61-2.55 (m, 2H), 2.01 (p, *J =* 6.2 Hz, 2H), 1.63 (p, *J =* 7.4 Hz, 2H), 1.48 (d, *J=* 6.7 Hz, 6H), 1.26-1.19 (m, 4H), 0.82 (t, *J =* 6.8 Hz, 3H).

### Example 303: Synthesis of Compound C53

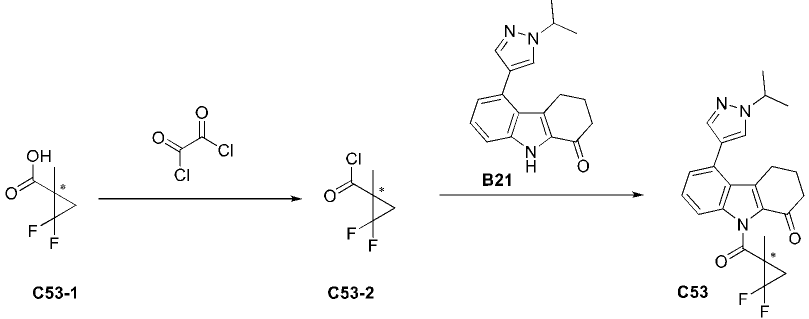

At 0°C, N,N-dimethylformamide (7.3 mg, 0.1 mmol) was added to a mixture of compound C53-1 (136 mg, 1 mmol, racemate) and oxalyl chloride (1.27 g, 10 mmol) in dichloromethane (5 mL). The reaction mixture was warmed to room temperature and stirred for 2 hours, then concentrated under vacuum to afford compound C53-2 as a yellow oil (150 mg, 0.97 mmol, racemate).

At room temperature, compound C53-2 (77 mg, 0.50 mmol, racemate) was added to a solution of compound B21 (100 mg, 0.33 mmol), 4-dimethylaminopyridine (40 mg, 0.33 mmol), and triethylamine (100 mg, 0.99 mmol) in dichloromethane (5 mL). The reaction mixture was stirred at room temperature for 1 hour and then diluted with dichloromethane (30 mL). The resulting organic phase was washed with water (10 mL × 2), dried over anhydrous sodium sulfate, and concentrated under vacuum. The residue was purified by reverse-phase preparative chromatography (acetonitrile/water = 20%-70%) to afford compound C53 as a white solid (4.9 mg, 0.012 mmol, racemate). ESI/MS (m/z): 412.1 [M+H]⁺.¹H NMR (500 MHz, DMSO-*d*₆) δ 8.03 (s, 1H), 7.63 (s, 1H), 7.61 (dd, *J =* 8.5, 0.9 Hz, 1H), 7.51 (dd, *J =* 8.5, 7.3 Hz, 1H), 7.17 (dd, *J =* 7.2, 1.0 Hz, 1H), 4.57 (p, *J =* 6.7 Hz, 1H), 2.77-2.66 (m, 3H), 2.49-2.44 (m, 1H), 2.13-2.08 (m, 1H), 2.07-2.01 (m, 2H), 1.84-1.78 (m, 1H), 1.52 (s, 3H), 1.49 (d, *J =* 6.7 Hz, 6H).

### Example304: Synthesis of Compound C55

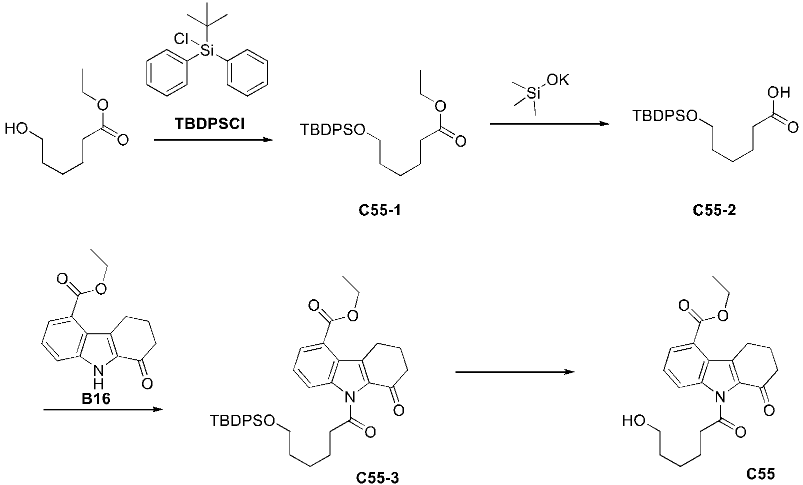

At 0°C, TBDPSCl (2.06 g, 7.49 mmol) was added to a solution of ethyl 6-hydroxyhexanoate (1.0 g, 6.24 mmol) and imidazole (637.4 mg, 9.36 mmol) in dichloromethane (15 mL). The reaction mixture was stirred at room temperature for 4 hours, diluted with dichloromethane (20 mL), washed with saturated sodium chloride solution, dried over anhydrous sodium sulfate, and concentrated under vacuum. The residue was purified by silica gel column chromatography (petroleum ether:ethyl acetate = 10:1) to afford compound C55-1 as a colorless liquid (2.5 g, 95.5%). ESI/MS (m/z): 421.1 [M+Na]⁺.

At room temperature, potassium trimethylsilanolate (0.46 g, 3.57 mmol) was added to a solution of compound C55-1 (1.0 g, 2.38 mmol) in tetrahydrofuran (10 mL). The mixture was stirred overnight. The reaction mixture was cooled to 0°C and adjusted to pH 3 with 4M aqueous hydrochloric acid. The mixture was extracted with ethyl acetate (30 mL × 2), and the combined organic layers were washed with saturated sodium chloride solution, dried over anhydrous sodium sulfate, and concentrated under vacuum to afford compound C55-2 as a colorless oil (0.94 g, 90.5%). ESI/MS (m/z): 393.1 [M+Na]⁺.

At 0°C, N,N-dimethylformamide (one drop) and oxalyl chloride (102.7 mg, 0.81 mmol) were added to a solution of compound C55-2 (126.0 mg, 0.49 mmol) in dichloromethane (2 mL). The reaction mixture was stirred at room temperature for 2 hours and then concentrated under vacuum to afford the acyl chloride intermediate. At 0°C, this acyl chloride intermediate was added to a solution of compound B16 (85.0 mg, 0.32 mmol), 4-dimethylaminopyridine (19.8 mg, 0.16 mmol), and triethylamine (98.3 mg, 0.97 mmol) in dichloromethane (2 mL). The mixture was stirred at room temperature for 2 hours, quenched with water (5 mL), and extracted with dichloromethane (10 mL × 3). The combined organic layers were washed with saturated sodium chloride solution, dried over anhydrous sodium sulfate, and concentrated under vacuum. The residue was purified by silica gel column chromatography (petroleum ether:ethyl acetate = 4:1) to afford compound C55-3 as a brown oil (110.0 mg, 90.0%). ESI/MS (m/z): 632.2 [M+Na]⁺.

At room temperature, triethylamine trihydrofluoride (0.2 mL) was added to a solution of compound C55-3 (110.0 mg, 0.17 mmol) in dichloromethane (2 mL). The mixture was stirred overnight. The reaction was quenched with sodium bicarbonate solution (10 mL) and extracted with dichloromethane (10 mL × 3). The combined organic layers were washed with saturated sodium chloride solution (10 mL), dried over anhydrous sodium sulfate, and concentrated under vacuum. The residue was purified by reverse-phase preparative chromatography (acetonitrile/water = 10-50%) to afford compound C55 as a pale yellow solid (39.5 mg, 59.6%). ESI/MS (m/z): 372.1 [M+H]⁺.¹H NMR (500 MHz, DMSO-*d*₆) δ 8.09 (dd, *J =* 8.5, 1.0 Hz, 1H), 7.70 (dd, *J =* 7.4, 1.0 Hz, 1H), 7.59 (dd, *J =* 8.5, 7.4 Hz, 1H), 4.39 (q, *J =* 7.2 Hz, 2H), 4.31 (t, *J =* 5.1 Hz, 1H), 3.35-3.31 (m, 2H), 3.05 (t, *J =* 6.0 Hz, 2H), 2.83 (t, *J =* 7.4 Hz, 2H), 2.66 (dd, *J =* 7.2, 5.7 Hz, 2H), 2.14 (p, *J =* 6.1 Hz, 2H), 1.63 (p, *J =* 7.5 Hz, 2H), 1.40-1.34 (m, 5H), 1.30-1.21 (m, 2H).

### Example 305: Synthesis of Compound C56

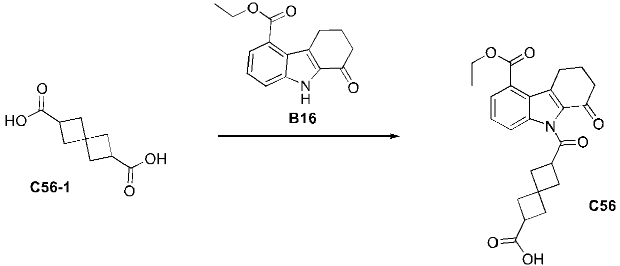

At room temperature, 4-dimethylaminopyridine (1.47 g, 12.000 mmol), N,N-diisopropylethylamine (2.76 g, 21.330 mmol), and bis(2-oxo-3-oxazolidinyl)phosphinic chloride (3.05 g, 12.000 mmol) were added to a solution of compound C56-1 (1.55 g, 8.000 mmol) in dichloromethane (35 mL). After stirring for 5 minutes, compound B16 (0.7 g, 2.670 mmol) was added, and stirring was continued at room temperature for 2 hours. The resulting mixture was diluted with ethyl acetate (100 mL), washed with saturated sodium chloride solution (30 mL), dried over anhydrous sodium sulfate, and concentrated under vacuum. The residue was purified by reverse-phase column chromatography (acetonitrile/water) to afford compound C56 as a yellow oil (700 mg). ESI/MS (m/z): 424.1 [M+H]⁺.¹H NMR (500 MHz, DMSO-*d*₆) δ 11.94 (br, 1H), 8.10 (dd, *J =* 8.5, 1.0 Hz, 1H), 7.70 (dd, *J =* 7.4, 1.0 Hz, 1H), 7.59 (dd, *J =* 8.5, 7.4 Hz, 1H), 4.39 (q, *J =* 7.1 Hz, 2H), 3.65 (p, *J* = 8.5 Hz, 1H), 3.04 (t, *J =* 6.0 Hz, 2H), 2.87 (p, *J =* 8.4 Hz, 1H), 2.67-2.63 (m, 2H), 2.29-2.24 (m, 1H), 2.24-2.17 (m, 2H), 2.16-2.06 (m, 7H), 1.36 (t, *J=* 7.1 Hz, 3H).

### Example 306: Synthesis of Compound C57

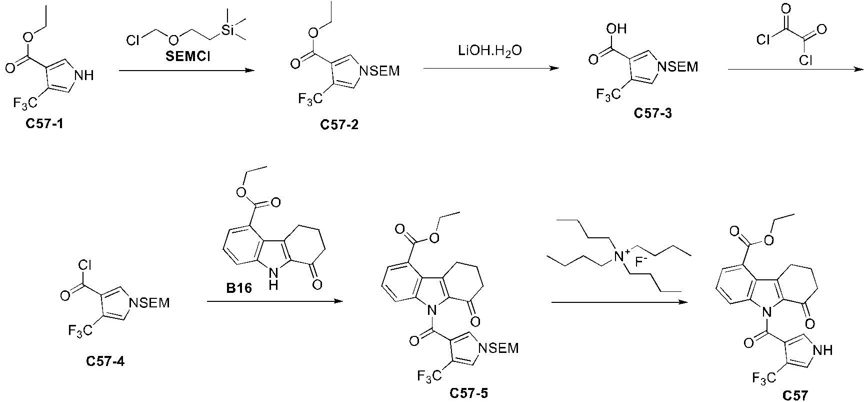

At 0°C, 60% sodium hydride (46.3 mg, 1.158 mmol) was added to a solution of compound C57-1 (200 mg, 0.965 mmol) in tetrahydrofuran (10 mL). After stirring for 30 minutes, SEMCl (241.5 mg, 1.448 mmol) was added. The reaction mixture was stirred at room temperature for 3 hours, quenched with water, and extracted with dichloromethane (2 × 20 mL). The combined organic layers were washed with saturated sodium chloride solution (20 mL), dried over anhydrous sodium sulfate, and concentrated under vacuum. The residue was purified by silica gel column chromatography (petroleum ether:ethyl acetate = 1:1) to afford compound C57-2 as a yellow oil (210 mg, 64.46%). ESI/MS (m/z): 338.2 [M+H]⁺.

At room temperature, lithium hydroxide monohydrate (124.4 mg, 2.965 mmol) was added to a solution of compound C57-2 (200 mg, 0.593 mmol) in methanol (2 mL), tetrahydrofuran (4 mL), and water (2 mL). The mixture was heated to 60°C and stirred overnight. After cooling to room temperature, the reaction mixture was adjusted to pH 3 with 1M aqueous hydrochloric acid and extracted with dichloromethane (2 × 20 mL). The combined organic layers were washed with saturated sodium chloride solution (20 mL), dried over anhydrous sodium sulfate, and concentrated under vacuum to afford compound C57-3 as a yellow solid (180 mg, 98.16%). ESI/MS (m/z): 310.2 [M+H]⁺.

At room temperature, oxalyl chloride (256.8 mg, 2.024 mmol) and N,N-dimethylformamide (3 drops) were added to a solution of compound C57-3 (160 mg, 0.506 mmol) in dichloromethane (5 mL). The reaction mixture was stirred at room temperature for 2 hours and then concentrated under vacuum to afford compound C57-4 as a pale yellow oil (230 mg, crude).

At room temperature, triethylamine (642.5 µL, 4.620 mmol) and compound C57-4 (227.3 mg, 0.693 mmol) were added to a solution of compound B16 (120 mg, 0.462 mmol) and N,N-dimethylaminopyridine (28.2 mg, 0.231 mmol) in dichloromethane (5 mL). The mixture was stirred for 12 hours, then quenched with water and extracted with dichloromethane (2 × 30 mL). The combined organic layers were washed with saturated sodium chloride solution (20 mL), dried over anhydrous sodium sulfate, and concentrated under vacuum. The residue was purified by silica gel column chromatography (petroleum ether:ethyl acetate = 1:1) to afford compound C57-5 as a white solid (160 mg, 63.10%). ESI/MS (m/z): 549.3 [M+H]⁺.

At room temperature, a 1M solution of tetrabutylammonium fluoride in tetrahydrofuran (528.6 µL, 0.528 mmol) was added to a solution of compound C57-5 (145 mg, 0.264 mmol) in tetrahydrofuran (10 mL). The reaction mixture was stirred at room temperature for 12 hours, then quenched with water and extracted with dichloromethane (2 × 30 mL). The combined organic layers were washed with saturated sodium chloride solution (20 mL), dried over anhydrous sodium sulfate, and concentrated under vacuum. The residue was purified by silica gel column chromatography (petroleum ether:ethyl acetate = 1:2) to afford a yellow solid. This yellow solid was further purified by preparative thin-layer chromatography (petroleum ether:ethyl acetate = 1:2) to afford compound C57 as a white solid (4.2 mg, 3.80%). ESI/MS (m/z): 419.2 [M+H]⁺.¹H NMR (400 MHz, DMSO-*d*₆) δ 12.14 (s, 1H), 7.78-7.76 (m, 1H), 7.75 (s, 1H), 7.60-7.54 (m, 2H), 7.24 (d, *J =* 2.0 Hz, 1H), 4.47 (q, *J =* 7.1 Hz, 2H), 3.16 (t, *J =* 5.9 Hz, 2H), 2.61-2.57 (m, 2H), 2.25-2.15 (m, 2H), 1.44 (t, *J* = 7.1 Hz, 3H).

### Example 307: Synthesis of Compound C58

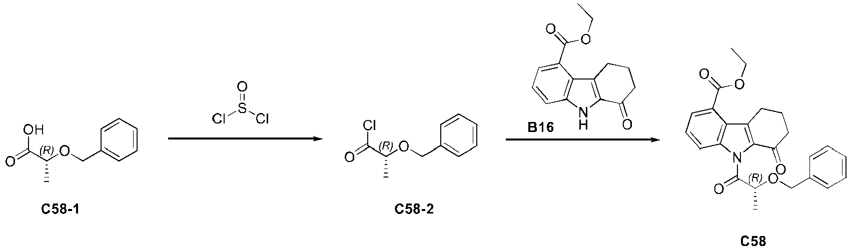

At room temperature, thionyl chloride (1.19 g, 10 mmol) was added to a solution of compound C58-1 (180 mg, 1 mmol) and N,N-dimethylformamide (7.3 mg, 0.1 mmol) in dichloromethane (2 mL). The reaction mixture was stirred at room temperature for 5 hours and then concentrated under vacuum to afford compound C58-2 as a yellow oil (190 mg, 0.7 mmol).

At room temperature, compound C58-2 (190 mg, 0.7 mmol) was added to a solution of compound B16 (129 mg, 0.50 mmol), triethylamine (150 mg, 1.5 mmol), and 4-dimethylaminopyridine (61 mg, 0.5 mmol) in dichloromethane (5 mL). The reaction mixture was stirred at room temperature for 2 hours and then diluted with dichloromethane (30 mL). The resulting organic phase was washed with water (10 mL × 2), dried over anhydrous sodium sulfate, and concentrated under vacuum. The residue was purified by reverse-phase preparative chromatography (acetonitrile/water: 10-65%) to afford compound C58 as a pale yellow oil (65 mg, 0.16 mmol). ESI/MS (m/z): 420.1 [M+H]⁺.¹H NMR (500 MHz, DMSO-*d*₆) δ 8.00 (dd, *J =* 8.4, 1.0 Hz, 1H), 7.74 (dd, *J =* 7.4, 1.0 Hz, 1H), 7.62 (dd, *J =* 8.5, 7.4 Hz, 1H), 7.26-7.21 (m, 3H), 7.11 (dd, *J =* 6.5, 2.9 Hz, 2H), 4.71 (q, *J =* 6.6 Hz, 1H), 4.45-4.37 (m, 4H), 3.05-2.98 (m, 2H), 2.62-2.51 (m, 2H), 2.12-2.03 (m, 1H), 2.03-1.93 (m, 1H), 1.38 (t, *J =* 7.1 Hz, 3H), 1.32 (d, *J =* 6.6 Hz, 3H).

### Example 308: Synthesis of Compound C59

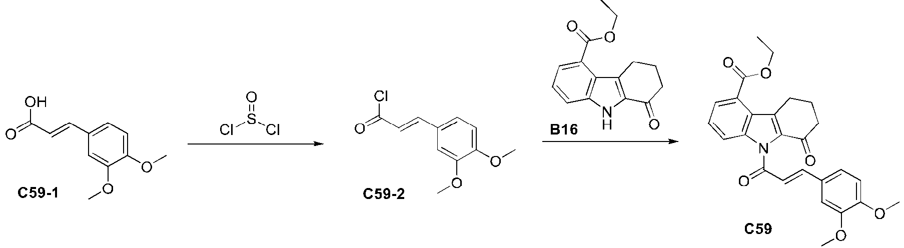

At room temperature, thionyl chloride (1.19 g, 10 mmol) was added to a solution of compound C59-1 (208 mg, 1 mmol) and N,N-dimethylformamide (7.3 mg, 0.1 mmol) in dichloromethane (2 mL). The reaction mixture was stirred at room temperature for 5 hours and then concentrated under vacuum to afford the crude product C59-2 as a yellow oil (201 mg, 0.89 mmol).

At room temperature, compound C59-2 (158 mg, 0.7 mmol) was added to a solution of compound B16 (129 mg, 0.50 mmol), triethylamine (150 mg, 1.5 mmol), and 4-dimethylaminopyridine (61 mg, 0.5 mmol) in dichloromethane (5 mL). The mixture was stirred for 2 hours, then diluted with dichloromethane (30 mL). The resulting organic phase was washed with water (10 mL × 2), dried over anhydrous sodium sulfate, and concentrated under vacuum. The residue was purified by reverse-phase preparative chromatography (acetonitrile/water: 10-65%) to afford compound C59 as a pale yellow solid (54 mg, 0.11 mmol). ESI/MS (m/z): 448.1 [M+H]⁺.¹H NMR (500 MHz, DMSO-*d*₆) δ 8.14 (dd, *J =* 8.5, 1.0 Hz, 1H), 7.72 (dd, *J =* 7.3, 1.0 Hz, 1H), 7.66 (d, *J =* 15.6 Hz, 1H), 7.59 (dd, *J =* 8.5, 7.4 Hz, 1H), 7.34 (d, *J =* 2.0 Hz, 1H), 7.32 (dd, *J =* 8.3, 2.0 Hz, 1H), 7.01 (d, *J =* 8.3 Hz, 1H), 6.97 (d, *J =* 15.6 Hz, 1H), 4.41 (q, *J =* 7.1 Hz, 2H), 3.80 (d, *J =* 9.0 Hz, 6H), 3.10 (t, *J =* 6.0 Hz, 2H), 2.66 (dd, *J =* 7.3*,* 5.6 Hz, 2H), 2.19 (p, *J =* 6.2 Hz, 2H), 1.38 (t, *J =* 7.1 Hz, 3H).

### Example 309: Synthesis of Compound C60

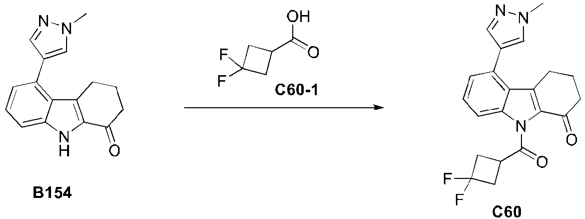

At 0°C, N,N-dimethylformamide (one drop) and oxalyl chloride (36.1 mg, 0.28 mmol) were added to a solution of compound C60-1 (38.7 mg, 0.28 mmol) in dichloromethane (0.5 mL). The reaction mixture was warmed to room temperature and stirred for 2 hours, then concentrated under vacuum to afford the acyl chloride intermediate. At 0°C, this acyl chloride intermediate was added dropwise to a solution of compound B154 (53.0 mg, 0.19 mmol), 4-pyrrolidinopyridine (28.1 mg, 0.19 mmol), and N,N-diisopropylethylamine (98.1 mg, 0.76 mmol) in dichloromethane (0.5 mL). The reaction mixture was stirred at room temperature for 2 hours, quenched with water (5 mL), and extracted with dichloromethane (10 mL × 3). The combined organic layers were washed with saturated sodium chloride solution (10 mL), dried over anhydrous sodium sulfate, and concentrated under vacuum. The residue was purified by reverse-phase preparative chromatography (acetonitrile/water = 10-70%) to afford compound C60 as a white solid (37.6 mg, 49.6%). ESI/MS (m/z): 384.1 [M+H]⁺.¹H NMR (500 MHz, DMSO-*d*₆) δ 7.99 (dd, *J* = 8.5, 0.9 Hz, 1H), 7.93 (s, 1H), 7.61 (d, *J =* 0.8 Hz, 1H), 7.53 (dd, *J =* 8.5, 7.3 Hz, 1H), 7.17 (dd, *J =* 7.3*,* 1.0 Hz, 1H), 3.93 (s, 3H), 3.65 (pd, *J =* 8.3, 3.2 Hz, 1H), 2.90-2.80 (m, 4H), 2.70 (t, *J =* 5.9 Hz, 2H), 2.62-2.56 (m, 2H), 2.03 (p, *J =* 6.1 Hz, 2H).

### Example 310: Synthesis of Compound C62

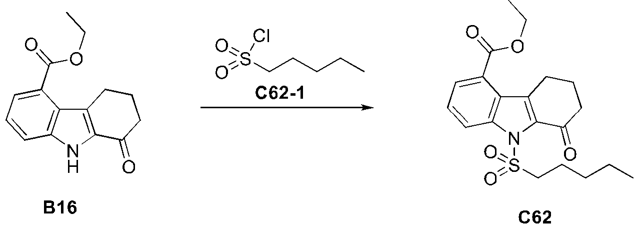

At 0°C, 60% sodium hydride (33.3 mg, 0.834 mmol) was added to a solution of compound B16 (150 mg, 0.556 mmol) in tetrahydrofuran (10 mL). After stirring for 30 minutes, compound C62-1 (142.2 mg, 0.834 mmol) was slowly added dropwise. The reaction mixture was stirred at room temperature for 2 hours, cooled to 0°C, quenched with water, and extracted with ethyl acetate (3 × 30 mL). The combined organic layers were washed with saturated sodium chloride solution (40 mL), dried over anhydrous sodium sulfate, and concentrated under vacuum. The residue was purified by reverse-phase chromatography (mobile phase A: water (10 mmol/L ammonium bicarbonate), mobile phase B: acetonitrile) to afford compound C62 as a white solid (55.9 mg, 25.70%). ESI/MS (m/z): 392.2 [M+H]⁺.¹H NMR (400 MHz, DMSO-*d*₆) δ 8.23 (dd, *J =* 8.5, 1.1 Hz, 1H), 7.70 (dd, *J =* 7.5, 1.1 Hz, 1H), 7.62 (dd, *J* = 8.6, 7.4 Hz, 1H), 4.39 (q, *J =* 7.1 Hz, 2H), 4.23-4.16 (m, 2H), 2.99 (t, *J =* 6.0 Hz, 2H), 2.69-2.62 (m, 2H), 2.09 (p, *J =* 6.2 Hz, 2H), 1.90-1.80 (m, 2H), 1.45-1.33 (m, 5H), 1.33-1.24 (m, 2H), 0.83 (t, *J =* 7.2 Hz, 3H).

### Example 311: Synthesis of Compound C63

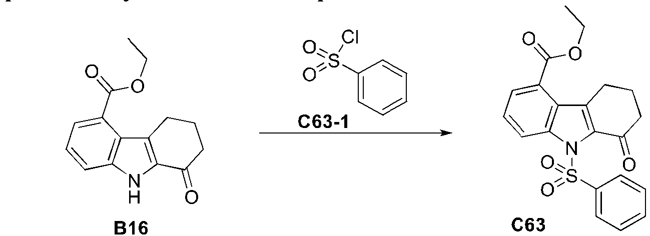

At room temperature, triethylamine (772.3 µL, 5.560 mmol) and compound C63-1 (147.2 mg, 0.834 mmol) were added to a solution of compound B16 (150 mg, 0.556 mmol) and N,N-dimethylaminopyridine (33.9 mg, 0.278 mmol) in dichloromethane (10 mL). The mixture was stirred overnight, then quenched with water and extracted with dichloromethane (2 × 30 mL). The combined organic layers were washed with saturated sodium chloride solution (20 mL), dried over anhydrous sodium sulfate, and concentrated under vacuum. The residue was purified by reverse-phase chromatography (mobile phase A: water (0.1% formic acid), mobile phase B: acetonitrile) to afford compound C63 as a white solid (50.1 mg, 22.69%). ESI/MS (m/z): 398.1 [M+H]⁺.¹H NMR (400 MHz, DMSO-*d*₆) δ 8.48 (dd, *J* = 8.5*,* 1.2 Hz, 1H), 8.15-8.07 (m, 2H), 7.79-7.72 (m, 2H), 7.72-7.68 (m, 1H), 7.68-7.61 (m, 2H), 4.39 (q, *J =* 7.1 Hz, 2H), 2.96 (t, *J =* 6.0 Hz, 2H), 2.61-2.53 (m, 2H), 2.10-1.99 (m, 2H), 1.36 (t, *J =* 7.1 Hz, 3H).

### Example 312: Synthesis of Compound C64

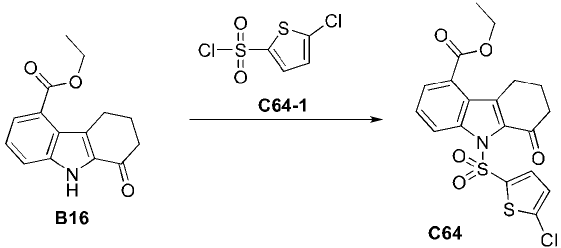

At room temperature, triethylamine (772.3 µL, 5.560 mmol) and compound C64-1 (180.9 mg, 0.834 mmol) were added to a solution of compound B16 (150 mg, 0.556 mmol) and N,N-dimethylaminopyridine (33.9 mg, 0.278 mmol) in dichloromethane (10 mL). The mixture was stirred overnight, then quenched with water and extracted with dichloromethane (2 × 30 mL). The combined organic layers were washed with saturated sodium chloride solution (20 mL), dried over anhydrous sodium sulfate, and concentrated under vacuum. The residue was purified by reverse-phase chromatography (mobile phase A: water (0.1% formic acid), mobile phase B: acetonitrile) to afford compound C64 as a white solid (47.6 mg, 19.56%). ESI/MS (m/z): 438.1 [M+H]⁺.¹H NMR (400 MHz, DMSO-*d*₆) δ 8.35 (d, *J =* 8.5 Hz, 1H), 8.13 (d, *J* = 4.2 Hz, 1H), 7.75 (d, *J =* 7.4 Hz, 1H), 7.73-7.63 (m, 1H), 7.36 (d, *J =* 4.5 Hz, 1H), 4.39 (q, *J =* 7.0 Hz, 2H), 2.98 (t, *J =* 5.9 Hz, 2H), 2.72-2.60 (m, 2H), 2.15-2.02 (m, *J =* 8.2, 6.8 Hz, 2H), 1.36 (t, *J =* 7.0 Hz, 3H).

### Example 313: Synthesis of Compound C65

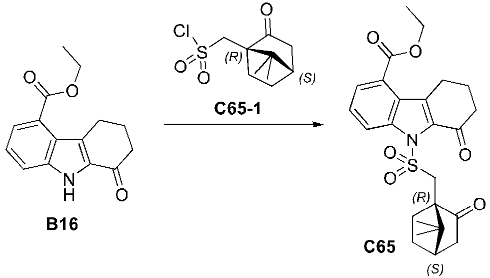

At room temperature, triethylamine (187.4 mg, 1.850 mmol) was added to a solution of compound B16 (100 mg, 0.370 mmol) and N,N-dimethylaminopyridine (22.6 mg, 0.185 mmol) in dichloromethane (10 mL). After stirring for 10 minutes, compound C65-1 (139.3 mg, 0.555 mmol) was slowly added to the above solution. The mixture was stirred overnight at room temperature. The reaction mixture was diluted with water (20 mL) and extracted with dichloromethane (3 × 30 mL). The combined organic layers were washed with saturated sodium chloride solution (40 mL), dried over anhydrous sodium sulfate, and concentrated under vacuum. The residue was purified by reverse-phase chromatography (mobile phase A: water (10 mmol/L ammonium bicarbonate), mobile phase B: acetonitrile) to afford compound C65 as a white solid (21.8 mg, 12.48%). ESI/MS (m/z): 472.2 [M+H]⁺.¹H NMR (400 MHz, Chloroform-*d*) δ 8.39 (dd, *J* = 8.6*,* 1.0 Hz, 1H), 7.75 (dd, *J =* 7.5, 0.9 Hz, 1H), 7.50 (dd, *J =* 8.7, 7.5 Hz, 1H), 4.47-4.42 (m, 2H), 4.40 (d, *J =* 8.6 Hz, 1H), 4.30 (d, *J =* 14.6 Hz, 1H), 3.21-3.02 (m, 2H), 2.84-2.73 (m, 1H), 2.68-2.55 (m, 2H), 2.44 (ddd, *J =* 18.5, 4.9, 3.2 Hz, 1H), 2.23-2.07 (m, 4H), 1.99 (d, *J =* 18.4 Hz, 1H), 1.91-1.81 (m, 1H), 1.54-1.46 (m, 1H), 1.44 (t, *J =* 7.1 Hz, 3H), 1.28 (s, 3H), 1.01 (s, 3H).

### Example 314: Synthesis of Compound C66

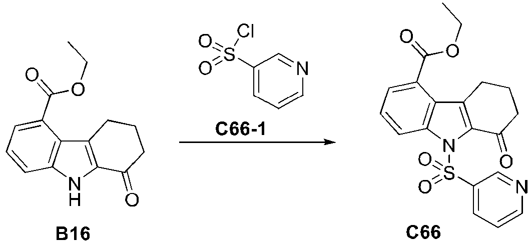

At room temperature, triethylamine (299.9 mg, 2.965 mmol) was added to a solution of compound B16 (160 mg, 0.593 mmol) and N,N-dimethylaminopyridine (14.5 mg, 0.119 mmol) in dichloromethane (10 mL). After stirring for 10 minutes, compound C66-1 (157.9 mg, 0.889 mmol) was slowly added to the above solution. The mixture was stirred overnight at room temperature. The reaction was quenched with water (20 mL) and extracted with ethyl acetate (3 × 30 mL). The combined organic layers were washed with saturated sodium chloride solution (50 mL), dried over anhydrous sodium sulfate, and concentrated under vacuum. The residue was purified by reverse-phase chromatography (mobile phase A: water (0.1% formic acid), mobile phase B: acetonitrile) to afford compound C66 as a white solid (38.9 mg, 16.47%). ESI/MS (m/z): 399.0 [M+H]⁺.¹H NMR (400 MHz, DMSO-*d*₆) δ 9.30 (d, *J* = 2.5 Hz, 1H), 8.95-8.87 (m, 1H), 8.58-8.49 (m, 2H), 7.77 (d, *J =* 7.3 Hz, 1H), 7.74-7.67 (m, 2H), 4.39 (q, *J =* 7.1 Hz, 2H), 2.97 (t, *J =* 6.0 Hz, 2H), 2.58 (t, *J =* 6.4 Hz, 2H), 2.06 (p, *J =* 5.9 Hz, 2H), 1.36 (t, *J =* 7.1 Hz, 3H).

### Example 315: Synthesis of Compound C67

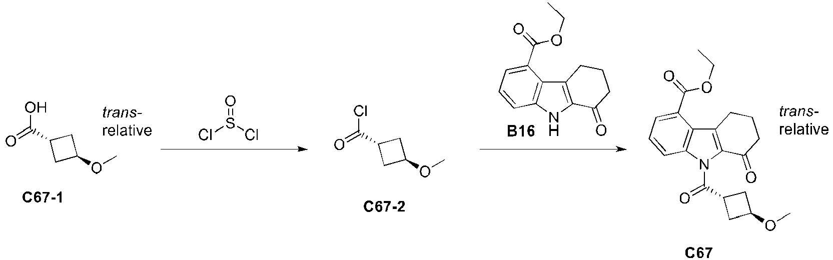

At room temperature, thionyl chloride (914 mg, 7.7 mmol) was added to a solution of compound C67-1 (100 mg, 0.77 mmol) and N,N-dimethylformamide (5.6 mg, 0.077 mmol) in dichloromethane (1 mL). The reaction mixture was stirred at room temperature for 5 hours and then concentrated under vacuum to afford compound C67-2 as a yellow oil (100 mg, 0.67 mmol).

At -5°C, 60% sodium hydride (48 mg, 1.2 mmol) was added to a solution of compound B16 (77 mg, 0.28 mmol) in tetrahydrofuran (5 mL). After stirring for 10 minutes, compound C67-2 (60 mg, 0.4 mmol) was added to the above reaction mixture. The mixture was stirred at 0°C for 2 hours, then quenched with saturated ammonium chloride solution (30 mL) and extracted with ethyl acetate (10 mL × 2). The combined organic layers were dried over anhydrous sodium sulfate and concentrated under vacuum. The residue was purified by reverse-phase preparative chromatography (acetonitrile/water: 10%-50%) to afford compound C67 as a white solid (8 mg, 0.022 mmol, trans, relative). ESI/MS (m/z): 370.1 [M+H]⁺.¹H NMR (500 MHz, DMSO-*d*₆) δ 8.14 (dd, *J* = 8.5, 1.0 Hz, 1H), 7.71 (dd, *J* = 7.5*,* 1.0 Hz, 1H), 7.61 (dd, *J =* 8.5*,* 7.4 Hz, 1H), 4.39 (q, *J =* 7.1 Hz, 2H), 4.13-4.05 (m, 1H), 3.71-3.63 (m, 1H), 3.13 (s, 3H), 3.04 (t, *J =* 6.0 Hz, 2H), 2.64 (dd, *J =* 7.3, 5.7 Hz, 2H), 2.44-2.38 (m, 2H), 2.17-2.10 (m, 4H), 1.36 (t, *J =* 7.1 Hz, 3H).

### Example 316: Synthesis of Compound C69

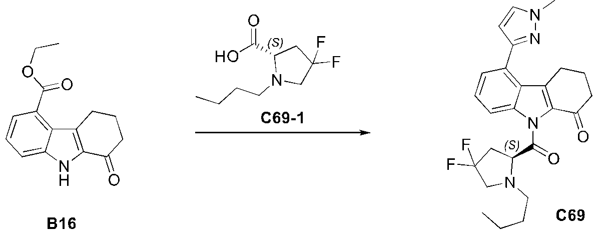

At room temperature, triethylamine (236.5 mg, 2.34 mmol) and 4-pyrrolidinopyridine (13.3 mg, 0.09 mmol) were added to a solution of compound B16 (62.0 mg, 0.23 mmol) and compound C69-1 (51.0 mg, 0.23 mmol) in 1,2-dichloroethane. After thorough mixing, N,N,N',N'-tetramethylchloroformamidinium hexafluorophosphate (72.1 mg, 0.26 mmol) was added, and stirring was continued for 1 hour. The mixture was then heated to 85 °C and stirred for 4 hours. After cooling to room temperature, the reaction mixture was diluted with dichloromethane (20 mL), washed with saturated sodium chloride solution (10 mL), dried over anhydrous sodium sulfate, and concentrated under vacuum. The residue was purified by preparative thin-layer silica gel chromatography (petroleum ether:ethyl acetate = 5:1) to afford compound C69 as a pale yellow solid (20.9 mg, 19.0%). ESI/MS (m/z): 447.2 [M+H]⁺.¹H NMR (500 MHz, DMSO-*d*₆) δ 8.09 (dd, *J =* 8.4, 1.0 Hz, 1H), 7.75 (dd, *J =* 7.4, 1.0 Hz, 1H), 7.64 (dd, *J =* 8.5, 7.4 Hz, 1H), 4.40 (q, *J =* 7.1 Hz, 2H), 4.24-4.18 (m, 1H), 3.40-3.32 (m, 1H), 3.18-3.13 (m, 1H), 3.10-2.97 (m, 2H), 2.77-2.71 (m, 1H), 2.70-2.53 (m, 3H), 2.29-2.23 (m, 1H), 2.22-2.12 (m, 3H), 1.37 (t, *J =* 7.1 Hz, 3H), 1.10-1.02 (m, 1H), 0.97-0.86 (m, 2H), 0.83-0.73 (m, 1H), 0.53 (t, *J* = 7.1 Hz, 3H).

### Example 317: Synthesis of Compound C71

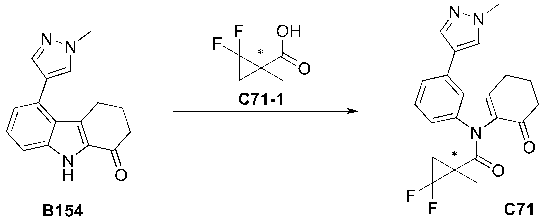

At 0°C, N,N-dimethylformamide (one drop) and oxalyl chloride (37.5 mg, 0.30 mmol) were added to a solution of compound C71-1 (53.2 mg, 0.27 mmol, racemate) in dichloromethane (0.5 mL). The reaction mixture was stirred at room temperature for 2 hours and then concentrated under vacuum to afford the acyl chloride intermediate. At 0°C, this acyl chloride intermediate was added to a solution of compound B154 (25.0 mg, 0.09 mmol), 4-pyrrolidinopyridine (13.3 mg, 0.09 mmol), and N,N-diisopropylethylamine (57.9 mg, 0.45 mmol) in dichloromethane (0.5 mL). The reaction mixture was stirred at room temperature for 2 hours, then quenched with water (5 mL) and extracted with dichloromethane (10 mL × 3). The combined organic layers were washed with saturated sodium chloride solution (10 mL), dried over anhydrous sodium sulfate, and concentrated under vacuum. The residue was purified by reverse-phase preparative chromatography (acetonitrile/water = 10-80%) to afford compound C71 as an off-white solid (30.2 mg, 86.2%, racemate). ESI/MS (m/z): 384.1 [M+H]⁺.¹H NMR (500 MHz, DMSO-*d*₆) δ 7.95 (s, 1H), 7.63 (d, *J =* 0.8 Hz, 1H), 7.61 (dd, *J* = 8.5, 1.0 Hz, 1H), 7.51 (m, 1H), 7.15 (dd, *J =* 7.3, 1.0 Hz, 1H), 3.93 (s, 3H), 2.76-2.69 (m, 3H), 2.49-2.44 (m, 1H), 2.15-2.00 (m, 3H), 1.81 (m, 1H), 1.52 (s, 3H).

### Example 318: Synthesis of Compound C72

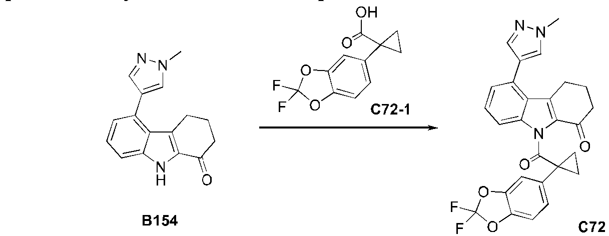

At 0°C, N,N-dimethylformamide (one drop) and oxalyl chloride (60.2 mg, 0.47 mmol) were added to a solution of compound C72-1 (114.9 mg, 0.47 mmol) in dichloromethane (1 mL). The reaction mixture was stirred at room temperature for 2 hours and then concentrated under vacuum to afford the acyl chloride intermediate. At 0°C, this acyl chloride intermediate was added to a solution of compound B154 (53.0 mg, 0.19 mmol), 4-pyrrolidinopyridine (28.1 mg, 0.19 mmol), and N,N-diisopropylethylamine (122.6 mg, 0.95 mmol) in dichloromethane (0.5 mL). The reaction mixture was stirred at room temperature for 2 hours, then quenched with water (5 mL) and extracted with dichloromethane (10 mL × 3). The combined organic layers were washed with saturated sodium chloride solution, dried over anhydrous sodium sulfate, and concentrated under vacuum. The residue was purified by reverse-phase preparative chromatography (acetonitrile/water = 10-70%) to afford compound C72 as a white solid (11.5 mg, 11.9%). ESI/MS (m/z): 490.1 [M+H]⁺.¹H NMR (500 MHz, DMSO-*d*₆) δ 7.88 (d, *J =* 0.8 Hz, 1H), 7.62 (dd, *J =* 8.4, 0.9 Hz, 1H), 7.53 (d, *J =* 0.8 Hz, 1H), 7.50 (dd, *J =* 8.5, 7.3 Hz, 1H), 7.16 (d, *J =* 8.4 Hz, 1H), 7.10 (dd, *J* = 7.3, 0.9 Hz, 1H), 6.99 (d, *J =* 1.8 Hz, 1H), 6.79 (dd, *J =* 8.4, 1.8 Hz, 1H), 3.90 (s, 3H), 2.48 (d, *J =* 5.9 Hz, 2H), 2.37-2.28 (m, 2H), 1.84 (q, *J =* 3.7 Hz, 2H), 1.59 (ddd, *J =* 19.7, 7.0, 4.7 Hz, 4H).

### Example 319: Synthesis of Compound C73

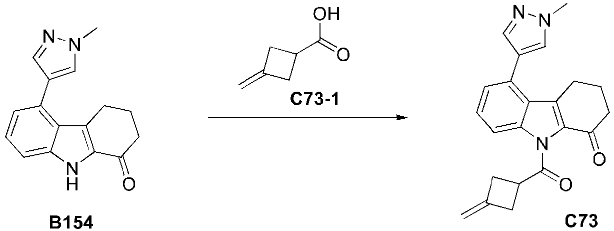

At 0°C, N,N-dimethylformamide (one drop) and oxalyl chloride (36.1 mg, 0.28 mmol) were added to a solution of compound C73-1 (31.9 mg, 0.28 mmol) in dichloromethane (0.5 mL). The reaction mixture was stirred at room temperature for 2 hours and then concentrated under vacuum to afford the acyl chloride intermediate. At 0°C, this acyl chloride intermediate was added to a solution of compound B154 (53.0 mg, 0.19 mmol), 4-pyrrolidinopyridine (28.1 mg, 0.19 mmol), and N,N-diisopropylethylamine (98.1 mg, 0.76 mmol) in dichloromethane (0.5 mL). The reaction mixture was stirred at room temperature for 2 hours, then quenched with water (5 mL) and extracted with dichloromethane (10 mL × 3). The combined organic layers were washed with saturated sodium chloride solution, dried over anhydrous sodium sulfate, and concentrated under vacuum. The residue was purified by reverse-phase preparative chromatography (acetonitrile/water = 10-70%) to afford compound C73 as a white solid (41.3 mg, 59.3%). ESI/MS (m/z): 360.1 [M+H]⁺.¹H NMR (500 MHz, DMSO-*d*₆) δ 7.95 (dd, *J =* 8.5, 0.9 Hz, 1H), 7.93 (d, *J =* 0.7 Hz, 1H), 7.61 (d, *J =* 0.8 Hz, 1H), 7.51 (dd, *J =* 8.5, 7.3 Hz, 1H), 7.15 (dd, *J =* 7.3, 1.0 Hz, 1H), 4.80 (p, *J =* 2.4 Hz, 2H), 3.93 (s, 3H), 3.79 (tt, *J =* 8.8, 7.4 Hz, 1H), 2.95-2.81 (m, 4H), 2.70 (t, *J =* 6.0 Hz, 2H), 2.58 (dd, *J =* 7.2, 5.7 Hz, 2H), 2.07-1.98 (m, 2H).

### Example 320: Synthesis of Compound C74

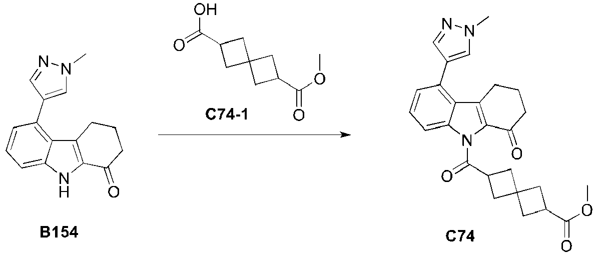

At 0°C, N,N-dimethylformamide (one drop) and oxalyl chloride (30.0 mg, 0.24 mmol) were added to a solution of compound C74-1 (42.6 mg, 0.21 mmol) in dichloromethane (0.5 mL). The reaction mixture was stirred at room temperature for 2 hours and then concentrated under vacuum to afford the acyl chloride intermediate. At 0°C, this acyl chloride intermediate was added to a solution of compound B154 (30.0 mg, 0.11 mmol), 4-pyrrolidinopyridine (15.9 mg, 0.11 mmol), and N,N-diisopropylethylamine (69.4 mg, 0.54 mmol) in dichloromethane (0.5 mL). The reaction mixture was stirred at room temperature for 2 hours, then quenched with water (5 mL) and extracted with dichloromethane (10 mL × 3). The combined organic layers were washed with saturated sodium chloride solution, dried over anhydrous sodium sulfate, and concentrated under vacuum. The residue was purified by reverse-phase preparative chromatography (acetonitrile/water = 10-80%) to afford compound C74 as a white solid (25.7 mg, 52.6%). ESI/MS (m/z): 446.1 [M+H]⁺.¹H NMR (500 MHz, DMSO-*d*₆) δ 7.93 (d, *J =* 0.8 Hz, 1H), 7.89 (dd, *J =* 8.5, 1.0 Hz, 1H), 7.61 (d, *J =* 0.8 Hz, 1H), 7.50 (dd, *J =* 8.5, 7.3 Hz, 1H), 7.13 (dd, *J =* 7.3, 1.0 Hz, 1H), 3.92 (s, 3H), 3.66 (p, *J =* 8.5 Hz, 1H), 3.56 (s, 3H), 2.98 (p, *J =* 8.4 Hz, 1H), 2.69 (t, *J =* 5.9 Hz, 2H), 2.58 (dd, *J =* 7.2, 5.6 Hz, 2H), 2.31-2.08 (m, 8H), 2.05-1.97 (m, 2H).

### Example321: Synthesis of Compound C75

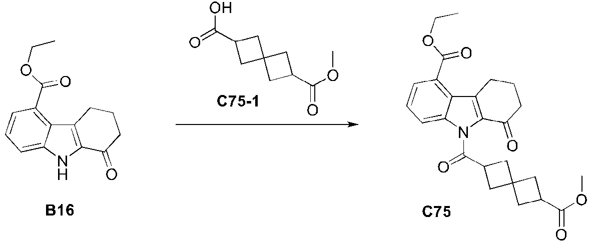

At 0°C, N,N-dimethylformamide (one drop) and oxalyl chloride (47.7 mg, 0.37 mmol) were added to a mixture of compound C75-1 (29.3 mg, 0.15 mmol) in dichloromethane (0.5 mL). The mixture was stirred at room temperature for 2 hours and then concentrated under vacuum to afford the acyl chloride intermediate. At 0°C, 4-dimethylaminopyridine (9.0 mg, 0.074 mmol), N,N-diisopropylethylamine (47.7 mg, 0.37 mmol), and the above acyl chloride intermediate were added to a solution of compound B16 (20.0 mg, 0.074 mmol) in dichloromethane (0.5 mL). The reaction mixture was stirred at room temperature for 2 hours, then diluted with dichloromethane (10 mL), washed sequentially with 0.5M aqueous hydrochloric acid solution (10 mL) and saturated sodium chloride solution (10 mL), dried over anhydrous sodium sulfate, and concentrated under vacuum. The residue was purified by reverse-phase preparative chromatography (acetonitrile/water = 10-80%) to afford compound C75 as a brown oil (4.1 mg, 12.3%). ESI/MS (m/z): 438.1 [M+H]⁺.

### Example 322: Cell-based pharmacodynamic assay method and results

Complete media such as RPMI 1640 (Gibco, A1049101), EMEM (ATCC, 30-2003), DMEM (Gibco, C11995500CP), McCoy's 5A (Gibco, 16600082), and F-12K (ATCC, 30-2004) were prepared by supplementing with varying concentrations of FBS (Biological Industries, 04-002-1A) and 1% Penicillin-Streptomycin (Gibco, 15140122) and was thoroughly mixed. Tumor cell lines including HeLa (ATCC, CCL-2; EMEM + 10% FBS), TOV-21G (Cobioer, CBP60292; 1:1 mixture of MCDB 105 and Medium 199 + 15% FBS), OVCAR-3 (ATCC, HTB-161; RPMI 1640 + 20% FBS + 0.01 mg/ml bovine insulin), SK-OV-3 (ATCC, HTB-77; McCoy's 5A + 10% FBS), A2780 (Cobioer, CBP60283; RPMI 1640 + 10% FBS), OC316 (Cobioer, CBP60620; RPMI 1640 + 10% FBS), HuH-7 (Cobioer, CBP60202; DMEM + 10% FBS), SNU-398 (ATCC, CRL-2233; RPMI 1640 + 10% FBS), NCI-H2286 (ATCC, CRL-5938; RPMI 1640 + 5% FBS), NCI-H82 (ATCC, HTB-175; RPMI 1640 + 10% FBS), A549 (ATCC, CCL-185; F-12K + 10% FBS), SK-MEL-3 (ATCC, HTB-69; McCoy's 5A + 15% FBS), PSN-1 (Cobioer, CBP61215; RPMI 1640 + 10% FBS), LoVo (ATCC, CCL-229; F-12K + 10% FBS), DLD-1 (ATCC, CCL-221; RPMI 1640 + 10% FBS), HCT-15 (ATCC, CCL-225; RPMI 1640 + 10% FBS), A498 (ATCC, HTB-44; EMEM + 10% FBS), LNCaP (ATCC, CRL-1740; RPMI 1640 + 10% FBS), SNU-1 (ATCC, CRL-5971; RPMI 1640 + 10% FBS), SCC-9 (Cobioer, CBP60428; DMEM/F12 (1:1) + 10% FBS + 0.5 mM sodium pyruvate + 400 ng/ml hydrocortisone), SU-DHL-5 (ATCC, CRL-2958; RPMI 1640 + 10% FBS), Toledo (ATCC, CRL-2631; RPMI 1640 + 10% FBS), and SJCRH30 (ATCC, CRL-2061; RPMI 1640 + 10% FBS) were thawed. Cells were passaged approximately twice, and those in good growth condition (typically passages 4-6) were selected for experiments. The medium was aspirated, and the cells were washed once with 0.25% trypsin (Invitrogen, 25200-072). The waste liquid was discarded, and 3 mL of fresh trypsin was added to the culture flask for digestion. When the cells began to detach, 9 mL of complete medium was added to neutralize the trypsin digestion, and the mixture was gently mixed. The cell suspension was transferred to a centrifuge tube using a pipette and centrifuged at 1000 rpm for 5 minutes. The supernatant was discarded, and 5 mL of medium was added to the centrifuge tube to gently resuspend the cells evenly. The cell concentration was adjusted to 1×10⁴/mL-3×10⁴/mL using a cell counter (Beckman Coulter, Vi-cellTM XR). The cell suspension was added to a 96-well plate (Corning, 3903) at 100 µL/well. The plate was labeled with details such as cell name, seeding density, and date, and then placed in a CO₂ incubator (Thermo, Thermo 3111) overnight. Test compounds were prepared as 20 mM stock solutions in DMSO (Sigma, D4540-1L) and added to the corresponding cell wells using an HPD300 automated dispenser according to the designed layout. The plates were incubated in a 37°C, 5% CO₂ incubator for 72 hours. CellTiter-Glo Buffer was thawed at room temperature. The lyophilized CellTiter-Glo substrate was equilibrated to room temperature. The CellTiter-Glo Buffer was added to the CellTiter-Glo substrate and thoroughly mixed. The cell plates were removed and equilibrated to room temperature, and 50 µL of the mixed CellTiter-Glo reagent was added to each well. The plates were protected from light and shaken for 10 minutes, followed by incubation for 10 minutes. The plates were placed in an Enspire reader (luminescence measurement time: 0.5 s) to record luminescence results. The inhibition rate was calculated using the following formula: Inhibition rate (%) = (1 - (RLU_compound - RLU_blank) / (RLU_DMSO - RLU_blank)) × 100%. Dose-response curves were plotted using XLFit to calculate IC₅₀ values. A four-parameter model was used: [fit = (A + ((B - A) / (1 + ((C/x) ^ D)))]. The test results for HeLa cells are shown in Table 1.

**Table 1: IC₅₀ values of some test compounds against human cervical cancer HeLa cells**

| NO. | IC₅₀ against HeLa cells (µM) |
|---|---|
| B01 | ≤ 0.2 |
| B09 | ≤ 0.2 |
| B10 | ≤ 0.2 |
| B14 | ≥ 5.0 |
| B15 | ≤ 0.2 |
| B16 | ≤ 0.2 |
| B17 | ≤ 0.2 |
| B18 | ≤ 0.5 |
| B19 | ≤ 0.2 |
| B23 | ≤ 0.2 |
| B24 | ≤ 0.2 |
| B25 | ≤ 0.2 |
| B26 | ≤ 0.2 |
| B27 | ≤ 0.5 |
| B30 | ≤ 0.2 |
| B31 | ≤ 0.2 |
| B32 | ≤ 0.5 |
| B33 | ≤ 0.2 |
| B34 | ≤ 0.5 |
| B36 | ≤ 0.2 |
| B37 | ≤ 1.0 |
| B38 | ≤ 0.2 |
| B39 | ≤ 0.5 |
| B40 | ≤ 0.2 |
| B41 | ≤ 0.2 |
| B42 | ≤ 0.2 |
| B43 | ≤ 0.5 |
| B44 | ≤ 0.2 |
| B45 | ≤ 0.2 |
| B46 | ≤ 0.2 |
| B48 | ≤ 0.5 |
| B50 | ≤ 0.2 |
| B51 | ≤ 0.2 |
| B55 | ≤ 0.2 |
| B56 | ≤ 0.2 |
| B58 | ≤ 0.2 |
| B61 | ≤ 0.2 |
| B62 | ≤ 0.2 |
| B63 | ≤ 0.2 |
| B65 | ≤ 0.2 |
| B66 | ≤ 0.2 |
| B67 | ≤ 0.2 |
| B68 | ≤ 0.2 |
| B69 | ≤ 0.2 |
| B70 | ≤ 0.2 |
| B71 | ≤ 0.2 |
| B74 | ≤ 0.2 |
| B75 | ≤ 0.5 |
| B76 | ≤ 0.2 |
| B80 | ≤ 0.5 |
| B82 | ≤ 0.2 |
| B83 | ≤ 0.2 |
| B85 | ≤ 0.2 |
| B87 | ≤ 0.2 |
| B89 | ≤ 0.2 |
| B91 | ≤ 0.2 |
| B94 | ≤ 0.5 |
| B95 | ≤ 0.2 |
| B96 | ≤ 0.2 |
| B97 | ≤ 0.5 |
| B99 | ≤ 0.2 |
| C01 | ≤ 0.2 |
| C03 | ≤ 0.2 |
| C04 | ≤ 0.2 |
| C05 | ≤ 0.2 |
| E01 | ≤ 0.5 |
| E02 | ≤ 0.5 |
| E03 | ≤ 0.5 |
| E04R | ≤ 0.2 |
| E05R | ≤ 0.2 |
| E07 | ≤ 0.2 |
| E08 | ≤ 0.2 |
| E09 | ≤ 0.5 |
| E10 | ≤ 0.2 |
| E11 | ≤ 0.2 |
| E13 | ≤ 0.2 |
| E14 | ≤ 0.2 |
| E29 | ≤ 0.2 |
| E30 | ≤ 0.2 |
| E31 | ≤ 0.2 |
| E32 | ≤ 0.2 |
| E33 | ≤ 0.5 |
| E34 | ≤ 0.2 |
| E35 | ≤ 0.2 |
| E36 | ≤ 0.2 |
| E37 | ≤ 0.2 |
| E38 | ≤ 0.2 |
| E39 | ≤ 0.2 |
| E40 | ≤ 0.2 |
| E41 | ≤ 0.5 |
| E42 | ≤ 0.2 |
| E43 | ≤ 0.2 |
| E44 | ≤ 0.5 |
| E45 | ≤ 0.2 |
| E46 | ≤ 0.2 |
| E47 | ≤ 0.2 |
| E48 | ≤ 0.2 |
| E49 | ≤ 0.5 |
| E50 | ≤ 0.2 |
| E52 | ≤ 0.2 |
| E54 | ≤ 0.2 |
| E55 | ≤ 0.2 |
| E56 | ≤ 0.2 |
| E57 | ≤ 0.2 |
| E58 | ≤ 0.2 |
| E59 | ≤ 0.2 |
| E60 | ≤ 0.2 |
| E61 | ≤ 0.2 |
| E62 | ≤ 0.2 |
| E63 | ≤ 0.5 |
| E64 | ≤ 0.5 |
| E65 | ≤ 0.2 |
| E66 | ≤ 0.2 |
| E67 | ≤ 0.2 |
| E68 | ≤ 0.2 |
| E69 | ≤ 0.2 |
| E70 | ≤ 0.2 |
| E71 | ≤ 0.2 |
| E72 | ≤ 0.2 |
| B103 | ≤ 0.2 |
| B105 | ≤ 0.5 |
| B107 | ≤ 0.2 |
| B110 | ≤ 0.2 |
| B111 | ≤ 0.2 |
| B114 | ≤ 0.2 |
| B116 | ≤ 0.2 |
| B117 | ≤ 0.2 |
| B118 | ≤ 0.2 |
| B120 | ≤ 0.2 |
| B121 | ≤ 0.5 |
| B123 | ≤ 0.2 |
| B124 | ≤ 0.2 |
| B126 | ≤ 0.2 |
| B129 | ≤ 0.2 |
| B130 | ≤ 0.2 |
| B131 | ≤ 0.5 |
| B132 | ≤ 0.5 |
| B134 | ≤ 0.2 |
| B136 | ≤ 0.2 |
| B137 | ≤ 0.2 |
| B138 | ≤ 0.5 |
| B139 | ≤ 0.2 |
| B140 | ≤ 0.2 |
| B141 | ≤ 0.2 |
| B142 | ≤ 0.2 |
| B147 | ≤ 0.2 |
| B148 | ≤ 0.2 |
| B149 | ≤ 0.2 |
| B150 | ≤ 0.2 |
| B153 | ≤ 0.2 |
| B154 | ≤ 0.2 |
| B155 | ≤ 0.2 |
| B156 | ≤ 0.2 |
| B160 | ≤ 0.2 |
| B163 | ≤ 0.2 |
| B164 | ≤ 0.2 |
| B166 | ≤ 0.5 |
| B173 | ≤ 0.2 |
| B175 | ≤ 0.5 |
| B177 | ≤ 0.2 |
| B180 | ≤ 0.5 |
| B181 | ≤ 0.2 |
| B186 | ≤ 0.5 |
| B189 | ≤ 0.5 |
| B191 | ≤ 0.2 |
| B193 | ≤ 0.2 |
| B194 | ≤ 0.2 |
| B196 | ≤ 0.2 |
| C14 | ≤ 0.2 |
| C15 | ≤ 0.2 |
| C16 | ≤ 0.2 |
| C17 | ≤ 0.2 |
| C18 | ≤ 0.2 |
| C19 | ≤ 0.2 |
| C20 | ≤ 0.2 |
| C21 | ≤ 0.2 |
| C22 | ≤ 0.2 |
| C23 | ≤ 0.2 |
| C24 | ≤ 0.2 |
| C25 | ≤ 0.2 |
| C26 | ≤ 0.2 |
| C27 | ≤ 0.2 |
| C28 | ≤ 0.2 |
| C29 | ≤ 0.2 |
| C30 | ≤ 0.2 |
| C31 | ≤ 0.2 |
| C32 | ≤ 0.2 |
| C33 | ≤ 0.2 |
| C34 | ≤ 0.2 |
| C35 | ≤ 0.2 |
| C36 | ≤ 0.2 |
| C37 | ≤ 0.2 |
| C38 | ≤ 0.2 |
| C42 | ≤ 0.2 |
| C43 | ≤ 0.2 |
| C44 | ≤ 0.2 |
| C45 | ≤ 0.2 |
| C48 | ≤ 0.2 |
| C49 | ≤ 0.2 |
| C50 | ≤ 0.2 |
| C51 | ≤ 0.2 |
| C52 | ≤ 0.2 |
| C55 | ≤ 0.2 |
| C56 | ≤ 0.2 |
| C57 | ≤ 0.5 |
| C58 | ≤ 0.2 |
| C59 | ≤ 0.2 |
| C60 | ≤ 0.2 |

Compounds B16, B67, and C05 exhibited IC₅₀ values ranging from 0.2 µM to 10 µM against the following tumor cell lines: ovarian cancer cell lines (TOV-21G, OVCAR-3, SK-OV-3, A2780, OC316), liver cancer cell lines (HuH-7, SNU-398), small cell lung cancer cell lines (NCI-H2286, NCI-H82), non-small cell lung cancer cell line (A549), melanoma cell line (SK-MEL-3), pancreatic cancer cell line (PSN-1), colorectal cancer cell lines (LoVo, DLD-1, HCT-15), renal cancer cell line (A498), prostate cancer cell line (LNCaP), gastric cancer cell line (SNU-1), tongue squamous cell carcinoma cell line (SCC-9), leukemia cell lines (SU-DHL-5, Toledo), and rhabdomyosarcoma cell line (SJCRH30).

Furthermore, CalcuSyn 2.0 software (Biosoft, Cambridge, UK), based on the Chou and Talalay method (Chou and Talalay, 1984, Adv Enzyme Regul 22: 27), was used to calculate the Combination Index (CI) at the single-agent IC₅₀ for representative compounds E63, E01, and C05 in combination with various other compounds. The software categorizes CI values as follows: CI < 0.1 indicates very strong synergism, CI 0.1-0.3 indicates strong synergism, CI 0.3-0.7 indicates synergism, CI 0.7-0.85 indicates moderate synergism, CI 0.85-0.90 indicates mild synergism, CI 0.9-1.1 indicates additive effects, and CI > 1.10 indicates antagonism. The results showed that when compounds E63, E01, and C05 were combined with PARP inhibitors (Olaparib, Rucaparib, Niraparib, Talazoparib, Fluzoparib, Pamiparib, Saruparib), proteasome inhibitor (Ixazomib), MEK kinase inhibitor (Cobimetinib), DNA methyltransferase inhibitor (SGI-1027), histone deacetylase inhibitor (Trichostatin A), nucleoside analog (6-Mercaptopurine, 6-MP), chemotherapeutic agents (cisplatin, Cabazitaxel), or GLUT1/GLUT3 inhibitors (KL-11743, BAY-876), they exhibited varying degrees of combinatorial effects on the proliferation inhibition of the aforementioned tumor cell lines. The CI values at IC₅₀ ranged from 0.01 to 0.8, indicating moderate to very strong synergism.

All documents mentioned in this invention are incorporated herein by reference as if each document were individually incorporated by reference. Furthermore, it should be understood that after reading the above teachings of the present invention, those skilled in the art may make various modifications or alterations to the invention. These equivalent forms shall also fall within the scope defined by the appended claims of this application.

## Claims

1. A compound represented by formula (I) or formula (II), a stereoisomer, geometric isomer, hydrate, solvate, prodrug, or pharmaceutically acceptable salt thereof, 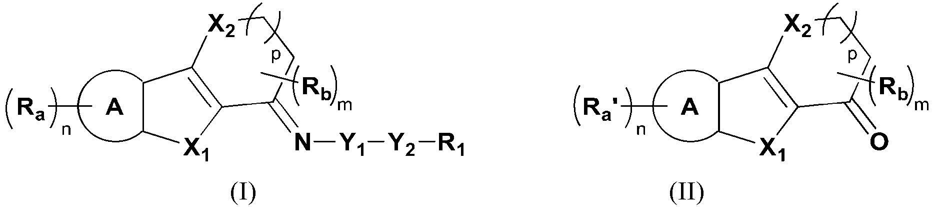
wherein ring A is selected from the group consisting of C₃-C₇ saturated or partially unsaturated carbocycle, 3-8 membered saturated or partially unsaturated heterocycle, C₆-C₁₀ aromatic ring, and 5-12 membered heteroaromatic ring; ring A may be a monocyclic, spirocyclic, fused, or bridged ring structure;
Y₁ and Y₂ are each independently selected from the group consisting of chemical bond, C₁-C₆ alkylene, C₃-C₁₀ saturated or partially unsaturated carbocycle, 3-10 membered saturated or partially unsaturated heterocycle, C₆-C₁₀ aromatic ring, 5-12 membered heteroaromatic ring, -NH-, 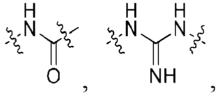 O and S; and Y₁ and Y₂ are each independently optionally substituted with 0 to 4 substituents selected from the group consisting of deuterium, hydroxyl, halogen, oxo group (=O), 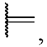 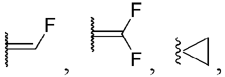 carboxyl, NH₂, mercapto, C₁-C₄ alkyl, C₂-C₄ alkenyl, C₂-C₄ alkynyl, C₂-C₄ acyl, C₂-C₆ ester group, C₁-C₄ alkoxy, C₁-C₄ alkylthio, C₁-C₄ haloalkyl, C₂-C₄ haloacyl, C₁-C₄ haloalkoxy, C₁-C₄ haloalkylthio, C₃-C₇ saturated or partially unsaturated carbocyclyl, 3-8 membered saturated or partially unsaturated heterocyclyl, C₆-C₁₀ aryl, and C₅-C₁₂ heteroaryl;
R₁ is selected from the group consisting of hydrogen, deuterium, C₁-C₆ alkyl, C₂-C₆ alkenyl, C₂-C₆ alkynyl, C₁-C₆ alkylamino (C₁-C₆ alkyl-NH-), C₁-C₆ alkoxy, C₁-C₆ haloalkyl, C₂-C₆ haloalkenyl, C₂-C₆ haloalkynyl, C₁-C₆ haloalkylamino, C₁-C₆ haloalkoxy, C₃-C₇ saturated or partially unsaturated carbocyclyl, 3-8 membered saturated or partially unsaturated heterocyclyl, C₆-C₁₀ aryl, 5-12 membered heteroaryl, C₁-C₄ alkyl-(C₃-C₇ saturated or partially unsaturated carbocyclyl), C₁-C₄ alkyl-(3-8 membered saturated or partially unsaturated heterocyclyl), C₁-C₄ alkyl-(C₆-C₁₀ aryl), C₁-C₄ alkyl-(5-12 membered heteroaryl), and S(O)₂(C₁-C₆ alkyl); wherein said alkyl, alkenyl, alkynyl, alkylamino, alkoxy, haloalkyl, haloalkenyl, haloalkynyl, haloalkylamino, haloalkoxy, carbocyclyl, heterocyclyl, aryl, and heteroaryl are each independently optionally substituted with 0, **1,** 2, 3 or 4 substituents selected from the group consisting of deuterium, hydroxyl, halogen, oxo group (=O), carboxyl, NH₂, mercapto, C₁-C₄ alkyl, C₂-C₄ acyl, C₂-C₆ ester group, C₁-C₄ alkoxy, C₁-C₄ alkylthio, C₁-C₄ haloalkyl, C₂-C₄ haloacyl, C₁-C₄ haloalkoxy, C₁-C₄ haloalkylthio, C₃-C₇ saturated or partially unsaturated carbocyclyl, 3-8 membered saturated or partially unsaturated heterocyclyl, C₆-C₁₀ aryl, and C₅-C₁₂ heteroaryl; said carbocyclyl, heterocyclyl, aryl, and heteroaryl may be monocyclic, spirocyclic, fused, or bridged ring structures;
X₁ is selected from the group consisting of: O, NR₂ and S; wherein R₂ is selected from the group consisting of H, C₁-C₄ alkyl, S(=O)₂R₁₁, S(=O)₂(C₁-C₆ alkylene)R₁₁, C(O)R₁₁, C(O)(C₁-C₆ alkylene)R₁₁, 3-12 membered saturated or partially unsaturated heterocyclyl, and 5-12 membered heteroaryl;
and one or more hydrogen atoms on R₂ may optionally be substituted by substituents selected from the group consisting of C₁-C₄ alkyl, C₂-C₄ alkenyl, C₂-C₄ alkynyl, C₁-C₄ alkoxy, and -C₀-C₄ alkylene-C₆-C₁₀ aryl; or two substituents on R₂ together form a -(CH₂)ₛ- structure; herein the s is 2 or 3.
X₂ is selected from the group consisting of CH₂, CD₂, CHR_{b}, NH, NR_{b}, S, S(=O), S(=O)₂, and S(=O)(=NH);
each Rₐ is independently selected from the group consisting of deuterium, hydroxyl, halogen, amino, nitro, C₁-C₄ alkyl, C₂-C₄ alkenyl, C₂-C₄ alkynyl, C₂-C₆ ester group, C₁-C₄ alkoxy, C₁-C₄ haloalkyl, C₁-C₄ haloalkoxy, C₃-C₇ saturated or partially unsaturated carbocyclyl, 3-8 membered saturated or partially unsaturated heterocyclyl, C₆-C₁₀ aryl, 5-12 membered heteroaryl, -NH-CH₂-(3-12 membered saturated or partially unsaturated heterocyclyl), -NH-CH₂-(5-12 membered heteroaryl), -P(O)R_{c1}R_{d1}, -S(O)₂R_{c1}, -S(O)₂NH₂, -S(O)(NH₂)(R_{c1}), -C(O)C(O)₂R_{c1}, - C(O)NHR_{c1}, -NH-C(O)R_{c1}, -C(O)OR_{c1}, -NH-S(O)₂R_{c1}, -N=S(O)R_{c1}R_{d1}, -CH₂-S(O)(NH)(R_{c1}), -NR_{c1}R_{d1} and 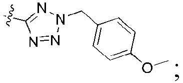 wherein the R_{c1} and R_{d1} are each independently selected from the group consisting of deuterium, C₁-C₄ alkyl, C₂-C₄ alkenyl, C₂-C₄ alkynyl, C₃-C₇ saturated or partially unsaturated carbocyclyl, 3-8 membered saturated or partially unsaturated heterocyclyl, C₆-C₁₀ aryl, and 5-12 membered heteroaryl; or R_{c1}, R_{d1}, and the atom(s) to which they are attached together form a 3-8 membered saturated or partially unsaturated heterocyclyl or a 5-12 membered heteroaryl; wherein one or more hydrogen atoms on Rₐ may optionally be substituted by substituents selected from the group consisting of deuterium atom, hydroxyl, halogen, oxo group (=O), 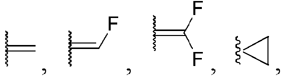 C₁-C₄ alkyl, C₂-C₄ alkenyl, C₂-C₄ alkynyl, S(O)₂CH₃, C₃-C₇ saturated or partially unsaturated carbocyclyl, -C(O)N(C₁-C₄ alkyl)₂, and -N(C₁-C₄ alkyl)₂;
each Rₐ' is independently selected from the group consisting of: deuterium, hydroxyl, halogen, amino, nitro, cyano, C₁-C₄ alkyl, C₂-C₄ alkenyl, C₂-C₄ alkynyl, C₂-C₆ ester group, C₁-C₄ alkoxy, C₁-C₄ haloalkyl, C₁-C₄ haloalkoxy, C₁-C₄ amido, C₃-C₇ saturated or partially unsaturated carbocyclyl, 3-12 membered saturated or partially unsaturated heterocyclyl, C₆-C₁₀ aryl, 5-12 membered heteroaryl, -NH-CH₂-(3-12 membered saturated or partially unsaturated heterocyclyl), -NH-CH₂-(5-12 membered heteroaryl), -P(O)R_{c}R_{d}, -S(O)₂R_{c}, -S(O)₂NH₂, -S(O)(NH₂)(R_{c}), - C(O)C(O)₂R_{c}, -C(O)NHR_{c}, -C(O)NR_{c}R_{d}, -NH-C(O)R_{c}, -OC(O)R_{c}, -C(O)OR_{c}, - C(O)O(C₁-C₄ alkylene)R_{c}, -NH-S(O)₂R_{c}, -C(O)NH(C₁-C₄ alkylene)R_{c}, -N=S(O)R_{c}R_{d}, -CH₂-S(O)(NH)(R_{c}), -NR_{c}R_{d}, and 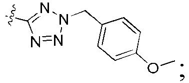
wherein R_{c} and R_{d} are each independently selected from the group consisting of: deuterium, C₁-C₄ alkyl, C₁-C₄ alkoxy, C₂-C₄ alkenyl, C₂-C₄ alkynyl, C₃-C₇ saturated or partially unsaturated carbocyclyl, 3-8 membered saturated or partially unsaturated heterocyclyl, C₆-C₁₀ aryl, 8-12 membered fused bicyclic ring, 5-12 membered heteroaryl, -N(C₁-C₄ alkyl)₂, -CONH₂, and -CON(C₁-C₄ alkyl)₂; or R_{c}, R_{d} and the nitrogen atom to which they are attached together form a 3-8 membered saturated or partially unsaturated heterocyclyl or a 5-12 membered heteroaryl; wherein one or more hydrogen atoms on Rₐ' may optionally be substituted by substituents selected from the group consisting of deuterium atom, hydroxyl, halogen, amino, cyano, carboxyl, oxo group (=O), 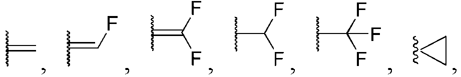 C₁-C₄ haloalkyl, C₁-C₄ haloalkoxy, C₁-C₄ alkyl, C₂-C₄ alkynyl, C₁-C₄ alkoxy, C₁-C₄ alkylthio, HO-(C₁-C₄ alkylene)-, C₁-C₄ alkylthio, C₂-C₄ alkenyl, C₂-C₆ ester group, S(O)₂CH₃, C₃-C₇ saturated or partially unsaturated carbocyclyl, -C(O)N(C₁-C₄ alkyl)₂, -N(C₁-C₄ alkyl)₂, and C₆-C₁₀ aryl;
n is 0, 1, 2, 3, or 4;
each R_{b} is independently selected from the group consisting of deuterium, hydroxyl, halogen, amino, nitro, oxo group (=O), C₁-C₄ alkyl, C₂-C₄ alkenyl, C₂-C₄ alkynyl, C₁-C₄ alkoxy, C₁-C₄ haloalkyl, C₁-C₄ haloalkoxy, -NH-(C₁-C₄ alkyl), -NH-(C₁-C_{y} alkyl-hydroxy), and 4-7 membered heterocyclyl; or two R_{b} groups, together with the carbon atom to which they are attached, form a C₃-C₇ saturated or partially unsaturated carbocyclyl, a 3-8 membered saturated or partially unsaturated heterocyclyl, a C₆-C₁₀ aryl, or a 5-12 membered heteroaryl.
R₁₁ is selected from the group consisting of amino, C₁-C₁₀ alkyl, C₁-C₁₀ alkoxy, C₂-C₁₀ alkenyl, C₂-C₁₀ alkynyl, C₃-C₇ saturated or partially unsaturated carbocyclyl, 3-12 membered saturated or partially unsaturated heterocyclyl, -C₂-C₄ alkynylene-3-12 membered saturated or partially unsaturated heterocyclyl, -O-3-12 membered saturated or partially unsaturated heterocyclyl, -C₆-C₁₀ aryl, -C₂-C₄ alkenylene-C₆-C₁₀ aryl, 5-12 membered heteroaryl, 8-12 membered fused bicyclic ring, -NH-CH₂-3-12 membered saturated or partially unsaturated heterocyclyl, and -NH-CH₂-5-12 membered heteroaryl; wherein one or more hydrogen atoms on R₁₁ may optionally be substituted by substituents selected from the group consisting of deuterium atom, hydroxyl, halogen, amino, carboxylic acid, oxo group (=O), 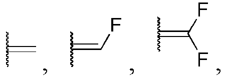 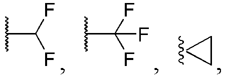 C₁-C₄ haloalkyl, C₁-C₄ haloalkoxy, C₁-C₄ alkyl, C₂-C₄ alkynyl, C₁-C₄ alkoxy, C₁-C₄ alkylthio, C₂-C₄ alkenyl, C₂-C₆ ester group, S(O)₂CH₃, C₃-C₇ saturated or partially unsaturated carbocyclyl, -C(O)N(C₁-C₄ alkyl)₂, -N(C₁-C₄ alkyl)₂, C₆-C₁₀ aryl, and 3-12 membered saturated or partially unsaturated heterocyclyl;
m is 0, 1, 2, 3 or 4;
p is 0, 1, 2 or 3;
with the proviso that when Rₐ is methyl, n is 1, and m is 0, -Y₁-Y₂-R₁ is not a group selected from: OH, NH₂, -C(O)NH₂,

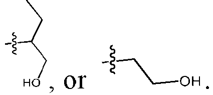

2. The compound according to claim 1, stereoisomer, geometric isomer, hydrate, solvate, prodrug, or pharmaceutically acceptable salt thereof, the ring A is C₆-C₁₀ aromatic ring or a 5-12 membered heteroaromatic ring;
preferably, ring A is benzene ring, pyrimidine ring, or pyridine ring.

3. The compound according to claim 1, stereoisomer, geometric isomer, hydrate, solvate, prodrug, or pharmaceutically acceptable salt thereof, **characterized in that** the R₁ is selected from the group consisting of C₁-C₅ alkyl, C₁-C₅ alkylamino, C₁-C₅ alkoxy, C₁-C₅ haloalkyl, C₃-C₆ saturated or partially unsaturated carbocyclyl, 4-7 membered saturated or partially unsaturated heterocyclyl, C₆-C₁₀ aryl, 5-10 membered heteroaryl, C₁-C₄ alkyl-(C₃-C₆ saturated or partially unsaturated carbocyclyl), C₁-C₄ alkyl-(4-7 membered saturated or partially unsaturated heterocyclyl), C₁-C₄ alkyl-(C₆-C₁₀ aryl), and C₁-C₄ alkyl-(5-10 membered heteroaryl); wherein said alkyl, haloalkyl, alkylamino, carbocyclyl, heterocyclyl, aryl, and heteroaryl are each independently optionally substituted with 0-4 substituents selected from the group consisting of deuterium, hydroxyl, halogen, oxo group (=O), carboxyl, mercapto, C₁-C₄ alkyl, C₂-C₄ acyl, C₁-C₄ alkoxy, C₁-C₄ alkylthio, C₃-C₆ saturated or partially unsaturated carbocyclyl, 4-7 membered saturated or partially unsaturated heterocyclyl, C₆-C₁₀ aryl, and C₅-C₁₀ heteroaryl;
preferably, the Y₁ and Y₂ are chemical bonds, and R₁ is selected from the group consisting of 4-7 membered saturated or partially unsaturated heterocyclyl, 5-10 membered heteroaryl, C₁-C₄ alkyl-(4-7 membered saturated or partially unsaturated heterocyclyl), and C₁-C₄ alkyl-(5-10 membered heteroaryl);
more preferably, Y is 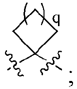 wherein q is 0, 1, 2, or 3, and R₁ is not H;
further preferably, R₁ has a structure selected from the group consisting of

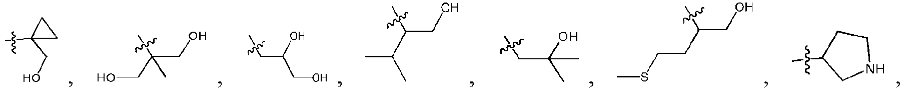

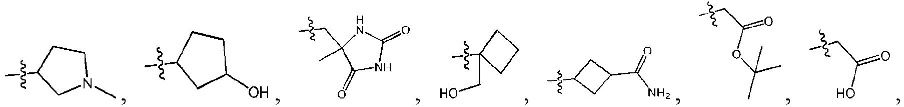

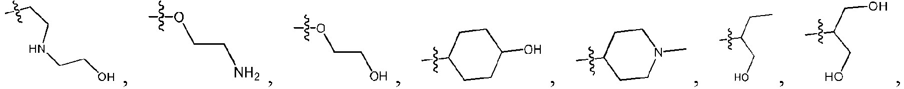

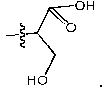

4. The compound according to claim 1, stereoisomer, geometric isomer, hydrate, solvate, prodrug, or pharmaceutically acceptable salt thereof, **characterized in that** the X₁ is NH;
X₂ is CH₂, S or NH.

5. The compound according to claim 1, stereoisomer, geometric isomer, hydrate, solvate, prodrug, or pharmaceutically acceptable salt thereof, **characterized in that** the compound has a structure represented by formula (IA), formula (IVA), or formula (IIA): 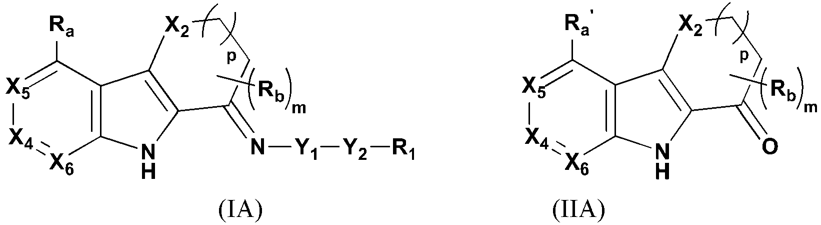 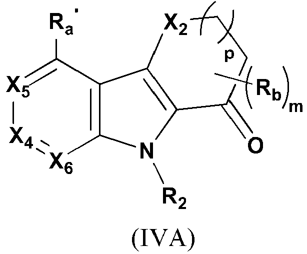
wherein the definitions of Y₁, Y₂, R₁, R₂, Rₐ, Rₐ', R_{b}, X₂, m, and p are as described above;
X₄, X₅ and X₆ are each independently selected from the group consisting of CRₐ₁ and N; wherein Rₐ₁ is selected from the group consisting of H, D, hydroxyl, halogen, amino, nitro, C₁-C₄ alkyl, C₂-C₄ alkenyl, and C₂-C₄ alkynyl;
preferably, Rₐ has a structure selected from the group consisting of C₁-C₄ alkyl, C₁-C₄ alkoxy, -C(O)OR_{c1}, and -NR_{c1}R_{d1}; wherein R_{c1} and R_{d1} are as defined above;
preferably, Rₐ' has a structure selected from the group consisting of deuterium, hydroxyl, halogen, amino, nitro, cyano, C₁-C₄ alkyl, C₂-C₆ ester group, C₁-C₄ haloalkyl, C₃-C₇ saturated or partially unsaturated carbocyclyl, 3-12 membered saturated or partially unsaturated heterocyclyl, C₆-C₁₀ aryl, 5-12 membered heteroaryl, -NH-CH₂-(3-12 membered saturated or partially unsaturated heterocyclyl), -NH-CH₂-(5-12 membered saturated or partially unsaturated heteroaryl), -P(O)R_{c}R_{d}, -S(O)₂R_{c}, -S(O)₂NH₂, -S(O)(NH₂)(R_{c}), -NH-S(O)₂R_{c}, - N=S(O)R,R_{d}, -CH₂-S(O)(NH)(R_{c}), -C(O)NHR_{c}, -C(O)NR_{c}R_{d}, -OC(O)R_{c}, -C(O)OR_{c}, -C(O)O(CH₂)R_{c}, -C(O)NH(C₁-C₄ alkylene)R_{c} and -NR_{c}R_{d}; wherein R_{c} and R_{d} are as defined above;
wherein one or more hydrogen atoms on Rₐ₁, Rₐ and Rₐ' may optionally be substituted by substituents selected from the group consisting of deuterium atom, hydroxyl, halogen, amino, cyano, carboxyl, oxo group (=O), 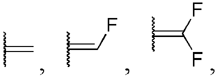 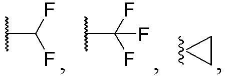 C₁-C₄ alkyl, C₂-C₄ alkenyl, C₂-C₄ alkynyl, C₁-C₄ alkoxy, HO-(C₁-C₄ alkylene)-, C₁-C₄ alkylthio, C₂-C₄ alkenyl, C₂-C₆ ester group, S(O)₂CH₃, C₃-C₇ saturated or partially unsaturated carbocyclyl, -C(O)N(C₁-C₄ alkyl)₂, -N(C₁-C₄ alkyl)₂, and C₆-C₁₀ aryl.

6. The compound according to claim 1, stereoisomer, geometric isomer, hydrate, solvate, prodrug, or pharmaceutically acceptable salt thereof, the compound has a structure represented by formula (IIIA) or formula (IVA): 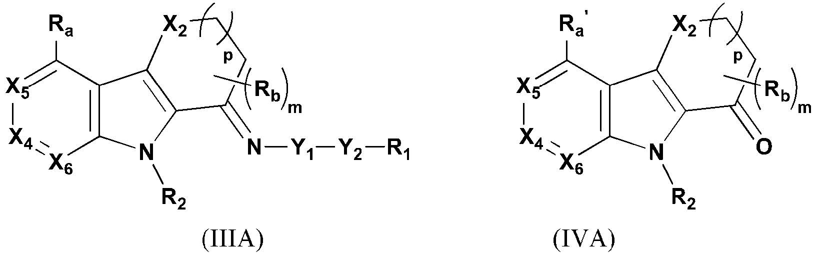 wherein the definitions of Y₁, Y₂, R₁, R₂, Rₐ, Rₐ', R_{b}, X₂, m and p are as described in claim 1, X₄, X₅ and X₆ are as described in claim 5.

7. The compound according to claim 1, stereoisomer, geometric isomer, hydrate, solvate, prodrug, or pharmaceutically acceptable salt thereof, the compound has a structure selected from the group consisting of:
| No. | Compound Structure | No. | Compound Structure |
|---|---|---|---|
| E01 | 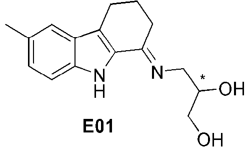 | B01 | 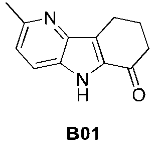 |
| E02 | 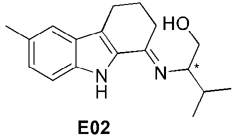 | B02 | 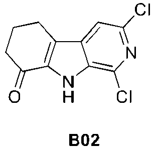 |
| E03 | 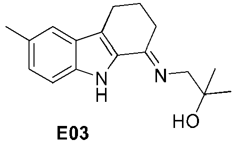 | B03 | 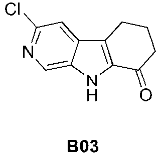 |
| E04-R | 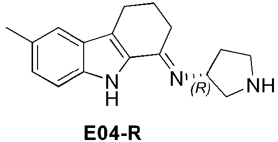 | B04 | 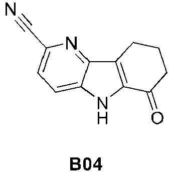 |
| E05-R | 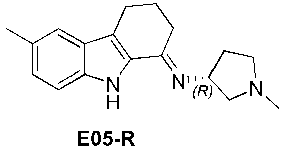 | B05 | 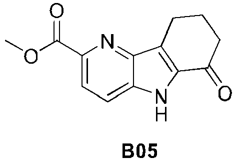 |
| E06-SR | 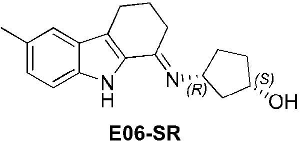 | B06 | 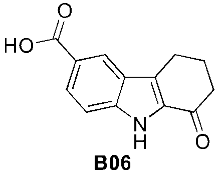 |
| E07 | 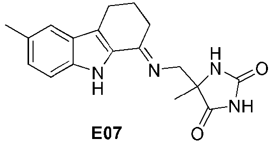 | B07 | 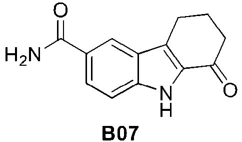 |
| E08 | 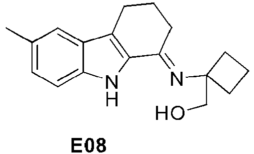 | B08 | 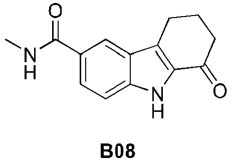 |
| E09 | 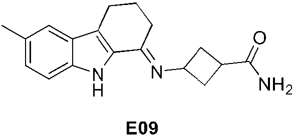 | B09 | 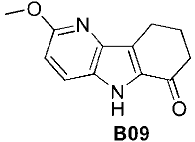 |
| E10 | 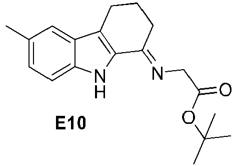 | B10 | 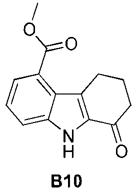 |
| E11 | 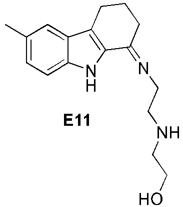 | B11 | 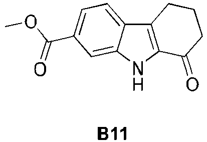 |
| E12 | 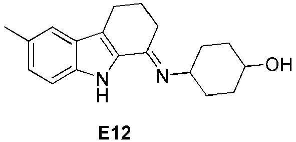 | B12 | 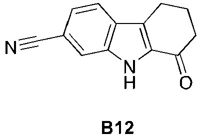 |
| E13 | 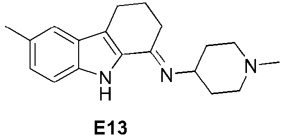 | B13 | 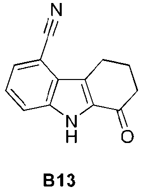 |
| E14 | 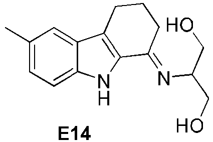 | B14 | 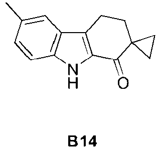 |
| E15 | 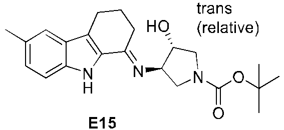 | B15 | 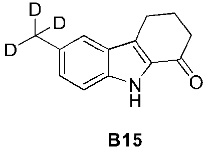 |
| E16 | 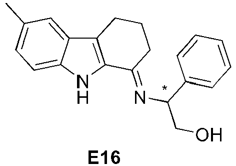 | B16 | 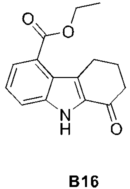 |
| E17 | 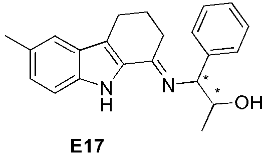 | B17 | 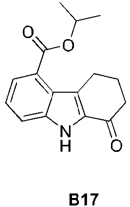 |
| E18 | 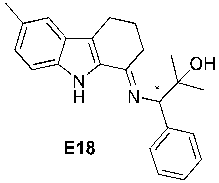 | B18 | 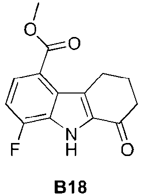 |
| E19 | 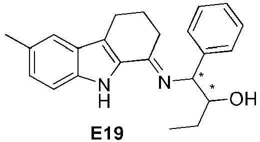 | B19 | 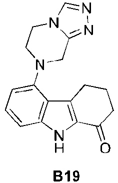 |
| E20 | 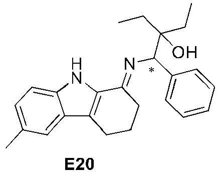 | B20 | 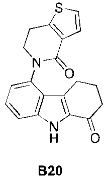 |
| E21 | 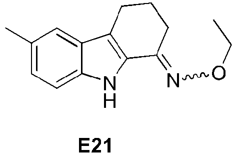 | B21 | 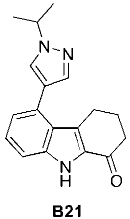 |
| E22 | 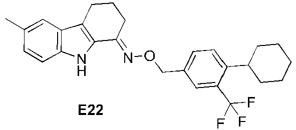 | B22 | 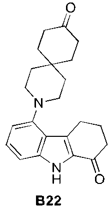 |
| E23 | 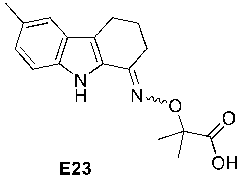 | B23 | 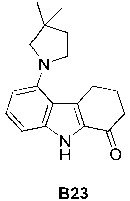 |
| E24 | 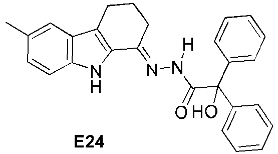 | B24 | 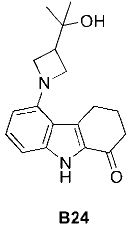 |
| E25 | 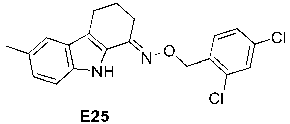 | B25 | 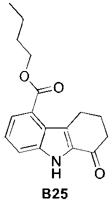 |
| E26 | 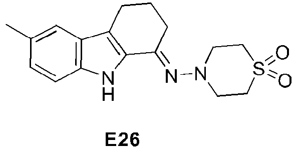 | B26 | 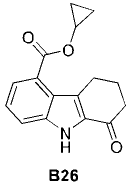 |
| E27 | 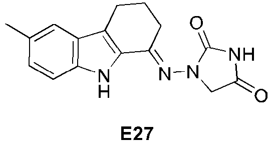 | B27 | 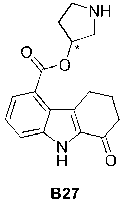 |
| E28 | 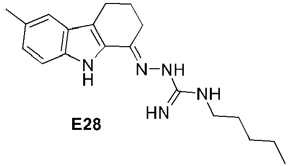 | B28 | 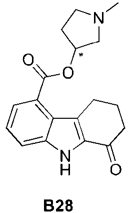 |
| E29 | 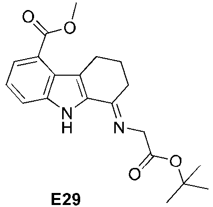 | B29 | 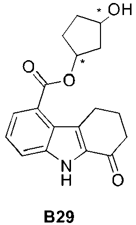 |
| E30 | 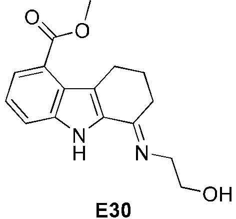 | B30 | 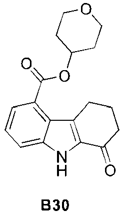 |
| E31 | 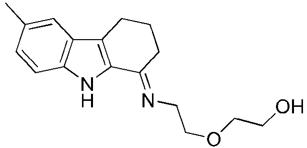 | B31 | 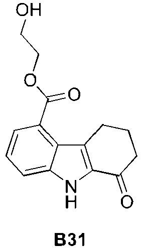 |
| E32 | 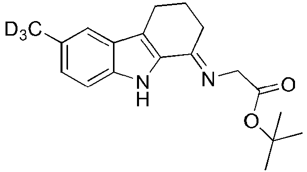 | B32 | 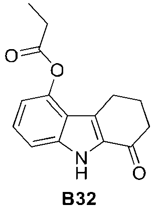 |
| E33 | 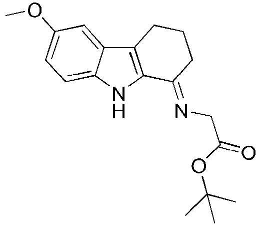 | B33 | 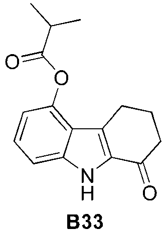 |
| E34 | 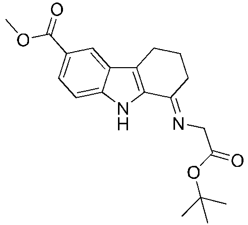 | B34 | 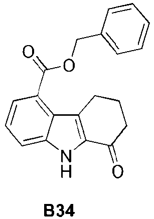 |
| E35 | 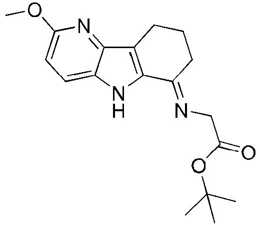 | B35 | 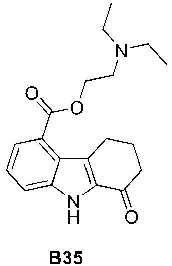 |
| E36 | 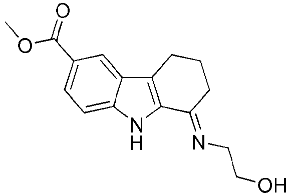 | B36 | 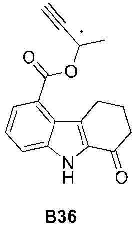 |
| E37 | 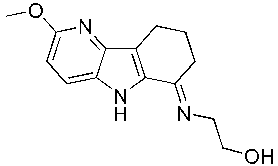 | B37 | 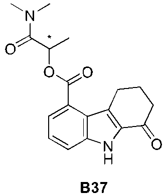 |
| E38 | 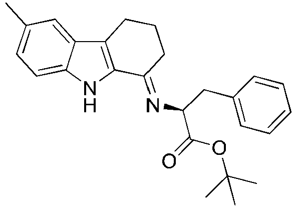 | B38 | 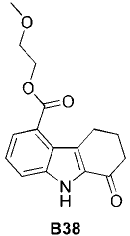 |
| E39 | 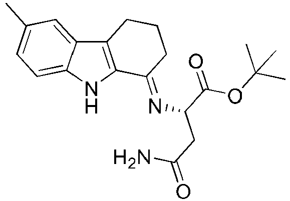 | B39 | 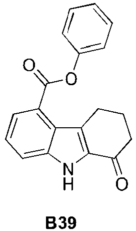 |
| E40 | 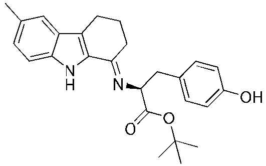 | B40 | 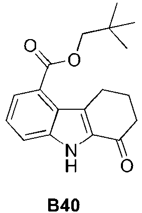 |
| E41 | 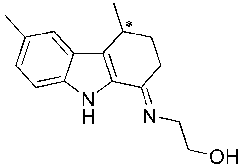 | B41 | 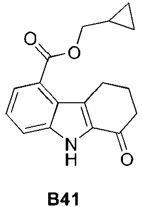 |
| E42 | 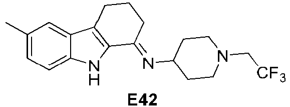 | B42 | 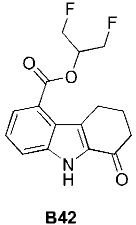 |
| E43 | 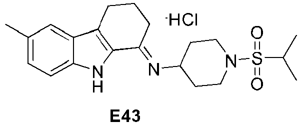 | B43 | 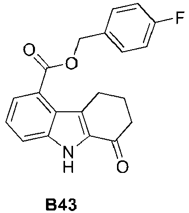 |
| E44 | 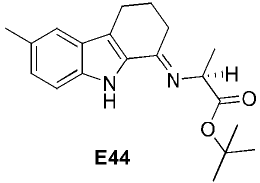 | B44 | 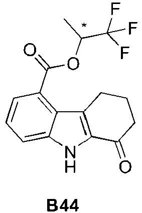 |
| E45 | 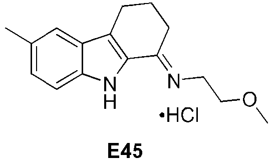 | B45 | 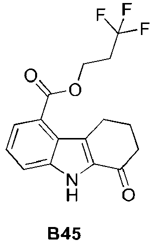 |
| E46 | 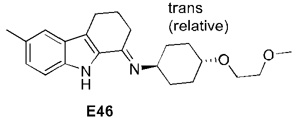 | B46 | 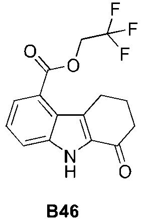 |
| E47 | 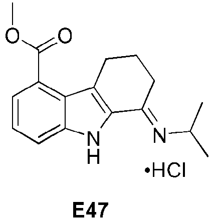 | B47 | 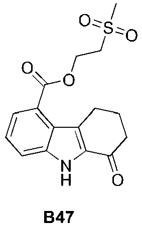 |
| E48 | 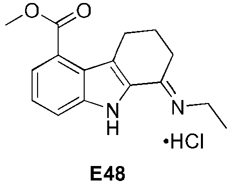 | B48 | 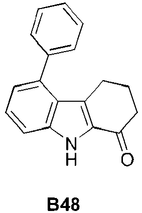 |
| E49 | 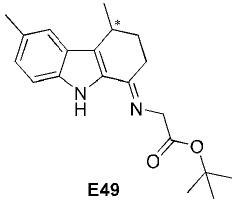 | B49 | 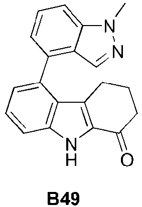 |
| E50 | 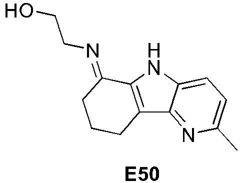 | B50 | 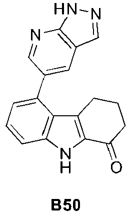 |
| E51 | 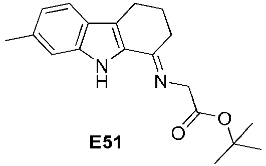 | B51 | 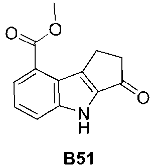 |
| E52 | 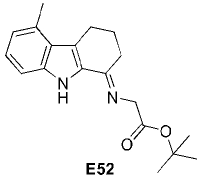 | B52 | 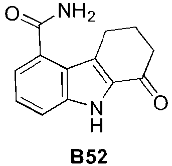 |
| E53 | 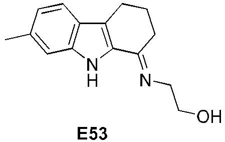 | B53 | 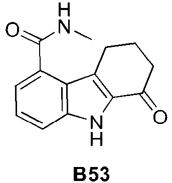 |
| E54 | 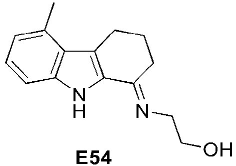 | B54 | 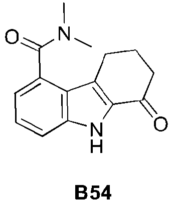 |
| E55 | 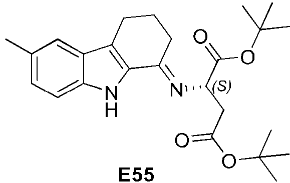 | B55 | 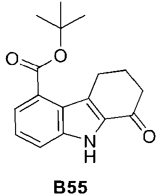 |
| E56 | 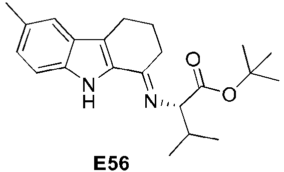 | B56 | 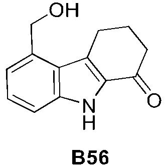 |
| E57 | 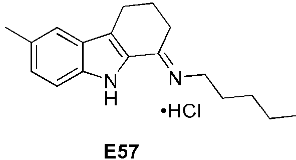 | B57 | 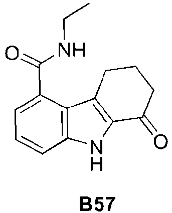 |
| E58 | 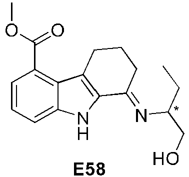 | B58 | 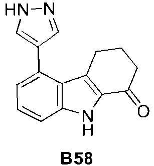 |
| E59 | 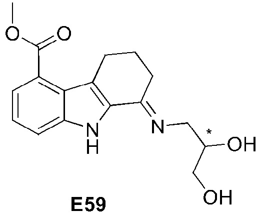 | B59 | 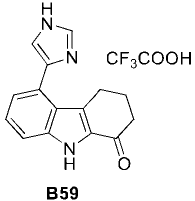 |
| E60 | 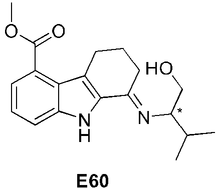 | B60 | 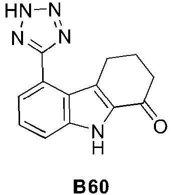 |
| E61 | 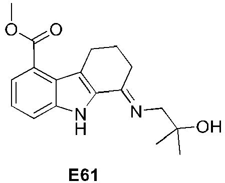 | B61 | 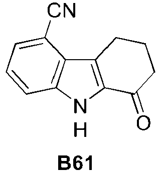 |
| E62 | 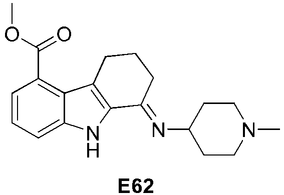 | B62 | 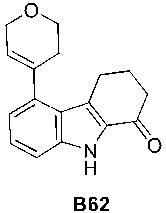 |
| E63 | 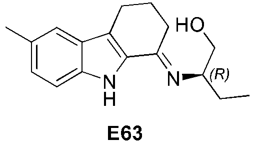 | B63 | 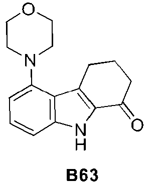 |
| E64 | 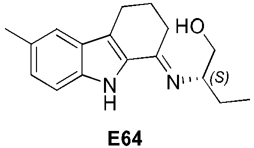 | B64 | 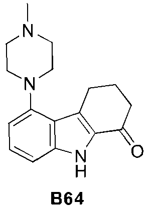 |
| E65 | 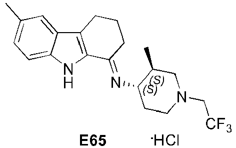 | B65 | 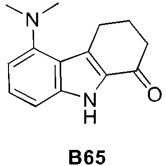 |
| E66 | 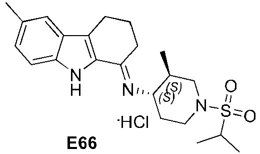 | B66 | 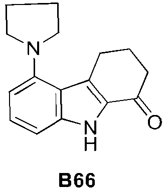 |
| E67 | 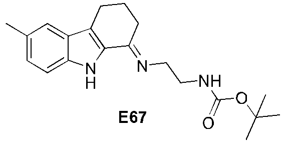 | B67 | 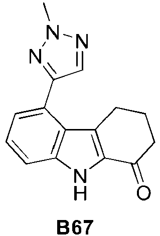 |
| E68 | 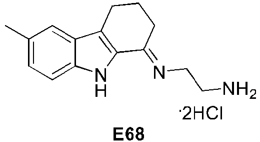 | B68 | 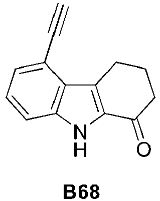 |
| E69 | 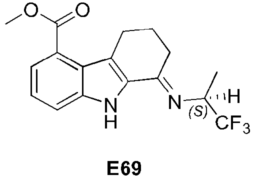 | B69 | 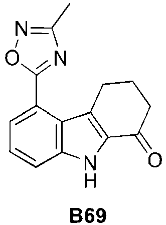 |
| E70 | 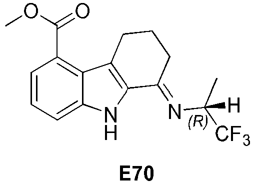 | B70 | 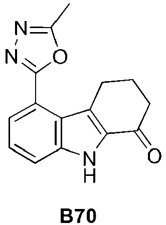 |
| E71 | 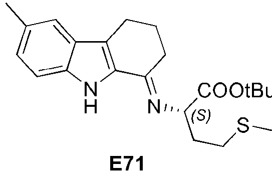 | B71 | 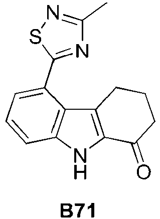 |
| E72 | 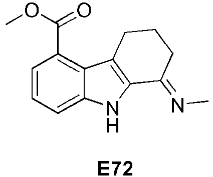 | B72 | 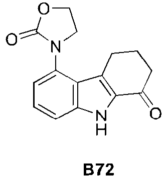 |
| E73 | 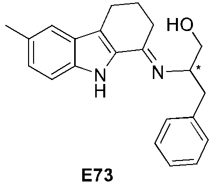 | B73 | 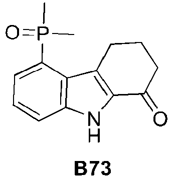 |
| C01 | 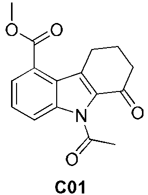 | B74 | 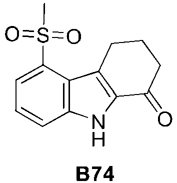 |
| C02 | 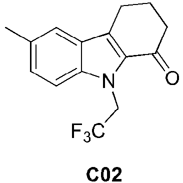 | B75 | 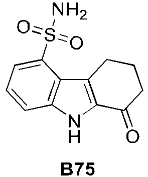 |
| C03 | 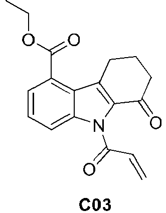 | B76 | 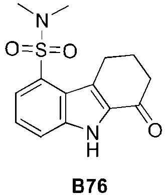 |
| C04 | 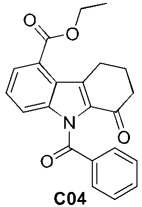 | B77 | 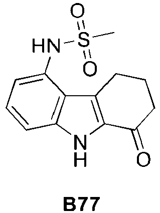 |
| C05 | 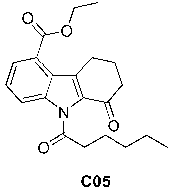 | B78 | 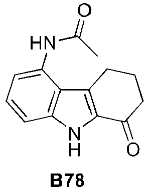 |
| C06 | 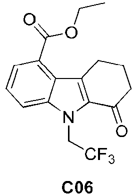 | B79 | 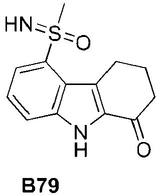 |
| C07 | 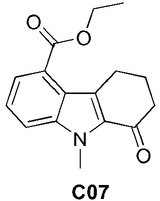 | B80 | 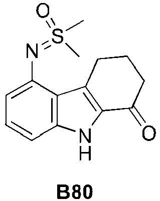 |
| C08 | 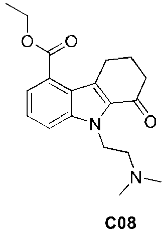 | B81 | 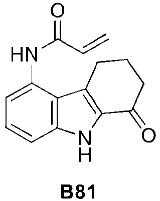 |
| B82 | 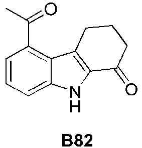 | B83 | 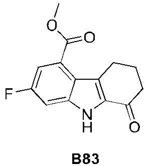 |
| B84 | 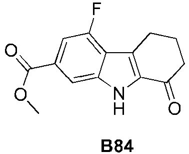 | B85 | 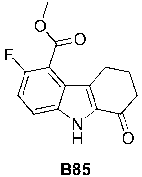 |
| B86 | 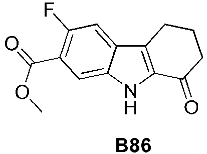 | B87 | 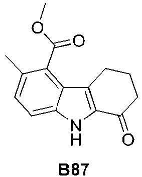 |
| B88 | 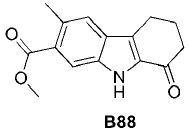 | B89 | 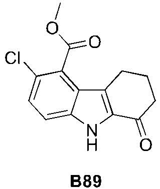 |
| B90 | 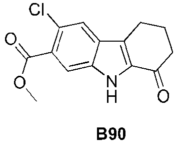 | B91 | 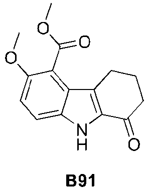 |
| B92 | 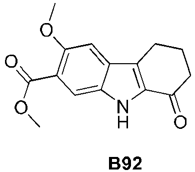 | B93 | 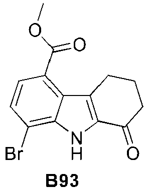 |
| B94 | 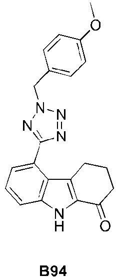 | B95 | 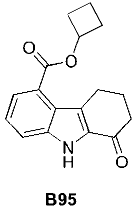 |
| B96 | 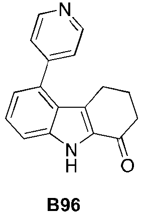 | B97 | 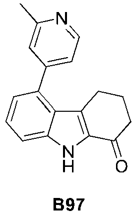 |
| B98 | 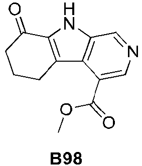 | B99 | 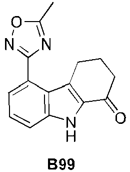 |
| B100 | 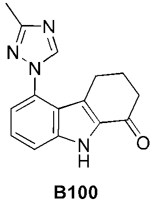 | B103 | 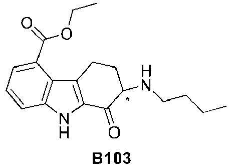 |
| B104 | 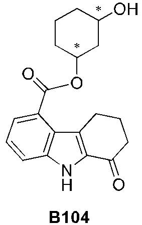 | B105 | 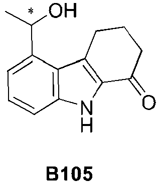 |
| B106 | 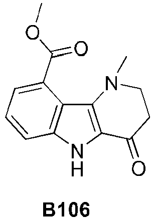 | B107 | 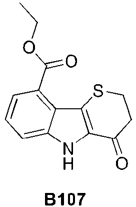 |
| B108 | 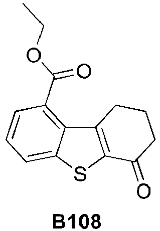 | B109 | 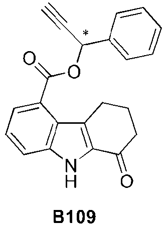 |
| B110 | 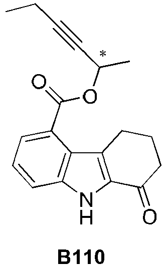 | B111 | 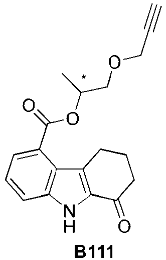 |
| B112 | 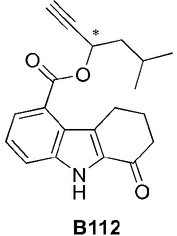 | B113 | 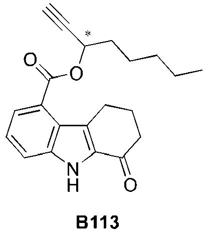 |
| B114 | 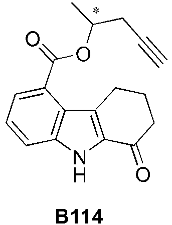 | B115 | 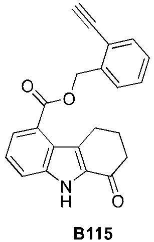 |
| B116 | 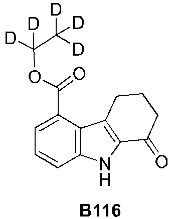 | B117 | 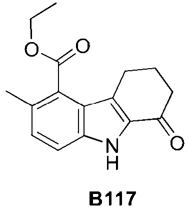 |
| B118 | 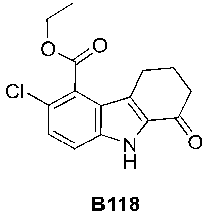 | B119 | 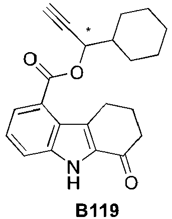 |
| B120 | 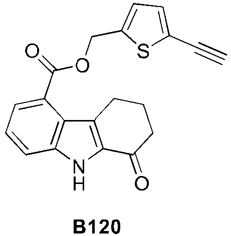 | B121 | 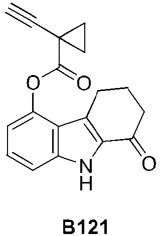 |
| B122 | 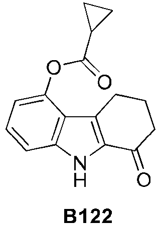 | B123 | 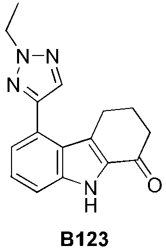 |
| B124 | 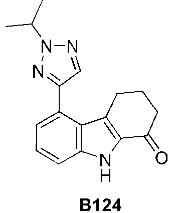 | B125 | 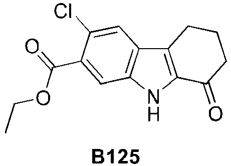 |
| B126 | 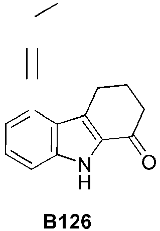 | B127 | 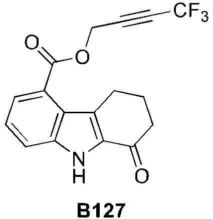 |
| B128 | 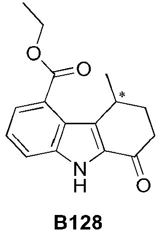 | B129 | 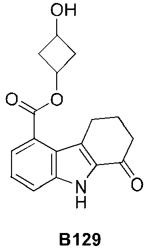 |
| B130 | 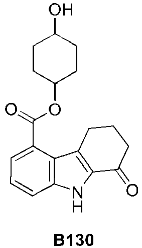 | B131 | 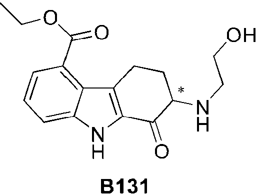 |
| B132 | 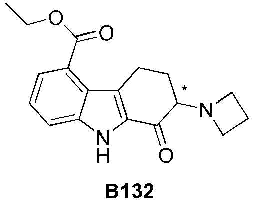 | B133 | 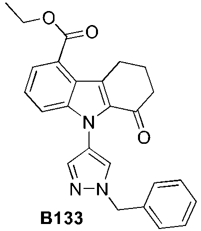 |
| B134 | 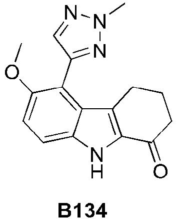 | B135 | 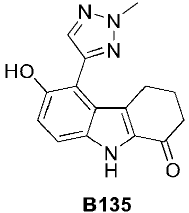 |
| B136 | 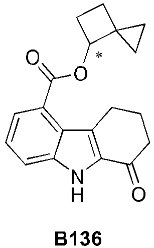 | B137 | 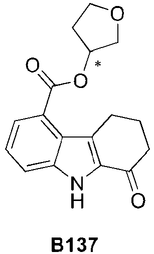 |
| B138 | 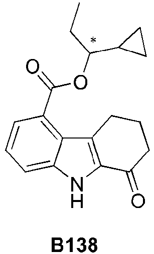 | B139 | 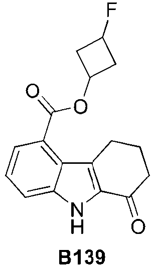 |
| B140 | 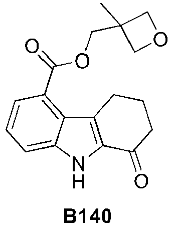 | B141 | 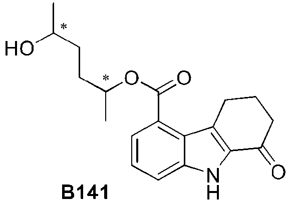 |
| B142 | 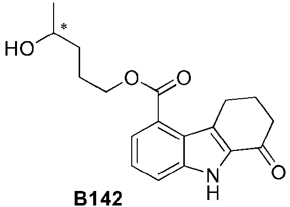 | B142 S | 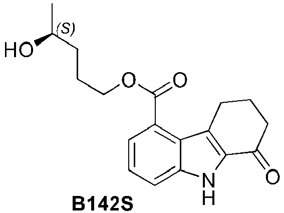 |
| B145 | 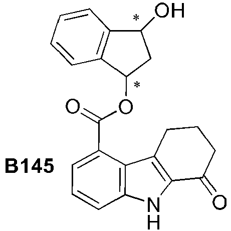 | B146 | 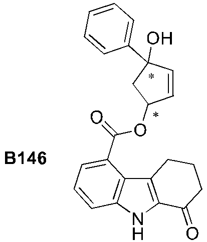 |
| B147 | 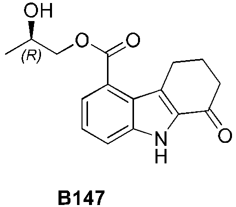 | B148 | 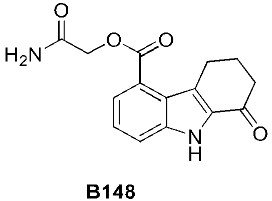 |
| B149 | 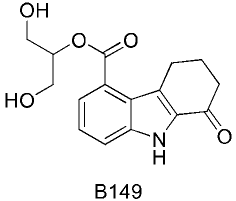 | B150 | 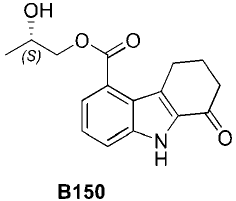 |
| B151 | 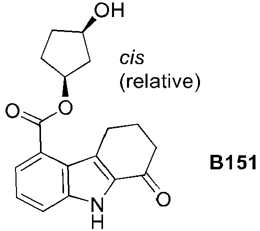 | B152 | 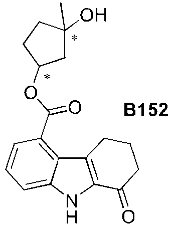 |
| B153 | 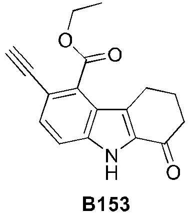 | B154 | 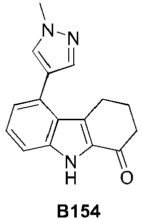 |
| B155 | 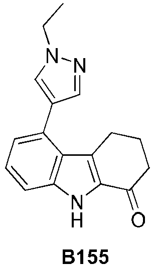 | B156 | 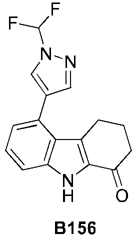 |
| B157 | 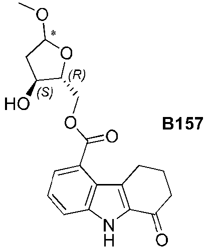 | B158 | 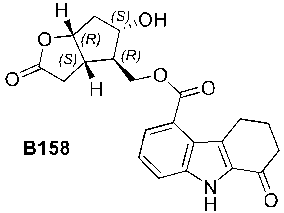 |
| B159 | 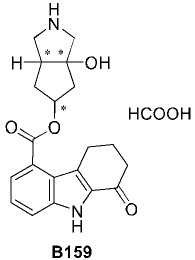 | B160 | 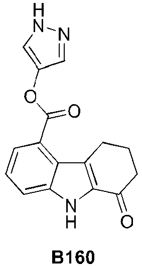 |
| B161 | 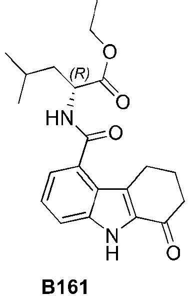 | B162 | 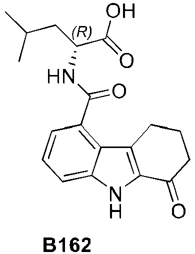 |
| B163 | 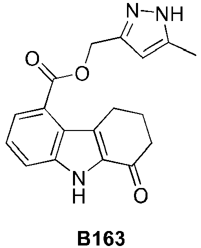 | B164 | 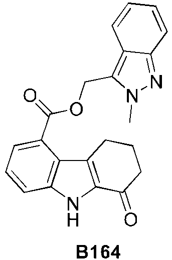 |
| B165 | 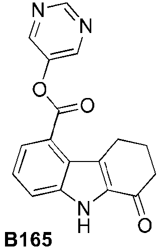 | B166 | 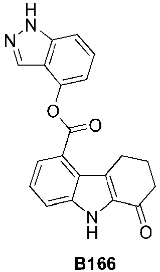 |
| B167 | 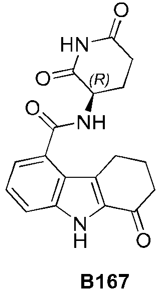 | B168 | 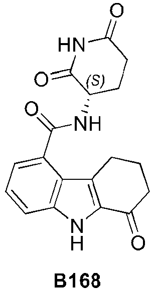 |
| B169 | 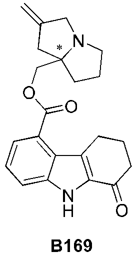 | B170 | 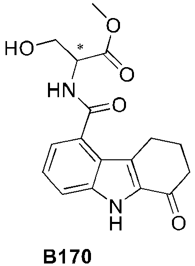 |
| B171 | 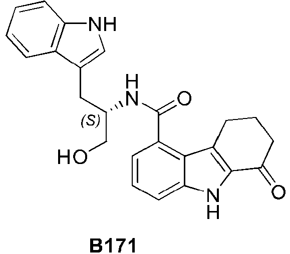 | B172 | 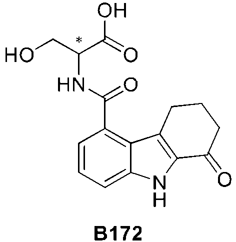 |
| B173 | 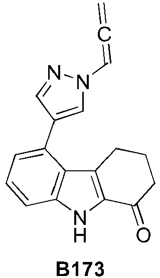 | B174 | 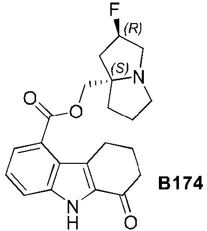 |
| B175 | 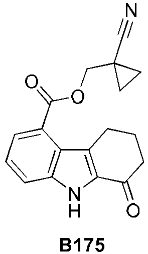 | B176 | 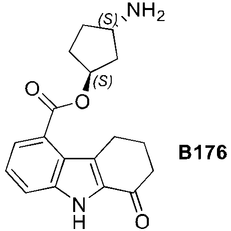 |
| B177 | 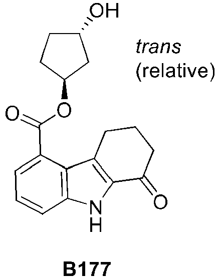 | B178 | 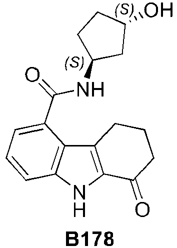 |
| B179 | 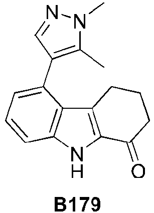 | B180 | 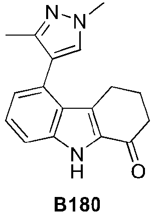 |
| B181 | 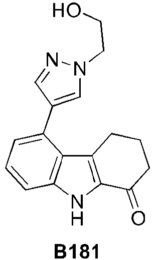 | B182 | 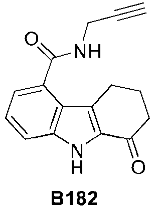 |
| B183 | 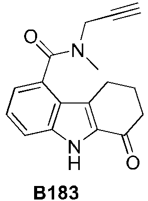 | B184 | 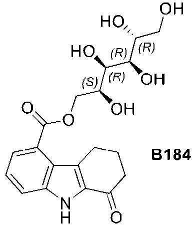 |
| B185 | 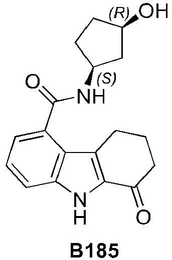 | B186 | 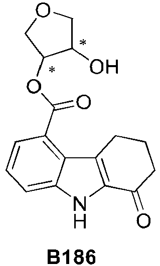 |
| B187 | 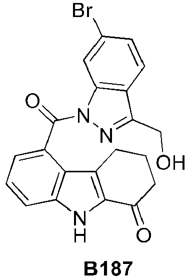 | B188 | 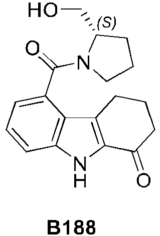 |
| B189 | 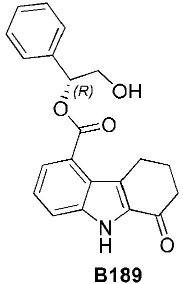 | B190 | 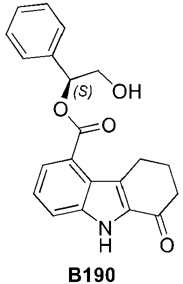 |
| B191 | 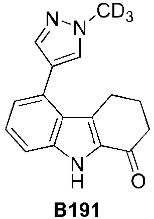 | B192 | 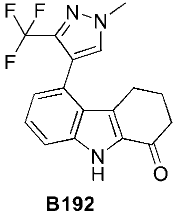 |
| B193 | 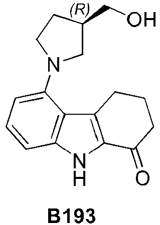 | B194 | 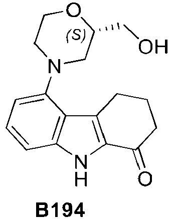 |
| B195 | 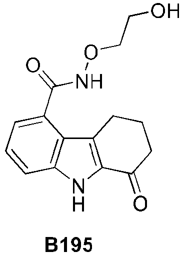 | B196 | 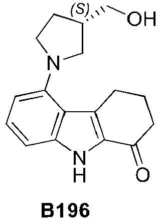 |
| B197 | 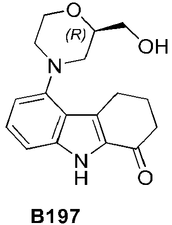 | B198 | 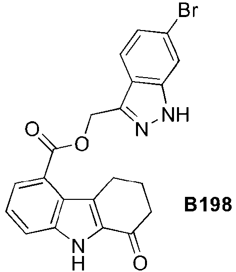 |
| B199 | 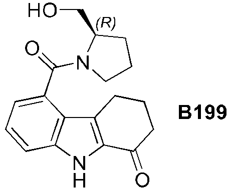 | B200 | 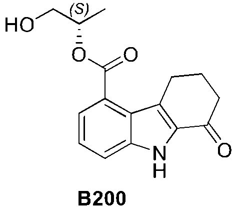 |
| B201 | 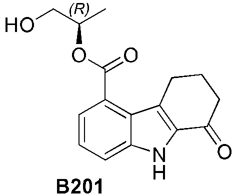 | B202 | 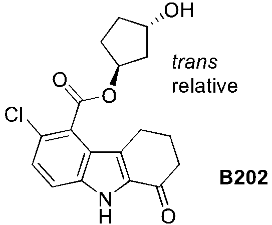 |
| B203 | 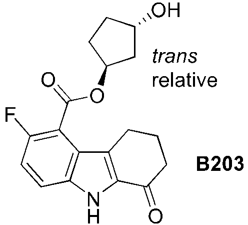 | B204 | 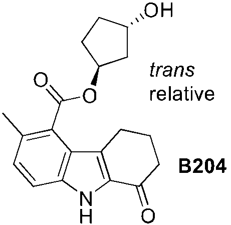 |
| C11 | 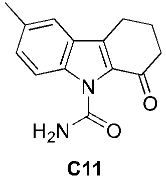 | C12 | 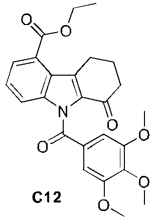 |
| C13 | 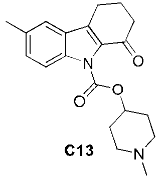 | C14 | 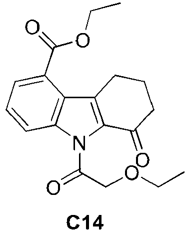 |
| C15 | 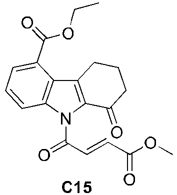 | C16 | 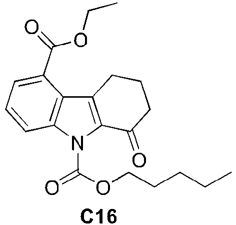 |
| C17 | 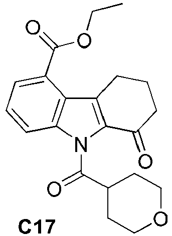 | C18 | 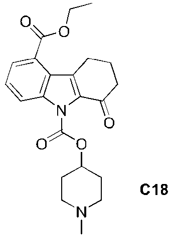 |
| C19 | 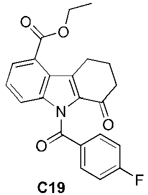 | C20 | 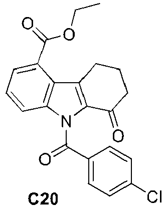 |
| C21 | 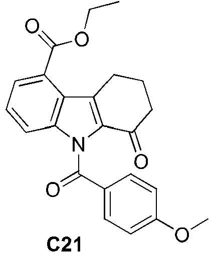 | C22 | 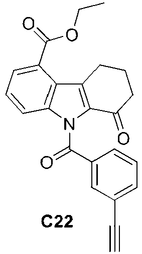 |
| C23 | 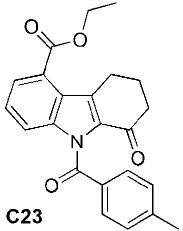 | C24 | 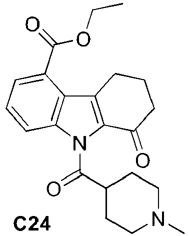 |
| C25 | 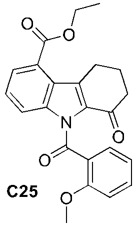 | C26 | 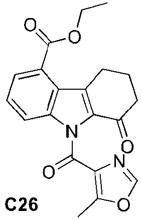 |
| C27 | 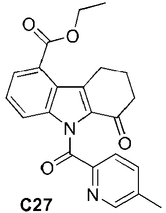 | C28 | 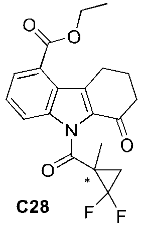 |
| C29 | 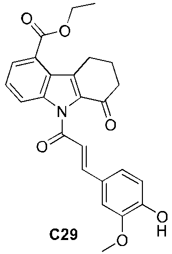 | C30 | 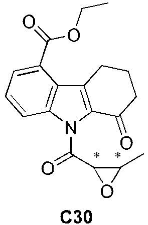 |
| C31 | 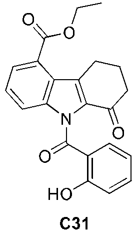 | C32 | 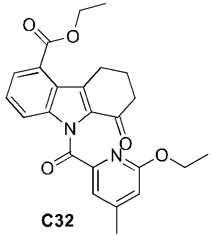 |
| C33 | 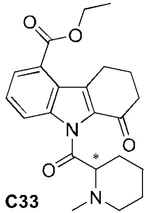 | C34 | 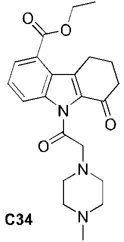 |
| C35 | 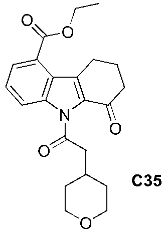 | C36 | 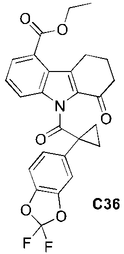 |
| C37 | 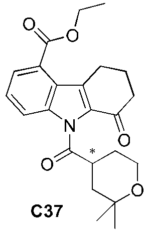 | C38 | 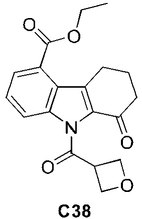 |
| C39 | 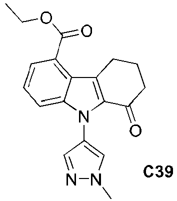 | C40 | 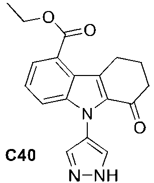 |
| C41 | 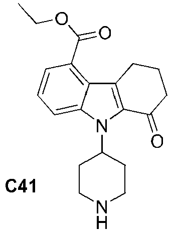 | C42 | 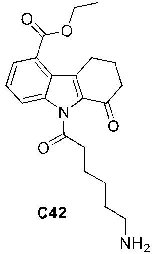 |
| C43 | 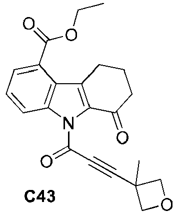 | C44 | 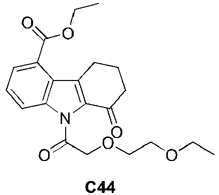 |
| C45 | 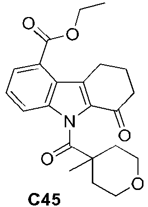 | C48S | 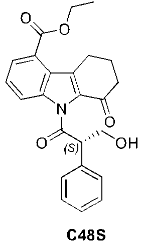 |
| C48 | 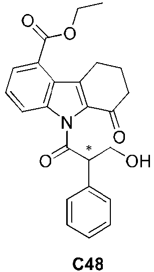 | C49 | 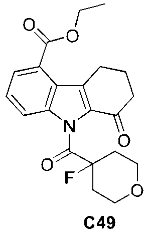 |
| C50 | 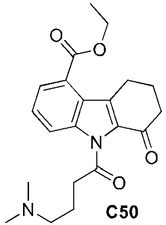 | C51 | 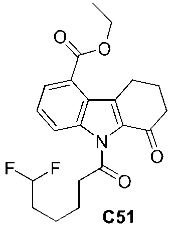 |
| C52 | 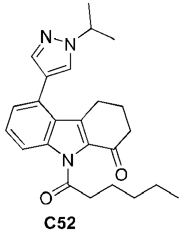 | C53 | 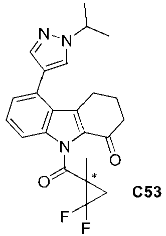 |
| C53S | 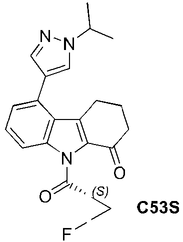 | C55 | 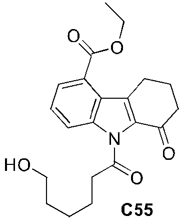 |
| C56 | 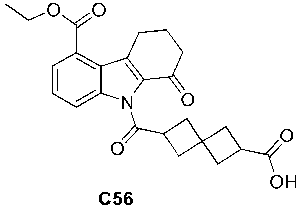 | C57 | 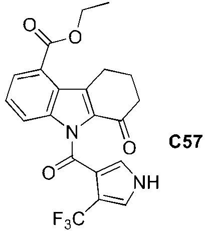 |
| C58 | 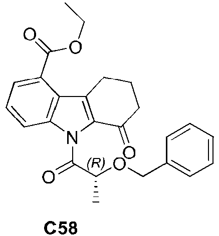 | C59 | 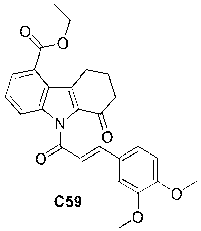 |
| C60 | 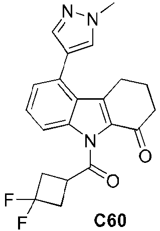 | C61 | 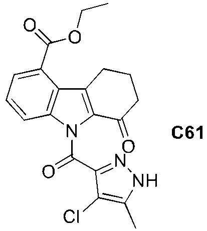 |
| C62 | 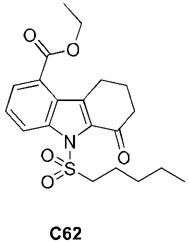 | C63 | 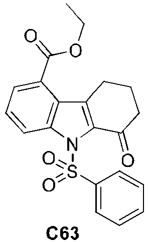 |
| C64 | 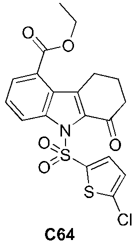 | C65 | 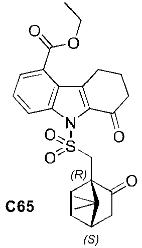 |
| C66 | 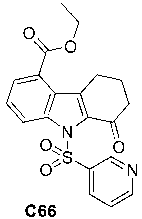 | C67 | 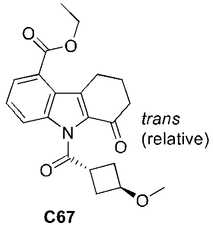 |
| C68 | 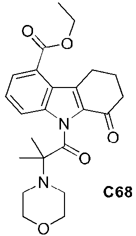 | C69 | 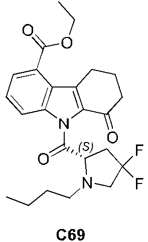 |
| C70 | 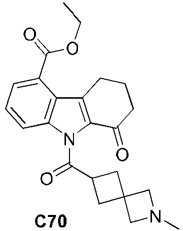 | C71 | 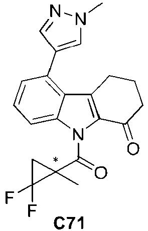 |
| C72 | 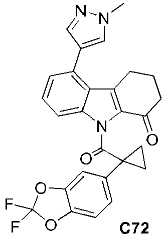 | C73 | 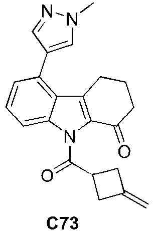 |
| C74 | 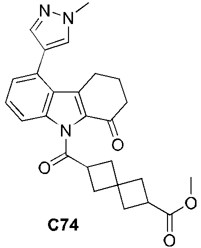 | C75 | 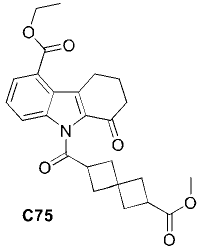 |
the "*" indicates a chiral center.

8. A pharmaceutical composition comprising:
(1) a compound according to any of claims 1-7, or a stereoisomer, geometric isomer, hydrate, solvate, prodrug, or pharmaceutically acceptable salt thereof; and
optionally (2) a pharmaceutically acceptable carrier, excipient, or other active pharmaceutical ingredients;
preferably, the pharmaceutical composition further comprises a second therapeutic component, and the second therapeutic component is selected from the group consisting of PARP (poly ADP-ribose polymerase) inhibitors, proteasome inhibitors, kinase inhibitors, DNA methyltransferase inhibitors, histone deacetylase inhibitors, nucleoside drugs that interfere with nucleic acid metabolism in tumor cells, viruses, and bacteria, chemotherapeutic agents that inhibit tumor cell DNA replication such as platinum-based drugs, paclitaxel and its derivatives, drugs and treatment regimens that inhibit the uptake and metabolism of glucose, amino acids, fatty acids, etc., or other therapeutic components and treatment regimens that can produce synergistic antitumor effects with the aforementioned tetrahydrocarbazole compounds.
more preferably, the PARP inhibitor is Olaparib, Rucaparib, Niraparib, Talazoparib, Fluzoparib, Pamiparib or Saruparib;
the proteasome inhibitor is Ixazomib or Bortezomib;
the kinase inhibitor is a MEK inhibitor; the MEK inhibitor is Cobimetinib;
the DNA methyltransferase inhibitor is SGI-1027;
the histone deacetylase inhibitor is Trichostatin A;
the nucleoside analog is 6-Mercaptopurine (6-MP);
the chemotherapeutic agent is Cisplatin or Cabazitaxel;
the metabolic inhibitor is the GLUT1/GLUT3 inhibitor KL-11743 or BAY-876; and the metabolic treatment regimen is intermittent fasting.

9. A use of the compound according to any one of claim 1-7, or a stereoisomer, geometric isomer, hydrate, solvate, prodrug, or pharmaceutically acceptable salt thereof, or the pharmaceutical composition according to claim 8, in the manufacture of a medicament for treating and/or preventing cancer;
preferably, the cancer is selected from the group consisting of liver cancer, lung cancer, breast cancer, pancreatic cancer, gastric cancer, colorectal cancer, kidney cancer, prostate cancer, cervical cancer, ovarian cancer, anal and perianal cancer, vulvar cancer, vaginal cancer, penile cancer, head and neck cancer, glioma, skin cancer, mesothelioma, muscle cancer, leukemia, lymphoma, and cancers and related lesions caused by oncogenic viruses such as herpesvirus, adenovirus, hepatitis virus, retrovirus, and the papovavirus family.

10. The use according to claim 9, **characterized in that** the treatment and/or prevention of cancer comprises: administering the compound represented by formula (I) or (II) according to any of claim 1-7, or a stereoisomer, geometric isomer, hydrate, solvate, prodrug, or pharmaceutically acceptable salt thereof, or the pharmaceutical composition according to claim 8 in combination with a component selected from the group consisting of: PARP inhibitors, proteasome inhibitors, MEK inhibitors, DNA methyltransferase inhibitors, histone deacetylase inhibitors, nucleoside analogs that interfere with nucleic acid metabolism in tumor cells, viruses, and bacteria, chemotherapeutic agents that inhibit tumor cell DNA replication such as platinum-based drugs, paclitaxel and its derivatives, drugs and treatment regimens that inhibit the uptake and metabolism of glucose, amino acids, fatty acids, etc., or other therapeutic components and treatment regimens that can produce synergistic antitumor effects with the compound of formula (I).

11. The use according to claim 9, **characterized in that** the PARP inhibitor is Olaparib, Rucaparib, Niraparib, Talazoparib, Fluzoparib, Pamiparib, or Saruparib; the proteasome inhibitor is Ixazomib or Bortezomib; the kinase inhibitor is Cobimetinib; the DNA methyltransferase inhibitor is SGI-1027; the histone deacetylase inhibitor is Trichostatin A; the nucleoside analog is 6-Mercaptopurine (6-MP); the chemotherapeutic agent is Cisplatin or Cabazitaxel; the metabolic inhibitor is the GLUT1/GLUT3 inhibitor KL-11743 or BAY-876; and the metabolic treatment regimen is intermittent fasting.
